(19) Europäisches Patentamt
European Patent Office
Office européen des brevets

(11) EP 2 269 990 A1

(12) # EUROPEAN PATENT APPLICATION

published in accordance with Art. 153(4) EPC

(43) Date of publication:
**05.01.2011 Bulletin 2011/01**

(21) Application number: **09724818.1**

(22) Date of filing: **25.03.2009**

(51) Int Cl.:
***C07D 231/56*** *(2006.01)* ***A61K 31/416*** *(2006.01)*
***A61K 31/4178*** *(2006.01)* ***A61K 31/4245*** *(2006.01)*
***A61K 31/427*** *(2006.01)* ***A61K 31/437*** *(2006.01)*
***A61K 31/4439*** *(2006.01)* ***A61K 31/496*** *(2006.01)*
***A61K 31/4985*** *(2006.01)* ***A61K 31/519*** *(2006.01)*
***A61K 31/55*** *(2006.01)* ***A61P 25/14*** *(2006.01)*
***A61P 25/18*** *(2006.01)* ***A61P 25/24*** *(2006.01)*
***A61P 25/28*** *(2006.01)* ***A61P 43/00*** *(2006.01)*

(86) International application number:
**PCT/JP2009/001337**

(87) International publication number:
**WO 2009/119088 (01.10.2009 Gazette 2009/40)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **25.03.2008 JP 2008077479**

(71) Applicant: **Takeda Pharmaceutical Company Limited**
**Osaka 541-0045 (JP)**

(72) Inventors:
- **MOCHIZUKI, Michiyo**
**Osaka-shi**
**Osaka 532-8686 (JP)**
- **MIURA, Shotaro**
**Osaka-shi**
**Osaka 532-8686 (JP)**

(74) Representative: **Held, Stephan et al**
**Meissner, Bolte & Partner GbR**
**Widenmayerstraße 48**
**D-80538 München (DE)**

(54) **HETEROCYCLIC COMPOUND**

(57) An object of the present invention is to provide a novel AMPA receptor potentiator. A compound represented by the following formula (I) or a salt thereof:
wherein in formula (I)
$R^1$ represents
(1) a halogen atom, or (2) cyano group, or the like;

Ra and Rb each independently represent a hydrogen atom or $C_{1-4}$ alkyl;
L represents a bond, or a spacer in which the number of atoms in the main chain is 1 to 8;
Ring A represents
(1) a non-aromatic carbon ring of 4-8 carbon atoms, or
(2) a 4- to 8-membered non-aromatic heterocycle
either of which is optionally substituted with 1 or more substituents selected from
(a) halogen atoms, and (b) cyano group; and

Ar represents
a substituted phenyl group, or
optionally substituted 5- or 6-membered aromatic heterocyclic group.



Printed by Jouve, 75001 PARIS (FR)

**Description**

[Technical Field]

The present invention relates to a heterocyclic compound, particularly a heterocyclic compound which potentiates the AMPA receptor.

[Background of the Invention]

Glutamic acid is the most abundant excitatory neurotransmitter in the central nervous system of mammals. Glutamic acid plays a major role in the regulation of cognition, mood, and motor function; these processes are unstable in mental illness and nervous disorders.
The AMPA receptor is a receptor for the excitatory neurotransmitter, glutamic acid; AMPA (α-amino-3-hydroxy-5-isoxazole-4-propionic acid) was named based on its selective activation of this receptor.
The importance of the AMPA receptor in brain physiology is well known, and compounds which potentiate the AMPA receptor are expected to be useful as drugs for preventing or treating mental illness, neurodegenerative diseases, memory impairment, sleep disorders, and the like.
Heterocyclic compounds represented by the following general formula which potentiate the AMPA receptor have been disclosed as such compounds in PTL 1.

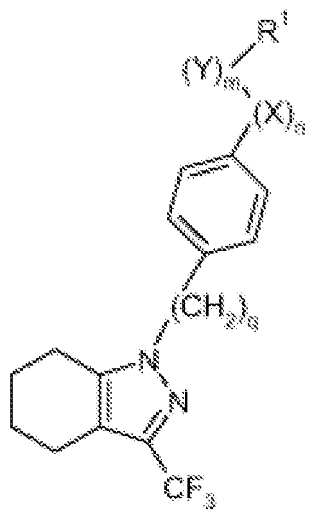

Heterocyclic compounds represented by the following general formula which potentiate the AMPA receptor have also been disclosed in PTL 2.

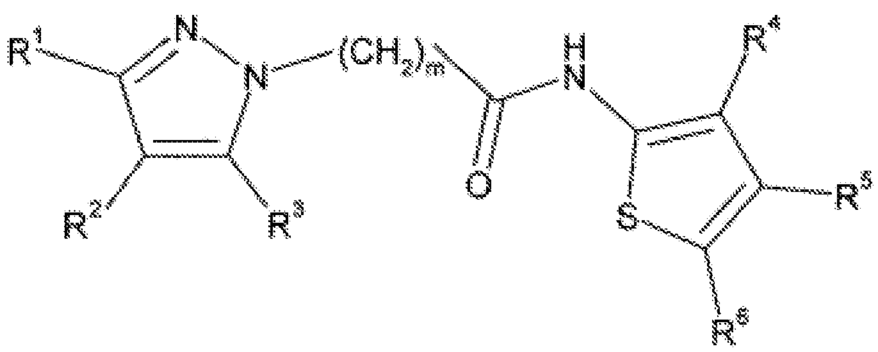

However, there is a need for the development of more heterocyclic compounds which potentiate the AMPA receptor.

[PTL 1]
International Publication WO 2007/107539 Pamphlet
[PTL 2]
International Publication WO 2008/003452 Pamphlet

[Summary of Invention]

[Technical Problem]

An object of the present invention is to provide a heterocyclic compound which potentiates the AMPA receptor.

[Solution to Problem]

The present inventors found that compounds represented by the following formula (I) or salts thereof (herein also referred to as compounds (I)) potentiate the AMPA receptor, and the present invention was perfected upon further investigation.
A compound represented by the formula

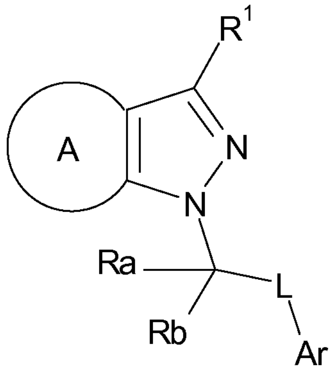
(I)

[wherein
$R^1$ represents

(1) a halogen atom,
(2) cyano group,
(3) alkyl group optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups, alkanoyloxy groups,
alkanoyl groups,
carbamoyl group, and
mono- or di-alkylcarbamoyl groups,
(4) optionally substituted cycloalkyl group,
(5) group represented by the formula:

$-OR^{x1}$,
$-SR^{x2}$,
$-CO-R^{x2}$,

-CS-$R^{x2}$,
-SO-$R^{x2}$,
-$SO_2$-$R^{x2}$,
-$NH_2$, or
-$NR^{x1}R^{x2}$

(where $R^{x1}$ represents a substituent selected from the following substituent group A, and $R^{x2}$ represents hydrogen or a substituent selected from the following substituent group A),
or
(6) optionally substituted non-aromatic heterocyclic group;

Ra and Rb each independently represent a hydrogen atom or $C_{1-4}$ alkyl group;
L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1), either of which is optionally substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) optionally substituted cycloalkyl groups,
(e) groups represented by the formula -$OR^{y1}$,
-$SR^{y1}$,
-CO-$R^{y1}$,
-CS-$R^{y1}$,
-SO-$R^{y1}$,
-$SO_2$-$R^{y1}$, or
-$NR^{y1}R^{y2}$
(where $R^{y1}$ and $R^{y2}$ each independently represent hydrogen or 1 or 2 substituents selected from the following substituent group A),
(f) oxo group, and
(g) optionally substituted non-aromatic heterocyclic groups;

Ar represents
an optionally substituted aryl group, or optionally substituted 5- or 6-membered aromatic heterocyclic group (when the aryl group or aromatic heterocyclic group has 2 or more substituents, two adjacent substituents together may form an optionally substituted 5- to 8-membered ring).
Provided that, when L is a bond, Ar is not an unsubstituted phenyl group or unsubstituted 5- or 6-membered aromatic

heterocyclic group.
Substituent group A consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,
(xii) $C_{3-8}$ cycloalkenyl groups,
(xiii) cross-linked $C_{7-10}$ cycloalkyl groups optionally substituted with $C_{1-6}$ alkyl group(s),
(xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfonyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.

That is, the present invention is intended to provide the compounds in [1] to [6] below, prodrug in [7], pharmaceuticals in [8] and [9], AMPA receptor potentiators (AMPA receptor potentiators may also be referred to as AMPA receptor positive modulators, AMPAkines, AMPA receptor allosteric modulators, AMPA receptor positive allosteric modulators, and positive allosteric activators of AMPA receptor) in [10] to [12], methods in [13] and [14], and uses in [15] and [16].

[1]

A compound represented by the formula

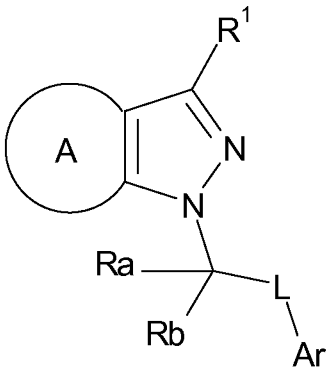

(I)

wherein

$R^1$ represents

(1) a halogen atom,
(2) cyano group,
(3) alkyl group optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfonyl groups,
alkylsulfonyl groups,
mono- or di-alkyl-sulfamoyl groups,
alkanoyloxy groups,
alkanoyl groups,
carbamoyl group, and
mono- or di-alkylcarbamoyl groups,
(4) optionally substituted cycloalkyl group,
(5) group represented by the formula:

$-OR^{x1}$,
$-SR^{x2}$,
$-CO-R^{x2}$,
$-CS-R^{x2}$,
$-SO-R^{x2}$,
$-SO_2-R^{x2}$,
$-H_2$, or
$-NR^{x1}R^{x2}$

(where $R^{x1}$ represents a substituent selected from the following substituent group A, and $R^{x2}$ represents hydrogen or a substituent selected from the following substituent group B), or
(6) optionally substituted non-aromatic heterocyclic group;

Ra and Rb each independently represent a hydrogen atom or $C_{1-4}$ alkyl group;
L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1),

either of which is optionally substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) optionally substituted cycloalkyl groups,
(e) groups represented by the formula:

$-OR^{y1}$,
$-SR^{y1}$,
$-CO-R^{y1}$,
$-CS-R^{y1}$,
$-SO-R^{y1}$,
$-SO_2-R^{y1}$, or
$-NR^{y1}R^{y2}$

(where $R^{y1}$ and $R^{y2}$ each independently represent a hydrogen atom or 1 or 2 substituents selected from the following substituent group A),
(f) oxo group, and
(g) optionally substituted non-aromatic heterocyclic groups;

Ar represents
a substituted phenyl group, or
optionally substituted 5- or 6-membered aromatic heterocyclic group (when the phenyl group or aromatic heterocyclic group has 2 or more substituents, two adjacent substituents together may form an optionally substituted 5- to 8-membered ring).
Provided that, when L is a bond, Ar is a substituted phenyl group or substituted 5- or 6-membered aromatic heterocyclic group (when the phenyl group or aromatic heterocyclic group has 2 or more substituents, two adjacent substituents together may form an optionally substituted 5- to 8-membered ring). Substituent group A consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,

(xii) $C_{3-8}$ cycloalkenyl groups,
(xiii) cross-linked $C_{7-10}$ cycloalkyl groups optionally substituted with $C_{1-6}$ alkyl group(s),,
(xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfinyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.

Substituent group B consists of the groups of substituent group A except for $C_{1-6}$ alkoxy groups optionally substituted with halogen atom(s) , and 6- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.
(Provided that

(1) the compounds in which $R^1$ is -CO-NH$R^t$ (wherein $R^t$ is an optionally substituted $C_4$ or higher hydrocarbon group.);
(2) compounds represented by the formula

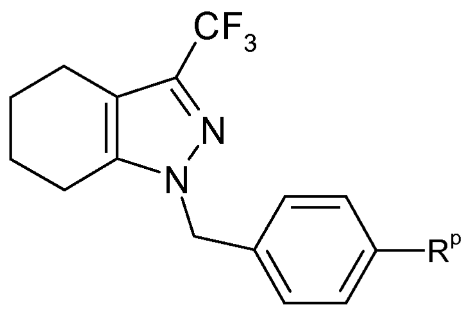

[wherein $R^p$ represents a substituent.];
(3) compounds represented by the formula

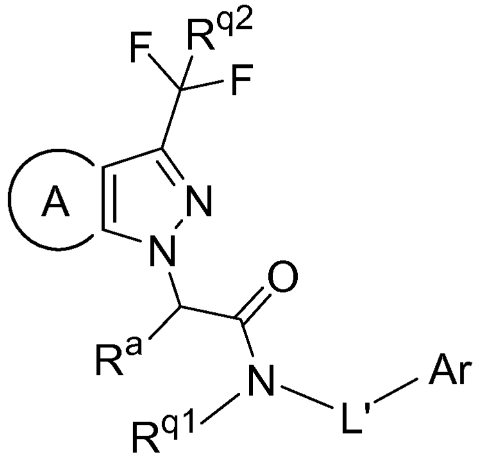

[wherein
$R^{q2}$ represents a hydrogen atom or fluorine atom,
$R^{q1}$ represents a hydrogen atom or substituent,
L' represents a bond, or a spacer in which the number of atoms in the main chain is 1 to 6,
Ring A represents an optionally substituted non-aromatic carbon ring of 4-8 carbon atoms, and the other symbols are synonymous with the above.];
(4) compounds represented by the formula

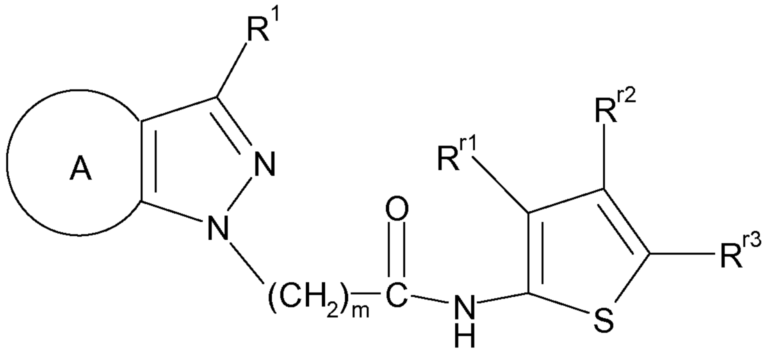

[wherein
$R^1$ represents trifluoromethyl,
$R^{r1}$ represents hydroxymethyl, carboxyl, or optionally substituted carbamoyl,
$R^{r2}$ and $R^{r3}$ may each independently represent hydrogen, $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,
Ring A represents cyclohexene, and
m represents the integer 2.]; and
(5) compounds represented by the formulas

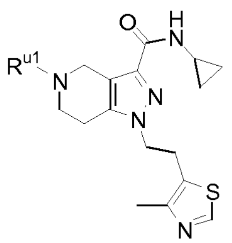

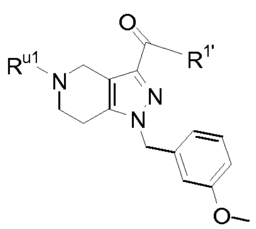

,

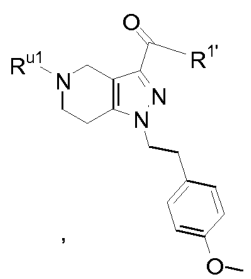

,

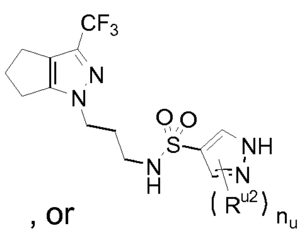

, or

[wherein
$R^{1'}$ represents a dimethylamino group, monoethylamino group, or monocyclopropylamino group,
$R^{u1}$ represents -CO-$R^{u1'}$ ($R^{u1'}$ represents a substituent.), optionally substituted $C_{1-4}$ alkyl group, cycloalkyl group, or optionally substituted 6-membered non-aromatic heterocycle,
$R^{u2}$ represents an optionally halogenated $C_{1-2}$ alkyl group, and $n_u$ represents an integer of 1 to 3.]; and
(6)
3-(3,6-dimethyl-4-oxo-4,5,6,7-tetrahydro-1H-indazol-1-yl)propyl 4-methylbenzenesulfonate;
3-(5-bromo-3,6-dimethyl-4,7-dioxo-4,7-dihydro-1H-indazol-1-yl)propyl 4-methylbenzenesulfonate;
3-(5-amino-3,6-dimethyl-4,7-dioxo-4,7-dihydro-1H-indazol-1-yl)propyl 4-methylbenzenesulfonate;
2-bromo-4-[(3-methyl-4-oxo-4,5,6,7-tetrahydro-1H-indazol-1-yl)methyl]benzonitrile;
[1-(4-fluorobenzyl)-1,4,5,6,7,8-hexahydrocyclohepta[c]pyrazol-3-yl]methanol;
[1-(4-methoxybenzyl)-1,4,5,6,7,8-hexahydrocyclohepta[c]pyrazol-3-yl]methanol;
methyl 4-ethyl-5-methyl-2-({2-[3-(trifluoromethyl)-5,6-dihydrocyclopenta[c]pyrazol-1(4H)-yl]butanoyl}amino)thiophene-3-carboxylate;
methyl 5-ethyl-2-({2-[3-(trifluoromethyl)-5,6-dihydrocyclopenta[c]pyrazol-1(4H)-yl]butanoyl}amino)thiophene-3-carboxylate; and
ethyl 4-methyl-2-({2-[3-(trifluoromethyl)-5,6-dihydrocyclopenta[c]pyrazol-1(4H)-yl]butanoyl}amino)-1,3-thiazole-5-carboxylate are excluded.)

or a salt thereof (herein also referred to as compound (I-1)).

[2]

The compound according to [1], wherein $R^1$ is an optionally halogenated $C_{1-6}$ alkyl.

[3]

The compound according to [1], wherein Ra and Rb are hydrogen atoms.

[4]

The compound according to [1], wherein L is a bond, -CONH-, $-CH_2CH_2CONH-$, $-CH_2CH(CH_3)CONH-$, $-CH_2CH_2CH_2CONH-$, $-CH_2CONH-$, $-CH_2NHCO-$, $-CH_2-$, or $-CH_2O-$.

[5]

The compound according to [1], wherein $R^1$ is an optionally halogenated $C_{1-6}$ alkyl,
Ra and Rb are hydrogen atoms, and
L is a bond, -CONH-, $-CH_2CH_2CONH-$, $-CH_2CH(CH_3)CONH-$, $-CH_2CH_2CH_2CONH-$, $-CH_2CONH-$, $- CH_2NHCO-$, $-CH_2-$, or $-CH_2O-$.

[6]

A compound selected from
1-{[4-(pyrrolidin-1-ylcarbonyl)-1,3-thiazol-2-yl]methyl}-3-(trifluoromethyl)-4,5,6,7-tetrahydro-1H-indazole,
2-({[3-(trifluoromethyl)-4,5,6,7-tetrahydro-1H-pyrazolo[3,4-c]pyridin-1-yl]acetyl}amino)-4,5,6,7-tetrahydro-1-benzothiophene-3-carboxamide,
2-({[3-(trifluoromethyl)-4,5,6,7-tetrahydro-1H-pyrazolo[4,3-c]pyridin-1-yl]acetyl}amino)-4,5,6,7-tetrahydro-1-benzothiophene-3-carboxamide,
1,2-dimethyl-6-(methylsulfanyl)-N-{2-[3-(trifluoromethyl)-4,5,6,7-tetrahydro-1H-indazol-1-yl]ethyl}-1H-thieno[3,4-d]imidazole-4-carboxamide,
5-methyl-7-(trifluoromethyl)-3-(5-{[3-(trifluoromethyl)-4,5,6,7-tetrahydro-1H-indazol-1-yl]methyl}-1,2,4-oxadiazol-3-yl)pyrazolo[1,5-a]pyrimidine,
N-(5-chloro-2-methoxyphenyl)-2-[3-(trifluoromethyl)-4,5,6,7-tetrahydro-1H-pyrazolo[3,4-c]pyridin-1-yl]acetamide,
4-hydroxy-3-(methylsulfanyl)-N-{2-[3-(trifluoromethyl)-4,5,6,7-tetrahydro-1H-indazol-1-yl]ethyl}-4,5,6,7-tetrahydro-2-benzothiophene-1-carboxamide,
2-[5-acetyl-3-(trifluoromethyl)-4,5,6,7-tetrahydro-1H-pyrazolo[4,3-c]pyridin-1-yl]-N-(5-chloro-2-methoxyphenyl)acetamide,

5,7-dimethyl-3-(5-{[3-(trifluoromethyl)-4,5,6,7-tetrahydro-1H-indazol-1-yl]methyl}-1,2,4-oxadiazol-3-yl)pyrazolo[1,5-a]pyrimidine,
1-[(3-pyrazin-2-yl-1,2,4-oxadiazol-5-yl)methyl]-3-(trifluoromethyl)-4,5,6,7-tetrahydro-1H-indazole,
5-methyl-7-(trifluoromethyl)-3-(5-{[3-(trifluoromethyl)-5,6-dihydrocyclopenta[c]pyrazol-1(4H)-yl]methyl}-1,2,4-oxadiazol-3-yl)pyrazolo[1,5-a]pyrimidine,
1-{2-[(5-chloro-2-methoxyphenyl)amino]-2-oxoethyl}-N-methyl-3-(trifluoromethyl)-1,4,6,7-tetrahydro-5H-pyrazolo[4,3-c]pyridine-5-carboxamide,
1-[(3-thiophen-2-yl-1,2,4-oxadiazol-5-yl)methyl]-3-(trifluoromethyl)-1,4,6,7-tetrahydropyrano[4,3-c]pyrazole, and
1-[(5-thiophen-2-yl-1,3,4-oxadiazol-2-yl)methyl]-3-(trifluoromethyl)-4,5,6,7-tetrahydro-1H-indazole, or a salt thereof.

[7]

A prodrug of the compound according to [1].

[8]

A pharmaceutical comprising the compound according to [1] or a prodrug thereof.

[9]

The pharmaceutical according to [8], which is an AMPA receptor potentiator.

[10]

The AMPA receptor potentiator according to [9], which is a drug for preventing or treating depression, schizophrenia, or attention-deficit hyperactivity disorder (ADHD).

[11]

An AMPA receptor potentiator comprising a compound represented by the formula

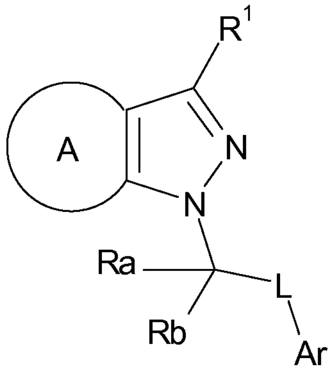 (I)

wherein
$R^1$ represents

(1) a halogen atom,
(2) cyano group,
(3) alkyl group optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,

alkylsulfanyl groups,
alkylsulfinyl groups,
alkylsulfonyl groups,
mono- or di-alkyl-sulfamoyl groups,
alkanoyloxy groups,
alkanoyl groups,
carbamoyl group, and
mono- or di-alkylcarbamoyl groups,
(4) optionally substituted cycloalkyl group,
(5) group represented by the formula:

$-OR^{x1}$,
$-SR^{x2}$,
$-CO-R^{x2}$,
$-CS-R^{x2}$,
$-SO-R^{x2}$,
$-SO_2-R^{x2}$,
$-H_2$, or
$-NR^{x1}R^{x2}$

(where $R^{x1}$ represents a substituent selected from the following substituent group A, and $R^{x2}$ represents hydrogen or a substituent selected from the following substituent group A),
or

(6) optionally substituted non-aromatic heterocyclic group;
Ra and Rb each independently represent a hydrogen atom or $C_{1-4}$ alkyl group;
L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1), either of which is optionally substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
allcylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) optionally substituted cycloalkyl groups,
(e) groups represented by the formula $-OR^{y1}$,
$-SR^{y1}$,
$-CO-R^{y1}$,

-CS-$R^{y1}$,
-SO-$R^{y1}$,
-$SO_2$-$R^{y1}$, or
-$NR^{y1}R^{y2}$
(where $R^{y1}$ and $R^{y2}$ each independently represent hydrogen or 1 or 2 substituents selected from the following substituent group A),
(f) oxo group, and
(g) optionally substituted non-aromatic heterocyclic groups;

Ar represents
an optionally substituted aryl group, or optionally substituted 5- or 6-membered aromatic heterocyclic group (when the aryl group or aromatic heterocyclic group has 2 or more substituents, two adjacent substituents together may form an optionally substituted 5- to 8-membered ring).
Provided that, when L is a bond, Ar is not an unsubstituted phenyl group or unsubstituted 5- or 6-membered aromatic heterocyclic group.
Substituent group A consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,
(xii) $C_{3-8}$ cycloalkenyl groups,
(xiii) cross-linked $C_{7-10}$ cycloalkyl groups optionally substituted with $C_{1-6}$ alkyl group(s),,
(xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfinyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.

(Provided that compounds represented by

(1) the formula

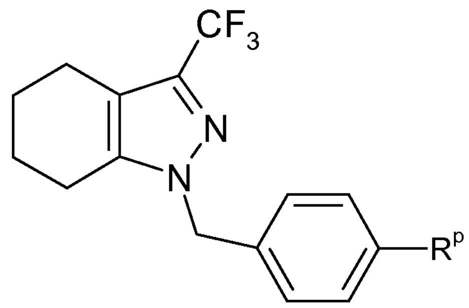

[wherein $R^p$ represents a substituent.];

(2) compounds represented by the formula

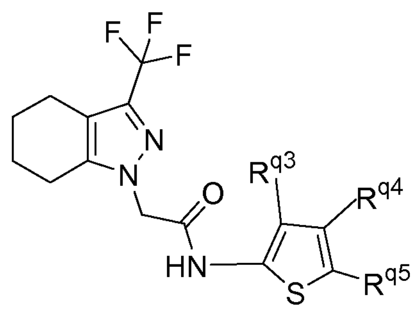

[wherein

$R^{q3}$ represents a hydrogen atom or substituent, and

$R^{q4}$ and $R^{q5}$, which may be the same or different, represent $C_{1-6}$ alkyl groups or are bonded together to form a 6-membered non-aromatic ring.];

(3) compounds represented by the formula

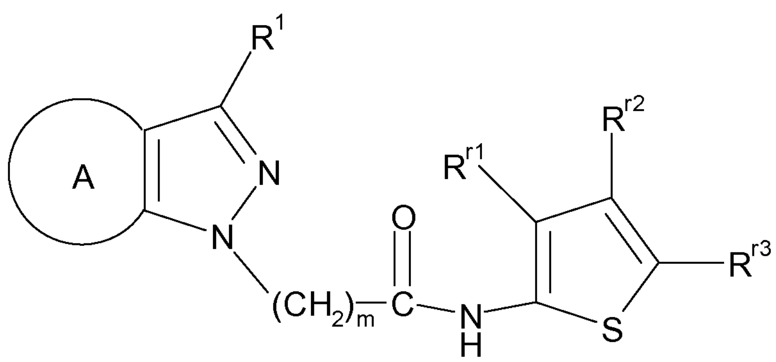

[wherein

$R^1$ represents trifluoromethyl,

$R^{r1}$ represents hydroxymethyl, carboxyl, or optionally substituted carbamoyl,

$R^{r2}$ and $R^{r3}$ may each independently represent hydrogen, a $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,

Ring A represents cyclohexene, and

m represents the integer 2.] are excluded.)

or a salt thereof.

[12]

The AMPA receptor potentiator according to [1], which is a drug for preventing or treating depression, schizophrenia, or attention-deficit hyperactivity disorder (ADHD).

[13]

A method for preventing or treating diseases involving the AMPA receptor in mammals, comprising administering to such mammals a compound represented by the formula

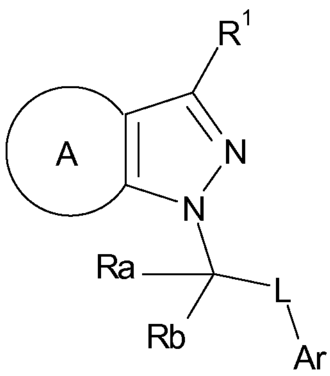
(I)

[wherein
$R^1$ represents

(1) a halogen atom,
(2) cyano group,
(3) alkyl group optionally substituted with substituent(s) selected from halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carbamoyl group, and
mono- or di-alkylcarbamoyl groups,
(4) optionally substituted cycloalkyl group,
(5) group represented by the formula:

$-OR^{x1}$,
$-SR^{x2}$,
$-CO-R^{x2}$,
$-CS-R^{x2}$,
$-SO-R^{x2}$,
$-SO_2-R^{x2}$,
$-NH_2$, or
$-NR^{x1}Rx^2$

(where $R^{x1}$ represents a substituent selected from the following substituent group A, and $R^{x2}$ represents hydrogen

or a substituent selected from the following substituent group A), or
(6) optionally substituted non-aromatic heterocyclic group;
Ra and Rb each independently represent a hydrogen atom or $C_{1-4}$ alkyl group;
L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1), either of which is optionally substituted with one or more substituents selected from

(a) halogen atoms,
(b) cyano group,
(c) alkyl groups optionally substituted with substituent(s) selected from halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) optionally substituted cycloalkyl groups,
(e) groups represented by the formula $-OR^{y1}$,
$-SR^{y1}$,
$-CO-R^{y1}$,
$-CS-R^{y1}$,
$-SO-R^{y1}$,
$-SO_2-R^{y1}$, or
$-NR^{y1}R^{y2}$
(where $R^{y1}$ and $R^{y2}$ each independently represent hydrogen or 1 or 2 substituents selected from the following substituent group A),
(f) oxo group, and
(g) optionally substituted non-aromatic heterocyclic groups;

Ar represents
an optionally substituted aryl group, or optionally substituted 5- or 6-membered aromatic heterocyclic group (when the aryl group or aromatic heterocyclic group has 2 or more substituents, two adjacent substituents together may form an optionally substituted 5- to 8-membered ring).
Provided that, when L is a bond, Ar is not an unsubstituted phenyl group or unsubstituted 5- or 6-membered aromatic heterocyclic group.
Substituent group A consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,

(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,
(xii) $C_{3-8}$ cycloalkenyl groups,
(xiii) cross-linked $C_{7-10}$ cycloalkyl groups optionally substituted with $C_{1-6}$ alkyl group(s),
(xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfinyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.

(Provided that

(1) compounds represented by the formula

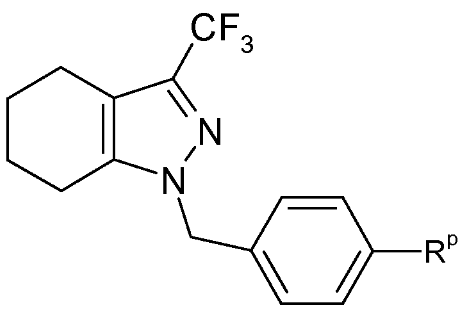

[wherein $R^p$ represents a substituent.];
(2) compounds represented by the formula

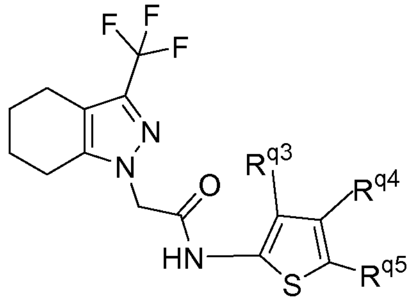

[wherein
$R^{q3}$ represents a hydrogen atom or substituent, and
$R^{q4}$ and $R^{q5}$, which may be the same or different, represent $C_{1-6}$ alkyl groups or are bonded together to form a 6-membered non-aromatic ring.];
(3) compounds represented by the formula

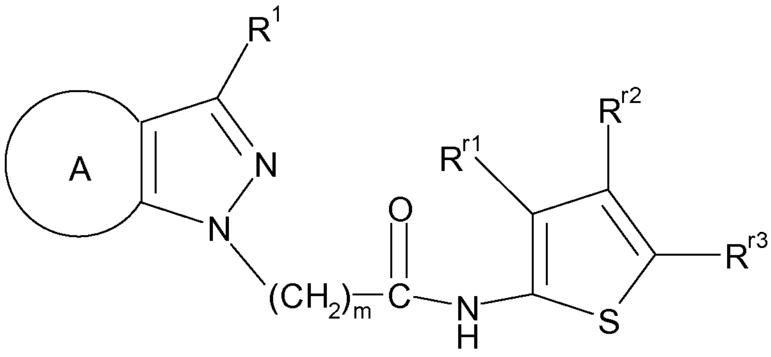

[wherein
$R^1$ represents trifluoromethyl,
$R^{r1}$ represents hydroxymethyl, carboxyl, or optionally substituted carbamoyl,
$R^{r2}$ and $R^{r3}$ may each independently represent hydrogen, a $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,
Ring A represents cyclohexene, and
m represents the integer 2.] are excluded.)
or a salt thereof or prodrug thereof.

[14]

The method according to [13], wherein the disease involving the AMPA receptor is depression, schizophrenia, or attention-deficit hyperactivity disorder (ADHD).

[15]

The use of a compound for the production of an AMPA receptor potentiator, represented by the formula

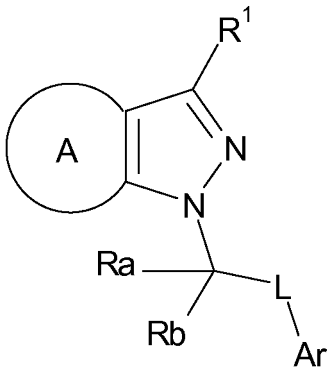
(I)

[wherein $R^1$ represents

(1) a halogen atom,
(2) cyano group,
(3) alkyl group optionally substituted with substituent(s) selected from halogen atoms,

cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carbamoyl group, and
mono- or di-alkylcarbamoyl groups,
(4) optionally substituted cycloalkyl group,
(5) group represented by the formula:

$-OR^{x1}$,
$-SR^{x2}$,
$-CO-R^{x2}$,
$-CS-R^{x2}$,
$-SO-R^{x2}$,
$-SO_2-R^{x2}$,
$-HH_2$, or
$-NR^{x1}R^{x2}$

(where $R^{x1}$ represents a substituent selected from the following substituent group A, and $R^{x2}$ represents hydrogen or a substituent selected from the following substituent group A),
or
(6) optionally substituted non-aromatic heterocyclic group;
Ra and Rb each independently represent a hydrogen atom or $C_{1-4}$ alkyl group;
L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1), either of which is optionally substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and

mono- or di-alkylcarbamoyl groups,
(d) optionally substituted cycloalkyl groups,
(e) groups represented by the formula $-OR^{y1}$,
$-SR^{y1}$,
$-CO-R^{y1}$,
$-CS-R^{y1}$,
$-SO-R^{y1}$,
$-SO_2-R^{y1}$, or
$-NR^{y1}R^{y2}$
(where $R^{y1}$ and $R^{y2}$ each independently represent hydrogen or 1 or 2 substituents selected from the following substituent group A),
(f) oxo group, and
(g) optionally substituted non-aromatic heterocyclic groups;

Ar represents
an optionally substituted aryl group, or optionally substituted 5- or 6-membered aromatic heterocyclic group (when the aryl group or aromatic heterocyclic group has 2 or more substituents, two adjacent substituents together may form an optionally substituted 5- to 8-membered ring).
Provided that, when L is a bond, Ar is not an unsubstituted phenyl group or unsubstituted 5- or 6-membered aromatic heterocyclic group.
Substituent group A consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,
(xii) $C_{3-8}$ cycloalkenyl groups,
(xiii) cross-linked $C_{7-10}$ cycloalkyl groups optionally substituted with $C_{1-6}$ alkyl group(s), (xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfinyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.

(Provided that

(1) compounds represented by the formula

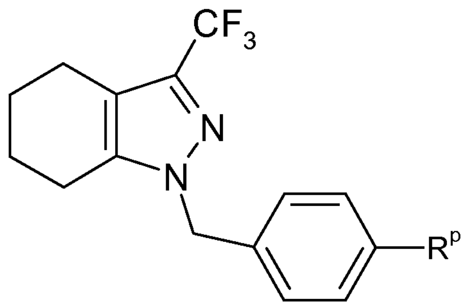

[wherein $R^p$ represents a substituent.];

(2) compounds represented by the formula

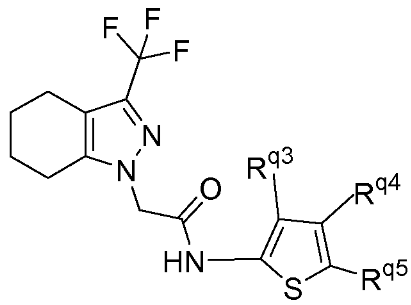

[wherein

$R^{q3}$ represents a hydrogen atom or substituent, and

$R^{q4}$ and $R^{q5}$, which may be the same or different, represent $C_{1-6}$ alkyl groups or are bonded together to form a 6-membered non-aromatic ring.];

(3) compounds represented by the formula

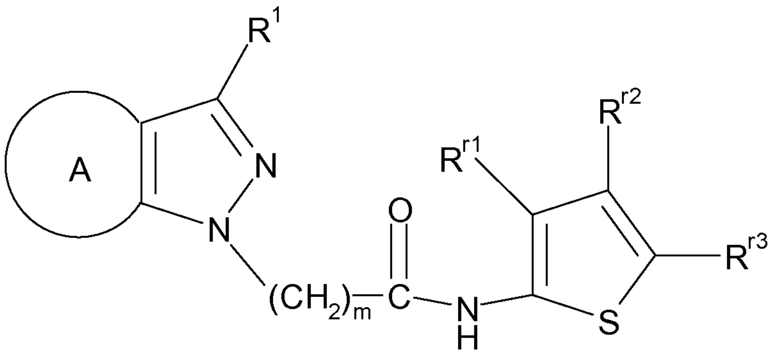

[wherein

$R^1$ represents trifluoromethyl,

$R^{r1}$ represents hydroxymethyl, carboxyl, or optionally substituted carbamoyl,

$R^{r2}$ and $R^{r3}$ may each independently represent hydrogen, a $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,

Ring A represents cyclohexene, and

m represents the integer 2.] are excluded.)

or a salt thereof or a prodrug thereof.

[16]

The use according to [15], wherein the AMPA receptor potentiator is a drug for preventing or treating depression, schizophrenia, or attention-deficit hyperactivity disorder (ADHD).

The present invention is also intended to provide the compounds in [1'] to [3'] below, pharmaceuticals in [4'] and [5'], AMPA receptor potentiators in [6'] to [8'], and the like.

[1']

A compound represented by the formula

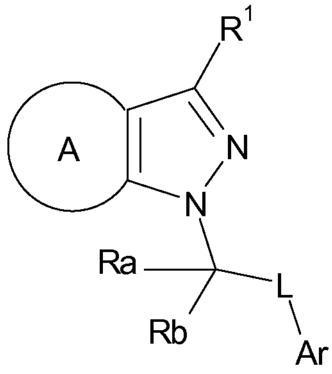

(I)

wherein

$R^1$ represents

(1) a halogen atom,
(2) cyano group,
(3) alkyl group optionally substituted with substituent(s) selected from halogens, cyano, hydroxy, alkoxy groups, cycloalkyl, optionally substituted amino, mercapto (thiol), alkylsulfinyl (alkylsulfanyl), alkylsulphenyl (alkylsulfanyl), alkylsulfonyl, mono- or di-alkyl-sulfamoyl, alkanoyloxy, alkanoyl, carbamoyl, and mono- or di-alkylcarbamoyl,
(4) optionally substituted cycloalkyl group,
(5) group represented by $-OR^{x1}$, $-SR^{x2}$, $-CO-R^{x2}$, $-CS-R^{x2}$, $-SO-R^{x2}$, $-SO_2-R^{x2}$, $-NH_2$, or $-NR^{x1}R^{x2}$ (where $R^{x1}$ represents a substituent, and $R^{x2}$ represents hydrogen or a substituent), or
(6) optionally substituted non-aromatic heterocyclic group;
Ra and Rb each independently represent a hydrogen atom or $C_{1-4}$ alkyl group;
L represents a bond, or a spacer in which the number of atoms in the main chain is 1 to 8;
Ring A represents

(i) a non-aromatic carbon ring of 4-8 carbon atoms, or (ii) 4- to 8-membered non-aromatic heterocycle which may have no nitrogen atoms or 1 nitrogen atom and may also have hetero atoms selected from oxygen and sulfur,

either of which is optionally substituted with one or more substituents selected from

(1) halogen atoms,
(2) cyano group,
(3) alkyl groups optionally substituted with substituent(s) selected from halogens, cyano, hydroxy, alkoxy groups, cycloalkyl, optionally substituted amino, mercapto, alkylsulfinyl, alkylsulphenyl, alkylsulfonyl, mono- or di-alkylsulfamoyl, alkanoyloxy groups, alkanoyl, carbamoyl, and mono- or di-alkylcarbamoyl,
(4) optionally substituted cycloalkyl groups,
(5) optionally substituted amino group,
(6) groups represented by the formula $-OR^{y1}$, $-SR^{y1}$, $-CO-R^{y1}$, $-CS-R^{y1}$, $-SO-R^{y1}$, $-SO_2-R^{y1}$, or $-NR^{y1}R^{y2}$ (where $R^{y1}$ and $R^{y2}$ each independently represent hydrogen or a substituent),

(7) oxo, and
(8) optionally substituted non-aromatic heterocyclic groups; and
Ar represents an optionally substituted aryl group or optionally substituted aromatic heterocyclic group (when the aryl group or aromatic heterocyclic group has 2 or more substituents, two adjacent substituents together may form a 5- to 6-membered ring)

(Provided that

(1) the compounds in which $R^1$ is -CO-NHR$^t$ (wherein $R^t$ is an optionally substituted $C_4$ or higher hydrocarbon group.);
(2) compounds represented by the formula

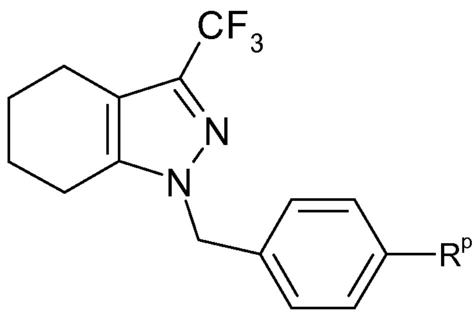

[wherein $R^p$ represents a substituent.];
(3) compounds represented by the formula

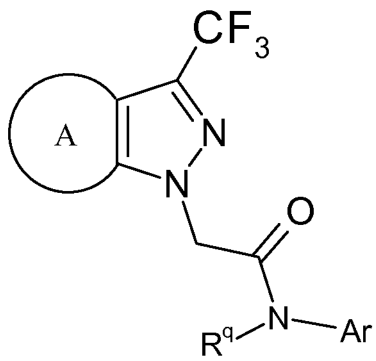

[wherein

Ring A represents a non-aromatic carbon ring of 4-8 carbon atoms optionally substituted with 1 or more substituents selected from

(1) halogen atoms,
(2) cyano group,
(3) alkyl groups optionally substituted with substituent(s) selected from halogens, cyano, hydroxy, alkoxy groups, cycloalkyl, optionally substituted amino, mercapto, alkylsulfinyl, alkylsulphenyl, alkylsulfonyl, mono- or di-alkyl-sulfamoyl, alkanoyloxy groups, alkanoyl, carbamoyl, and mono- or di-alkylcarbamoyl,
(4) optionally substituted cycloalkyl groups,
(5) optionally substituted amino group,
(6) groups represented by the formula -OR$^{y1}$, -SR$^{y1}$, -CO-R$^{y1}$, -CS-R$^{y1}$, -SO-R$^{y1}$, -$SO_2$-R$^{y1}$, or -NR$^{y1}$R$^{y2}$ (where R$^{y1}$ and R$^{y2}$ each independently represent hydrogen or a substituent),
(7) oxo, and
(8) optionally substituted non-aromatic heterocyclic groups,
$R^q$ represents hydrogen or a substituent, and
the other symbols are synonymous with the above.];

(4) compounds represented by the formula

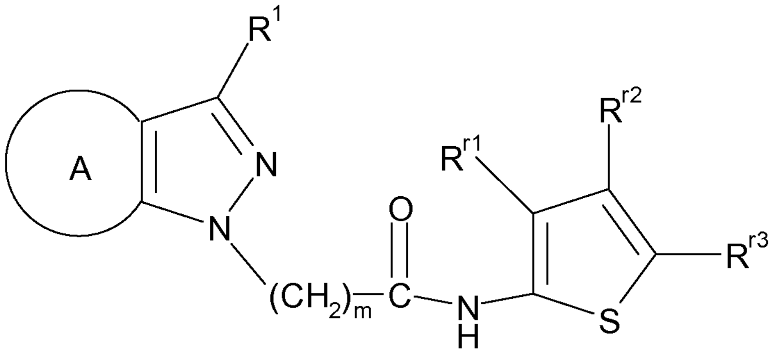

[wherein
$R^1$ represents trifluoromethyl,
$R^{r1}$ represents hydroxymethyl, carboxyl, or optionally substituted carbamoyl,
$R^{r2}$ and $R^{r3}$ may each independently represent hydrogen, $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,
Ring A represents cyclohexene, and
m represents the integer 2.]; and
(5)
1-(2-aminobenzyl)-6-chloro-3,5-dimethyl-1,5-dihydro-4H-pyrazolo[4,3-c]pyridin-4-one;
1-(3,4-dimethoxybenzyl)-3,4-dimethylpyrano[2,3-c]pyrazol-6(1H)-one;
3,4-dimethyl-1-(4-nitrobenzyl)pyrano[2,3-c]pyrazol-6(1H)-one;
3,4-dimethyl-1-(3-nitrobenzyl)pyrano[2,3-c]pyrazol-6(1H)-one;
3,4-dimethyl-1-(2-nitrobenzyl)pyrano[2,3-c]pyrazol-6(1H)-one;
1-(4-methoxybenzyl)-3,4-dimethylpyrano[2,3-c]pyrazol-6(1H)-one;
1-(3-methoxybenzyl)-3,4-dimethylpyrano[2,3-c]pyrazol-6(H)-one;
1-(2-methoxybenzyl)-3,4-dimethylpyrano[2,3-c]pyrazol-6(H)-one;
1-(4-chlorobenzyl)-3,4-dimethylpyrano[2,3-c]pyrazol-6(1H)-one;
1-(3-chlorobenzyl)-3,4-dimethylpyrano[2,3-c]pyrazol-6(1H)-one;
1-(2-chlorobenzyl)-3,4-dimethylpyrano[2,3-c]pyrazol-6(1H)-one;
3-(3,6-dimethyl-4-oxo-4,5,6,7-tetrahydro-1H-indazol-1-yl)propyl 4-methylbenzenesulfonate;
3-(5-bromo-3,6-dimethyl-4,7-dioxo-4,7-dihydro-1H-indazol-1-yl)propyl 4-methylbenzenesulfonate;
3-(5-amino-3,6-dimethyl-4,7-dioxo-4,7-dihydro-1H-indazol-1-yl)propyl 4-methylbenzenesulfonate;
2-bromo-4-[(3-methyl-4-oxo-4,5,6,7-tetrahydro-1H-indazol-1-yl)methyl]benzonitrile;
ethyl 7-oxo-1-[(5-phenyl-1,3-oxazol-2-yl)methyl]-4,5,6,7-tetrahydro-1H-indazole-3-carboxylate;
ethyl 1-[(5-ethyl-1,3-oxazol-2-yl)methyl]-7-oxo-4,5,6,7-tetrahydro-1H-indazole-3-carboxylate;
[1-(4-fluorobenzyl)-1,4,5,6,7,8-hexahydrocyclopenta[c]pyrazol-3-yl]methanol;
[1-(4-methoxybenzyl)-1,4,5,6,7,8-hexahydrocyclopenta[c]pyrazol-3-yl]methanol;
{[1-(4-chlorobenzyl)-4,5,6,7-tetrahydro-1H-indazol-3-yl]oxy}acetonitrile;
1-(4-chlorobenzyl)-3-(1H-tetrazol-5-ylmethoxy)-4,5,6,7-tetrahydro-1H-indazole;
N-[3-(2-furanyl)-1-methylpropyl]-N-methyl-[3-(trifluoromethyl)-5,6-dihydrocyclopenta[c]pyrazol-(4H)-yl]acetamide;
N-[3-(2-furanyl)-1-methylpropyl]-[3-(trifluoromethyl)-5,6-dihydrocyclopenta[c]pyrazol-1 (4H)-yl]acetamide;
N-(2-thienylmethyl)-[3-(trifluoromethyl)-4,5,6,7-tetrahydro-1H-indazol-1-yl]acetamide;
N-[2-(5-methoxy-2-methyl-1H-indol-3-yl)ethyl]-[3-(trifluoromethyl)-4,5,6,7-tetrahydro-1H-indazol-1-yl]acetamide;
N-(2-phenylethyl)-[3-(trifluoromethyl)-5,6-dihydrocyclopenta[c]pyrazol-1(4H)-yl]acetamide;
N-(1-phenylethyl)-[3-(trifluoromethyl)-5,6-dihydrocyclopenta[c]pyrazol-1(4H)-yl]acetamide;
N-[(4-chlorophenyl)methyl]-[3-(trifluoromethyl)-5,6-dihydrocyclopenta[c]pyrazol-1(4H)-yl]acetamide;
N-methyl-N-(phenylmethyl)-[3-(trifluoromethyl)-4,5,6,7-tetrahydro-1H-indazol-1-yl]acetamide;
N-(2-thienylmethyl)-[3-(trifluoromethyl)-5,6-dihydrocyclopenta[c]pyrazol-1(4H)-yl]acetamide;
N-(2-furanylmethyl)-[3-(trifluoromethyl)-4,5,6,7-tetrahydro-1H-indazol-1-yl]acetamide;
N-[3-[3-(trifluoromethyl)-5,6-dihydrocyclopenta[c]pyrazol-2(4H)-yl]propyl]-[3-(trifluoromethyl)-5,6,7,8-tetrahydrocyclohepta[c]pyrazol-1(4H)-yl]acetamide;
N-phenyl-N-(phenylmethyl)-[3-(trifluoromethyl)-4,5,6,7-tetrahydro-1H-indazol-1-yl]acetamide;

N-methyl-N-phenyl-[3-(trifluoromethyl)-4,5,6,7-tetrahydro-1H-indazol-1-yl]acetamide;
N-methyl-N-(phenylmethyl)-[3-(trifluoromethyl)-5,6-dihydrocyclopenta[c]pyrazol-1(4H)-yl]acetamide;
N-(2-furanylmethyl)-[3-(trifluoromethyl)-5,6-dihydrocyclopentapyrazol-1(4H)-yl]acetamide;
N-(phenylmethyl)-[3-(trifluoromethyl)-4,5,6,7-tetrahydro-1H-indazol-1-yl]acetamide;
N,N-bis(phenylmethyl)-[3-(trifluoromethyl)-4,5,6,7-tetrahydro-1H-indazol-1-yl]acetamide; methyl 4-ethyl-5-methyl-2-({2-[3-(trifluoromethyl)-5,6-dihydrocyclopenta[c]pyrazol-1(4H)-yl]butanoyl}amino)thiophene-3-carboxylate;
N-(1-phenylethyl)-[3-(trifluoromethyl)-4,5,6,7-tetrahydro-1H-indazol-1-yl]acetamide;
N-(3-pyridinylmethyl)-[3-(trifluoromethyl)-4,5,6,7-tetrahydro-1H-indazol-1-yl]acetamide;
methyl 5-ethyl-2-({2-[3-(trifluoromethyl)-5,6-dihydrocyclopenta[c]pyrazol-1(4H)-yl]butanoyl}amino)thiophene-3-carboxylate;
ethyl 4-methyl-2-({2-[3-(trifluoromethyl)-5,6-dihydrocyclopenta[c]pyrazol-1(4H)-yl]butanoyl}amino)-1,3-thiazole-5-carboxylate;
N-[(4-methoxyphenyl)methyl]-[3-(trifluoromethyl)-5,6-dihydrocyclopenta[c]pyrazol-1(4H)-yl]acetamide;
N-[(4-fluorophenyl)methyl]-[3-(trifluoromethyl)-5,6-dihydrocyclopenta[c]pyrazol-1(4H)-yl]acetamide;
N-[(4-chlorophenyl)methyl]-[3-(trifluoromethyl)-4,5,6,7-tetrahydro-1H-indazol-1-yl]acetamide;
N-[(4-fluorophenyl)methyl]-[3-(trifluoromethyl)-4,5,6,7-tetrahydro-1H-indazol-1-yl]acetamide;
N-phenyl-N-(phenylmethyl)-[3-(trifluoromethyl)-5,6-dihydrocyclopenta[c]pyrazol-1(4H)-yl]acetamide;
N-(phenylmethyl)-[3-(trifluoromethyl)-5,6-dihydrocyclopenta[c]pyrazol-1(4H)-yl]acetamide;
N-[(4-methoxyphenyl)methyl]-[3-(trifluoromethyl)-4,5,6,7-tetrahydro-1H-indazol-1-yl]acetamide; and
N-(2-phenylethyl)-[3-(trifluoromethyl)-4,5,6,7-tetrahydro-1H-indazol-1-yl]acetamide

are excluded.)
or a salt thereof;

[2']

the compound according to [1'] above, wherein
$R^1$ is an optionally halogenated $C_{1-6}$ alkyl;
Ra and Rb are each a hydrogen atom;
L is a bond, or

(1) $-(CH_2)_s-$ (wherein s is an integer of 1 to 6),
(2) $-(CH_2)_{n1}-CONH-(CH_2)_{n2}-$ (wherein n1 is an integer of 0 to 3, and n2 is an integer of 0 to 3), or
(3) $-(CH_2)_t-O-$ (wherein t is an integer of 1 to 6),

any of which is optionally substituted with substituent(s) selected from $C_{1-6}$ alkyl groups, di-$C_{1-6}$ alkylamino-$C_{1-6}$ alkyl groups, and 5- to 6-membered non-aromatic heterocycle-$C_{1-6}$ alkyl groups;
Ring A is cyclohexene; and
Ar is
a phenyl group or 5- or 6-membered aromatic heterocyclic group (when the 5- or 6-membered aromatic heterocyclic group has 2 or more substituents, two adjacent substituents together may form a 6-membered ring),
either of which is optionally substituted with substituent(s) selected from

(a) halogen atoms
(b) $C_{1-6}$ alkoxy groups
(c) $C_{1-6}$ alkyl groups
(d) carbamoyl group optionally substituted with substituent(s) selected from

(i) $C_{1-6}$ alkyl groups optionally substituted with substituent(s) selected from

(1) hydroxy group,
(2) $C_{1-6}$ alkoxy groups,
(3) dimethylamino group,
(4) carbamoyl group,
(5) 5- or 6-membered heterocyclic groups, and
(6) phenyl group optionally substituted with substituent(s) selected from halogen atoms and sulfamoyl groups,

(ii) phenyl group optionally substituted with substituent(s) selected from halogen atoms, $C_{1-6}$ alkyl groups, halogenated $C_{1-6}$ alkyl groups, $C_{1-6}$ alkoxy groups, $C_{1-6}$ alkyl-sulfamoyl groups, and carbamoyl group,
(iii) tricyclic bridged cyclic groups, and
(iv) 5- to 10-membered heterocyclic groups optionally substituted with substituent(s) selected from $C_{1-6}$ alkyl groups, $C_{1-6}$ alkoxy groups, $C_{1-6}$ alkoxy-carbonyl groups, carbamoyl group, and oxo group,

(e) carboxy group,
(f) $C_{1-6}$ alkoxy-carbonyl groups,
(g) cyclic amino-carbonyl group optionally substituted with substituent(s) selected from 5- or 6-membered heterocyclic groups and mono- or di-$C_{1-6}$ alkyl-sulfamoyl groups, and
(h) sulfamoyl groups;

[3']

a prodrug of the compound according to [1'];

[4']

a pharmaceutical comprising the compound according to [1'] or a prodrug thereof;

[5']

the pharmaceutical according to [4'], which is an AMPA receptor potentiator;

[6']

the AMPA receptor potentiator according to [5'], which is a drug for preventing or treating depression, schizophrenia, or attention-deficit hyperactivity disorder (ADHD);

[7']

An AMPA receptor potentiator, comprising a compound represented by the formula

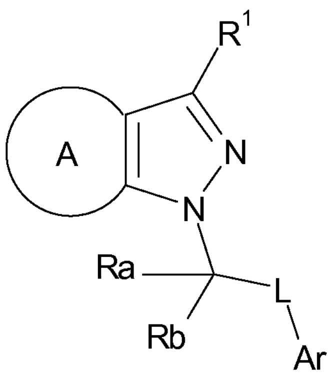
(I)

wherein
$R^1$ represents

(1) a halogen atom,
(2) cyano group,
(3) alkyl group optionally substituted with substituent(s) selected from halogens, cyano, hydroxy, alkoxy groups, cycloalkyl, optionally substituted amino, mercapto, alkylsulfinyl, alkylsulphenyl, alkylsulfonyl, mono- or di-alkyl-sulfamoyl, alkanoyloxy groups, alkanoyl, carbamoyl, and mono- or di-alkylcarbamoyl,
(4) optionally substituted cycloalkyl group,
(5) group represented by $-OR^{x1}$, $-SR^{x2}$, $-CO-R^{x2}$, $-CS-R^{x2}$, $-SO-R^{x2}$, $-SO_2-R^{x2}$, $-NH_2$, or $-NR^{x1}R^{x2}$ (where $R^{x1}$ represents a substituent, and $R^{x2}$ represents hydrogen or a substituent), or

(6) optionally substituted non-aromatic heterocyclic group;

Ra and Rb each independently represent a hydrogen atom or $C_{1-4}$ alkyl group;
L represents a bond, or a spacer in which the number of atoms in the main chain is 1 to 8;
Ring A represents

(i) a non-aromatic carbon ring of 4-8 carbon atoms, or (ii) 4- to 8-membered non-aromatic heterocycle which may have no nitrogen atoms or 1 nitrogen atom and may also have hetero atoms selected from oxygen and sulfur, either of which is optionally substituted with one or more substituents selected from

(1) halogen atoms,
(2) cyano group,
(3) alkyl groups optionally substituted with substituent(s) selected from halogens, cyano, hydroxy, alkoxy groups, cycloalkyl, optionally substituted amino, mercapto, alkylsulfinyl, alkylsulphenyl, alkylsulfonyl, mono- or di-alkylsulfamoyl, alkanoyloxy groups, alkanoyl, carbamoyl, and mono- or di-alkylcarbamoyl,
(4) optionally substituted cycloalkyl groups,
(5) optionally substituted amino group,
(6) groups represented by the formula $-OR^{y1}$, $-SR^{y1}$, $-CO-R^{y1}$, $-CS-R^{y1}$, $-SO-R^{y1}$, $-SO_2-R^{y1}$, or $-NR^{y1}R^{y2}$ (where $R^{y1}$ and $R^{y2}$ each independently represent hydrogen or a substituent),
(7) oxo, and
(8) optionally substituted non-aromatic heterocyclic groups; and

Ar represents an optionally substituted aryl group or optionally substituted aromatic heterocyclic group (when the aryl group or aromatic heterocyclic group has 2 or more substituents, two adjacent substituents may together form a 5- or 6-membered ring)
(Provided that

(1) compounds represented by

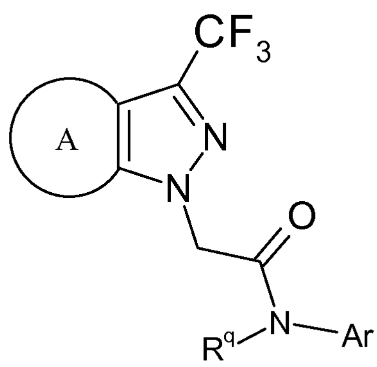

[wherein
Ring A represents a non-aromatic carbon ring of 4-8 carbon atoms optionally substituted with 1 or more substituents selected from

(1) halogen atoms,
(2) cyano group,
(3) alkyl groups optionally substituted with substituent(s) selected from halogens, cyano, hydroxy, alkoxy groups, cycloalkyl, optionally substituted amino, mercapto, alkylsulfinyl, alkylsulphenyl, alkylsulfonyl, mono- or di-alkylsulfamoyl, alkanoyloxy groups, alkanoyl, carbamoyl, and mono- or di-alkylcarbamoyl,
(4) optionally substituted cycloalkyl groups,
(5) optionally substituted amino group,
(6) groups represented by the formula $-OR^{y1}$, $-SR^{y1}$, $-CO-R^{y1}$, $-CS-R^{y1}$, $-SO-R^{y1}$, $-SO_2-R^{y1}$, or $-NR^{y1}R^{y2}$ (where $R^{y1}$ and $R^{y2}$ each independently represent hydrogen or a substituent),
(7) oxo, and
(8) optionally substituted non-aromatic heterocyclic groups,

$R^q$ represents hydrogen or a substituent, and the other symbols are synonymous with the above.]; and
(2) compounds represented by the formula

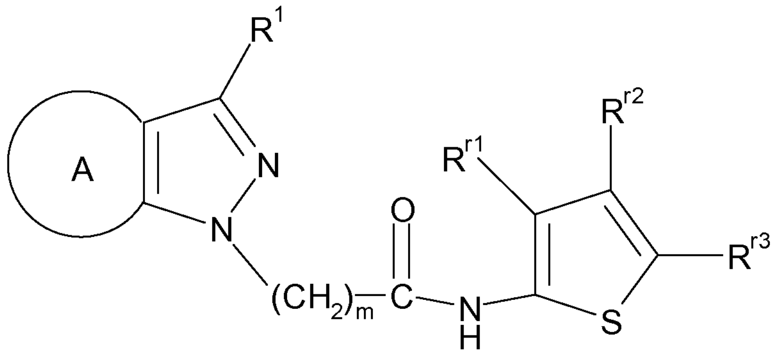

[wherein
$R^1$ represents trifluoromethyl,
$R^{r1}$ represents hydroxymethyl, carboxyl, or optionally substituted carbamoyl,
$R^{r2}$ and $R^{r3}$ may each independently represent hydrogen, $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,
Ring A represents cyclohexene, and
m represents the integer 2.] are excluded.)
or a salt thereof (herein also referred to as compound (I'). As is evident from the definition, compound (I') also overlaps with compound (I). The same symbols are also shared in common in the formulas.);

[8']

the AMPA receptor potentiator according to [7'], which is a drug for preventing or treating depression, schizophrenia, or attention-deficit hyperactivity disorder (ADHD);
and the like are provided.

[Advantageous Effects of the Invention]

The present invention provides a compound which potentiates the AMPA receptor and is useful as a drug for preventing or treating depression, schizophrenia, attention-deficit hyperactivity disorder (ADHD), or the like.

[Description of Embodiments]

Unless otherwise noted, "aromatic rings" herein will be interpreted in accordance with Huckel's rule, which means a ring wherein the number of electrons related to the aromaticity in the ring is 4n+2 (n is a natural number). On the other hand, a "non-aromatic ring" means a ring that is not an aromatic ring.
Aryl groups herein mean aromatic hydrocarbon groups.
Arrows in the structural formulas herein indicate bonding with another atom. Examples of "halogen atoms" herein include fluorine, chlorine, bromine, and iodine atoms.
Examples of "optionally substituted hydrocarbon groups" herein include optionally substituted alkyl groups, optionally substituted alkenyl groups, optionally substituted alkynyl groups, optionally substituted aralkyl groups, optionally substituted aryl groups, optionally substituted cycloalkyl groups, and optionally substituted cycloalkenyl groups.
Examples of "optionally substituted alkyl groups" herein include $C_{1-6}$ alkyl groups (such as methyl, ethyl, propyl, isopropyl, butyl, isobutyl, sec-butyl, tert-butyl, pentyl, neopentyl, and hexyl) optionally substituted with 1 or more (preferably 1 to 4, and more preferably 1 to 3) substituents selected from

(i) halogen atoms,
(ii) cyano group,
(iii) hydroxy group,
(iv) nitro group,
(v) formyl group,

(vi) amino group,
(vii) mono- or di-$C_{1-6}$ alkylamino groups (such as methylamino, ethylamino, propylamino, dimethylamino, diethylamino, and dipropylamino),
(viii) $C_{1-6}$ alkyl-carbonylamino groups (such as acetylamino and ethylcarbonylamino),
(ix) $C_{1-6}$ alkoxy-carbonylamino groups (such as methoxycarbonylamino, ethoxycarbonylamino, and propoxycarbonylamino),
(x) $C_{3-8}$ cycloalkyl groups (such as cyclopropyl, cyclobutyl, cyclopentyl, and cyclohexyl) optionally condensed with a benzene ring,
(xi) $C_{3-8}$ cycloalkenyl groups (such as cyclopropenyl, cyclobutenyl, cyclopentenyl, and cyclohexenyl) optionally condensed with a benzene ring,
(xii) $C_{6-14}$ aryl groups (such as phenyl, 1-naphthyl, and 2-naphthyl) optionally substituted with substituent(s) selected from halogen atoms (such as fluorine, chlorine, bromine, and iodine atoms) and $C_{1-6}$ alkoxy groups (such as methoxy, ethoxy, and propoxy),
(xiii) $C_{1-6}$ alkoxy groups (such as methoxy, ethoxy, propoxy, isopropoxy, butoxy, isobutoxy, sec-butoxy, and tert-butoxy) optionally substituted with halogen atom(s) (such as fluorine, chlorine, bromine, and iodine atoms),
(xiv) $C_{7-16}$ aralkyloxy groups (such as benzyloxy),
(xv) $C_{6-14}$ aryloxy groups (such as phenoxy) optionally substituted with substituent(s) selected from $C_{1-6}$ alkoxy groups (such as methoxy), $C_{1-6}$ alkyl groups (such as methyl), and halogen atoms (such as fluorine, chlorine, bromine, and iodine atoms),
(xvi) carboxyl group,
(xvii) $C_{1-6}$ alkoxy-carbonyl groups (such as methoxycarbonyl, ethoxycarbonyl, propoxycarbonyl, isopropoxycarbonyl, and tert-butoxycarbonyl),
(xviii) $C_{7-16}$ aralkyloxy-carbonyl groups (such as benzyloxycarbonyl),
(xix) $C_{6-14}$ aryloxy-carbonyl groups (such as phenoxycarbonyl),
(xx) $C_{1-6}$ alkyl-carbonyl groups (such as acetyl, ethylcarbonyl, propylcarbonyl, isopropylcarbonyl, and 2,2-dimethylpropylcarbonyl),
(xxi) $C_{3-8}$ cycloalkyl-carbonyl groups (such as cyclopropylcarbonyl, cyclobutylcarbonyl, cyclopentylcarbonyl, and cyclohexylcarbonyl),
(xxii) $C_{7-16}$ aralkyl-carbonyl groups (such as benzylcarbonyl),
(xxiii) carbamoyl group,
(xxiv) thiocarbamoyl group,
(xxv) mono- or di-$C_{1-6}$ alkyl-carbamoyl groups (such as methylcarbamoyl, ethylcarbamoyl, propylcarbamoyl, isopropylcarbamoyl, dimethylcarbamoyl, diethylcarbamoyl, and dipropylcarbamoyl),
(xxvi) mono- or di-$C_{7-16}$ aralkyl-carbamoyl groups (such as benzylcarbamoyl and dibenzylcarbamoyl),
(xxvii) thiol group,
(xxviii) $C_{1-6}$ alkylthio groups (such as methylthio, ethylthio, and propylthio),
(xxix) $C_{7-16}$ aralkylthio groups (such as benzylthio),
(xxx) $C_{1-6}$ alkylsulfinyl groups (such as methylsulfinyl, ethylsulfinyl, propylsulfinyl, and butylsulfinyl), (xxxi) $C_{6-10}$ arylsulfinyl groups (such as phenylsulfinyl and naphthylsulfinyl),
(xxxii) $C_{1-6}$ alkylsulfonyl groups (such as methylsulfonyl, ethylsulfonyl, propylsulfonyl, and isopropylsulfonyl),
(xxxiii) $C_{3-8}$ cycloalkylsulfonyl groups (such as cyclopropylsulfonyl, cyclobutylsulfonyl, and cyclopentylsulfonyl),
(xxxiv) $C_{6-14}$ arylsulfonyl groups (such as phenylsulfonyl, 1-naphthylsulfonyl, and 2-naphthylsulfonyl), (xxxv) $C_{7-16}$ aralkylsulfonyl groups (such as benzylsulfonyl),
(xxxvi) 5- to 8-membered non-aromatic heterocyclic groups having 1 to 4 hetero atoms in addition to carbon atoms, selected from nitrogen, sulfur, and oxygen atoms (such as pyrrolidinyl, tetrahydrofuryl, tetrahydrothienyl, piperidinyl, tetrahydropyranyl, morpholinyl, thiomorpholinyl, and piperazinyl) [the non-aromatic heterocyclic group is optionally substituted with $C_1$_6 alkyl groups (such as methyl)],
(xxxvii) 5- to 8-membered aromatic heterocyclic groups having 1 to 4 hetero atoms in addition to carbon atoms, selected from nitrogen, sulfur, and oxygen atoms (such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl), the aromatic heterocyclic group may be substituted with a halogen atom or $C_{1-6}$ alkyl group (such as methyl) and may be condensed with a benzene ring,
(xxxviii) 5- to 8-membered non-aromatic heterocycle-carbonyl groups having 1 to 4 hetero atoms in addition to carbon atoms, selected from nitrogen, sulfur, and oxygen atoms (such as pyrrolidinylcarbonyl, tetrahydrofurylcarbonyl, tetrahydrothienylcarbonyl, piperidylcarbonyl, tetrahydropyranylcarbonyl, morpholinylcarbonyl, thiomorpholinylcarbonyl, and piperazinylcarbonyl),
(xxxix) 5- to 8-membered aromatic heterocycle-carbonyl groups having 1 to 4 hetero atoms in addition to carbon

atoms, selected from nitrogen, sulfur, and oxygen atoms (such as furylcarbonyl, thienylcarbonyl, pyrrolylcarbonyl, oxazolylcarbonyl, isoxazolycarbonyl, thiazolylcarbonyl, isothiazolylcarbonyl, imidazolylcarbonyl, pyrazolylcarbonyl, 1,2,3-oxadiazolylcarbonyl, 1,2,4-oxadiazolylcarbonyl, 1,3,4-oxadiazolylcarbonyl, furazanylcarbonyl, 1,2,3-thiadiazolylcarbonyl, 1,2,4-thiadiazolylcarbonyl, 1,3,4-thiadiazolylcarbonyl, 1,2,3-triazolylcarbonyl, 1,2,4-triazolylcarbonyl, tetrazolylcarbonyl, pyridylcarbonyl, pyridazinylcarbonyl, pyrimidinylcarbonyl, pyrazinylcarbonyl, and triazinylcarbonyl),
(xl) ureido group,
(xli) $C_{1-6}$ alkyl-ureido groups (such as methylureido, ethylureido, and propylureido),
(xlii) $C_{6-14}$ aryl-ureido groups (such as phenylureido, 1-naphthylureido, and 2-naphthylureido)
(xliii) $C_{1-4}$ alkylenedioxy groups (such as methylenedioxy, ethylenedioxy, and propylenedioxy),
(xliv) aminosulfonyl group,
(xlv) mono-N-$C_{1-6}$ alkylaminosulfonyl groups (such as methylaminosulfonyl and ethylaminosulfonyl), (xlvi) di-N,N-$C_{1-6}$ alkylaminosulfonyl groups (such as dimethylaminosulfonyl and diethylaminosulfonyl), (xlvii) cross-linked $C_{7-10}$ cycloalkyl groups (such as bicyclo[3.1.1]heptyl and adamantyl) optionally substituted with $C_{1-6}$ alkyl groups (such as methyl), and
(xlviii) $C_{6-14}$ arylthio groups (such as phenylthio).

The above compounds (I) are also referred to herein as compounds of the present invention.
Examples of "optionally substituted alkenyl groups" used herein include $C_{2-6}$ alkenyl groups (such as vinyl, 1-propenyl, allyl, isopropenyl, butenyl, and isobutenyl) optionally substituted with 1 or more (preferably 1 to 4, and more preferably 1 to 3) substituents given above as examples of "substituents" in "optionally substituted alkyl groups."
Examples of "optionally substituted alkynyl groups" used herein include $C_{2-6}$ alkynyl groups (such as ethynyl, propargyl, butynyl, and 1-hexynyl) optionally substituted with 1 or more (preferably 1 to 4, and more preferably 1 to 3) groups given above as examples of "substituents" in "optionally substituted alkyl groups."
Examples of "optionally substituted aralkyl groups" herein include $C_{7-12}$ aralkyl groups (such as benzyl, 2-phenylethyl, 1-phenylethyl, and 3-phenylpropyl) optionally substituted with 1 or more (preferably 1 to 4, and more preferably 1 to 3) substituents selected from

(i) groups given above as examples of "substituents" in "optionally substituted alkyl groups,"
(ii) $C_{1-6}$ alkyl groups (such as methyl, ethyl, propyl, isopropyl, butyl, isobutyl, sec-butyl, tert-butyl, pentyl, neopentyl, and hexyl) optionally substituted with substituent(s) selected from halogen atoms (such as fluorine, chlorine, bromine, and iodine atoms), $C_{1-6}$ alkoxy groups (such as methoxy, ethoxy, and propoxy), $C_{6-14}$ arylsulfonyl groups, and heterocyclic groups (such as morpholinyl, pyridyl, imidazopyridyl, and benzoimidazolyl),
(iii) $C_{7-16}$ aralkyl groups (such as benzyl, 2-phenylethyl, 1-phenylethyl, 3-phenylpropyl, and 4-phenylbutyl), and
(iv) 5- to 8-membered aromatic heterocycle-oxy groups having 1 to 4 hetero atoms in addition to carbon atoms, selected from nitrogen, sulfur, and oxygen atoms (such as furyloxy, thienyloxy, pyrrolyloxy, oxazolyloxy, isoxazolyloxy, thiazolyloxy, isothiazolyloxy, imidazolyloxy, pyrazolyloxy, 1,2,3-oxadiazolyloxy, 1,2,4-oxadiazolyloxy, 1,3,4-oxadiazolyloxy, furazanyloxy, 1,2,3-thiadiazolyloxy, 1,2,4-thiadiazolyloxy, 1,3,4-thiadiazolyloxy, 1,2,3-triazolyloxy, 1,2,4-triazolyloxy, tetrazolyloxy, pyridyloxy, pyridazinyloxy, pyrimidinyloxy, pyrazinyloxy, and triazinyloxy),

and the like.
The substituents of the "optionally substituted aralkyl groups" herein may be present in the aryl moiety and/or alkylene moiety of the aralkyl group.
Examples of "optionally substituted aryl groups" used herein include $C_{6-14}$ aryl groups (such as phenyl and naphthyl) optionally substituted with 1 or more (preferably 1 to 4, and more preferably 1 to 3) groups given above as examples of "substituents" in "optionally substituted aralkyl groups."
Examples of "optionally substituted cycloalkyl groups" used herein include $C_{3-8}$ cycloalkyl groups (such as cyclopropyl, cyclobutyl, cyclopentyl, and cyclohexyl) optionally substituted with 1 or more (preferably 1 to 4, and more preferably 1 to 3) groups given above as examples of "substituents" in "optionally substituted aralkyl groups." The substituents of "optionally substituted cycloalkyl groups" may also be bonded to each other to form rings (such as cycloalkane rings {i.e. $C_{3-6}$ cycloalkane rings such as cyclopropane ring, cyclobutane ring, cyclopentane ring, or cyclohexane ring} and arene rings {i.e. $C_{6-10}$ arene rings such as benzene ring or naphthalene ring}).
Examples of "optionally substituted cycloalkenyl groups" used herein include $C_{3-8}$ cycloalkenyl groups (such as cyclopropenyl, cyclobutenyl, cyclopentenyl, and cyclohexenyl) optionally substituted with 1 or more (preferably 1 to 4, and more preferably 1 to 3) groups given above as examples of "substituents" in "optionally substituted aralkyl groups." The substituents of "optionally substituted cycloalkenyl groups" may also be bonded to each other to form rings (such as cycloalkane rings {i.e. $C_{3-6}$ cycloalkane rings such as cyclopropane ring, cyclobutane ring, cyclopentane ring, or cyclohexane ring} and arene rings {i.e. $C_{6-10}$ arene rings such as benzene ring or naphthalene ring}).

Examples of "acyl groups" herein include "optionally substituted alkylcarbonyl groups," "optionally substituted alkenylcarbonyl groups," "optionally substituted alkynylcarbonyl groups," "optionally substituted aralkylcarbonyl groups," "optionally substituted arylcarbonyl groups," "optionally substituted cycloalkylcarbonyl groups," "optionally substituted alkoxycarbonyl groups," "optionally substituted alkenyloxycarbonyl groups," "optionally substituted alkynyloxycarbonyl groups, "optionally substituted aralkyloxycarbonyl groups," "optionally substituted aryloxycarbonyl groups," "optionally substituted cycloalkyloxycarbonyl groups," and "carboxyl group."

Examples of "optionally substituted alkylcarbonyl groups" used herein include $C_{1-6}$ alkyl-carbonyl groups (such as methylcarbonyl, ethylcarbonyl, propylcarbonyl, isopropylcarbonyl, butylcarbonyl, isobutylcarbonyl, sec-butylcarbonyl, tert-butylcarbonyl, pentylcarbonyl, and hexylcarbonyl) optionally substituted with 1 or more (preferably 1 to 4, and more preferably 1 to 3) groups given above as examples of "substituents" in "optionally substituted alkyl groups."

Examples of "optionally substituted alkenylcarbonyl groups" used herein include $C_{2-6}$ alkenyl-carbonyl groups (such as vinylcarbonyl, 1-propenylcarbonyl, allylcarbonyl, isopropenylcarbonyl, butenylcarbonyl, and isobutenylcarbonyl) optionally substituted with 1 or more (preferably 1 to 4, and more preferably 1 to 3) substituents given above as examples of "substituents" in "optionally substituted alkyl groups."

Examples of "optionally substituted alkynylcarbonyl groups" used herein include $C_{2-6}$ alkynyl-carbonyl groups (such as ethynylcarbonyl, propargylcarbonyl, butynylcarbonyl, and 1-hexynylcarbonyl) optionally substituted with 1 or more (preferably 1 to 4, and more preferably 1 to 3) substituents given above as examples of "substituents" in "optionally substituted alkyl groups."

Examples of "optionally substituted aralkylcarbonyl groups" used herein include $C_{7-12}$ aralkyl-carbonyl groups (such as benzylcarbonyl, 2-phenylethylcarbonyl, 1-phenylethylcarbonyl, and 3-phenylpropylcarbonyl) optionally substituted with 1 or more (preferably 1 to 4, and more preferably 1 to 3) substituents given above as examples of "substituents" in "optionally substituted aralkyl groups."

Examples of "optionally substituted arylcarbonyl groups" used herein include $C_{6-14}$ arylcarbonyl groups (such as phenylcarbonyl and naphthylcarbonyl) optionally substituted with 1 or more (preferably 1 to 4, and more preferably 1 to 3) groups given above as examples of "substituents" in "optionally substituted aralkyl groups."

Examples of "optionally substituted cycloalkylcarbonyl groups" used herein include $C_{3-8}$ cycloalkylcarbonyl groups (such as cyclopropylcarbonyl, cyclobutylcarbonyl, cyclopentylcarbonyl, and cyclohexylcarbonyl) optionally substituted with 1 or more (preferably 1 to 4, and more preferably 1 to 3) groups given above as examples of "substituents" in "optionally substituted aralkyl groups."

Examples of "optionally substituted alkoxycarbonyl groups" used herein include $C_{1-6}$ alkoxy-carbonyl groups (such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentoxycarbonyl, and hexyloxycarbonyl) optionally substituted with 1 or more (preferably 1 to 4, and more preferably 1 to 3) groups given above as examples of "substituents" in "optionally substituted alkyl groups."

Examples of "optionally substituted alkenyloxycarbonyl groups" used herein include $C_{2-6}$ alkenyloxycarbonyl groups (such as vinyloxycarbonyl, 1-propenyloxycarbonyl, allyloxycarbonyl, isopropenyloxycarbonyl, butenyloxycarbonyl, and isobutenyloxycarbonyl) optionally substituted with 1 or more (preferably 1 to 4, and more preferably 1 to 3) substituents given above as examples of "substituents" in "optionally substituted alkyl groups."

Examples of "optionally substituted alkynyloxycarbonyl groups" used herein include $C_{2-6}$ alkynyloxycarbonyl groups (such as ethynyloxycarbonyl, propalgyloxycarbonyl, butynyloxycarbonyl, and 1-hexynyloxycarbonyl) optionally substituted with 1 or more (preferably 1 to 4, and more preferably 1 to 3) substituents given above as examples of "substituents" in "optionally substituted alkyl groups."

Examples of "optionally substituted aralkyloxycarbonyl groups" used herein include $C_{7-12}$ aralkyloxycarbonyl groups (such as benzyloxycarbonyl, 2-phenylethyloxycarbonyl, 1-phenylethyloxycarbonyl, and 3-phenylpropyloxycarbonyl) optionally substituted with 1 or more (preferably 1 to 4, and more preferably 1 to 3) substituents given above as examples of "substituents" in "optionally substituted aralkyl groups."

Examples of "optionally substituted aryloxycarbonyl groups" used herein include $C_{6-14}$ aryl-oxycarbonyl groups (such as phenyloxycarbonyl and naphthyloxycarbonyl) optionally substituted with 1 or more (preferably 1 to 4, and more preferably 1 to 3) groups given above as examples of "substituents" in "optionally substituted aralkyl groups."

Examples of "optionally substituted cycloalkyloxycarbonyl groups" used herein include $C_{3-8}$ cycloalkyloxycarbonyl (such as cyclopropyloxycarbonyl, cyclobutyloxycarbonyl, cyclopentyloxycarbonyl, and cyclohexyloxycarbonyl) groups optionally substituted with 1 or more (preferably 1 to 4, and more preferably 1 to 3) groups given above as examples of "substituents" in "optionally substituted aralkyl groups."

Examples of "optionally substituted heterocyclic groups" used herein include "optionally substituted non-aromatic heterocyclic groups" and "optionally substituted aromatic heterocyclic groups."

Examples of "optionally substituted non-aromatic heterocyclic groups" used herein include 5- to 8-membered non-aromatic heterocyclic groups having 1 to 4 hetero atoms in addition to carbon atoms, selected from nitrogen, sulfur, and oxygen atoms (such as pyrrolidinyl, tetrahydrofuryl, tetrahydrothienyl, piperidyl, tetrahydropyranyl, morpholinyl, thiomorpholinyl, piperazinyl, azepanyl, and 1,4-diazepanyl), which may have 1 to 3 groups given above as examples of "substituents" in "optionally substituted aralkyl groups," and which may be condensed with a benzene ring.

Examples of "non-aromatic heterocyclic groups" in "optionally substituted non-aromatic heterocyclic groups" also include (1) 4- to 8-membered non-aromatic heterocyclic groups having 1 to 4 hetero atoms in addition to carbon atoms, selected from nitrogen, sulfur, and oxygen atoms (such as pyrrolidinyl, tetrahydrofuryl, tetrahydrothienyl, piperidyl, tetrahydropyranyl, morpholinyl, thiomorpholinyl, and piperazinyl) and (2) groups resulting from the condensation of such 4- to 8- non-aromatic heterocyclic groups with a benzene ring. Examples of "substituents" in such "optionally substituted non-aromatic heterocyclic groups" include the same groups as the "substituents" in the above "optionally substituted aralkyl groups."

Examples of "optionally substituted aromatic heterocyclic groups" used herein include 5- to 8-membered aromatic heterocyclic groups having 1 to 4 hetero atoms in addition to carbon atoms, selected from nitrogen, sulfur, and oxygen atoms (such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl), which may have 1 to 3 groups given above as examples of "substituents" in "optionally substituted aralkyl groups," and which may be condensed with a benzene ring. Examples of "optionally substituted 5- or 6-membered aromatic heterocyclic groups" used herein include "optionally substituted aromatic heterocyclic groups" in which the aromatic heterocyclic group moieties are 5- or 6-membered. When the aromatic heterocyclic groups have 2 or more substituents, two adjacent substituents may together form an optionally substituted 5- to 8-membered ring.

Examples of "optionally substituted 5- to 8-membered rings" include unsaturated carbon rings having 5-8 carbon atoms (preferably 5 or 6 carbon atoms) such as cyclopentadiene, cyclopentene, cyclohexene, cyclohexadiene, and benzene; as well as 5- to 8-membered (preferably 5- or 6-membered) unsaturated heterocycles having 1 or 2 hetero atoms in addition to carbon atoms, selected from nitrogen, sulfur, and oxygen atoms, such as dihydropyrrole, pyrrole, dihydrofuran, furan, dihydrothiophene, thiophene, dihydroisoxazole, isoxazole, dihydrooxazole, oxazole, dihydroisothiazole, isothiazole, dihydrothiazole, thiazole, dihydropyran, pyran, dihydrothiopyran, thiopyran, dihydroimidazole, imidazole, tetrahydropyridine, dihydropyridine, pyridine, pyrimidine, dihydropyrimidine, and tetrahydropyrimidine.

Examples of "alkyl groups" used herein include $C_{1-6}$ alkyl groups (such as methyl, ethyl, propyl, isopropyl, butyl, isobutyl, sec-butyl, tert-butyl, pentyl, and hexyl).

Examples of "$C_{1-4}$ alkyl groups" used herein include those with 1 to 4 carbon atoms, that is, methyl, ethyl, propyl, isopropyl, butyl, isobutyl, sec-butyl, and tert-butyl.

Examples of "alkoxy groups" used herein include $C_{1-6}$ alkoxy groups (such as methoxy, ethoxy, propoxy, isopropoxy, butoxy, isobutoxy, sec-butoxy, and tert-butoxy).

Examples of "cycloalkyl groups" used herein include $C_{3-8}$ cycloalkyl groups (such as cyclopropyl, cyclobutyl, cyclopentyl, and cyclohexyl).

Examples of "alkylsulfanyl groups" used herein include groups represented by R-S- (R is an alkyl group.). Examples of "alkylsulfinyl groups" used herein include groups represented by R-SO- (R is an alkyl group.). Examples of "alkylsulfonyl groups" used herein include groups represented by R-$SO_2$- (R is an alkyl group.).

Examples of "mono- or di-alkyl-sulfamoyl groups" used herein include groups represented by NHR-SO- or $NR^2$-SO- (R, which may be the same or different in each instance, is an alkyl group.).

Examples of "alkanoyloxy groups" used herein include groups represented by R-CO-O- (R is an alkyl group.).

Examples of "alkanoyl groups" used herein include groups represented by R-CO- (R is an alkyl group.). Examples of "alkoxycarbonyl groups" used herein include groups represented by R-O-CO- (R is an alkyl group.).

Examples of "mono- or di-alkyl-carbamoyl groups" used herein include groups represented by NHR-CO- or $NR^2$-CO- (R, which may be the same or different in each instance, is an alkyl group.).

Substituent group A herein consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloallcyl groups,
(xii) $C_{3-8}$ cycloalkenyl groups,

(xiii) cross-linked $C_{7-10}$ cycloalkyl groups optionally substituted with $C_{1-6}$ allcyl group(s)"
(xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfmyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.

Substituent group B herein consists of the groups of substituent group A except for $C_{1-6}$ alkoxy groups optionally substituted with halogen atom(s), and 6- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.
Compounds (I) potentiate the AMPA receptor.
Among compounds (I), compounds (I-1) are novel compounds.
The symbols in formula (I) are described below.
$R^1$ represents

(1) a halogen atom,
(2) cyano group,
(3) alkyl group optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carbamoyl group, and
mono- or di-alkylcarbamoyl groups,
(4) optionally substituted cycloalkyl group,
(5) group represented by the formula:
$-OR^{x1}$,
$-SR^{x2}$,
$-CO-R^{x2}$,

-CS-$R^{x2}$,
-SO-$R^{x2}$,
-$SO_2$-$R^{x2}$,
-$NH_2$, or
-$NR^{x1}R^{x2}$
(where $R^{x1}$ represents a substituent selected from the following substituent group A, and $R^{x2}$ represents hydrogen or a substituent selected from the following substituent group A),
or
(6) optionally substituted non-aromatic heterocyclic group.

$R^1$ is preferably an alkyl group optionally substituted with 1 or more (preferably 1 to 3) substituents selected from halogen atoms and hydroxyl group, or -CO-$R^{x2}$ (In the formula, $R^{x2}$ is a $C_{1-6}$ alkoxy group optionally substituted with halogen atom(s).).

$R^1$ is more preferably an optionally halogenated $C_{1-6}$ alkyl group, and even more preferably trifluoromethyl.

Ra and Rb each independently represent a hydrogen atom or $C_{1-4}$ alkyl. Ra and Rb are preferably hydrogen atoms.

L is a bond, or a spacer in which the number of atoms in the main chain is 1 to 8. The "main chain" of the "spacer in which the number of atoms in the main chain is 1 to 8" represented by L is a divalent straight chain linking the Ar ring to a carbon atom in -CHRaRb-, and the "number of atoms in the main chain" is counted so as to result in the minimum atoms of the main chain. The "main chain" consists of 1 to 8 atoms selected from carbon and hetero atoms (such as O (oxygen), S (sulfur), and N (nitrogen)), and may be saturated or unsaturated. the S (Sulfur) may also be in the form of an oxide. The "spacer in which the number of atoms in the main chain is 1 to 8" represented by L may have 1 or more (preferably 1 to 3) substituents (preferably side-chain).
Preferred examples of such substituents include $C_{1-6}$ alkyl groups, di-$C_{1-6}$ alkylamino-$C_{1-6}$ alkyl groups, and 5- or 6-membered non-aromatic heterocycle-$C_{1-6}$ alkyl groups. Examples of "$C_{1-6}$ alkyls (groups)" in such "$C_{1-6}$ alkyl groups," "di-$C_{1-6}$ alkylamino-$C_{1-6}$ alkyl groups," and "5- or 6-membered non-aromatic heterocycle-$C_{1-6}$ alkyl groups" include methyl, ethyl, propyl, isopropyl, butyl, isobutyl, sec-butyl, tert-butyl, pentyl, and hexyl. Examples of "5- or 6-membered non-aromatic heterocycle-" (that is, 5- or 6-membered aromatic heterocyclic groups) in such "5- or 6-membered non-aromatic heterocycle-$C_{1-6}$ alkyl groups" include 5- or 6-membered non-aromatic heterocyclic groups having 1 to 4 hetero atoms in addition to carbon atoms, selected from nitrogen, sulfur, and oxygen atoms (such as pyrrolidinyl, tetrahydrofuryl [such as 2-tetrahydrofuryl], tetrahydrothienyl, piperidinyl, tetrahydropyranyl, morpholinyl, thiomorpholinyl, and piperazinyl). When L has 2 or more substituents, two of them may together form a ring (preferably 3- to 5-membered saturated ring (more preferably, cyclopropane, pyrrolidine, or thiazolidine)).

L is preferably
a bond, or

(1) -$(CH_2)_s$- (wherein s is an integer of 1 to 6) (such as -$CH_2$-),
(2) -$(CH_2)_{n1}$-CONH-$(CH_2)_{n2}$- (wherein n1 is an integer of 0 to 3, and n2 is an integer of 0 to 3.) {such as -$(CH_2)_2$-C(O)-NH-, -$(CH_2)_3$-C(O)-NH-, -$(CH_2)_2$-C(O)-NH-$CH_2$-, or $(CH_2)_3$-C(O)-NH-$CH_2$)},
(3) -$(CH_2)_{n1}$-NHCO-$(CH_2)_{n2}$- (wherein n1 is an integer of 0 to 3, and n2 is an integer of 0 to 3.) {such as -$CH_2$-NH-C(O)-},
(4) -$(CH_2)_t$-O- (wherein t is an integer of 1 to 6) (such as -$(CH_2)_2$-O-), or
(5) -$(CH_2)_{n1}$-CONH-$(CH_2)_{n3}$-S- (wherein n1 is an integer of 0 to 3, and n3 is an integer of 0 to 3, providing that the sum of n1 and n3 is 0 to 5.) {such as -C(O)-NH-$(CH_2)_2$-S-},
any of which is optionally substituted with substituent(s) selected from $C_{1-6}$ alkyl groups (such as methyl), di-$C_{1-6}$ alkylamino-$C_{1-6}$ alkyl groups (such as N,N'-dimethylaminomethyl), and 5- to 6-membered non-aromatic heterocycle-$C_{1-6}$ alkyl groups (such as tetrahydrofurylmethyl), and when there are 2 or more substituents, two of them together may form a ring (preferably 3- to 5-membered saturated ring (more preferably cyclopropane, pyrrolidine, or thiazolidine));

L is more preferably
a bond, or
-$(CH_2)_2$-C(O)-NH-, -$(CH_2)_3$-C(O)-NH-, -$CH_2$-, -$CH(CH_3)$-C(O)-NH-, -$CH_2$-NH-C(O)-, -$CH_2$O-, -C(O)-NH-, -C(O)-NH-$CH_2$-, -C(O)-NH-$(CH_2)_2$-, -C(O)-NH-$CH(CH_3)$-, -$(CH_2)_2$-C(O)-NH-$CH_2$-, -$(CH_2)_2$-C(O)-NH-$CH(CH_3)$-, -$CH_2$-$CH(CH_3)$-C(O)-NH-$CH_2$-, -$CH_2$-$CH(CH_3)$-C(O)-NH-$CH(CH_3)$-, -$(CH_2)_2$-C(O)-NH-$(CH_2)_3$-, -$CH_2$-$CH(CH_3)$-C(O)-NH-$(CH_2)_3$-, -$(CH_2)_3$-C(O)-NH-$(CH_2)_3$-, -$(CH_2)_3$-C(O)-NH-$CH(CH_3)$-, -C(O)-NH-$(CH_2)_2$-S-,

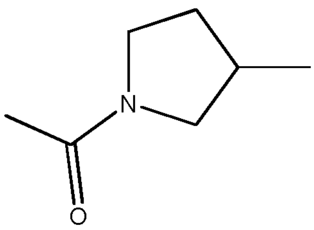

,

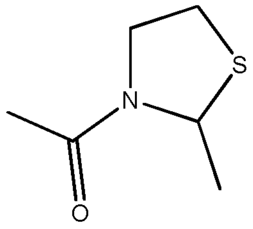

or

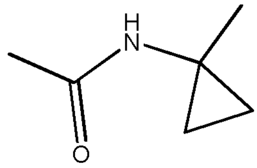

any of which is optionally substituted with substituent(s) selected from 5- to 6-membered non-aromatic heterocycle-$C_{1-6}$ alkyl groups (such as tetrahydrofurylmethyl);

L is more preferably

a bond, or $-(CH_2)_2-C(O)-NH-$, $-(CH_2)_3-C(O)-NH-$, $-CH_2-$, $CH(CH_3)-C(O)-NH-$, $-CH_2-NH-C(O)-$, $-CH_2O-$, C(O)-NH-, -C(O)-NH-$CH_2$-, -C(O)-NH-$(CH_2)_2$-, -C(O)-NH-$CH(CH_3)$-, $-(CH_2)_2-C(O)-NH-CH_2-$, $-(CH_2)_2-C(O)-NH-CH(CH_3)-$, or

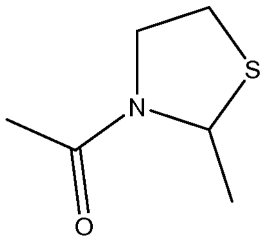

.

Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1),
either of which is optionally substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfmyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) cycloalkyl groups optionally substituted with substituent(s) selected from halogen atoms and $C_{1-6}$ alkyl groups,
(e) groups represented by the formula $-OR^{y1}$,
$-SR^{y1}$,
-CO-$R^{y}$1,
$-CS-R^{y1}$,

-SO-$R^{y1}$,
-SO2-$R^{y1}$, or
-$NR^{y1}R^{y2}$

(where $R^{y1}$ and $R^{y2}$ each independently represent a hydrogen atom or 1 or 2 substituents selected from substituent group A),

(f) oxo group, and
(g) non-aromatic heterocyclic groups optionally substituted with substituent(s) selected from halogen atoms and $C_{1-6}$ alkyl groups.

Examples of such "non-aromatic carbon rings of 4-8 carbon atoms" include $C_{4-8}$ cycloalkanes (such as cyclobutane, cyclopentane, cyclohexane, cycloheptane, and cyclooctane), $C_{4-8}$ cycloalkenes (such as cyclobutene, cyclopentene, cyclohexene, cycloheptene, and cyclooctene), and $C_{4-8}$ cycloalkadienes (such as cyclobutadiene, cyclopentadiene, cyclohexadiene, cycloheptadiene, and cyclooctadiene). Of these, rings having 5-8 carbon atoms are preferred.

Examples of such "4-8 membered non-aromatic heterocycles having 1 to 3 hetero atoms selected from nitrogen, oxygen, and sulfur atoms (except the number of nitrogen atoms is 0 or 1)" include dihydropyrrole, dihydrooxazole, dihydrothiazole, tetrahydropyridine, dihydropyran, dihydrothiopyran, dihydroxazine, oxazine, dihydrothiazine, thiazine, dihydrofuran, dihydrothiophene, dihydroimidazole, tetrahydroazepine, dihydroazepine, hexahydroazocine, and tetrahydroazocine. Of these, 5- to 7-membered rings are preferred.

Ring A is preferably

(1) a non-aromatic carbon ring of 4-8 carbon atoms (preferably cyclopentene or cyclohexene) or
(2) 4- to 8-membered non-aromatic heterocycle having 1 to 3 hetero atoms selected from nitrogen, oxygen, and sulfur atoms (except the number of nitrogen atoms is 0 or 1) (preferably tetrahydropyridine or dihydropyran),
either of which is optionally substituted with 1 or more substituents selected from

a) alkyl groups (preferably methyl, ethyl, or isopropyl) optionally substituted with substituent(s) selected from

(1) hydroxy group,
(2) alkoxy groups,
(3) cycloalkyl groups (preferably cyclopropyl),
(4) amino group optionally substituted with 1 or 2 substituents selected from substituent group A,
(5) mercapto group,
(6) alkylsulfanyl groups,
(7) alkylsulfinyl groups,
(8) alkylsulfonyl groups,
(9) mono- or di-alkyl-sulfamoyl groups,
(10) alkanoyloxy groups,
(11) alkanoyl groups,
(12) carboxyl group,
(13) alkoxycarbonyl groups (preferably ethoxycarbonyl),
(14) carbamoyl group, and
(15) mono- or di-alkylcarbamoyl groups (preferably mono-methylcarbamoyl and di-methylcarbamoyl),

b) cycloalkyl groups optionally substituted with substituent(s) selected from halogen atoms and $C_{1-6}$ alkyl groups,
c) groups represented by the formula -CO-$R^{y1}$ (preferably carbamoyl, methylcarbamoyl, dimethylcarbamoyl, or acetyl) or
-$SO_2$-$R^{y1}$ (preferably methylsulfonyl)
(wherein $R^{y1}$ and $R^{y2}$ are each independently a hydrogen atom, $C_{1-6}$ alkyl group (preferably methyl) optionally substituted with halogen atom(s), $C_{1-6}$ alkoxy group (preferably methoxy, ethoxy, or tert-butoxy) optionally substituted with halogen atom(s), amino group, or mono- or di-$C_{1-6}$ alkylamino group (preferably mono-methylamino or di-methylamino)),
d) oxo group, and
e) non-aromatic heterocyclic groups optionally substituted with substituent(s) selected from halogen atoms and $C_{1-6}$ alkyl groups.

Ar represents an optionally substituted phenyl group, or

optionally substituted 5- or 6-membered aromatic heterocyclic group.
When the phenyl group or 5- or 6-membered aromatic heterocyclic group has 2 or more substituents, two adjacent substituents may together form an optionally substituted 5- to 8-membered ring, and preferably 5-or 6-membered ring. However, when L is a bond, Ar is not an unsubstituted phenyl group or unsubstituted 5-or 6-membered aromatic heterocyclic group.
Examples of substituents in the "optionally substituted phenyl group" and "optionally substituted 5- or 6-membered aromatic heterocyclic group" represented by Ar include

a) halogen atoms,
b) cyano group,
c) nitro group,
d) amino group,
e) mono- or di-$C_{1-6}$ alkylamino groups (such as methylamino, ethylamino, propylamino, dimethylamino, diethylamino, and dipropylamino),
f) $C_{1-6}$ alkyl-carbonylamino groups (such as acetylamino and ethylcarbonylamino),
g) $C_{1-6}$ alkoxy-carbonylamino groups (such as methoxycarbonylamino, ethoxycarbonylamino, and propoxycarbonylamino),
h) $C_{3-8}$ cycloalkyl groups optionally condensed with a benzene ring (such as cyclopropyl, cyclobutyl, cyclopentyl, and cyclohexyl),
i) $C_{3-8}$ cycloalkenyl groups optionally condensed with a benzene ring (such as cyclopropenyl, cyclobutenyl, cyclopentenyl, and cyclohexenyl),
j) $C_{6-14}$ aryl groups (such as phenyl, 1-naphthyl, and 2-naphthyl) optionally substituted with substituent(s) selected from halogen atoms (such as fluorine, chlorine, bromine, and iodine atoms), $C_{1-6}$ alkoxy groups (such as methoxy, ethoxy, and propoxy), mono- or di-$C_{1-6}$ alkylamino groups (such as dimethylamino), and $C_{6-14}$ aryl groups (such as phenyl),
k) cross-linked $C_{7-10}$ cycloalkyl groups (such as bicyclo[3.1.1]heptyl and adamantyl) optionally substituted with $C_{1-6}$ alkyl group(s) (such as methyl),
l) hydroxy group,
m) $C_{1-6}$ alkoxy groups (such as methoxy, ethoxy, propoxy, isopropoxy, butoxy, isobutoxy, sec-butoxy, and tert-butoxy) optionally substituted with substituent(s) selected from halogen atoms (such as fluorine, chlorine, bromine, and iodine atoms) and $C_{3-8}$ cycloalkyl groups,
n) $C_{7-16}$ aralkyloxy groups (such as benzyloxy),
o) $C_{6-14}$ aryloxy groups (such as phenoxy) optionally substituted with substituent(s) selected from $C_{1-6}$ alkoxy groups (such as methoxy), $C_{1-6}$ alkyl groups (such as methyl), and halogen atoms (such as fluorine, chlorine, bromine, and iodine atoms),
p) 5- to 8-membered aromatic heterocycle-oxy groups having 1 to 4 hetero atoms in addition to carbon atoms, selected from nitrogen, sulfur, and oxygen atoms (such as furyloxy, thienyloxy, pyrrolyloxy, oxazolyloxy, isoxazolyloxy, thiazolyloxy, isothiazolyloxy, imidazolyloxy, pyrazolyloxy, 1,2,3-oxadiazolyloxy, 1,2,4-oxadiazolyloxy, 1,3,4-oxadiazolyloxy, furazanyloxy, 1,2,3-thiadiazolyloxy, 1,2,4-thiadiazolyloxy, 1,3,4-thiadiazolyloxy, 1,2,3-triazolyloxy, 1,2,4-triazolyloxy, tetrazolyloxy, pyridyloxy, pyridazinyloxy, pyrimidinyloxy, pyrazinyloxy, and triazinyloxy),
q) formyl group,
r) $C_{1-6}$ alkyl-carbonyl groups (such as acetyl, ethylcarbonyl, propylcarbonyl, isopropylcarbonyl, and 2,2-dimethylpropylcarbonyl),
s) $C_{3-8}$ cycloalkyl-carbonyl groups (such as cyclopropylcarbonyl, cyclobutylcarbonyl, cyclopentylcarbonyl, and cyclohexylcarbonyl),
t) $C_{7-16}$ aralkyl-carbonyl groups (such as benzylcarbonyl), $C_{6-14}$ aryl-carbonyl groups (such as benzoyl)
u) optionally substituted 5- to 8-membered non-aromatic heterocycle-carbonyl groups having 1 to 4 hetero atoms in addition to carbon atoms, selected from nitrogen, sulfur, and oxygen atoms (such as pyrrolidinylcarbonyl, tetrahydrofurylcarbonyl, tetrahydrothienylcarbonyl, piperidinylcarbonyl, tetrahydropyranylcarbonyl, morpholinylcarbonyl, thiomorpholinylcarbonyl, and piperazinylcarbonyl),
v) 5- to 8-membered aromatic heterocycle-carbonyl groups having 1 to 4 hetero atoms in addition to carbon atoms, selected from nitrogen, sulfur, and oxygen atoms (such as furylcarbonyl, thienylcarbonyl, pyrrolylcarbonyl, oxazolylcarbonyl, isoxazolycarbonyl, thiazolylcarbonyl, isothiazolylcarbonyl, imidazolylcarbonyl, pyrazolylcarbonyl, 1,2,3-oxadiazolylcarbonyl, 1,2,4-oxadiazolylcarbonyl, 1,3,4-oxadiazolylcarbonyl, furazanylcarbonyl, 1,2,3-thiadiazolylcarbonyl, 1,2,4-thiadiazolylcarbonyl, 1,3,4-thiadiazolylcarbonyl, 1,2,3-triazolylcarbonyl, 1,2,4-triazolylcarbonyl, tetrazolylcarbonyl, pyridylcarbonyl, pyridazinylcarbonyl, pyrimidinylcarbonyl, pyrazinylcarbonyl, and triazinylcarbonyl),
w) carbamoyl group,
x) mono- or di-$C_{1-6}$ alkyl-carbamoyl groups (such as methylcarbamoyl, ethylcarbamoyl, propylcarbamoyl, isopropylcarbamoyl, dimethylcarbamoyl, diethylcarbamoyl, and dipropylcarbamoyl) optionally substituted with substituents selected from

(a) halogen atoms (such as fluorine, chlorine, bromine, and iodine),
(b) cyano,
(c) hydroxyl group,
(d) $C_{1-6}$ alkoxy groups (such as methoxy, ethoxy, propoxy, and isopropoxy),
(e) $C_{3-8}$ cycloalkyl groups (such as cyclopropyl, cyclobutyl, cyclopentyl, and cyclohexyl),
(f) mono- or di-$C_{1-6}$ alkylamino groups (such as methylamino, ethylamino, propylamino, isopropylamino, dimethylamino, diethylamino, dipropylamino, and diisopropylamino)
(g) carbamoyl group, and
(h) 5- to 6-membered heterocyclic groups (such as imidazolyl, thienyl, morpholinyl, pyrrolidinyl, tetrahydrofuryl, tetrahydrothienyl, piperidinyl, tetrahydropyranyl, thiomorpholinyl, piperazinyl, furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl),

y) mono- or di-$C_{7-16}$ aralkyl-carbamoyl groups (such as benzylcarbamoyl or dibenzylcarbamoyl) optionally substituted with substituent(s) selected from

(a) halogen atoms (such as fluorine, chlorine, bromine, and iodine),
(b) $C_{1-6}$ alkyl groups (such as methyl, ethyl, or isopropyl),
(c) sulfamoyl groups, and
(d) mono- or di-$C_{1-6}$ alkyl-sulfamoyl groups (such as methylaminosulfonyl, ethylaminosulfonyl, or isopropylaminosulfonyl),

z) tricyclic bridged ring-carbamoyl (such as bicyclo[3.1.1]heptyl and adamantyl) optionally substituted with substituent (s) selected from halogen atoms and $C_{1-6}$ alkyl groups,
aa) 5- to 10-membered heterocycle-carbamoyl (such as furylcarbamoyl, thienylcarbamoyl, pyrrolylcarbamoyl, oxazolylcarbamoyl, isoxazolylcarbamoyl, thiazolylcarbamoyl, isothiazolylcarbamoyl, imidazolylcarbamoyl, pyrazolylcarbamoyl, 1,2,3-oxadiazolylcarbamoyl, 1,2,4-oxadiazolylcarbamoyl, 1,3,4-oxadiazolylcarbamoyl, furazanylcarbamoyl, 1,2,3-thiadiazolylcarbamoyl, 1,2,4-thiadiazolylcarbamoyl, 1,3,4-thiadiazolylcarbamoyl, 1,2,3-triazolylcarbamoyl, 1,2,4-triazolylcarbamoyl, tetrazolylcarbamoyl, pyridylcarbamoyl, pyridazinylcarbamoyl, pyrimidinylcarbamoyl, pyrazinylcarbamoyl, and triazinylcarbamoyl) optionally substituted with substituent(s) selected from

(a) halogen atoms (such as fluorine, chlorine, bromine, and iodine),
(b) $C_{1-6}$ alkyl groups (such as methyl, ethyl, and isopropyl),
(c) $C_{1-6}$ alkoxy groups (such as methoxy, ethoxy, propoxy, isopropoxy),
(d) $C_{1-6}$ alkoxy-carbonyl groups (such as methoxycarbonyl, ethoxycarbonyl, propoxycarbonyl, and isopropoxycarbonyl),
(e) carbamoyl group, and
(f) oxo group,

bb) carboxyl group,
cc) $C_{1-6}$ alkoxy-carbonyl groups (such as methoxycarbonyl, ethoxycarbonyl, propoxycarbonyl, isopropoxycarbonyl, and tert-butoxycarbonyl),
dd) $C_{7-16}$ aralkyloxy-carbonyl groups (such as benzyloxycarbonyl),
ee) $C_{6-14}$ aryloxy-carbonyl groups (such as phenoxycarbonyl),
ff) thiol group,
gg) $C_{1-6}$ alkylsulfanyl groups (such as methylsulfanyl, ethylsulfanyl, and propylsulfanyl) optionally substituted with halogen atom(s) (such as fluorine, chlorine, bromine, and iodine atoms),
hh) $C_{7-16}$ aralkylsulfanyl groups (such as benzylthio),
ii) Ci-6 alkylsulfinyl groups (such as methylsulfinyl, ethylsulfinyl, propylsulfinyl, and butylsulfmyl),
jj) $C_{6-10}$ arylsulfinyl groups (such as phenylsulfinyl and naphthylsulfinyl),
kk) $C_{1-6}$ alkylsulfonyl groups (such as methylsulfonyl, ethylsulfonyl, propylsulfonyl, and isopropylsulfonyl),
ll) $C_{3-8}$ cycloalkylsulfonyl groups (such as cyclopropylsulfonyl, cyclobutylsulfonyl, and cyclopentylsulfonyl),
mm) $C_{6-14}$ arylsulfonyl groups (such as phenylsulfonyl, 1-naphthylsulfonyl, and 2-naphthylsulfonyl),
nn) $C_{7-16}$ aralkylsulfonyl groups (such as benzylsulfonyl),

oo) aminosulfonyl group,
pp) mono-N-$C_{1-6}$ alkylaminosulfonyl groups (such as methylaminosulfonyl and ethylaminosulfonyl),
qq) di-N,N-$C_{1-6}$ alkylaminosulfonyl groups (such as dimethylaminosulfonyl and diethylaminosulfonyl),
rr) sulfamoyl groups,
ss) mono- or di-$C_{1-6}$ alkylaminosulfonyl groups (such as isopropylaminosulfonyl),
tt) thiocarbamoyl group,
uu) 5- to 10-membered non-aromatic heterocyclic groups having 1 to 4 hetero atoms in addition to carbon atoms, selected from nitrogen, sulfur, and oxygen atoms (such as pyrrolidinyl, tetrahydrofuryl, tetrahydrothienyl, piperidyl, tetrahydropyranyl, morpholinyl, thiomorpholinyl, piperazinyl, tetrahydroazepinyl, and tetrahydrotriazoloazepinyl) [the non-aromatic heterocyclic groups are optionally substituted with substituent(s) selected from

(1) halogen atoms,
(2) $C_{1-6}$ alkyl groups (such as methyl) optionally substituted with substituent(s) selected from halogen atoms and $C_{1-6}$ alkyl groups,
(3) $C_{1-6}$ alkoxy groups (preferably methoxy),
(4) $C_{1-6}$ alkoxy-carbonyl groups (preferably methoxycarbonyl),
(5) carbamoyl group,
(6) $C_{1-6}$ alkanoylamino groups (preferably acetylamino group),
(7) oxo group,
(8) $C_{6-14}$ aryl groups (such as phenyl),
mono- or di-$C_{1-6}$ alkylaminosulfonyl groups (such as isopropylaminosulfonyl), and
(9) $C_{6-14}$ arylamino groups (such as aniline) optionally substituted with $C_1$-$_6$ alkanoylamino groups (such as acetylamino group)],

vv) 5- to 10-membered aromatic heterocyclic groups having 1 to 4 hetero atoms in addition to carbon atoms, selected from nitrogen, sulfur, and oxygen atoms (such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, benzothiophenyl, benzothiazolyl, pyrrolopyridinyl, pyrazolopyrimidinyl, indolyl, azepinyl, and triazoloazepinyl), [the aromatic heterocyclic groups are optionally substituted with substituent(s) selected from

(1) halogen atoms,
(2) $C_{1-6}$ alkyl groups (such as methyl) optionally substituted with substituent(s) selected from halogen atoms and $C_{1-6}$ alkyl groups,
(3) $C_{1-6}$ alkoxy groups (preferably methoxy),
(4) $C_{1-6}$ alkoxy-carbonyl groups (preferably methoxycarbonyl),
(5) carbamoyl group,
(6) $C_{1-6}$ alkanoylamino groups (preferably acetylamino group),
(7) oxo group,
(8) $C_{6-14}$ aryl groups (such as phenyl),
(9) mono- or di-$C_{1-6}$ alkylaminosulfonyl groups (such as isopropylaminosulfonyl), and
(10) $C_{6-14}$ arylamino groups (such as aniline) optionally substituted with $C_{1-6}$ alkanoylamino groups (such as acetylamino group)],

and are optionally condensed with a benzene ring (such as benzothienyl)],
ww) ureido group,
xx) $C_{1-6}$ alkyl-ureido groups (such as methylureido, ethylureido, and propylureido),
yy) $C_{6-14}$ aryl-ureido groups (such as phenylureido, 1-naphthylureido, and 2-naphthylureido)
zz) $C_{1-4}$ alkylenedioxy groups (such as methylenedioxy, ethylenedioxy, and propylenedioxy),
aaa) $C_{1-6}$ alkyl groups (such as methyl, ethyl, propyl, isopropyl, butyl, isobutyl, sec-butyl, tert-butyl, pentyl, neopentyl, and hexyl) optionally substituted with substituent(s) selected from $C_{6-14}$ arylthio groups (such as phenylthio) or the like,
bbb) $C_{6-14}$ aryl group (preferably phenyl)-carbamoyl optionally substituted with substituent(s) selected from halogen atoms (preferably fluorine or chlorine atoms), carbamoyl, $C_{1-6}$ alkyl groups, $C_{1-6}$ alkoxy groups (preferably methoxy, ethoxy, or propoxy), and mono- or di-$C_{1-6}$ alkylaminosulfonyl groups (preferably isopropylaminosulfonyl),
ccc) $C_{1-6}$ alkyl groups (such as methyl, ethyl, propyl, isopropyl, butyl, isobutyl, sec-butyl, tert-butyl, pentyl, neopentyl, and hexyl) optionally substituted with substituent(s) selected from halogen atoms (such as fluorine, chlorine, bromine, and iodine atoms), hydroxy, mono- or di-$C_{1-6}$ alkylamino groups (preferably dimethylamino), $C_{1-6}$ alkoxy groups (such as methoxy, ethoxy, and propoxy), $C_{6-14}$ arylsulfonyl groups, $C_{6-14}$ aryloxy groups (such as phenoxy) optionally

substituted with halogen atom(s) (such as fluorine, chlorine, bromine, or iodine atom), and heterocyclic groups (such as morpholinyl, pyridyl, imidazopyridyl, and benzimidazolyl), and

ddd) $C_{7-16}$ aralkyl groups (such as benzyl, 2-phenylethyl, 1-phenylethyl, 3-phenylpropyl, and 4-phenylbutyl) optionally substituted with a halogen atom (preferably fluorine),

wherein the number of substituents is 1 or more (preferably 1 to 4, and more preferably 1 to 3).

Examples of 5- to 8-membered rings (preferably 5- or 6-membered rings) which may be formed by two adjacent substituents of the "aryl group" or "aromatic heterocyclic group" represented by Ar include unsaturated carbon rings of 5-6 carbon atoms such as cyclopentadiene, cyclopentene, cyclohexene, cyclohexadiene, and benzene; and 5- or 6-membered unsaturated heterocycles having 1 or 2 hetero atoms in addition to carbon atoms, selected from nitrogen, sulfur, and oxygen atoms, such as thiadiazole, triazole, dioxole, pyrazine, dihydropyrazine, tetrahydropyrazine, pyridazine, oxadiazole, dihydropyrrole, pyrrole, dihydrofuran, furan, dihydrothiophene, thiophene, dihydroisoxazole, isoxazole, dihydrooxazole, oxazole, dihydroisothiazole, isothiazole, dihydrothiazole, thiazole, dihydropyran, pyran, dihydrothiopyran, thiopyran, dihydroimidazole, imidazole, tetrahydropyridine, dihydropyridine, pyridine, pyrimidine, dihydropyrimidine, and tetrahydropyrimidine. Such 5- to 8-membered (and preferably 5- or 6-membered rings) may have 1 or more (preferably 1 to 3) substituents selected from halogens (preferably fluorine or chlorine), hydroxy group, $C_{1-6}$ alkyl groups (preferably methyl, ethyl, or isopropyl), and oxo group.

Ar is preferably

(A) a phenyl group or

(B) 5 to 6-membered aromatic heterocyclic group (preferably pyrrolyl, imidazolyl, isoxazolyl, oxazolyl, oxadiazole, thienyl, pyrimidinyl, pyrazolyl, furyl, thiazolyl, pyridyl) (when the phenyl group or 5 to 6-membered aromatic heterocyclic group has 2 or more substituents, two adjacent substituents may together form a 5- to 8-membered (preferably 5 to 6-membered) ring (preferably cyclohexene, imidazole, dihydropyrrole, dihydropyridine, cyclopentene, benzene, tetrahydropyridine, pyridazine, dihydropyrazine, tetrahydropyrazine, furan, dihydrofuran, thiophene, 1,3-dioxole, 2,1,3-thiadiazole, 1,2,3-triazole, or benzene) optionally substituted with 1 or more (preferably 1 to 3) substituents selected from halogens (preferably fluorine or chlorine), hydroxy group, $C_{1-6}$ alkyl groups (preferably methyl, ethyl, or isopropyl), and oxo group)

either of which is optionally substituted with substituent(s) selected from

a) halogen atoms (preferably chlorine or fluorine)

b) cyano

c) mono- or di-$C_{1-6}$ alkylamino groups (preferably dimethylamino),

d) cyclic amino (preferably piperazinyl) optionally substituted with $C_{1-6}$ alkyl group(s) (preferably methyl),

e) $C_{1-6}$ alkyl groups (preferably methyl, ethyl, propyl, isopropyl, or tert-butyl)

optionally substituted with substituent(s) selected from

(1) halogen atoms (preferably fluorine),

(2) hydroxy,

(3) mono- or di-$C_{1-6}$ alkylamino groups (preferably dimethylamino),

(4) 5- 8-membered non-aromatic heterocyclic groups having 1 to 4 hetero atoms in addition to carbon atoms, selected from nitrogen, sulfur, and oxygen atoms (preferably pyrrolidinyl),

(5) $C_{1-6}$ alkoxy groups (preferably methoxy or ethoxy),

(6) $C_{6-14}$ aryl groups (preferably phenyl) optionally substituted with halogen atom(s) (preferably fluorine) and

(7) $C_{6-14}$ aryloxy groups (preferably phenoxy) optionally substituted with halogen atom(s) (preferably fluorine or chlorine),

f) $C_{3-8}$ cycloalkyl groups (such as cyclopropyl),

g) $C_{6-14}$ aryl groups (preferably phenyl)

optionally substituted with substituent(s) selected from

(1) halogen atoms (preferably fluorine or chlorine),

(2) mono- or di-$C_{1-6}$ alkylamino groups (preferably dimethylamino) and

(3) $C_{6-14}$ aryl groups (preferably phenyl),

h) $C_{1-6}$ alkoxy groups (preferably methoxy or ethoxy)

optionally substituted with substituent(s) selected from halogen atoms (preferably fluorine) and $C_{3-8}$ cycloalkyl groups (preferably cyclopropyl),

i) $C_{6-14}$ aryloxy groups (preferably phenoxy) optionally substituted with halogen atom(s) (preferably chlorine),
j) 5- to 8-membered aromatic heterocycle-oxy groups having 1 to 4 hetero atoms in addition to carbon atoms, selected from nitrogen, sulfur, and oxygen atoms (preferably pyridyloxy),
k) $C_{6-14}$ aryl-carbonyl groups (preferably benzoyl)
l) cyclic amino-carbonyl groups (preferably pyrrolidinylcarbonyl, piperazinylcarbonyl, and morpholinylcarbonyl) optionally substituted with substituent(s) selected from 5- or 6-membered heterocyclic groups (preferably furyl or pyridyl) and mono- or di-$C_{1-6}$ alkyl-sulfamoyl groups (preferably dimethylaminosulfonyl),
m) carbamoyl group
optionally substituted with substituent(s) selected from

(1) $C_{1-6}$ alkyl groups (preferably methyl, ethyl, propyl, or isopropyl)
optionally substituted with substituent(s) selected from

(a) hydroxy group,
(b) $C_{1-6}$ alkoxy groups (preferably methoxy),
(c) mono- or di-$C_{1-6}$ alkylamino groups (preferably dimethylamino),
(d) carbamoyl group,
(e) 5- or 6-membered heterocyclic groups (preferably imidazolyl, thienyl, or morpholinyl), and
(f) phenyl group optionally substituted with substituent(s) selected from halogen atoms (preferably fluorine) and sulfamoyl groups,

(2) $C_{6-14}$ aryl groups (preferably phenyl) optionally substituted with substituent(s) selected from

(a) halogen atoms (preferably chlorine or fluorine),
(b) $C_{1-6}$ alkyl groups (preferably methyl or propyl),
(c) halogenated $C_{1-6}$ alkyl groups (preferably trifluoromethyl),
(d) $C_{1-6}$ alkoxy groups (preferably methoxy),
(e) sulfamoyl groups (preferably isopropylaminosulfonyl),
(f) $C_{1-6}$ alkyl-sulfonyl groups (preferably isopropylsulfonyl), and
(g) carbamoyl group,

(3) tricyclic bridged cyclic groups (preferably tricyclo[3.3.1.1 ~ 3, 7~]decyl), and
(4) 5- to 10-membered heterocyclic groups (preferably pyridyl, thienyl, pyrazolyl, thiazolyl, dihydroisoindolyl, or tetrahydrobenzothiophenyl)
optionally substituted with substituent(s) selected from

(a) $C_{1-6}$ alkyl groups (preferably methyl or ethyl),
(b) $C_{1-6}$ alkoxy groups (preferably methoxy),
(c) $C_{1-6}$ alkoxy-carbonyl groups (preferably methoxycarbonyl),
(d) carbamoyl group, and
(e) oxo group,

n) carboxyl group,
o) $C_{1-6}$ alkoxy-carbonyl groups (preferably methoxycarbonyl, ethoxycarbonyl, or tert-butoxycarbonyl),
p) $C_{1-6}$ alkylsulfanyl groups (preferably methylsulfanyl) optionally substituted with halogen atom(s) (preferably fluorine),
q) $C_{1-6}$ alkylsulfonyl groups (preferably methylsulfonyl),
r) sulfamoyl groups,
s) mono- or di-$C_{1-6}$ alkylaminosulfonyl groups (preferably isopropylaminosulfonyl), and
t) 5- to 10-membered heterocyclic groups (preferably pyridyl, pyrimidinyl, pyrazinyl, oxazolyl, piperazinyl, thienyl, furyl, thiazolyl, benzothiophenyl, 6,7,8,9-tetrahydro-5H-[1,2,4]triazolo[4,3-a]azepinyl, 1,3-benzothiazolyl, pyrazolo[1,5-a]pyrimidinyl, indolyl, benzothiophenyl, and 1H-pyrrolo[2,3-b]pyridinyl) optionally substituted with substituent(s) selected from

(1) $C_{1-6}$ alkyl groups (preferably methyl, ethyl, or tert-butyl) optionally substituted with halogen atom(s) (preferably fluorine),
(2) $C_{1-6}$ alkoxy groups (preferably methoxy),
(3) $C_{1-6}$ alkoxy-carbonyl groups (preferably methoxycarbonyl),

(4) carbamoyl group,
(5) $C_{1-6}$ alkanoylamino groups (preferably acetylamino group),
(6) oxo group,
(7) $C_{6-14}$ aryl groups (preferably phenyl),
(8) mono- or di-$C_{1-6}$ alkylaminosulfonyl groups (preferably isopropylaminosulfonyl), and
(9) $C_{6-14}$ arylamino groups (preferably aniline) optionally substituted with $C_{1-6}$ alkanoylamino groups (preferably acetylamino group).

The following are specific examples of moieties excluding substituents for Ar when forming the "5- to 8-membered ring."

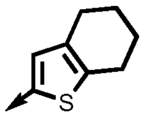 , 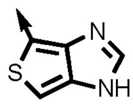 , 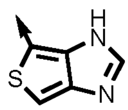 , 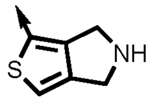 , 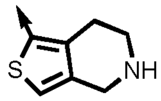 , 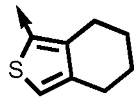

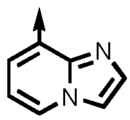 , 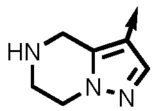 , 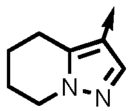 , 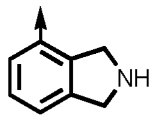

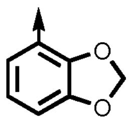 , 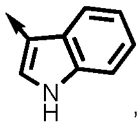 , 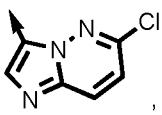 , 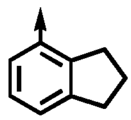 , 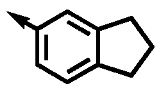

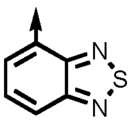 , 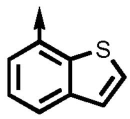 , 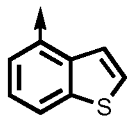 , 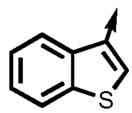 , 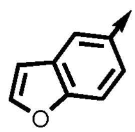

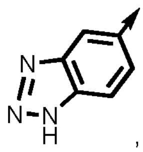 , 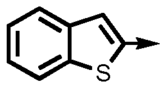 , 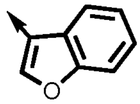 , 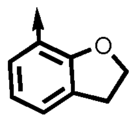

Ar is preferably the formula

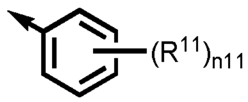, 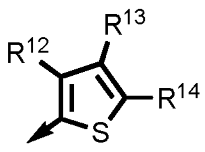, 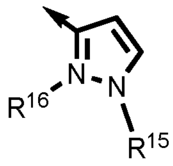, 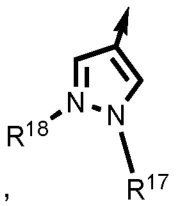,

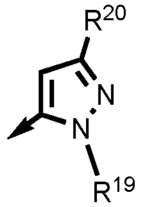, 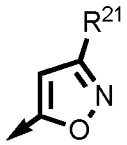, 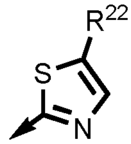, 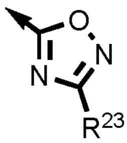, 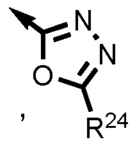,

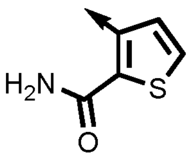, 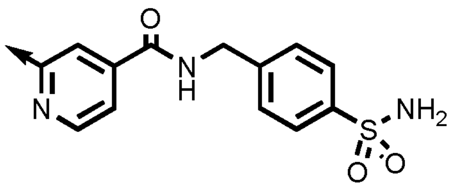, 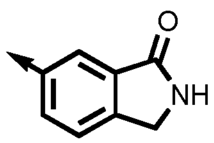,

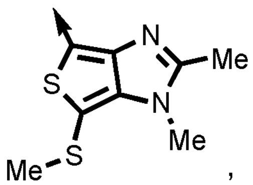, 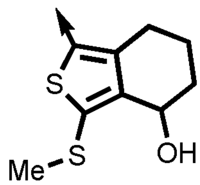, 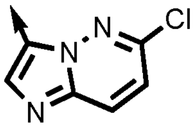,

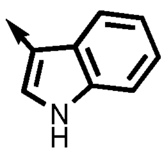, 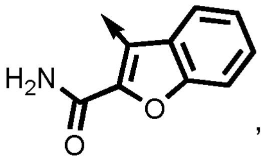, 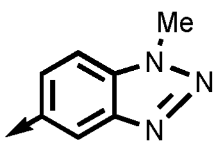, or 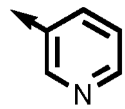

(In the formula,
n11 indicate an integer of 1 to 3,
$R^{11}$ is
a halogen atom (preferably fluorine or chlorine)
cyano,
$C_{1-6}$ alkyl group (preferably methyl or ethyl) optionally substituted with a halogen atom (preferably fluorine), hydroxy, or $C_{1-6}$ alkoxy group (preferably methoxy),
carbamoyl group,
di-$C_{1-6}$ alkylcarbamoyl group (preferably diethylcarbamoyl),
5- or 6-membered aromatic heterocycle-carbamoyl group (preferably pyridylcarbamoyl group),
$C_{1-6}$ alkylcarbonyl group (preferably acetyl), or $C_{1-6}$ alkylsulfonyl group (preferably methylsulfonyl),
$R^{12}$ is a hydrogen atom, $C_{1-6}$ alkyl group (preferably methyl), or carbamoyl group,
$R^{13}$ is a $C_{1-6}$ alkyl group (preferably methyl),
$R^{14}$ is a $C_{1-6}$ alkyl group (preferably methyl),
$R^{13}$ and $R^{14}$ may together form a ring (preferably cyclohexene),
$R^{15}$ is a hydrogen atom or $C_{1-6}$ alkyl group (preferably methyl or ethyl),
$R^{16}$ is a $C_{1-6}$ alkyl group (preferably methyl), or carbamoyl group,
$R^{17}$ is a hydrogen atom or $C_{1-6}$ alkyl group (preferably ethyl),

$R^{18}$ is a hydrogen atom or $C_{1-6}$ alkyl group (preferably tert-butyl),
$R^{19}$ is a hydrogen atom or $C_{1-6}$ alkyl group (preferably methyl),
$R^{20}$ is a di-$C_{3-8}$ cycloalkyl group (preferably cyclopropyl) or a 5- or 6-membered aromatic heterocycle (preferably thienyl),
$R^{21}$ is a $C_{1-6}$ alkyl group (preferably ethyl or isopropyl),
$R^{22}$ is a di-$C_{1-6}$ alkylcarbamoyl group (preferably dimethylcarbamoyl), 5- or 6-membered aromatic heterocycle-carbamoyl group (preferably pyridylcarbamoyl group), or 5- or 6-membered heterocyclic carbonyl (preferably pyrrolidinylcarbonyl),
$R^{23}$ is
a $C_{6-14}$ aryl group (preferably phenyl) optionally substituted with a halogen atom (preferably fluorine), or
a 5- to 10-membered aromatic heterocycle (preferably thienyl, pyridyl, pyrimidinyl, pyrazinyl, pyrrolo[1,5-a]pyrimidinyl, indolyl, or pyrrolo[2,3-b]pyridinyl) optionally substituted with $C_{1-6}$ alkyl group(s) (preferably methyl or tert-butyl) optionally substituted with halogen atom(s) (preferably fluorine),
$R^{24}$ is a 5- to 10-membered aromatic heterocycle (preferably thienyl or pyrrolo[1,5-a]pyrimidinyl) optionally substituted with $C_{1-6}$ alkyl group(s) (preferably methyl) optionally substituted with halogen atom(s) (preferably fluorine).

Preferred examples of compound (I) include compounds in which
$R^1$ is
an alkyl group optionally substituted with 1 or more (preferably 1 to 3) substituents selected from halogen atoms and hydroxyl group;
Ra and Rb are each independently a hydrogen atom or $C_{1-4}$ alkyl;
L is a bond, or

(1) -$(cm^2)_s$- (wherein s is an integer of 1 to 6) (such as -$CH_2$-),
(2) -$(CH_2)_{n1}$-CONH-$(CH_2)_{n2}$- (wherein n1 is an integer of 0 to 3, and n2 is an integer of 0 to 3.) {such as -$(CH_2)_2$-C(O)-NH-, -$(CH_2)_3$-C(O)-NH-, -$(CH_2)_2$-C(O)-NH-$CH_2$-, or $(CH_2)_3$C(O)-NH-$CH_2$)),
(3) -$(CH_2)_{n1}$-NHCO-$(CH_2)_{n2}$- (wherein n1 is an integer of 0 to 3, and n2 is an integer of 0 to 3.) {such as -$CH_2$-NH-C(O)-},
(4) -$(CH_2)_t$-O- (wherein t is an integer of 1 to 6) (such as -$(CH_2)_2$-O-), or
(5) -$(CH_2)_{n1}$-CONH-$(CH_2)_{n3}$-S- (wherein n1 is an integer of 0 to 3, and n3 is an integer of 0 to 3, providing that the sum of n1 and n3 is 0 to 5.) {such as -C(O)-NH-$(CH_2)_2$-S-},
any of which is optionally substituted with substituent(s) selected from $C_{1-6}$ alkyl groups (such as methyl), di-$C_{1-6}$ alkylamino-$C_{1-6}$ alkyl groups (such as N,N'-dimethylaminomethyl), and 5- to 6-membered non-aromatic heterocycle-$C_{1-6}$ alkyl groups (such as tetrahydrofurylmethyl), and when there are 2 or more substituents, two of them together may form a ring (preferably 3- to 5-membered saturated ring (more preferably cyclopropane, pyrrolidine, or thiazolidine));

Ring A is

(1) a non-aromatic carbon ring of 4-8 carbon atoms (preferably cyclopentene or cyclohexene) or
(2) 4- to 8-membered non-aromatic heterocycle having 1 to 3 hetero atoms selected from nitrogen, oxygen, and sulfur atoms (except the number of nitrogen atoms is 0 or 1) (preferably tetrahydropyridine or dihydropyran),
either of which is optionally substituted with 1 or more substituents selected from

a) alkyl groups (preferably methyl, ethyl, or isopropyl) optionally substituted with substituent(s) selected from

(1) hydroxy group,
(2) alkoxy groups,
(3) cycloalkyl groups (preferably cyclopropyl),
(4) amino group optionally substituted with 1 or 2 substituents selected from substituent group A,
(5) mercapto group,
(6) alkylsulfanyl groups,
(7) alkylsulfinyl groups,
(8) alkylsulfonyl groups,
(9) mono- or di-alkyl-sulfamoyl groups,
(10) alkanoyloxy groups,
(11) alkanoyl groups,
(12) carboxyl group,
(13) alkoxycarbonyl groups (preferably ethoxycarbonyl),

(14) carbamoyl group, and
(15) mono- or di-alkylcarbamoyl groups (preferably mono-methylcarbamoyl and di-methylcarbamoyl),

b) cycloalkyl groups optionally substituted with substituent(s) selected from halogen atoms and $C_{1-6}$ alkyl groups,
c) groups represented by the formula -CO-$R^{y1}$ (preferably carbamoyl, methylcarbamoyl, dimethylcarbamoyl, or acetyl) or
-$SO_2$-$R^{y1}$ (preferably methylsulfonyl)
(wherein $R^{y1}$ and $R^{y2}$ are each independently a hydrogen atom, $C_{1-6}$ alkyl group (preferably methyl) optionally substituted with halogen atom(s), $C_{1-6}$ alkoxy group (preferably methoxy, ethoxy, or tert-butoxy) optionally substituted with halogen atom(s), amino group, or mono- or di-$C_{1-6}$ alkylamino group (preferably mono-methylamino or di-methylamino)),
d) oxo group, and
e) non-aromatic heterocyclic groups optionally substituted with substituent(s) selected from halogen atoms and $C_{1-6}$ alkyl groups.

Ar is

(A) a phenyl group or
(B) 5 to 6-membered aromatic heterocyclic group (preferably pyrrolyl, imidazolyl, isoxazolyl, oxazolyl, oxadiazole, thienyl, pyrimidinyl, pyrazolyl, furyl, thiazolyl, pyridyl) (when the phenyl group or 5 to 6-membered aromatic heterocyclic group has 2 or more substituents, two adjacent substituents may together form a 5- to 8-membered (preferably 5 to 6-membered) ring (preferably cyclohexene, imidazole, dihydropyrrole, dihydropyridine, cyclopentene, benzene, tetrahydropyridine, pyridazine, dihydropyrazine, tetrahydropyrazine, furan, dihydrofuran, thiophene, 1,3-dioxole, 2,1,3-thiadiazole, 1,2,3-triazole, or benzene) either of which is optionally substituted with substituent(s) selected from

a) halogen atoms (preferably chlorine or fluorine)
b) cyano
c) mono- or di-$C_{1-6}$ alkylamino groups (preferably dimethylamino),
d) cyclic amino groups (preferably piperazinyl) optionally substituted with $C_{1-6}$ alkyl group(s) (preferably methyl)
e) $C_{1-6}$ alkyl groups (preferably methyl, ethyl, propyl, isopropyl, or tert-butyl)
optionally substituted with substituent(s) selected from

(1) halogen atoms (preferably fluorine),
(2) hydroxy,
(3) mono- or di-$C_{1-6}$ alkylamino groups (preferably dimethylamino),
(4) 5- 8-membered non-aromatic heterocyclic groups having 1 to 4 hetero atoms in addition to carbon atoms, selected from nitrogen, sulfur, and oxygen atoms (preferably pyrrolidinyl),
(5) $C_{1-6}$ alkoxy groups (preferably methoxy or ethoxy),
(6) $C_{6-14}$ aryl groups (preferably phenyl) optionally substituted with halogen atom(s) (preferably fluorine) and
(7) $C_{6-14}$ aryloxy groups (preferably phenoxy) optionally substituted with halogen atom(s) (preferably fluorine or chlorine),

f) $C_{3-8}$ cycloalkyl groups (such as cyclopropyl),
g) $C_{6-14}$ aryl groups (preferably phenyl)
optionally substituted with substituent(s) selected from

(1) halogen atoms (preferably fluorine or chlorine),
(2) mono- or di-$C_{1-6}$ alkylamino groups (preferably dimethylamino) and
(3) $C_{6-14}$ aryl groups (preferably phenyl),

h) $C_{1-6}$ alkoxy groups (preferably methoxy or ethoxy)
optionally substituted with substituent(s) selected from halogen atoms (preferably fluorine) and $C_{3-8}$ cycloalkyl groups (preferably cyclopropyl),
i) $C_{6-14}$ aryloxy groups (preferably phenoxy) optionally substituted with halogen atom(s) (preferably chlorine),
j) 5- to 8-membered aromatic heterocycle-oxy groups having 1 to 4 hetero atoms in addition to carbon

atoms, selected from nitrogen, sulfur, and oxygen atoms (preferably pyridyloxy),
k) $C_{6-14}$ aryl-carbonyl groups (preferably benzoyl)
l) cyclic amino-carbonyl groups (preferably pyrrolidinylcarbonyl, piperazinylcarbonyl, and morpholinylcarbonyl)
optionally substituted with substituent(s) selected from 5- or 6-membered heterocyclic groups (preferably furyl or pyridyl) and mono- or di-$C_{1-6}$ alkyl-sulfamoyl groups (preferably dimethylaminosulfonyl),
m) carbamoyl group
optionally substituted with substituent(s) selected from

(1) $C_{1-6}$ alkyl groups (preferably methyl, ethyl, propyl, or isopropyl)
optionally substituted with substituent(s) selected from

(a) hydroxy group,
(b) $C_{1-6}$ alkoxy groups (preferably methoxy),
(c) mono- or di-$C_{1-6}$ alkylamino groups (preferably dimethylamino),
(d) carbamoyl group,
(e) 5- or 6-membered heterocyclic groups (preferably imidazolyl, thienyl, or morpholinyl), and
(f) phenyl group optionally substituted with substituent(s) selected from halogen atoms (preferably fluorine) and sulfamoyl groups,

(2) $C_{6-14}$ aryl groups (preferably phenyl) optionally substituted with substituent(s) selected from

(a) halogen atoms (preferably chlorine or fluorine),
(b) $C_{1-6}$ alkyl groups (preferably methyl or propyl),
(c) halogenated $C_{1-6}$ alkyl groups (preferably trifluoromethyl),
(d) $C_{1-6}$ alkoxy groups (preferably methoxy),
(e) sulfamoyl groups (preferably isopropylaminosulfonyl),
(f) $C_{1-6}$ alkyl-sulfonyl groups (preferably isopropylsulfonyl), and
(g) carbamoyl group,

(3) tricyclic bridged cyclic groups (preferably tricyclo[3.3.1.1 ~ 3, 7~]decyl), and
(4) 5- to 10-membered heterocyclic groups (preferably pyridyl, thienyl, pyrazolyl, thiazolyl, dihydroisoindolyl, or tetrahydrobenzothiophenyl)

optionally substituted with substituent(s) selected from

(a) $C_{1-6}$ alkyl groups (preferably methyl or ethyl),
(b) $C_{1-6}$ alkoxy groups (preferably methoxy),
(c) $C_{1-6}$ alkoxy-carbonyl groups (preferably methoxycarbonyl),
(d) carbamoyl group, and
(e) oxo group,

n) carboxyl group,
o) $C_{1-6}$ alkoxy-carbonyl groups (preferably methoxycarbonyl, ethoxycarbonyl, or tert-butoxycarbonyl),
p) $C_{1-6}$ alkylsulfanyl groups (preferably methylsulfanyl) optionally substituted with halogen atom(s) (preferably fluorine),
q) $C_{1-6}$ alkylsulfonyl groups (preferably methylsulfonyl),
r) sulfamoyl groups,
s) mono- or di-$C_{1-6}$ alkylaminosulfonyl groups (preferably isopropylaminosulfonyl), and
t) 5- to 10-membered heterocyclic groups (preferably pyridyl, pyrimidinyl, pyrazinyl, oxazolyl, piperazinyl, thienyl, furyl, thiazolyl, benzothiophenyl, 6,7,8,9-tetrahydro-5H-[1,2,4]triazolo[4,3-a]azepinyl, 1,3-benzothiazolyl, pyrazolo[1,5-a]pyrimidinyl, indolyl, benzothiophenyl, and 1H-pyrrolo[2,3-b]pyridinyl) optionally substituted with substituent(s) selected from

(1) $C_{1-6}$ alkyl groups (preferably methyl, ethyl, or tert-butyl) optionally substituted with halogen atom(s) (preferably fluorine),
(2) $C_{1-6}$ alkoxy groups (preferably methoxy),
(3) $C_{1-6}$ alkoxy-carbonyl groups (preferably methoxycarbonyl),

(4) carbamoyl group,
(5) $C_{1-6}$ alkanoylamino groups (preferably acetylamino group),
(6) oxo group,
(7) $C_{6-14}$ aryl groups (preferably phenyl),
(8) mono- or di-$C_{1-6}$ alkylaminosulfonyl groups (preferably isopropylaminosulfonyl), and
(9) $C_{6-14}$ arylamino groups (preferably aniline) optionally substituted with $C_{1-6}$ alkanoylamino groups (preferably acetylamino group).

Preferred examples of compound (I) also include compounds in which
$R^1$ is an optionally halogenated $C_{1-6}$ alkyl (such as trifluoromethyl),
Ra and Rb are each a hydrogen atom,
L is a bond, or
$-(CH_2)_2-C(O)-NH-$, $-(CH_2)_3-C(O)-NH-$, $-CH_2-$, $CH(CH_3)-C(O)-NH-$, $-CH_2-NH-C(O)-$, $-CH_2O-$ $-C(O)-NH-$, - $C(O)-NH-CH_2-$, $-C(O)-NH-(CH_2)_2-$, $-C(O)-NH-CH(CH_3)-$, $-(CH_2)_2-C(O)-NH-CH_2-$, $-(CH_2)_2-C(O)-NH-CH(CH_3)-$, $-CH_2-CH(CH_3)-C(O)-NH-CH_2-$, $-CH_2-CH(CH_3)-C(O)-NH-CH(CH_3)-$, $-(CH_2)_2-C(O)-NH-(CH_2)_3-$, $-CH_2-CH(CH_3)-C(O)-NH-(CH_2)_3-$, $-(CH_2)_3-C(O)-NH-(CH_2)_3-$, $-C(O)-NH-(CH_2)_2-S-$,

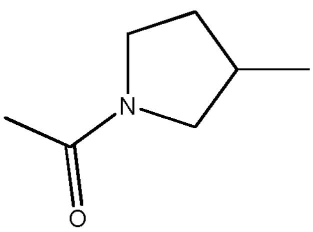
,
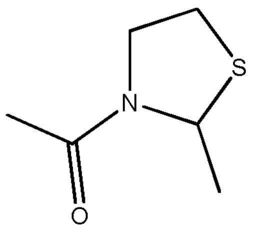
or
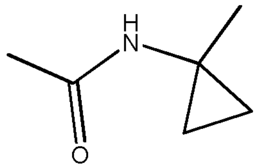

any of which is optionally substituted with substituent(s) selected from 5- to 6-membered non-aromatic heterocycle-$C_{1-6}$ alkyl groups (such as tetrahydrofurylmethyl);
Ring A is

(1) a non-aromatic carbon ring of 4-8 carbon atoms (preferably cyclopentene or cyclohexene) or
(2) 4- to 8-membered non-aromatic heterocycle having one hetero atom selected from nitrogen or oxygen atoms (preferably tetrahydropyridine or dihydropyran),
either of which is optionally substituted with 1 or more substituents selected from alkyl groups (preferably methyl, ethyl, or isopropyl) optionally substituted with substituent(s) selected from hydroxy group,
cycloalkyl groups (preferably cyclopropyl),
carboxyl group,
alkoxycarbonyl groups (preferably ethoxycarbonyl),
carbamoyl group, and
mono- or di-alkylcarbamoyl groups (preferably mono-methylcarbamoyl and di-methylcarbamoyl), cycloalkyl groups optionally substituted with substituent(s) selected from halogen atoms and $C_{1-6}$ alkyl groups,
groups represented by the formula $-CO-R^{y1}$ (preferably carbamoyl, methylcarbamoyl, dimethylcarbamoyl, or acetyl), and
$-SO_2-R^{y1}$ (preferably methylsulfonyl)
(wherein $R^{y1}$ and $R^{y2}$ are each independently a hydrogen atom, $C_{1-6}$ alkyl group (preferably methyl) optionally substituted with halogen atom(s), $C_{1-6}$ alkoxy group (preferably methoxy, ethoxy, or tert-butoxy), amino group optionally substituted with halogen atom(s), or mono- or di-$C_{1-6}$ alkylamino group (preferably mono-methylamino or di-methylamino)),
Ar is

(A) a phenyl group or
(B) 5 to 6-membered aromatic heterocyclic group (preferably pyrrolyl, imidazolyl, isoxazolyl, oxazolyl, oxadiazole, thienyl, pyrimidinyl, pyrazolyl, furyl, thiazolyl, pyridyl) (when the phenyl group or 5 to 6-membered aromatic heterocyclic group has 2 or more substituents, two adjacent substituents may together form a 5- to 8-membered (preferably 5 to 6-membered) ring (preferably cyclohexene, imidazole, dihydropyrrole, dihydropyridine, cyclopentene, benzene, tetrahydropyridine, pyridazine, dihydropyrazine, tetrahydropyrazine, furan, dihydrofuran, thiophene, 1,3-dioxole, 2,1,3-thiadiazole, 1,2,3-triazole, or benzene) optionally substituted with substituent(s) selected from halogens (preferably chlorine), hydroxy group, $C_{1-6}$ alkyl groups (preferably methyl), and oxo

group,
either of which is optionally substituted with substituent(s) selected from

a) halogen atoms (preferably chlorine or fluorine),
b) cyano,
c) mono- or di-$C_{1-6}$ alkylamino groups (preferably dimethylamino),
d) cyclic amino (preferably piperazinyl) optionally substituted with $C_{1-6}$ alkyl group(s) (preferably methyl), and
e) $C_{1-6}$ alkyl groups (preferably methyl, ethyl, propyl, isopropyl, or tert-butyl)
optionally substituted with substituent(s) selected from

(1) halogen atoms (preferably fluorine),
(2) hydroxy,
(3) mono- or di-$C_{1-6}$ alkylamino groups (preferably dimethylamino),
(4) 5- to 8-membered non-aromatic heterocyclic groups having 1 to 4 hetero atoms in addition to carbon atoms, selected from nitrogen, sulfur, and oxygen atoms (preferably pyrrolidinyl),
(5) $C_{1-6}$ alkoxy groups (preferably methoxy or ethoxy),
(6) $C_{6-14}$ aryl groups (preferably phenyl) optionally substituted with halogen atom(s) (preferably fluorine), and
(7) $C_{6-14}$ aryloxy groups (preferably phenoxy) optionally substituted with halogen atom(s) (preferably fluorine or chlorine),

f) $C_{3-8}$ cycloalkyl groups (such as cyclopropyl),
g) $C_{6-14}$ aryl groups (preferably phenyl)
optionally substituted with substituent(s) selected from

(1) halogen atoms (preferably fluorine or chlorine),
(2) mono- or di-$C_{1-6}$ alkylamino groups (preferably dimethylamino) and
(3) $C_{6-14}$ aryl groups (preferably phenyl),

h) $C_{1-6}$ alkoxy groups (preferably methoxy or ethoxy) optionally substituted with substituent(s) selected from halogen atoms (preferably fluorine) and $C_{3-8}$ cycloalkyl groups (preferably cyclopropyl),
i) $C_{6-14}$ aryloxy groups (preferably phenoxy) optionally substituted with halogen atom(s) (preferably chlorine),
j) 5- to 8-membered aromatic heterocycle-oxy groups having 1 to 4 hetero atoms in addition to carbon atoms, selected from nitrogen, sulfur, and oxygen atoms (preferably pyridyloxy),
k) $C_{6-14}$ aryl-carbonyl groups (preferably benzoyl)
l) cyclic amino-carbonyl groups (preferably pyrrolidinylcarbonyl, piperazinylcarbonyl, and morpholinylcarbonyl)
optionally substituted with substituent(s) selected from 5- or 6-membered heterocyclic groups (preferably furyl or pyridyl) and mono- or di-$C_{1-6}$ alkyl-sulfamoyl groups (preferably dimethylaminosulfonyl),
m) carbamoyl group
optionally substituted with substituent(s) selected from

(1) $C_{1-6}$ alkyl groups (preferably methyl, ethyl, propyl, or isopropyl)
optionally substituted with substituent(s) selected from

(a) hydroxy group,
(b) $C_{1-6}$ alkoxy groups (preferably methoxy),
(c) mono- or di-$C_{1-6}$ alkylamino groups (preferably dimethylamino),
(d) carbamoyl group,
(e) 5- or 6-membered heterocyclic groups (preferably imidazolyl, thienyl, or morpholinyl), and
(f) phenyl group optionally substituted with substituent(s) selected from halogen atoms (preferably fluorine) and sulfamoyl groups,

(2) $C_{6-14}$ aryl groups (preferably phenyl) optionally substituted with substituent(s) selected from

(a) halogen atoms (preferably chlorine or fluorine),
(b) $C_{1-6}$ alkyl groups (preferably methyl or propyl),
(c) halogenated $C_{1-6}$ alkyl groups (preferably trifluoromethyl),

(d) $C_{1-6}$ alkoxy groups (preferably methoxy),
(e) sulfamoyl groups (preferably isopropylaminosulfonyl),
(f) $C_{1-6}$ alkyl-sulfonyl groups (preferably isopropylsulfonyl), and
(g) carbamoyl group,

(3) tricyclic bridged cyclic groups (preferably tricyclo[3.3.1.1 ~ 3, 7~]decyl), and
(4) 5- to 10-membered heterocyclic groups (preferably pyridyl, thienyl, pyrazolyl, thiazolyl, dihydroisoindolyl, or tetrahydrobenzothiophenyl)
optionally substituted with substituent(s) selected from

(a) $C_{1-6}$ alkyl groups (preferably methyl or ethyl),
(b) $C_{1-6}$ alkoxy groups (preferably methoxy),
(c) $C_{1-6}$ alkoxy-carbonyl groups (preferably methoxycarbonyl),
(d) carbamoyl group, and
(e) oxo group,

n) carboxyl group,
o) $C_{1-6}$ alkoxy-carbonyl groups (preferably methoxycarbonyl, ethoxycarbonyl, or tert-butoxycarbonyl),
p) $C_{1-6}$ alkylsulfanyl groups (preferably methylsulfanyl) optionally substituted with halogen atom(s) (preferably fluorine),
q) $C_{1-6}$ alkylsulfonyl groups (preferably methylsulfonyl),
r) sulfamoyl groups,
s) mono- or di-$C_{1-6}$ alkylaminosulfonyl groups (preferably isopropylaminosulfonyl), and
t) 5- to 10-membered heterocyclic groups (preferably pyridyl, pyrimidinyl, pyrazinyl, oxazolyl, piperazinyl, thienyl, furyl, thiazolyl, benzothiophenyl, 6,7,8,9-tetrahydro-5H-[1,2,4]triazolo[4,3-a]azepinyl, 1,3-benzothiazolyl, pyrazolo[1,5-a]pyrimidinyl, indolyl, benzothiophenyl, and 1H-pyrrolo[2,3-b]pyridinyl) optionally substituted with substituent(s) selected from

(1) $C_{1-6}$ alkyl groups (preferably methyl, ethyl, or tert-butyl) optionally substituted with halogen atom(s) (preferably fluorine),
(2) $C_{1-6}$ alkoxy groups (preferably methoxy),
(3) $C_{1-6}$ alkoxy-carbonyl groups (preferably methoxycarbonyl),
(4) carbamoyl group,
(5) $C_{1-6}$ alkanoylamino groups (preferably acetylamino group),
(6) oxo group,
(7) $C_{6-14}$ aryl groups (preferably phenyl),
(8) mono- or di-$C_{1-6}$ alkylaminosulfonyl groups (preferably isopropylaminosulfonyl), and
(9) $C_{6-14}$ arylamino groups (preferably aniline) optionally substituted with $C_{1-6}$ alkanoylamino groups (preferably acetylamino group).

Preferred examples of compound (I) also include compounds in which
$R^1$ is an optionally halogenated $C_{1-6}$ alkyl (such as trifluoromethyl),
Ra and Rb are hydrogen atoms,
L is a bond, or

(1) $-(CH_2)_s-$ (wherein s is an integer of 1 to 6) (such as $-CH_2-$),
(2) $-(CH_2)_{n1}-CONH-(CH_2)_{n2}-$ (wherein n1 is an integer of 0 to 3, and n2 is an integer of 0 to 3.) (such as $-(CH_2)_2-C(O)-NH-$, $-(CH_2)_2-C(O)-NH-CH_2-$), or
(3) $-(CH_2)_t-O-$ (wherein t is an integer of 1 to 6) (such as $-(CH_2)_2-O-$),
any of which is optionally substituted with substituent(s) selected from $C_{1-6}$ alkyl groups, di-$C_{1-6}$ alkylamino-$C_{1-6}$ alkyl groups (such as N,N-dimethylaminomethyl), and 5- to 6-membered non-aromatic heterocycle-$C_{1-6}$ alkyl groups (such as tetrahydrofurylmethyl),
Ring A is cyclohexene,
Ar is
a phenyl group or 5- or 6-membered aromatic heterocyclic group (such as pyrazolyl, furyl, thiazolyl, pyridyl) (when the 5- or 6-membered aromatic heterocyclic group has 2 or more substituents, two adjacent substituents together may form a 6-membered ring (i.e. 6-membered carbon ring such as cyclohexene)) either of which is optionally substituted with substituent(s) selected from

(a) halogen atoms (preferably chlorine or fluorine),
(b) $C_{1-6}$ alkoxy groups (preferably methoxy or ethoxy)
(c) $C_{1-6}$ alkyl groups (such as methyl),
(d) carbamoyl group, optionally substituted with substituent(s) selected from

(i) $C_{1-6}$ alkyl groups, which may a substituent selected from

(1) hydroxy group,
(2) $C_{1-6}$ alkoxy groups (such as methoxy),
(3) dimethylamino group,
(4) carbamoyl group,
(5) 5- or 6-membered heterocyclic groups (such as imidazolyl, thienyl, or morpholino), and
(6) phenyl group optionally substituted with substituent(s) selected from halogen atoms (such as fluorine) and sulfamoyl groups,

(ii) phenyl group optionally substituted with substituent(s) selected from halogen atoms (such as chlorine and fluorine), $C_{1-6}$ alkyl groups (such as methyl and propyl), halogenated $C_{1-6}$ alkyl groups (such as trifluoromethyl), $C_{1-6}$ alkoxy groups (such as methoxy), $C_{1-6}$ alkyl-sulfamoyl groups (such as isopropylsulfamoyl), and carbamoyl group,
(iii) tricyclic bridged cyclic groups (such as tricyclo[3.3.1.1 ~ 3, 7~]decyl), and
(iv) 5- to 10-membered heterocyclic groups (such as thienyl, thiazolyl, pyrazolyl, pyridyl, dihydroisoindolyl, or tetrahydrobenzothiophenyl) optionally substituted with substituent(s) selected from $C_{1-6}$ alkyl groups (such as methyl or ethyl), $C_{1-6}$ alkoxy groups (such as methoxy), $C_{1-6}$ alkoxy-carbonyl groups (such as methoxycarbonyl), carbamoyl group, and oxo group,

(e) carboxyl group,
(f) $C_{1-6}$ alkoxy-carbonyl groups (such as methoxycarbonyl and ethoxycarbonyl),
(g) cyclic amino-carbonyl groups (such as pyrrolidinylcarbonyl, piperazinylcarbonyl, and morpholinylcarbonyl) optionally substituted with substituent(s) selected from 5- or 6-membered heterocyclic groups (such as furyl and pyridyl) and mono- or di-$C_{1-6}$ alkyl-sulfamoyl groups, and
(h) sulfamoyl groups.

Preferred examples of compound (I) include compounds in which
$R^1$ is trifluoromethyl,
Ra and Rb are hydrogen atoms,
L is a bond, or $-(CH_2)_2-C(O)-NH-$, $-(CH_2)_3-C(O)-NH-$, $-CH_2-CH(Me)-C(O)-NH-$, $-CH_2-NH-C(O)-$, $-CH_2O--C(O)-NH-$, $-C(O)-NH-CH_2-$, $-C(O)-NH-(CH_2)_2-$, $-C(O)-NH-CH(CH_3)-$, $-(CH_2)_2-C(O)-NH-CH_2-$, $-(CH_2)_2-C(O)-NH-CH(CH_3)-$, or

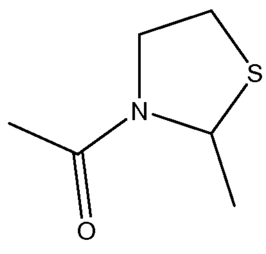

Ring A is cyclopentene, cyclohexene, tetrahydropyridine, or dihydropyran
any of which is optionally substituted with 1 or more substituents selected from

(a) alkyl groups (preferably methyl or ethyl) optionally substituted with substituent(s) selected from hydroxy group, cycloalkyl groups (preferably cyclopropyl), carboxyl group, alkoxycarbonyl groups (preferably ethoxycarbonyl), carbamoyl group, and
mono- or di-alkylcarbamoyl groups (preferably mono-methylcarbamoyl or di-methylcarbamoyl), and
(b) groups represented by the formula: $-CO-R^{y1}$ ($R^{y1}$ is the same as above.) (preferably carbamoyl, methylcarbamoyl, dimethylcarbamoyl, and acetyl),

Ar is a group represented by the formula

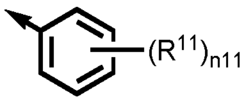 , 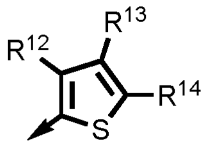 , 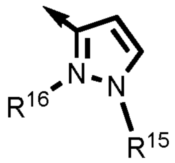 , 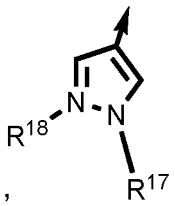 ,

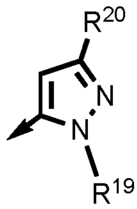 , 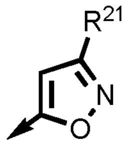 , 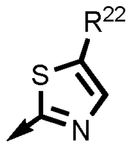 , 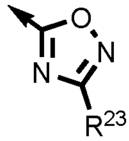 , 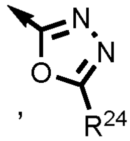 ,

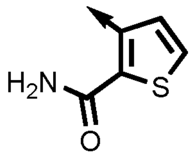 , 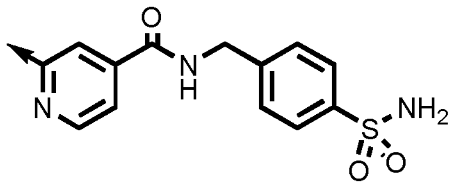 , 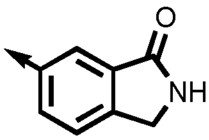 ,

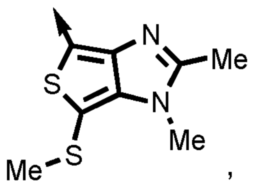 , 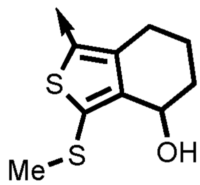 , 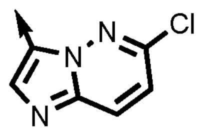 ,

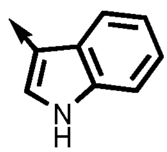 , 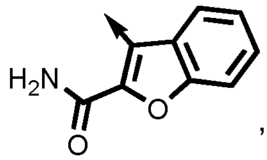 , 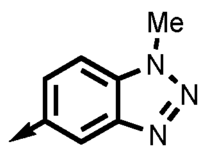 or 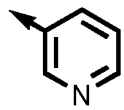

(In the formulas, n11 indicate an integer of 1 to 3,
$R^{11}$ is
a halogen atom (preferably fluorine or chlorine)
cyano,
$C_{1-6}$ alkyl group (preferably methyl or ethyl) optionally substituted with a halogen atom (preferably fluorine), hydroxy, or $C_{1-6}$ alkoxy group (preferably methoxy),
carbamoyl group,
di-$C_{1-6}$ alkylcarbamoyl group (preferably diethylcarbamoyl),
5- or 6-membered aromatic heterocycle-carbamoyl group (preferably pyridylcarbamoyl group),
$C_{1-6}$ alkylcarbonyl group (preferably acetyl), or
$C_{1-6}$ alkylsulfonyl group (preferably methylsulfonyl),
$R^{12}$ is a hydrogen atom, $C_{1-6}$ alkyl group (preferably methyl), or carbamoyl group,
$R^{13}$ is a $C_{1-6}$ alkyl group (preferably methyl),
$R^{14}$ is a $C_{1-6}$ alkyl group (preferably methyl),
$R^{13}$ and $R^{14}$ may together form a ring (preferably cyclohexene),
$R^{15}$ is a hydrogen atom or $C_{1-6}$ alkyl group (preferably methyl or ethyl),
$R^{16}$ is a $C_{1-6}$ alkyl group (preferably methyl), or carbamoyl group,

$R^{17}$ is a hydrogen atom or $C_{1-6}$ alkyl group (preferably ethyl),
$R^{18}$ is a hydrogen atom or $C_{1-6}$ alkyl group (preferably tert-butyl),
$R^{19}$ is a hydrogen atom or $C_{1-6}$ alkyl group (preferably methyl),
$R^{20}$ is a di-$C_{3-8}$ cycloalkyl group (preferably cyclopropyl) or a 5- or 6-membered aromatic heterocycle (preferably thienyl),
$R^{21}$ is a $C_{1-6}$ alkyl group (preferably ethyl or isopropyl),
$R^{22}$ is a di-$C_{1-6}$ alkylcarbamoyl group (preferably dimethylcarbamoyl), 5- or 6-membered aromatic heterocycle-carbamoyl group (preferably pyridylcarbamoyl group), or 5- or 6-membered heterocyclic carbonyl (preferably pyrrolidinylcarbonyl),
$R^{23}$ is
a $C_{6-14}$ aryl group (preferably phenyl) optionally substituted with a halogen atom (preferably fluorine), or a 5- to 10-membered aromatic heterocycle (preferably thienyl, pyridyl, pyrimidinyl, pyrazinyl, pyrrolo[1,5-a]pyrimidinyl, indolyl, or pyrrolo[2,3-b]pyridinyl) optionally substituted with $C_{1-6}$ alkyl group(s) (preferably methyl or tert-butyl) optionally substituted with halogen atom(s) (preferably fluorine) ,
$R^{24}$ is a 5- to 10-membered aromatic heterocycle (preferably thienyl or pyrrolo[1,5-a]pyrimidinyl) optionally substituted with $C_{1-6}$ alkyl group(s) (preferably methyl) optionally substituted with halogen atom(s)
(preferably fluorine).

The following compounds are also outside the scope of the compounds of the present invention.

(1) Compounds represented by the formula

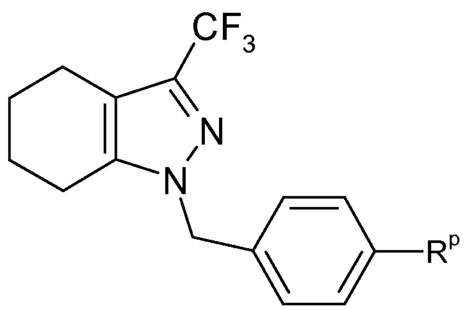

[In the formula, $R^p$ represents a substituent.];
(2) the formula

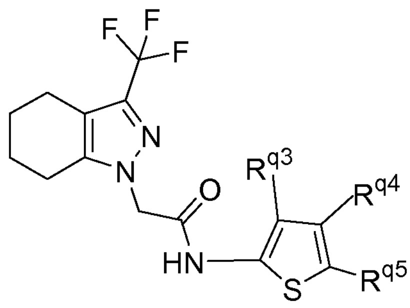

[In the formula,
$R^{q3}$ represents a hydrogen atom or substituent, and $R^{q4}$ and $R^{q5}$, which may be the same or different, represent a $C_{1-6}$ alkyl group, or are bonded together to form a 6-membered non-aromatic ring.];
(3) the formula

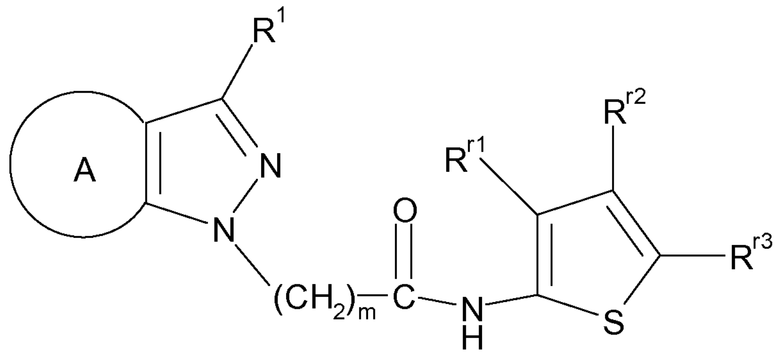

[In the formula,
$R^1$ represents trifluoromethyl,
$R^{r1}$ represents hydroxymethyl, carboxyl, or optionally substituted carbamoyl group,
$R^{r2}$ and $R^{r3}$ each independently represent hydrogen, $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,
ring A represents cyclohexene, and
m represents the integer 2.]

The following compounds may also lie outside the scope of the compounds of the present invention.

(1) the compounds in which $R^1$ is -CO-NHR$^t$ (wherein $R^t$ is an optionally substituted $C_4$ or higher hydrocarbon group.);
(2) compounds represented by the formula

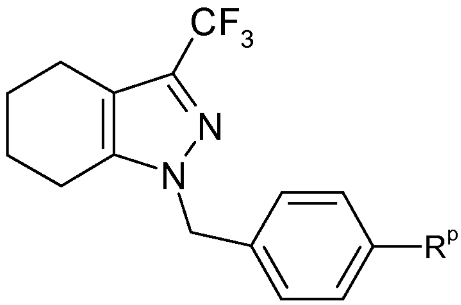

[In the formula, $R^p$ represents a substituent.];
(3) compounds represented by the formula

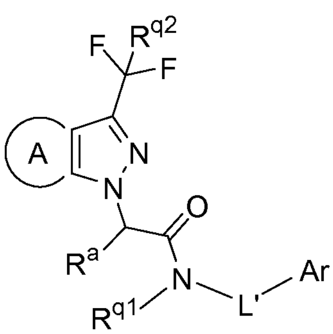

[In the formula,
$R^{q2}$ represents a hydrogen atom or fluorine atom,
$R^{q1}$ represents a hydrogen atom or substituent,
L' represents a bond, or a spacer in which the number of atoms in the main chain is 1 to 6, Ring A represents an

optionally substituted non-aromatic carbon ring of 4-8 carbon atoms, and the other symbols are synonymous with the above.];

(4) compounds represented by the formula

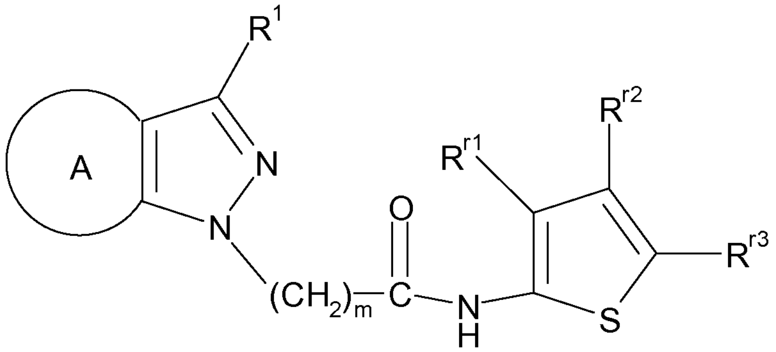

[In the formula,

$R^1$ represents trifluoromethyl,

$R^{r1}$ represents hydroxymethyl, carboxyl, or optionally substituted carbamoyl,

$R^{r2}$ and $R^{r3}$ each independently represent hydrogen, $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,

Ring A represents cyclohexene, and

m represents the integer 2.]; and

ring A represents cyclohexene,

m represents the integer 2.]; and

(5) compounds represented by the formulas

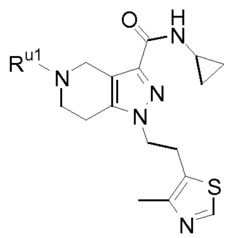

,

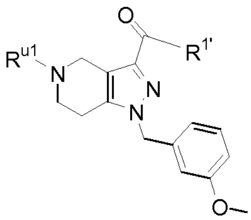

,

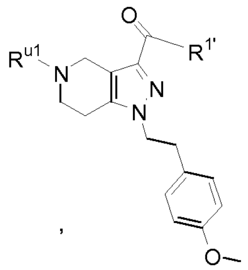

or

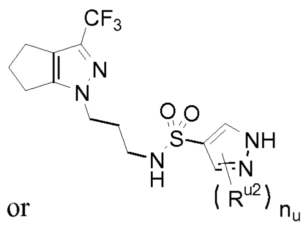

[In the formula,

$R^{1'}$ represents a dimethylamino group, monoethylamino group, or monocyclopropylamino group,

$R^{u1}$ represents -CO-$R^{u1'}$ ($R^{u1'}$ represents a substituent.), optionally substituted $C_{1-4}$ alkyl group, cycloalkyl group, or optionally substituted 6-membered non-aromatic heterocycle,

$R^{u2}$ represents an optionally halogenated $C_{1-2}$ alkyl group, and

$n_u$ represents an integer of 1 to 3.]; and

(6)

3-(3,6-dimethyl-4-oxo-4,5,6,7-tetrahydro-1H-indazol-1-yl)propyl 4-methylbenzenesulfonate;

3-(5-bromo-3,6-dimethyl-4,7-dioxo-4,7-dihydro-1H-indazol-1-yl)propyl 4-methylbenzenesulfonate;

3-(5-amino-3,6-dimethyl-4,7-dioxo-4,7-dihydro-1H-indazol-1-yl)propyl 4-methylbenzenesulfonate;

2-bromo-4-[(3-methyl-4-oxo-4,5,6,7-tetrahydro-1H-indazol-1-yl)methyl]benzonitrile;

[1-(4-fluorobenzyl)-1,4,5,6,7,8-hexahydrocyclohepta[c]pyrazol-3-yl]methanol;

[1-(4-methoxybenzyl)-1,4,5,6,7,8-hexahydrocyclohepta[c]pyrazol-3-yl]methanol;

methyl 4-ethyl-5-methyl-2-({2-[3-(trifluoromethyl)-5,6-dihydrocyclopenta[c]pyrazol-1(4H)-yl]butanoyl}amino)thiophene-3-carboxylate;

methyl 5-ethyl-2-({2-[3-(trifluoromethyl)-5,6-dihydrocyclopenta[c]pyrazol-1(4H)-yl]butanoyl}amino)thiophene-3-carboxylate; and

ethyl 4-methyl-2-({2-[3-(trifluoromethyl)-5,6-dihydrocyclopenta[c]pyrazol-1(4H)-yl]butanoyl}amino)-1,3-thiazole-5-carboxylate.

The following compounds may also lie outside the scope of the compounds of the present invention.

(1) the compounds in which $R^1$ is -CO-NHR$^t$ (wherein $R^t$ is an optionally substituted $C_4$ or higher hydrocarbon group.);
(2) compounds represented by the formula

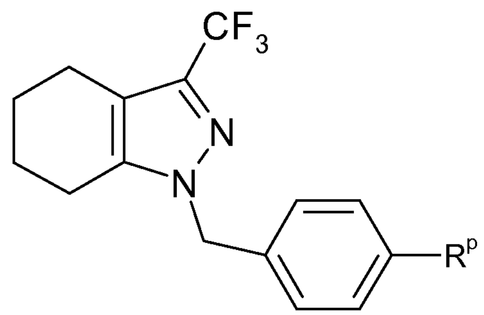

[In the formula, Rp represents a substituent.];
(3) compounds represented by the formula

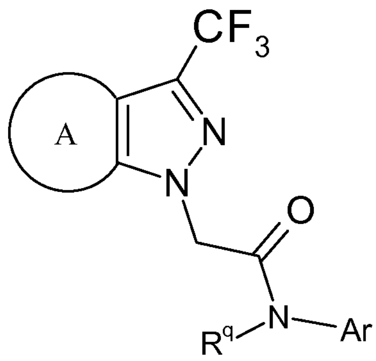

[In the formula,
ring A represents

(i) a non-aromatic carbon ring of 4-8 carbon atoms,
optionally substituted with one or more substituents selected from

(1) halogen atoms,
(2) cyano group,
(3) alkyl groups optionally substituted with substituent(s) selected from halogens, cyano, hydroxy, alkoxy groups, cycloalkyl, optionally substituted amino, mercapto, alkylsulfinyl, alkylsulphenyl, alkylsulfonyl, mono- or di-alkyl-sulfamoyl, alkanoyloxy groups, alkanoyl, carbamoyl, and mono- or di-alkylcarbamoyl,
(4) optionally substituted cycloalkyl groups,
(5) optionally substituted amino group,
(6) groups represented by the formulas -OR$^{y1}$, -SR$^{y1}$, -CO-R$^{y1}$, -CS-R$^{y1}$, -SO-R$^{y1}$, -SO$_2$-R$^{y1}$, or -NR$^{y1}$R$^{y2}$ (where R$^{y1}$ and R$^{y2}$ each independently represent hydrogen or a substituent),
(7) oxo, and
(8) optionally substituted non-aromatic heterocyclic groups;

Rq represents hydrogen or a substituent, and
the other symbols are synonymous with the above.]1
(4) compounds represented by the formula

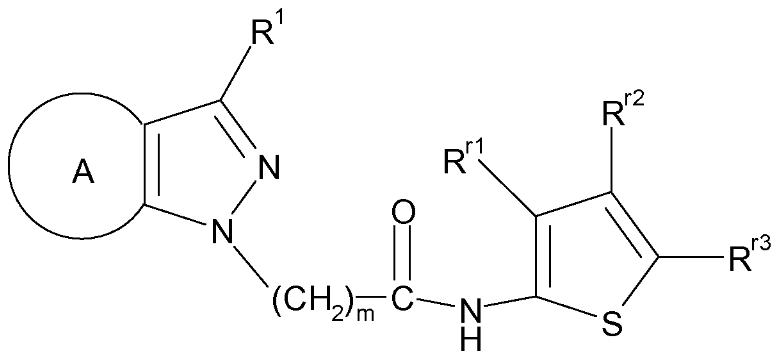

[In the formula,
$R^1$ represents trifluoromethyl,
$R^{r1}$ represents hydroxymethyl, carboxyl, or optionally substituted carbamoyl,
$R^{r2}$ and $R^{r3}$ each independently represent hydrogen, $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,
ring A represents cyclohexene, and
m represents the integer 2.]; and
(5)
1-(2-aminobenzyl)-6-chloro-3,5-dimethyl-1,5-dihydro-4H-pyrazolo[4,3-c]pyridin-4-one;
1-(3,4-dimethoxybenzyl)-3,4-dimethylpyrano[2,3-c]pyrazol-6(1H)-one;
3,4-dimethyl-1-(4-nitrobenzyl)pyrano[2,3-c]pyrazol-6(1H)-one;
3,4-dimethyl-1-(3-nitrobenzyl)pyrano[2,3-c]pyrazol-6(1H)-one;
3,4-dimethyl-1-(2-nitrobenzyl)pyrano[2,3-c]pyrazol-6(1H)-one;
1-(4-methoxybenzyl)-3,4-dimethylpyrano[2,3-c]pyrazol-6(1H)-one;
1-(3-methoxybenzyl)-3,4-dimethylpyrano[2,3-c]pyrazol-6(1H)-one;
1-(2-methoxybenzyl)-3,4-dimethylpyrano[2,3-c]pyrazol-6(1H)-one;
1-(4-chlorobenzyl)-3,4-dimethylpyrano [2,3-c]pyrazol-6(1H)-one;
1-(3-chlorobenzyl)-3,4-dimethylpyrano[2,3-c]pyrazol-6(1H)-one;
1-(2-chlorobenzyl)-3,4-dimethylpyrano [2,3-c]pyrazol-6(1H)-one;
3-(3,6-dimethyl-4-oxo-4,5,6,7-tetrahydro-1H-indazol-1-yl)propyl4-methylbenzenesulfonate;
3-(5-bromo-3,6-dimethyl-4,7-dioxo-4,7-dihydro-1H-indazol-1-yl)propyl 4-methylbenzenesulfonate;
3-(5-amino-3,6-dimethyl-4,7-dioxo-4,7-dihydro-1H-indazol-1-yl)propyl 4-methylbenzenesulfonate;
2-bromo-4-[(3-methyl-4-oxo-4,5,6,7-tetrahydro-1H-indazol-1-yl)methyl]benzonitrile;
ethyl 7-oxo-1-[(5-phenyl-1,3-oxazol-2-yl)methyl]-4,5,6,7-tetrahydro-1H-indazole-3-carboxylate;
ethyl 1-[(5-ethyl-1,3-oxazol-2-yl)methyl]-7-oxo-4,5,6,7-tetrahydro-1H-indazole-3-carboxylate; [1-(4-fluorobenzyl)-1,4,5,6,7,8-hexahydrocyclopenta[c]pyrazol-3-yl]methanol;
[1-(4-methoxybenzyl)-1,4,5,6,7,8-hexahydrocyclopenta[c]pyrazol-3-yl]methanol;
{[1-(4-chlorobenzyl)-4,5,6,7-tetrahydro-1H-indazol-3-yl]oxy}acetonitrile;
1-(4-chlorobenzyl)-3-(1H-tetrazol-5-yhnethoxy)-4,5,6,7-tetrahydro-1H-indazole;
N-[3-(2-furanyl)-1-methylpropyl]-N-methyl-[3-(trifluoromethyl)-5,6-dihydrocyclopenta[c]pyrazol-14H)-yl]acetamide;
N-[3-(2-furanyl)-1-methylpropyl]-[3-(trifluoromethyl)-5,6-dihydrocyclopenta[c]pyrazol-1(4H)-yl]acetamide;
N-(2-thienylmethyl)-[3-(trifluoromethyl)-4,5,6,7-tetrahydro-1H-indazol-1-yl]acetamide;
N-[2-(5-methoxy-2-methyl-1H-indol-3-yl)ethyl]-[3-(trifluoromethyl)-4,5,6,7-tetrahydro-1H-indazol-1-yl]acetamide;
N-(2-phenylethyl)-[3-(trifluoromethyl)-5,6-dihydrocyclopenta[c]pyrazol-1(4H)-yl]acetamide;
N-(1-phenylethyl)-[3-(trifluoromethyl)-5,6-dihydrocyclopenta[c]pyrazol-1(4H)-yl]acetamide;
N-[(4-chlorophenyl)methyl]-[3-(trifluoromethyl)-5,6-dihydrocyclopenta[c]pyrazol-1(4H)-yl]acetamide;
N-methyl-N-(phenylmethyl)-[3-(trifluoromethyl)-4,5,6,7-tetrahydro-1H-indazol-1-yl]acetamide;
N-(2-thienylmethyl)-[3-(trifluoromethyl)- 5,6-dihydrocyclopenta[c]pyrazol-1(4H)-yl]acetamide;
N-(2-furanylmethyl)- [3-(trifluoromethyl)- 4,5,6,7-tetrahydro-1H-indazol-1-yl]acetamide;
N-[3-[3-(trifluoromethyl)-5,6-dihydrocyclopenta[c]pyrazol-2(4H)-yl]propyl]-[3-(trifluoromethyl)-5,6,7,8-tetrahydrocyclohepta[c]pyrazol-1 (4H)-yl] acetamide;
N-phenyl-N-(phenylmethyl)-[3-(trifluoromethyl)-4,5,6,7-tetrahydro-1H-indazol-1-yl]acetamide;
N-methyl-N-phenyl-[3-(trifluoromethyl)-4,5,6,7-tetrahydro-1H-indazol-1-yl]acetamide;

N-methyl-N-(phenylmethyl)-[3-(trifluoromethyl)-5,6-dihydrocyclopenta[c]pyrazol-[(4H)-yl]acetamide;
N-(2-furanylmethyl)-[3-(trifluoromethyl)-5,6-dihydrocyclopentapyrazol-[(4H)-yl]acetamide;
N-(phenylmethyl)-[3-(trifluoromethyl)-4,5,6,7-tetrahydro-1H-indazol-1-yl]acetamide;
N,N-bis(phenylmethyl)-[3-(trifluoromethyl)-4,5,6,7-tetrahydro-1H-indazol-1-yl]acetamide;
methyl4-ethyl-5-methyl-2-({2-[3-(trifluoromethyl)-5,6-dihydrocyclopenta[c]pyrazol-1(4H)-yl]butanoyl}amino)thiophene-3-carboxylate;
N-(1-phenylethyl)-[3-(trifluoromethyl)-4,5,6,7-tetrahydro-1H-indazol-1-yl]acetamide;
N-(3-pyridinylmethyl)-[3-(trifluoromethyl)-4,5,6,7-tetrahydro-1H-indazol-1-yl]acetamide;
methyl 5-ethyl-2-({2-[3-(trifluoromethyl)-5,6-dihydrocyclopenta[c]pyrazol-1(4H)-yl]butanoyl}amino)thiophene-3-carboxylate;
ethyl 4-methyl-2-({2-[3-(trifluoromethyl)-5,6-dihydrocyclopenta[c]pyrazol-1(4H)-yl]butanoyl} amino)-1,3-thiazole-5-carboxylate;
N-[(4-methoxyphenyl)methyl]-[3-(trifluoromethyl)-5,6-dihydrocyclopenta[c]pyrazol-1(4H)-yl]acetamide; N-[(4-fluorophenyl)methyl]-[3-(trifluoromethyl)-5,6-dihydrocyclopenta[c]pyrazol-1(4H)-yl]acetamide; N-[(4-chlorophenyl)methyl]-[3-(trifluoromethyl)-4,5,6,7-tetrahydro-1H-indazol-1-yl]acetamide; N-[(4-fluorophenyl)methyl]-[3-(trifluoromethyl)-4,5,6,7-tetrahydro-1H-indazol-1-yl]acetamide; N-phenyl-N-(phenylmethyl)-[3-(trifluoromethyl)-5,6-dihydrocyclopenta[c]pyrazol-1(4H)-yl]acetamide; N-(phenylmethyl)-[3-(trifluoromethyl)-5,6-dihydrocyclopenta[c]pyrazol-1(4H)-yl]acetamide; N-[(4-methoxyphenyl)methyl]-[3-(trifluoromethyl)-4,5,6,7-tetrahydro-1H-indazol-1-yl]acetamide; and N-(2-phenylethyl)-[3-(trifluoromethyl)-4,5,6,7-tetrahydro-1H-indazol-1-yl]acetamide
are excluded.

Examples of particularly desirable compounds of the invention include compounds selected from
1-{[[4-(pyrrolidin-1-ylcarbonyl)-1,3-thiazol-2-yl]methyl}-3-(trifluoromethyl)-4,5,6,7-tetrahydro-1H-indazole,
2-({[3-(trifluoromethyl)-4,5,6,7-tetrahydro-1H-pyrazolo[3,4-c]pyridin-1-yl]acetyl}amino)-4,5,6,7-tetrahydro-1-benzothiophene-3-carboxamide,
2-({[3-(trifluoromethyl)-4,5,6,7-tetrahydro-1H-pyrazolo[4,3-c]pyridin-1-yl]acetyl]amino)-4,5,6,7-tetrahydro-1-benzothiophene-3-carboxamide,
1,2-dimethyl-6-(methylsulfanyl)-N-{2-[3-(trifluoromethyl)-4,5,6,7-tetrahydro-1H-indazol-1-yl]ethyl]-1H-thieno[3,4-d]imidazole-4-carboxamide,
5-methyl-7-(trifluoromethyl)-3-(5-{[3-(trifluoromethyl)-4,5,6,7-tetrahydro-1H-indazol-1-yl]methyl]-1,2,4-oxadiazol-3-yl) pyrazolo[1,5-a]pyrimidine,
N-(5-chloro-2-methoxyphenyl)-2-[3-(trifluoromethyl)-4,5,6,7-tetrahydro-1H-pyrazolo[3,4-c]pyridin-1-yl]acetamide,
4-hydroxy-3-(methylsulfanyl)-N-{2-[3-(trifluoromethyl)-4,5,6,7-tetrahydro-1H-indazol-1-yl]ethyl]-4,5,6,7-tetrahydro-2-benzothiophene-1-carboxamide,
2-[5-acetyl-3-(trifluoromethyl)-4,5,6,7-tetrahydro-1H-pyrazolo[4,3-c]pyridin-1-yl]-N-(5-chloro-2-methoxyphenyl)acetamide,
5,7-dimethyl-3-(5-{[3-(trifluoromethyl)-4,5,6,7-tetrahydro-1H-indazol-1-yl]methyl]-1,2,4-oxadiazol-3-yl)pyrazolo[1,5-a] pyrimidine,
1-[(3-pyrazin-2-yl-1,2,4-oxadiazol-5-yl)methyl]-3-(trifluoromethyl)-4,5,6,7-tetrahydro-1H-indazole, 5-methyl-7-(trifluoromethyl)-3-(5-{[3-(trifluoromethyl)-5,6-dihydrocyclopenta[c]pyrazol-1(4H)-yl]methyl}-1,2,4-oxadiazol-3-yl)pyrazolo[1,5-a]pyrimidine,
1-{2-[(5-chloro-2-methoxyphenyl)amino]-2-oxoethyl}-N-methyl-3-(trifluoromethyl)-1,4,6,7-tetrahydro-5H-pyrazolo[4,3-c]pyridine-5-carboxamide,
1-[(3-thiophen-2-yl-1,2,4-oxadiazol-5-yl)methyl]-3-(trifluoromethyl)-1,4,6,7-tetrahydropyrano[4,3-c]pyrazole, and
1-[(5-thiophen-2-yl-1,3,4-oxadiazol-2-yl)methyl]-3-(trifluoromethyl)-4,5,6,7-tetrahydro-1H-indazole, or a salt thereof.

Examples of salts for when compound (I) is in the form of a salt include salts with inorganic bases, salts with organic bases, salts with inorganic acids, salts with organic acids, and salts with basic or acidic amino acids.
Preferable examples of salts with inorganic bases include salts with alkali metals such as sodium salts and potassium salts; salts with alkaline earth metals such as calcium salts and magnesium salts; aluminum salts; and ammonium salts.
Preferable examples of salts with organic bases include salts with trimethylamine, triethylamine, pyridine, picoline, ethanolamine, diethanolamine, triethanolamine, dicyclohexylamine, and N,N-dibenzylethylenediamine.
Preferable examples of salts with inorganic acids include salts with hydrochloric acid, hydrobromic acid, nitric acid, sulfuric acid, and phosphoric acid.
Preferable examples of salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.
Preferable examples of salts with basic amino acids include salts with arginine, lysine, and ornithine. Preferable examples of salts with acidic amino acids include salts with aspartic acid and glutamic acid.

A prodrug of compound (I) may be used in the same manner as compound (I). A prodrug of compound (I) refers to a compound that is converted to compound (I) by a reaction involving an enzyme, gastric acid, or the like under the physiological conditions in the body; that is, a compound that is converted to compound (I) by enzymatic oxidation, reduction, hydrolysis, or the like, or a compound that is converted to compound (I) by hydrolysis or the like involving gastric acid or the like.

Examples of prodrugs of compound (I) include compounds in which an amino group of compound (I) is acylated, alkylated, or phosphorylated (such as compounds in which an amino group of compound (I) is eicosanoylated, alanylated, pentylaminocarbonylated, (5-methyl-2-oxo-1,3-dioxolen-4-yl) methoxycarbonylated, tetrahydrofuranylated, pyrrolidylmethylated, pivaloyloxymethylated, or tert-butylated); compounds in which a hydroxyl group of compound (I) is acylated, alkylated, phosphorylated, or borated (such as compounds in which a hydroxyl group of compound (I) is acetylated, palmitoylated, propanoylated, pivaloylated, succinylated, fumarylated, alanylated, or dimethylaminomethylcarbonylated); and compounds in which a carboxy group of compound (I) is esterified or amidated (such as compounds in which a carboxy group of compound (I) is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, or methylamidated). These compounds can be produced from compound (I) by a well known method. A prodrug of compound (I) may also be a compound that is converted to compound (I) under physiological conditions as described in "Iyakuhin No Kaihatsu (Development of Pharmaceutical Products)", Vol. 7, Molecular Design, pp. 163-198, Hirokawa Shoten (1990).

[Production Method]

Methods for producing the compounds of the invention will be described below.
The compounds of the invention can be produced, for example, by the following methods or methods based thereon.
The compounds in the reaction formulas may be in the form of salts. Examples of such salts include the same ones noted above for salts of compound (I).

<Production of Compound (I)>

Production Method A

Compound (I) can be produced, for example, by a reaction between
Compound (II)

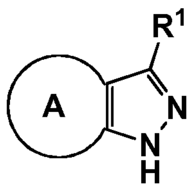

(II)

(wherein the symbols are synonymous with the above) and
Compound (III)

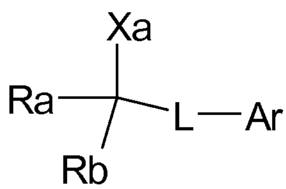

(III)

(wherein Xa represents a leaving group, and the other symbols are synonymous with the above).

Examples of the "leaving group" represented by Xa include halogen atoms such as chlorine, bromine, and iodine, $C_{6-14}$ arylsulfonyloxy groups such as p-toluenesulfonyloxy group, $C_{1-6}$ alkylsulfonyloxy groups such as methanesulfonyloxy group, and preferably a halogen atom such as chlorine, bromine, or iodine.

The reaction between compound (II) and compound (III) is preferably carried out in a solvent, examples of which include aromatic hydrocarbons such as toluene, ethers such as 1,4-dioxane or tetrahydrofuran, and amides such as N,N-dimethyl formamide, in the presence of a base such as potassium tert-butoxide, sodium hydride, potassium carbonate, or cesium carbonate.
The reaction is preferably carried out by dissolving compound (II) in a solvent such as N,N-dimethyl formamide, adding potassium tert-butoxide, and then adding compound (III).
In the reaction, compound (III) is ordinarily used in an amount of about 1 to about 5 mol per mol starting compound, and the amount of the base is about 0.1 to about 100 equivalents, and preferably 1 to 5 equivalents. The reaction temperature is ordinarily 0°C to 200°C, and preferably 0°C to 100°C. The reaction time is about 0.1 to about 100 hours, and preferably about 0.5 to about 50 hours.

Production Method B

When compound (I) is compound (Ia)

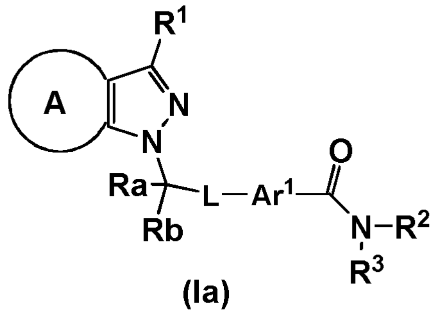

(Ia)

(In the formula, $Ar^1$ represents an optionally substituted aryl group or optionally substituted aromatic heterocyclic group, $R^2$ and $R^3$ each represent a hydrogen atom, optionally substituted $C_{1-6}$ alkyl group, optionally substituted $C_{6-14}$ aryl group, optionally substituted tricyclic bridged group, or optionally substituted 5- to 10- membered heterocyclic group, or $R^2$ and $R^3$ may together form a ring structure. The other symbols are synonymous with the above.), compound (I) can be produced, for example, by

1) a method in which Compound (Ib)

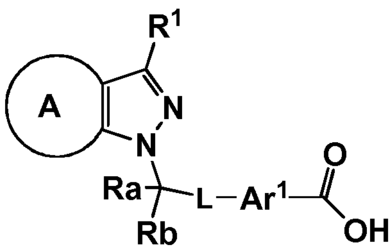

(Ib)

(The symbols in the formula are synonymous with the above) and Compound (IV)

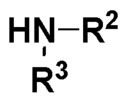

(IV)

(In the formula, the symbols are synonymous with the above.) are condensed with a well known dehydrocondensation agent;
2) a method in which the carboxyl group of compound (Ib) is activated by a well known activation method, and

compound (IV) is then reacted; or

3) a method in which a derivative of compound (Ib) and compound (IV) are reacted; and the like.

Method 1)

Compound (Ia) can be produced by condensing compound (Ib) and compound (IV) with a well known dehydrocondensation agent.
Examples of dehydrocondensation agents used in this reaction include N,N'-dicyclohexylcarbodiimide, 1-ethyl-3-(3-dimethylaminopropyl)carbodiimide (WSC) or hydrochloride thereof, N,N'-carbonyldiimidazole,
1H-benzotriazol-1-yloxytris(dimethylamino)phosphonium hexafluorophosphate, O-(7-azabenzotriazol-1-yl)-1,1,3,3-tetramethyluronium hexafluorophosphate (HATU), 2-chloro-1,3-dimethylimidazolium chloride, and bromotripyrrolidinophosphonium hexafluorophosphate.
The reaction may be carried out as needed, for example, in the presence of 1-hydroxy-1H-benzotriazole (HOBt); or a base such as N,N-diisopropylethylamine, N-methylmorpholine, triethylamine, or 4-(N,N-dimethylamino)pyridine.
The reaction is preferably carried out in a well known solvent, examples of which include amides such as N,N-dimethyl formamide, N,N-dimethyl acetamide, and N-methyl pyrrolidone; halogenated hydrocarbons such as dichloromethane; esters such as ethyl acetate; hydrocarbons such as cyclohexane and n-hexane; aromatic hydrocarbons such as toluene; ethers such as tetrahydrofuran, diethyl ether, dioxane, and 1,2-dimethoxyethane; and nitriles such as acetonitrile.
The reaction is preferably carried out by dissolving compound (Ib) and compound (IV) in a solvent such as N,N-dimethyl formamide, and adding O-(7-azabenzotriazol-1-yl)-1,1,3,3-tetramethyluronium hexafluorophosphate (HATU) as the dehydrocondensation agent in the presence of N,N-diisopropylethylamine.
In the reaction, compound (IV) is ordinarily used in an amount of about 1 to about 5 mol per mol starting compound, and the amount of the condensation agent is about 1 to about 100 equivalents, and preferably 1 to 5 equivalents. The reaction temperature is ordinarily 0°C to 100°C, and preferably 0°C to 60°C. The reaction time is about 0.1 to about 100 hours, and preferably about 0.5 to about 50 hours.

Method 2)

Compound (Ia) can also be produced by activating the carboxyl group of compound (Ib) by a well known activation method, and then reacting compound (IV).
A common method can be used as the method for activating the carboxyl group of compound (IV), such as methods in which an acid anhydride is produced using chloroformic acid ester, pivaloyl chloride, 2,4,6-trichlorobenzoyl chloride, or the like;
methods in which an acid halide is produced using thionyl chloride, oxalyl chloride, or the like; and methods in which an ester of 1-hydroxybenzotriazole, pentafluorophenol, or the like is produced using a dehydrocondensation agent.
Typical examples include methods for producing acid halides. Examples of acid halides include Compound (Ic)

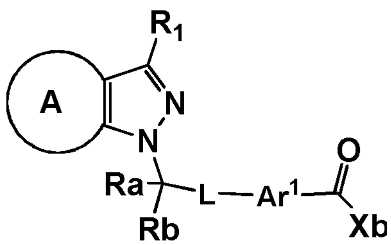

(Ic)

(In the formula, Xb represents a halogen atom, and the other symbols are synonymous with the above). Such acid halides can be produced, for example, by treating compound (Ib) with a halogenating agent such as thionyl chloride or oxalyl chloride. N,N-dimethyl formamide may be added, for example, as an additive in such cases.
The reaction is preferably carried out in, or without, a well known solvent, examples of which include halogenated hydrocarbons such as dichloromethane, ethers such as tetrahydrofuran and diethyl ether, and aromatic hydrocarbons such as toluene.
The reaction is preferably carried out by adding oxalyl chloride to compound (Ib) in the presence of N,N-dimethyl formamide in tetrahydrofuran.
In the reaction, the halogenating agent is ordinarily used in an amount of about 1 to about 100 equivalents, and preferably 1 to 5 equivalents, per mol starting compound. The reaction temperature is ordinarily -78°C to 100°C, and preferably 0°C to 100°C. The reaction time is about 0.1 to about 100 hours, and preferably about 0.5 to about 50 hours. Compound (Ia) is obtained by activating the carboxyl group of compound (Ib) and then reacting compound (IV). The reaction is preferably carried out in a well known solvent (examples of which include halogenated hydrocarbons such as dichloromethane; ethers such as tetrahydrofuran and diethyl ether; and amides such as N,N-dimethyl formamide, N,N-dimethyl acetamide, and N-methyl pyrrolidone) in the presence of a base such as triethylamine or pyridine.
The reaction is preferably carried out by activating the carboxyl group of compound (Ib) to obtain compound (Ic), and then adding compound (IV) in the presence of a base such as triethylamine in tetrahydrofuran, for example.
The reaction is carried out ordinarily at a reaction temperature of -78°C to 150°C, and preferably 0°C to 100°C, using an acid halide and compound (IV) ordinarily in an amount of about 1 to about 5 mol per mol starting compound. The reaction time is about 0.1 to about 100 hours, and preferably about 0.5 to about 50 hours.

Method 3)

Compound (Ia) can also be produced by a reaction between a derivative of compound (Ib) and compound (IV). Examples of derivatives of compound (Ib) include optionally substituted $C_{1-6}$ alkyl (such as methyl, ethyl, n-propyl, i-propyl, n-butyl, and tert-butyl) esters, optionally substituted phenyl esters, optionally substituted silyl esters, optionally substituted mono-$C_{1-6}$ alkyl amides, and optionally substituted di-$C_{1-6}$ alkyl amides. Examples of substituents for these include halogen atoms, nitro group, hydroxy group, and $C_{1-6}$ alkoxy groups. The number of substituents is about 1 to 3. The reaction is carried out, for example, by a method in which a derivative of compound (Ib), preferably a lower alkyl ester (especially a methyl ester or ethyl ester) of compound (Ib), and compound (IV) are both present and are heated. The reaction is carried out ordinarily at a reaction temperature of 0°C to 200°C, and preferably 40°C to 200°C, using compound (IV) ordinarily in an amount of about 1 to about 5 mol per mol starting compound. The reaction time is about 0.1 to about 100 hours, and preferably about 0.5 to about 50 hours.

<Production of Compound (Ib)>

Compound (Ib) used in the production of compound (Ia) can be produced, for example, by method 1) or method 2) for hydrolyzing
Compound (Id)

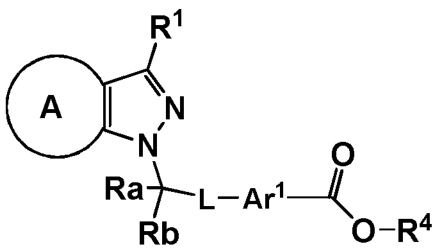

(Id)

(In the formula, $R^4$ represents a $C_{1-6}$ alkyl group, and the other symbols are synonymous with the above.).

Method 1)

The reaction is generally carried out using a method for hydrolyzing the ester under basic conditions, such as by treatment with an alkali such as lithium hydroxide, sodium hydroxide, or potassium hydroxide. The reaction is preferably carried out by dissolving compound (Id) in an alcohol such as methanol or ethanol, or a water-soluble solvent such as tetrahydrofuran or dioxane, or a solvent mixture thereof, and treating the mixture with an alkaline aqueous solution such as sodium hydroxide aqueous solution or lithium hydroxide aqueous solution.
In the reaction, the alkaline aqueous solution is ordinarily used in an amount of about 1 to about 10 equivalents per mol starting compound. The reaction temperature is ordinarily 0°C to 100°C, and preferably 20°C to 100°C. The reaction time is about 0.1 to about 100 hours, and preferably about 0.5 to about 50 hours.

Method 2)

Compound (Ib) can also be produced by a method for hydrolyzing an ester of compound (Id) under acidic conditions. The reaction can be carried out, for example, by treatment with an acid such as hydrochloric acid, sulfuric acid, or nitric acid. The reaction is preferably carried out by dissolving compound (Id) in an alcohol such as methanol or ethanol, or a water-soluble solvent such as tetrahydrofuran or dioxane, or a solvent mixture thereof, and treating the mixture with an aqueous solution of an acid such as hydrochloride acid or sulfuric acid.
In the reaction, the acid aqueous solution is ordinarily used in an amount of about 1 to about 10 equivalents per mol starting compound. The reaction temperature is ordinarily 0°C to 100°C, and preferably 20°C to 100°C. The reaction time is about 0.1 to about 100 hours, and preferably about 0.5 to about 50 hours.

<Production of Compound (Id)>

Compound (Id) used in the production of compound (Ib) can be produced, for example, by a reaction between Compound (II)

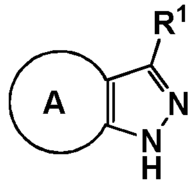

(II)

(In the formula, the symbols are synonymous with the above.) and Compound (IIIa)

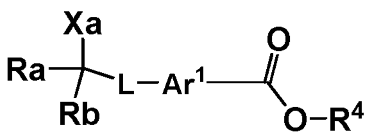

(IIIa)

(In the formula, the symbols are synonymous with the above.).
The reaction between compound (II) and compound (IIIa) is preferably carried out in a solvent, examples of which include aromatic hydrocarbons such as toluene, ethers such as 1,4-dioxane or tetrahydrofuran, and amides such as N,N-dimethyl formamide, in the presence of a base such as potassium tert-butoxide, sodium hydride, potassium carbonate, and cesium carbonate.
The reaction is preferably carried out by dissolving compound (II) in a solvent such as N,N-dimethyl formamide, adding potassium tert-butoxide, and then adding compound (IIIa).
In the reaction, compound (IIIa) is ordinarily used in an amount of about 1 to about 5 mol per mol starting compound, and the amount of the base is about 0.1 to about 100 equivalents, and preferably 1 to 5 equivalents. The reaction temperature is ordinarily 0°C to 200°C, and preferably 0°C to 100°C. The reaction time is about 0.1 to about 100 hours, and preferably about 0.5 to about 50 hours.
When compound (I) is
Compound (Ie)

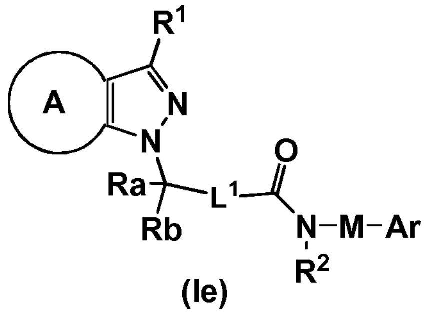

(Ie)

(In the formula, $L^1$ is a bond or an optionally substituted spacer in which the number of atoms in the main chain is 1 to 6, and M is a bond or an optionally substituted spacer in which the number of atoms in the optionally substituted main chain is 1 to 4. However, the sum of the number of atoms in the main chain of L1 and the main chain of M is 0 to 6. The other symbols are synonymous with the above.), compound (I) can be produced, for example, by

1) a method in which Compound (V)

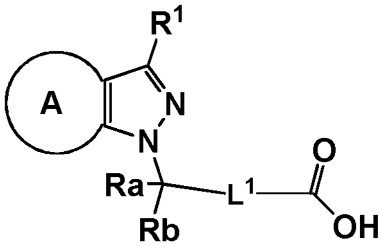

(V)

(In the formula, the symbols are synonymous with the above.) and Compound (IV)

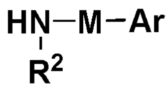

(VI)

(In the formula, the symbols are synonymous with the above.) are condensed with a well known dehydrocondensation agent;
2) a method in which the carboxyl group of compound (V) is activated by a well known activation method, and compound (VI) is then reacted; or
3) a method in which a derivative of compound (V) and compound (VI) are reacted; and the like.

Method 1)

Compound (IE) can be produced by condensing compound (V) and compound (VI) with a well known dehydrocondensation agent.
Examples of dehydrocondensation agents used in this reaction include N,N'-dicyclohexylcarbodiimide, 1-ethyl-3-(3-dimethylaminopropyl)carbodiimide (WSC) or hydrochloride thereof, N,N'-carbonyldiimidazole, 1H-benzotriazol-1-yloxytris(dimethylamino)phosphonium hexafluorophosphate, O-(7-azabenzotriazol-1-yl)-1,1,3,3-tetramethyluronium hexafluorophosphate (HATU), 2-chloro-1,3-dimethylimidazolium chloride, and bromotripyrrolidinophosphonium hexafluorophosphate.
The reaction may be carried out as needed, for example, in the presence of 1-hydroxy-1H-benzotriazole (HOBt); or a base such as N,N-diisopropylethylamine, N-methylmorpholine, triethylamine, and 4-(N,N-dimethylamino)pyridine.
The reaction is preferably carried out in a well-known solvent, examples of which include amides such as N,N-dimethyl formamide, N,N-dimethyl acetamide, and N-methyl pyrrolidone; halogenated hydrocarbons such as dichloromethane;

esters such as ethyl acetate; hydrocarbons such as cyclohexane and n-hexane; aromatic hydrocarbons such as toluene; ethers such as tetrahydrofuran, diethyl ether, dioxane, and 1,2-dimethoxyethane; or nitriles such as acetonitrile.
The reaction is preferably carried out by dissolving compound (V) and compound (VI) in a solvent such as N,N-dimethyl formamide, and adding O-(7-azabenzotriazol-1-yl)-1,1,3,3-tetramethyluronium hexafluorophosphate (HATU) as the dehydrocondensation agent in the presence of N,N-diisopropylethylamine.
In the reaction, compound (V) is ordinarily used in an amount of about 1 to about 5 mol per mol starting compound, and the amount of the condensation agent is about 1 to about 100 equivalents, and preferably 1 to 5 equivalents. The reaction temperature is ordinarily 0°C to 100°C, and preferably 0°C to 60°C. The reaction time is about 0.1 to about 100 hours, and preferably about 0.5 to about 50 hours.

Method 2)

Compound (Ie) can also be produced by activating the carboxyl group of compound (V) by a well known activation method, and then reacting compound (VI).
A common method can be used as the method for activating the carboxyl group of compound (V), such as methods in which an acid anhydride is produced using chloroformic acid ester, pivaloyl chloride, 2,4,6-trichlorobenzoyl chloride, or the like;
methods in which an acid halide is produced using thionyl chloride, oxalyl chloride, or the like; and methods in which an ester of 1-hydroxybenzotriazole, pentafluorophenol, or the like is produced using a dehydrocondensation agent.
Typical examples include methods for producing acid halides, and examples of acid halides include Compound (VII)

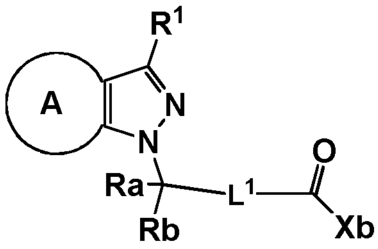

(VII)

(In the formula, the symbols are synonymous with the above), which can be produced, for example, by treating compound (V) with a halogenating agent such as thionyl chloride or oxalyl chloride; examples of additives include N,N-dimethyl formamide.
The reaction is preferably carried out in, or without, a well known solvent, examples of which include halogenated hydrocarbons such as dichloromethane, ethers such as tetrahydrofuran and diethyl ether, and aromatic hydrocarbons such as toluene.
The reaction is preferably carried out by adding oxalyl chloride to compound (V) in the presence of N,N-dimethyl formamide in tetrahydrofuran.
In the reaction, the halogenating agent is ordinarily used in an amount of about 1 to about 100 equivalents, and preferably 1 to 5 equivalents, per mol starting compound. The reaction temperature is ordinarily -78°C to 100°C, and preferably 0°C to 100°C. The reaction time is about 0.1 to about 100 hours, and preferably about 0.5 to about 50 hours.
Compound (Ie) is obtained by activating the carboxyl group of compound (V) and then reacting compound (VI). The reaction is preferably carried out in a well known solvent, examples of which include halogenated hydrocarbons such as dichloromethane, ethers such as tetrahydrofuran and diethyl ether, and amides such as N,N-dimethyl formamide, N,N-dimethyl acetamide, and N-methyl pyrrolidone.
The reaction is preferably carried out by activating the carboxyl group of compound (V) to obtain compound (VII), and then adding compound (Ie) in the presence of a base such as triethylamine in tetrahydrofuran, for example.
The reaction is carried out ordinarily at a reaction temperature of -78°C to 150°C, and preferably 0°C to 100°C, using an acid halide and compound (VI) ordinarily in an amount of about 1 to about 5 mol per mol starting compound. The reaction time is about 0.1 to about 100 hours, and preferably about 0.5 to about 50 hours.

Method 3)

Compound (Ie) can also be produced by a reaction between a derivative of compound (V) and compound (VI).

Examples of derivatives of compound (V) include optionally substituted $C_{1-6}$ alkyl (such as methyl, ethyl, n-propyl, i-propyl, n-butyl, and tert-butyl) esters, optionally substituted phenyl esters, optionally substituted silyl esters, optionally substituted mono-$C_{1-6}$ alkyl amides, and optionally substituted di-$C_{1-6}$ alkyl amides. Examples of substituents for these include halogen atoms, nitro group, hydroxy group, and $C_{1-6}$ alkoxy groups. The number of substituents is about 1 to 3.
The reaction is carried out, for example, by a method in which a derivative of compound (Ie), preferably a lower alkyl ester (especially a methyl ester or ethyl ester) of compound (Ie), and compound (VI) are both present and are heated (preferably heated to between 40°C and 200°C).
The reaction is carried out ordinarily at a reaction temperature of 0°C to 200°C, and preferably 40°C to 200°C, using compound (VI) ordinarily in an amount of about 1 to about 5 mol per mol starting compound. The reaction time is about 0.1 to about 100 hours, and preferably about 0.5 to about 50 hours.

<Production of Compound (V)>

Compound (V) used in the production of compound (Ie) can be produced, for example, by method 1) or 2) for hydrolyzing
Compound (VIII)

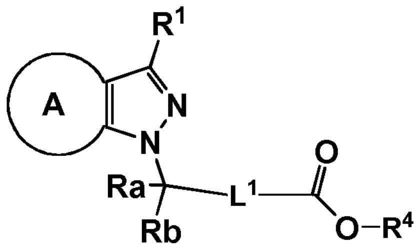

(VIII)

(In the formula, the symbols are synonymous with the above).

Method 1)

The reaction is generally carried out using a method for hydrolyzing the ester under basic conditions, such as by treatment with an alkali such as lithium hydroxide, sodium hydroxide, or potassium hydroxide. The reaction is preferably carried out by dissolving compound (VIII) in an alcohol such as methanol or ethanol, or a water-soluble solvent such as tetrahydrofuran or dioxane, or a solvent mixture thereof, and treating the mixture with an alkaline aqueous solution such as sodium hydroxide aqueous solution or lithium hydroxide aqueous solution.
In the reaction, the alkaline aqueous solution is ordinarily used in an amount of about 1 to about 10 equivalents per mol starting compound. The reaction temperature is ordinarily 0°C to 100°C, and preferably 20°C to 100°C. The reaction time is about 0.1 to about 100 hours, and preferably about 0.5 to about 50 hours.

Method 2)

Compound (V) used in the production of compound (Ie) can also be produced by a method for hydrolyzing the ester of compound (VIII) under acidic conditions. The reaction can be carried out, for example, by treatment with an acid such as hydrochloric acid, sulfuric acid, or nitric acid. The reaction is preferably carried out by dissolving compound (VIII) in an alcohol such as methanol or ethanol, or a water-soluble solvent such as tetrahydrofuran or dioxane, or a solvent mixture thereof, and treating the mixture with an aqueous solution of an acid such as hydrochloride acid, sulfuric acid, or nitric acid.
In the reaction, the acid aqueous solution is ordinarily used in an amount of about 1 to about 10 equivalents per mol starting compound. The reaction temperature is ordinarily 0°C to 100°C, and preferably 20°C to 100°C. The reaction time is about 0.1 to about 100 hours, and preferably about 0.5 to about 50 hours.

<Production of Compound (VIII)>

Compound (VIII) used in the production of compound (V) can be produced, for example, by a reaction between

Compound (II)

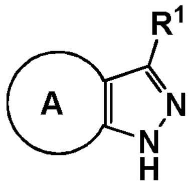

(II)

(In the formula, the symbols are synonymous with the above.) and Compound (IX)

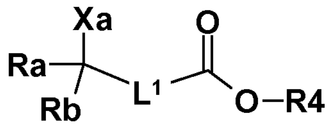

(IX)

(In the formula, the symbols are synonymous with the above.).
The reaction between compound (II) and compound (IX) is preferably carried out in a solvent, examples of which include aromatic hydrocarbons such as toluene, ethers such as 1,4-dioxane or tetrahydrofuran, or amides such as N,N-dimethyl formamide, in the presence of a base such as potassium tert-butoxide, sodium hydride, potassium carbonate, and cesium carbonate.
The reaction is preferably carried out by dissolving compound (II) in a solvent such as N,N-dimethyl formamide, adding potassium tert-butoxide, and then adding compound (IX).
In the reaction, compound (IX) is ordinarily used in an amount of about 1 to about 5 mol per mol starting compound, and the amount of the base is about 0.1 to about 100 equivalents, and preferably 1 to 5 equivalents. The reaction temperature is ordinarily 0°C to 200°C, and preferably 0°C to 100°C. The reaction time is about 0.1 to about 100 hours, and preferably about 0.5 to about 50 hours.

Production Method C

When Compound (I) is
Compound (If)

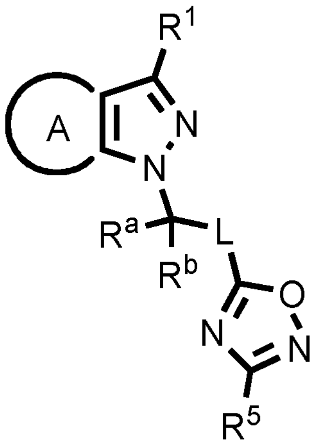

(If)

(In the formula, $R^5$ is an optionally substituted $C_{1-6}$ alkyl group, optionally substituted aromatic hydrocarbon group, or optionally substituted 5- to 10-membered heterocyclic group. The other symbols are synonymous with the above.), Compound (I) can be produced, for example, by a method in which Compound (XI)

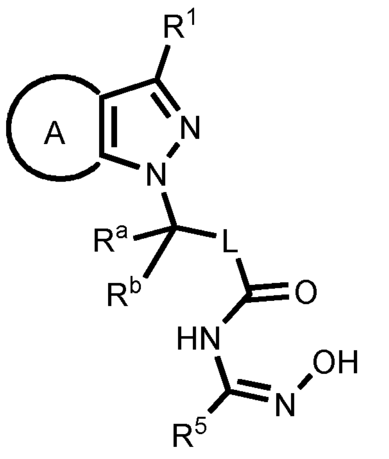

**(XI)**

(In the formula, the symbols are synonymous with the above) which is produced by

1) a method in which Compound (Va)

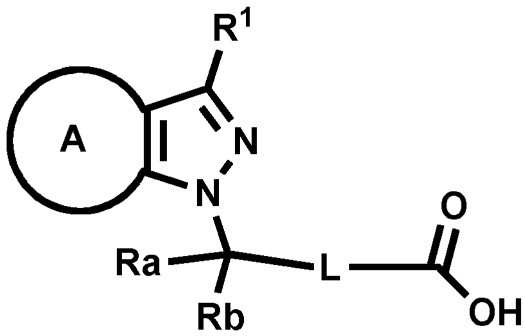

**(Va)**

(In the formula, the symbols are synonymous with the above) and Compound (X)

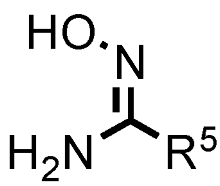

(X)

(In the formula, the symbols are synonymous with the above) are condensed with a well known dehydrocondensation agent;
2) a method in which the carboxyl group of Compound (Va) is activated by a well known activation method, and Compound (X) is then reacted; or
3) a method in which a derivative of Compound (Va) and Compound (X) are reacted;
or the like, is
4) subjected to intramolecular dehydrocondensation.

Method 1)

Compound (XI) can be produced by condensing compound (Va) and compound (X) with a well known dehydrocondensation agent.
Examples of dehydrocondensation agents used in this reaction include N,N'-dicyclohexylcarbodiimide, 1-ethyl-3-(3-dimethylaminopropyl)carbodiimide (WSC) or hydrochloride thereof, N,N'-carbonyldiimidazole, 1H-benzotriazol-1-yloxytris(dimethylamino)phosphonium hexafluorophosphate, O-(7-azabenzotriazol-1-yl)-1,1,3,3-tetramethyluronium hexafluorophosphate (HATU), 2-chloro-1,3-dimethylimidazolium chloride, and bromotripyrrolidinophosphonium hexafluorophosphate.
The reaction may be carried out as needed, for example, in the presence of 1-hydroxy-1H-benzotriazole (HOBt); or a base such as N,N-diisopropylethylamine, N-methylmorpholine, triethylamine, and 4-(N,N-dimethylamino)pyridine.
The reaction is preferably carried out in a well-known solvent, examples of which include amides such as N,N-dimethyl formamide, N,N-dimethyl acetamide, and N-methyl pyrrolidone; halogenated hydrocarbons such as dichloromethane; esters such as ethyl acetate; hydrocarbons such as cyclohexane and n-hexane; aromatic hydrocarbons such as toluene; ethers such as tetrahydrofuran, diethyl ether, dioxane, and 1,2-dimethoxyethane; and nitriles such as acetonitrile.
The reaction is preferably carried out by dissolving compound (V) and compound (X) in a solvent such as N,N-dimethyl formamide, and adding 1-ethyl-3-(3-dimethylaminopropyl)carbodiimide (WSC) or hydrochloride thereof as the dehydrocondensation agent in the presence of 1-hydroxybenzotriazole (HOBt). In the reaction, compound (X) is ordinarily used in an amount of about 1 to about 5 mol per mol starting compound, and the amount of the condensation agent is about 1 to about 100 equivalents, and preferably 1 to 5 equivalents. The reaction temperature is ordinarily 0°C to 100°C, and preferably 0°C to 60°C. The reaction time is about 0.1 to about 100 hours, and preferably about 0.5 to about 50 hours.

Method 2)

Compound (XI) can also be produced by activating the carboxyl group of compound (Va) by a well known activation method, and then reacting compound (X).
A common method can be used as the method for activating the carboxyl group of compound (Va), such as methods in which an acid anhydride is produced using chloroformic acid ester, pivaloyl chloride, 2,4,6-trichlorobenzoyl chloride, or the like;
methods in which an acid halide is produced using thionyl chloride, oxalyl chloride, or the like; and methods in which an ester of 1-hydroxybenzotriazole, pentafluorophenol, or the like is produced using a dehydrocondensation agent.
Typical examples include methods for producing acid halides. Examples of acid halides include Compound (VIIa)

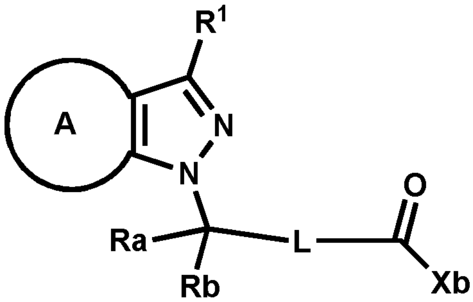

(VIIa)

(In the formula, the symbols are synonymous with the above). Such acid halides can be produced, for example, by treating compound (VIIa) with a halogenating agent such as thionyl chloride or oxalyl chloride. N,N-dimethyl formamide may be added, for example, as an additive in such cases.
The reaction is preferably carried out in, or without, a well known solvent, examples of which include halogenated hydrocarbons such as dichloromethane, ethers such as tetrahydrofuran and diethyl ether, and aromatic hydrocarbons such as toluene.
The reaction is preferably carried out by adding oxalyl chloride to compound (Va) in the presence of N,N-dimethyl formamide in tetrahydrofuran.
In the reaction, the halogenating agent is ordinarily used in an amount of about 1 to about 100 equivalents, and preferably 1 to 5 equivalents, per mol starting compound. The reaction temperature is ordinarily -78°C to 100°C, and preferably 0°C to 100°C. The reaction time is about 0.1 to about 100 hours, and preferably about 0.5 to about 50 hours.
Compound (XI) is obtained by activating the carboxyl group of compound (Va) and then reacting compound (X). The

reaction is preferably carried out in a well known solvent (examples of which include halogenated hydrocarbons such as dichloromethane; ethers such as tetrahydrofuran and diethyl ether; and amides such as N,N-dimethyl formamide, N,N-dimethyl acetamide, and N-methyl pyrrolidone) in the presence of a base such as triethylamine or pyridine.
The reaction is preferably carried out by activating the carboxyl group of compound (Va) to obtain compound (VIIa), and then adding compound (X) in the presence of a base such as triethylamine in tetrahydrofuran, for example.
The reaction is carried out ordinarily at a reaction temperature of -78°C to 150°C, and preferably 0°C to 100°C, using an acid halide and compound (X) ordinarily in an amount of about 1 to about 5 mol per mol starting compound. The reaction time is about 0.1 to about 100 hours, and preferably about 0.5 to about 50 hours.

Method 3)

Compound (XI) can also be produced by a reaction between a derivative of compound (Va) and compound (X). Examples of derivatives of compound (Va) include optionally substituted $C_{1-6}$ alkyl (such as methyl, ethyl, n-propyl, i-propyl, n-butyl, and tert-butyl) esters, optionally substituted phenyl esters, optionally substituted silyl esters, optionally substituted mono-$C_{1-6}$ alkyl amides, and optionally substituted di-$C_{1-6}$ alkyl amides. Examples of substituents for these include halogen atoms, nitro group, hydroxy group, and $C_{1-6}$ alkoxy groups. The number of substituents is about 1 to 3.
The reaction is carried out, for example, by a method in which a derivative of compound (Va), preferably a lower alkyl ester (especially a methyl ester or ethyl ester) of compound (Va), and compound (X) are both present and are heated.
The reaction is carried out ordinarily at a reaction temperature of 0°C to 200°C, and preferably 40°C to 200°C, using compound (X) ordinarily in an amount of about 1 to about 5 mol per mol starting compound. The reaction time is about 0.1 to about 100 hours, and preferably about 0.5 to about 50 hours.

Method 4)

Compound (If) can be produced by dehydrating compound (XI).
The dehydration reaction of compound (XI) is preferably carried out in a well-known solvent, examples of which include amides such as N,N-dimethyl formamide, N,N-dimethyl acetamide, and N-methyl pyrrolidone; halogenated hydrocarbons such as dichloromethane; esters such as ethyl acetate; hydrocarbons such as cyclohexane and n-hexane; aromatic hydrocarbons such as toluene and xylene; aromatic heterocycles such as pyridine; ethers such as tetrahydrofuran, diethyl ether, dioxane, and 1,2-dimethoxyethane; alcohols such as methanol and ethanol; nitriles such as acetonitrile; organic acids such as acetic acid; aqueous solution of inorganic acids such as hydrochloric acid; or water.
The reaction may be carried out as needed, for example, in the presence of an acid halide such as acetic acid chloride, propionic acid chloride, or benzoic acid chloride; an acid such as p-toluenesulfonic acid, acetic acid, trifluoroacetic acid, or hydrochloric acid; a base such as sodium methoxide, potassium tert-butoxide, sodium hydride, potassium carbonate, or cesium carbonate; tetrabutylammonium bromide; sodium acetate; or Burgess reagent; or a condensation agent such as N,N'-dicyclohexylcarbodiimide, 1-ethyl-3-(3-dimethylaminopropyl)carbodiimide (WSC) or hydrochloride thereof, N, N'-carbonyldiimidazole, 1H-benzotriazol-1-yloxytris(dimethylamino)phosphonium hexafluorophosphate, O-(7-azabenzotriazol-1-yl)-1,1,3,3-tetramethyluronium hexafluorophosphate (HATU), 2-chloro-1,3-dimethylimidazolium chloride, and bromotripyrrolidinophosphonium hexafluorophosphate.
The reaction may also be carried out under Mitsunobu reaction conditions using an azocarboxylate ester such as diethyl azodicarboxylate or diisopropyl azodicarboxylate, and a phosphine such as triphenylphosphine.
The reaction is preferably carried out by dissolving compound (XI) in a solvent such as pyridine, and by heating and stirring or microwaving the mixture.
The reaction is ordinarily carried out at a reaction temperature of 0°C to 200°C. The reaction time is about 0.1 to about 100 hours, and preferably about 0.5 to about 50 hours.

Production Method D

When Compound (I) is
Compound (Ig)

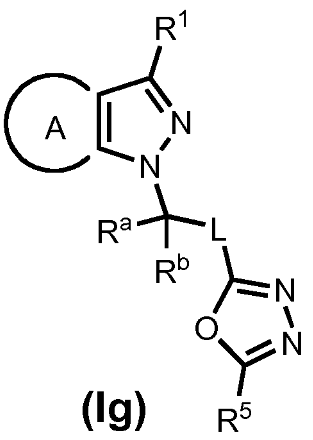

(Ig)

(In the formula, the symbols are synonymous with the above.), Compound (I) can be produced, for example, by a method in which
Compound (XIII)

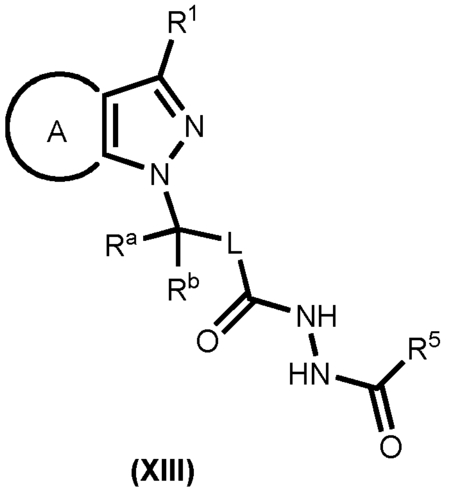

(XIII)

(In the formula, the symbols are synonymous with the above)
which is produced by

1) a method in which Compound (Va)

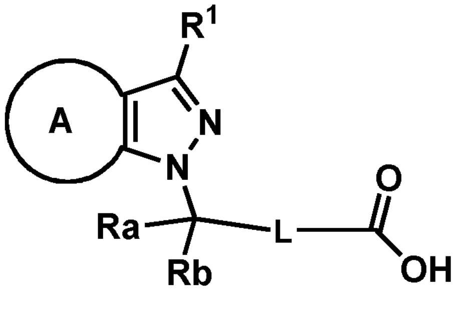

(Va)

(In the formula, the symbols are synonymous with the above) and Compound (XII)

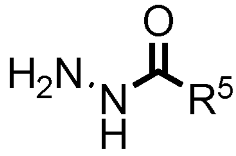

(XII)

(In the formula, the symbols are synonymous with the above) are condensed with a well known dehydrocondensation agent;

2) a method in which the carboxyl group of Compound (Va) is activated by a well known activation method, and Compound (X) is then reacted; or

3) a method in which a derivative of Compound (V) and Compound (X) are reacted; or the like, is

4) subjected to intramolecular dehydrocondensation.

Method 1)

Compound (XIII) can be produced by condensing compound (Va) and compound (XII) with a well known dehydrocondensation agent.
Examples of dehydrocondensation agents used in this reaction include N,N'-dicyclohexylcarbodiimide, 1-ethyl-3-(3-dimethylaminopropyl)carbodiimide (WSC) or hydrochloride thereof, N,N'-carbonyldiimidazole, 1H-benzotriazol-1-yloxytris(dimethylamino)phosphonium hexafluorophosphate, O-(7-azabenzotriazol-1-yl)-1,1,3,3-tetramethyluronium hexafluorophosphate (HATU), 2-chloro-1,3-dimethylimidazolium chloride, and bromotripyrrolidinophosphonium hexafluorophosphate.
The reaction may be carried out as needed, for example, in the presence of 1-hydroxy-1H-benzotriazole (HOBt); or a base such as N,N-diisopropylethylamine, N-methylmorpholine, triethylamine, and 4-(N,N-dimethylamino)pyridine.
The reaction is preferably carried out in a well-known solvent, examples of which include amides such as N,N-dimethyl formamide, N,N-dimethyl acetamide, and N-methyl pyrrolidone; halogenated hydrocarbons such as dichloromethane; esters such as ethyl acetate; hydrocarbons such as cyclohexane and n-hexane; aromatic hydrocarbons such as toluene; ethers such as tetrahydrofuran, diethyl ether, dioxane, and 1,2-dimethoxyethane; and nitriles such as acetonitrile.
The reaction is preferably carried out by dissolving compound (Va) and compound (XII) in a solvent such as acetonitrile, and adding 2-chloro-1,3-dimethylimidazolium chloride as the dehydrocondensation agent in the presence of triethylamine.
In the reaction, compound (XII) is ordinarily used in an amount of about 1 to about 5 mol per mol starting compound, and the amount of the condensation agent is about 1 to about 100 equivalents, and preferably 1 to 5 equivalents. The reaction temperature is ordinarily 0°C to 100°C, and preferably 0°C to 60°C. The reaction time is about 0.1 to about 100 hours, and preferably about 0.1 to about 50 hours.

Method 2)

Compound (XIII) can also be produced by activating the carboxyl group of compound (Va) by a well known activation method, and then reacting compound (XII).
A common method can be used as the method for activating the carboxyl group of compound (Va), such as methods in which an acid anhydride is produced using chloroformic acid ester, pivaloyl chloride, 2,4,6-trichlorobenzoyl chloride, or the like;
methods in which an acid halide is produced using thionyl chloride, oxalyl chloride, or the like; and methods in which an ester of 1-hydroxybenzotriazole, pentafluorophenol, or the like is produced using a dehydrocondensation agent.
Typical examples include methods for producing acid halides. Examples of acid halides include Compound (VII)

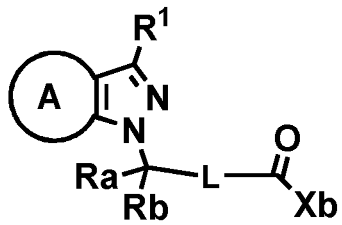

(VIIa)

(In the formula, the symbols are synonymous with the above). Such acid halides can be produced, for example, by treating compound (VIIa) with a halogenating agent such as thionyl chloride or oxalyl chloride. N,N-dimethyl formamide may be added, for example, as an additive in such cases.
The reaction is preferably carried out in, or without, a well known solvent, examples of which include halogenated hydrocarbons such as dichloromethane, ethers such as tetrahydrofuran and diethyl ether, and aromatic hydrocarbons such as toluene.
The reaction is preferably carried out by adding oxalyl chloride to compound (Va) in the presence of N,N-dimethyl formamide in tetrahydrofuran.
In the reaction, the halogenating agent is ordinarily used in an amount of about 1 to about 100 equivalents, and preferably 1 to 5 equivalents, per mol starting compound. The reaction temperature is ordinarily -78°C to 100°C, and preferably 0°C to 100°C. The reaction time is about 0.1 to about 100 hours, and preferably about 0.5 to about 50 hours.
The reaction is preferably carried out in a well known solvent (examples of which include halogenated hydrocarbons such as dichloromethane; ethers such as tetrahydrofuran and diethyl ether; and amides such as N,N-dimethyl formamide, N,N-dimethyl acetamide, and N-methyl pyrrolidone) in the presence of a base such as triethylamine or pyridine.
The reaction is preferably carried out by activating the carboxyl group of compound (Va) to obtain compound (VIIa), and then adding compound (XII) in the presence of a base such as triethylamine in tetrahydrofuran, for example.
The reaction is carried out ordinarily at a reaction temperature of -78°C to 150°C, and preferably 0°C to 100°C, using an acid halide and compound (XII) ordinarily in an amount of about 1 to about 5 mol per mol starting compound. The reaction time is about 0.1 to about 100 hours, and preferably about 0.1 to about 50 hours.

Method 3)

Compound (XIII) can also be produced by a reaction between a derivative of compound (Va) and compound (XII). Examples of derivatives of compound (Va) include optionally substituted $C_{1-6}$ alkyl (such as methyl, ethyl, n-propyl, i-propyl, n-butyl, and tert-butyl) esters, optionally substituted phenyl esters, optionally substituted silyl esters, optionally substituted mono-$C_{1-6}$ alkyl amides, and optionally substituted di-$C_{1-6}$ alkyl amides. Examples of substituents for these include halogen atoms, nitro group, hydroxy group, and $C_{1-6}$ alkoxy groups. The number of substituents is about 1 to 3.
The reaction is carried out, for example, by a method in which a derivative of compound (Va), preferably a lower alkyl ester (especially a methyl ester or ethyl ester) of compound (Va), and compound (XII) are both present and are heated.
The reaction is carried out ordinarily at a reaction temperature of 0°C to 200°C, and preferably 40°C to 200°C, using compound (XII) ordinarily in an amount of about 1 to about 5 mol per mol starting compound. The reaction time is about 0.1 to about 100 hours, and preferably about 0.5 to about 50 hours.

Method 4)

Compound (Ig) can be produced by dehydrating compound (XIII).
The dehydration reaction of compound (XIII) is preferably carried out in a well-known solvent, examples of which include amides such as N,N-dimethyl formamide, N,N-dimethyl acetamide, and N-methyl pyrrolidone; halogenated hydrocarbons such as dichloromethane; esters such as ethyl acetate; hydrocarbons such as cyclohexane and n-hexane; aromatic hydrocarbons such as toluene and xylene; aromatic heterocycles such as pyridine; ethers such as tetrahydrofuran, diethyl ether, dioxane, and 1,2-dimethoxyethane; alcohols such as methanol and ethanol; nitriles such as acetonitrile; organic acids such as acetic acid; aqueous solution of inorganic acids such as hydrochloric acid; or water.
The reaction may be carried out as needed, for example, in the presence of an acid halide such as acetic acid chloride, propionic acid chloride, or benzoic acid chloride; an acid such as p-toluenesulfonic acid, acetic acid, trifluoroacetic acid, or hydrochloric acid; a base such as sodium methoxide, potassium tert-butoxide, sodium hydride, potassium carbonate, or cesium carbonate; tetrabutylammonium bromide; sodium acetate; or Burgess reagent; or a condensation agent such as N,N'-dicyclohexylcarbodiimide, 1-ethyl-3-(3-dimethylaminopropyl)carbodiimide (WSC) or hydrochloride thereof, N,N'-carbonyldiimidazole, 1H-benzotriazol-1-yloxytris(dimethylamino)phosphonium hexafluorophosphate, O-(7-azabenzotriazol-1-yl)-1,1,3,3-tetramethyluronium hexafluorophosphate (HATU), 2-chloro-1,3-dimethylimidazolium chloride, or bromotripyrrolidinophosphonium hexafluorophosphate.
The reaction is preferably carried out by dissolving compound (XIII) in a solvent such as acetonitrile, and by heating and stirring or microwaving the mixture.
The reaction is ordinarily carried out at a reaction temperature of 0°C to 200°C. The reaction time is about 0.1 to about 100 hours, and preferably about 0.5 to about 50 hours.

Production Method E

Compound (Ia) can also be produced, for example, by a reaction between Compound (II)

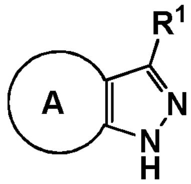

(II)

(In the formula, the symbols are synonymous with the above.) and Compound (XIV)

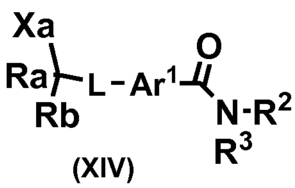

(XIV)

(In the formula, the symbols are synonymous with the above.).
The reaction between compound (II) and compound (XIV) is preferably carried out in a solvent, examples of which include aromatic hydrocarbons such as toluene, ethers such as 1,4-dioxane or tetrahydrofuran, and amides such as N,N-dimethyl formamide, in the presence of a base such as potassium tert-butoxide, sodium hydride, potassium carbonate, or cesium carbonate.
The reaction is preferably carried out by dissolving compound (II) in a solvent such as N,N-dimethyl formamide, adding potassium tert-butoxide, and then adding compound (XIV).
In the reaction, compound (XIV) is ordinarily used in an amount of about 1 to about 5 mol per mol starting compound, and the amount of the base is about 0.1 to about 100 equivalents, and preferably 1 to 5 equivalents. The reaction temperature is ordinarily 0°C to 200°C, and preferably 0°C to 100°C. The reaction time is about 0.1 to about 100 hours, and preferably about 0.5 to about 50 hours.

Production Method F

Compound (Ia) and compound (Id) can also be produced, for example, by a reaction between Compound (XV)

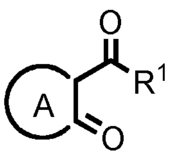

(XV)

(In the formula, the symbols are synonymous with the above.) and Compound (XVI)

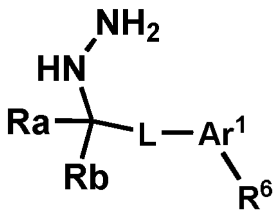

(XVI)

(In the formula, $R^6$ represents $-CONR^2R^3$ or $-CO_2R^4$, and the other symbols are synonymous with the above.).

The reaction between compound (XV) and compound (XVI) is preferably carried out in a solvent, examples of which include aromatic hydrocarbons such as toluene; ethers such as 1,4-dioxane and tetrahydrofuran; alcohols such as ethanol and n-butanol; and amides such as N,N-dimethyl formamide.

The reaction may also be carried out as needed, for example, in the presence of an acid such as p-toluenesulfonic acid, acetic acid, trifluoroacetic acid, or hydrochloric acid.

The reaction is preferably carried out by dissolving compound (XV) and compound (XVI) in a solvent such as ethanol or toluene, and adding p-toluenesulfonic acid.

In the reaction, compound (XV) is ordinarily used in an amount of about 1 to about 5 mol, and preferably 1 to 2 equivalents, per mol compound (XIV), and the amount of acid is about 0.1 to about 100 equivalents, and preferably 0.1 to 2 equivalents. The reaction temperature is ordinarily 0°C to 200°C, and preferably 20°C to 150°C. The reaction time is about 0.1 to about 100 hours, and preferably about 0.5 to about 50 hours.

In compounds (I), (Ia), (Ie), (If), and (Ig) thus obtained, the intramolecular functional groups can be converted to the intended functional groups by incorporating a well known chemical reaction. Examples of such chemical reactions include oxidation, reduction, alkylation, hydrolysis, amination, amidation, esterification, aryl coupling reactions, and deprotection.

When the starting compound has an amino group, carboxyl group, hydroxy group, or carbonyl group as a substituent in the above reactions, a protective group that is commonly used in peptide chemistry or the like may be introduced to these groups, and the protective group can be removed as needed after the reaction to obtain the target compound.

Examples of amino-protecting groups include formyl, as well as the following optionally substituted examples: $C_{1-6}$ alkylcarbonyl (such as acetyl and ethylcarbonyl), phenylcarbonyl, $C_{1-6}$ alkoxycarbonyl (such as methoxycarbonyl, ethoxycarbonyl, and tert-butoxycarbonyl), phenyloxycarbonyl, $C_{7-10}$ aralkyl-carbonyls (such as benzylcarbonyl), trityl, phthaloyl, and N,N-dimethylaminomethylene. Examples of substituents for the "amino-protecting groups" include halogen atoms (such as fluorine, chlorine, bromine, and iodine), $C_{1-6}$ alkyl-carbonyls (such as methylcarbonyl, ethylcarbonyl, and butylcarbonyl), and nitro group, the number of which is 1 or more (such as 3).

Examples of carboxyl-protecting groups include $C_{1-6}$ alkyl groups, $C_{7-11}$ aralkyl groups (such as benzyl), phenyl group, trityl group, substituted silyl groups (such as trimethylsilyl, triethylsilyl, dimethylphenylsilyl, tert-butyldimethylsilyl, and tert-butyldiethylsilyl), and $C_{2-6}$ alkenyl groups (such as 1-allyl). These groups are optionally substituted with 1 to 3 halogen atoms, $C_{1-6}$ alkoxy groups, or nitro groups, etc.

Examples of hydroxy-protecting groups include $C_{1-6}$ alkyl groups, phenyl group, trityl group, $C_{7-10}$ aralkyl groups (such as benzyl), formyl group, $C_{1-6}$ alkyl-carbonyl groups, benzoyl group, $C_{7-10}$ aralkyl-carbonyl groups (such as benzylcarbonyl), 2-tetrahydropyranyl group, 2-tetrahydrofuranyl group, substituted silyl groups (such as trimethylsilyl, triethylsilyl, dimethylphenylsilyl, tert-butyldimethylsilyl, and tert-butyldiethylsilyl), and $C_{2-6}$ alkenyl groups (such as 1-allyl). These groups are optionally substituted with 1 to 3 halogen atoms, $C_{1-6}$ alkyl groups, $C_{1-6}$ alkoxy groups, or nitro groups, etc.

Examples of carbonyl-protecting groups include cyclic acetals (such as 1,3-dioxane) and acyclic acetals (such as di-$C_{1-6}$ alkyl acetals).

The above protective groups can be removed by a well known method such as the methods described in Protective Groups in Organic Synthesis, John Wiley and Sons (1980). Examples include methods using an acid, base, UV light, hydrazine, phenyl hydrazine, sodium N-methyldithiocarbamate, tetrabutylammonium fluoride, palladium acetate, or trialkylsilyl halide (such as trimethylsilyl iodide or trimethylsilyl bromide), or reduction and the like.

Compounds (I), (Ia), (Ie), (If), and (Ig) can be isolated and purified by well known means such as solvent extraction, liquid conversion, transfer dissolution, concentration, vacuum concentration, crystallization, recrystallization, and chromatography. Starting compounds of compounds (I), (Ia), (Ie), (If), and (Ig), and salts thereof, can also be isolated and purified by the same well known means as above or the like, but may also be provided as starting material in subsequent processing in the form of the reaction mixture as such without being isolated.

In either case, compound (I) can be synthesized through the following additional well known reactions as needed, either individually or in any combination: deprotection, acylation, alkylation, hydrogenation, oxidation, reduction, carbon chain extension, or substituent replacement.

When compound (I) is in the form of an isomer such as an optical isomer, stereoisomer, positional isomer, or rotational isomer, any such isomers or mixtures are encompassed by compound (I). For example, when optical isomers are present in compound (I), an optical isomer resolved from the racemic mixture is encompassed by compound (I). These isomers can be obtained in the form of individual products by methods of synthesis that are well known per se (such as concentration, vacuum concentration, solvent extraction, column chromatography, and recrystallization).

Compound (I) may be in the form of crystals, which are encompassed by compound (I), whether of a single crystal type or a mixture of crystal types. Crystals can be produced by crystallization using methods of crystallization that are well known per se.

Compound (I) may be in the form of a solvate (such as a hydrate) or a nonsolvate (such as an acid anhydride), both of which are encompassed by compound (I).

Compounds labeled or substituted with an isotope (such as $^{2}H$, $^{3}H$, $^{14}C$, $^{35}S$, and $^{125}I$) or the like are encompassed by compound (I).

The compounds of the present invention, which have excellent action in potentiating the AMPA receptor, are useful for preventing and treating the following diseases and the like in mammals (such as mice, rats, hamsters, rabbits, cats, dogs, cows, sheep, monkeys, and humans):

(1) mental illness [such as depression, major depression, bipolar depression, dysthymic disorder, emotional disorders (such as seasonal affective disorder), recurrent depression, postpartum depression, stress disorders, depressive symptoms, mania, anxiety, generalized anxiety disorder, anxiety syndrome, panic disorders, phobias, social phobias, social anxiety disorders, obsessive compulsive disorders, mental post-traumatic stress disorder, post-traumatic stress disorder, Tourette's syndrome, autism, adjustment disorders, bipolar disorder, neuroses, schizophrenia (schizophrenic psychoses), neurosis, chronic fatigue syndrome, anxiety neurosis, compulsive neurosis, panic disorder, epilepsy, anxiety symptoms, dysphoria, emotional disorders, cyclothymia, nervous erethism, syncope, addiction, decreased sexual desire, attention-deficit hyperactivity disorder (ADHD), major psychotic depression, intractable major depression, and refractory depression],

(2) neurodegenerative diseases [such as Alzheimer's disease, Alzheimer's type senile dementia, Parkinson's disease, Huntington's chorea, multi-infarct dementia, frontotemporal dementia, Parkinson's type frontotemporal dementia, progressive supranuclear palsy, Pick's syndrome, Niemann-Pick's syndrome, corticobasal degeneration, Down's syndrome, vascular dementia, post-encephalitic Parkinsonism, dementia with Lewy bodies, HIV-associated dementia, amyotrophic lateral sclerosis (ALS), motor neuron disease (MND), Creutzfeldt-Jakob disease or prion disease, cerebral palsy, progressive supranuclear palsy, and multiple sclerosis],

(3) cognitive and memory impairment associated with aging [such as age-related memory impairment and senile dementia],

(4) sleep disorders [such as intrinsic sleep disorders (such as psychophysiological insomnia), extrinsic sleep disorders, circadian rhythm sleep disorders (such as desynchronous syndrome (jet lag), shift work sleep disorder, irregular sleep-wake pattern, delayed sleep phase syndrome, advanced sleep phase syndrome, and non-24-hour sleep-wake syndrome), parasomnias, sleep disorders associated with medical or psychiatric disorders (such as chronic obstructive pulmonary disease, Alzheimer's disease, Parkinson's disease, cerebrovascular dementia, schizophrenia, depression, and anxiety neurosis), stress insomnia, insomnia, insomnia neurosis, and sleep apnea syndrome],

(5) respiratory depression caused by anesthesia, traumatic disease, neurodegenerative disease, or the like, and

(6) traumatic brain injury, anorexia syndrome, eating disorders, anorexia nervosa, bulimia, other eating disorders, alcohol dependency, alcohol abuse, alcohol amnestic syndrome, alcohol-induced delusional syndrome, alcoholophilia, alcohol withdrawal, alcoholic psychosis, alcohol toxicity, alcoholic jealousy, alcoholic mania, alcohol-dependent mental disorders, alcoholic psychosis, pharmacophilia, pharmacophobia, pharmacomania, drug withdrawal, migraine, stress head ache, tension head ache, diabetic neuropathy, obesity, diabetes mellitus, muscle cramps, Meniere's disease, dysautonomia, alopecia, glaucoma, hypertension, heart disease, tachycardia, congestive heart failure, hyperpnea, bronchial asthma, apnea, sudden infant death syndrome, inflammatory diseases, allergy diseases, impotence, climacteric disturbance, infertility, cancer, HIV-induced immune deficiency syndrome, stress-induced immunodeficiency syndrome, cerebrospinal meningitis, acromegaly, incontinence, metabolic syndrome, osteoporosis, peptic ulcers, irritable bowel syndrome, inflammatory bowel disease, ulcerative colitis, Crohn's disease, stress-induced gastrointestinal disorders, nervous vomiting, peptic ulcer, diarrhea, constipation, post-operative ileus, and stress-induced gastrointestinal disorders.

The compounds of the present invention have excellent action in potentiating the AMPA receptor, and have better therapeutic efficacy against the above diseases can thus be anticipated.

The compounds of the present invention have low toxicity (are better as pharmaceuticals in terms of, for example, acute toxicity, chronic toxicity, genotoxicity, reproductive toxicity, cardiac toxicity, drug interactions, and carcinogenicity), and can be safely administered orally or parenterally, as it is as a medicament, or in the form of a pharmaceutical composition while mixed with a pharmaceutically acceptable carrier or the like, to mammals (such as humans, monkeys, cows, horses, swine, mice, rats, hamsters, rabbits, cats, dogs, sheep, and goats). "Parenteral" includes administration that is intravenous, intramuscular, subcutaneous, pernasal, intradermal, instillation, intracerebral, rectal, intravaginal, intraperitoneal, intratumoral, or near tumors, and direct administration to lesions.

The dosage of the compound of the present invention will vary depending on the route of administration, symptoms, and the like, but when given orally to patients (adults weighing 40 to 80 kg, such as 60 kg) with schizophrenia, for example, the dose is, for example, 0.001 to 1000 mg/kg body weight per day, preferably 0.01 to 100 mg/kg body weight per day, and even more preferably 0.1 to 10 mg/kg per day. This amount can be given divided once to three times per day.

Examples of dosage forms for when the compound of the present invention is in the form of a pharmaceutical composition include tablets (such as sugar-coated tablets, film-coated tablets, and orally disintegrable tablets), film agents (such as orally disintegrable films), pills, capsules, granules" subtle granules, dispersions, powders, syrups, emulsions, suspensions, injections, controlled-release injections, inhalants, and ointments. These formulations may be prepared by common methods (such as methods described in the Japanese Pharmacopoeia).

A variety of organic or inorganic carriers commonly used as materials for formulation (starting material) may be used as the above "pharmaceutically acceptable carrier." Excipients, lubricants, binders, disintegrants, and the like may be used in solid formulations, for example, and solvents, dissolution aids, suspending agents, isotonizing agents, buffers, soothing agents, and the like may be used in liquid formulations. Additives such as preservatives, antioxidants, colorants, and sweeteners can also be used as needed.

The pharmaceutical composition will vary depending on the dosage form, method of administration, carrier, and the like, but can be produced by a common method by adding the compound of the present invention ordinarily in a proportion of 0.01 to 100% (w/w), and preferably 0.1 to 95% (w/w), relative to the entire amount of the formulation.

The compound of the present invention may also be used with other active ingredients (hereinafter also referred to simply as concomitant drugs).

Examples of concomitant drugs are given below.

Benzodiazepines (such as chlordiazepoxide, diazepam, potassium clorazepate, lorazepam, clonazepam, and alprazolam), L-type calcium channel blockers (such as pregabalin), tricyclic or tetracyclic antidepressants (such as imipramine hydrochloride, amitriptyline hydrochloride, desipramine hydrochloride, and clomipramine hydrochloride), selective serotonin reuptake inhibitors (such as fluvoxamine maleate, fluoxetine hydrochloride, citalopram bromate, sertraline hydrochloride, paroxetine hydrochloride, and escitalopram oxalate), serotonin-noradrenaline reuptake inhibitors (such as venlafaxine hydrochloride, duloxetine hydrochloride, and desvenlafaxine hydrochloride), noradrenaline reuptake inhibitors (such as reboxetine mesylate), mirtazapine, trazodone hydrochloride, nefazodone hydrochloride, bupropion hydrochloride, setiptiline maleate, 5-$HT_{1A}$ agonists (such as buspirone hydrochloride, tandospirone citrate, and osemozotan hydrochloride), 5-$HT_3$ antagonists (such as cyamemazine), non-heart-selective beta blockers (such as propranolol hydrochloride and oxyprenolol hydrochloride), histamine $H_1$ antagonists (such as hydroxyzine hydrochloride), therapeutic agents for schizophrenia (such as chlorpromazine, haloperidol, sulpiride, clozapine, trifluoroperazine hydrochloride, fluphenazine hydrochloride, olanzapine, quetiapine fumarate, risperidone, and aripiprazole), CRF antagonists, other anxiolytics (such as meprobamate), tachykinin antagonists (such as MKI-869 and saredutant), drugs having action on metabolic glutamate receptors, CCK antagonists, beta 3 adrenergic antagonists (such as amibegron hydrochloride), GAT-1 inhibitors (such as tiagabine hydrochloride), N-type calcium channel blockers, type-2 carbonic anhydrase inhibitors, NMDA glycine site agonists, NMDA antagonists (such as memantine), peripheral benzodiazepine receptor agonists, vasopressin antagonists, vasopressin V1b antagonists, vasopressin VIa antagonists, phosphodiesterase inhibitors, opioid antagonists, opioid agonists, uridine, nicotinic acid receptor agonists, thyroid hormone (T3, T4), TSH, TRH, MAO inhibitors (such as phenelzine sulfate, tranylcypromine sulfate, and moclobemide), 5-$HT_{2A}$ antagonists, 5-$HT_{2A}$ inverse agonists, COMT inhibitors (such as entacapone), therapeutic agents for bipolar disorders (such as lithium carbonate, sodium valproate, lamotrigine, riluzole, and felbamate), cannabinoid CB1 antagonists (such as rimonabant), FAAH inhibitors, sodium channel blockers, anti-ADHD agents (such as methylphenidate hydrochloride and methamphetamine hydrochloride), alcohol dependency medication, autism medication, chronic fatigue syndrome medication, anticonvulsants, fibromyalgia medication, headache medication, insomnia medication (such as etizolam, zopiclone, triazolam, zolpidem, ramelteon, and indiplon), smoking cessation medication, myasthenia gravis medication, cerebral infarction medication, medication for mania, narcolepsy medication, pain medication, dysthymia medication, dysautonomia medication, medication for male and female sexual dysfunction, migraine medication, medication for pathological gambling, restless leg syndrome medication, substance dependency medication, medication for alcohol-related diseases, irritable bowel syndrome medication, Alzheimer's medication (such as donepezil, galantamin, and memantine), Parkinson's medication, ALS medication (such as riluzole and neurotrophic factors), dyslipidemia medication such as cholesterol-lowering medication (such as the statin series (sodium pravastatin, atorvastatin, simvastatin, rosuvastatin

etc.), fibrates (clofibrate, etc.), and squalene synthesis inhibitors), medication for abnormal behavior or agents for reducing wandering due to dementia (such as analgesics and anxiolytics), apoptosis inhibitors, anti-obesity agents, diabetic medication, hypertensive medication, hypotensive medication, medication for rheumatism (DMARD), antitumor agents, parathyroid gland medication (PTH), calcium receptor antagonists, sex hormones or derivatives thereof (such as progesterone, estradiol, and estradiol benzoate), neuro-differentiation promoters, nerve regeneration promoters, nonsteroidal anti-inflammatory drugs (such as meloxicam, tenoxicam, indomethacin, ibuprofen, celecoxib, rofecoxib, aspirin, and indomethacin), steroids (such as dexamethasone and cortisone acetate), anticytokine agents (such as TNF inhibitors and MAP kinase inhibitors), antibody drugs, nucleic acids or nucleic acid derivatives, and aptamer drugs.

Compounds of the present invention and concomitant drugs can be combined so as to obtain better effects such as the following:

(1) the dose can be reduced compared to when the compound of the present invention or the concomitant drug is given alone,
(2) drugs can be used with compounds of the present invention according to the patient's symptoms (such as mild or severe),
(3) a longer treatment period can be established by selecting a concomitant drug in which the mechanism of action is different than that of the compound of the present invention,
(4) longer lasting therapeutic efficacy can be achieved by selecting a concomitant drug in which the mechanism of action is different than that of the compound of the present invention, and
(5) synergistic effects can be obtained by jointly using the compounds of the present invention and a concomitant drug.

The combined use of the compound of the present invention and a concomitant drug is referred to below as "concomitant agents of the present invention."

During the use of the concomitant agents of the present invention, the time at which the compound of the present invention and the concomitant drug are administered is not limited, and the compound of the present invention or pharmaceutical composition thereof and the concomitant drug or pharmaceutical composition thereof may be administered simultaneously or at different times to the subject of treatment. The dosage of the concomitant drug can be based on the clinically used dose, and can be selected as desired depending on the subject of treatment, route of administration, disease, combination, and the like.

The dosing configuration of the concomitant agent of the present invention is not particularly limited, and the compound of the present invention and the concomitant drug may be combined when administered. Examples of such a dosing configuration include (1) administration of a single formulation obtained by the simultaneous formulation of the compound of the present invention and the concomitant drug, (2) simultaneous administration, by the same route of administration, of two formulations obtained by the separate formulation of the compound of the present invention and the concomitant drug, (3) administration at different times, by the same route of administration, of two formulations obtained by the separate formulation of the compound of the present invention and the concomitant drug, (4) simultaneous administration, by different routes of administration, of two formulations obtained by the separate formulation of the compound of the present invention and the concomitant drug, and (5) administration at different times, by different routes of administration, of two formulations obtained by the separate formulation of the compound of the present invention and the concomitant drug (for example, the administration of the compound of the present invention and the concomitant drug, in that order, or in the opposite order).

The concomitant agent of the present invention has low toxicity, and the compound of the present invention and/or above concomitant drugs can, for example, be mixed with a pharmaceutically acceptable carrier in accordance with a well known method and can be safely administered orally or parenterally (such as locally, rectally, or intravenously) in the form of tablets (including sugar-coated tablets and film-coated tablets), powders, subtle granules, capsules (including soft capsules), liquids, injections, suppositories, controlled-release agents, or the like. Injections can be administered by intravenous, intramuscular, subcutaneous, or intraorgan administration or directly to lesions.

Examples of pharmaceutically acceptable carriers which may be used to produce the concomitant agent of the present invention include a variety of organic or inorganic carrier substances commonly used as carriers. For example, excipients, lubricants, binders, and disintegrants can be used in solid formulations. Solvents, dissolution aids, suspending agents, isotonizing agents, buffers, soothing agents, and the like can be used in liquid formulations. Common additives such as preservatives, antioxidants, colorants, sweeteners, adsorbents, and humectants can further more be used in moderation as needed.

The compounding ratio of the compound of the present invention and the concomitant drug in the concomitant agent of the present invention can be suitably selected depending on the subject of treatment, route of administration, disease, and the like.

For example, the content of the compound of the present invention in the concomitant agent of the present invention will vary depending on the dosage form, but is ordinarily about 0.01 to 100 percent by weight, preferably about 0.1 to

50 percent by weight, and more preferably about 0.5 to 20 percent by weight, relative to the entire formulation.
The content of the concomitant drug in the concomitant agent of the present invention will vary depending on the dosage form, but is ordinarily about 0.01 to 100 percent by weight, preferably about 0.1 to 50 percent by weight, and more preferably about 0.5 to 20 percent by weight, relative to the entire formulation.
The content of additives such as the carrier in the concomitant agent of the present invention will vary depending on the dosage form, but is ordinarily about 1 to 99.99 percent by weight, and preferably about 10 to about 90 percent by weight, relative to the entire formulation.
The content may also be the same when the compound of the present invention and the concomitant drug are separately formulated.

[Working Examples]

The present invention will be illustrated in further detail by the following reference examples, working examples, preparation example, and test example, but the present invention is not thereby limited.
In the following reference examples and working examples, "room temperature" ordinarily indicates a temperature from about 10°C to about 35°C. Unless otherwise noted, "%" indicates percent by weight. Other abbreviations used in this document are defined below. s: singlet; d: doublet; t: triplet; q: quartet; m: multiplet; br: broad; J: coupling constant.
Abbreviations used in the reference examples and working examples are defined below.
LC-MS: liquid chromatography-mass spectrometry
ESI: electrospray ionization
TLC: thin layer chromatography
DMSO: dimethyl sulfoxide; DMF: N,N-dimethyl formamide; EA: ethyl acetate; DCM: dichloromethane;
PE: petroleum ether; WSC: 1-ethyl-3-(3-dimethylaminopropyl)carbodiimide hydrochloride; HOBt: 1-hydroxybenzotriazole hydrate; HATU: 2-(7-aza-1H-benzotriazol-1-yl)-1,1,3,3-tetramethyluronium hexafluorophosphate; DIEA: N,N-diisopropylethylamine; LHMDS: lithium hexamethyldisilazide; THF: tetrahydrofuran; M: molar concentration.
LC-MS analysis in the following examples was performed under the following conditions.

1.
Measuring equipment: LC-MS System, by Waters Corporation
Column: CAPCELLPAK C18, S-3 μm, 1.5 × 3.5 mm (Shiseido)
Solvent: Solution A: water containing 0.1% trifluoroacetic acid; Solution B: acetonitrile containing 0.1% trifluoroacetic acid
Gradient cycle: 0.00 min (Solution A/Solution B = 90/10), 2.00 min (Solution A/Solution B = 5/95), 2.75 min (Solution A/Solution B = 90/10), 3.45 min (Solution A/Solution B = 90/10)
Injected amount: 10 μL; flow rate: 0.5 mL/min; detection method: UV 220 nm
MS conditions, ionization method: ESI
2.
Measuring equipment: LC-MS System, by Agilent
Column: ZORBAX C18, S-1.8 μm, 3.0 × 30 mm (Agilent)
Solvent: Solution A: water containing 10 mM ammonium acetate; Solution B: acetonitrile containing 10 mM ammonium acetate
Gradient cycle: 0.00 min (Solution A/Solution B = 90/10), 2.00 min (Solution A/Solution B = 5/95), 2.75 min (Solution A/Solution B = 5/95), 2.76 min (Solution A/Solution B = 90/10), 3.45 min (Solution A/Solution B = 90/10)
Injected amount: 10 μL; flow rate: 1.2 mL/min; detection method: UV 220 nm
MS conditions, ionization method: ESI
3.
Measuring equipment: Quattro Micro by Micromass, and HP1100 by Agilent Technology, or HPLC Mass Spectrometer LCMS-2010A by Shimadzu Corporation, or MUX System by Waters Corporation (ZQ by Micromass)
Column: CAPCELLPAK C18, UG-120, 1.5 × 35 mm (Shiseido), or DEVELOSIL COMBI-RP-5.2 × 35 mm (Nomura Chemical Co., Ltd.)
Solvent: Solution A: 5 mM ammonium acetate/2% acetonitrile/water; Solution B: 5 mM ammonium acetate 95% acetonitrile/water
Gradient cycle: 0.00 min (Solution A/Solution B = 100/0), 2.00 min (Solution A/Solution B = 0/100), 3.00 min (Solution A/Solution B = 0/100), 3.01 min (Solution A/Solution B = 100/0), 3.80 min (Solution A/Solution B = 100/0)
Injected amount: 10 μL; flow rate: 0.5 mL/min; detection method: UV 220 nm
MS conditions, ionization method: ESI
4.
Measuring equipment: 4-channel LC/MS System equipped with MUX, by Waters Corporation Column: CAPCELLPAK C18, UG-120, S-3 μm, 1.5 × 35 mm (Shiseido)
Solvent: Solution A: water containing 5 mM ammonium acetate; Solution B: acetonitrile containing 5 mM ammonium acetate
Gradient cycle: 0.00 min (Solution A/Solution B=100/0), 2.00 min (Solution A/Solution B=0/100), 3.00 min (Solution A/Solution B=0/100), 3.01 min (Solution A/Solution B=100/0), 3.30 min (Solution A/Solution B=100/0)
Injected amount: 2 μL, flow rate: 0.5 mL/min, detection method: UV 220 nm
Ionization method: ESI
5.
HPLC component: Agilent 1200
MS component: Agilent 6300
Column: Welchrom XB-C18, 5 μm, 4.6 × 50 mm
Solvent: Solution A: water; Solution B: acetonitrile
Gradient cycle: 0.00 min (Solution A/Solution B=95/5), 6.00 min (Solution A/Solution B=5/95), 6.50 min (Solution A/Solution B=5/95); or 0.00 min (Solution A/Solution B=90/10), 6.00 min (Solution A/Solution B=5/95), 6.50 min (Solution A/Solution B=5/95); or 0.00 min (Solution A/Solution B=80/20), 6.00 min (Solution A/Solution B=5/95), 6.50 min (Solution A/Solution B=5/95); or 0.00 min (Solution A/Solution B=70/30), 6.00 min (Solution A/Solution B=5/95), 6.50 min (Solution A/Solution B=5/95); or 0.00 min (Solution A/Solution B=60/40), 6.00 min (Solution A/Solution B=5/95), 6.50 min (Solution A/Solution B=5/95); or 0.00 min (Solution A/Solution B=50/50), 6.00 min (Solution A/Solution B=5/95), 6.50 min (Solution A/Solution B=5/95); or 0.00 min (Solution A/Solution B=40/60), 6.00 min (Solution A/Solution B=5/95), 6.50 min (Solution A/Solution B=5/95)
Flow rate: 1.5 mL/min, detection method UV 214 or 254 nm
Ionization method: ESI
Purification by preparative HPLC in the following examples was performed under the following conditions.

1.
Equipment: Semi-preparative purification system by Gilson
Column: YMC CombiPrep Pro C18 RS, S-5 μm, 50 × 20 mm
Solvent: Solution A: water containing 0.1% trifluoroacetic acid; Solution B: acetonitrile containing 0.1% trifluoroacetic acid
Gradient cycle: 0.00 min (Solution A/Solution B = 90/10), 1.20 min (Solution A/Solution B = 90/10), 4.75 min (Solution A/Solution B = 0/100), 7.30 min (Solution A/Solution B = 0/100), 7.40 min (Solution A/Solution B = 90/10), 7.50 min (Solution A/Solution B = 90/10)
Flow rate: 25 mL/min, detection method: UV 220 nm
2.
Equipment: Preparative purification system by Waters Corporation
Column: Waters SunFire C18, S-5 μm, 30 × 50 mm
Solvent: Solution A: water containing 0.1% trifluoroacetic acid; Solution B: acetonitrile containing 0.1% trifluoroacetic acid
Gradient cycle: 0.00 min (Solution A/Solution B = 90/10), 1.20 min (Solution A/Solution B = 90/10), 5.20 min (Solution A/Solution B = 0/100), 7.00 min (Solution A/Solution B = 0/100), 7.00 min (Solution A/Solution B = 90/10), 8.50 min (Solution A/Solution B = 90/10)
Flow rate: 70 mL/min, detection method: UV 220 nm
3.
Equipment: Preparative purification system by Waters Corporation
Column: YMC CombiPrep ODS-A, S-5 μm, 50 × 20 mm
Solvent: Solution A: water containing 0.1% trifluoroacetic acid; Solution B: acetonitrile containing 0.1% trifluoroacetic acid
Gradient cycle: 0.00 min (Solution A/Solution B = 90/10), 0.20 min (Solution A/Solution B = 90/10), 4.20 min (Solution A/Solution B = 0/100), 6.30 min (Solution A/Solution B = 0/100), 6.30 min (Solution A/Solution B = 90/10), 7.50 min (Solution A/Solution B = 90/10)
Flow rate: 25 mL/min, detection method: UV 220 nm
4.
Equipment: High throughput purification system by Gilson
Column: CAPCELL PAK C18 UG-120, S-5 μm, 20 × 50 mm or YMC CombiPrep Hydrosphere C18 HS-340-CC, S-5 μm, 20 × 50 mm (Shiseido)
Solvent: Solution A: water containing 0.1% trifluoroacetic acid; Solution B: acetonitrile containing 0.1% trifluoroacetic acid
Gradient cycle: 0.00 min (Solution A/Solution B = 95/5), 1.10 min (Solution A/Solution B = 95/95), 5.00 min (Solution

A/Solution B = 0/100), 6.40 min (Solution A/Solution B = 0/100), 6.50 min (Solution A/Solution B = 95/5)
Flow rate: 20 mL/min, detection method: UV 220 nm

5.

Equipment: High throughput purification system by Gilson
Column: YMC CombiPrep, Pro C18 RS, S-5 μm, 20 × 50 mm (YMC)
Solvent: Solution A: water containing 10 mM ammonium carbonate; Solution B: acetonitrile Gradient cycle: 0.00 min (Solution A/Solution B = 95/5), 1.10 min (Solution A/Solution B = 95/5), 4.60 min (Solution A/Solution B = 0/100), 6.40 min (Solution A/Solution B = 0/100), 6.50 min (Solution A/Solution B = 95/5), 6.60 min (Solution A/Solution B = 95/5)
Injected amount: 1000 μL; flow rate: 25 mL/min; detection method: UV 220 nm, 254 nm

6.

Equipment: Purification system by Gilson
Column: Welchrom XB-C18, 5 μm, 150 × 20 mm
Solvent: Solution A: acetonitrile containing 0.1% trifluoroacetic acid; Solution B: water containing 0.1% trifluoroacetic acid
Gradient cycle: 0.00 min (Solution A/Solution B=10/90), 5.00 min (Solution A/Solution B=10/90), 20.00 min (Solution A/Solution B=70/30), 25.00 min (Solution A/Solution B=70/30), 30.00 min (Solution A/Solution B=10/90); or 0.00 min (Solution A/Solution B=10/90), 5.00 min (Solution A/Solution B=10/90), 20.00 min (Solution A/Solution B=80/20), 25.00 min (Solution A/Solution B=80/20), 30.00 min (Solution A/Solution B=10/90); etc.
Flow rate: 25 mL/min, detection method: UV 220 nm
The eluate obtained by purification by preparative HPLC may be concentrated at reduced pressure after the removal of the trifluoroacetic acid through a PL-$HCO_3$ MP solid phase elution column by Polymer Laboratory.

Reference Example 1

Methyl 4-[3-(trifluoromethyl)-4,5,6,7-tetrahydro-1H-indazol-1-yl]butanoate

To a mixture of 3-(trifluoromethyl)-4,5,6,7-tetrahydro-1H-indazole (1.9 g, 10 mmol), potassium tert-butoxide (1.34 g, 12 mmol), and DMF (50 ml), was added methyl 4-bromobutanoate (2.17 g, 12 mmol) at 0 °C, and the mixture was stirred at room temperature for 13 hours. To the rea ction mixture, was added water, and the mixture was extracted with ethyl acetate. The organi c layer was washed with water, and brine, dried over magnesium sulfate, and concentrated un der reduced pressure. The obtained residue was purified by column chromatography on silica gel [developing solvent: hexane-ethyl acetate (5:2)] to give the titled compound (2.2 g) as a colo rless oil (yield 76%).
MS (ESI+);291 (M+H)

Reference Example 2

4-[3-(Trifluoromethyl)-4,5,6,7-tetrahydro-1H-indazol-1-yl]butanoic acid

A solution of methyl 4-[3-(trifluoromethyl)-4,5,6,7-tetrahydro-1H-indazol-1-yl]butanoate obtai ned in Reference Example 1 (2.2 g, 7.58 mmol), and 1N aqueous sodium hydroxide (23 ml) in a mixture of methanol (15 ml) and THF (15 ml) was stirred at room temperature for 1 hour. Th e reaction mixture was concentrated under reduced pressure, acidified with 1 N hydrochloride acid, and extracted with ethyl acetate. The organic layer was washed with water, and brine, d ried over magnesium sulfate, and concentrated under reduced pressure. The obtained residue was crystallized from hexane to give the titled compound (1.1 g) as white crystals (yield 53%). MS (ESI+);277 (M+H)

Reference Example 3

Methyl 2-methyl-4-[3-(trifluoromethyl)-4,5,6,7-tetrahydro-1H-indazol-1-yl]butanoate

To a mixture of 3-(trifluoromethyl)-4,5,6,7-tetrahydro-1H-indazole (1.9 g, 10 mmol), potassium tert-butoxide (1.34 g, 12 mmol), potassium iodide (1.66 g, 10 mmol), and DMF (50 ml), was ad ded methyl 4-bromo-2-methylbutanoate (2.25 g, 15 mmol) at 0 °C, and then the mixture was st irred at room temperature for 13 hours. To the reaction mixture, was added water, and the mi xture was extracted with ethyl acetate. The organic layer was washed with water, and brine, dried over magnesium sulfate, and concentrated under reduced pressure. The obtained residu e was purified by column chromatography on silica gel [basic silica gel, developing solvent: he xane-THF (6:1)] to give the titled compound (1.4 g) as a colorless oil (yield 46%).
MS (ESI+);305 (M+H)

Reference Example 4

2-Methyl-4-[3-(trifluoromethyl)-4,5,6,7-tetrahydro-1H-indazol-1-yl]butanoic acid A solution of methyl 2-methyl-4-[3-(trifluoromethyl)-4,5,6,7-tetrahydro-1H-indazol-1-yl]butano ate obtained in Reference Example 3 (1.4 g, 4.6 mmol) and 1N aqueous sodium hydroxide (14 ml) in a mixture of methanol (10 ml) and THF (10 ml) was stirred at room temperature for 1 h our. The reaction mixture was concentrated under reduced pressure, acidified with 1 N hydroc hloride acid, and extracted with ethyl acetate. The organic layer was washed with water, and saturated saline, dried over magnesium sulfate, and concentrated under reduced pressure. Th e obtained residue was crystallized from hexane to give the titled compound(1.0 g) as white cr ystals (yield 75%).
MS (ESI+);291 (M+H)

Reference Example 5

Methyl 5-[3-(trifluoromethyl)-4,5,6,7-tetrahydro-1H-indazol-1-yl]pentanoate

To a mixture of 3-(trifluoromethyl)-4,5,6,7-tetrahydro-1H-indazole (1.9 g, 10 mmol), potassium tert-butoxide (1.34 g, 12 mmol), and DMF (50 ml), was added methyl 5-bromopentanoate (2.3 4 g, 12 mmol) at 0 °C, and then the mixture was stirred at room temperature for 13 hours. To the reaction mixture, was added water, and the mixture was extracted with ethyl acetate. The organic layer was washed with water, and brine, dried over magnesium sulfate, and concentr ated under reduced pressure. The obtained residue was purified by column chromatography o n silica gel [developing solvent: hexane-ethyl acetate (5:2)] to give the titled compound (2.1 g) as a colorless oil (yield 69%).
MS (ESI+);305 (M+H)

Reference Example 6

5-[3-(Trifluoromethyl)-4,5,6,7-tetrahydro-1H-indazol-1-yl]pentanoic acid

A solution of methyl 5-[3-(trifluoromethyl)-4,5,6,7-tetrahydro-1H-indazol-1-yl]pentanoate obta ined in Reference Example 5 (2.1 g, 6.9 mmol), and 1N aqueous sodium hydroxide (20 ml) in a mixed solution of methanol (15 ml) and THF (15 ml) was stirred at room temperature for 1 ho ur. The reaction mixture was concentrated under reduced pressure, acidified with 1 N hydroc hloride acid, and extracted with ethyl acetate. The organic layer was washed with water, and saturated saline, dried over magnesium sulfate, and concentrated under reduced pressure. Th e obtained residue was crystallized from hexane, and recrystallized from hexane-diisopropyl e ther to give the titled compound (0.87 g) as white crystals (yield 44%).
MS (ESI+);291 (M+H)

Reference Example 7

tert-Butyl 3-oxo-4-(trifluoroacetyl)piperidine-1-carboxylate

To a solution of tert-butyl 3-oxopiperidinecarboxylate (20.0 g, 100.5 mmol) in dimethoxyethan e (130 mL), at -78 °C, was added dropwise 1M LHMDS/THF solution (120.6 mL). After 1 hour, ethyl trifluoroacetate (18.6 g, 130.6 mmol) was added dropwise to the mixture. The reaction mixture was stirred for 1 h. The dry ice-bath was removed and further stirred for about 2 hour s. The mixture was poured into aqueous ammonium chloride, and extracted with ethyl acetate . The organic layer was washed with saturated saline, dried over anhydrous sodium sulfate, a nd concentrated under reduced pressure. The residue was purified by silica gel chromatograp hy ($PE/CH_2Cl_2$ = 1:1) to give the titled compound (13.0 g, yield 44%) as a colorless oil. LCMS: m/z = 294 $[M-H]^+$.
$^1H$ NMR (300 MHz, $CDCl_3$) δ ppm 1.48 (s, 9 H), 2.57 (t, J = 5.7 Hz, 2 H), 3.56 (t, J = 5.7 Hz, 2 H), 4.22 (s, 2 H), 14.60 (s, 1 H).

Reference Example 8

tert-Butyl 7a-hydroxy-3-(trifluoromethyl)-1,3a,4,5,7,7a-hexahydro-6H-pyrazolo[3,4-c]pyridine-6-carboxylate

To a solution of tert-butyl 3-oxo-4-(trifluoroacetyl)piperidine-1-carboxylate (13 g, 44 mmol) in ethanol (200 mL), was added hydrazine hydrate (11 g, 220 mmol). The mixture was stirred for 1h at room temperature and then heated to reflux for 2 h. The reaction mixture was concentr ated under reduced pressure, and the residue was purified by silica gel chromatography (PE/E A = 5:1) to give the titled compound (6 g, yield 47%) as a white solid.
MS Calcd.: 309; MS Found: 192 $[M+H-Boc-H_2O]$.

$^1H$ NMR (300 MHz, $CDCl_3$) δ ppm 1.45 (s, 9H), 1.83-1.91 (m, 3H), 2.82-2.91 (m, 1H), 3.08-3.15 (m, 1H), 3.58 (d, J = 15.9 Hz, 1H), 4.28 (d, J = 15.0 Hz, 1H), 4.89 (d, J = 15.9 Hz, 1H), 5.96 (s, 1H).

Reference Example 9

tert-Butyl 1-(2-ethoxy-2-oxoethyl)-3-(trifluoromethyl)-1,4,5,7-tetrahydro-6H-pyrazolo[3,4-c]pyr idine-6-carboxylate

To a stirred mixture of tert-butyl 7a-hydroxy-3-(trifluoromethyl)-1,3a,4,5,7,7a-hexahydro-6H-pyrazolo[3,4-c]pyridine-6-carboxylate (500 mg, 1.72 mmol), potassium carbonate (712 mg, 5.16 mmol), and acetone (50 mL), was added ethyl bromoacetate (344 mg, 2.06 mmol). The reactio n was heated to reflux overnight. The mixture was filtered, and the solvent was evaporated off under reduced pressure to give the crude titled compound.

Reference Example 10

[6-(tert-Butoxycarbonyl)-3-(trifluoromethyl)-4,5,6,7-tetrahydro-1H-pyrazolo[3,4-c]pyridin-1-yl] acetic acid

To a solution of crude tert-butyl 1-(2-ethoxy-2-oxoethyl)-3-(trifluoromethyl)-1,4,5,7-tetrahydro-6H-pyrazolo[3,4-c]pyridine-6-carboxylate (1.72 mmol) obtained in Reference Example 9 in met hanol (10 mL), was added 2N sodium hydroxide solution (10 mL), and the mixture was heated to 40-50 °C overnight. The reaction mixture was cooled to room temperature, methanol was e vaporated off under reduced pressure, water (20 mL) was added thereto, and the mixture was extracted with ethyl acetate 3 times. The aqueous layer was adjusted to pH 4-5 with 6N hydro chloric acid, and extracted with ethyl acetate. The organic layer was washed with saturated s aline, dried over anhydrous sodium sulfate, and concentrated under reduced pressure to give t he titled compound (290 mg, yield 48%) as a white solid.
MS Calcd.: 349; MS Found: 250 [$M^+$+H-Boc].
$^1H$ NMR (300 MHz, $CDCl_3$) δ ppm 1.42 (s, 9H), 2.55-2.58 (m, 2H), 3.52-3.56 (m, 2H), 4.46 (s, 4 H).

Reference Example 11

tert-Butyl 4-oxo-3-(trifluoroacetyl)piperidine-1-carboxylate

To a mixture of tert-butyl 4-oxopiperidinecarboxylate (20.0 g, 100.5 mmol), and dimethoxyeth ane (130 mL) at -78 °C, was added dropwise 1M LHMDS/THF (120.6 mL, 1.2 equiv.). After 1 h our, ethyl trifluoroacetate (18.55 g, 130.65 mmol) was added dropwise to the mixture, the mixt ure was stirred for 1 h. The dry ice-bath was removed and further stirred for about 2 hours. T he reaction mixture was poured into aqueous ammonium chloride, and extracted with ethyl ac etate. The organic layer was washed with saturated saline, dried over anhydrous sodium sulfa te, and concentrated under reduced pressure. The residue was not purified and used next step directly.
LCMS: m/z = 294 $[M-H]^+$.
$^1H$ NMR (300 MHz, $CDCl_3$) δ ppm 1.47 (s, 9 H), 2.59 (t, J = 6.0 Hz, 2 H), 3.63 (t, J = 6.0 Hz, 2 H), 4.35 (s, 2 H), 14.76 (s, 1 H).

Reference Example 12

tert-Butyl 3-(trifluoromethyl)-1,4,6,7-tetrahydro-5H-pyrazolo[4,3-c]pyridine-5-carboxylate

To a mixture of crude tert-butyl 4-oxo-3-(trifluoroacetyl)piperidine-1-carboxylate (100.5 mmol, 1.0 equiv.) obtained in Reference Example 11, and ethanol (200 mL), was added hydrazine hy drate (9.65 g, 301.5 mmol), and the mixture was heated to reflux overnight. The solvent was e vaporated off under reduced pressure, water was added thereto, and the mixture was extracte d with ethyl acetate. The organic layer was washed with saturated saline, dried over anhydro us sodium sulfate, and concentrated under reduced pressure. The obtained residue was crysta llized from diethyl ether, and PE to give crude product (13.2g) as a white solid. The crude prod uct was purified by flash column chromatography on silica gel (PE/EA = 5: 1 to 2:1) to give the titled compound (8.92 g, 30% yield) as a white solid.
MS Calcd.: 291; MS Found: 192 [M+H-Boc].
$^1H$ NMR (300 MHz, $CDCl_3$): δ 1.49 (s, 9H), 2.78 (t, J = 5.4 Hz, 2H), 3.72 (t, J = 5.4 Hz, 2H), 4.5 2 (s, 2H), 11.01 (brs, 1H).

Reference Example 13

tert-Butyl 1-(2-ethoxy-2-oxoethyl)-3-(trifluoromethyl)-1,4,6,7-tetrahydro-5H-pyrazolo[4,3-c]pyr idine-5-carboxylate

To a mixture of tert-butyl 3-(trifluoromethyl)-1,4,6,7-tetrahydro-5H-pyrazolo[4,3-c]pyridine-5-carboxylate (5.0

g, 17.2 mmol), potassium carbonate (7.12 g, 51.6 mmol), and acetone (100 mL) , was added ethyl bromoacetate (4.30 g, 25.2 mmol), and the mixture was heated to reflux over night. The reaction mixture was filtered, and washed twice with acetone. The filtrate was conc entrated under reduced pressure. The residue was not purified and used next step directly.

Reference Example 14

[5-(tert-Butoxycarbonyl)-3-(trifluoromethyl)-4,5,6,7-tetrahydro-1H-pyrazolo[4,3-c]pyridin-1-yl] acetic acid

To a solution of crude tert-butyl 1-(2-ethoxy-2-oxoethyl)-3-(trifluoromethyl)-1,4,6,7-tetrahydro-5H-pyrazolo[4,3-c]pyridine-5-carboxylate obtained in Reference Example 13 (17.2 mmol) in et hanol (50 mL), was added 2N aqueous sodium hydroxide (17.5 mL, 34.4 mmol), and the mixtu re was stirred at 40-50 °C overnight. The reaction mixture was cooled to room temperature, et hanol was evaporated off under reduced pressure, water (20 mL) was added thereto, and the mixture was extracted with ethyl acetate 3 times. The aqueous layer was adjusted to pH 2 wit h 6N hydrochloric acid, extracted with ethyl acetate. The organic layer was washed with satur ated saline, dried over sodium sulfate, and concentrated under reduced pressure to give the tit led compound (2.74 g, yield 46%) as yellow solid.
MS Calcd.: 349; MS Found: 250 [M+H-Boc].
$^1$ H NMR (300 MHz, $CDCl_3$) δ ppm 1.48 (s, 9H), 2.64-2.68 (m, 2H), 3.72-3.74 (m, 2H), 4.49 (s, 2 H), 4.89 (s, 2H).

Reference Example 15

2-[6-Benzyl-3-(trifluoromethyl)-4,5,6,7-tetrahydro-1H-pyrazolo[3,4-c]pyridin-1-yl]-N-(5-chloro-2-methoxyphenyl)acetamide

To a solution of N-(5-chloro-2-methoxyphenyl)-2-[3-(trifluoromethyl)-4,5,6,7-tetrahydro-1H-py razolo[3,4-c]pyridin-1-yl]acetamide (200 mg, 0.52 mmol), and benzaldehyde (550 mg, 5.20 mm ol) in methanol (20 mL) stirred at room temperature, was added sodium cyanoborohydride (16 4 mg, 2.60 mmol). The mixture was stirred at room temperature for 3 hours, and concentrated under reduced pressure. To the residue, was added ice water (20 mL), and the mixture was ex tracted with dichloromethane. The organic layer was washed with saturated saline, dried over anhydrous sodium sulfate, and concentrated under reduced pressure. The obtained solid was washed with diethyl ether to give the titled compound (85 mg, yield 34%) as a white solid. MS Calcd.: 478; MS Found: 479 (M+H).
$^1$ H NMR (400 MHz, $CDCl_3$) δ ppm 2.73-2.78 (m, 4H), 3.55 (s, 2H), 3.74 (s, 2H), 3.81 (s, 3H), 4. 73 (s, 2H), 6.75 (d, J = 8.8 Hz, 1H), 7.02 (dd, J = 8.8, 2.4 Hz, 1H), 7.29-7.34 (m, 5H), 8.37 (d, J = 2.4 Hz, 1H), 8.86 (br s, 1H).

Reference Example 16

2-[5-Benzyl-3-(trifluoromethyl)-4,5,6,7-tetrahydro-1H-pyrazolo[4,3-c]pyridin-1-yl]-N-(5-chloro-2-methoxyphenyl)acetamide

A solution of N-(5-chloro-2-methoxyphenyl)-2-[3-(trifluoromethyl)-4,5,6,7-tetrahydro-1H-pyraz olo[4,3-c]pyridin-1-yl]acetamide (100 mg, 0.26 mmol), benzyl bromide (53 mg, 0.31 mmol), and potassium carbonate (108 mg, 0.78 mmol) in ethanol (20 mL) was heated to reflux overnight. The reaction mixture was concentrated under reduced pressure. The obtained residue was pur ified by column chromatography on silica gel (PE/EA = 3:1) to give the titled compound (80 mg , yield 64%) as a white solid.
MS Calcd.: 478; MS Found: 479 (M+H).
$^1$ H NMR (400 MHz, $CDCl_3$) δ ppm 2.72 (t, J = 5.6 Hz, 2H), 2.80 (t, J = 5.6 Hz, 2H), 3.62 (s, 2H ), 3.74 (s, 2H), 3.80 (s, 3H), 4.82 (s, 2H), 6.75 (d, J = 9.2 Hz, 1H), 7.02 (dd, J = 9.2, 2.8 Hz, 1H), 7.27-7.35 (m, 5H), 8.38 (d, J = 2.8 Hz, 1H), 8.89 (br s, 1H).

Reference Example 17

Diethyl 1H-pyrazole-3,5-dicarboxylate

To a mixture of pyrazole-3,5-dicarboxylic acid (10.0 g, 128 mmol), and ethanol (150 mL), was a dded thionyl chloride (12 mL) at 0 °C. The mixture was stirred at room temperature overnight . The solvent was evaporated off under reduced pressure to give the titled compound (12.0 g, y ield 99%).
$^1$ H NMR (300 MHz, $CDCl_3$) δ ppm 1.39 (t, J = 6.9 Hz, 6H), 4.35 (q, J = 6.9 Hz, 4H), 7.34 (s, 1H ), 11.68 (brs, 1H).

Reference Example 18

Diethyl 1-(4-ethoxy-4-oxobutyl)-1H-pyrazole-3,5-dicarboxylate

To a solution of diethyl 1H-pyrazole-3,5-dicarboxylate (6.08 g, 28.7 mmol) in acetone (200 mL), were added potassium carbonate (11.88 g, 86.1 mmol), and the following ethyl 4-bromobutano ate (4.9 mL, 34.5 mmol), and the mixture was refluxed overnight, cooled down, and then filter ed. The filtrate was concentrated under reduced pressure to give crude titled compound (7.58 g, yield 82%).
$^1$ H NMR (300 MHz, $CDCl_3$) δ ppm 1.24 (t, J = 6.9 Hz, 3H), 1.39 (t, J = 6.9 Hz, 3H), 1.40 (t, J = 6.9 Hz, 3H), 2.16-2.26 (m, 2H), 2.31-2.36 (m, 2H), 4.11 (q, J = 6.9 Hz, 2H), 4.36 (q, J = 6.9 Hz, 2 H), 4.41 (q, J = 6.9 Hz, 2H), 4.70 (t, J = 6.9 Hz, 2H), 7.34 (s, 1H).

Reference Example 19

Diethyl 4-oxo-4,5,6,7-tetrahydropyrazolo[1,5-a]pyridine-2,5-dicarboxylate

To a solution cooled to °C of diethyl 1-(4-ethoxy-4-oxobutyl)-1H-pyrazole-3,5-dicarboxylate (2.0 0 g, 6.13 mmol) in THF (150 mL), was added sodium hydride (60%, in mineral oil, 294 mg, 7.3 6 mmol), and the mixture was stirred at room temperature for 5 hours. The reaction mixture was poured into 1% hydrochloric acid (100 mL), and extracted with ethyl acetate (50 mL). The organic layer was washed with saturated saline, dried over anhydrous sodium sulfate, and co ncentrated under reduced pressure. The obtained residue was purified by flash column chrom atography on silica gel (PE: EA = 5:1) to give the titled compound (900 mg, yield 52%). LCMS: m/z = 281 [M+H].

Reference Example 20

4-Oxo-4,5,6,7-tetrahydropyrazolo[1,5-a]pyridine-2-carboxylic acid

To a stirred solution of diethyl 4-oxo-4,5,6,7-tetrahydropyrazolo[1,5-a]pyridine-2,5-dicarboxyla te (200 mg, 0.71 mmol) in water (10 mL), was added concentrated hydrochloric acid (5 mL). Th e mixture was heated to reflux for 2 hours, cooled to room temperature, and extracted with dic hloromethane (20 mL × 2). The organic layer was washed with saturated saline (30 mL), dried over anhydrous sodium sulfate, and concentrated under reduced pressure to give the crude tit led compound (100 mg, yield 78%) as a yellow solid.
$^1$ H NMR (300 MHz, DMSO-$d_6$) δ ppm 2.29-2.37 (m, 2H), 2.66 (t, J = 6.3 Hz, 2H), 4.39 (t, J = 6. 0 Hz, 2H), 7.77 (s, 1H).

Reference Example 21

3-Nitro-4-oxo-4,5,6,7-tetrahydropyrazolo[1,5-a]pyridine-2-carboxylic acid

To a solution of 4-oxo-4,5,6,7-tetrahydropyrazolo[1,5-a]pyridine-2-carboxylic acid (100 mg, 0.5 6 mmol) in trifluoroacetic acid (3 mL), was added trifluoroacetic acid anhydride (817 mg, 3.89 mmol), and the mixture was cooled to 0 °C, and ammonium nitrate (89 mg, 1.11 mmol) was ad ded thereto. The mixture was stirred at room temperature overnight. The reaction mixture wa s concentrated under reduced pressure to give the crude titled compound. The crude title com pound which was used to the next step without further purification.

Reference Example 22

A solution of crude 3-nitro-4-oxo-4,5,6,7-tetrahydropyrazolo[1,5-a]pyridine-2-carboxylic acid, a nd thionyl chloride obtained in Reference Example 21 (1 mL) in methanol (35 mL) was heated to flux overnight. The reaction mixture was concentrated under reduced pressure, and the resi due was purified by flash column chromatography on silica gel (PE:EA = 10 :1) to give the title d compound (100 mg, yield 75%(2 steps)).
$^1$ H NMR (300 MHz, $CDCl_3$) δ ppm 2.43-2.51 (m, 2H), 2.80 (t, J = 6.3 Hz, 2H), 3.94 (s, 3H), 4.5 0 (t, J = 6.0 Hz, 2H).

Reference Example 23

3-Nitro-4-oxo-4,5,6,7-tetrahydropyrazolo[1,5-a]pyridine-2-carboxamide

To a solution of methyl 3-nitro-4-oxo-4,5,6,7-tetrahydropyrazolo[1,5-a]pyridine-2-carboxylate (600 mg, 2.51 mmol) in dichloromethane (10 mL), was added saturated ammonia/methanol solution (30 mL), and the mixture was

stirred at room temperature overnight. The solvent was concentrated under reduced pressure, and the obtained residue was purified by flash column chromatography on silica gel (dichloromethane: methanol = 20:1) to give the titled compound (430 mg, yield 76%).
$^1$ H NMR (300 MHz, DMSO-$d_6$) δ ppm 2.32-2.41 (m, 2H), 2.71-2.76 (m, 2H), 4.40-4.44 (m, 2H), 7.79 (br s, 1H), 8.09 (br s, 1H).

Reference Example 24

3-Amino-4-oxo-4,5,6,7-tetrahydropyrazolo[1,5-a]pyridine-2-carboxamide To a mixture of 3-nitro-4-oxo-4,5,6,7-tetrahydropyrazolo[1,5-a]pyridine-2-carboxamide (430 m g, 1.92 mmol), and methanol (30 mL)/DCM (0 mL), was added palladium catalyst (10 wt. %, o n activated carbon, 50 mg), and the mixture was stirred under hydrogen atmosphere of 1 atom at room temperature overnight. The reaction mixture was filtered, and the filtrate was conce ntrated under reduced pressure to give the titled compound (310 mg, yield 93%).
LCMS: m/z = 195 [M+H].

Reference Example 25

tert-Butyl (2-bromoethyl)carbamate

2-Bromoethanamine hydrobromate (20.4 g, 0.10 mol), and di-tert-butyl dicarbonate (24.0 g, 0. 11 mol) were added to a solution THF (150 mL), and water (150 mL). Then, sodium hydrogen carbonate (16.8 g, 0.20 mol) was added in portions thereto, and the mixture was stirred at roo m temperature overnight. Ethyl acetate (150 mL) was added thereto, and the organic layer wa s separated. The organic layer was washed with 1N hydrochloric acid (80 mL), and saturated saline (100 mL), and dried over anhydrous sodium sulfate. The solvent was evaporated off und er reduced pressure to give the titled compound (20.0 g, yield 90%).

Reference Example 26

Dimethyl 4-nitro-1H-pyrazole-3,5-dicarboxylate

To a solution of dimethyl 1H-pyrazole-3,5-dicarboxylate (12.5 g, 68 mmol) in trifluoroacetic acid (114 mL), was added trifluoroacetic acid anhydride (95.5 mL), and cooled to 0 °C. Ammonium nitrate (27 g, 340 mmol) was added slowly in four portions thereto, and the mixture was stirred at room temperature overnight. The solvent was evaporated off under reduced pressure, ethyl acetate (200 mL) was added thereto, and the mixture was added to a saturated sodium hydrogen carbonate aqueous solution (100 mL). The organic layer was separated, and dried over anhydrous sodium sulfate. The solvent was evaporated off under reduced pressure, and the residue was purified by flash column chromatography on silica gel (PE/EA = 2:1 to 1:1) to give the titled compound (5.5 g, yield 35%).
LCMS: m/e = 229 (M+H).
$^1$ H NMR (300 MHz, $CDCl_3$) δ ppm 3.97 (s, 6H).

Reference Example 28

Dimethyl 1-{2-[(tert-butoxycarbonyl)amino]ethyl}-4-nitro-1H-pyrazole-3,5-dicarboxylate

A mixture of dimethyl 4-nitro-1H-pyrazole-3,5-dicarboxylate (6.4 g, 28 mmol), tert-butyl (2-bro moethyl)carbamate (6.6 g, 30 mmol), and potassium carbonate (4.7 g, 34 mmol) in DMF (30 m L) was stirred at room temperature for 3 hours. The reaction mixture was diluted with water ( 100 mL), and extracted with ethyl acetate (100 mL × 2). The organic layer was washed with w ater (200 mL × 2), and saturated saline (200 mL), dried over anhydrous sodium sulfate, filtere d, and concentrated under reduced pressure. The obtained residue was purified by flash colum n chromatography on silica gel (dichloromethane) to give the titled compound (4.2 g, yield 81% ).
$^1$ H NMR (300 MHz, $CDCl_3$) δ ppm 1.39 (s, 9H), 3.61-3.63 (m, 2H), 3.94 (s, 6H), 4.70-4.73 (m, 2 H).

Reference Example 29

Methyl 3-nitro-4-oxo-4,5,6,7-tetrahydropyrazolo[1,5-a]pyrazine-2-carboxylate

To a solution of dimethyl 1-{2-[(tert-butoxycarbonyl)amino]ethyl}-4-nitro-1H-pyrazole-3,5-dica rboxylate (1.5 g, 4.0 mmol) in dichloromethane (6 mL), was added trifluoroacetic acid (6 mL), and the mixture was stirred at room temperature for 2 hours. The solution was concentrated under reduced pressure. To the obtained oily substance, was added diethyl ether, and the mix ture was stimulated by ultrasonic. The obtained solid was dissolved in methanol (5 mL). Whil e the mixture was stirred vigorously, triethylamine (2.3 mL) was added thereto. The resulting mixture was stirred for 30 minutes. The precipitate was filtered off, washed with methanol, a nd dried to give the titled compound (500 mg, yield 52%).
LCMS: m/z = 241 (M+H).
$^1$ H NMR (300 MHz, DMSO-$d_6$) δ ppm 3.68-3.73 (m, 2H), 3.83 (s, 3H), 4.44-4.84 (m, 2H), 8.81 ( s, 1H).

Reference Example 30

Methyl 5-methyl-3-nitro-4-oxo-4,5,6,7-tetrahydropyrazolo[1,5-a]pyrazine-2-carboxylate

Sodium hydride (60%, in mineral oil, 438 mg, 11 mmol) was added to a solution of methyl 3-ni tro-4-oxo-4,5,6,7-tetrahydropyrazolo[1,5-a]pyrazine-2-carboxylate (2.4 g, 10 mmol) in DMF (10 mL), at room temperature, and the mixture was stirred for 1 hour. Ethyl iodide (1.7 g, 12 mm ol) was added thereto, and the resulting reaction mixture was stirred for another 1 hour. The reaction mixture was poured into 25% sodium bicarbonate aqueous solution (30 mL), and extr acted with diethyl ether (30 mL), and ethyl acetate (30 mL). The organic extract was washed with saturated saline, dried over anhydrous sodium sulfate, and concentrated under reduced pressure. The obtained yellow solid was purified by flash column chromatography on silica gel (PE:EA = 1:1 to 0:1 ) to give the titled compound (2.0 g, yield 79%).
LCMS: m/z = 255 (M+H).
$^1$ H NMR (300 MHz, $CDCl_3$) δ ppm 3.16 (s, 3H), 3.84-3.88 (m, 2H), 3.94 (s, 3H), 4.49-4.53 (m, 2 H).

Reference Example 31

3-Nitro-4-oxo-4,5,6,7-tetrahydropyrazolo[1,5-a]pyrazine-2-carboxamide

To a solution of methyl 3-nitro-4-oxo-4,5,6,7-tetrahydropyrazolo[1,5-a]pyrazine-2-carboxylate ( 1.20 g, 5.0 mmol) in DMF (30 mL), was added 2.0 M ammonia/ethanol solution (30 mL). The mixture was stirred for 2 days. The solvent was evaporated off under reduced pressure to give the crude titled compound (1.14 g, quant.).
$^1$ H NMR (300 MHz, DMSO-$d_6$) δ ppm 3.67-3.72 (m, 2H), 4.40-4.44 (m, 2H), 7.74 (s, 1H), 8.02 ( s, 1H), 8.73-8.75 (m, 1H).

Reference Example 32

5-Methyl-3-nitro-4-oxo-4,5,6,7-tetrahydropyrazolo[1,5-a]pyrazine-2-carboxamide

To a solution of methyl 5-methyl-3-nitro-4-oxo-4,5,6,7-tetrahydropyrazolo[1,5-a]pyrazine-2-car boxylate (650 mg, 2.6 mmol) in DMF (30 mL), was added 2.0 M ammonia/ethanol solution (30 mL). The mixture was stirred for 2 days. The solvent was evaporated off under reduced pressu re to give the crude titled compound (600 mg).

Reference Example 33

N,5-Dimethyl-3-nitro-4-oxo-4,5,6,7-tetrahydropyrazolo[1,5-a]pyrazine-2-carboxamide

To a solution of methyl 5-methyl-3-nitro-4-oxo-4,5,6,7-tetrahydropyrazolo[1,5-a]pyrazine-2-car boxylate (1.40 g, 5.5 mmol) in DMF (30 mL), was added 33% methylamine/ethanol (30 mL). T he mixture was stirred for 2 days. The solvent was evaporated off under reduced pressure to g ive the crude titled compound (1.10 g, yield 79%).
$^1$ H NMR (300 MHz, DMSO-$d_6$) δ ppm 2.73-2.75 (m, 3H), 3.02 (s, 3H), 3.87-3.91 (m, 2H), 4.48-4 .52 (m, 2H), 8.61-8.64 (m, 1H).

Reference Example 34

3-Amino-4-oxo-4,5,6,7-tetrahydropyrazolo[1,5-a]pyrazine-2-carboxamide

To a mixture of 3-nitro-4-oxo-4,5,6,7-tetrahydropyrazolo[1,5-a]pyrazine-2-carboxamide (1.14 g, 5.1 mmol), and acetic acid (20 mL), was added palladium-activated carbon (10 wt. %, 114 mg), under hydrogen atmosphere, at room temperature, and the mixture was stirred for 48 hours. After removal of the catalyst, filtrate was concentrated under reduced pressure, and recrystall ized from methanol to give the titled compound (630 mg, yield 64%) as a white solid. $^1$ H NMR (300 MHz, DMSO-$d_6$) δ ppm 3.53-3.58 (m, 2H), 4.16-4.19 (m, 2H), 5.21 (s, 2H), 7.13 ( s, 1H), 7.33 (s, 1H), 7.89 (m, 1H).

Reference Example 35

3-Amino-5-methyl-4-oxo-4,5,6,7-tetrahydropyrazolo[1,5-a]pyrazine-2-carboxamide

To a mixture of 5-methyl-3-nitro-4-oxo-4,5,6,7-tetrahydropyrazolo[1,5-a]pyrazine-2-carboxami de (600 mg, 2.5 mmol), and acetic acid (10 mL), was added palladium-activated carbon (10 wt. %, 64 mg), under hydrogen atmosphere, at room temperature, and the mixture was stirred for 48 hours. After removal of the catalyst, filtrate was concentrated under reduced pressure, an d recrystallized from methanol to give the titled compound (450 mg, yield 86%) as a white soli d.
$^1$ H NMR (300 MHz, DMSO-$d_6$) δ ppm 2.97 (s, 3H), 3.70-3.73 (m, 2H), 4.26-4.29 (m, 2H), 5.25 ( s, 2H), 7.15 (s, 1H), 7.36 (s, 1H).

Reference Example 36

3-Amino-N,5-dimethyl-4-oxo-4,5,6,7-tetrahydropyrazolo[1,5-a]pyrazine-2-carboxamide To a mixture of N,5-dimethyl-3-nitro-4-oxo-4,5,6,7-tetrahydropyrazolo[1,5-a]pyrazine-2-carbox amide (1.10 g, 4.3 mmol), and acetic acid (20 mL), was added palladium-activated carbon (10 wt. %, 110 mg), under hydrogen atmosphere, at room temperature, and the mixture stirred for 48 hours. After removal of the catalyst, filtrate was concentrated under reduced pressure, an d recrystallized from methanol to give the titled compound (880 mg, yield 92%) as a white soli d.
$^1$ H NMR (300 MHz, DMSO-$d_6$) δ ppm 2.71-2.73 (m, 3H), 2.96 (s, 3H), 3.69-3.73 (m, 2H), 4.24-4 .28 (m, 2H), 5.23 (s, 2H), 7.95-7.96 (m, 1H).

Reference Example 37

[4-(Bromomethy)phenyl](pyrrolidin-1-yl)methanone

A solution of 4-(bromomethy)benzoic acid (10.0 g, 46.5 mmol), and oxalyl chloride (11.8 g, 93.0 mmol) in ethyl acetate (200 mL) was stirred at 40 °C for 1 hour under nitrogen atmosphere. T he solvent was evaporated off. The residue was dried under reduced pressure to give 4-(bromo methy)benzoyl chloride. A solution of pyrrolidine (4.0 g, 56 mmol) in ethyl acetate (20 mL) was added dropwise to a solution of 4-(bromomethy)benzoyl chloride, and DIEA (7.22 g, 56 mmol) in ethyl acetate (100 mL). The reaction mixture was stirred at room temperature for 2 hours, and 1N hydrochloric acid (100 mL) was added thereto. The organic layer was separated, wash ed with water, and brine, dried over anhydrous sodium sulfate, and concentrated under reduc ed pressure to give the crude titled compound (7.7 g, yield 62%).
MS Calcd.: 267; MS Found: 268(M+H).
$^1$ H NMR (400 MHz, $CDCl_3$)δ ppm 1.85-1.96 (m, 4H), 3.43 (t, J = 6.8 Hz, 2H), 3.65 (t, J = 6.8 H z, 2H), 4.49 (s, 2H), 7.41-7.43 (m, 2H), 7.49-7.53 (m, 2H).

Reference Example 38

[4-(Hydrazinylmethyl)phenyl](pyrrolidin-1-yl)methanone

To a solution of [4-(bromomethy)phenyl](pyrrolidin-1-yl)methanone (1000 mg, 3.73 mmol) in e thanol (20 mL), was added hydrazine hydrate (597 mg, 14.92 mmol). The reaction mixture wa s refluxed under nitrogen atmosphere overnight. After cooling, solvent was evaporated off und er reduced pressure. To the obtained residue, was added DCM, washed with water, and brine, dried over anhydrous sodium sulfate, and concentrated under reduced pressure to give the cru de titled compound (750 mg), which was used to the next step without purification.

Reference Example 39

2-Acetylcyclohexanone

A solution of 1-(cyclohex-1-en-1-yl)pyrrolidine (4.8 g, 32 mmol), and triethylamine (3.5 g, 34.6 mmol) in chloroform (30 mL) was cooled to 0 °C, acetyl chloride (2.75 g, 35 mmol) was added d ropwise thereto. The mixture was allowed to warm to room temperature and stirred overnight The reaction mixture was acidified with 10N hydrochloric acid, and refluxed for 5 hours. The organic layer was separated, and the solvent was evaporated off under reduced pressure, and the residue was purified by column chromatography on silica gel (PE/EA=10:1) to give the titl ed compound (2.15 g, yield 48%).

$^1$ H NMR (400 MHz, $CDCl_3$)δ ppm 1.70-1.78 (m, 4H), 2.05 (s, 3H), 2.31-2.37 (m, 4H), 15.20 (s, 1H).

Reference Example 40

2-Propanoylcyclohexanone

1-(Cyclohex-1-en-1-yl)pyrrolidine (4.8 g, 32 mmol), and triethylamine (3.5 g, 34.6 mmol) in chl oroform (30 mL) was cooled to 0 °C, propanoyl chloride (3.2 g, 35 mmol) was added dropwise t hereto. The mixture was allowed to warm to room temperature and stirred overnight. The rea ction mixture was acidified with 10N hydrochloric acid, and refluxed for 5 hours. The organic l ayer was separated, the solvent was evaporated off under reduced pressure, and the residue w as purified by column chromatography on silica gel (PE/EA=10:1) to give the titled compound ( 1.85 g, yield 35%).

Reference Example 41

2-(2-Methylpropanoyl)cyclohexanone

A solution of 1-(cyclohex-1-en-1-yl)pyrrolidine (7.6 g, 50 mmol), and triethylamine (6.3 g, 62 m mol) in chloroform (50 mL) was cooled to 0 °C, 2-methylpropanoyl chloride (6.3 g, 59 mmol) wa s added dropwise thereto. The mixture was stirred at room temperature overnight. The reacti on mixture was acidified with 10N hydrochloric acid, and then refluxed for 5 hours. The organ ic layer was separated, the solvent was evaporated off under reduced pressure, and the residu e was purified by column chromatography on silica gel (PE/EA=10:1) to give the titled compou nd (1.68 g, yield 20%).
$^1$ H NMR (400 MHz, $CDCl_3$)δ ppm 1.13 (d, J = 6.8 Hz, 6H), 1.68-1.73 (m, 4H), 2.36-2.41 (m, 4H ), 2.87-2.94 (m, 1H), 16.30 (s, 1H).

Reference Example 42

2-Pentanoylcyclohexanone

A solution of 1-(cyclohex-1-en-1-yl)pyrrolidine (7.6 g, 50 mmol), and triethylamine (6.3 g, 62 m mol) in chloroform (50 mL) was cooled to 0 °C, pentanoyl chloride (7.1 g, 59 mmol) was added dropwise thereto. The mixture was stirred at room temperature overnight. The reaction mixtu re was acidified with 10N hydrochloric acid, and refluxed for 5 hours. The organic layer was se parated, the solvent was evaporated off under reduced pressure, and the residue was purified by column chromatography on silica gel (PE/EA=10:1) to give the crude titled compound (1.98 g, yield 22%).

Reference Example 43

2-(3,3,3-Trifluoropropanoyl)cyclohexanone

A solution of 1-(cyclohex-1-en-1-yl)pyrrolidine (4.8 g, 32 mmol), and triethylamine (3.5 g, 34.6 mmol) in chloroform (30 mL) was cooled to 0 °C, 3,3,3-trifluoropropanoyl chloride (5.12 g, 35 m mol) was added dropwise thereto. The mixture was stirred at room temperature overnight. Th e reaction mixture was acidified with 10N hydrochloric acid, and refluxed for 5 hours. The org anic layer was separated, the solvent was evaporated off under reduced pressure, and the resi due was purified by column chromatography on silica gel (PE/EA = 10/1) to give the titled com pound (2.08 g, crude).

Reference Example 44

3-(2,2,2-Trifluoroethyl)-4,5,6,7-tetrahydro-1H-indazole

A solution of 2-(3,3,3-trifluoropropanoyl)cyclohexanone obtained in Reference Example 43 (20 80 mg), hydrazine hydrate (2000 mg, 50.0 mmol), and p-toluenesulfonic acid (100 mg) in tolue ne (110 mL) was stirred at 90 °C for 3 hours. The solvent was concentrated under reduced pre ssure, and the obtained residue was used to the next step without purification.

Reference Example 45

Methyl oxo(2-oxocyclohexyl)acetate

To a solution of sodium ethoxide (3.3 g, 49.0 mmol) in ethanol at 5 °C, was added a mixture of cyclohexanone (4.0 g, 40.8 mmol), and dimethyl oxalate (5.8 g, 49.0 mmol). The mixture was st irred at room temperature for 6 hours. The reaction was quenched by adding water (200 mL), and ethyl acetate (150 mL), aqueous layer was separated, acidified with concentrated hydroch loric acid, and extracted twice with ethyl acetate (150 mL). The organic layer was dried over s odium sulfate, and concentrated under reduced pressure. The obtained residue was purified b y column chromatography on silica gel (EA/PE = 1:5) to give the titled compound (6.8 g, yield 91%).
$^1$ H NMR (400 MHz, $CDCl_3$)δ ppm 1.66-1.76 (m, 4H), 2.45-2.50 (m, 4H), 3.87 (s, 3H), 15.22 (s, 1H).

Reference Example 46

Methyl 4,5,6,7-tetrahydro-1H-indazole-3-carboxylate

To a solution of methyl oxo(2-oxocyclohexyl)acetate (6.8 g, 36.9 mmol) in acetic acid (100 mL), was added hydrazine hydrate (3.69 g, 73.9 mmol). The reaction mixture was refluxed under ni trogen atmosphere for 4 hours. The solvent was concentrated under reduced pressure. To the obtained residue, was added DCM, and the mixture was washed with a saturated sodium hyd rogen carbonate aqueous solution, water, and brine, dried over anhydrous sodium sulfate, and concentrated under reduced pressure. The obtained the crude titled compound (4.2 g, yield 63 %) was used to the next step without purification.
MS Calcd.: 180; MS Found: 181(M+H).
$^1$ H NMR (400 MHz, $CDCl_3$)δ ppm 1.75-1.83 (m, 4H), 2.67-2.76 (m, 4H), 3.69 (s, 3H).

Reference Example 47

tert-Butyl {2-[3-(trifluoromethyl)-4,5,6,7-tetrahydro-1H-indazol-1-yl]ethyl}carbamate

To a mixture of 3-(trifluoromethyl)-4,5,6,7-tetrahydro-1H-indazole (1.0 g, 5.26 mmol), tert-but yl (2-bromoethyl) carbamate (1.41 g, 6.31 mmol), and DMF (8 ml), was added potassium tert-b utoxide (0.7 g, 6.31 mmol) at 0 °C, and then the mixture was stirred at room temperature for 1 3 hours. To the reaction mixture, was added water, and then the mixture was extracted with e thyl acetate. The organic layer was washed with water, and brine, dried over magnesium sulf ate, and concentrated under reduced pressure. The obtained residue was purified by column c hromatography on silica gel [developing solvent: hexane-ethyl acetate (90:10-75:25)], and recr ystallized from ethyl acetate-hexane to give the titled compound (0.67 g) as white crystals (yie ld 38%).
MS (ESI+) :234 (M-Boc+H)
1H NMR (300 MHz, $CDCl_3$) δ ppm 1.43 (9 H, s), 1.69 - 1.87 (4 H, m), 2.52 - 2.62 (4 H, m), 3.53 (2 H, q, J=6.1 Hz), 4.05 - 4.16 (2 H, m), 4.81 (1 H, brs.).

Reference Example 48

2-[3-(Trifluoromethyl)-4,5,6,7-tetrahydro-1H-indazol-1-yl]ethanamine hydrochloride tert-butyl {2-[3-(trifluoromethyl)-4,5,6,7-tetrahydro-1H-indazol-1-yl]ethyl}carbamate (0.67 g, 2.01 mmol), and 4N hydrochloride acid /ethyl acetate solition (2 ml) were stirred at room temp erature for 3 hours. The reaction was stirred for 1 hr at ice-bath temperature, and then the cr ystals were filtered off, and washed with ethyl acetate to give the titled compound (0.24 g) as white crystals (yield 44%).
MS (ESI+) :234 (M+H)
1H NMR (300 MHz, DMSO-$d_6$) δ ppm 1.62 - 1.83 (4 H, m), 2.44 - 2.54 (2 H, m), 2.65 (2 H, t, J= 5.9 Hz), 3.14 - 3.28 (2 H, m), 4.28 (2 H, t, J=6.4 Hz), 8.05 (3 H, brs.).

Reference Example 49

Ethyl 2-methyl-6-(methylsulfanyl)-1H-thieno[3,4-d]imidazole-4-carboxylate

To a solution of ethyl 3,4-diamino-5-(methylsulfanyl)thiophene-2-carboxylate hydrochloride (1 0 g, 37.2 mmol) synthesized according to a method described in the literature (Yakugaku Zass hi, 99 (11), 1081 (1979)), triethylamine (15.6 ml, 111.6 mmol), and THF (100 ml), on ice, was a dded acetyl chloride (2.65 ml, 37.2 mmol) at 0 °C. The mixture

was stirred at room temperatur e for 13 hours. To the reaction mixture, was added water, extracted with ethyl acetate, and th en the organic layer was washed with water, and brine, dried over magnesium sulfate, and co ncentrated under reduced pressure. The obtained residue was purified by column chromatogr aphy on silica gel [developing solvent: hexane-ethyl acetate (60:40-30:70)], and washed with h exane-ethyl acetate. The obtained crystals (5.5 g, 20.0 mmol), and acetic acid (60 ml) were stir red for 5 days while heating to reflux. The reaction mixture was concentrated under reduced p ressure. To the obtained residue, was added a saturated sodium hydrogen carbonate aqueous solution, and the mixture was extracted with ethyl acetate. The organic layer was washed wit h water, and brine, dried over magnesium sulfate, and concentrated under reduced pressure. The obtained residue was purified by column chromatography on silica gel [developing solvent :hexane-ethyl acetate (70:30-40:60)], and washed with hexane to give the titled compound (2.0 g) as white crystals (yield 39%).
MS (ESI+) :257 (M+H)
1H NMR (300 MHz, $CDCl_3$) δ ppm 1.36 (3 H, t, J=7.0 Hz), 2.54 (3 H, s), 2.65 (3 H, s), 4.33 (2 H , q, J=6.9 Hz), 9.70 (1 H, brs.).

Reference Example 50

Ethyl 1,2-dimethyl-6-(methylsulfanyl)-1H-thieno[3,4-d]imidazole-4-carboxylate

To a solution of ethyl 2-methyl-6-(methylsulfanyl)-1H-thieno[3,4-d]imidazole-4-carboxylate (1. 0 g, 3.90 mmol), methyl iodide (0.29 ml, 4.68 mmol), and DMF (10 ml), was added sodium hydr ide (0.17 g, 4.29 mmol) (60% dispersion in oil) at 0 °C. The mixture was stirred at room temper ature for 13 hours. To the reaction mixture, was added water, and the mixture was extracted with ethyl acetate. The organic layer was washed with water, and brine, dried over magnesiu m sulfate, and concentrated under reduced pressure. The obtained residue was purified by col umn chromatography on silica gel [developing solvent: hexane-ethyl acetate (50:50-30:70)], an d the second eluted fraction was recrystallized from ethyl acetate-hexane to give the titled co mpound (0.26 g) as white crystals (yield 25%).
MS (ESI+) :271 (M+H)
1H NMR (300 MHz, $CDCl_3$) δ ppm 1.40 (3 H, t, J=7.0 Hz), 2.52 (3 H, s), 2.53 (3 H, s), 3.82 (3 H , s), 4.42 (2 H, q, J=7.2 Hz).

Reference Example 51

Ethyl 1,2-dimethyl-4-(methylsulfanyl)-1H-thieno[3,4-d]imidazole-6-carboxylate

The first eluted fraction in the column chromatography on silica gel of Reference Example 50 was purified by column chromatography on silica gel [basic silica gel, developing solvent: hexa ne-ethyl acetate (85:15-75:25)], and washed with hexane to give the titled compound (0.32 g) a s white crystals (yield 30%).
MS (ESI+) :271 (M+H)
1H NMR (300 MHz, $CDCl_3$) δ ppm 1.37 (3 H, t, J=7.2 Hz), 2.50 (3 H, s), 2.64 (3 H, s), 4.00 (3 H , s), 4.31 (2 H, q, J=7.2 Hz).

Reference Example 52

1,2-Dimethyl-6-(methylsulfanyl)-1H-thieno[3,4-d]imidazole-4-carboxylic acid A solution of ethyl 1,2-dimethyl-6-(methylsulfanyl)-1H-thieno[3,4-d]imidazole-4-carboxylate (0 .26 g, 0.96 mmol), 2N aqueous sodium hydroxide (1.9 ml), and ethanol (6 ml) was stirred at 40 °C for 13 hours. 1N hydrochloride acid was added to neutralize the reaction mixture, and the mixture was stirred for 1 hour at room temperature. The crystals were filtered off, and washe d with water to give the titled compound (0.16 g) as white crystals (yield 68%).
1H NMR (300 MHz, DMSO-$d_6$) δ ppm 2.45 (3 H, s), 2.52 (3 H, s), 3.77 (3 H, s).

Reference Example 53

1,2-Dimethyl-4-(methylsulfanyl)-1H-thieno[3,4-d]imidazole-6-carboxylic acid

A solution of ethyl 1,2-dimethyl-4-(methylsulfanyl)-1H-thieno[3,4-d]imidazole-6-carboxylate (0 .32 g, 1.18 mmol), 2N aqueous sodium hydroxide (1.78 ml), and ethanol (8 ml) was stirred at 4 0 °C for 13 hours. To the reaction mixture, 1N hydrochloric acid was added to neutralize, and t hen the mixture was stirred for 1 hour at room temperature. The crystals were filtered off, an d washed with water to give the titled compound (0.22 g) as white crystals (yield 79%).
MS (ESI+) :243 (M+H)
1H NMR (300 MHz, DMSO-$d_6$) δ ppm 2.43 (3 H, s), 2.63 (3 H, s), 3.91 (3 H, s), 12.71 (1 H, brs.)

Reference Example 54

Methyl 3-(acetylamino)-4-aminothiophene-2-carboxylate

To a solution of methyl 3-(acetylamino)-4-nitrothiophene-2-carboxylate (1.5 g, 6.14 mmol) synt hesized according to a method described in the literature (Bioorg. Med. Chem. Lett., 1997, 7, 1 733), calcium chlorite (0.38 g, 3.07 mmol), ethanol (30 ml), and water (6 ml), was added iron p owder (1.72 g, 30.7 mmol) at 80 °C. The mixture was stirred for 4 hours, and the insoluble mat erial was removed by filtration, and the filtrate was concentrated under reduced pressure. To the obtained residue, was added ethyl acetate, washed with water, and saturated saline, dried over magnesium sulfate, and concentrated under reduced pressure. The obtained residue was purified by column chromatography on silica gel [developing solvent: hexane-ethyl acetate (70 :30-30:70)], and recrystallized from ethyl acetate-hexane to give the titled compound (0.91 g) a s white crystals (yield 69%).
MS (ESI+) :215 (M+H)
1H NMR (300 MHz, $CDCl_3$) δ ppm 2.25 (3 H, s), 3.86 (3 H, s), 4.50 (2 H, brs.), 6.39 (1 H, s), 9.5 0 (1 H, brs.).

Reference Example 55

Methyl 2-methyl-1H-thieno[3,4-d]imidazole-4-carboxylate

Methyl 3-(acetylamino)-4-aminothiophene-2-carboxylate (0.91 g, 4.25 mmol), and acetic acid (1 5 ml) was stirred for 4 days while heating to reflux. The reaction mixture was concentrated un der reduced pressure. To the obtained residue, was added a saturated sodium hydrogen carbo nate aqueous solution. The mixture was extracted with ethyl acetate. The organic layer was w ashed with water, and brine, dried over magnesium sulfate, and concentrated under reduced pressure. The obtained residue was purified by column chromatography on silica gel [developi ng solvent: hexane-ethyl acetate (60:40-0:100)], and washed with hexane-ethyl acetate to give the titled compound (0.62 g) as white crystals (yield 74%).
MS (ESI+) :197 (M+H)
1H NMR (300 MHz, $CDCl_3$) δ ppm 2.56 (3 H, s), 3.90 (3 H, s), 7.25 (1 H, s), 9.31 (1 H, brs.).

Reference Example 56

Methyl 1,2-dimethyl-1H-thieno[3,4-d]imidazole-4-carboxylate

To a solution of methyl 2-methyl-1H-thieno[3,4-d]imidazole-4-carboxylate (0.6 g, 3.06 mmol), a nd DMF (10 ml), was added sodium hydride (0.13 g, 3.36 mmol) (60% dispersion in oil) at ice-b ath temperature, and the mixture was stirred for 30 minutes. To the mixture, was added met hyl iodide (0.38 ml, 6.12 mmol), the mixture was stirred for 13 hours at room temperature, an d then water was added to the reaction mixture, and the mixture was extracted with ethyl ace tate. The organic layer was washed with water, and brine, dried over magnesium sulfate, and concentrated under reduced pressure. The obtained residue was purified by column chromato graphy on silica gel [basic silica gel, developing solvent: hexane-ethyl acetate (85:15-75:25)], a nd recrystallized from ethyl acetate-hexane to give the titled compound (93 mg) as white cryst als (yield 14%).
MS (ESI+) :211 (M+H)
1H NMR (300 MHz, $CDCl_3$) δ ppm 2.51 (3 H, s), 3.87 (3 H, s), 4.03 (3 H, s), 7.20 (1 H, s).

Reference Example 57

1,2-dimethyl-1H-thieno[3,4-d]imidazole-4-carboxylic acid

A solution of methyl 1,2-dimethyl-1H-thieno[3,4-d]imidazole-4-carboxylate (62 mg, 0.29 mmol) , 2N aqueous sodium hydroxide (0.3 ml), and ethanol (4 ml) was stirred at room temperature f or 13 hours and while heating to reflux for 4 hours. 1N hydrochloric acid was added to neutral ize the reaction mixture, and the mixture was stirred for 1 hour at room temperature. The cry stals were filtered off, and washed with water to give the titled compound (38 mg) as white cry stals (yield 66%).
MS (ESI+) :197 (M+H)
1H NMR (300 MHz, $CDCl_3$) δ ppm 2.43 (3 H, s), 3.94 (3 H, s), 7.47 (1 H, s).

Reference Example 58

N'-Hydroxy-5-methyl-7-(trifluoromethyl)pyrazolo[1,5-a]pyrimidine-3-carboximidamide

To a solution of 5-methyl-7-trifluoromethylpyrazolo[1,5-a]pyrimidine-3-carbonitrile (3.02 g, 13 .4 mmol) in ethanol (150 ml), were added hydroxyamine hydrochloride (1.86 g, 26.7 mmol), an d potassium carbonate (5.56 g, 40.2 mmol), and the mixture was heated to reflux for 3 hours. After cooling, the insoluble material was removed by filtration, and washed with ethanol. The filtrate was concentrated under reduced pressure. The obtained residue was purified by colum n chromatography on silica gel [developing solvent: hexane-ethyl acetate (50:50-20:80)], and t he obtained crystals were washed with diisopropyl ether to give the titled compound (963 mg) as yellow crystals (yield 28%).
MS (ESI+) :260 (M+H).
$^{1}$ H NMR (300 MHz, $CDCl_3$) δ ppm 2.72 (3 H, s), 4.69 (1 H, br. s.), 5.87 (2 H, br. s.), 7.07 (1 H, s ), 8.53 (1 H, s).

Reference Example 59

N'-Hydroxy-5,7-bis(trifluoromethyl)pyrazolo[1,5-a]pyrimidine-3-carboximidamide

Using 5,7-bis(trifluoromethyl)pyrazolo[1,5-a]pyrimidine-3-carbonitrile, the title compound wa s obtained in the same manner as in Reference Example 58.
MS (ESI+) :314 (M+H)

Reference Example 60

N'-Hydroxy-5-phenyl-7-(trifluoromethyl)pyrazolo[1,5-a]pyrimidine-3-carboximidamide

Using 5-phenyl-7-(trifluoromethyl)pyrazolo[1,5-a]pyrimidine-3-carbonitrile, the title compoun d was obtained in the same manner as in Reference Example 58.
MS (ESI+) :322 (M+H)

Reference Example 61

5-tert-Butyl-N'-hydroxy-7-(trifluoromethyl)pyrazolo[1,5-a]pyrimidine-3-carboximidamide Using 5-tert-butyl-7-(trifluoromethyl)pyrazolo[1,5-a]pyrimidine-3-carbonitrile, the title compo und was obtained in the same manner as in Reference Example 58.
MS (ESI+) :302 (M+H)

Reference Example 62

N'-Hydroxy-5,7-dimethylpyrazolo[1,5-a]pyrimidine-3-carboximidamide

Using 5,7-dimethylpyrazolo[1,5-a]pyrimidine-3-carbonitrile, the title compound was obtained i n the same manner as in Reference Example 58.
MS (ESI+) :206 (M+H)

Reference Example 63

N'-Hydroxy-1-benzothiophene-3-carboximidamide

Using 1-benzothlophene-3-carbonitrile, the title compound was obtained in the same manner as in Reference Example 58.
MS (ESI+) :193 (M+H)

Reference Example 64

N'-Hydroxy-1H-pyrrolo[2,3-b]pyrimidine-3-carboximidamide

Using 1H-pyrrolo[2,3-b]pyrimidine-3-carbonitrile, the title compound was obtained in the sam e manner as in Reference Example 58.

MS (ESI+) :177 (M+H)

Reference Example 65

N'-Hydroxy-1H-indole-2-carboximidamide

Using 1H-indole-2-carbonitrile, the title compound was obtained in the same manner as in Ref erence Example 58.
MS (ESI+) :176 (M+H)

Reference Example 66

4-(Dimethylamino)-N'-hydroxybenzenecarboximidamide

Using 4-(dimethylamino)benzonitrile, the title compound was obtained in the same manner as in Reference Example 58.
MS (ESI+) :180 (M+H)

Reference Example 67

N'-Hydroxypyrimidine-2-carboximidamide

Using pyrimidine-2-carbonitrile, the title compound was obtained in the same manner as in R eference Example 58.
MS (ESI+) :139 (M+H)

Reference Example 68

5,7-Dimethylpyrazolo[1,5-a]pyrimidine-3-carbohydrazide

Acetylacetone (250 mg, 2.5 mmol) and methyl 5-amino-1H-pyrazole-4-carboxylate (390 mg, 2.5 mmol) were dissolved in acetic acid (10 mL), and the solution was heated to reflux for 4 hours . The reaction mixture was concentrated under reduced pressure. The obtained solid was was hed with diisopropyl ether to give crude crystals of ethyl 5,7-dimethylpyrazolo[1,5-a]pyrimidin e-3-carboxylate (550 mg). The crude crystals were dissolved in ethanol (50 ml), added hydrazi ne monohydrate (625 mg, 12.5 mmol), and heated to reflux for 16 hours. The reaction mixture was concentrated under reduced pressure. The obtained crude crystals were washed with diet hyl ether to give the titled compound (500 mg) as a colorless solid (yield 97%).
MS (ESI+) :206(M+H)
$^1$ H NMR (400 MHz, $CDCl_3$) δ ppm 2.64 (3 H, s), 2.80 (3 H, s), 4.12 (2 H, br. s.), 6.73 (1 H, s), 8. 62 (1 H, s), 9.16 (1 H, br. s.)

Reference Example 69

Diethyl 3-(bromomethy)-5-(methylsulfanyl)thiophene-2,4-dicarboxylate A solution of diethyl 3-methyl-5-(methylsulfanyl)thiophene-2,4-dicarboxylate (0.50 g, 1.73 mm ol), N-bromosuccinimide (0.40 g, 2.25 mmol) and 2,2'-azobis (isobutyronitrile) (0.028 g, 0.17 m mol) in chlorobenzene (10 ml) was stirred at 100 °C for 3.5 hours. To the reaction solution, was added water, and the mixture was extracted with ethyl acetate. The organic layer was washe d with water, and saturated saline, dried over sodium sulfate, and concentrated under reduce d pressure. The obtained residue was purified by column chromatography on silica gel [develo ping solvent: hexane-ethyl acetate (98:2-95:5)] to give the titled compound (0.46 g) as colorless crystals (yield 72%).
1H NMR (300 MHz, CDCl3) δ ppm 1.40 (3 H, t, J = 7.2 Hz), 1.45 (3 H, t, J = 7.2 Hz), 2.61 (3 H, s), 4.32 - 4.48 (4 H, m), 5.27 (2 H, s)

Reference Example 70

Ethyl 5-methyl-3-(methylsulfanyl)-4-oxo-5,6-dihydro-4H-thieno[3,4-c]pyrrole-1-carboxylate

2.0 M methylamine/tetrahydrofuran solution (34 ml, 68.1 mmol) was diluted with tetrahydrof uran (20 ml) and cooled to 0 °C. To this solution, was added a solution of diethyl 3-(bromometh y)-5-(methylsulfanyl)thiophene-2,4-dicarboxylate (2.50 g, 6.81 mmol) in tetrahydrofuran (5 ml ), and the mixture was stirred at 0 °C for 1 hour and at room temperature overnight. The react ion solution was concentrated under reduced pressure. Ethanol (30 ml) and potassium carbon ate (2.82 g, 20.4 mmol) were added thereto. The mixture was stirred at room temperature over night. The reaction solution was concentrated under reduced pressure. Water was added there to, and the mixture was extracted with ethyl acetate. The organic layer was washed with wate r, and saturated saline, dried over sodium sulfate, and concentrated under reduced pressure. The obtained residue was purified by column chromatography on silica gel [developing solvent : hexane-ethyl acetate (3:1-1:1)]. The obtained solid was washed with ethyl acetate-hexane(9:1 ) to give the titled compound (0.78 g) as colorless crystals (yield 42%).
MS (ESI+) :272 (M+H)
1H NMR (300 MHz, CDCl3) δ ppm 1.37 (3 H, t, J = 7.2 Hz), 2.70 (3 H, s), 3.09 (3 H, s), 4.33 (2 H, q, J = 6.9 Hz), 4.39 (2 H, s)

Reference Example 71

5-Methyl-3-(methylsulfanyl)-4-oxo-5,6-dihydro-4H-thieno[3,4-c]pyrrole-1-carboxylic acid

To a solution of ethyl 5-methyl-3-(methylsulfanyl)-4-oxo-5,6-dihydro-4H-thieno[3,4-c]pyrrole-1-carboxylate (146 mg, 0.538 mmol) in ethanol (1 ml), was added 2N aqueous sodium hydroxid e (0.55 ml), and the mixture was stirred at room temperature for 2 hours. To the reaction mixt ure, was added 2N hydrochloride acid (0.5 ml), ethanol was evaporated off under reduced pres sure. The obtained crystals were washed with water to give the titled compound (136 mg) as c olorless crystals (yield >99%).
MS (ESI+) :244 (M+H).
$^1$ H NMR (300 MHz, DMSO-$d_6$) δ ppm 2.69 (3 H, s), 2.95 (3 H, s), 4.31 - 4.52 (2 H, m), 13.21 (1 H, br. s.).

Reference Example 72

Diethyl 3-(cyanomethyl)-5-(methylsulfanyl)thiophene-2,4-dicarboxylate

To a solution of diethyl 3-(bromomethy)-5-(methylsulfanyl)thiophene-2,4-dicarboxylate (0.19 g , 0.52 mmol) is ethanol (2 ml), a solution of potassium cyanide (51 mg, 0.78 mmol) in water (0. 1 ml) was added, and the mixture was heated to reflux overnight. The reaction mixture was co ncentrated under reduced pressure, water was added thereto, and the mixture was extracted with ethyl acetate. The organic layer was washed with water, and saturated saline, dried over sodium sulfate, and concentrated under reduced pressure. The obtained residue was purified by column chromatography on silica gel [developing solvent: hexane-ethyl acetate (9:1-4:1)] to give the titled compound (81 mg) as colorless crystals (yield 50%).
1H NMR (300 MHz, $CDCl_3$) δ ppm 1.40 (3 H, t, J = 7.2 Hz), 1.46 (3 H, t, J=7.2 Hz), 2.62 (3 H, s ), 4.32 - 4.48 (4 H, m), 4.53 (2 H, s)

Reference Example 73

Ethyl 7-oxo-4,5,6,7-tetrahydrothieno[2,3-c]pyridine-3-carboxylate

A mixture of diethyl 3-(cyanomethyl)-5-(methylsulfanyl)thiophene-2,4-dicarboxylate (4.97 g, 1 5.9 mmol), 2.0 M ammonia/ethanol solution (31 ml, 63.4 mmol), Raney cobalt (60 g) and ethan ol (50 ml) was stirred under hydrogen atmosphere (1 atm) at room temperature overnight. Th e reaction solution was filtered, and the filtrate was concentrated under reduced pressure. Th e residue was purified by column chromatography on basic silica gel [developing solvent: tetra hydrofuran], and following column chromatography on silica gel [developing solvent: hexane-e thyl acetate (9:1-7: 3)] to give the titled compound (1.21 g) as colorless crystals (yield 34%). MS (ESI+) :226 (M+H)
$^1$ H NMR (300 MHz, $CDCl_3$) δ ppm 1.38 (3 H, t, J = 7.2 Hz), 3.26 (2 H, t, J = 7.0 Hz), 3.65 (2 H, td, J = 7.0, 2.6 Hz), 4.34 (2 H, q, J = 7.2 Hz), 6.08 (1 H, br. s.), 8.28 (1 H, s)

Reference Example 74

Ethyl 6-methyl-7-oxo-4,5,6,7-tetrahydrothieno[2,3-c]pyridine-3-carboxylate

To a solution of ethyl 7-oxo-4,5,6,7-tetrahydrothieno[2,3-c]pyridine-3-carboxylate (1.15 g, 5.10 mmol), methyl iodide (0.40 ml, 6.36 mmol) in N,N-dimethylformamide (10 ml), was added sodi um hydride (0.20 g, 5.09 mmol) (60% dispersion in oil), and the mixture was stirred at room te mperature overnight. To the mixed solution, methyl iodide(0.13 ml, 2.09 mmol), and sodium h ydride (50 mg, 5.09 mmol) (60% dispersion in oil) were further added, and the mixture

was sti rred at room temperature for 1 hour. To the solution, was added saturated aqueous ammoniu m chloride, and the mixture was extracted with ethyl acetate. The organic layer was washed with water, and saturated saline, dried over sodium sulfate, and concentrated under reduced pressure. The obtained residue was purified by column chromatography on silica gel [developi ng solvent: hexane-ethyl acetate (9:1-3:2)] to give the titled compound (1.00 g) as colorless crys tals (yield 82%).
$^1H$ NMR (300 MHz, $CDCl_3$) δ ppm 1.38 (3 H, t, J=7.2 Hz), 3.11 (3 H, s), 3.27 (2 H, t, J=7.1 Hz), 3.63 (2 H, t, J=7.1 Hz), 4.33 (2 H, q, J=7.2 Hz), 8.22 (1 H, s)

Reference Example 75

6-Methyl-7-oxo-4,5,6,7-tetrahydrothieno[2,3-c]pyridine-3-carboxylic acid

To a solution of ethyl 6-methyl-7-oxo-4,5,6,7-tetrahydrothieno[2,3-c]pyridine-3-carboxylate (17 6 mg, 0.736 mmol) in ethanol (1 ml), was added 2N aqueous sodium hydroxide (0.75 ml), and t he mixture was stirred at room temperature for 2 hours. To the reaction mixture, was added 2 N hydrochloride acid (0.75 ml). Ethanol was evaporated off under reduced pressure. The obtai ned crystals were washed with water to give the titled compound (132 mg) as colorless crystal s (yield 85%).
MS (ESI+) :212 (M+H).
$^1H$ NMR (300 MHz, DMSO-$d_6$) δ ppm 2.96 (3 H, s), 3.09 - 3.18 (2 H, m), 3.60 (2 H, t, J=7.0 Hz) , 8.44 (1 H, s), 12.98 (1 H, br. s.).

Reference Example 76

Butyl 2-amino-6-(trifluoromethyl)pyridine-3-carboxylate

A mixture of 2-chloro-6-(trifluoromethyl)pyridine-3-carboxylic acid (3.95 g, 17.5 mmol), and 8 N ammonia/ methanol solution (30 mL) was stirred at 120 °C for 24 hours. After cooling, the re action mixture was concentrated under reduced pressure to give 2-amino-6-(trifluoromethyl)p yridine-3-carboxylic acid. To n-butanol (45 ml), were added thionyl chloride (4.6 ml, 63.0 mmol ) dropwise at -78 °C, and following the obtained 2-amino-6-(trifluoromethyl)pyridine-3-carboxy lic acid. The mixture was stirred for 3 hours while heating gradually to room temperature, an d then stirred at 50 °C for 36 hours, and at 65 °C for 48 hours. After cooling, the reaction mixt ure was poured into a saturated sodium hydrogen carbonate aqueous solution, and extracted with ethyl acetate. The organic layer was washed with saturated saline, dried over sodium sul fate, and concentrated under reduced pressure. The obtained residue was purified by column c hromatography on silica gel [developing solvent: hexane-ethyl acetate (98:2-90:10)] to give the titled compound (4.00 g) as colorless crystals (yield 87%).
MS (ESI+) :263 (M+H).
$^1H$ NMR (300 MHz, $CDCl_3$) δ ppm 0.91 - 1.04 (3 H, m), 1.37 - 1.57 (2 H, m), 1.68 - 1.87 (2 H, m ), 4.32 (1.4 H, t, J=6.6 Hz), 4.40 (0.6 H, t, J=6.6 Hz), 6.96 (0.7 H, d, J=7.9 Hz), 7.70 (0.3 H, d, J =7.9 Hz), 8.23 - 8.36 (1 H, m).

Reference Example 77

2-{2-[3-(Trifluoromethyl)-4,5,6,7-tetrahydro-1H-indazol-1-yl]ethyl}-1H-isoindole-1,3(2H)-dione To a solution of 3-(trifluoromethyl)-4,5,6,7-tetrahydro-1H-indazole (35 mg, 0.130 mmol) in DM F (50 ml), was added 2-(2-bromoethyl)-1H-isoindole-1,3(2H)-dione (7.94 g, 31.3 mmol), and pot assium tert-butoxide (3.51 g, 31.3 mmol), and the mixture was stirred at room temperature fo r 15 hours . To the reaction mixture, was added water, and the mixture was extracted with et hyl acetate. The organic layer was washed with water, and saturated saline, dried over sodiu m sulfate, and concentrated under reduced pressure. The obtained residue was purified by col umn chromatography on silica gel [developing solvent: hexane-ethyl acetate (98:2-87:13)] to gi ve the titled compound (3.79 g) as colorless crystals (yield 40%).
MS (ESI+) :364 (M+H).
$^1H$ NMR (300 MHz, $CDCl_3$) δ ppm 1.64 - 1.88 (4 H, m), 2.47 - 2.69 (4 H, m), 3.97 - 4.12 (2 H, m ), 4.30 (2 H, t, J=6.2 Hz), 7.68 - 7.76 (2 H, m), 7.78 - 7.89 (2 H, m).

Reference Example 78

2-Bromo-4-chloro-1-[(2-methyl-2-propen-1-yl)oxy]benzene

To a solution of 2-bromo-4-chlorophenol (25.0 g, 121 mmol) in DMF (150 ml), was added potas sium carbonate

(18.4 g, 133 mmol), and 3-chloro-2-methyl-1-propene (13.1 ml, 133 mmol), at r oom temperature for 1 hour, and the mixture was stirred at 50 °C for 4 hours. After cooling, to the reaction mixture, was added water, and the mixture was extracted with ethyl acetate. Th e organic layer was washed with 0.5N aqueous sodium hydroxide, water, and saturated saline , dried over sodium sulfate, and concentrated under reduced pressure to give the titled compo und (31.1 g) as an oily substance (yield 98%).
MS (ESI+) :261 (M+H).
$^1$ H NMR (300 MHz, $CDCl_3$) δ ppm 1.84 (3 H, s), 4.48 (2 H, s), 5.02 (1 H, s), 5.14 (1 H, s), 6.80 ( 1 H, d, J=8.9 Hz), 7.20 (1 H, dd, J=8.8, 2.5 Hz), 7.54 (1 H, d, J=2.4 Hz).

Reference Example 79

2-Bromo-4-chloro-6-(2-methyl-2-propen-1-yl)phenol

A solution of 2-bromo-4-chloro-1-[(2-methyl-2-propen-1-yl)oxy]benzene (5.34 g, 20.4 mmol) in N,N-diethylaniline (15 ml) was stirred at 195 °C for 8 hours. After cooling, to the reaction mixt ure, was added 1N hydrochloride acid. The mixture was extracted with ethyl acetate. The org anic layer was washed with 1N hydrochloric acid, water, and saturated saline, dried over sodi um sulfate, and concentrated under reduced pressure. The obtained residue was purified by co lumn chromatography on silica gel [developing solvent: hexane-ethyl acetate (100:0-98:2)] to g ive the titled compound (4.32 g) as an oily substance (yield 81%).
$^1$ H NMR (300 MHz, $CDCl_3$) δ ppm 1.73 (3 H, s), 3.36 (2 H, s), 4.73 (1 H, s), 4.88 (1 H, s), 5.58 ( 1 H, s), 7.06 (1 H, d, J=2.3 Hz), 7.34 (1 H, d, J=2.4 Hz).

Reference Example 80

7-Bromo-5-chloro-2,2-dimethyl-2,3-dihydro-1-benzofuran

To a solution of 2-bromo-4-chloro-6-(2-methyl-2-propen-1-yl)phenol (1.05 g, 3.97 mmol) in tolu ene (8 ml), was added boron trifluoride - diethyl ether complex (0.55 ml, 4.37 mmol), and the mixture was stirred at 75 °C for 2 hours. After cooling, to the reaction mixture, was added wat er, and the mixture was extracted with ethyl acetate. The organic layer was washed with 0.5N aqueous sodium hydroxide, water, and saturated saline, dried over sodium sulfate, and conce ntrated under reduced pressure to give the titled compound (985 mg) as an oily substance (yie ld 95%).
MS (ESI+) :261 (M+H).
$^1$ H NMR (300 MHz, $CDCl_3$) δ ppm 1.51 (6 H, s), 3.08 (2 H, s), 7.02 - 7.04 (1 H, m), 7.24 - 7.28 ( 1 H, m).

Reference Example 81

5-Chloro-N-(diphenylmethylidene)-2,2-dimethyl-2,3-dihydro-1-benzofuran-7-amine

A mixture of 7-bromo-5-chloro-2,2-dimethyl-2,3-dihydro-1-benzofuran (510 mg, 1.95 mmol), be nzophenonimine (0.45 ml, 2.92 mmol), palladium (II) acetate (22 mg, 0.0975 mmol), (+/-)-2,2'-b is(diphenylphosphino)-1,1'-binaphthyl (182 mg, 0.293 mmol), sodium tert-butoxide (244 mg, 2. 54 mmol), and toluene (5 ml) was stirred at 100 °C for 4 hours. After cooling, to the reaction m ixture, was added water, and the mixture was extracted with ethyl acetate. The organic layer was washed with saturated saline, dried over sodium sulfate, and concentrated under reduced pressure. The obtained residue was purified by column chromatography on silica gel [developi ng solvent: hexane-ethyl acetate (100:0-97:3)] to give the titled compound (740 mg) as an oily s ubstance (yield >99%).
MS (ESI+) :362 (M+H).
$^1$ H NMR (300 MHz, $CDCl_3$) δ ppm 1.26 (6 H, s), 2.84 (2 H, s), 6.57 (1 H, d, J=2.1 Hz), 6.69 (1 H , d, J=2.1 Hz), 7.09 - 7.33 (2 H, m), 7.33 - 7.65 (5 H, m), 7.68 - 7.87 (3 H, m).

Reference Example 82

5-Chloro-2,2-dimethyl-2,3-dihydro-1-benzofuran-7-amine

To a solution of 5-chloro-N-(diphenylmethylidene)-2,2-dimethyl-2,3-dihydro-1-benzofuran-7-a mine (700 mg, 1.93 mmol) in THF (8 ml), was added 1N hydrochloride acid (3 ml), and the mix ture was at room temperature for 15 hours. THF was evaporated off under reduced pressure. To the obtained residue, was added 1N hydrochloric acid, and washed with hexane. The aqueo us layer was neutralized with 8N aqueous sodium hydroxide, and extracted with ethyl acetate . The organic layer was washed with saturated saline, dried over sodium sulfate, and concentr ated under

reduced pressure to give the titled compound (369 mg) as an oily substance (yield 9 7%).
MS (ESI+) :198 (M+H).
1H NMR (200 MHz, $CDCl_3$) δ ppm 1.47 (6 H, s) 2.96 (2 H, s) 3.57 (2H, br s), 6.51-6.55 (2H, m).

Reference Example 83

1-[2-(2,3-Dihydro-1H-indol-1-yl)-2-oxoethyl]-3-(trifluoromethyl)-4,5,6,7-tetrahydro-1H-indazol e

The title compound was obtained in the same manner as in Example 3.
Yield: 13.7 mg
MS (ESI+) :350 (M+H)

Reference Example 84

6,7-dimethoxy-1-methyl-2-{[3-(trifluoromethyl)-4,5,6,7-tetrahydro-1H-indazol-1-yl]acetyl}-1,2, 3,4-tetrahydroisoquinoline

The title compound was obtained in the same manner as in Example 3.
Yield: 13.1 mg
MS (ESI+) :438 (M+H)

Reference Example 85

1-{[3-(Trifluoromethyl)-4,5,6,7-tetrahydro-1H-indazol-1-yl]acetyl}-1,2-dihydro-3H-indazol-3-on e

The title compound was obtained in the same manner as in Example 3.
Yield: 7.9 mg
MS (ESI+) :365 (M+H)

Example 1

Methyl 3-{[3-(trifluoromethyl)-4,5,6,7-tetrahydro-1H-indazol-1-yl]methyl}benzoate

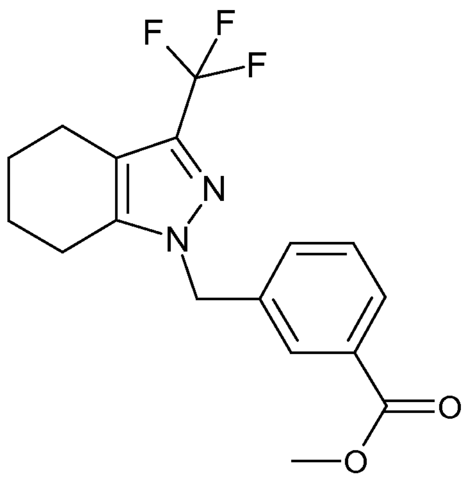

To a mixture of 3-(trifluoromethyl)-4,5,6,7-tetrahydro-1H-indazole (3.8 g, 20 mmol), potassium tert-butoxide (2.69 g, 24 mmol), and DMF (100 ml), was added methyl 3-(bromomethy)benzoa te (5.5 g, 24 mmol) at 0 °C, and then the mixture was stirred at room temperature for 13 hours . To the reaction mixture, was added water, and the mixture was extracted with ethyl acetate. The organic layer was washed with water, and brine, dried over magnesium sulfate, and conc entrated under reduced pressure. The obtained residue was purified by column chromatograp hy on silica gel [basic silica gel, developing solvent: hexane-THF (5:1-3:1)] to give the titled co mpound (2.3 g) as a colorless oil (yield 34%).
MS (ESI+): 339 (M+H)

Example 2

3-{[3-(Trifluoromethyl)-4,5,6,7-tetrahydro-1H-indazol-1-yl]methyl}benzoic acid

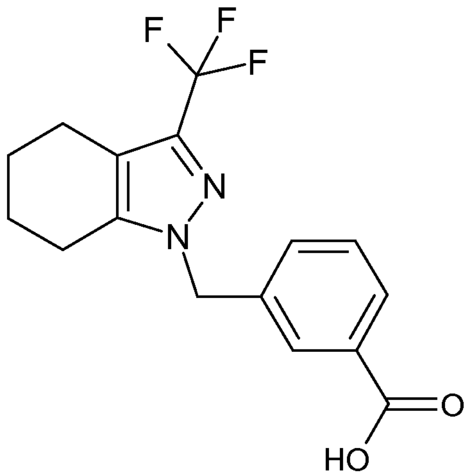

A solution of methyl 3-{[3-(trifluoromethyl)-4,5,6,7-tetrahydro-1H-indazol-1-yl]methyl}benzoat e obtained in Example 1 (2.36 g, 6.98 mmol), and 1N aqueous sodium hydroxide (20 ml) in a m ixture of ethanol (20 ml) and THF (20 ml) was stirred at room temperature for 1 hour. The rea ction mixture was concentrated, acidified with 1 N hydrochloric acid, and extracted with ethyl acetate. The organic layer was washed with water, and brine, dried over magnesium sulfate, and concentrated under reduced pressure. The obtained residue was crystallized from hexane to give the titled compound (1.95 g) as white crystals (yield 86%).
MS (ESI+):325 (M+H)

Example 3

N-(3-Chlorophenyl)-3-{[3-(trifluoromethyl)-4,5,6,7-tetrahydro-1H-indazol-1-yl]methyl}benzami de

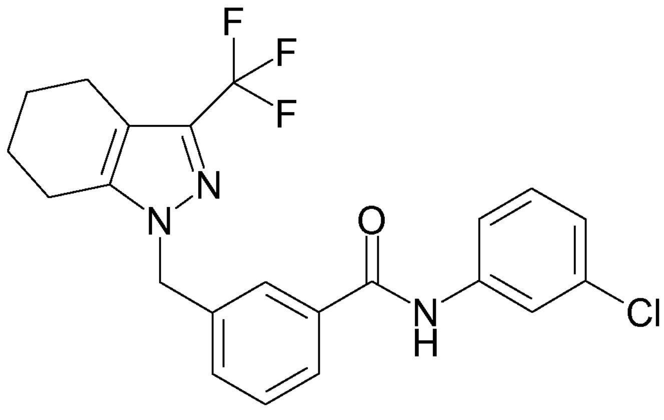

A 0.12 M solution of 3-{[3-(trifluoromethyl)-4,5,6,7-tetrahydro-1H-indazol-1-yl]methyl}benzoic acid obtained in Example 2 in DMF (500 μl, 60 μmol), a 0.144 M solution of 3-chloroaniline in DMF (500 μl, 72 μmol), and a 0.24 M solution of HATU and DIEA in DMF (500 μl, 120 μmol) were mixed at room temperature, and the mixture was stirred at 60 °C for 24 hours. The react ion mixture was cooled to room temperature, ethyl acetate(3 ml), and 2% sodium hydrogen car bonate aqueous solution (1.5 ml) was added thereto, and the mixture was extracted, and the o rganic layer was collected with upper phase sep tube (Wako Pure Chemical Industries). The s olvent was evaporated off under reduced pressure.

The residue was dissolved in DMSO-metha not(1:1) (1 ml), and purified with preparative HPLC to give the titled compound.
Yield: 6.4 mg
MS(ESI+):434(M+H)

Example 4

Methyl 2-{[3-(trifluoromethyl)-4,5,6,7-tetrahydro-1H-indazol-1-yl]methyl}benzoate

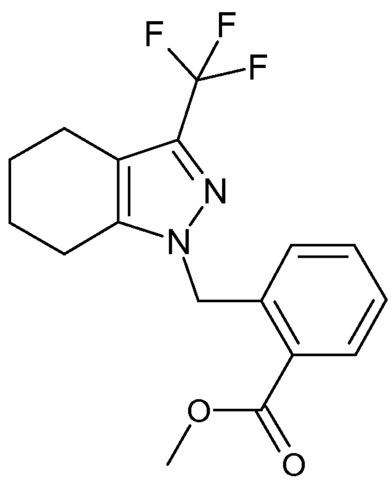

To 3-(trifluoromethyl)-4,5,6,7-tetrahydro-1H-indazole (1.9 g, 10 mmol), potassium tert-butoxid e (1.34 g, 12 mmol), potassium iodide (1.66 g, 10 mmol), and DMF (50 ml), was added methyl 2-(bromomethy)benzoate (2.76 g, 15 mmol) at 0 °C, and then the mixture was stirred at room t emperature for 13 hours. To the reaction mixture, was added water, and the mixture was extr acted with ethyl acetate. The organic layer was washed with water, and brine, dried over mag nesium sulfate, and concentrated under reduced pressure. The obtained residue was purified by column chromatography on silica gel [basic silica gel, developing solvent: hexane-THF (6:1) ] to give the titled compound (1.66 g) as a colorless oil (yield 49%).
MS (ESI+):339 (M+H)

Example 5

2-{[3-(Trifluoromethyl)-4,5,6,7-tetrahydro-1H-indazol-1-yl]methyl}benzoic acid

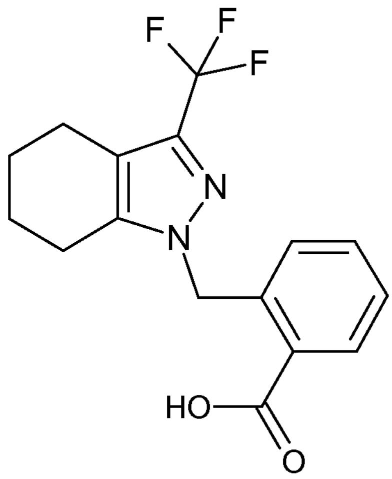

To a solution of methyl methyl 2-{[3-(trifluoromethyl)-4,5,6,7-tetrahydro-1H-indazol-1-yl]meth yl}benzoate (1.66 g, 4.91 mmol) obtained in Example 4, and 1N aqueous sodium hydroxide (15 ml) in a mixture of methanol (10 ml) and THF (10 ml) was stirred at room temperature for 1 h our. The reaction mixture was concentrated, acidified with 1 N hydrochloric acid, and extracte d with ethyl acetate. The organic layer was washed with water, and brine, dried over magnesi um sulfate, and concentrated under reduced pressure. The obtained residue was crystallized f rom hexane to give the titled compound (1.15 g) as white crystals (yield 72%).
MS (ESI+):325 (M+H)

Example 6

N-Pyridin-4-yl-3-{[3-(trifluoromethyl)-4,5,6,7-tetrahydro-1H-indazol-1-yl]methyl}benzamide

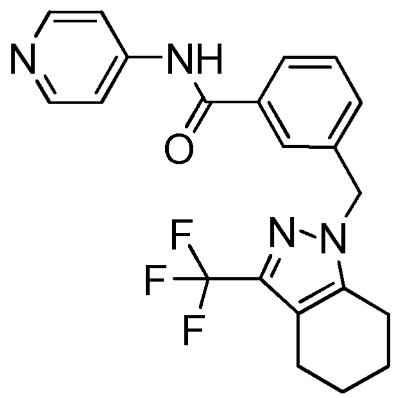

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 1.6 mg
MS(ESI+):401(M+H)

Example 7

N-Phenyl-3-{[3-(trifluoromethyl)-4,5,6,7-tetrahydro-1H-indazol-1-yl]methyl}benzamide

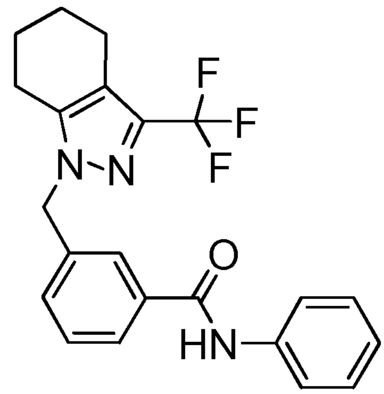

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.0 mg
MS(ESI+):400(M+H)

Example 8

N-(1-Phenylethyl)-3-{[3-(trifluoromethyl)-4,5,6,7-tetrahydro-1H-indazol-1-yl]methyl}benzamid e

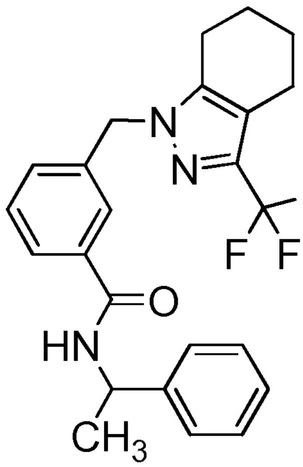

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 8.3 mg
MS(ESI+):428(M+H)

Example 9

1-[3-(Pyrrolidin-1-ylcarbonyl)benzyl]-3-(trifluoromethyl)-4,5,6,7-tetrahydro-1H-indazole

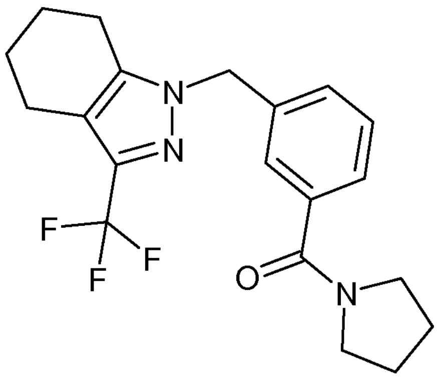

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.3 mg
MS(ESI+):378(M+H)

Example 10

1-[3-(Morpholin-4-ylcarbonyl)benzyl]-3-(trifluoromethyl)-4,5,6,7-tetrahydro-1H-indazole

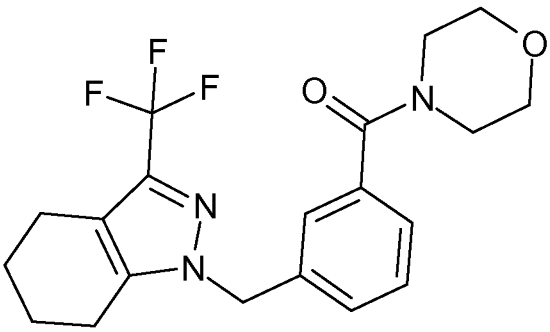

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.8 mg
MS(ESI+):414(M+H)

Example 11

N-(1-Methylethyl)-3-{[3-(trifluoromethyl)-4,5,6,7-tetrahydro-1H-indazol-1-yl]methyl}benzamid e

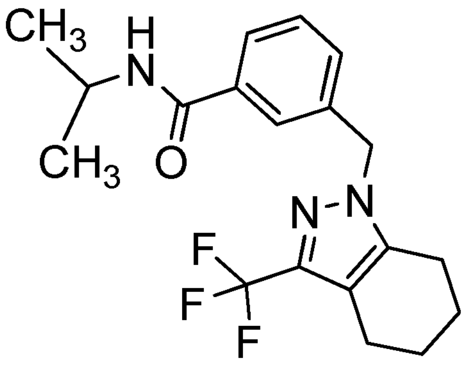

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.1 mg
MS(ESI+):366(M+H)

Example 12

N-(2-Methylphenyl)-3-{[3-(trifluoromethyl)-4,5,6,7-tetrahydro-1H-indazol-1-yl]methyl}benzami de

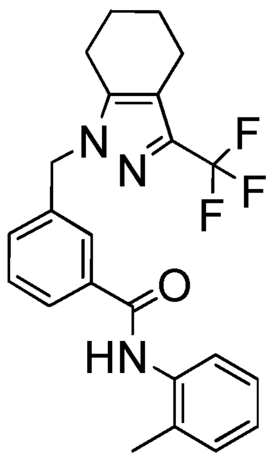

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.5 mg
MS(ESI+):414(M+H)

Example 13

N-(1-Methyl-1H-pyrazol-3-yl)-3-{[3-(trifluoromethyl)-4,5,6,7-tetrahydro-1H-indazol-1-yl]methy 1}benzamide

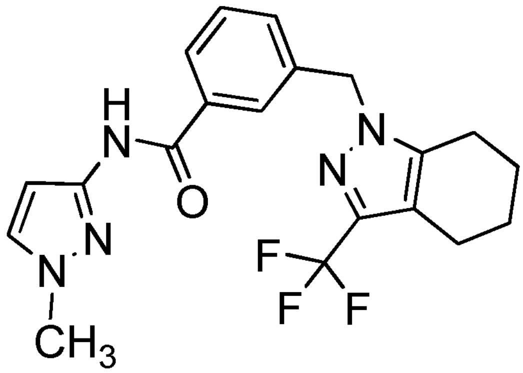

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 6.6 mg
MS(ESI+):404(M+H)

Example 14

N-{4-[(1-Methylethyl)sulfamoyl]phenyl}-3-{[3-(trifluoromethyl)-4,5,6,7-tetrahydro-1H-indazol-1-yl]methyl}benzamide

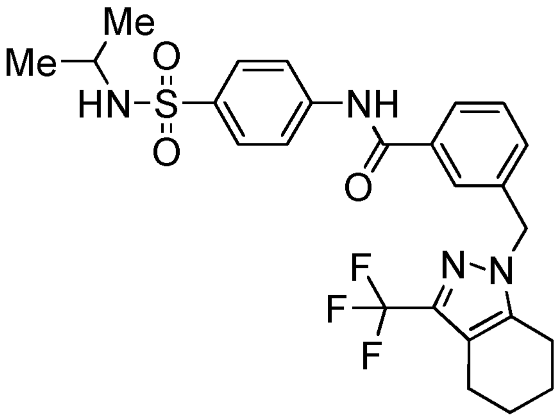

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 3.2 mg
MS(ESI+):521(M+H)

Example 15

N-Pyridin-2-yl-3-{[3-(trifluoromethyl)-4,5,6,7-tetrahydro-1H-indazol-1-yl]methyl}benzamide

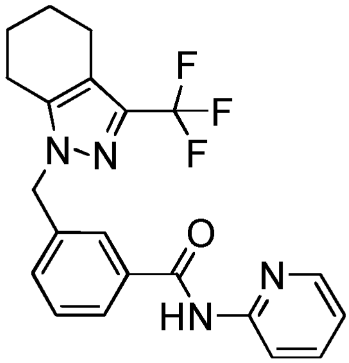

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 2.6 mg
MS(ESI+):401(M+H)

Example 16

N,N-Diethyl-3-{[3-(trifluoromethyl)-4,5,6,7-tetrahydro-1H-indazol-1-yl]methyl}benzamide

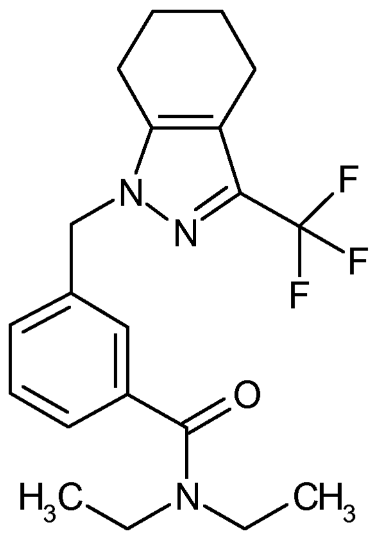

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 9.3 mg
MS(ESI+):380(M+H)

Example 17

N,N-Dimethyl-4-[(3-{[3-(trifluoromethyl)-4,5,6,7-tetrahydro-1H-indazol-1-yl]methyl}phenyl)car bonyl]piperazine-1-sulfonamide

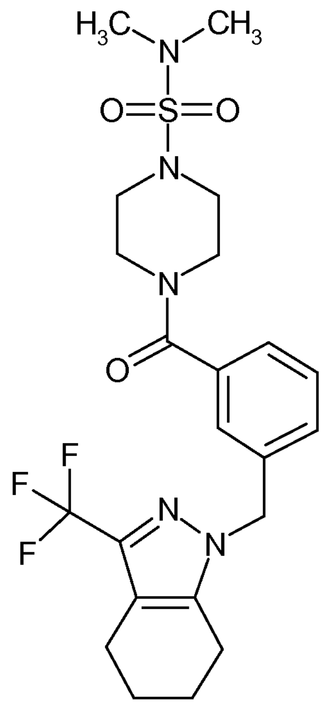

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 8.7 mg
MS(ESI+):500(M+H)

Example 18

N,N-Dimethyl-3-{[3-(trifluoromethyl)-4,5,6,7-tetrahydro-1H-indazol-1-yl]methyl}benzamide

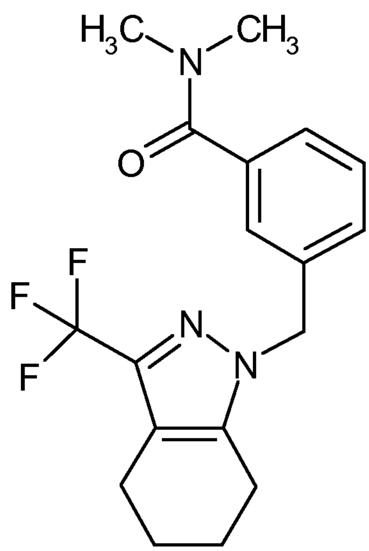

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 1.9 mg
MS(ESI+):352(M+H)

Example 19

N-(4-Methoxyphenyl)-3-{[3-(trifluoromethyl)-4,5,6,7-tetrahydro-1H-indazol-1-yl]methyl}benza mide

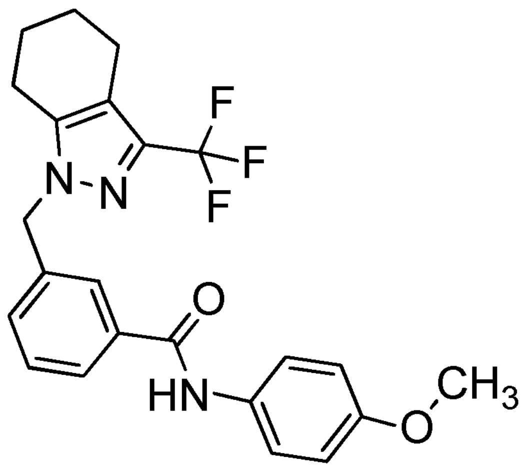

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.9 mg
MS(ESI+):430(M+H)

Example 20

N-Pyridin-3-yl-3-{[3-(trifluoromethyl)-4,5,6,7-tetrahydro-1H-indazol-1-yl]methyl}benzamide

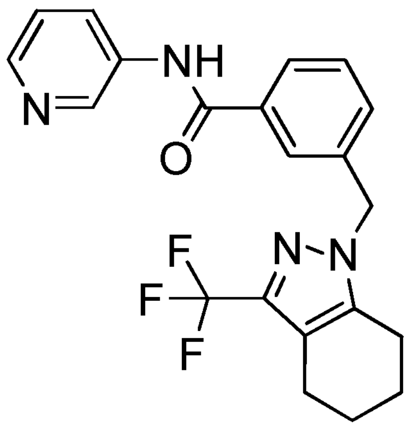

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 3.7 mg
MS(ESI+):401(M+H)

Example 21

N-(2-Methoxyethyl)-N-methyl-3-{[3-(trifluoromethyl)-4,5,6,7-tetrahydro-1H-indazol-1-yl]meth yl}benzamide

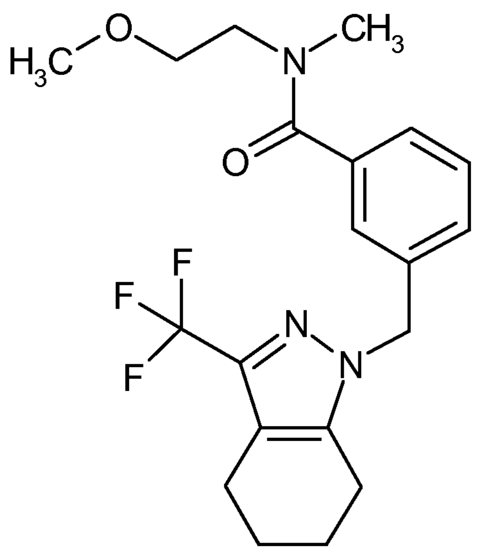

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 8.6 mg
MS(ESI+):396(M+H)

Example 22

N,N-Bis(1-methylethyl)-3-{[3-(trifluoromethyl)-4,5,6,7-tetrahydro-1H-indazol-1-yl]methyl}benz amide

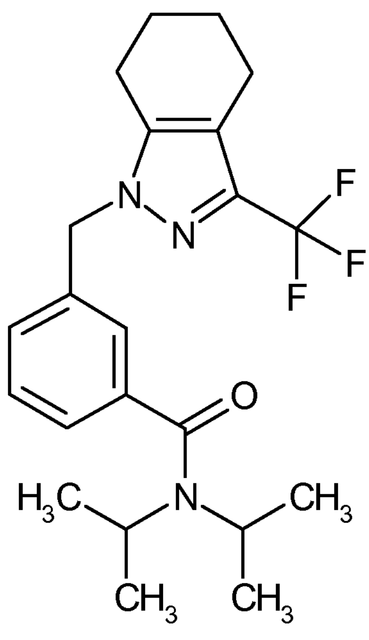

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 1.0 mg
MS(ESI+):408(M+H)

Example 23

N-(1,3-Thiazol-2-yl)-3-{[3-(trifluoromethyl)-4,5,6,7-tetrahydro-1H-indazol-1-yl]methyl}benzami de

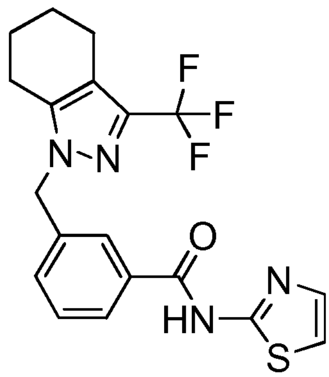

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 6.6 mg
MS(ESI+):407(M+H)

Example 24

N-(2-Fluorophenyl)-3-{[3-(trifluoromethyl)-4,5,6,7-tetrahydro-1H-indazol-1-yl]methyl}benzami de

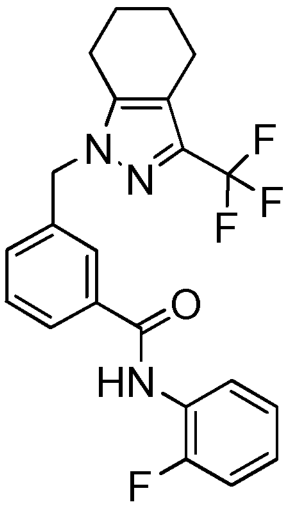

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 3.9 mg
MS(ESI+):418(M+H)

Example 25

N-(3-Methoxyphenyl)-3-{[3-(trifluoromethyl)-4,5,6,7-tetrahydro-1H-indazol-1-yl]methyl}benza mide

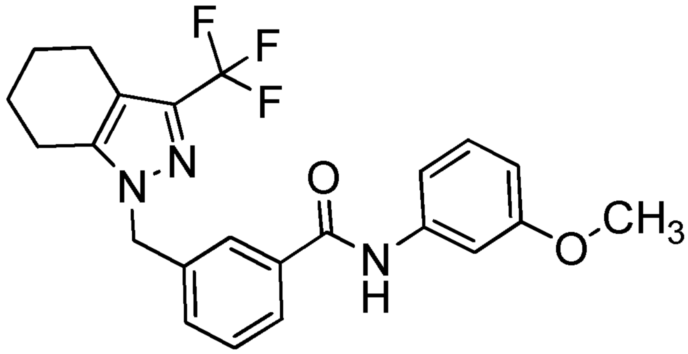

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.9 mg
MS(ESI+):430(M+H)

Example 26

1-{3-[(4-Pyridin-2-ylpiperazin-1-yl)carbonyl]benzyl}-3-(trifluoromethyl)-4,5,6,7-tetrahydro-1H-i ndazole

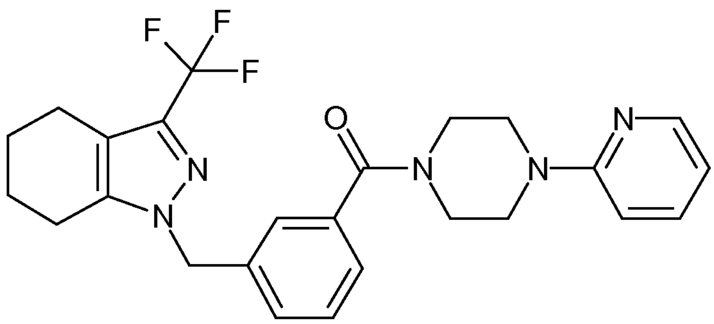

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 8.6 mg
MS(ESI+):470(M+H)

Example 27

N-[3-(1H-Imidazol-1-yl)propyl]-3-{[3-(trifluoromethyl)-4,5,6,7-tetrahydro-1H-indazol-1-yl]meth yl}benzamide

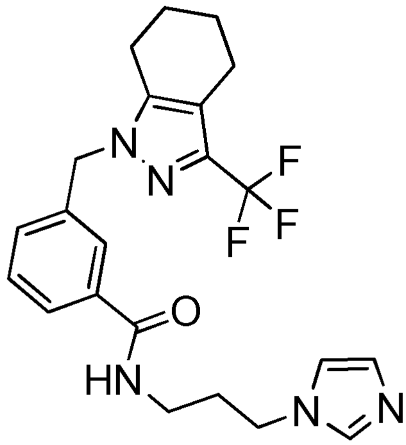

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.0 mg
MS(ESI+):432(M+H)

Example 28

N-(2-Morpholin-4-ylethyl)-3-{[3-(trifluoromethyl)-4,5,6,7-tetrahydro-1H-indazol-1-yl]methyl}be nzamide

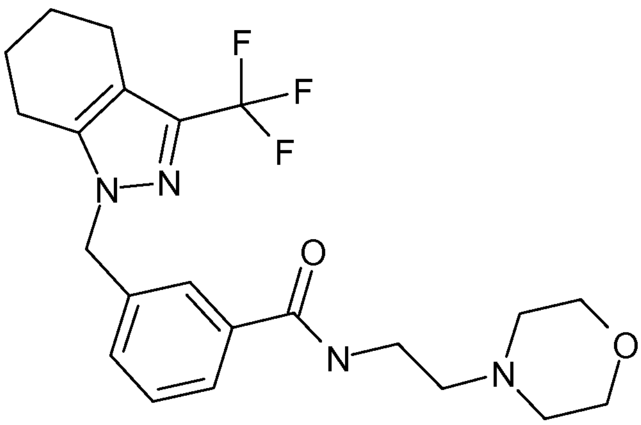

Using 3-{[3-(trifluoromethyl)-4,5,6,7-tetrahydro-1H-indazol-1-yl]methyl}benzoic acid obtained in Example 2, the title compound was obtained in the same manner as in Example 3.
Yield: 7.9 mg
MS(ESI+):437(M+H)

Example 29

N-(3-Chlorophenyl)-4-[3-(trifluoromethyl)-4,5,6,7-tetrahydro-1H-indazol-1-yl]butanamide

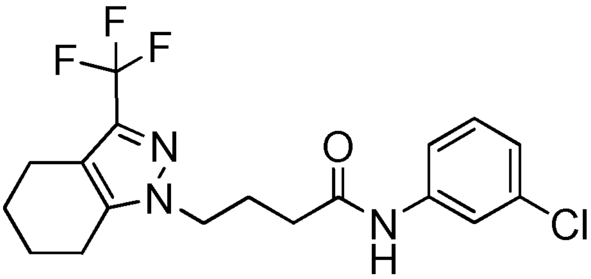

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 2.8 mg
MS(ESI+):386(M+H)

Example 30

N-Phenyl-4-[3-(trifluoromethyl)-4,5,6,7-tetrahydro-1H-indazol-1-yl]butanamide

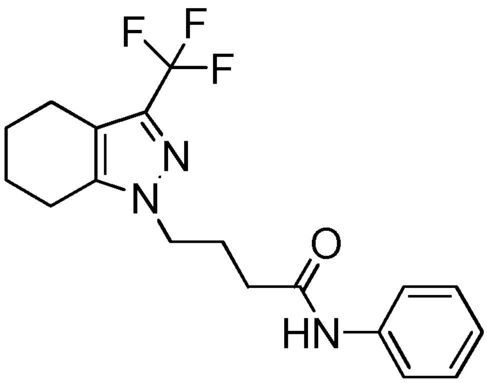

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 6.6 mg
MS(ESI+):352(M+H)

Example 31

N-(1-Phenylethyl)-4-[3-(trifluoromethyl)-4,5,6,7-tetrahydro-1H-indazol-1-yl]butanamide

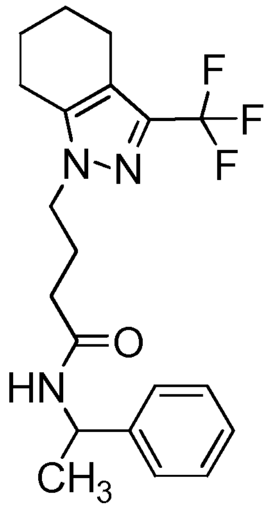

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 6.9 mg
MS(ESI+):380(M+H)

Example 32

N-(2-Methylphenyl)-4-[3-(trifluoromethyl)-4,5,6,7-tetrahydro-1H-indazol-1-yl]butanamide

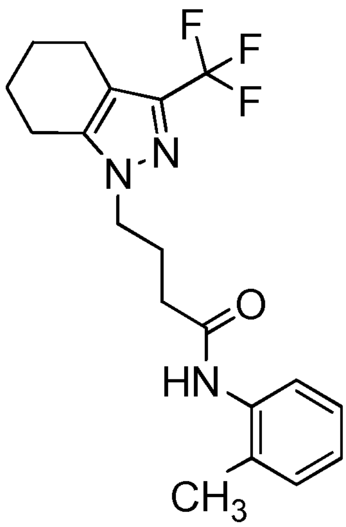

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 6.2 mg
MS(ESI+):366(M+H)

Example 33

N-(1-Methyl-1H-pyrazol-3-yl)-4-[3-(trifluoromethyl)-4,5,6,7-tetrahydro-1H-indazol-1-yl]butana mide

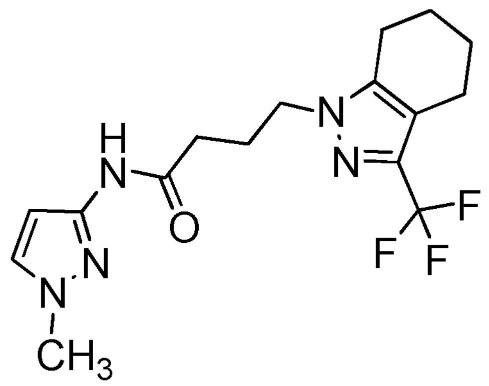

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 5.0 mg
MS(ESI+):356(M+H)

Example 34

N-{4-[(1-Methylethyl)sulfamoyl]phenyl}-4-[3-(trifluoromethyl)-4,5,6,7-tetrahydro-1H-indazol-1-yl]butanamide

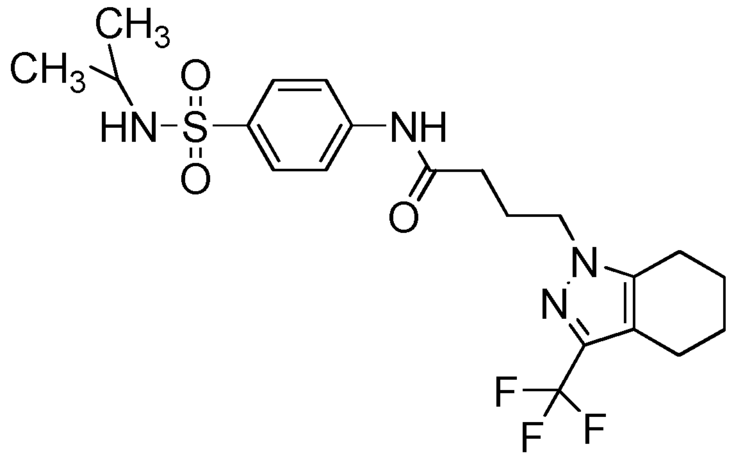

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 4.5 mg
MS(ESI+):473(M+H)

Example 35

N-Pyridin-2-yl-4-[3-(trifluoromethyl)-4,5,6,7-tetrahydro-1H-indazol-1-yl]butanamide

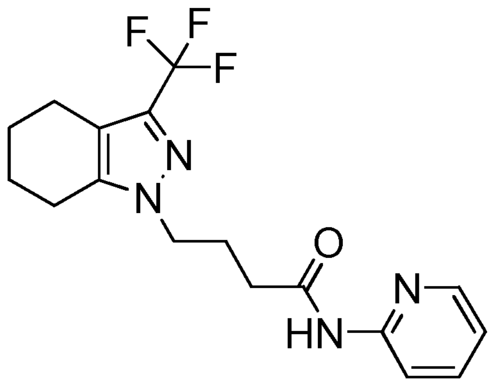

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 1.3 mg
MS(ESI+):353(M+H)

Example 36

N-(4-Methoxyphenyl)-4-[3-(trifluoromethyl)-4,5,6,7-tetrahydro-1H-indazol-1-yl]butanamide

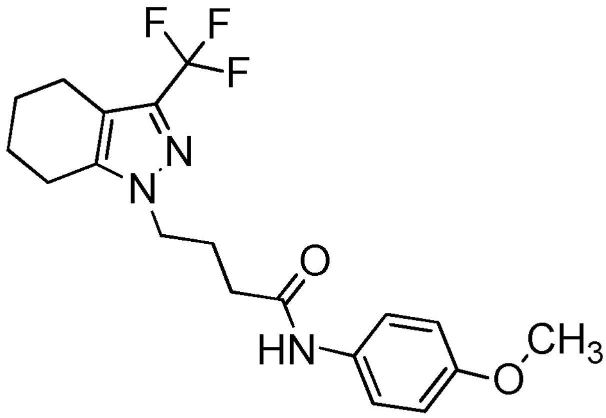

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 6.3 mg
MS(ESI+):382(M+H)

Example 37

N-Pyridin-3-yl-4-[3-(trifluoromethyl)-4,5,6,7-tetrahydro-1H-indazol-1-yl]butanamide

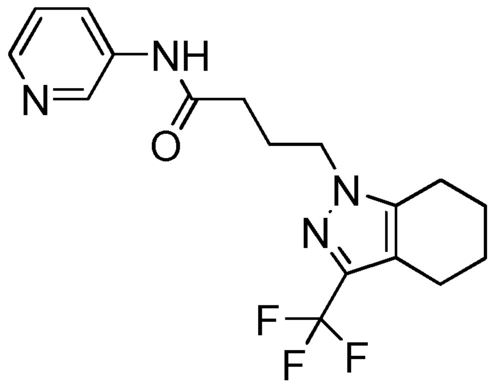

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 6.1 mg
MS(ESI+):353(M+H)

Example 38

N-1,3-Thiazol-2-yl-4-[3-(trifluoromethyl)-4,5,6,7-tetrahydro-1H-indazol-1-yl]butanamide

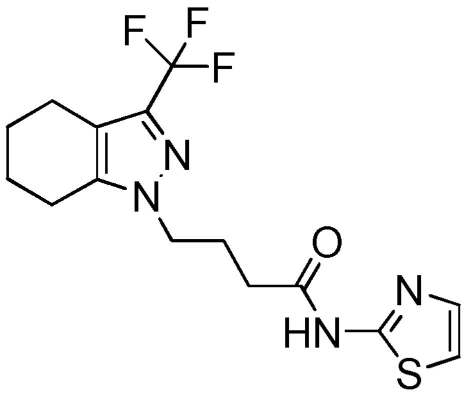

A 0.12 M solution of 4-[3-(trifluoromethyl)-4,5,6,7-tetrahydro-1H-indazol-1-yl]butanoic acid ob tained in Reference Example 2 in DMF (500 μl, 60 μmol), and a 0.144 M solution of 1,3-thiazol e-2-amine in DMF (500 μl, 72 μmol), and a 0.24 M solution of HATU and DIEA in DMF (500 μ 1, 120 μmol) were mixed at room temperature, and the mixture was stirred at 60 °C for 24 hou rs. The reaction mixture was cooled to room temperature, and ethyl acetate (3 ml), and 2% sod ium hydrogen carbonate aqueous solution (1.5 ml) were added thereto, and the mixture was e xtracted. The organic layer was collected with upper phase sep tube (Wako Pure Chemical Ind ustries). The solvent was evaporated off under reduced pressure. The residue was dissolved in DMSO-methanol (1:1) (1 ml), and purified with preparative HPLC to give the titled compoun d.
Yield: 4.2 mg
MS(ESI+):359(M+H)

Example 39

N-(2-Fluorophenyl)-4-[3-(trifluoromethyl)-4,5,6,7-tetrahydro-1H-indazol-1-yl]butanamide

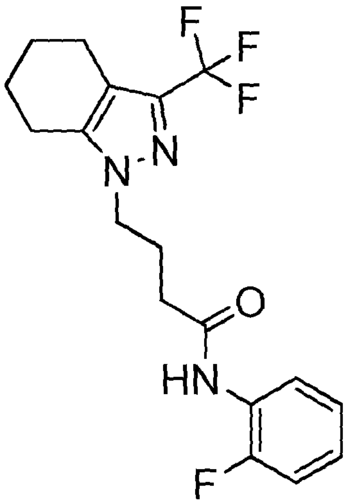

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 4.3 mg
MS(ESI+):370(M+H)

Example 40

N-(3.Methoxyphenyl)-4--[3-(trifluoromethyl)-4,5,6,7-tetrahydro-1H-indazol-1-yl]butanamide

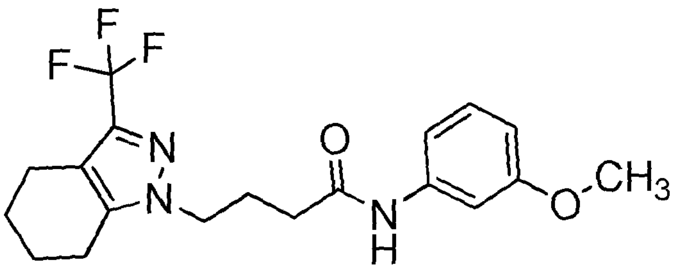

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 6.8 mg
MS(ESI+):382(M+H)

Example 41

N-(3-Chlorophenyl)-2-methyl-4-[3-(trifluoromethyl)-4,5,6,7-tetrahydro-1H-indazol-1-yl]butana mide

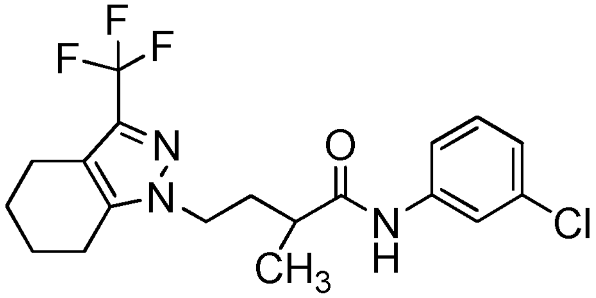

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 4.8 mg
MS(ESI+):400(M+H)

Example 42

2-Methyl-N-phenyl-4-[3-(trifluoromethyl)-4,5,6,7-tetrahydro-1H-indazol-1-yl]butanamide

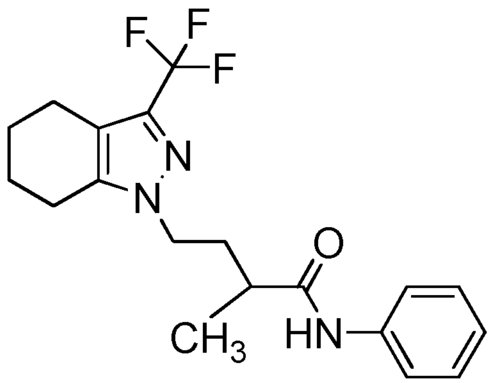

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 6.2 mg
MS(ESI+):366(M+H)

Example 43

2-Methyl-N-(1-phenylethyl)-4-[3-(trifluoromethyl)-4,5,6,7-tetrahydro-1H-indazol-1-yl]butanam ide

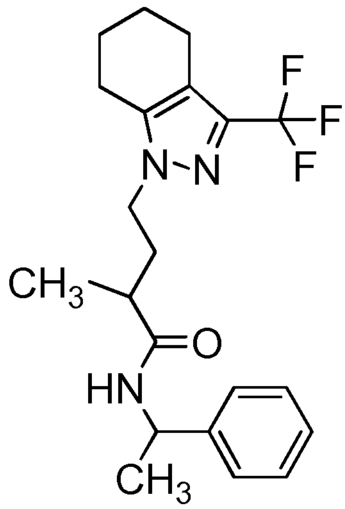

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 7.6 mg
MS(ESI+):394(M+H)

Example 44

2-Methyl-N-(2-methylphenyl)-4-[3-(trifluoromethyl)-4,5,6,7-tetrahydro-1H-indazol-1-yl]butana mide

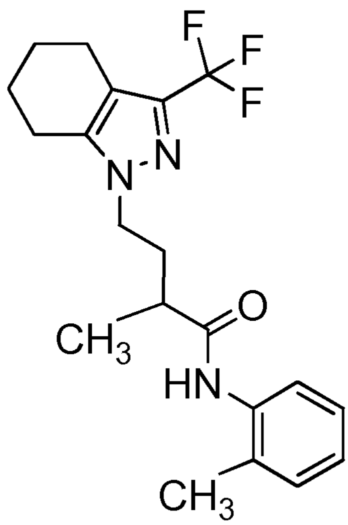

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 5.9 mg
MS(ESI+):380(M+H)

Example 45

2-Methyl-N-(1-methyl-1H-pyrazol-3-yl)-4-[3-(trifluoromethyl)-4,5,6,7-tetrahydro-1H-indazol-1-yl]butanamide

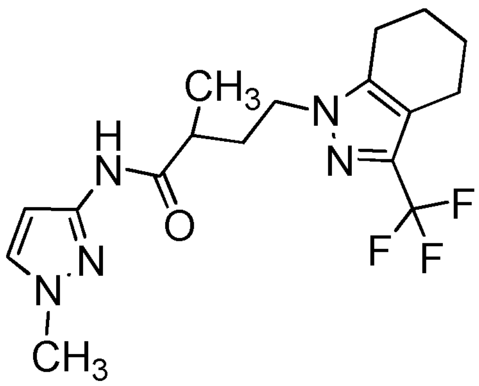

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 5.5 mg
MS(ESI+):370(M+H)

Example 46

2-Methyl-N-{4-[(1-methylethyl)sulfamoyl]phenyl}-4-[3-(trifluoromethyl)-4,5,6,7-tetrahydro-1H-indazol-1-yl]butanamide

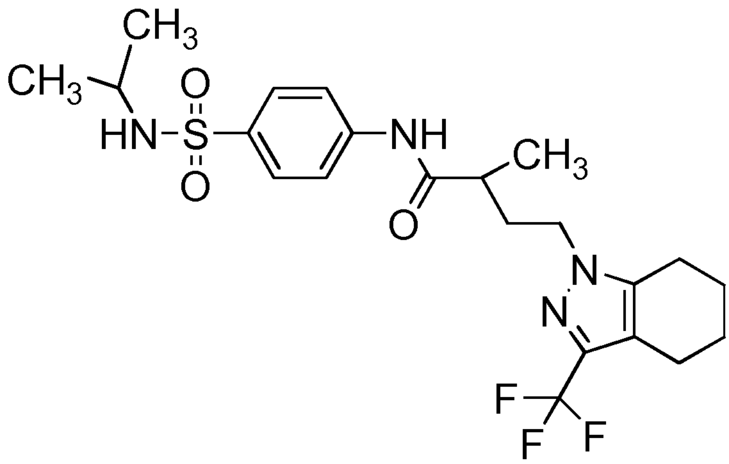

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 2.1 mg
MS(ESI+):487(M+H)

Example 47

2-Methyl-N-pyridin-2-yl-4-[3-(trifluoromethyl)-4,5,6,7-tetrahydro-1H-indazol-1-yl]butanamide

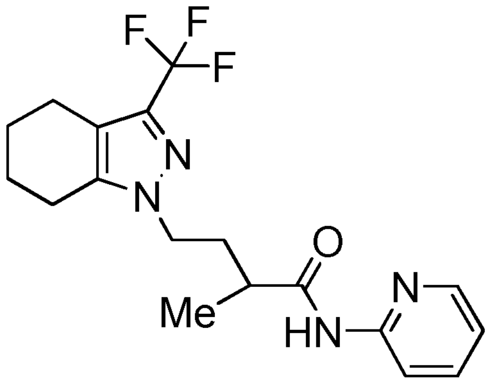

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 1.8 mg
MS(ESI+):367(M+H)

Example 48

N-(4-Methoxyphenyl)-2-methyl-4-[3-(trifluoromethyl)-4,5,6,7-tetrahydro-1H-indazol-1-yl]buta namide

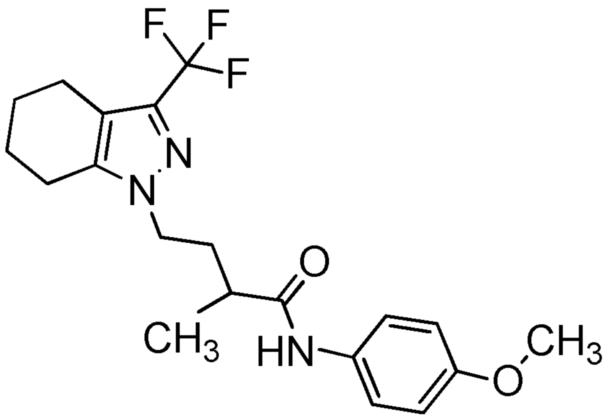

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 6.3 mg
MS(ESI+):396(M+H)

Example 49

2-Methyl-N-pyridin-3-yl-4-[3-(trifluoromethyl)-4,5,6,7-tetrahydro-1H-indazol-1-yl]butanamide

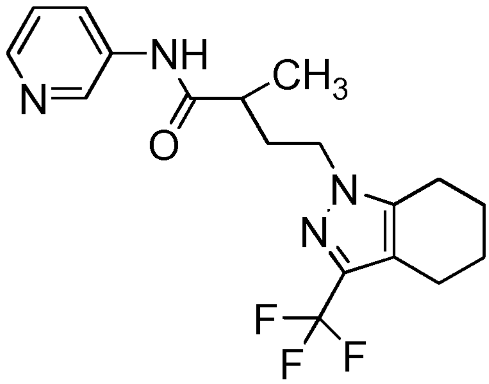

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 4.5 mg
MS(ESI+):367(M+H)

Example 50

2-Methyl-N-1,3-thiazol-2-yl-4-[3-(trifluoromethyl)-4,5,6,7-tetrahydro-1H-indazol-1-yl]butanam ide

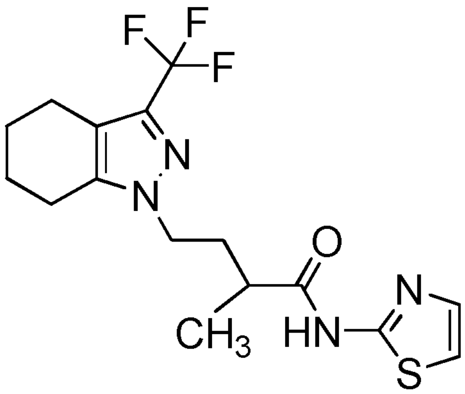

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 5.5 mg
MS(ESI+):373(M+H)

Example 51

N-(2-Fluorophenyl)-2-methyl-4-[3-(trifluoromethyl)-4,5,6,7-tetrahydro-1H-indazol-1-yl]butana mide

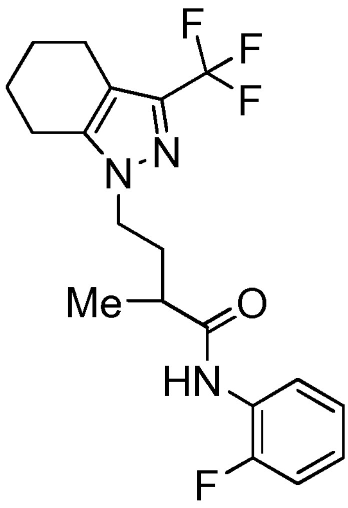

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 2.5 mg
MS(ESI+):384(M+H)

Example 52

N-(3-Methoxyphenyl)-2-methyl-4-[3-(trifluoromethyl)-4,5,6,7-tetrahydro-1H-indazol-1-yl]buta namide

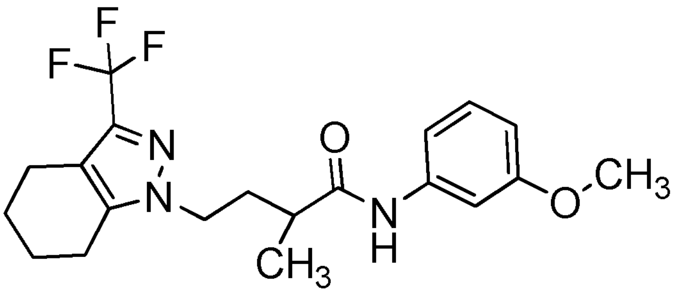

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 6.6 mg
MS(ESI+):396(M+H)

Example 53

N-(3-Chlorophenyl)-5-[3-(trifluoromethyl)-4,5,6,7-tetrahydro-1H-indazol-1-yl]pentanamide

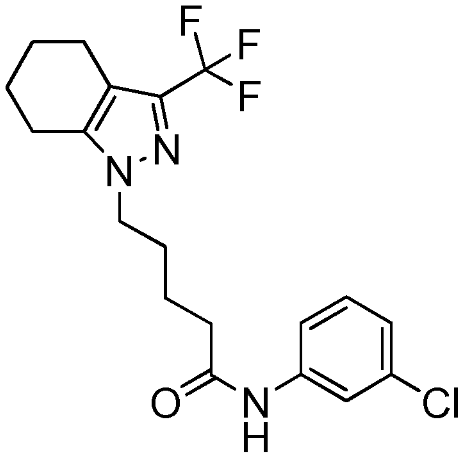

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 3.1 mg
MS(ESI+):400(M+H)

Example 54

N-Phenyl-5-[3-(trifluoromethyl)-4,5,6,7-tetrahydro-1H-indazol-1-yl]pentanamide

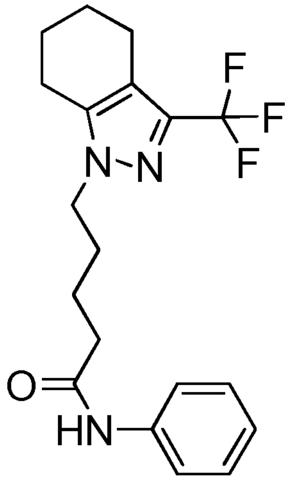

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 5.4 mg
MS(ESI+):366(M+H)

Example 55

N-(1-Phenylethyl)-5-[3-(trifluoromethyl)-4,5,6,7-tetrahydro-1H-indazol-1-yl]pentanamide

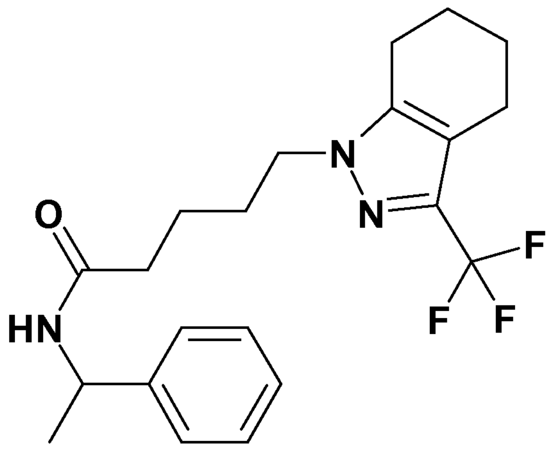

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 6.8 mg
MS(ESI+):394(M+H)

Example 56

N-(2-Methylphenyl)-5-[3-(trifluoromethyl)-4,5,6,7-tetrahydro-1H-indazol-1-yl]pentanamide

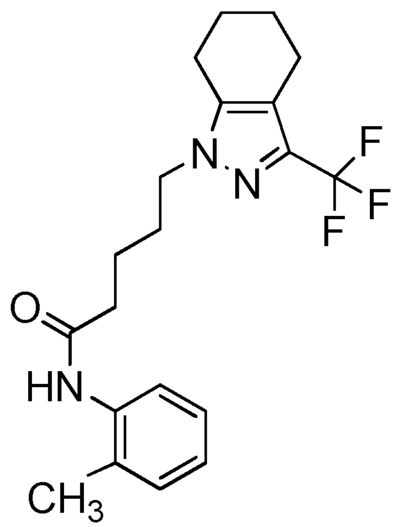

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 6.7 mg
MS(ESI+):380(M+H)

Example 57

N-(1-Methyl-1H-pyrazol-3-yl)-5-[3-(trifluoromethyl)-4,5,6,7-tetrahydro-1H-indazol-1-yl]pentan amide

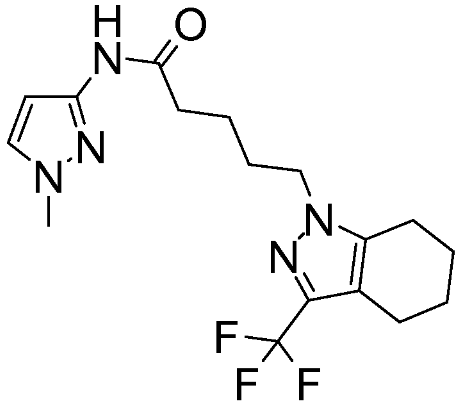

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 5.9 mg
MS(ESI+):370(M+H)

Example 58

N-{4-[(1-Methylethyl)sulfamoyl]phenyl}-5-[3-(trifluoromethyl)-4,5,6,7-tetrahydro-1H-indazol-1-yl]pentanamide

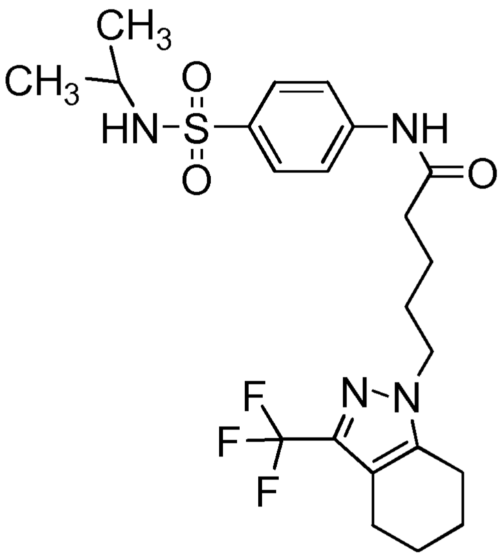

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 3.0 mg
MS(ESI+):487(M+H)

Example 59

N-Pyridin-2-yl-5-[3-(trifluoromethyl)-4,5,6,7-tetrahydro-1H-indazol-1-yl]pentanamide

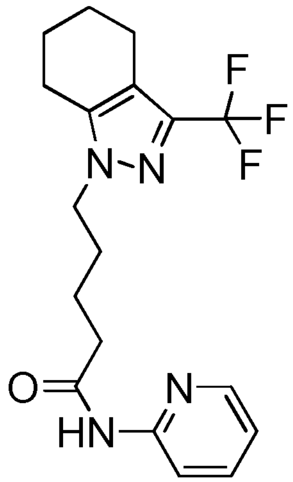

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 1.4 mg
MS(ESI+):367(M+H)

Example 60

N-(4-Methoxyphenyl)-5-[3-(trifluoromethyl)-4,5,6,7-tetrahydro-1H-indazol-1-yl]pentanamide

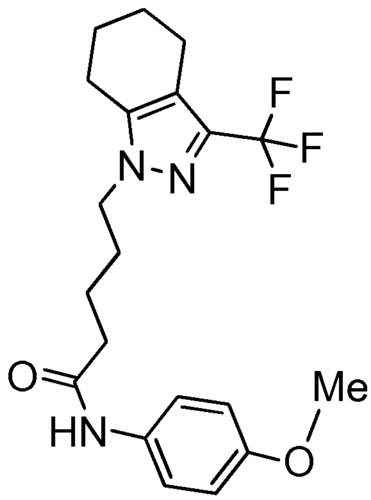

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 6.7 mg
MS(ESI+):396(M+H)

Example 61

N-Pyridin-3-yl-5-[3-(trifluoromethyl)-4,5,6,7-tetrahydro-1H-indazol-1-yl]pentanamide

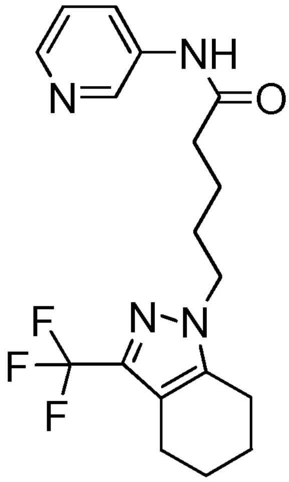

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 6.4 mg
MS(ESI+):367(M+H)

Example 62

N-(2-Fluorophenyl)-5-[3-(trifluoromethyl)-4,5,6,7-tetrahydro-1H-indazol-1-yl]pentanamide

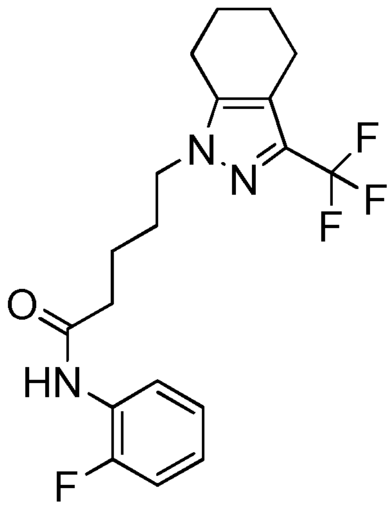

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 2.9 mg
MS(ESI+):384(M+H)

Example 63

N-(3-Methoxyphenyl)-5-[3-(trifluoromethyl)-4,5,6,7-tetrahydro-1H-indazol-1-yl]pentanamide

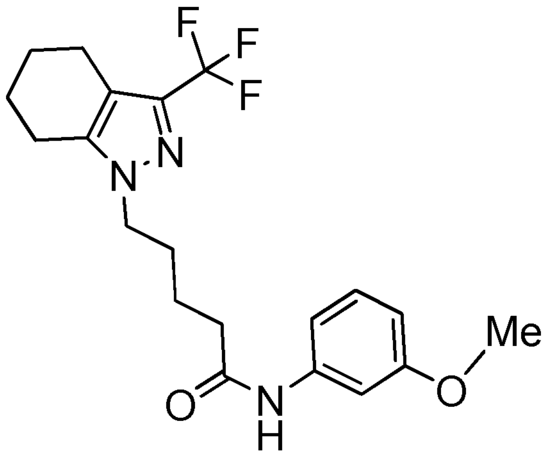

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 4.7 mg
MS(ESI+):396(M+H)

Example 64

N-(3-Chlorophenyl)-2-{[3-(trifluoromethyl)-4,5,6,7-tetrahydro-1H-indazol-1-yl]methyl}benzami de

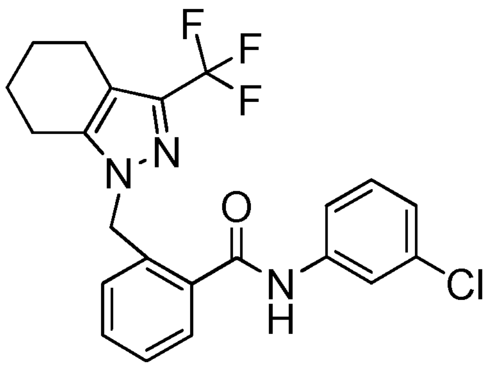

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 3.8 mg
MS(ESI+):434(M+H)

Example 65

N-Pyridin-4-yl-2-{[3-(trifluoromethyl)-4,5,6,7-tetrahydro-1H-indazol-1-yl]methyl}benzamide

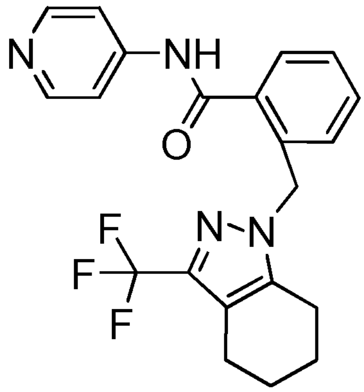

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 1.1 mg
MS(ESI+):401(M+H)

Example 66

N-Phenyl-2-{[3-(trifluoromethyl)-4,5,6,7-tetrahydro-1H-indazol-1-yl]methyl}benzamide

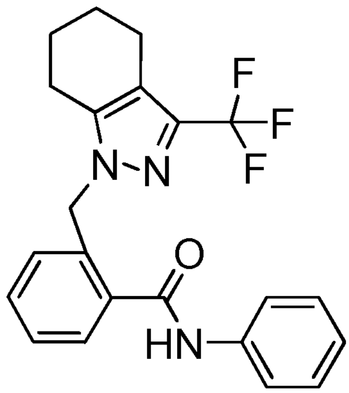

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 7.3 mg
MS(ESI+):400(M+H)

Example 67

N-(1-Phenylethyl)-2-{[3-(trifluoromethyl)-4,5,6,7-tetrahydro-1H-indazol-1-yl]methyl}benzamid e

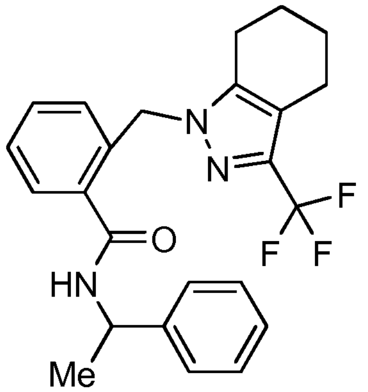

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 7.5 mg
MS(ESI+):428(M+H)

Example 68

N-(2-Methylphenyl)-2-{[3-(trifluoromethyl)-4,5,6,7-tetrahydro-1H-indazol-1-yl]methyl}benzami de

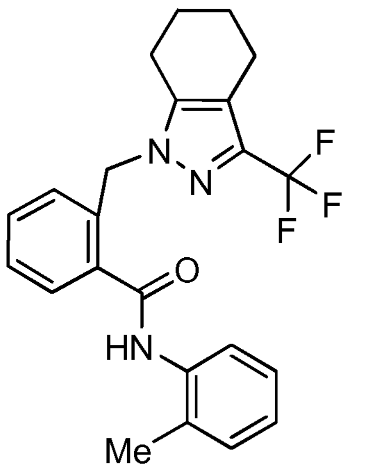

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 6.1 mg
MS(ESI+):414(M+H)

Example 69

N-(1-Methyl-1H-pyrazol-3-yl)-2-{[3-(trifluoromethyl)-4,5,6,7-tetrahydro-1H-indazol-1-yl]methy l}benzamide

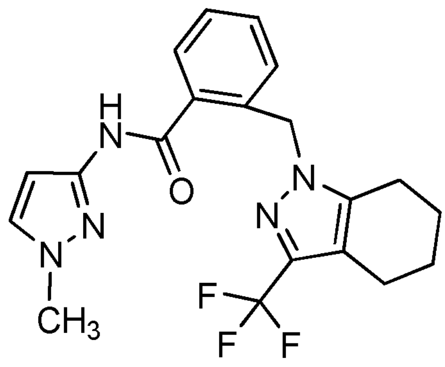

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 6.4 mg
MS(ESI+):404(M+H)

Example 70

N-{4-[(1-Methylethyl)sulfamoyl]phenyl}-2-{[3-(trifluoromethyl)-4,5,6,7-tetrahydro-1H-indazol-1-yl]methyl}benzamide

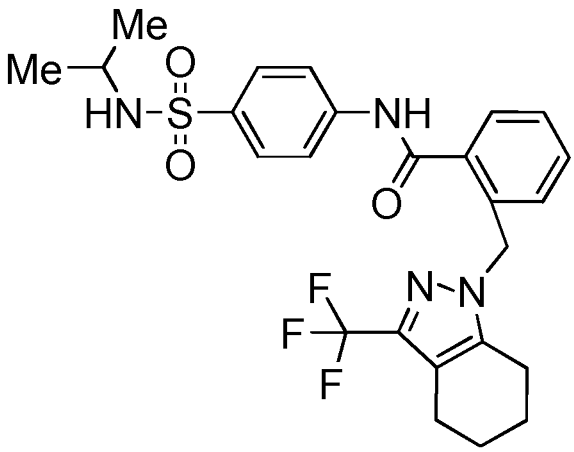

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 1.5 mg
MS(ESI+):521(M+H)

Example 71

N-Pyridin-2-yl-2-{[3-(trifluoromethyl)-4,5,6,7-tetrahydro-1H-indazol-1-yl]methyl}benzamide

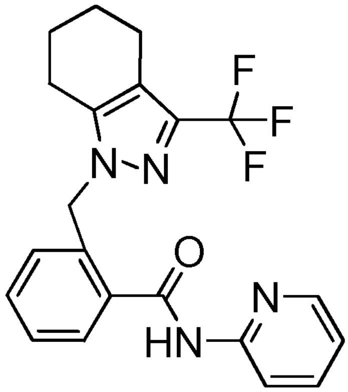

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 1.1 mg
MS(ESI+):401(M+H)

Example 72

N,N-Dimethyl-2-{[3-(trifluoromethyl)-4,5,6,7-tetrahydro-1H-indazol-1-yl]methyl}benzamide

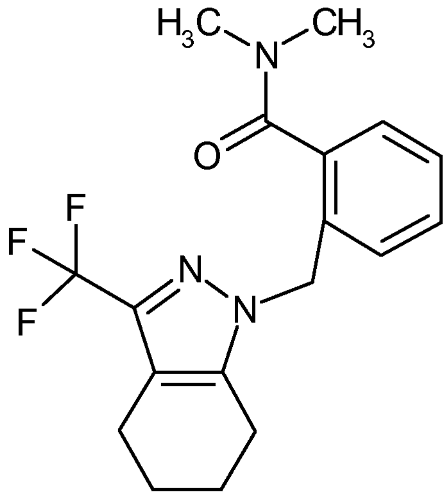

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 2.0 mg
MS(ESI+):352(M+H)

Example 73

N-(4-Methoxyphenyl)-2-{[3-(trifluoromethyl)-4,5,6,7-tetrahydro-1H-indazol-1-yl]methyl}benza mide

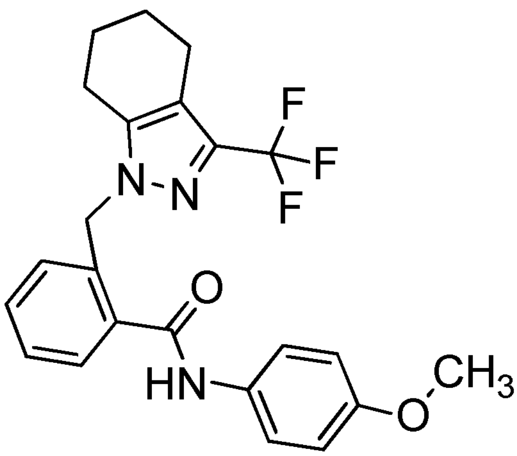

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 7.2 mg
MS(ESI+):430(M+H)

Example 74

N-Pyridin-3-yl-2-{[3-(trifluoromethyl)-4,5,6,7-tetrahydro-1H-indazol-1-yl]methyl}benzamide

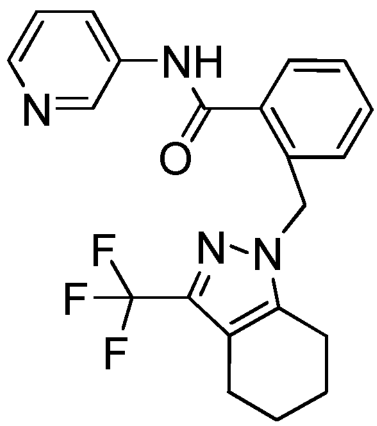

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 5.2 mg
MS(ESI+):401(M+H)

Example 75

N-(2-Methoxyethyl)-N-methyl-2-{[3-(trifluoromethyl)-4,5,6,7-tetrahydro-1H-indazol-1-yl]meth yl}benzamide

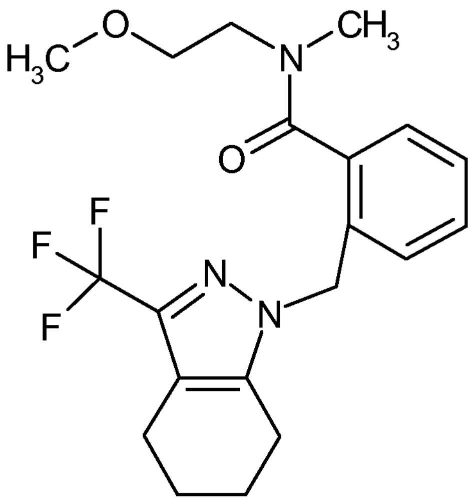

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 6.7 mg
MS(ESI+):396(M+H)

Example 76

N-1,3-Thiazol-2-yl-2-{[3-(trifluoromethyl)-4,5,6,7-tetrahydro-1H-indazol-1-yl]methyl}benzamid e

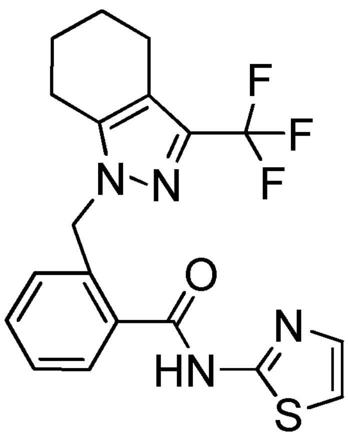

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 5.1 mg
MS(ESI+):407(M+H)

Example 77

N-(2-Fluorophenyl)-2-{[3-(trifluoromethyl)-4,5,6,7-tetrahydro-1H-indazol-1-yl]methyl}benzami de

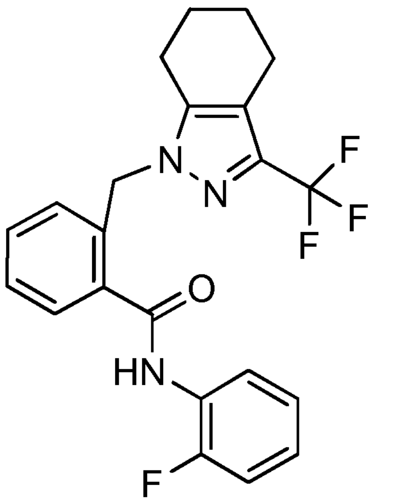

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 2.6 mg
MS(ESI+):418(M+H)

Example 78

N-(3-Methoxyphenyl)-2-{[3-(trifluoromethyl)-4,5,6,7-tetrahydro-1H-indazol-1-yl]methyl}benza mide

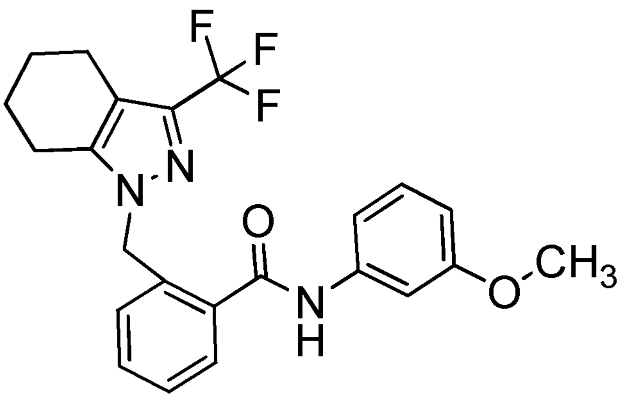

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 6.7 mg
MS(ESI+):430(M+H)

Example 79

1-{2-[(4-Pyridin-2-ylpiperazin-1-yl)carbonyl]benzyl}-3-(trifluoromethyl)-4,5,6,7-tetrahydro-1H-i ndazole

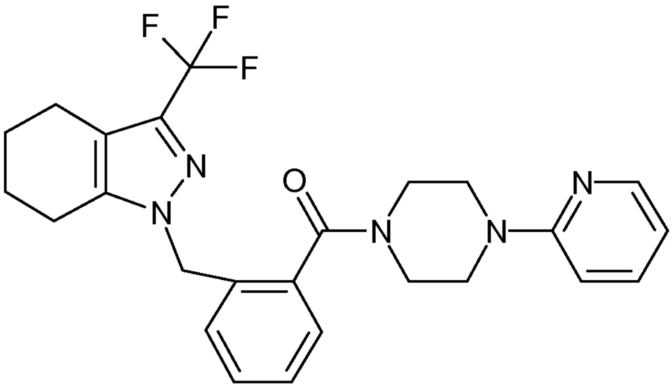

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 7.1 mg
MS(ESI+):470(M+H)

Example 80

N-[3-(1H-Imidazol-1-yl)propyl]-2-{[3-(trifluoromethyl)-4,5,6,7-tetrahydro-1H-indazol-1-yl]meth yl}benzamide

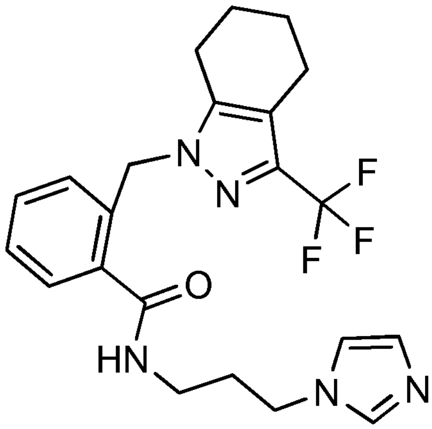

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 6.2 mg
MS(ESI+):432(M+H)

Example 81

N-(2-Morpholin-4-ylethyl)-2-{[3-(trifluoromethyl)-4,5,6,7-tetrahydro-1H-indazol-1-yl]methyl}be nzamide

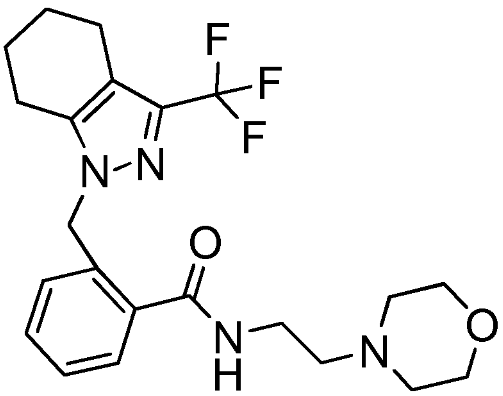

Using 2-{[3-(trifluoromethyl)-4,5,6,7-tetrahydro-1H-indazol-1-yl]methyl}benzoic acid obtained in Example 5, the title compound was obtained in the same manner as in Example 3.
Yield: 6.8 mg
MS(ESI+):437(M+H)

Example 82

Methyl 4-{2-[3-(trifluoromethyl)-4,5,6,7-tetrahydro-1H-indazol-1-yl]ethoxy}benzoate

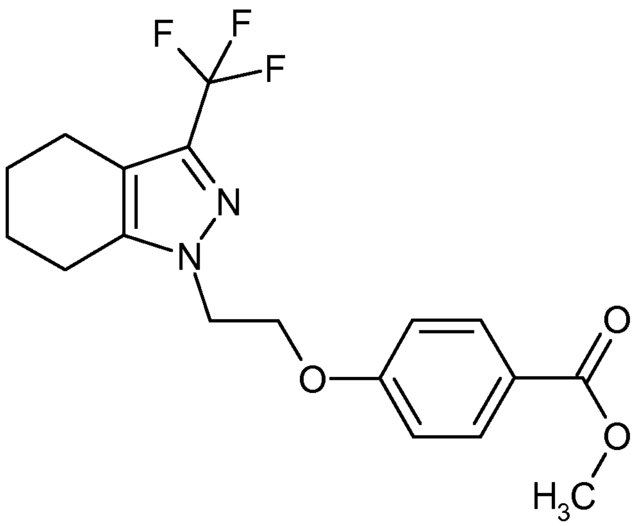

To a mixture of 3-(trifluoromethyl)-4,5,6,7-tetrahydro-1H-indazole (20 mg, 0.11 mmol), methyl 4-(2-bromoethoxy)benzoate (33 mg, 0.12 mmol), and DMF (2 ml), was added potassium tert-b utoxide (14 mg, 0.12 mmol), and the mixture was stirred for 13 hours at room temperature. To the reaction mixture, was added water, and the mixture was extracted with ethyl acetate. Th e organic layer was washed with water, and brine, dried over magnesium sulfate, and concent rated under reduced pressure. The obtained residue was purified by column chromatography on silica gel [developing solvent: hexane-ethyl acetate (9:1-4:1)], and recrystallized from ethyl acetate-hexane to give the titled compound (27 mg) as white crystals (yield 70%).
MS (ESI+) :369 (M+H)
1H NMR (300 MHz, $CDCl_3$) δ ppm 1.65 - 1.89 (4 H, m) 2.52 - 2.73 (4 H, m) 3.88 (3 H, s) 4.34 - 4.48 (4 H, m) 6.82 (2 H, d, J=8.9 Hz) 7.96 (2 H, d, J=8.9 Hz).

Example 83

Ethyl 2-{[3-(trifluoromethyl)-4,5,6,7-tetrahydro-1H-indazol-1-yl]methyl}-1,3-thiazole-4-carboxy late

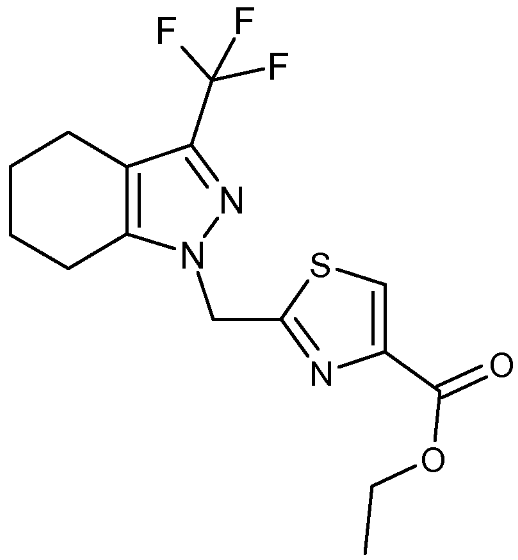

To a mixture of 3-(trifluoromethyl)-4,5,6,7-tetrahydro-1H-indazole (1.0 g, 5.26 mmol), ethyl 2-( chloromethyl)-1,3-thiazole-4-carboxylate (1.3 g, 6.31 mmol), and DMF (15 ml), was added pota ssium tert-butoxide (0.7 g, 6.31 mmol) at 0 °C, and then the mixture was stirred at room temp erature for 13 hours. To the reaction mixture, was added water, and the mixture was extracte d with ethyl acetate. The organic layer was washed with water, and brine, dried over magnesi um sulfate, and concentrated under reduced pressure. The obtained residue was purified by co lumn chromatography on silica gel [developing solvent: hexane-ethyl acetate (93:7-82:18)], an d recrystallized from ethyl acetate-hexane to give the titled compound (1.26 g) as pale yellow c rystals (yield 67%).
MS (ESI+) :360 (M+H)
1H NMR (300 MHz, $CDCl_3$) δ ppm 1.41 (3 H, t, J=7.2 Hz) 1.67 - 1.87 (4 H, m) 2.59 (4 H, q, J=5 .8 Hz) 4.43 (2 H, q, J=6.9 Hz) 5.59 (2 H, s) 8.14 (1 H, s).

Example 84

4-{2-[3-(Trifluoromethyl)-4,5,6,7-tetrahydro-1H-indazol-1-yl]ethoxy}benzoic acid

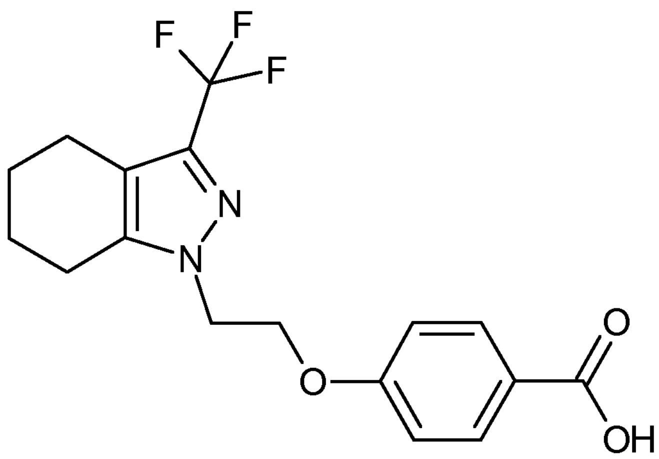

A solution of methyl 4-{2-[3-(trifluoromethyl)-4,5,6,7-tetrahydro-1H-indazol-1-yl]ethoxy}benzo ate obtained in Example 82 (1.37 g, 3.72 mmol), and 2N aqueous sodium hydroxide (4 ml) in et hanol (15 ml) was stirred at 60 °C for 13 hours. To the reaction mixture, 1 N hydrochloric acid (8 ml), and water (8 ml) were added, and then the mixture was stirred for 30 minutes at room temperature. The crystals were filtered off, washed with water, and dried to give the titled co mpound (1.12 g) as white crystals (yield 85%).
MS (ESI+):355 (M+H)
1H NMR (300 MHz, $CDCl_3$) δ ppm 1.67 - 1.88 (4 H, m) 2.52 - 2.60 (2 H, m) 2.68 (2 H, t, J=6.1 Hz) 4.34 - 4.46 (4 H, m) 6.79 - 6.86 (2 H, m) 7.93 - 8.02 (2 H, m).

Example 85

2-{[3-(Trifluoromethyl)-4,5,6,7-tetrahydro-1H-indazol-1-yl]methyl]-1,3-thiazole-4-carboxylic ac id

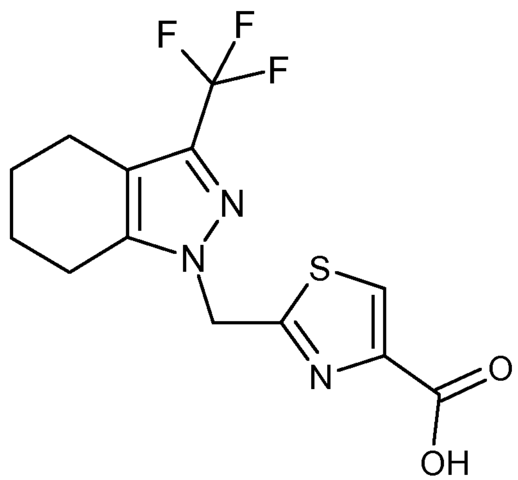

A mixture of ethyl 2-{[3-(trifluoromethyl)-4,5,6,7-tetrahydro-1H-indazol-1-yl]methyl}-1,3-thiaz ole-4-carboxylate obtained in Example 83 (1.22 g, 3.34 mmol), 2N aqueous sodium hydroxide ( 3.5 ml) and ethanol (15 ml) was stirred at 50 °C for 13 hours. After cooling to room temperatur e, 1N hydrochloric acid was added thereto, and the mixture was stirred at room temperature f or 1 hour, and then the crystals were filtered off, washed with water, and dried under reduced pressure to give the titled compound (0.97 g) as white crystals (yield 86%).
MS (ESI+) :332 (M+H)
1H NMR (300 MHz, $CDCl_3$) δ ppm 1.66 - 1.90 (4 H, m) 2.60 (4 H, t, J=6.2 Hz) 5.60 (2 H, s) 8.2 8(1 H, s).

Example 86

2-{[3-(Trifluoromethyl)-4,5,6,7-tetrahydro-1H-indazol-1-yl]methyl}pyridine-4-carboxylic acid

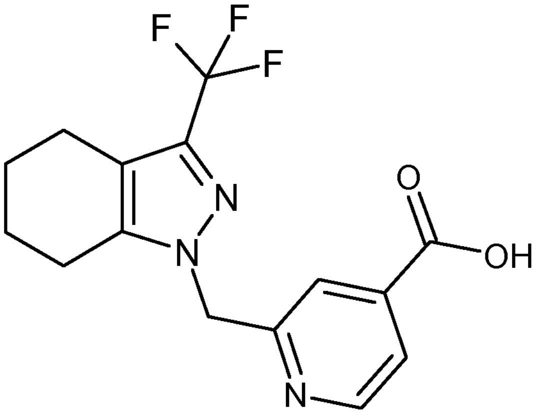

To a mixture of 3-(trifluoromethyl)-4,5,6,7-tetrahydro-1H-indazole (1.0 g, 5.26 mmol), methyl 2-(chloromethyl)pyridine-4-carboxylate (1.17 g, 6.31 mmol), and DMF (15 ml), was added pota ssium tert-butoxide (0.71 g, 6.31 mmol) at 0 °C, and the mixture was stirred at room temperat ure for 13 hours. To the reaction mixture, was added water, and the mixture was extracted wi th ethyl acetate. The organic layer was washed with water, and brine, dried over magnesium s ulfate, and concentrated under reduced pressure. The obtained residue was purified by colum n chromatography on silica gel [developing solvent: hexane-ethyl acetate (9:1-4:1)], recrystalli zed from ethyl acetate-hexane to give methyl 2-{[3-(trifluoromethyl)-4,5,6,7-tetrahydro-1H-ind azol-1-yl]methyl}pyridine-4-carboxylate (0.89 g) as yellow oily substance. The substance was d issolved in ethanol (15 ml), and then 2N aqueous sodium hydroxide (3.0 ml) was added thereto . The mixture was stirred at 50 °C for 5 hours. After cooling to room temperature, 1N hydrochl oric acid was added thereto, and the mixture was stirred at room temperature for 1 hour, and then the crystals were filtered off, and washed with water, dried under reduced pressure to gi ve the titled compound (0.72 g) as white crystals (yield 84%).
MS (ESI+) :326 (M+H)
1H NMR (300 MHz, $CDCl_3$) δ ppm 1.58 - 2.03 (4 H, m) 2.53 - 2.66 (4 H, m) 5.44 (2 H, s) 7.63 (1 H, s) 7.80 (1 H, d, J=5.3 Hz) 8.74 (1 H, d, J=5.3 Hz).

Example 87

N,N-Diethyl-4-{2-[3-(trifluoromethyl)-4,5,6,7-tetrahydro-1H-indazol-1-yl]ethoxy}benzamide

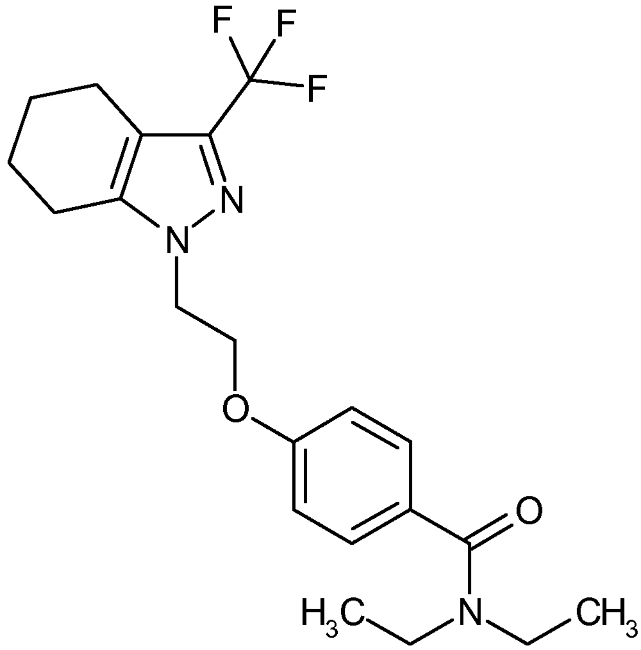

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 17.8 mg
MS(ESI+):410(M+H)

Example 88

1-Ethyl-4-{[(4-{2-[3-(trifluoromethyl)-4,5,6,7-tetrahydro-1H-indazol-1-yl]ethoxy}phenyl)carbon yl]amino}-1H-pyrazole-5-carboxamide

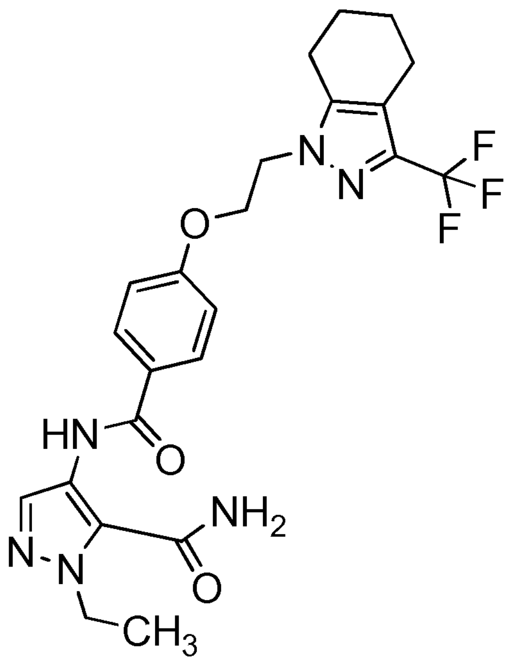

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 8.7 mg
MS(ESI+):491(M+H)

Example 89

N-(1-Methyl-1H-pyrazol-3-yl)-4-{2-[3-(trifluoromethyl)-4,5,6,7-tetrahydro-1H-indazol-1-yl]etho xy}benzamide

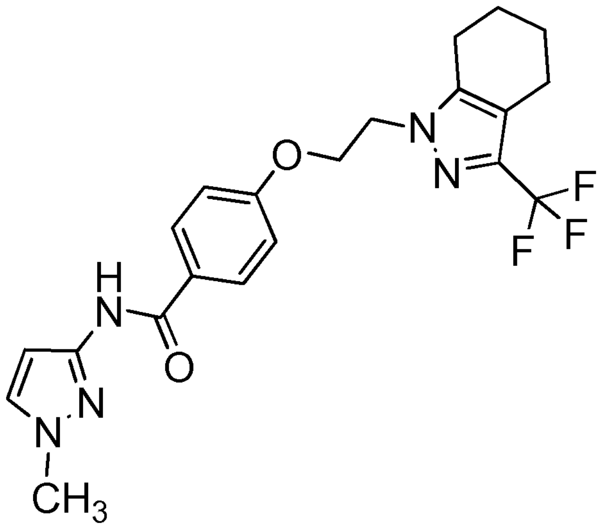

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 13.8 mg
MS(ESI+):434(M+H)

Example 90

4,5-Dimethyl-2-{[(4-{2-[3-(trifluoromethyl)-4,5,6,7-tetrahydro-1H-indazol-1-yl]ethoxy}phenyl)c arbonyl]amino}thiophene-3-carboxamide

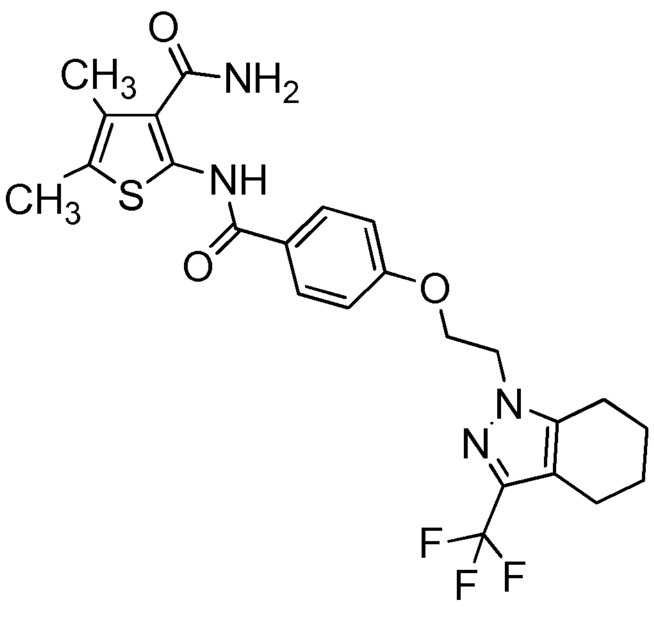

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 7.2 mg
MS(ESI+):507(M+H)

Example 91

Methyl 5-carbamoyl-4-methyl-2-{[(4-{2-[3-(trifluoromethyl)-4,5,6,7-tetrahydro-1H-indazol-1-yl] ethoxy}phenyl)carbonyl] amino}thiophene-3-carboxylate

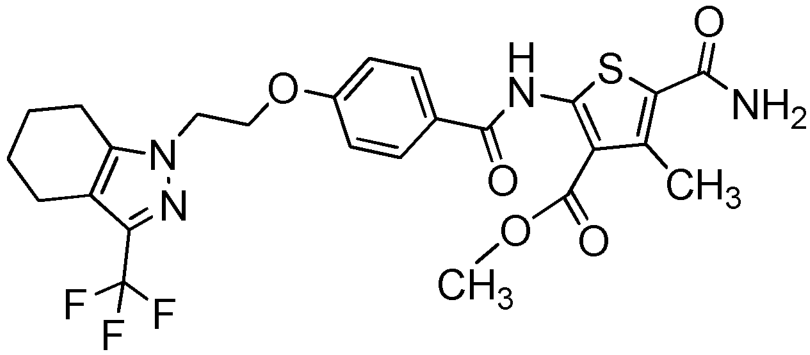

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 2.1 mg
MS(ESI+):551(M+H)

Example 92

N-(3-Oxo-2,3-dihydro-1H-isoindol-4-yl)-4-{2-[3-(trifluoromethyl)-4,5,6,7-tetrahydro-1H-indazo 1-1-yl]ethoxy}benzamide

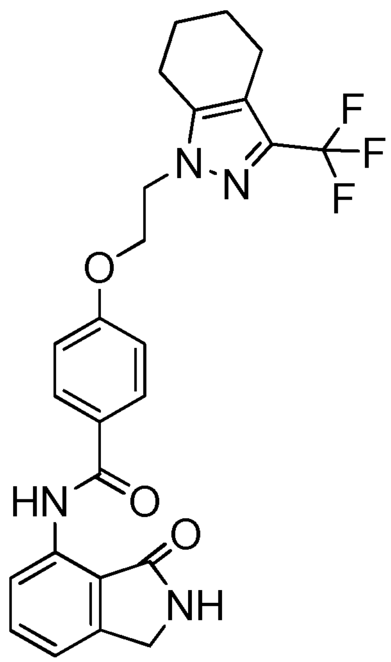

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 5.2 mg

MS(ESI+):485(M+H)

Example 93

N-Phenyl-4-{2-[3-(trifluoromethyl)-4,5,6,7-tetrahydro-1H-indazol-1-yl]ethoxy}benzamide

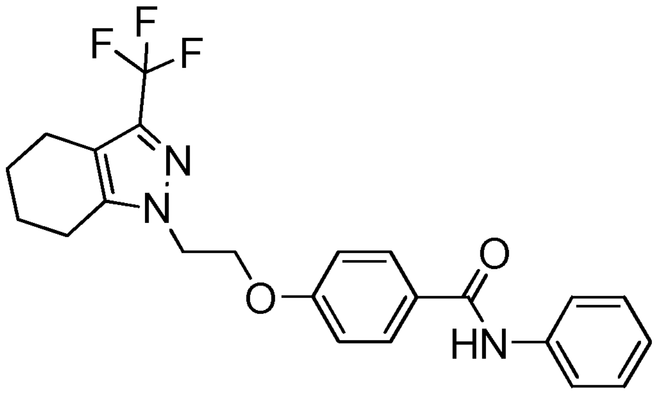

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 8.5 mg
MS(ESI+):430(M+H)

Example 94

5-Chloro-2-{[(4-{2-[3-(trifluoromethyl)-4,5,6,7-tetrahydro-1H-indazol-1-yl]ethoxy}phenyl)carbo nyl]amino}benzamide

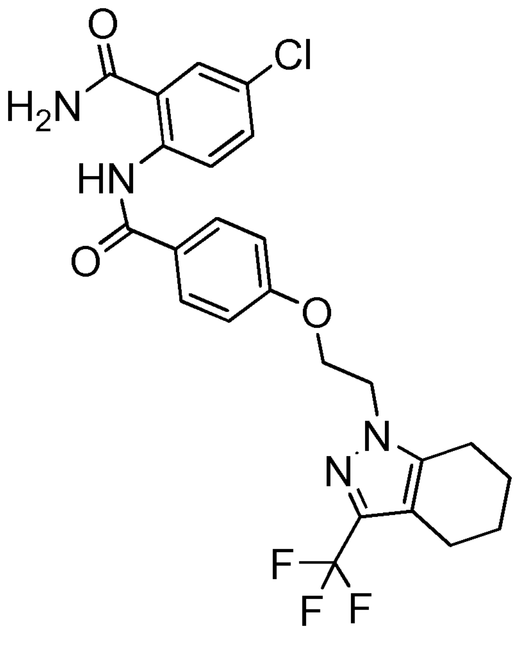

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.

Yield: 5.3 mg
MS(ESI+):507(M+H)

Example 95

N-(5-Chloro-2-methoxyphenyl)-4-{2-[3-(trifluoromethyl)-4,5,6,7-tetrahydro-1H-indazol-1-yl]eth oxy}benzamide

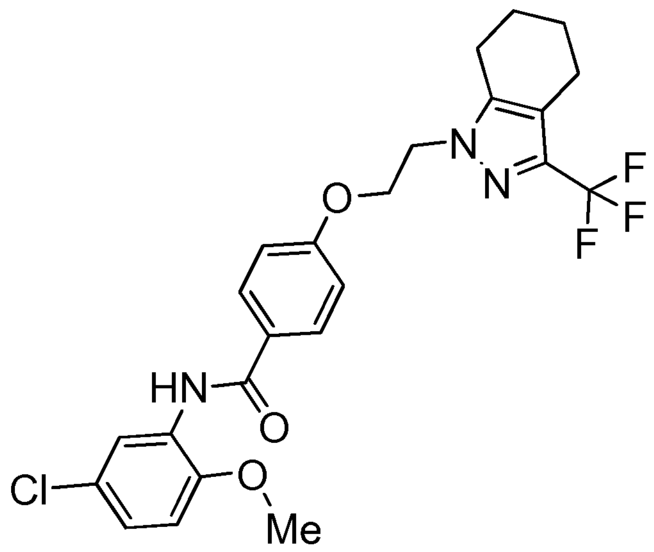

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 7.4 mg
MS(ESI+):494(M+H)

Example 96

N-(4-Fluorobenzyl)-4-{2-[3-(trifluoromethyl)-4,5,6,7-tetrahydro-1H-indazol-1-yl]ethoxy}benzam ide

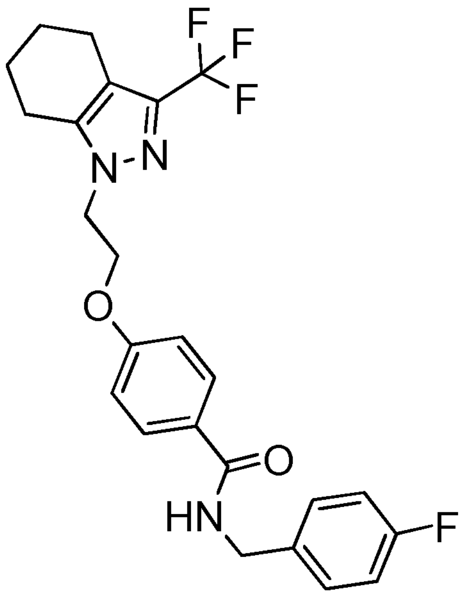

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 12.8 mg
MS(ESI+):462(M+H)

Example 97

N-[(3S,5S,7S)-Tricyclo[3.3.1.1$^{3,7}$]dec-1-yl]-4-{2-[3-(trifluoromethyl)-4,5,6,7-tetrahydro-1H-inda zol-1-yl]ethoxy}benzamide

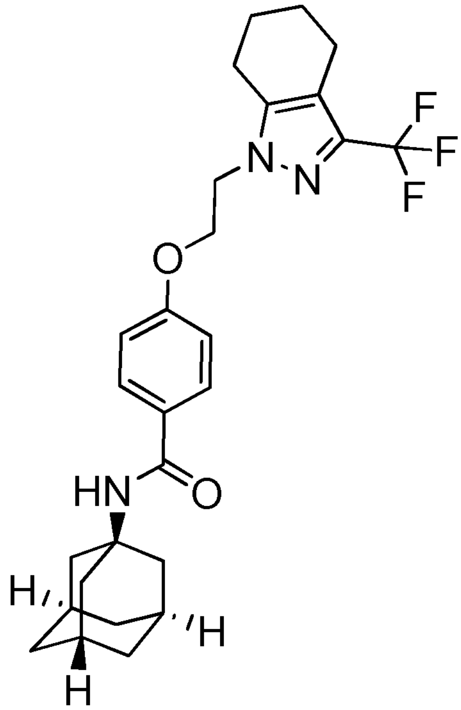

Using 4-{2-[3-(trifluoromethyl)-4,5,6,7-tetrahydro-1H-indazol-1-yl]ethoxy}benzoic acid obtaine d in Example 84, the title

compound was obtained in the same manner as in Example 3.
Yield: 19.3 mg
MS(ESI+):488(M+H)

Example 98

N-(Thiophen-2-ylmethyl)-4-{2-[3-(trifluoromethyl)-4,5,6,7-tetrahydro-1H-indazol-1-yl]ethoxy}b enzamide

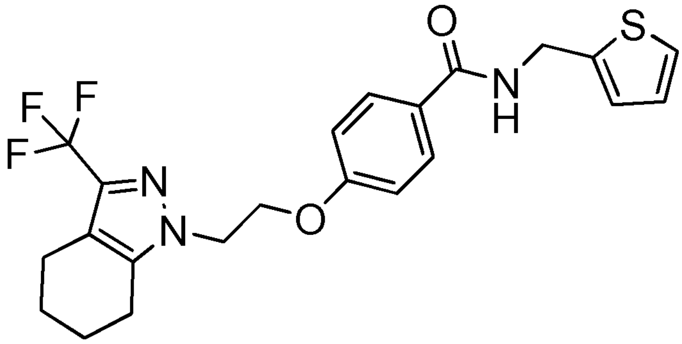

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 13.1 mg
MS(ESI+):450(M+H)

Example 99

N,N-dimethyl-4-{2-[3-(trifluoromethyl)-4,5,6,7-tetrahydro-1H-indazol-1-yl]ethoxy}benzamide

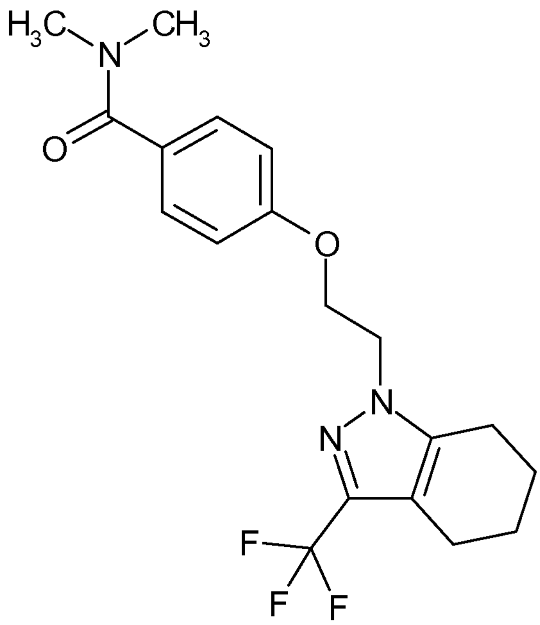

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.

Yield: 13.3 mg
MS(ESI+):382(M+H)

Example 100

N-(1-Methylethyl)-4-{2-[3-(trifluoromethyl)-4,5,6,7-tetrahydro-1H-indazol-1-yl]ethoxy}benzami de

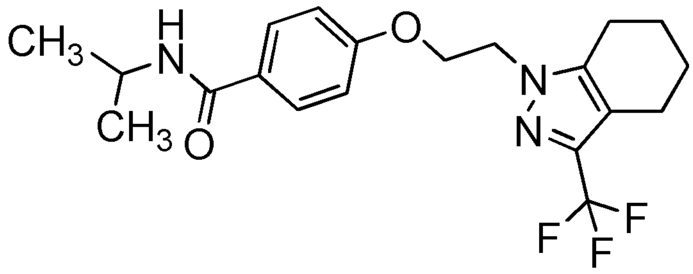

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 10.4 mg
MS(ESI+):396(M+H)

Example 101

1-{2-[4-(Pyrrolidin-1-ylcarbonyl)phenoxy]ethyl}-3-(trifluoromethyl)-4,5,6,7-tetrahydro-1H-inda zole

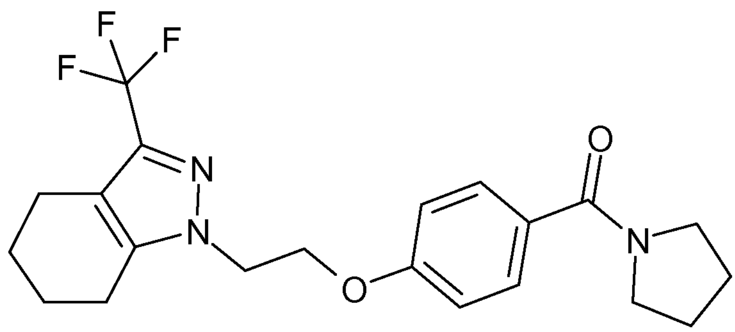

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 14.9 mg
MS(ESI+):408(M+H)

Example 102

N-(2-Hydroxyethyl)-4-{2-[3-(trifluoromethyl)-4,5,6,7-tetrahydro-1H-indazol-1-yl]ethoxy}benza mide

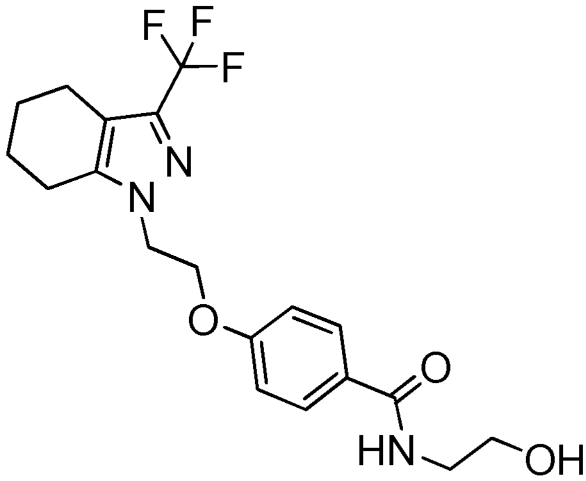

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 7.3 mg
MS(ESI+):398(M+H)

Example 103

N-(3-Amino-3-oxopropyl)-4-{2-[3-(trifluoromethyl)-4,5,6,7-tetrahydro-1H-indazol-1-yl]ethoxy}b enzamide

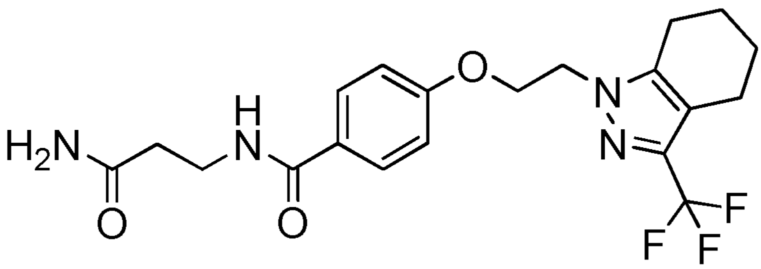

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 10.4 mg
MS(ESI+):425(M+H)

Example 104

N-[2-(Dimethylamino)-1-phenylethyl]-4-{2-[3-(trifluoromethyl)-4,5,6,7-tetrahydro-1H-indazol-1-yl]ethoxy}benzamide

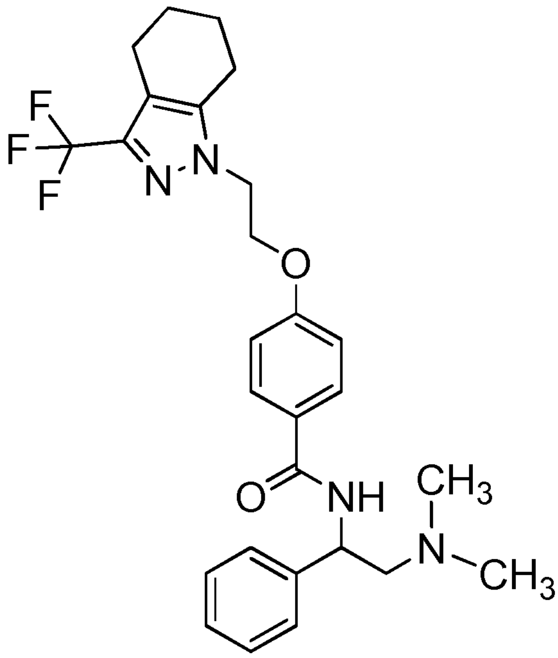

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 11.8 mg
MS(ESI+):501(M+H)

Example 105

N-1,3-Thiazol-2-yl-4-{2-[3-(trifluoromethyl)-4,5,6,7-tetrahydro-1H-indazol-1-yl]ethoxy}benzami de

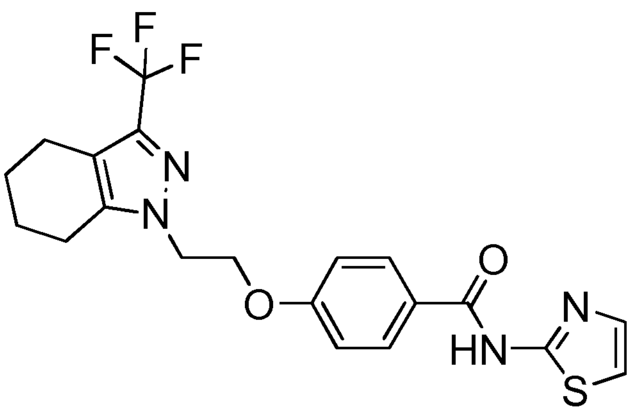

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 12.1 mg
MS(ESI+):437(M+H)

Example 106

N-(4-Sulfamoylbenzyl)-4-{2-[3-(trifluoromethyl)-4,5,6,7-tetrahydro-1H-indazol-1-yl]ethoxy}ben zamide

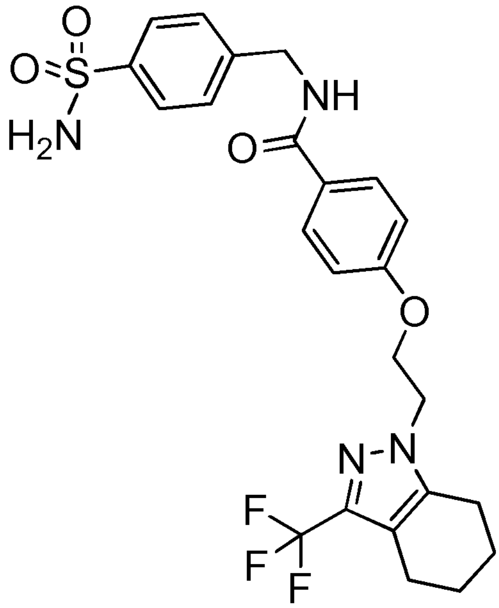

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 15.6 mg
MS(ESI+):523(M+H)

Example 107

1-(2-{4-[(2-Furan-2-ylpyrrolidin-1-yl)carbonyl]phenoxy}ethyl)-3-(trifluoromethyl)-4,5,6,7-tetrah ydro-1H-indazole

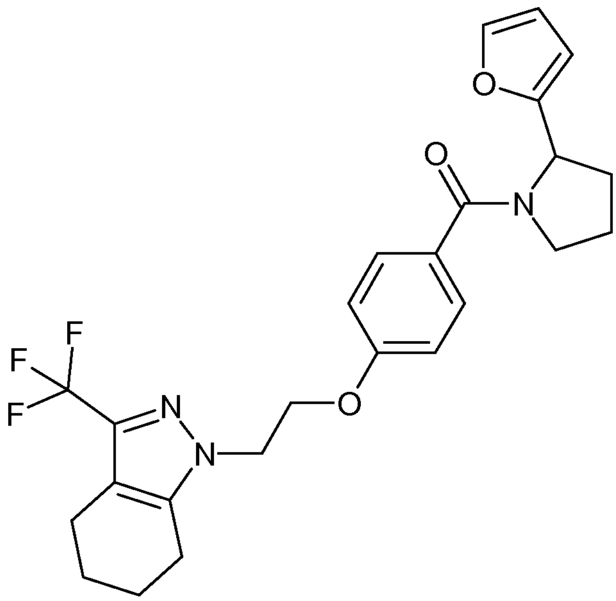

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 13.3 mg
MS(ESI+):474(M+H)

Example 108

N-Pyridin-3-yl-4-{2-[3-(trifluoromethyl)-4,5,6,7-tetrahydro-1H-indazol-1-yl]ethoxy}benzamide

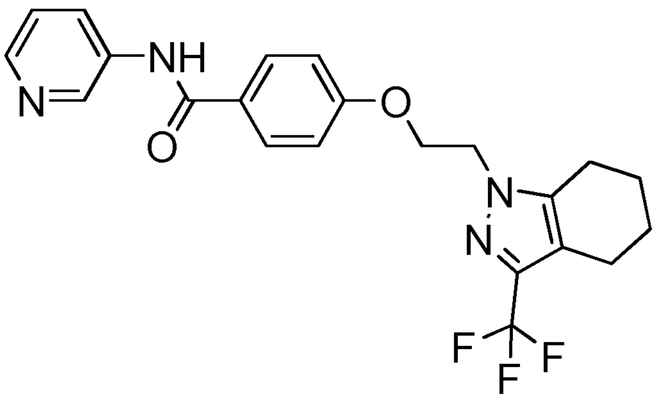

Using 4-{2-[3-(trifluoromethyl)-4,5,6,7-tetrahydro-1H-indazol-1-yl]ethoxy}benzoic acid obtaine d in Example 84, the title compound was obtained in the same manner as in Example 3.
Yield: 4.0 mg
MS(ESI+):431(M+H)

Example 109

N,N-Diethyl-2-{[3-(trifluoromethyl)-4,5,6,7-tetrahydro-1H-indazol-1-yl]methyl}pyridine-4-carb oxamide

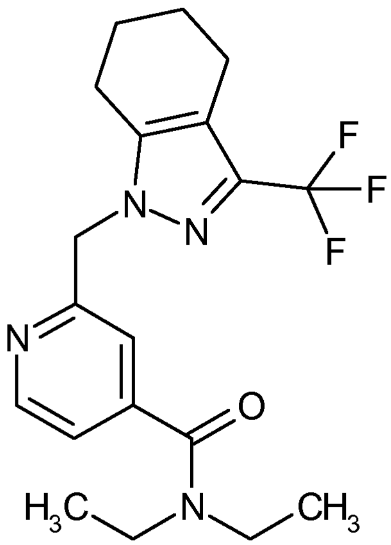

Using 2-{[3-(trifluoromethyl)-4,5,6,7-tetrahydro-1H-indazol-1-yl]methyl}pyridine-4-carboxylic acid obtained in Example 84, the title compound was obtained in the same manner as in Exa mple 3.
Yield: 14.8 mg
MS(ESI+):381(M+H)

Example 110

N-(5-Carbamoyl-1-ethyl-1H-pyrazol-4-yl)-2-{[3-(trifluoromethyl)-4,5,6,7-tetrahydro-1H-indazo 1-1-yl]methyl}pyridine-4-carboxamide

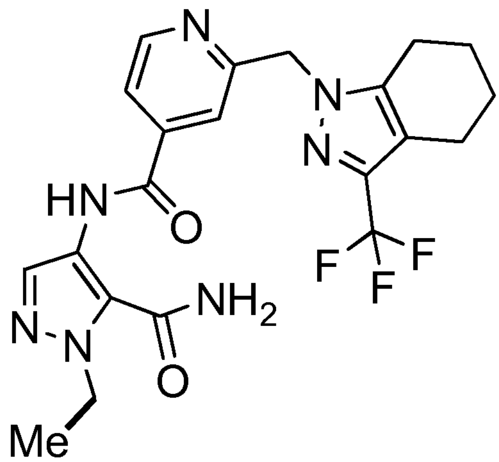

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 7.5 mg
MS(ESI+):462(M+H)

Example 111

N-(1-Methyl-1H-pyrazol-3-yl)-2-{[3-(trifluoromethyl)-4,5,6,7-tetrahydro-1H-indazol-1-yl]methy l}pyridine-4-carboxamide

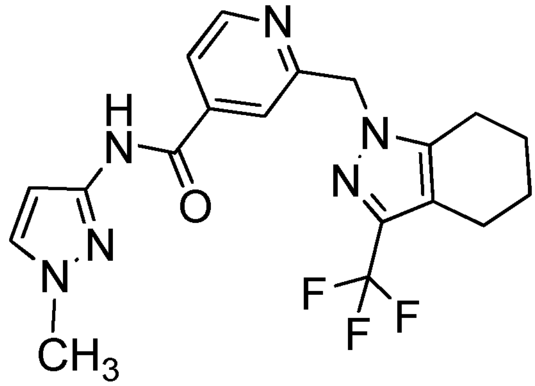

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 10.1 mg
MS(ESI+):405(M+H)

Example 112

N-(3-Carbamoyl-4,5-dimethylthiophen-2-yl)-2-{[3-(trifluoromethyl)-4,5,6,7-tetrahydro-1H-inda zol-1-yl]methyl}pyridine-4-carboxamide

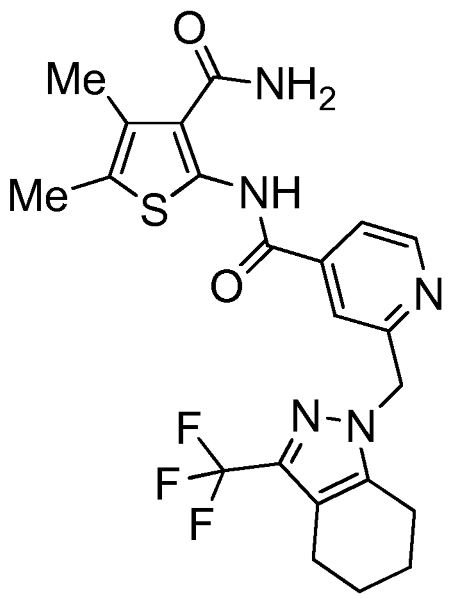

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 4.8 mg
MS(ESI+):478(M+H)

Example 113

N-(3-Carbamoyl-4,5,6,7-tetrahydro-1-benzothiophen-2-yl)-2-{[3-(trifluoromethyl)-4,5,6,7-tetrah ydro-1H-indazol-1-yl]methyl}pyridine-4-carboxamide

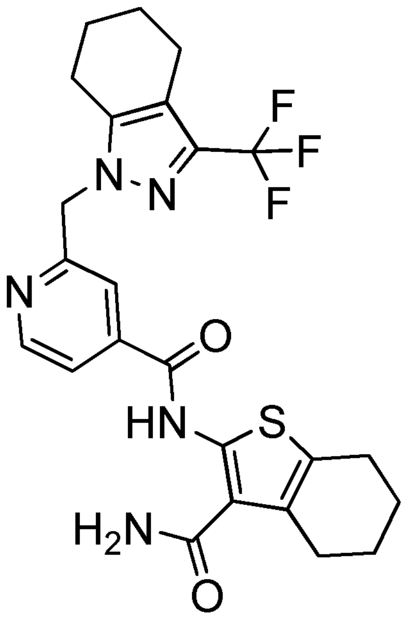

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 2.2 mg
MS(ESI+):504(M+H)

Example 114

N-Phenyl-2-{[3-(trifluoromethyl)-4,5,6,7-tetrahydro-1H-indazol-1-yl]methyl}pyridine-4-carbox amide

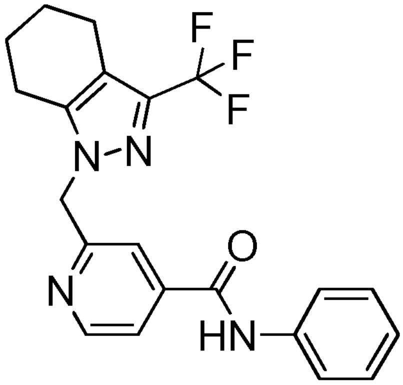

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 14.2 mg
MS(ESI+):401(M+H)

Example 115

N-(2-Carbamoyl-4-chlorophenyl)-2-{[3-(trifluoromethyl)-4,5,6,7-tetrahydro-1H-indazol-1-yl]me thyl}pyridine-4-carboxamide

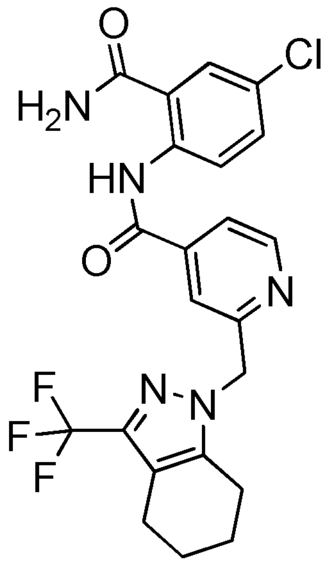

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 1.2 mg
MS(ESI+):478(M+H)

Example 116

N-(5-Chloro-2-methoxyphenyl)-2-{[3-(trifluoromethyl)-4,5,6,7-tetrahydro-1H-indazol-1-yl]meth yl}pyridine-4-carboxamide

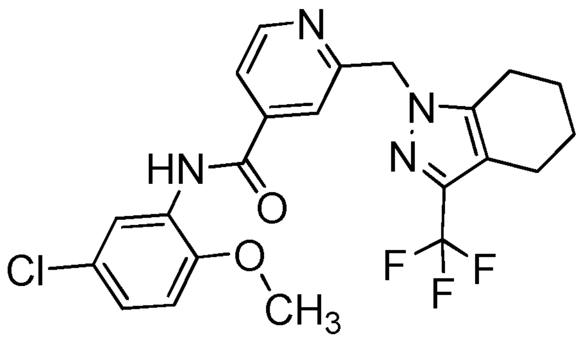

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 3.6 mg
MS(ESI+):465(M+H)

Example 117

N-(4-Fluorobenzyl)-2-{[3-(trifluoromethyl)-4,5,6,7-tetrahydro-1H-indazol-1-yl]methyl}pyridine-4-carboxamide

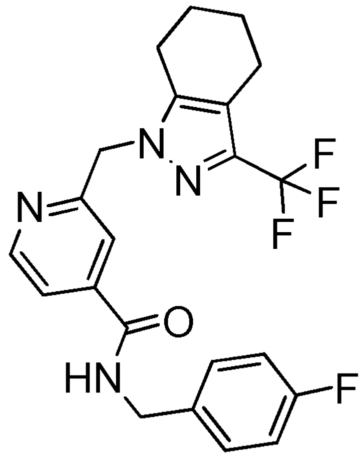

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 9.3 mg
MS(ESI+):433(M+H)

Example 118

N-[(3S,5S,7S)-tricyclo[3.3.1.1$^{3,7}$]dec-1-yl]-2-{[3-(trifluoromethyl)-4,5,6,7-tetrahydro-1H-indazo 1-1-yl]methyl}pyridine-4-carboxamide

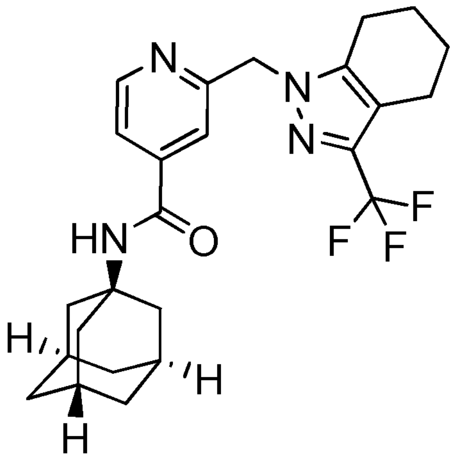

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 18.6 mg
MS(ESI+):479(M+H)

Example 119

N-(Thiophen-2-ylmethyl)-2-{[3-(trifluoromethyl)-4,5,6,7-tetrahydro-1H-indazol-1-yl]methyl}pyr idine-4-carboxamide

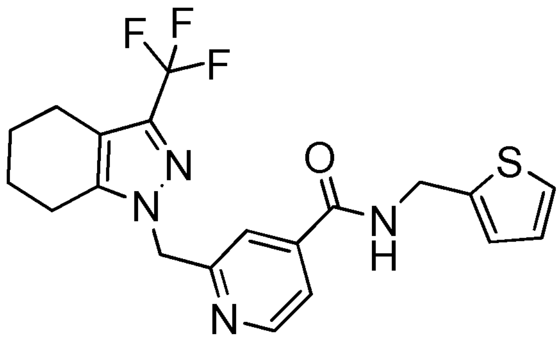

A 0.12 M solution of 2-{[3-(trifluoromethyl)-4,5,6,7-tetrahydro-1H-indazol-1-yl]methyl}pyridin e-4-carboxylic acid in DMF (500 μl, 60 μmol), and a 0.144 M solution of 1-thiophen-2-ylmethan amine in DMF (500 μl, 72 μmol), and a 0.24 M solution of HATU and DIEA in DMF (500 μl, 1 20 μmol) were mixed at room temperature, and the mixture was stirred at 60 °C for 24 hours. The reaction mixture was cooled to room temperature, ethyl acetate (3 ml), and 2% sodium hy drogen carbonate aqueous solution (1.5 ml) was added thereto, and the mixture was extracted. The organic layer was collected with upper phase sep tube (Wako Pure Chemical Industries). The solvent was evaporated off under reduced pressure, and the residue was dissolved in DM SO-methanol (1:1) (1 ml), and purified by preparative HPLC to give the titled compound.
Yield: 14.2 mg
MS(ESI+):421(M+H)

Example 120

N,N-Dimethyl-2-{[3-(trifluoromethyl)-4,5,6,7-tetrahydro-1H-indazol-1-yl]methyl}pyridine-4-ca rboxamide

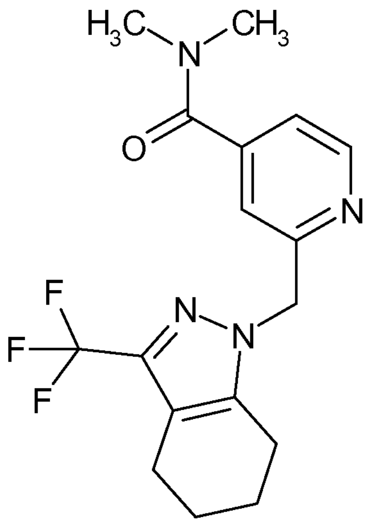

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 9.8 mg
MS(ESI+):353(M+H)

Example 121

N-(1-Methylethyl)-2-{[3-(trifluoromethyl)-4,5,6,7-tetrahydro-1H-indazol-1-yl]methyl}pyridine-4-carboxamide

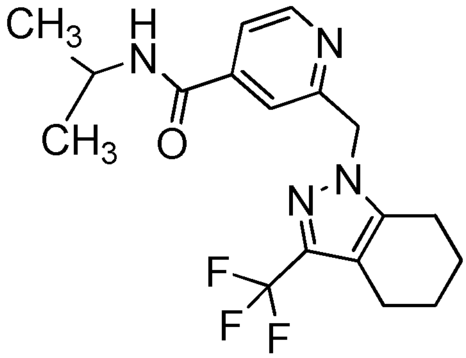

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 12.1 mg
MS(ESI+):367(M+H)

Example 122

N-(2-Hydroxyethyl)-2-{[3-(trifluoromethyl)-4,5,6,7-tetrahydro-1H-indazol-1-yl]methyl}pyridin e-4-carboxamide

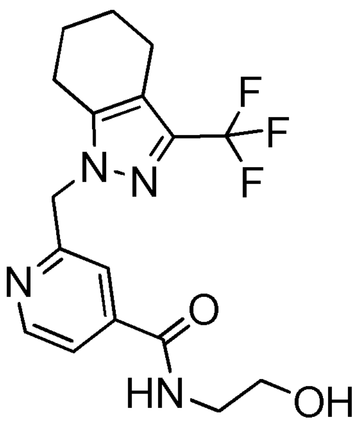

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 12.7 mg
MS(ESI+):369(M+H)

Example 123

N-(3-Amino-3-oxopropyl)-2-{[3-(trifluoromethyl)-4,5,6,7-tetrahydro-1H-indazol-1-yl]methyl}pyr idine-4-carboxamide

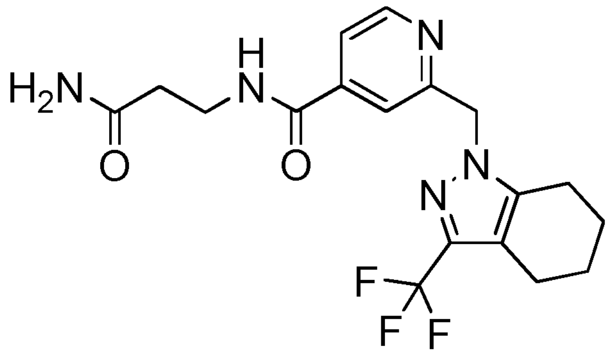

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 9.8 mg
MS(ESI+):396(M+H)

Example 124

N-[2-(Dimethylamino)-1-phenylethyl]-2-{[3-(trifluoromethyl)-4,5,6,7-tetrahydro-1H-indazol-1-yl]methyl}pyridine-4-carboxamide

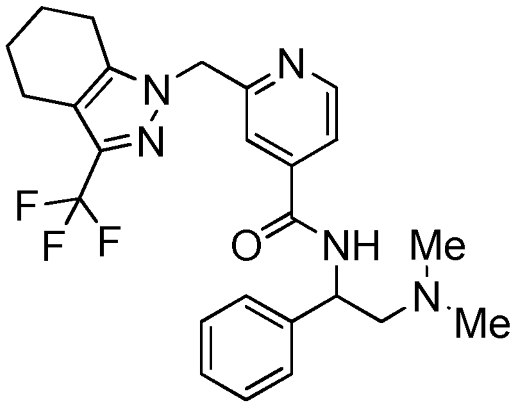

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 23.0 mg
MS(ESI+):472(M+H)

Example 125

N-1,3-Thiazol-2-yl-2-{[3-(trifluoromethyl)-4,5,6,7-tetrahydro-1H-indazol-1-yl]methyl}pyridine-4-carboxamide

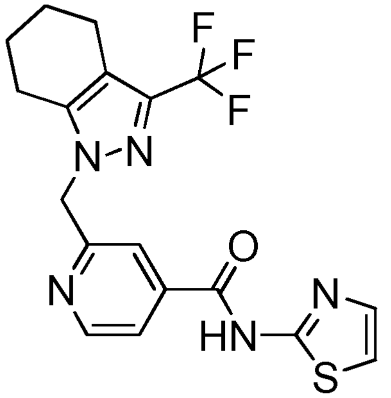

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 10.0 mg
MS(ESI+):408(M+H)

Example 126

N-(4-Sulfamoylbenzyl)-2-{[3-(trifluoromethyl)-4,5,6,7-tetrahydro-1H-indazol-1-yl]methyl}pyrid ine-4-carboxamide

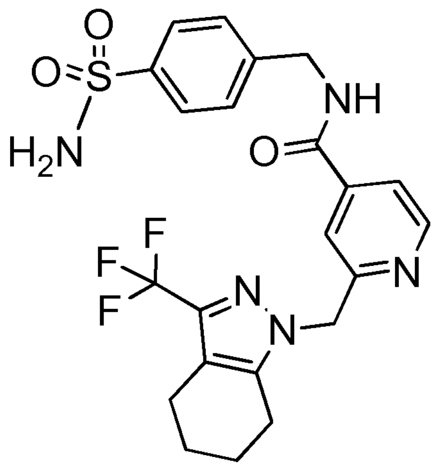

A0.12 M solution of 2-{[3-(trifluoromethyl)-4,5,6,7-tetrahydro-1H-indazol-1-yl]methyl}pyridin e-4-carboxylic acid in DMF (500 μl, 60 μmol), and a 0.144 M solution of 4-(aminomethyl)benze nesulfonamide in DMF (500 μl, 72 μmol), and a 0.24 M solution of HATU and DIEA in DMF ( 500 μl, 120 μmol) were mixed at room temperature, and the mixture was stirred at 60 °C for 2 4 hours. The reaction mixture was cooled to room temperature, ethyl acetate (3 ml), and 2% so dium hydrogen carbonate aqueous solution (1.5 ml) were added thereto, and the mixture was extracted. The organic layer was collected with upper phase sep tube (Wako Pure Chemical In dustries). The solvent was evaporated off under reduced pressure, and the residue was dissolv ed in DMSO-methanol (1:1) (1 ml), and purified with preparative HPLC to give the titled com pound.
Yield: 16.8 mg
MS(ESI+):494(M+H)

Example 127

1-({4-[(2-Furan-2-ylpyrrolidin-1-yl)carbonyl]pyridin-2-yl}methyl)-3-(trifluoromethyl)-4,5,6,7-tet rahydro-1H-indazole

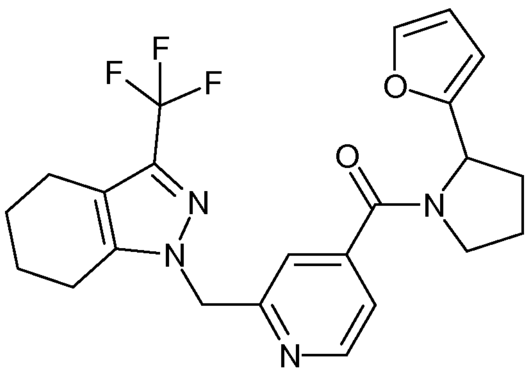

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 15.5 mg
MS(ESI+):445(M+H)

Example 128

N-Pyridin-3-yl-2-{[3-(trifluoromethyl)-4,5,6,7-tetrahydro-1H-indazol-1-yl]methyl}pyridine-4-ca rboxamide

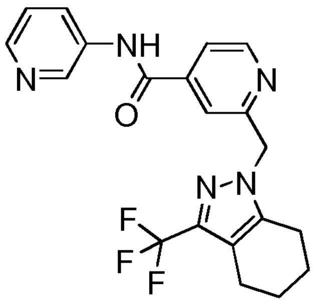

Using 2-{[3-(trifluoromethyl)-4,5,6,7-tetrahydro-1H-indazol-1-yl]methyl}pyridine-4-carboxylic acid obtained in Example 86, the title compound was obtained in the same manner as in Exa mple 3.
Yield: 7.8 mg
MS(ESI+):402(M+H)

Example 129

N,N-Diethyl-2-{[3-(trifluoromethyl)-4,5,6,7-tetrahydro-1H-indazol-1-yl]methyl}-1,3-thiazole-4-carboxamide

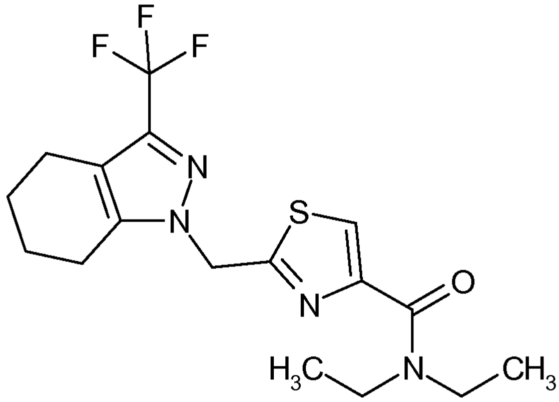

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 16.5 mg
MS(ESI+):387(M+H)

Example 130

N-(5-Carbamoyl-1-ethyl-1H-pyrazol-4-yl)-2-{[3-(trifluoromethyl)-4,5,6,7-tetrahydro-1H-indazo 1-1-yl]methyl}-1,3-thiazole-4-carboxamide

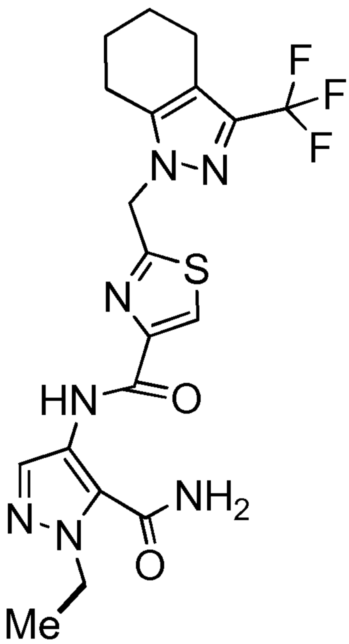

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 15.8 mg
MS(ESI+):468(M+H)

Example 131

N-(1-Methyl-1H-pyrazol-3-yl)-2-{[3-(trifluoromethyl)-4,5,6,7-tetrahydro-1H-indazol-1-yl]methy 1}-1,3-thiazole-4-carboxamide

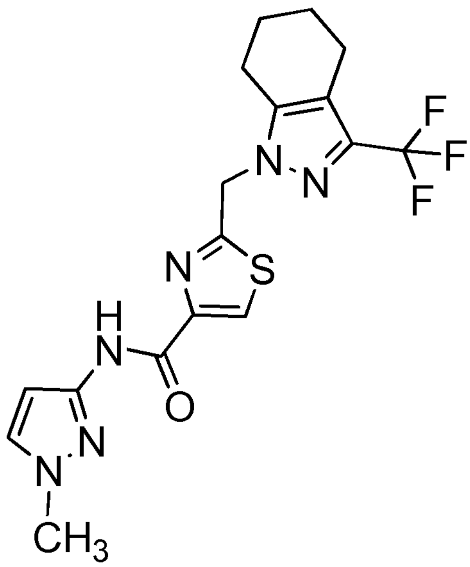

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 17.5 mg
MS(ESI+):411(M+H)

Example 132

N-(3-Carbamoyl-4,5-dimethylthiophen-2-yl)-2-{[3-(trifluoromethyl)-4,5,6,7-tetrahydro-1H-inda zol-1-yl]methyl}-1,3-thiazole-4-carboxamide

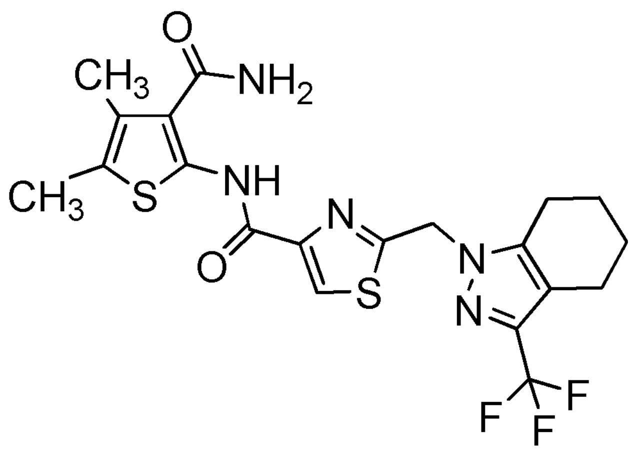

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 14.1 mg

MS(ESI+):484(M+H)

Example 133

Methyl 5-carbamoyl-4-methyl-2-{[(2-{[3-(trifluoromethyl)-4,5,6,7-tetrahydro-1H-indazol-1-yl]m ethyl}-1,3-thiazol-4-yl) carbonyl]amino}thiophene-3-carboxylate

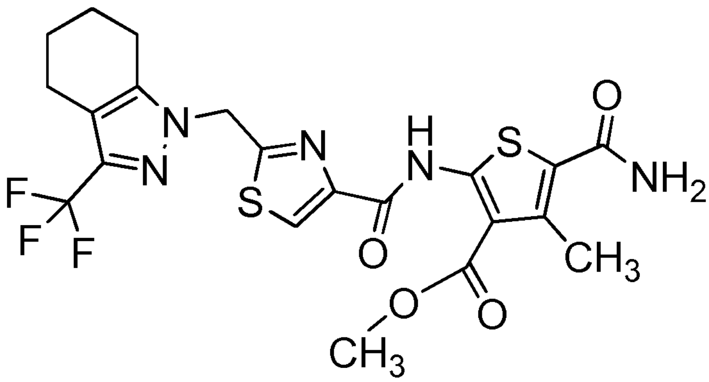

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 1.1 mg
MS(ESI+):528(M+H)

Example 134

N-(3-Carbamoyl-4,5,6,7-tetrahydro-1-benzothiophen-2-yl)-2-{[3-(trifluoromethyl)-4,5,6,7-tetrah ydro-1H-indazol-1-yl] methyl}-1,3-thiazole-4-carboxamide

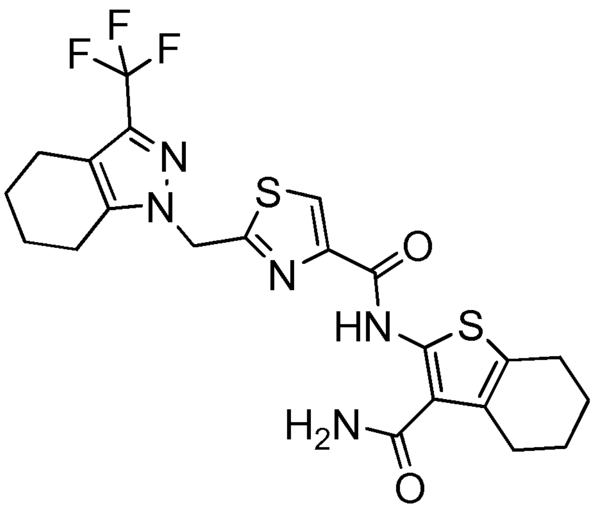

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 13.0 mg
MS(ESI+):510(M+H)

Example 135

N-(3-Oxo-2,3-dihydro-1H-isoindol-4-yl)-2-{[3-(trifluoromethyl)-4,5,6,7-tetrahydro-1H-indazol-1-yl]methyl}-1,3-thiazole-4-carboxamide

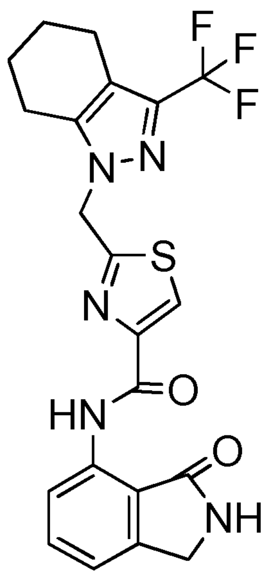

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 10.5 mg
MS(ESI+):462(M+H)

Example 136

N-Phenyl-2-{[3-(trifluoromethyl)-4,5,6,7-tetrahydro-1H-indazol-1-yl]methyl}-1,3-thiazole-4-car boxamide

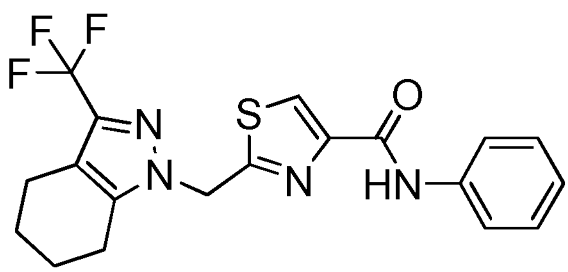

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 13.6 mg MS(ESI+):407(M+H)

Example 137

N-(2-Carbamoyl-4-chlorophenyl)-2-{[3-(trifluoromethyl)-4,5,6,7-tetrahydro-1H-indazol-1-yl]me thyl}-1,3-thiazole-4-carboxamide

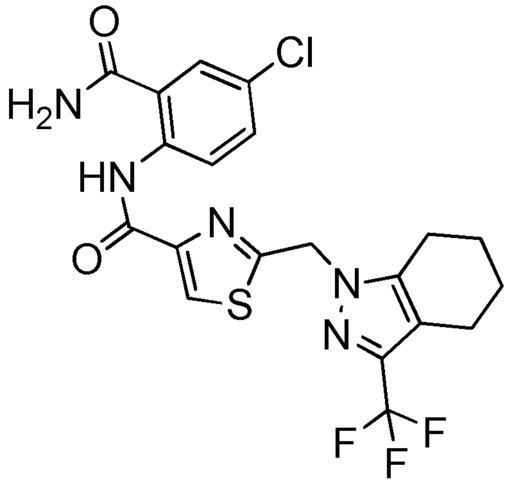

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 16.9 mg
MS(ESI+):484(M+H)

Example 138

N-(5-Chloro-2-methoxyphenyl)-2-{[3-(trifluoromethyl)-4,5,6,7-tetrahydro-1H-indazol-1-yl]meth yl}-1,3-thiazole-4-carboxamide

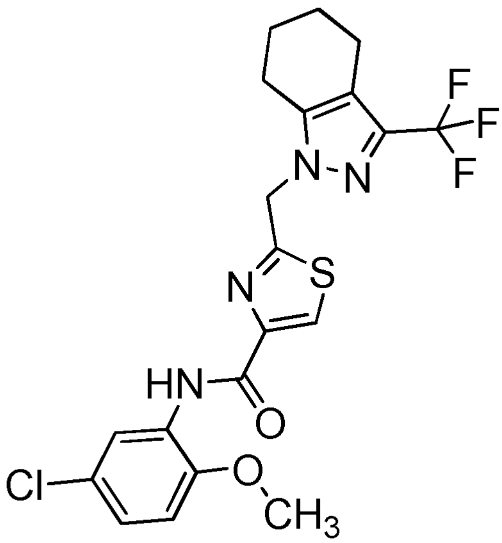

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 15.9 mg

MS(ESI+):471(M+H)

Example 139

N-(4-Fluorobenzyl)-2-{[3-(trifluoromethyl)-4,5,6,7-tetrahydro-1H-indazol-1-yl]methyl}-1,3-thia zole-4-carboxamide

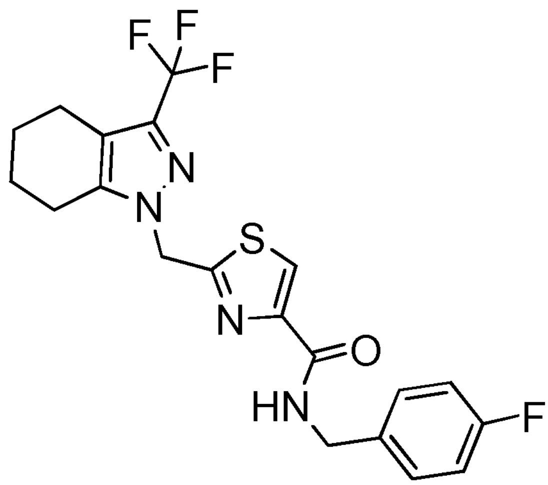

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 15.3 mg
MS(ESI+):439(M+H)

Example 140

N-[2-Chloro-5-(trifluoromethyl)phenyl]-2-{[3-(trifluoromethyl)-4,5,6,7-tetrahydro-1H-indazol-1-yl]methyl}-1,3-thiazole-4-carboxamide

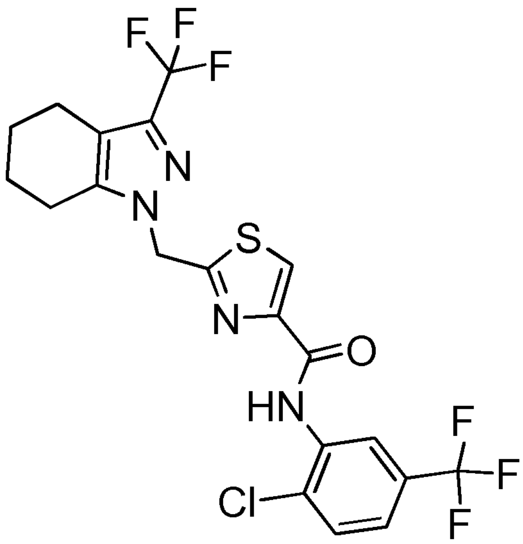

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.

Yield: 1.9 mg
MS(ESI+):509(M+H)

Example 141

N-[(3S,5S,7S)-Tricyclo[3.3.1.1$^{3,7}$]dec-1-yl]-2-{[3-(trifluoromethyl)-4,5,6,7-tetrahydro-1H-indaz ol-1-yl]methyl}-1,3-thiazole-4-carboxamide

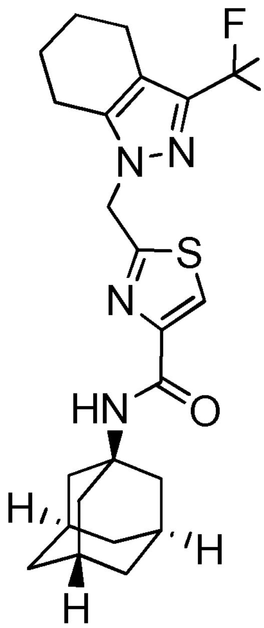

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 9.8 mg
MS(ESI+):465(M+H)

Example 142

N-(Thiophen-2-ylmethyl)-2-{[3-(trifluoromethyl)-4,5,6,7-tetrahydro-1H-indazol-1-yl]methyl}-1, 3-thiazole-4-carboxamide

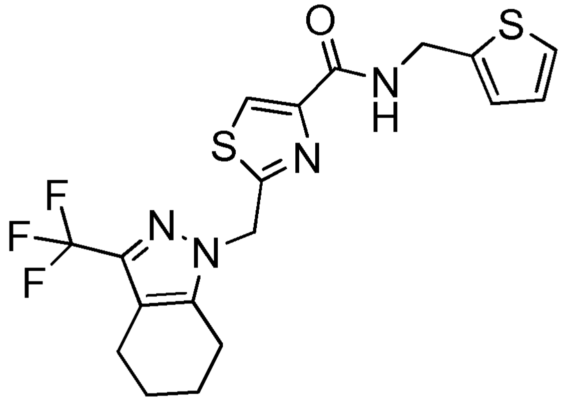

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 16.8 mg
MS(ESI+):427(M+H)

Example 143

N,N-Dimethyl-2-{[3-(trifluoromethyl)-4,5,6,7-tetrahydro-1H-indazol-1-yl]methyl}-1,3-thiazole-4-carboxamide

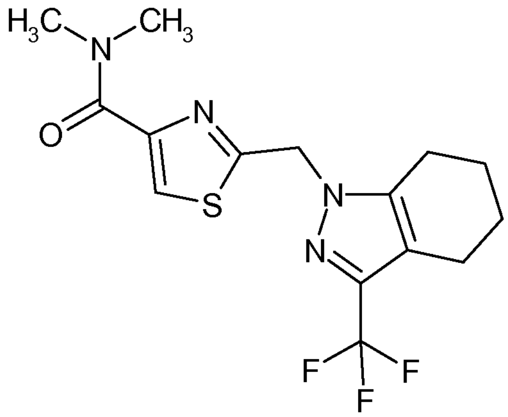

A 0.12 M solution of 2-{[3-(trifluoromethyl)-4,5,6,7-tetrahydro-1H-indazol-1-yl]methyl}-1,3-thi azole-4-carboxylic acid in DMF (500 μl, 60 μmol), and a 0.144 M solution of dimethylamine in DMF (500 μl, 72 μmol), and a 0.24 M solution of HATU and DIEA in DMF (500 μl, 120 μmol) were mixed at room temperature, and the mixture was stirred at 60 °C for 24 hours. The react ion mixture was cooled to room temperature, ethyl acetate(3 ml), and 2% sodium hydrogen car bonate aqueous solution (1.5 ml) were added thereto, and the mixture was extracted. The orga nic layer was collected with upper phase sep tube (Wako Pure Chemical Industries). The solve nt was evaporated off under reduced pressure. The obtained residue was dissolved in DMSO-methanol (1:1) (1 ml), and purified with preparative HPLC to give the titled compound.
Yield: 10.8 mg
MS(ESI+):359(M+H)

Example 144

N-(1-Methylethyl)-2-{[3-(trifluoromethyl)-4,5,6,7-tetrahydro-1H-indazol-1-yl]methyl}-1,3-thiaz ole-4-carboxamide

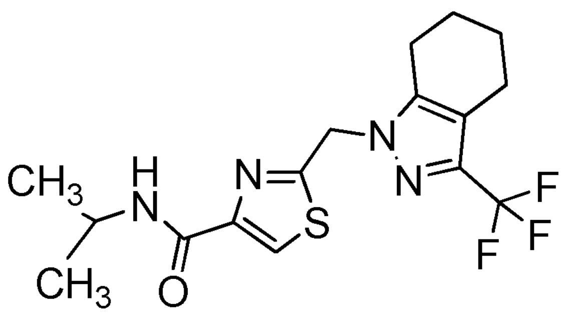

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.

Yield: 11.0 mg
MS(ESI+):373(M+H)

Example 145

1-{[4-(Pyrrolidin-1-ylcarbonyl)-1,3-thiazol-2-yl]methyl}-3-(trifluoromethyl)-4,5,6,7-tetrahydro-1 H-indazole

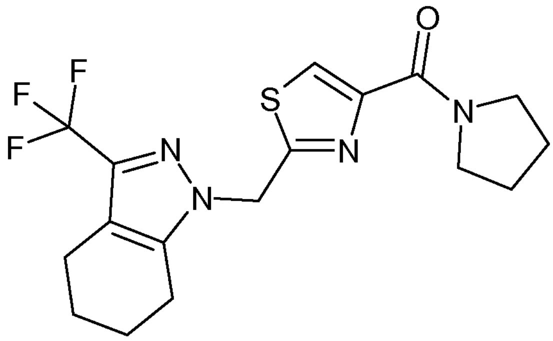

To a solution of 2-{[3-(trifluoromethyl)-4,5,6,7-tetrahydro-1H-indazol-1-yl]methyl}-1,3-thiazol e-4-carboxylic acid (185 mg, 0.56 mmol) in DMF (3 ml), were added WSC (128 mg, 0.67 mmol), HOBt (90 mg, 0.67 mmol), and pyrrolidine (56 μl, 0.67 mmol) at room temperature, and the m ixture was stirred for 13 hours. Water was added thereto, and the mixture was extracted with ethyl acetate. The organic layer was washed with water, and saturated saline, dried over mag nesium sulfate, and then concentrated under reduced pressure. The obtained residue was recr ystallized from ethyl acetate-hexane to give the titled compound (182 mg) as white crystals (yi eld 85%).
MS(ESI+):385(M+H)
1H NMR (300 MHz, $CDCl_3$) δ ppm 1.68 - 1.99 (8 H, m) 2.60 (4 H, t, J=6.1 Hz) 3.66 (2 H, t, J=6. 4 Hz) 3.82 (2 H, t, J=6.4 Hz) 5.52 (2 H, s) 8.00 (1 H, s).

Example 146

N-(2-Hydroxyethyl)-2-{[3-(trifluoromethyl)-4,5,6,7-tetrahydro-1H-indazol-1-yl]methyl}-1,3-thia zole-4-carboxamide

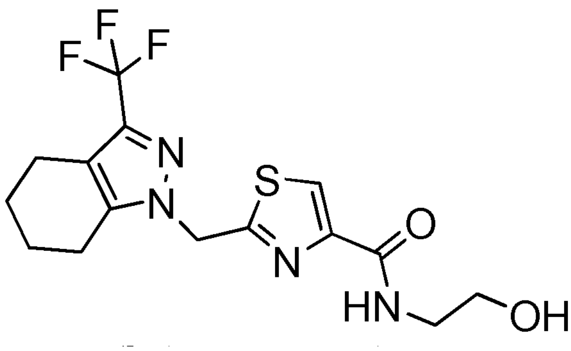

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 15.6 mg
MS(ESI+):375(M+H)

Example 147

N-(3-Amino-3-oxopropyl)-2-{[3-(trifluoromethyl)-4,5,6,7-tetrahydro-1H-indazol-1-yl]methyl}-1, 3-thiazole-4-carboxamide

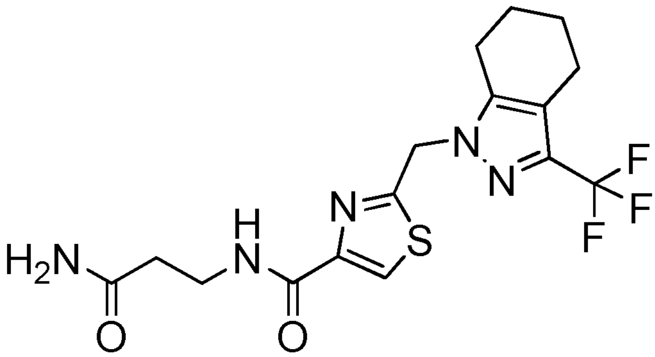

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 14.5 mg
MS(ESI+):402(M+H)

Example 148

N-[2-(Dimethylamino)-1-phenylethyl]-2-{[3-(trifluoromethyl)-4,5,6,7-tetrahydro-1H-indazol-1-yl]methyl}-1,3-thiazole-4-carboxamide

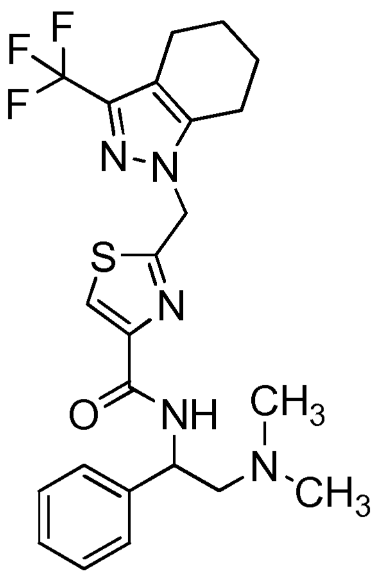

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 22.4 mg
MS(ESI+):478(M+H)

Example 149

N-1,3-Thiazol-2-yl-2-{[3-(trifluoromethyl)-4,5,6,7-tetrahydro-1H-indazol-1-yl]methyl}-1,3-thiaz ole-4-carboxamide

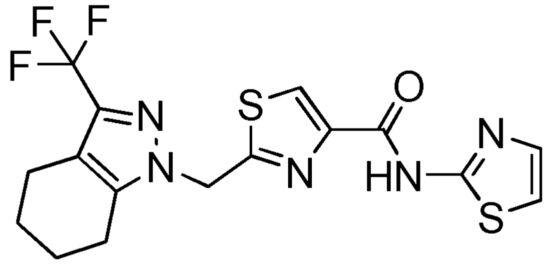

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 13.9 mg
MS(ESI+):414(M+H)

Example 150

N-(4-Sulfamoylbenzyl)-2-{[3-(trifluoromethyl)-4,5,6,7-tetrahydro-1H-indazol-1-yl]methyl}-1,3-t hiazole-4-carboxamide

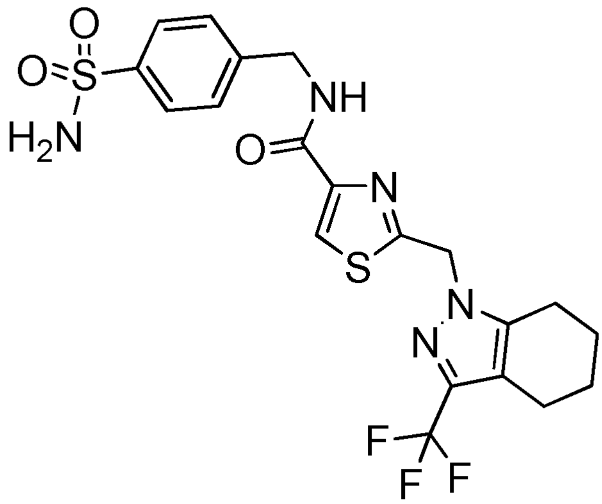

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 14.8 mg
MS(ESI+):500(M+H)

Example 151

1-({4-[(2-Furan-2-ylpyrrolidin-1-yl)carbonyl]-1,3-thiazol-2-yl}methyl)-3-(trifluoromethyl)-4,5,6, 7-tetrahydro-1H-indazole

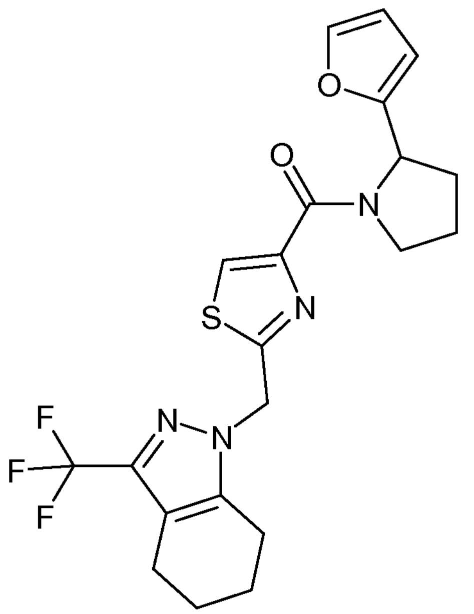

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 17.2 mg
MS(ESI+):451(M+H)

Example 152

N-Pyridin-3-yl-2-{[3-(trifluoromethyl)-4,5,6,7-tetrahydro-1H-indazol-1-yl]methyl}-1,3-thiazole-4-carboxamide

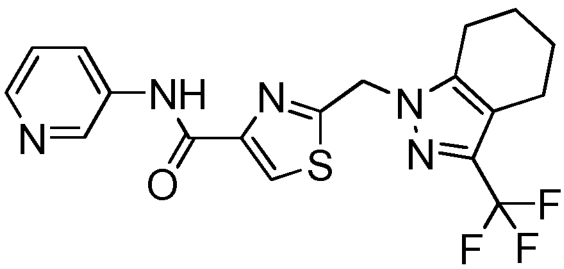

Using 2-{[3-(trifluoromethyl)-4,5,6,7-tetrahydro-1H-indazol-1-yl]methyl}-1,3-thiazole-4-carbox ylic acid obtained in Example 85, the title compound was obtained in the same manner as in E xample 3.
Yield: 17.9 mg
MS(ESI+):408(M+H)

Example 153

1-Ethyl-4-({4-[3-(trifluoromethyl)-4,5,6,7-tetrahydro-1H-indazol-1-yl]butanoyl}amino)-1H-pyrazole-5-carboxamide

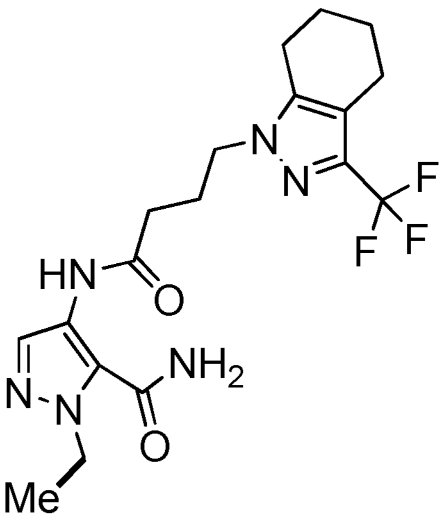

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 12.0 mg
MS(ESI+):413(M+H)

Example 154

2-({4-[3-(Trifluoromethyl)-4,5,6,7-tetrahydro-1H-indazol-1-yl]butanoyl}amino)-4,5,6,7-tetrahyd ro-1-benzothiophene-3-carboxamide

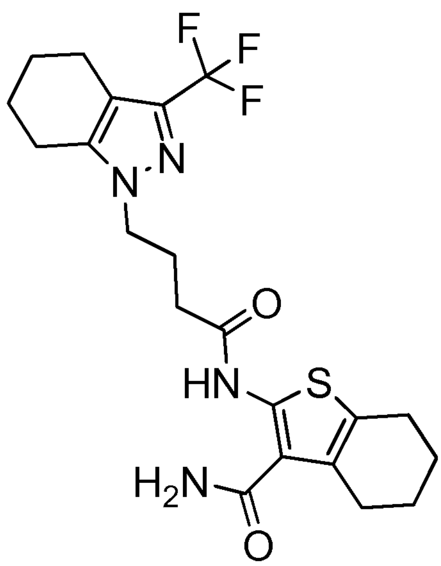

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 9.3 mg
MS(ESI+):455(M+H)

Example 155

5-Chloro-2-({4-[3-(trifluoromethyl)-4,5,6,7-tetrahydro-1H-indazol-1-yl]butanoyl}amino)benzam ide

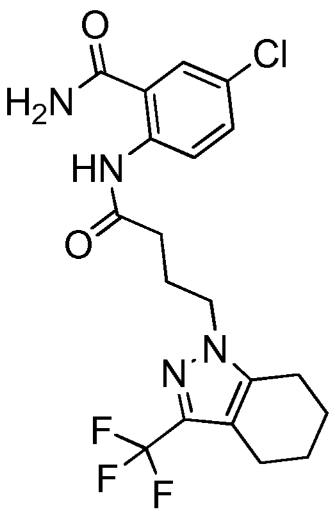

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 6.1 mg
MS(ESI+):429(M+H)

Example 156

N-(5-Chloro-2-methoxyphenyl)-4-[3-(trifluoromethyl)-4,5,6,7-tetrahydro-1H-indazol-1-yl]butan amide

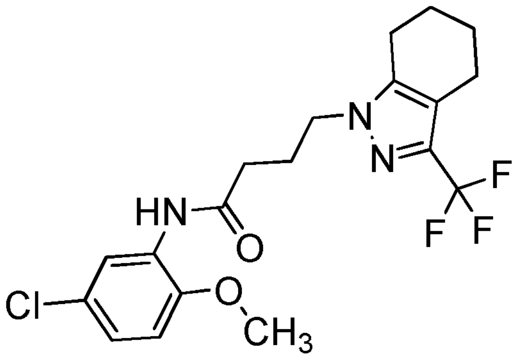

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 14.0 mg
MS(ESI+):416(M+H)

Example 157

3-({4-[3-(Trifluoromethyl)-4,5,6,7-tetrahydro-1H-indazol-1-yl]butanoyl}amino)thiophene-2-carb oxamide

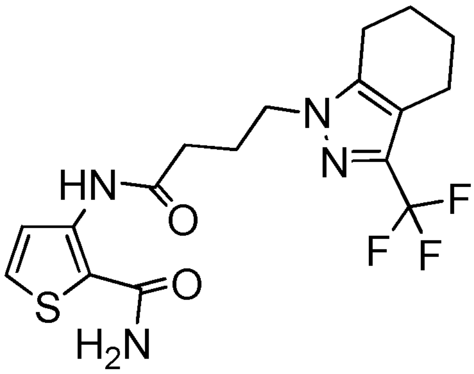

Using 4-[3-(trifluoromethyl)-4,5,6,7-tetrahydro-1H-indazol-1-yl]butanoic acid obtained in Refe rence Example 2, the title compound was obtained in the same manner as in Example 3. Yield: 9.1 mg
MS(ESI+):401(M+H)

Example 158

N-(Thiophen-2-ylmethyl)-4-[3-(trifluoromethyl)-4,5,6,7-tetrahydro-1H-indazol-1-yl]butanamid e

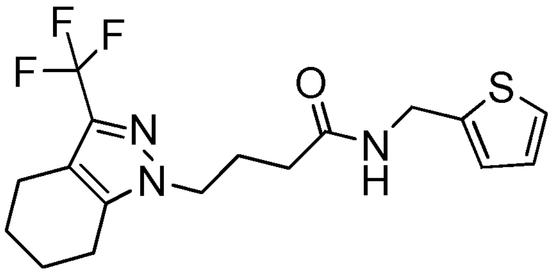

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 12.4 mg
MS(ESI+):372(M+H)

Example 159

N-(4-Sulfamoylbenzyl)-4-[3-(trifluoromethyl)-4,5,6,7-tetrahydro-1H-indazol-1-yl]butanamide

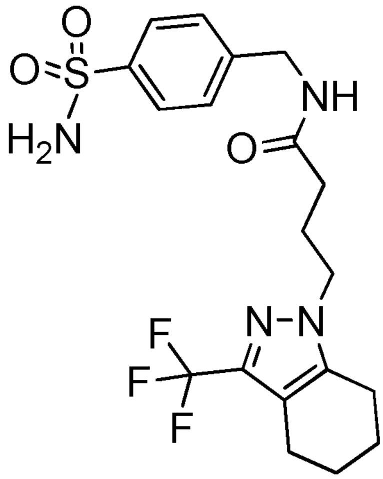

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 4.7 mg
MS(ESI+):445(M+H)

Example 160

N-(4-Fluorobenzyl)-4-[3-(trifluoromethyl)-4,5,6,7-tetrahydro-1H-indazol-1-yl]butanamide

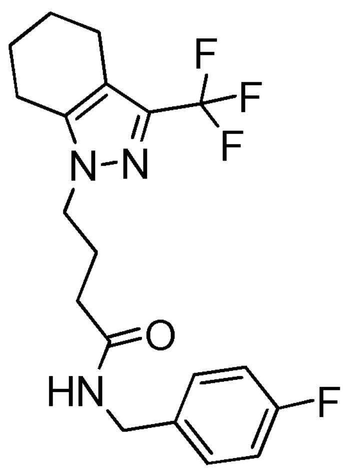

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 12.7 mg
MS(ESI+):384(M+H)

Example 161

N-(1-Thiophen-2-ylethyl)-4-[3-(trifluoromethyl)-4,5,6,7-tetrahydro-1H-indazol-1-yl]butanamid e

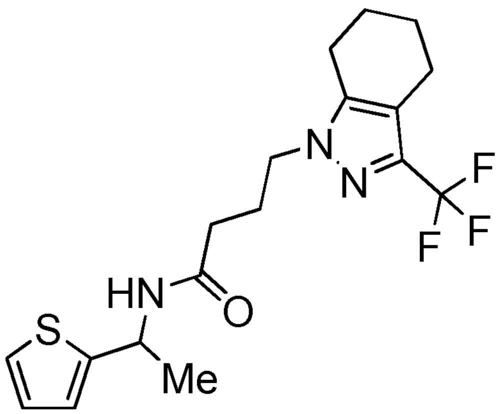

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 7.6 mg
MS(ESI+):386(M+H)

Example 162

1-Ethyl-4-({2-methyl-4-[3-(trifluoromethyl)-4,5,6,7-tetrahydro-1H-indazol-1-yl]butanoyl}amino )-1H-pyrazole-5-carboxamide

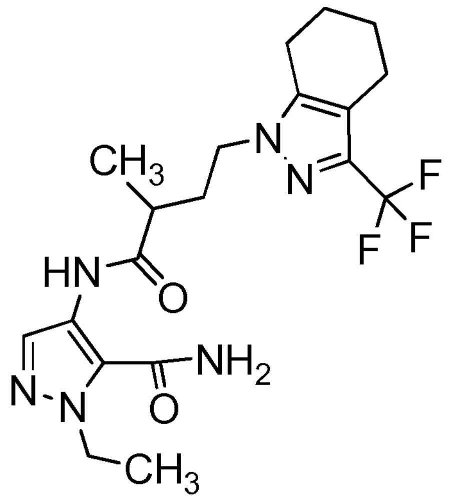

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 13.6 mg
MS(ESI+):427(M+H)

Example 163

4,5-dimethyl-2-({2-methyl-4-[3-(trifluoromethyl)-4,5,6,7-tetrahydro-1H-indazol-1-yl]butanoyl}a mino)thiophene-3-carboxamide

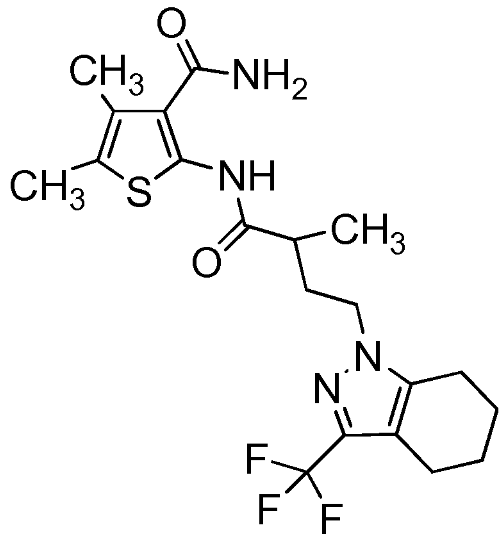

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 2.0 mg
MS(ESI+):443(M+H)

Example 164

2-({2-Methyl-4-[3-(trifluoromethyl)-4,5,6,7-tetrahydro-1H-indazol-1-yl]butanoyl}amino)-4,5,6, 7-tetrahydro-1-benzothiophene-3-carboxamide

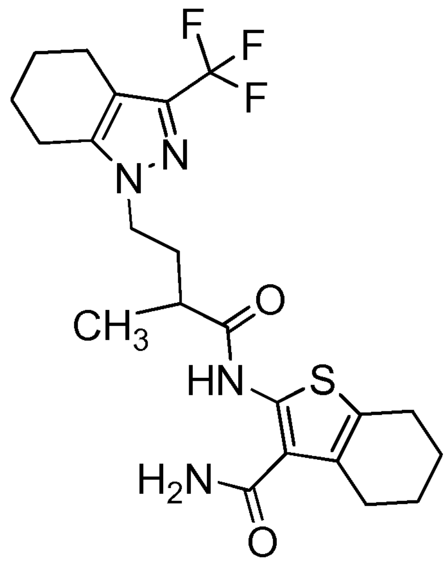

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 4.6 mg
MS(ESI+):469(M+H)

Example 165

5-Chloro-2-({2-methyl-4-[3-(trifluoromethyl)-4,5,6,7-tetrahydro-1H-indazol-1-yl]butanoyl}amin o)benzamide

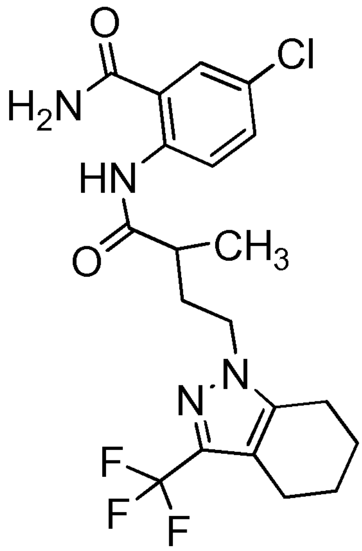

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 2.9 mg
MS(ESI+):443(M+H)

Example 166

N-(5-Chloro-2-methoxyphenyl)-2-methyl-4-[3-(trifluoromethyl)-4,5,6,7-tetrahydro-1H-indazol-1-yl]butanamide

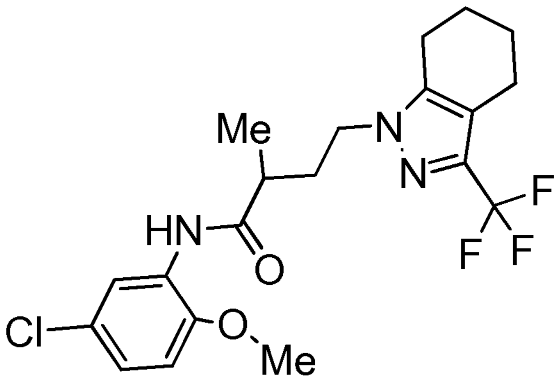

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 9.4 mg
MS(ESI+):430(M+H)

Example 167

3-({2-Methyl-4-[3-(trifluoromethyl)-4,5,6,7-tetrahydro-1H-indazol-1-yl]butanoyl}amino)thiophe ne-2-carboxamide

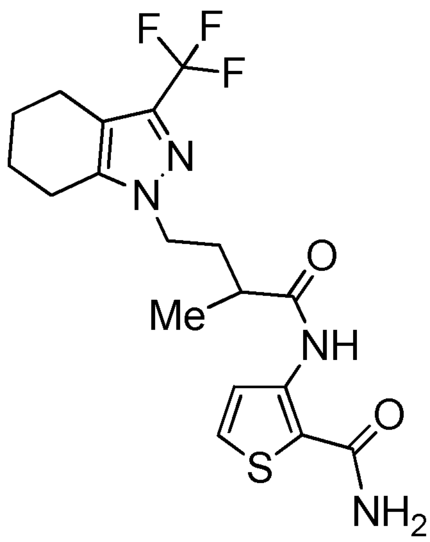

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 1.6 mg
MS(ESI+):415(M+H)

Example 168

N-(Furan-3-ylmethyl)-2-methyl-N-(tetrahydrofuran-2-ylmethyl)-4-[3-(trifluoromethyl)-4,5,6,7-tetrahydro-1H-indazol-1-yl]butanamide

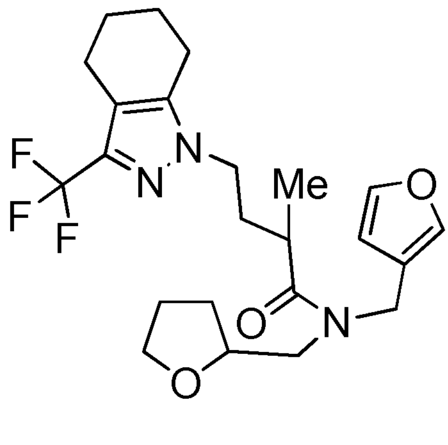

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 15.1 mg
MS(ESI+):454(M+H)

Example 169

2-Methyl-N-(thiophen-2-ylmethyl)-4-[3-(trifluoromethyl)-4,5,6,7-tetrahydro-1H-indazol-1-yl]bu tanamide

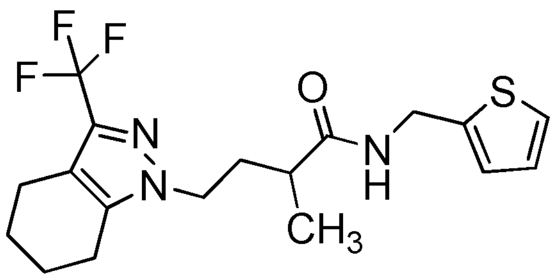

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 12.5 mg
MS(ESI+):386(M+H)

Example 170

N-[2-(Dimethylamino)-1-phenylethyl]-2-methyl-4-[3-(trifluoromethyl)-4,5,6,7-tetrahydro-1H-in dazol-1-yl]butanamide

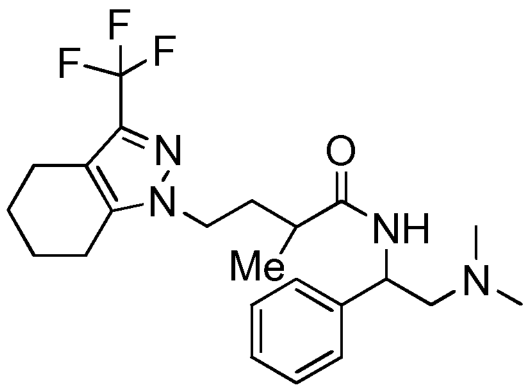

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 6.7 mg
MS(ESI+):437(M+H)

Example 171

2-Methyl-N-(4-sulfamoylbenzyl)-4-[3-(trifluoromethyl)-4,5,6,7-tetrahydro-1H-indazol-1-yl]buta namide

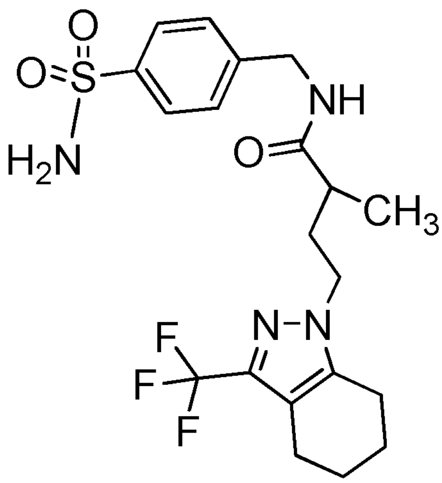

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 16.9 mg
MS(ESI+):459(M+H)

Example 172

N-(4-Fluorobenzyl)-2-methyl-4-[3-(trifluoromethyl)-4,5,6,7-tetrahydro-1H-indazol-1-yl]butana mide

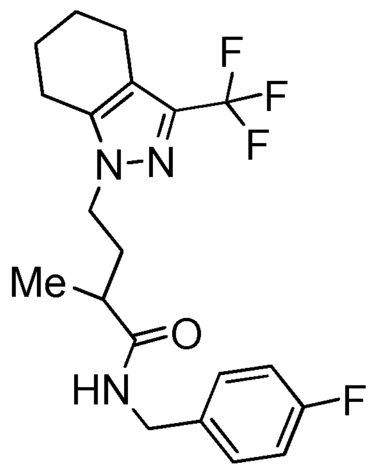

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 11.8 mg
MS(ESI+):398(M+H)

Example 173

2-Methyl-N-(1-thiophen-2-ylethyl)-4-[3-(trifluoromethyl)-4,5,6,7-tetrahydro-1H-indazol-1-yl]bu tanamide

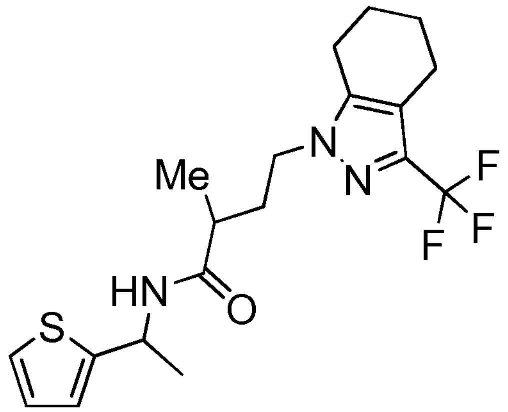

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 7.9 mg
MS(ESI+):400(M+H)

Example 174

1-Ethyl-4-({5-[3-(trifluoromethyl)-4,5,6,7-tetrahydro-1H-indazol-1-yl]pentanoyl}amino)-1H-pyr azole-5-carboxamide

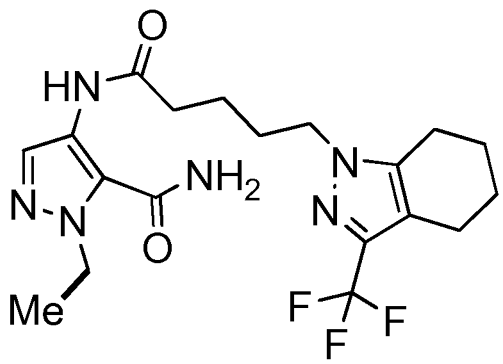

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 9.6 mg
MS(ESI+):427(M+H)

Example 175

5-Chloro-2-({5-[3-(trifluoromethyl)-4,5,6,7-tetrahydro-1H-indazol-1-yl]pentanoyl}amino)benzamide

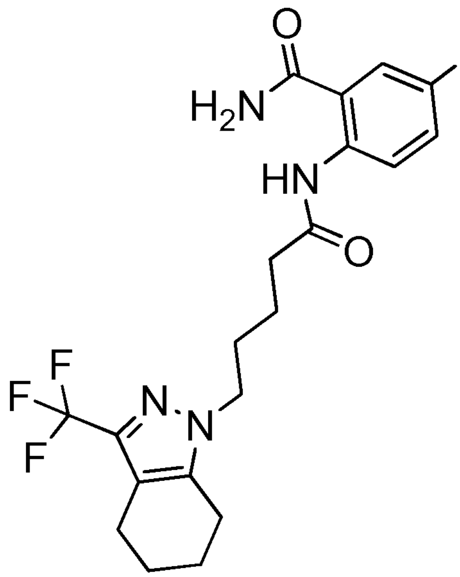

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 3.3 mg
MS(ESI+):443(M+H)

Example 176

N-(5-Chloro-2-methoxyphenyl)-5-[3-(trifluoromethyl)-4,5,6,7-tetrahydro-1H-indazol-1-yl]penta namide

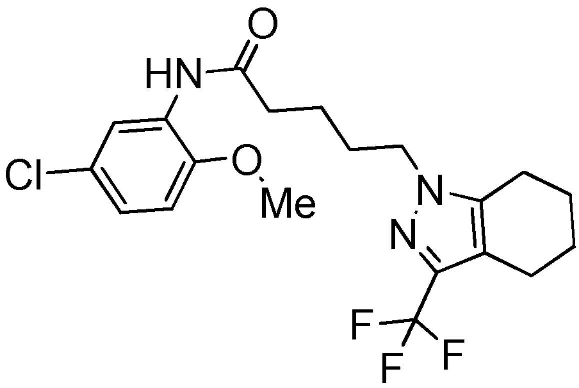

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 8.4 mg
MS(ESI+):430(M+H)

Example 177

3-({5-[3-(Trifluoromethyl)-4,5,6,7-tetrahydro-1H-indazol-1-yl]pentanoyl}amino)thiophene-2-car boxamide

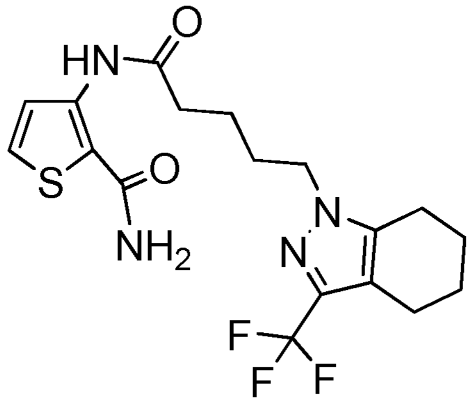

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 5.3 mg
MS(ESI+):415(M+H)

Example 178

N-[3-(1H-Imidazol-1-yl)propyl]-4-[3-(trifluoromethyl)-4,5,6,7-tetrahydro-1H-indazol-1-yl]butan amide

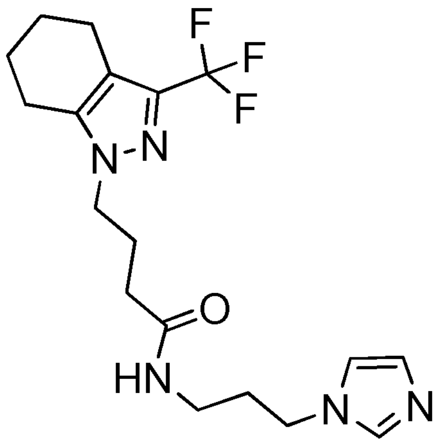

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 5.3 mg
MS(ESI+):384(M+H)

Example 179

N-[3-(1H-Imidazol-1-yl)propyl]-2-methyl-4-[3-(trifluoromethyl)-4,5,6,7-tetrahydro-1H-indazol-1-yl]butanamide

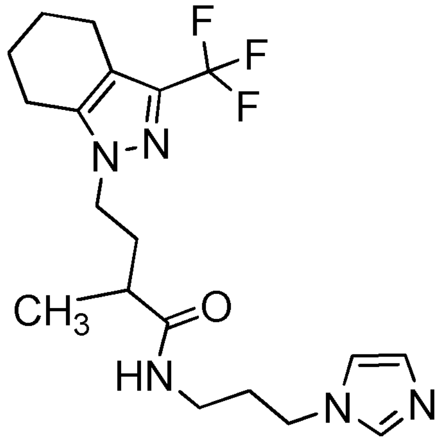

Using 2-methyl-4-[3-(trifluoromethyl)-4,5,6,7-tetrahydro-1H-indazol-1-yl]butanoic acid obtain ed in Reference Example 4, the title compound was obtained in the same manner as in Examp le 3.
Yield: 5.5 mg
MS(ESI+):398(M+H)

Example 180

N-[3-(1H-Imidazol-1-yl)propyl]-5-[3-(trifluoromethyl)-4,5,6,7-tetrahydro-1H-indazol-1-yl]penta namide

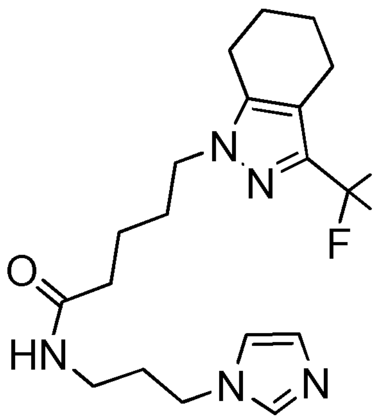

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 0.9 mg
MS(ESI+):398(M+H)

Example 181

4,5-Dimethyl-2-({4-[3-(trifluoromethyl)-4,5,6,7-tetrahydro-1H-indazol-1-yl]butanoyl}amino)thi ophene-3-carboxamide

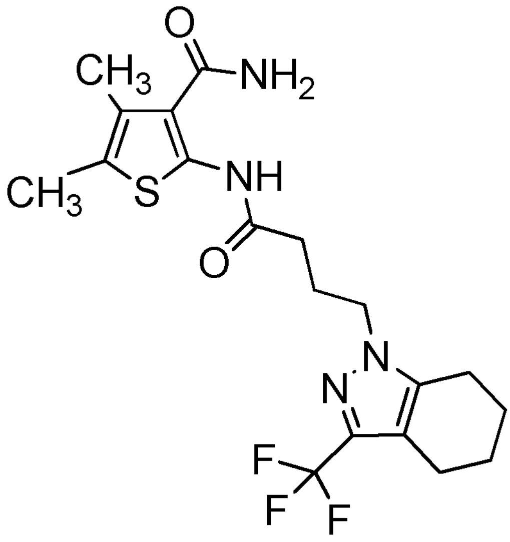

Using 4-[3-(trifluoromethyl)-4,5,6,7-tetrahydro-1H-indazol-1-yl]butanoic acid obtained in Refe rence Example 2, the title compound was obtained in the same manner as in Example 3.
Yield: 2.6 mg
MS(ESI+):429(M+H)

Example 182

4,5-Dimethyl-2-({5-[3-(trifluoromethyl)-4,5,6,7-tetrahydro-1H-indazol-1-yl]pentanoyl}amino)th iophene-3-carboxamide

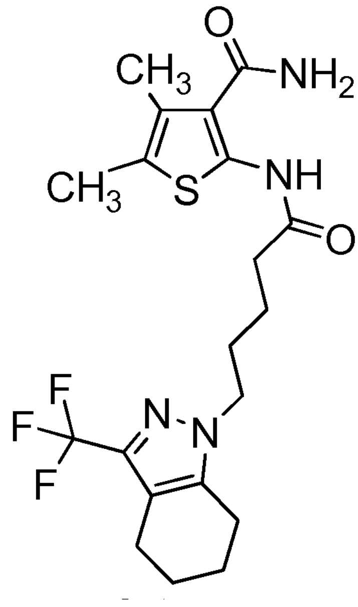

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 1.8 mg
MS(ESI+):443(M+H)

Example 183

N-(Furan-3-ylmethyl)-N-(tetrahydrofuran-2-ylmethyl)-5-[3-(trifluoromethyl)-4,5,6,7-tetrahydr o-1H-indazol-1-yl]pentanamide

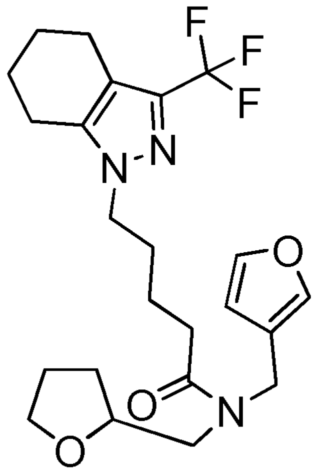

Using 5-[3-(trifluoromethyl)-4,5,6,7-tetrahydro-1H-indazol-1-yl]pentanoic acid obtained in Ref erence Example 6, the title compound was obtained in the same manner as in Example 3.
Yield: 13 mg
MS(ESI+):443(M+H)

Example 184

tert-Butyl 1-{2-[(3-carbamoyl-4,5,6,7-tetrahydro-1-benzothiophen-2-yl)amino]-2-oxoethyl}-3-(tr ifluoromethyl)-1,4,5,7-tetrahydro-6H-pyrazolo[3,4-c]pyridine-6-carboxylate

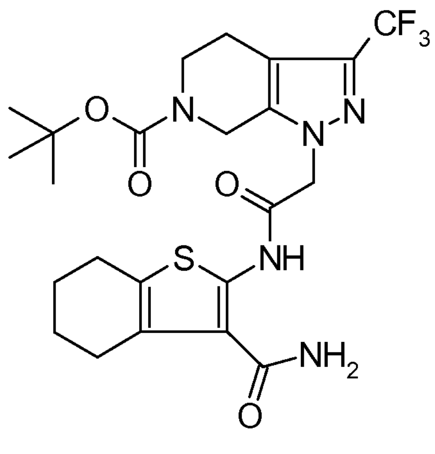

A solution of tert-butyl 7a-hydroxy-3-(trifluoromethyl)-1,3a,4,5,7,7a-hexahydro-6H-pyrazolo[3, 4-c]pyridine-6-carboxylate (500 mg, 1.72 mmol), 2-[(chloroacetyl)amino]-4,5,6,7-tetrahydro-1-b enzothiophene-3-carboxamide (563 mg, 2.06 mmol), and potassium carbonate (948 mg, 6.87 m mol) in DMF (50 mL) was heated to 80 °C for overnight. After cooled to room temperature, wa ter was added thereto, and the mixture was extracted with DCM. The organic layer was wash ed with water, and saturated saline, dried over anhydrous sodium sulfate, and concentrated u nder reduced pressure. The residue was purified by preparative HPLC to give the titled compo und (150 mg, 17% yield) as a yellow solid.

MS Calcd.: 527; MS Found: 528 [M+H].
$^1$H NMR (300 MHz, $CDCl_3$) δ ppm 1.46 (s, 9H), 1.83-1.84 (m, 4H), 2.65-2.71 (m, 6H), 3.67 (s, 2 H), 4.52-4.55 (m, 2H), 5.01 (s, 2H), 5.91 (s, 2H), 12.40 (s, 1H).

Example 185

2-({[3-(Trifluoromethyl)-4,5,6,7-tetrahydro-1H-pyrazolo[3,4-c]pyridin-1-yl]acetyl}amino)-4,5,6, 7-tetrahydro-1-benzothiophene-3-carboxamide

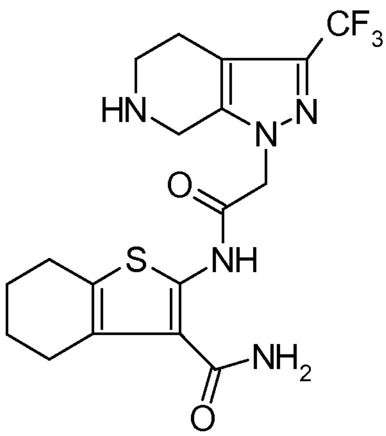

A solution of tert-butyl 1-{2-[(3-carbamoyl-4,5,6,7-tetrahydro-1-benzothiophen-2-yl)amino]-2-o xoethyl}-3-(trifluoromethyl)-1,4,5,7-tetrahydro-6H-pyrazolo[3,4-c]pyridine-6-carboxylate (170 mg, 0.32 mmol) in trifluoroacetic acid(5 mL)/DCM (10 mL) was stirred at room temperature f or 4 hours. The reaction mixture was concentrated under reduced pressure. Sodium hydrogen carbonate aqueous solution was added thereto, and the mixture was extracted with DCM. The organic layer was washed with water, and saturated saline, dried over anhydrous sodium sulf ate, and concentrated under reduced pressure to give the titled compound (50 mg, 36% yield) as a white solid.
MS Calcd.: 427; MS Found: 428 [M+H].
$^1$ H NMR (300 MHz, $CDCl_3$ ) δ ppm 1.74 (s, 1H), 1.83-1.87 (m, 4H), 2.66-2.69 (m, 6H), 3.08 (t, J = 6.0 Hz, 2H), 3.94 (s, 2H), 4.94 (s, 2H), 5.80 (s, 2H), 12.8 (brs, 1H).

Example 186

tert-Butyl 1-[4-(diethylcarbamoyl)benzyl]-3-(trifluoromethyl)-1,4,5,7-tetrahydro-6H-pyrazolo[3 ,4]-cpyridine-6-carboxylate

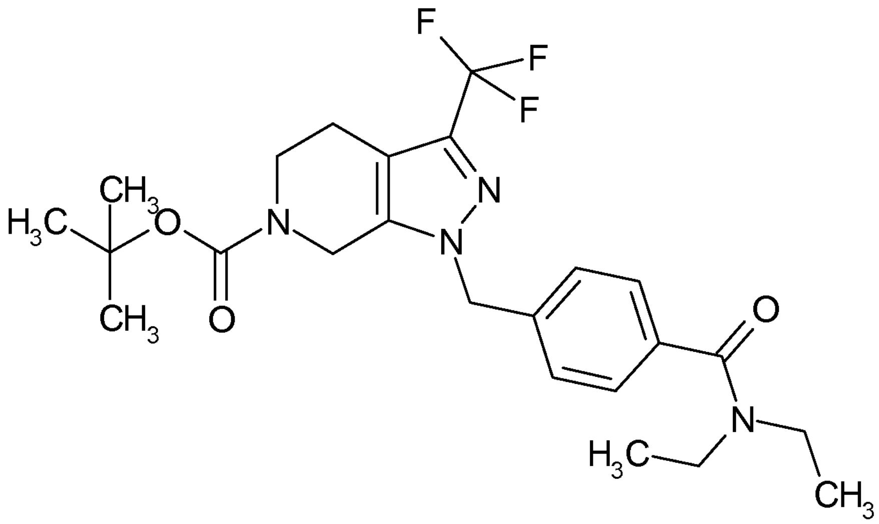

A mixture of tert-butyl 7a-hydroxy-3-(trifluoromethyl)-1,3a,4,5,7,7a-hexahydro-6H-pyrazolo[3, 4-c]pyridine-6-carboxylate (540 mg, 1.85 mmol), 4-(bromomethy)-N,N-diethylbenzamide (752 mg, 2.78 mmol), and potassium carbonate (1.02 g, 7.39 mmol) in DMF (3 mL) was heated to 80 °C overnight. The reaction mixture was cooled to room temperature, water was added thereto, and the mixture was extracted with ethyl acetate. The organic layer was washed with saturat ed saline, dried over sodium sulfate, and concentrated under reduced pressure. The residue w as purified by preparative TLC (PE/EA = 2:3) to give the titled compound (197 mg, 22% yield) as a white solid.

MS Calcd.: 480; MS Found: 481 [M+H].

$^1$ H NMR (300 MHz, $CDCl_3$) δ ppm 1.07-1.28 (m, 6H), 1.45 (s, 9H), 2.64-2.71 (m, 2H), 3.22-3.25 (m, 2H), 3.51-3.58 (m, 4H), 4.34 (s, 2H), 5.26 (s, 2H), 7.19 (d, J = 8.1 Hz, 2H), 7.35 (d, J = 8.1 Hz, 2H).

Example 187

N,N-Diethyl-4-{[3-(trifluoromethyl)-4,5,6,7-tetrahydro-1H-pyrazolo[4,3-c]pyridin-1-yl]methyl}b enzamide

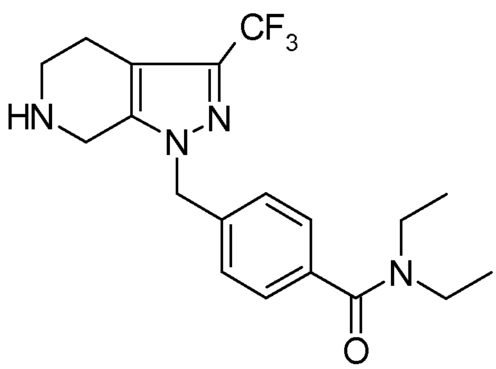

To a mixture of tert-butyl 1-[4-(diethylcarbamoyl)benzyl]-3-(trifluoromethyl)-1,4,5,7-tetrahydr o-6H-pyrazolo[3,4-c]pyridine-6-carboxylate (168 mg, 0.35 mmol) in dichloromethane (2 mL), w as added trifluoroacetic acid (4 mL). The mixture was stirred at room temperature overnight. Dichloromethane, and excess of trifluoroacetic acid was evaporated off under reduced pressure . To the residue, was added a saturated sodium hydrogen carbonate aqueous solution (5 mL), and the mixture was extracted with ethyl acetate. The organic layer was washed with saturat ed saline, dried over anhydrous sodium sulfate, and concentrated under reduced pressure to gi ve the titled compound (97 mg, 73% yield) as a yellow oily substance.

MS Calcd.: 380; MS Found: 381 [M+H].

$^1$ H NMR (300 MHz, $CDCl_3$) δ ppm 1.07-1.12 (m, 3H), 1.20-1.25 (m, 3H), 2.64 (t, J = 5.7 Hz, 2H ), 3.00 (t, J = 5.7 Hz, 2H), 3.22 (brs, 2H), 3.52 (brs, 2H), 4.74 (s, 2H), 5.24 (s, 2H), 7.15 (d, J = 8. 1 Hz, 2H), 7.34 (d, J = 8.1 Hz, 2H)

Example 188

N,N-Diethyl-4-{[5-methyl-3-(trifluoromethyl)-4,5,6,7-tetrahydro-1H-pyrazolo[4,3-c]pyridin-1-yl ]methyl}benzamide

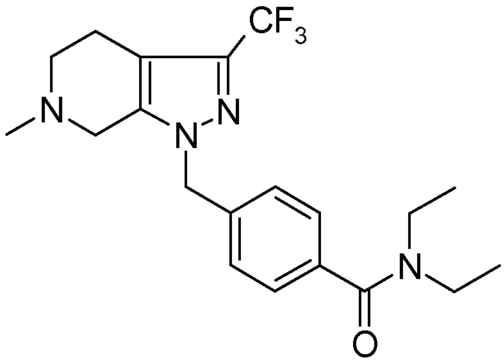

To a solution of N,N-diethyl-4-{[3-(trifluoromethyl)-4,5,6,7-tetrahydro-1H-pyrazolo[4,3-c]pyridi n-1-yl]methyl}benzamide (72 mg, 0.19 mmol) in formic acid (95%, 0.6 mL), was added formald ehyde (40%, 0.06 mL). The mixture was heated to reflux for 2.5 hours, cooled, and then concen trated under reduced pressure. Water was thereto, and the mixture was adjusted to pH 7 with sodium hydrogen carbonate, and extracted with ethyl acetate. The organic layer was washed with saturated saline, dried over anhydrous sodium sulfate, and concentrated under reduced pressure. The residue was purified by preparative TLC (PE/EA = 1:1) to give the titled compo und (35 mg, 47% yield) as a colorless gel.
MS Calcd.: 394; MS Found: 395 [M+H].
$^1$ H NMR (300 MHz, $CDCl_3$) δ ppm 1.09-1.24 (m, 6H), 2.43 (s, 3H), 2.63 (t, J = 5.7 Hz, 2H), 2.7 2 (t, J = 5.7 Hz, 2H), 3.22 (brs, 2H), 3.32 (s, 2H), 3.53 (brs, 2H), 5.24 (s, 2H), 7.12 (d, J = 8.4 Hz , 2H), 7.33 (d, J = 8.4 Hz, 2H).

Example 189

tert-Butyl 1-{2-[(3-carbamoyl-4,5,6,7-tetrahydro-1-benzothiophen-2-yl)amino]-2-oxoethyl}-3-(tr ifluoromethyl)-1,4,6,7-tetrahydro-5H-pyrazolo[4,3-c]pyridine-5-carboxylate

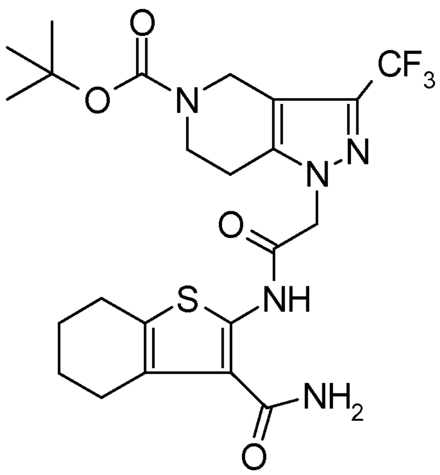

A mixture of tert-butyl 3-(trifluoromethyl)-1,4,6,7-tetrahydro-5H-pyrazolo[4,3-c]pyridine-5-car boxylate (1.00 g, 3.44 mmol), 2-[(chloroacetyl)amino]-4,5,6,7-tetrahydro-1-benzothiophene-3-ca rboxamide (1.03 g, 3.78 mmol), potassium carbonate (1.50 g, 10.32 mmol), and DMF(5 mL) wa s heated to 80 °C overnight. The reaction mixture was cooled to room temperature, was added water thereto, and the mixture was extracted with ethyl acetate. The organic layer was

washe d with saturated saline, dried over anhydrous sodium sulfate, and concentrated under reduce d pressure. The residue was purified by flash column chromatography on silica gel (DCM/met hanol = 200: 1 to 100: 1) to give the titled compound (250 mg, yield 14%) as a yellow solid. MS Calcd.: 527; MS Found: 428 [M-Boc+2H]. $^1$ H NMR (300 MHz, $CDCl_3$) δ ppm 1.48 (s, 9H), 1.83-1.85 (m, 4H),2.66-2.71 (m, 6H), 3.72-3.74 (m, 2H), 4.52-4.54 (m, 2H), 4.99 (s, 2H), 5.76 (s, 2H), 12.37 (s, 1H).

Example 190

2-({[3-(Trifluoromethyl)-4,5,6,7-tetrahydro-1H-pyrazolo[4,3-c]pyridin-1-yl]acetyl}amino)-4,5,6, 7-tetrahydro-1-benzothiophene-3-carboxamide

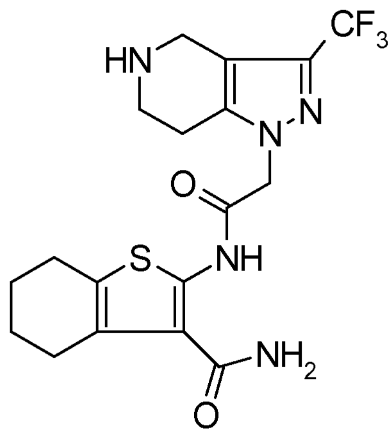

To a mixture of tert-butyl 1-{2-[(3-carbamoyl-4,5,6,7-tetrahydro-1-benzothiophen-2-yl)amino]-2-oxoethyl}-3-(trifluoromethyl)-1,4,6,7-tetrahydro-5H-pyrazolo[4,3-c]pyridine-5-carboxylate (1 96 mg, 0.37 mmol), and dichloromethane (6 mL), was added trifluoroacetic acid (3 mL, 38.8 m mol). The mixture was stirred at room temperature overnight. Dichloromethane, and excess of trifluoroacetic acid was evaporated off under reduced pressure, a saturated sodium hydrogen carbonate aqueous solution (5 mL) was added thereto, and the mixture was extracted with eth yl acetate. The organic layer was washed with saturated saline, dried over anhydrous sodium sulfate, and concentrated under reduced pressure to give the titled compound (120 mg, yield 7 7%) as a white solid.
MS Calcd.: 427; MS Found: 428 [M+H].
$^1$ H NMR (300 MHz, $CDCl_3$) δ ppm 1.83-1.85 (m, 4H), 2.63-2.69 (m, 6H), 3.14-3.18 (m, 2H), 3.9 6 (s, 2H), 4.98 (s, 2H), 5.80 (s, 2H), 12.25 (s, 1H).

Example 191

tert-Butyl 1-[4-(diethylcarbamoyl)benzyl]-3-(trifluoromethyl)-1,4,6,7-tetrahydro-5H-pyrazolo[4 ,3-c]pyridine-5-carboxylate

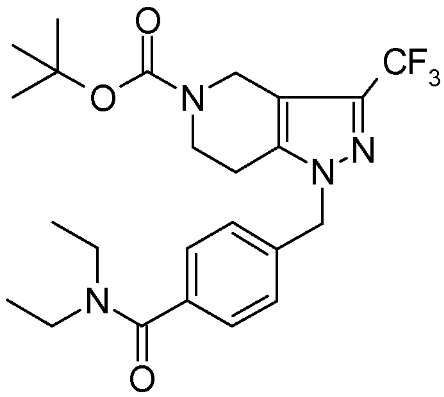

A mixture of tert-butyl 3-(trifluoromethyl)-1,4,6,7-tetrahydro-5H-pyrazolo[4,3-c]pyridine-5-car boxylate (718 mg, 2.47 mmol), 4-(bromomethy)-N,N-diethylbenzamide (1.00 g, 3.70 mmol, 1.5 equiv.), potassium carbonate (1.36 g, 9.88 mmol), and DMF(5 mL) was heated to 80 °C overnig ht. The reaction mixture was cooled to room temperature, water was added thereto, and the m ixture was extracted with ethyl acetate. The organic layer was washed with saturated saline, dried over anhydrous sodium sulfate, and concentrated under reduced pressure. The residue was purified by flash column chromatography on silica gel (PE/EA = 3: 1) to give the titled co mpound (260 mg, yield 22%) as a white solid.
MS Calcd.: 480; MS Found: 425 [M-$Et_2$ N+$NH_3^+$].
$^1$ H NMR (300 MHz, $CDCl_3$) δ ppm 1.07-1.11 (m, 3H), 1.21-1.28 (m, 3H), 1.46 (s, 9H), 2.52-2.54 (m, 2H), 3.21-3.24 (m, 2H), 3.50-3.54 (m, 2 H), 3.64-3.66 (m, 2H), 4.49 (s, 2H), 5.29 (s, 2H), 7.1 4 (d, J = 8.1 Hz, 2H), 7.34 (dd, J = 1.5, 6.6 Hz, 2H).

Example 192

N,N-Diethyl-4-{[3-(trifluoromethyl)-4,5,6,7-tetrahydro-1H-pyrazolo[4,3-c]pyridin-1-yl]methyl}b enzamide

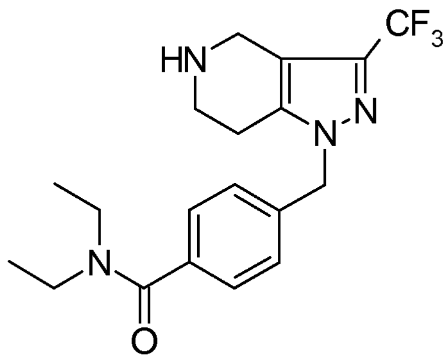

To a mixture of tert-butyl 1-[4-(diethylcarbamoyl)benzyl]-3-(trifluoromethyl)-1,4,6,7-tetrahydr o-5H-pyrazolo[4,3-c]pyridine-5-carboxylate (208 mg, 0.43 mmol), and dichloromethane (6 mL), was added trifluoroacetic acid (3 mL, 38.8 mmol,). The mixture was stirred at room temperatu re overnight. Dichloromethane, and excess of trifluoroacetic acid was evaporated off under red uced pressure, a saturated sodium hydrogen carbonate aqueous solution (5 mL) was added the reto, and the mixture was extracted with ethyl acetate. The organic layer was washed with sa turated saline, dried over anhydrous sodium sulfate, and concentrated under reduced pressur e to give the titled compound (158 mg, yield 97%) as a yellow oily substance.
MS Calcd.: 380; MS Found: 381 [M+H].
$^1$ H NMR (300 MHz, $CDCl_3$) δ ppm 1.10-1.25 (m, 6H), 2.50 (t, J = 5.7 Hz, 2H), 3.05-3.07 (m, 2H ), 3.22-3.24 (m, 2 H), 3.51-3.53 (m, 2H), 3.92 (s, 2H), 5.28 (s, 2H), 7.14 (d, J = 8.1 Hz, 2H), 7.33 (dd, J = 1.5, 6.6 Hz, 2H).

Example 193

N,N-Diethyl-4-{[5-methyl-3-(trifluoromethyl)-4,5,6,7-tetrahydro-1H-pyrazolo[4,3-c]pyridin-1-yl ]methyl}benzamide

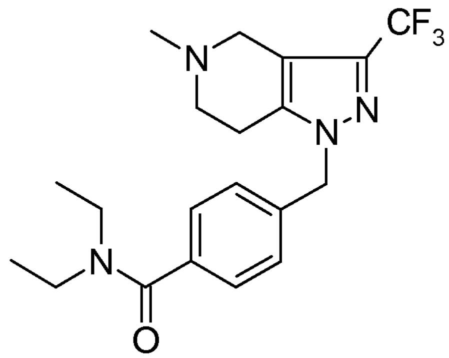

To a solution of N,N-diethyl-4-{[3-(trifluoromethyl)-4,5,6,7-tetrahydro-1H-pyrazolo[4,3-c]pyridi n-1-yl]methyl}benzamide (88 mg, 0.23 mmol) in formic acid (95%, 2 mL), aqueous formaldehyd e solution (40%, 1 mL) was added. The reaction mixture was heated to reflux for 2.5 hours, coo led, concentrated under reduced pressure. Water was added thereto, and the mixture was adj usted to pH 7 with sodium hydrogen carbonate, and extracted with ethyl acetate. The organic layer was washed with saturated saline, dried over anhydrous sodium sulfate, and concentrat ed under reduced pressure. The residue was purified by flash column chromatography on silic a gel (DCM/methanol = 200: 1 to 100: 1) to give the titled compound (50 mg, yield 55%) as a ye llow oily substance.
MS Calcd.: 394; MS Found: 395 [M+H].
$^1$ H NMR (300 MHz, $CDCl_3$) δ ppm 1.09-1.14 (m, 3H), 1.19-1.28 (m, 3H), 2.48 (s, 3H), 2.60 (t, J = 5.4 Hz, 2H), 2.68 (t, J = 5.4 Hz, 2H), 3.21-3.23 (m, 2H), 3.50-3.54 (m, 4H), 5.28 (s, 2H), 7.13 ( d, J = 7.8 Hz, 2H), 7.32 (dd, J = 1.5, 6.3 Hz, 2H).

Example 194

tert-Butyl 1-{2-[(5-chloro-2-methoxyphenyl)amino]-2-oxoethyl}-3-(trifluoromethyl)-1,4,5,7-tetr ahydro-6H-pyrazolo [3,4-c]pyridine-6-carboxylate

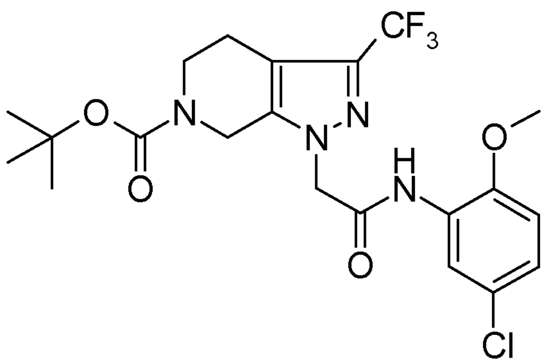

A solution of [6-(tert-butoxycarbonyl)-3-(trifluoromethyl)-4,5,6,7-tetrahydro-1H-pyrazolo[3,4-c] pyridin-1-yl]acetic acid (349 mg, 1.00 mmol), 5-chloro-2-methoxyphenylamine (187 mg, 1.20 m mol), HOBt (203 mg, 1.50 mmol), triethylamine (303 mg, 3.00 mmol), and WSC (288 mg, 1.50 mmol) in dichloromethane (100 mL), at room temperature, under nitrogen atmosphere, was st irred overnight. The reaction mixture was washed with water, and saturated saline, dried ove r sodium sulfate, and concentrated under reduced pressure. The residue was purified by colu mn chromatography on silica gel (PE/EA = 5:1) to give the titled compound (230 mg, yield 47% ) as a white solid.

MS Calcd.: 488; MS Found: 389 (M-Boc+2H).
$^1$ H NMR (300 MHz, DMSO-$d_6$ + $CD_3$ OD) δ ppm 1.48 (s, 9H), 2.69 (t, J = 5.4 Hz, 2H), 3.68 (t, J = 5.4 Hz, 2H), 3.92 (s, 3H), 4.64 (s, 2H), 5.15 (s, 2H), 7.05 (d, J = 8.7 Hz, 1H), 7.13 (dd, J = 8.7, 2.4 Hz, 1H), 8.18 (d, J = 2.1 Hz, 1H)

Example 195

tert-Butyl 1-(2-{[1-(4-chlorophenyl)ethyl]amino}-2-oxoethyl)-3-(trifluoromethyl)-1,4,5,7-tetrahy dro-6H-pyrazolo[3,4-c] pyridine-6-carboxylate

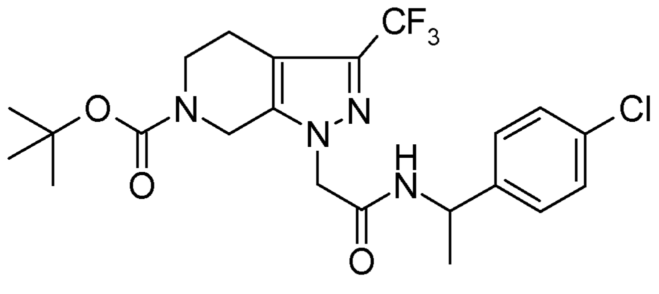

A solution of [6-(tert-butoxycarbonyl)-3-(trifluoromethyl)-4,5,6,7-tetrahydro-1H-pyrazolo[3,4-c] pyridin-1-yl]acetic acid (349 mg, 1.00 mmol), 1-(4-chlorophenyl)ethylamine (186 mg, 1.20 mmo 1), HOBt (203 mg, 1.50 mmol), triethylamine (303 mg, 3.00 mmol), and WSC (288 mg, 1.50 m mol) in dichloromethane (100 mL), at room temperature, under nitrogen atmosphere, was stir red overnight. The reaction mixture was washed with water, and saturated saline, dried over sodium sulfate, and concentrated under reduced pressure. The residue was purified by column chromatography on silica gel (PE/EA = 5:1) to give the titled compound (210 mg, yield 43%) a s a white solid.
MS Calcd.: 486; MS Found: 387 (M -Boc+2H).
$^1$ H NMR (300 MHz, $CDCl_3$) δ ppm 1.44 (d, J = 6.9 Hz, 3H), 1.47 (s, 9H), 2.66-2.70 (m, 2H), 3.5 8-3.67 (m, 2H), 4.46-4.52 (m, 2H), 4.65 (d, J = 3.6 Hz, 2H), 5.00-5.05 (m, 1H), 6.53 (br, 1H), 7.1 4-7.18 (m, 2H), 7.27-7.30 (m, 2H).

Example 196

N-(5-Chloro-2-methoxyphenyl)-2-[3-(trifluoromethyl)-4,5,6,7-tetrahydro-1H-pyrazolo[3,4-c]pyri din-1-yl]acetamide

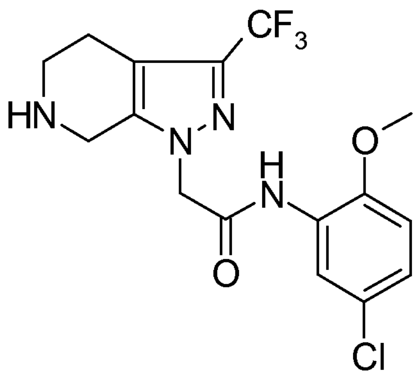

To a solution of tert-butyl1-{2-[(5-chloro-2-methoxyphenyl)amino]-2-oxoethyl}-3-(trifluoromet hyl)-1,4,5,7-tetrahydro-6H-pyrazolo[3,4-c]pyridine-6-carboxylate (150 mg, 0.31 mmol) in DCM (20 mL) stirred at room temperature, was added trifluoroacetic acid (2 mL). The mixture was stirred for 4 hours, and the solvent was evaporated off under reduced pressure. To the residue , were added DCM (40 mL), and potassium carbonate (1 g), the mixture was stirred for 30 min utes, and filtered. The filtrate was concentrated under reduced pressure. The residue was puri fied by preparative HPLC to give the titled compound (60 mg, yield 50%) as a white solid. MS Calcd.: 388; MS Found: 389 (M+H).

$^1$ H NMR (300 MHz, $CDCl_3$) δ ppm 2.67 (t, J = 6.0 Hz, 2H), 3.05 (t, J = 6.0 Hz, 2H), 3.81 (s, 3H ), 3.95 (s, 2H), 4.79 (s, 2H), 6.75 (d, J = 8.7 Hz, 1H), 7.01 (dd, J = 8.7, 2.4 Hz, 1H), 8.37 (d, J = 2 .7 Hz, 1H), 8.83 (br s, 1H).

Example 197

N-[1-(4-Chlorophenyl)ethyl]-2-[3-(trifluoromethyl)-4,5,6,7-tetrahydro-1H-pyrazolo[3,4-c]pyridi n-1-yl]acetamide

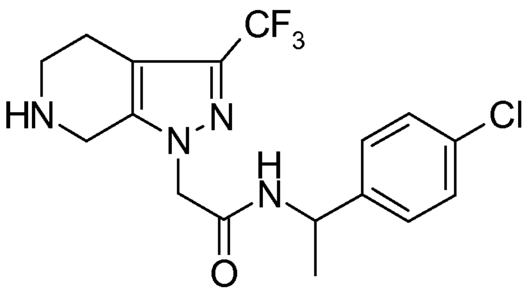

To a solution of tert-butyl 1-(2-{[1-(4-chlorophenyl)ethyl]amino}-2-oxoethyl)-3-(trifluoromethyl )-1,4,5,7-tetrahydro-6H-pyrazolo[3,4-c]pyridine-6-carboxylate (480 mg, 0.99 mmol,) in DCM (4 0 mL) stirred at room temperature, was added trifluoroacetic acid (10 mL). The mixture was s tirred for 4 hours, and the solvent was evaporated off under reduced pressure. To the residue, were added DCM (100 mL), and potassium carbonate (3 g), the mixture was stirred for 30 min utes, filtered. Thee filtrate was concentrated under reduced pressure to give the titled compou nd (350 mg, yield 92%) as a yellow solid.
MS Calcd.: 386; MS Found: 387 (M+H).
$^1$ H NMR (300 MHz, $CDCl_3$) δ ppm 1.42 (d, J = 6.9 Hz, 3H), 2.64 (t, J = 5.7 Hz, 2H), 3.01-3.05 ( m, 2H), 3.87-3.89 (m, 2H), 4.55-4.69 (m, 2H), 4.98-5.03 (m, 1H), 6.64 (d, J = 4.2 Hz, 1H), 7.12-7 .16 (m, 2H), 7.26-7.29 (m, 2H).

Example 198

N-(5-Chloro-2-methoxyphenyl)-2-[6-methyl-3-(trifluoromethyl)-4,5,6,7-tetrahydro-1H-pyrazolo [3,4-c]pyridin-1-yl]acetamide

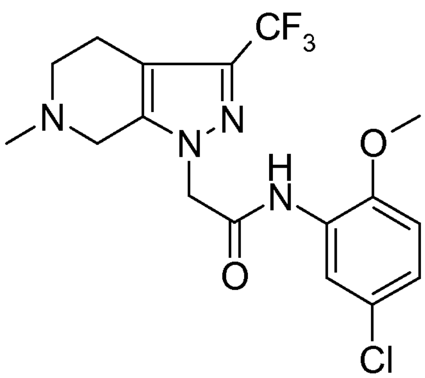

A solution of compound N-(5-chloro-2-methoxyphenyl)-2-[3-(trifluoromethyl)-4,5,6,7-tetrahydr o-1H-pyrazolo[3,4-c]pyridin-1-yl]acetamide (150 mg, 0.39 mmol), and 37 % aqueous formaldeh yde solution (0.1 mL) in formic acid (6 mL) was heated to reflux for 3 hours. The mixture was adjusted to PH 7 with a saturated aqueous solution of sodium carbonate, and extracted with DCM. The organic layer was washed with saturated saline, dried over sodium sulfate, and con centrated under reduced pressure. The obtained residue was purified by column chromatogra phy on silica gel (PE/EA = 3:1) to give the titled compound (59 mg, yield 38%) as a white solid.
MS Calcd.: 402; MS Found: 403 (M+H).

$^1$ H NMR (300 MHz, $CDCl_3$) δ ppm 2.51 (s, 3H), 2.66-2.76 (m, 4H), 3.52 (s, 2H), 3.79 (s, 3H), 4. 79 (s, 2H), 6.74 (d, J = 8.7 Hz, 1H), 7.01 (dd, J = 8.7, 2.4 Hz, 1H), 8.36 (d, J = 2.4 Hz, 1H), 8.77 (br s, 1H).

Example 199

N-[1-(4-Chlorophenyl)ethyl]-2-[3-(trifluoromethyl)-4,5,6,7-tetrahydro-1H-pyrazolo[3,4-c]pyridi n-1-yl]acetamide

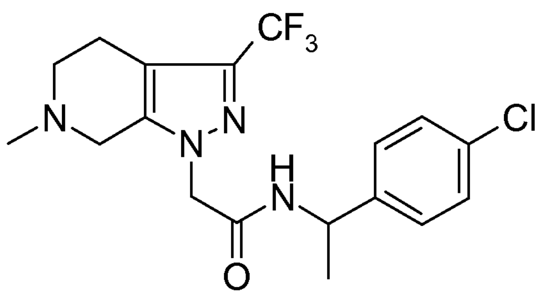

A solution of compound N-[1-(4-chlorophenyl)ethyl]-2-[3-(trifluoromethyl)-4,5,6,7-tetrahydro-1 H-pyrazolo[3,4-c]pyridin-1-yl]acetamide (260 mg, 0.67 mmol), and 37 % aqueous formaldehyde solution (0.1 mL) in formic acid (10 mL) was heated to reflux for 3 hours. The mixture was adj usted to PH 7 with a saturated aqueous solution of sodium carbonate, and extracted with DC M. The organic layer was washed with saturated saline, dried over sodium sulfate, and concen trated under reduced pressure. The obtained residue was purified by column chromatography on silica gel (PE/EA = 3: 1) to give the titled compound (120 mg, yield 45%) as a white solid.
MS Calcd.: 400; MS Found: 401 (M+H).
$^1$ H NMR (300 MHz, $CDCl_3$) δ ppm 1.42 (d, J = 7.2 Hz, 3H), 2.50 (s, 3H), 2.67-2.76 (m, 4H), 3.4 1-3.57 (m, 2H), 4.60 (d, J = 16.5 Hz, 1H), 4.67 (d, J = 16.5 Hz, 1H), 4.98-5.03 (m, 1H), 6.57 (d, J = 8.4 Hz, 1H), 7.12-7.16 (m, 2H), 7.26-7.29 (m, 2H).

Example 200

N-(5-Chloro-2-methoxyphenyl)-2-[6-(1-methylethyl)-3-(trifluoromethyl)-4,5,6,7-tetrahydro-1H-pyrazolo[3,4-c]pyridin-1-yl]acetamide

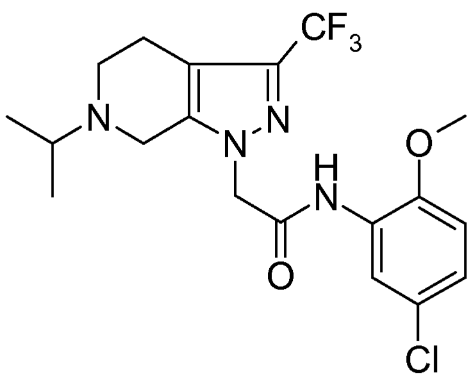

To a solution of N-(5-chloro-2-methoxyphenyl)-2-[3-(trifluoromethyl)-4,5,6,7-tetrahydro-1H-py razolo[3,4-c]pyridin-1-yl] acetamide (200 mg, 0.52 mmol), and acetone (300 mg, 5.20 mmol) in methanol (20 mL) stirred at room temperature, was added sodium cyanoborohydride (164 mg, 2.60 mmol). The mixture was stirred at room temperature overnight, and concentrated under reduced pressure. To the residue, was added ice water (20 mL) carefully, and the mixture was extracted with dichloromethane. The organic layer was washed with saturated saline, dried o ver anhydrous sodium sulfate, and concentrated under reduced pressure. The obtained solid w ashed with ether to give the titled compound (158 mg, yield 71%) as a white solid.

MS Calcd.: 430; MS Found: 431 (M+H).

$^1$ H NMR (400 MHz, $CDCl_3$) δ ppm 1.13 (d, J = 6.4 Hz, 6H), 2.74 (t, J = 5.6 Hz ,2H), 2.84 (t, J = 5.6 Hz ,2H), 2.99-3.55 (m, 1H), 3.65 (s, 2H), 3.80 (s, 3H), 4.82 (s, 2H), 6.75 (d, J = 9.2 Hz, 1H), 7.01 (dd, J = 8.8, 2.4 Hz ,1H), 8.38 (d, J = 2.8 Hz ,1H), 8.88 (br s, 1H).

Example 201

N-(5-Chloro-2-methoxyphenyl)-2-[6-(cyclopropylmethyl)-3-(trifluoromethyl)-4,5,6,7-tetrahydro-1H-pyrazolo[3,4-c]pyridin-1-yl]acetamide

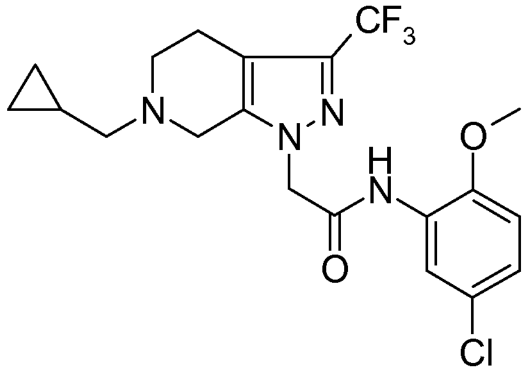

A solution of N-(5-chloro-2-methoxyphenyl)-2-[3-(trifluoromethyl)-4,5,6,7-tetrahydro-1H-pyraz olo[3,4-c]pyridin-1-yl] acetamide (100 mg, 0.26 mmol), (bromomethy)cyclopropane (104 mg, 0.7 7 mmol), and potassium carbonate (178 mg, 1.30 mmol) in acetone (150 mL) was heated to refl ux overnight. The reaction mixture was concentrated under reduced pressure, and purified wi th column chromatography on silica gel (PE/EA = 3:1) to give the titled compound (67 mg, yiel d 58%) as a white solid.

MS Calcd.: 442; MS Found: 443 (M+H).

$^1$ H NMR (400 MHz, DMSO-$d_6$) δ ppm 0.13-0.16 (m, 2H), 0.48-0.51 (m, 2H), 0.88-0.95 (m, 1H), 2.42 (d, J = 6.4 Hz, 2H), 2.55-2.61 (m, 2H), 2.74-2.76 (m, 2H), 3.64 (s, 2H), 3.88 (s, 3H), 5.18 (s, 2H), 7.10-7.18 (m, 2H), 8.08 (d, J = 2.8 Hz, 1H), 9.83 (s, 1H).

Example 202

Ethyl [1-{2-[(5-chloro-2-methoxyphenyl)amino]-2-oxoethyl}-3-(trifluoromethyl)-1,4,5,7-tetrahy dro-6H-pyrazolo[3,4-c]pyridin-6-yl]acetate

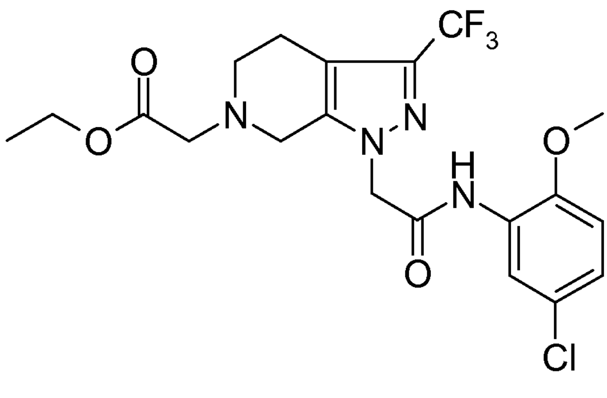

A solution of N-(5-chloro-2-methoxyphenyl)-2-[3-(trifluoromethyl)-4,5,6,7-tetrahydro-1H-pyraz olo[3,4-c]pyridin-1-yl]acetamide (1100 mg, 2.83 mmol), ethyl bromoacetate (568 mg, 3.40 mmol ), and potassium carbonate (1956 mg, 14.17 mmol) in acetone (150 mL) was heated to reflux o vernight. After the solvent was evaporated off, water was added thereto, and the mixture was extracted with DCM. The organic layer was washed with saturated saline, dried over anhydro us sodium sulfate, and concentrated under reduced pressure. The residue was washed with di ethyl ether, and filtered to give the titled compound (1000 mg, yield 75%) as a white solid.
MS Calcd.: 474; MS Found: 475 (M+H).
$^1$ H NMR (400 MHz, $CDCl_3$) δ ppm 1.31 (t, J = 7.2 Hz, 3H), 2.80 (t, J = 5.6 Hz, 2H), 2.95 (t, J = 6.0 Hz, 2H), 3.51 (s, 2H), 3.83-3.84 (m, 5H), 4.23 (q, J = 7.2 Hz, 2H), 4.81 (s, 2H), 6.78 (d, J = 8. 8 Hz, 1H), 7.04 (dd, J = 8.8, 2.4 Hz, 1H), 8.39 (d, J = 2.4 Hz, 1H), 8.81 (s, 1H).

Example 203

[1-{2-[(5-Chloro-2-methoxyphenyl)amino]-2-oxoethyl}-3-(trifluoromethyl)-1,4,5,7-tetrahydro-6 H-pyrazolo[3,4-c]pyridin-6-yl]acetic acid

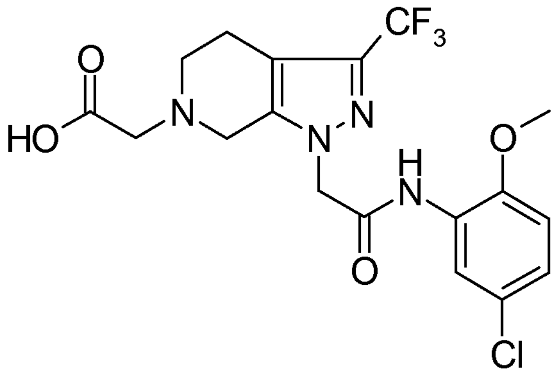

To a solution of ethyl [1-{2-[(5-chloro-2-methoxyphenyl)amino]-2-oxoethyl}-3-(trifluoromethyl)-1,4,5,7-tetrahydro-6H-pyrazolo[3,4-c]pyridin-6-yl]acetate (100 mg, 0.21 mmol) in THF (2 mL), and methanol (2 mL), was added a solution of lithium hydroxide (20 mg) in water (1 mL). The mixture was stirred at room temperature overnight. The solvent was removed under reduced pressure. The residue was adjusted to pH 7 with 4N hydrochloric acid. White solids were preci pitated, filtered, washed with cooled water, and dried under reduced pressure to give the title d compound (25 mg, yield 27%) as a white solid.
MS Calcd.: 446; MS Found: 447 (M+H).
$^1$H NMR (300 MHz, $CD_3$ OD) δ ppm 2.86-2.89 (m, 2H), 3.15-3.18 (m, 2H), 3.51 (s, 2H), 3.92 (s, 3H), 4.10 (s, 2H), 5.12 (s, 2H), 7.02 (d, J = 6.6 Hz, 1H), 7.11 (d, J = 6.3 Hz, 1H), 8.17 (s, 1H)

Example 204

2-[6-(2-Amino-2-oxoethyl)-3-(trifluoromethyl)-4,5,6,7-tetrahydro-1H-pyrazolo[3,4-c]pyridin-1-y 1]-N-(5-chloro-2-methoxyphenyl)acetamide

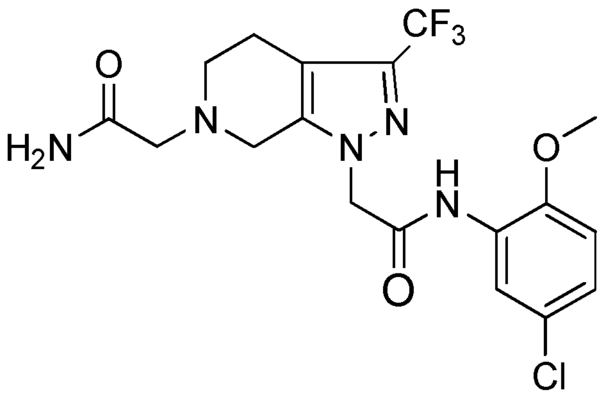

A solution of ethyl [1-{2-[(5-chloro-2-methoxyphenyl)amino]-2-oxoethyl}-3-(trifluoromethyl)-1,4 ,5,7-tetrahydro-6H-pyrazolo[3,4-c]pyridin-6-yl]acetate (100 mg, 0.21 mmol) in 2.0 M ammonia/ methanol (20 mL) was stirred at room temperature overnight. The reaction mixture was conce ntrated under reduced pressure. The obtained residue was purified by column chromatograph y on silica gel (DCM/methanol = 10:1) to give the titled compound (42 mg, yield 45%) as a whit e solid.
MS Calcd.: 445; MS Found: 446 (M+H).
$^1$ H NMR (300 MHz, $CDCl_3$) δ ppm 2.75-2.87 (m, 4H), 3.25 (s, 2H), 3.73 (s, 2H), 3.82 (s, 3H), 4. 80 (s, 2H), 5.61 (br, 1H), 6.76 (d, J = 8.7 Hz, 1H), 6.88 (br, 1H), 7.02 (dd, J = 8.7, 3.0 Hz, 1H), 8. 34 (d, J = 3.0 Hz, 1H), 8.76 (s, 1H)

Example 205

2-[1-{2-[(5-Chloro-2-methoxyphenyl)amino]-2-oxoethyl}-3-(trifluoromethyl)-1,4,5,7-tetrahydro-6H-pyrazolo[3,4-c]pyridin-6-yl]-N-methylacetamide

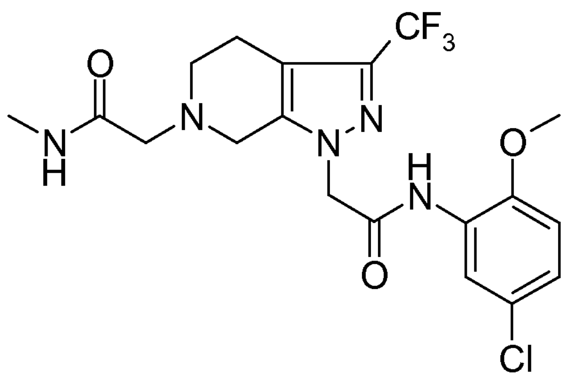

A mixture of ethyl [1-{2-[(5-chloro-2-methoxyphenyl)amino]-2-oxoethyl}-3-(trifluoromethyl)-1,4 ,5,7-tetrahydro-6H-pyrazolo[3,4-c]pyridin-6-yl]acetate (100 mg, 0.21 mmol), and 40 % methyla mine aqueous solution (20 mL) was stirred at room temperature overnight. The reaction mixt ure was concentrated under reduced pressure. The obtained residue was purified by column c hromatography on silica gel (DCM/methanol = 10:1) to give the titled compound (58 mg, yield 59%) as a white solid.
MS Calcd.: 459; MS Found: 460 (M+H).
$^1$ H NMR (300 MHz, $CDCl_3$) δ ppm 2.76-2.85 (m, 7H), 3.25 (s, 2H), 3.69 (s, 2H), 3.83 (s, 3H), 4. 79 (s, 2H), 6.76 (d, J = 8.7 Hz, 1H), 6.98 (br, 1H), 7.02 (dd, J = 8.7, 2.7 Hz, 1H), 8.35 (d, J = 2.7 Hz, 1H), 8.75 (br s, 1H)

Example 206

2-[1-{2-[(5-Chloro-2-methoxyphenyl)amino]-2-oxoethyl}-3-(trifluoromethyl)-1,4,5,7-tetrahydro-6H-pyrazolo[3,4-c]pyridin-6-yl]-N,N-dimethylacetamide

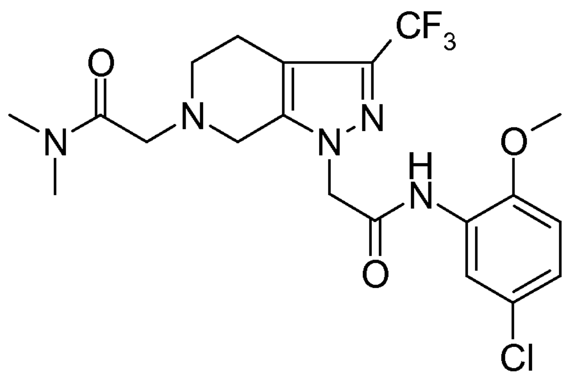

A mixture of ethyl [1-{2-[(5-chloro-2-methoxyphenyl)amino]-2-oxoethyl}-3-(trifluoromethyl)-1,4 ,5,7-tetrahydro-6H-pyrazolo[3,4-c]pyridin-6-yl]acetate (200 mg, 0.42 mmol), and 2.0 M dimeth ylamine/ethanol solution (20 mL), in a sealed tube, was stirred at 70 °C overnight. The solvent was evaporated off under reduced pressure, and the residue was purified by preparative HPL C to give the titled compound (65 mg, yield 33%) as a white solid.
MS Calcd.: 473; MS Found: 474 (M+H).
$^1$ H NMR (400 MHz, $CDCl_3$) δ ppm 2.78 (t, J = 5.6 Hz, 2H), 2.96-2.98 (m, 5H), 3.06 (s, 3H), 3.4 6 (s, 2H), 3.74 (s, 2H), 3.80 (s, 3H), 4.84 (s, 2H), 6.76 (d, J = 8.8 Hz, 1H), 7.02 (dd, J = 8.8, 2.8 Hz, 1H), 8.38 (d, J = 2.8 Hz, 1H), 8.84 (s, 1H)

Example 207

N-(5-Chloro-2-methoxyphenyl)-2-[6-(2-hydroxyethyl)-3-(trifluoromethyl)-4,5,6,7-tetrahydro-1 H-pyrazolo[3,4-c]pyridin-1-yl]acetamide

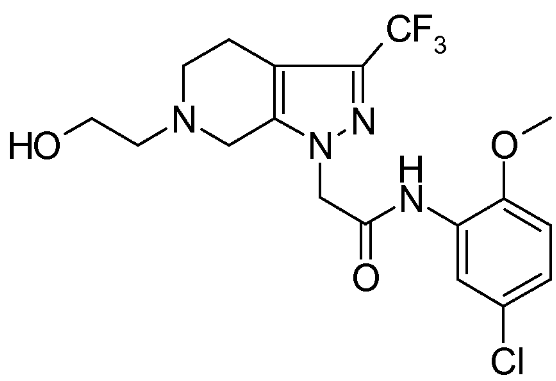

To a solution of ethyl [1-{2-[(5-chloro-2-methoxyphenyl)amino]-2-oxoethyl}-3-(trifluoromethyl)-1,4,5,7-tetrahydro-6H-pyrazolo[3,4-c]pyridin-6-yl]acetate (100 mg, 0.21 mmol) in THF (7 mL) s tirred at ice-bath temperature, was added lithium aluminum hydride (16 mg, 0.42 mmol). Th e mixture was stirred for 3 hours. To the reaction mixture, was added sodium sulfate decahyd rate (500 mg). The mixture was stirred overnight, and concentrated under reduced pressure. The obtained residue was purified by column chromatography on silica gel (DCM/methanol = 10:1) to give the titled compound (45 mg, yield 50%) as a white solid.
MS Calcd.: 432; MS Found: 433 (M+H).
$^1$ H NMR (400 MHz, $CDCl_3$) δ ppm 2.76-2.88 (m, 6H), 3.70 (s, 2H), 3.73-3.76 (m, 2H), 3.84 (s, 3 H), 4.83 (s, 2H), 6.79 (d, J = 8.8 Hz, 1H), 7.05 (d, J = 8.8, 2.4 Hz, 1H), 8.40 (d, J = 2.4 Hz, 1H), 8.84 (s, 1H)).

Example 208

N-(5-Chloro-2-methoxyphenyl)-2-[6-(methylsulfonyl)-3-(trifluoromethyl)-4,5,6,7-tetrahydro-1 H-pyrazolo[3,4-c]pyridin-1-yl]acetamide

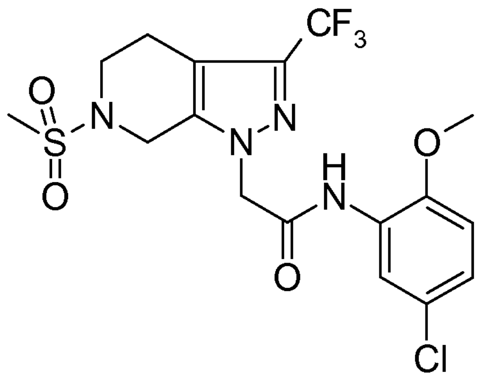

To a solution of N-(5-chloro-2-methoxyphenyl)-2-[3-(trifluoromethyl)-4,5,6,7-tetrahydro-1H-py razolo[3,4-c]pyridin-1-yl] acetamide (100 mg, 0.26 mmol) in pyridine (5 mL) stirred at ice-bath temperature, was added methanesulfonyl chloride (51 mg, 0.52 mmol,), the mixture was stirre d at room temperature for 2 hours. The reaction mixture was concentrated under reduced pres sure, and the residue was purified by column chromatography on silica gel (PE/EA = 2:1) to gi ve the titled compound (48 mg, yield 40%) as a yellow solid.
MS Calcd.: 466, MS Found: 467 (M+H).
$^1$ H NMR (400 MHz, $CDCl_3$) δ ppm 2.87 (t, J = 5.6 Hz, 2H), 2.93 (s, 3H), 3.59 (t, J = 5.6 Hz, 2H ), 3.84 (s, 3H), 4.52 (s, 2H), 4.90 (s, 2H), 6.78 (d, J = 8.8 Hz, 1H), 7.03 (dd, J = 8.8, 2.8 Hz, 1H), 8.32 (d, J = 2.8 Hz, 1H), 8.63 (s, 1H).

Example 209

1-{2-[(5-Chloro-2-methoxyphenyl)amino]-2-oxoethyl}-3-(trifluoromethyl)-1,4,5,7-tetrahydro-6 H-pyrazolo[3,4-c]pyridine-6-carboxamide

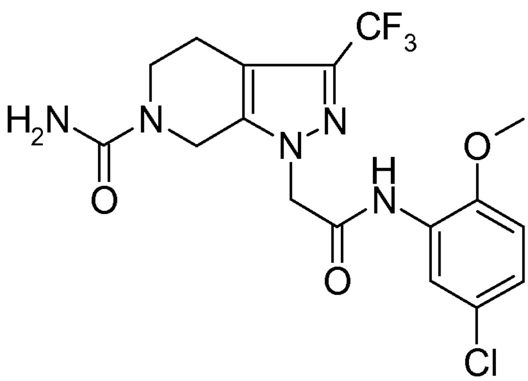

A solution of N-(5-chloro-2-methoxyphenyl)-2-[3-(trifluoromethyl)-4,5,6,7-tetrahydro-1H-pyraz olo[3,4-c]pyridin-1-yl] acetamide (100 mg, 0.26 mmol), and bis(trichloromethyl) carbonate (76 mg, 0.26 mmol) in ethyl acetate (50 mL) was heated to reflux for 3 hours. The solvent was eva porated off under reduced pressure, DCM (30 mL), and following 2.0 M ammonia/methanol sol ution (1 mL) were added thereto. The mixture was stirred for 1 hour at room temperature. Th e reaction mixture was concentrated under reduced pressure. The obtained residue was purifi ed by column chromatography on silica gel (DCM/methanol = 10:1) to give the titled compoun d (50 mg, yield 45%) as a white solid.
MS Calcd.: 431; MS Found: 432 (M+H).

$^1$ H NMR (400 MHz, DMSO-$d_6$) δ ppm 2.58-2.61 (m, 2H), 3.56 (t, J = 5.6 Hz, 2H), 3.89 (s, 3H), 4.51 (s, 2H), 5.20 (s, 2H), 6.19 (s, 2H), 7.10-7.16 (m, 2H), 8.09 (d, J = 2.4 Hz, 1H), 9.86 (s, 1H).

Example 210

1-{2-[(5-Chloro-2-methoxyphenyl)amino]-2-oxoethyl}-N-methyl-3-(trifluoromethyl)-1,4,5,7-tetr ahydro-6H-pyrazolo [3,4-c]pyridine-6-carboxamide

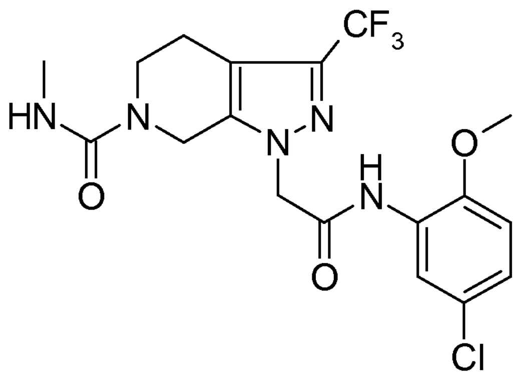

A solution of N-(5-chloro-2-methoxyphenyl)-2-[3-(trifluoromethyl)-4,5,6,7-tetrahydro-1H-pyraz olo[3,4-c]pyridin-1-yl] acetamide (100 mg, 0.26 mmol), and bis(trichloromethyl) carbonate (76 mg, 0.26 mmol)in ethyl acetate (50 mL) was heated to reflux for 3 hours. After the solvent was evaporated off under reduced pressure, DCM (30 mL) and following 40 % methylamine aqueo us solution (1 mL) were added thereto. The mixture was stirred for 1 hour at room temperatur e. The reaction mixture was concentrated under reduced pressure. The obtained residue was p urified by column chromatography on silica gel (DCM/methanol = 10:1) to give the titled comp ound (70 mg, yield 61%) as a white solid. MS Calcd.: 445, MS Found: 446 (M+H).
$^1$ H NMR (400 MHz, $CDCl_3$) δ ppm 2.77 (t, J = 5.6 Hz, 2H), 2.86 (d, J = 4.8 Hz, 3H), 3.61 (t, J = 5.6 Hz, 2H), 3.84 (s, 3H), 4.62 (s, 2H), 4.69-4.70 (m, 1H), 4.88 (s, 2H), 6.78 (d, J = 8.8 Hz, 1H), 7.05 (dd, J = 8.8, 2.4 Hz, 1H), 8.37 (d, J = 2.4 Hz, 1H), 8.77 (s, 1H).

Example 211

1-{2-[(5-Chloro-2-methoxyphenyl)amino]-2-oxoethyl}-N,N-dimethyl-3-(trifluoromethyl)-1,4,5,7-tetrahydro-6H-pyrazolo [3,4-c]pyridine-6-carboxamide

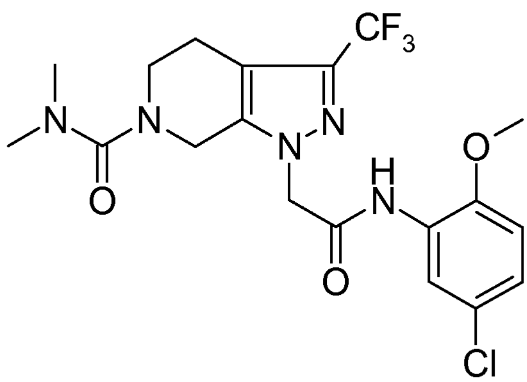

A solution of N-(5-chloro-2-methoxyphenyl)-2-[3-(trifluoromethyl)-4,5,6,7-tetrahydro-1H-pyraz olo[3,4-c]pyridin-1-yl] acetamide (100 mg, 0.26 mmol), and bis(trichloromethyl) carbonate (76 mg, 0.26 mmol) in ethyl acetate (50 mL) was

heated to reflux for 3 hours. After the solvent wa s evaporated off under reduced pressure, DCM (30 mL), and 2.0 M dimethylamine/ethanol sol ution (1 mL) were added thereto. The mixture was stirred for 1 hour at room temperature. Th e reaction mixture was concentrated under reduced pressure. The obtained residue was purifi ed by column chromatography on silica gel (DCM/methanol = 10:1) to give the titled compoun d (60 mg, yield 50%) as a white solid.
MS Calcd.: 459, MS Found: 460 (M+H).
$^1$ H NMR (400 MHz, $CDCl_3$) δ ppm 2.82 (t, J = 5.6 Hz, 2H), 2.90 (s, 6H), 3.45 (t, J = 5.6 Hz, 2H ), 3.83 (s, 3H), 4.36 (s, 2H), 4.87 (s, 2H), 6.77 (d, J = 8.8 Hz, 1H), 7.03 (dd, J = 8.8, 2.8 Hz, 1H), 8.38 (d, J = 2.8 Hz, 1H), 8.81 (br s, 1H).

Example 212

tert-Butyl 1-{2-[(5-chloro-2-methoxyphenyl)amino]-2-oxoethyl}-3-(trifluoromethyl)-1,4,6,7-tetr ahydro-5H-pyrazolo [4,3-c]pyridine-5-carboxylate

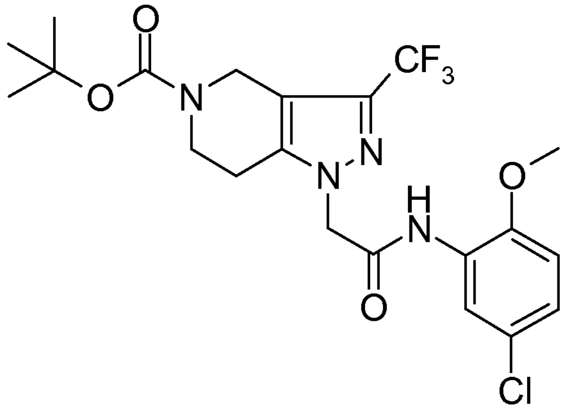

A solution of [5-(tert-butoxycarbonyl)-3-(trifluoromethyl)-4,5,6,7-tetrahydro-1H-pyrazolo[4,3-c] pyridin-1-yl]acetic acid (700 mg, 2.00 mmol), 5-chloro-2-methoxyaniline (374 mg, 2.40 mmol), HOBt (406 mg, 3.00 mmol), triethylamine (606 mg, 6.00 mmol), and WSC (576 mg, 3.00 mmol) in dichloromethane (200 mL) was stirred at room temperature under nitrogen atmosphere ov ernight. The reaction mixture was washed with water, and saturated saline, dried over sodiu m sulfate, and concentrated under reduced pressure. The residue was purified by column chro matography on silica gel (PE/EA = 5:1) to give the titled compound (490 mg, yield 50%) as a ye llow solid.
MS Calcd.: 488; MS Found: 389 (M -Boc+2H).
$^1$H NMR (300 MHz, $CDCl_3$) δ ppm 1.48 (s, 9H), 2.73 (t, J = 5.7 Hz, 2H), 3.74 (t, J = 5.7 Hz, 2H ), 3.81 (s, 3H), 4.53 (br s, 2H), 4.85 (s, 2H), 6.75 (d, J = 8.4 Hz, 1H), 7.02 (dd, J = 8.4, 2.4 Hz, 1 H), 8.36 (d, J = 2.7 Hz, 1H), 8.78 (br s, 1H).

Example 213

tert-Butyl 1-(2-{[1-(4-chlorophenyl)ethyl]amino}-2-oxoethyl)-3-(trifluoromethyl)-1,4,6,7-tetrahy dro-5H-pyrazolo[4,3-c] pyridine-5-carboxylate

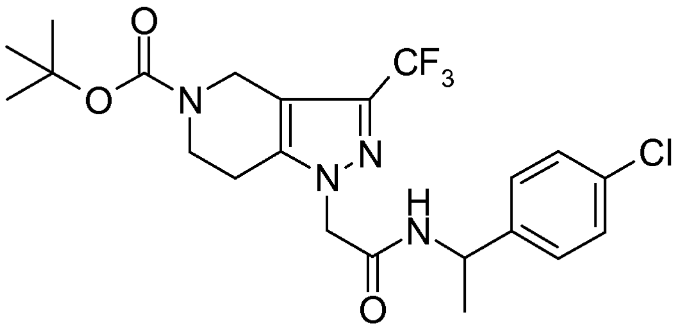

A solution of [5-(tert-butoxycarbonyl)-3-(trifluoromethyl)-4,5,6,7-tetrahydro-1H-pyrazolo[4,3-c] pyridin-1-yl]acetic acid (700 mg, 2.00 mmol), 1-(4-chlorophenyl)ethylamine (372 mg, 2.40 mmo 1, 1.2 equiv.), HOBt (406 mg, 3.00 mmol), triethylamine (606 mg, 6.00 mmol), and WSC (576 m g, 3.00 mmol) in dichloromethane (200 mL) was stirred at room temperature under nitrogen a tmosphere overnight. The reaction mixture was washed with water, and saturated saline, drie d over sodium sulfate, and concentrated under reduced pressure. The residue was purified by column chromatography on silica gel (PE/EA = 5:1) to give the titled compound (520 mg, yield 53%) as yellow solid.
MS Calcd.: 486; MS Found: 487 (M+H).
$^1$ H NMR (300 MHz, DMSO-$d_6$) δ ppm 1.37 (d, J = 7.2 Hz, 3H), 1.41 (s, 9H), 2.62-2.64 (m, 2H), 3.57-3.61 (m, 2H), 4.40 (s, 2H), 4.85-4.90 (m, 3H), 7.32-7.40 (m, 4H), 8.90 (d, J = 7.5 Hz, 1H).

Example 214

N-(5-Chloro-2-methoxyphenyl)-2-[3-(trifluoromethyl)-4,5,6,7-tetrahydro-1H-pyrazolo[4,3-c]pyri din-1-yl]acetamide

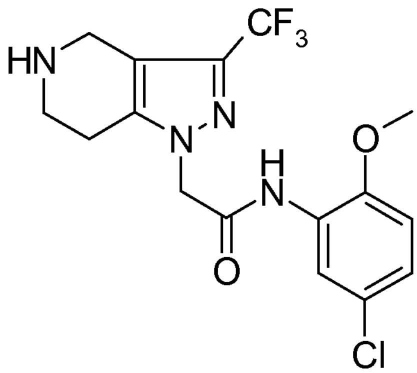

A solution of tert-butyl 1-{2-[(5-chloro-2-methoxyphenyl)amino]-2-oxoethyl}-3-(trifluoromethyl )-1,4,6,7-tetrahydro-5H-pyrazolo[4,3-c]pyridine-5-carboxylate (300 mg, 0.62 mmol) in DCM (40 mL) was stirred at room temperature, trifluoroacetic acid (5 mL) was added thereto. The mixt ure was stirred for 4 hours, and the solvent was evaporated off under reduced pressure. To the residue, were added DCM (80 mL), and potassium carbonate (2 g), and the mixture was stirre d for 30 minutes, and filtered. The filtrate was concentrated under reduced pressure. The resi due was purified by preparative HPLC to give the titled compound (180 mg, yield 75%) as a w hite solid.
MS Calcd.: 388; MS Found: 389 (M+H).
$^1$ H NMR (300 MHz, $CDCl_3$) δ ppm 2.73 (t, J = 5.7 Hz, 2H), 2.94 (t, J = 5.7 Hz, 2H), 3.36 (s, 1H ), 3.66 (s, 2H), 3.79 (s, 3H), 4.84 (d, J = 2.7 Hz, 2H), 6.74 (dd, J = 8.7, 1.5 Hz, 1H), 7.00 (dd, J = 8.7, 2.4 Hz, 1H), 8.36 (d, J = 2.7 Hz, 1H), 8.90 (br s, 1H).

Example 215

N-[1-(4-Chlorophenyl)ethyl]-2-[3-(trifluoromethyl)-4,5,6,7-tetrahydro-1H-pyrazolo[4,3-c]pyridi n-1-yl]acetamide

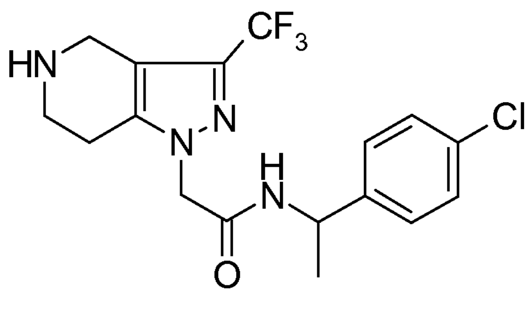

To a solution of tert-butyl 1-(2-{[1-(4-chlorophenyl)ethyl]amino}-2-oxoethyl)-3-(trifluoromethyl )-1,4,6,7-tetrahydro-5H-pyrazolo[4,3-c]pyridine-5-carboxylate (150 mg, 0.31 mmol) in DCM (10 mL) stirred at room temperature, was added trifluoroacetic acid (3 mL). The mixture was stir red for 4 hours, and the solvent was evaporated off under reduced pressure. To the residue, we re added DCM (30 mL), and potassium carbonate (1 g), and the mixture was stirred for 30 min utes, and filtered. The filtrate was concentrated under reduced pressure to give the titled com pound (105 mg, yield 88%) as a yellow solid.

MS Calcd.: 386; MS Found: 387 (M+H).

$^{1}$ H NMR (300 MHz, $CD_3$ OD) δ ppm 1.47 (d, J = 6.9 Hz, 3H), 2.94-2.96 (m, 2H), 3.43 (t, J = 6.3 Hz, 2H), 4.20 (s, 2H), 4.91 (s, 2H), 4.96-4.98 (m, 1H), 7.31 (s, 4H).

Example 216

N-(5-Chloro-2-methoxyphenyl)-2-[5-methyl-3-(trifluoromethyl)-4,5,6,7-tetrahydro-1H-pyrazolo [4,3-c]pyridin-1-yl]acetamide

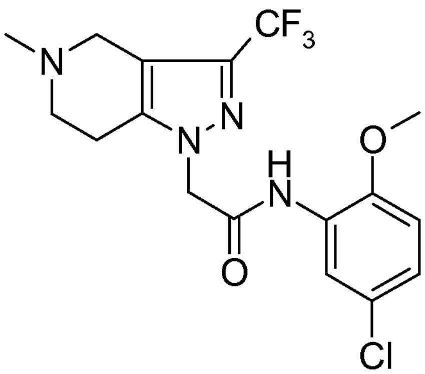

A solution of N-(5-chloro-2-methoxyphenyl)-2-[3-(trifluoromethyl)-4,5,6,7-tetrahydro-1H-pyraz olo[4,3-c]pyridin-1-yl] acetamide (420 mg, 1.08 mmol), and 37 % aqueous formaldehyde solutio n (0.3 mL) in formic acid (15 mL) was heated to reflux for 3 hours. The reaction mixture was adjusted to pH 7 with saturated sodium carbonate aqueous solution, and extracted with DCM. The organic layer was washed with saturated saline, dried over sodium sulfate, and concentr ated under reduced pressure. The obtained residue was purified by column chromatography o n silica gel (PE/EA = 3:1) to give the titled compound (195 mg, yield 45%) as a yellow solid.

MS Calcd.: 402; MS Found: 403 (M+H).

$^{1}$ H NMR (300 MHz, $CDCl_3$) δ ppm 2.50 (s, 3H), 2.77-2.80 (m, 4H), 3.53 (s, 2H), 3.78 (s, 3H), 4. 84 (s, 2H), 6.74 (d, J = 8.7 Hz, 1H), 7.00 (dd, J = 8.7, 2.4 Hz, 1H), 8.36 (d, J = 2.4 Hz, 1H), 8.78 (br s, 1H).

Example 217

N-[1-(4-Chlorophenyl)ethyl]-2-[5-methyl-3-(trifluoromethyl)-4,5,6,7-tetrahydro-1H-pyrazolo[4, 3-c]pyridin-1-yl]acetamide

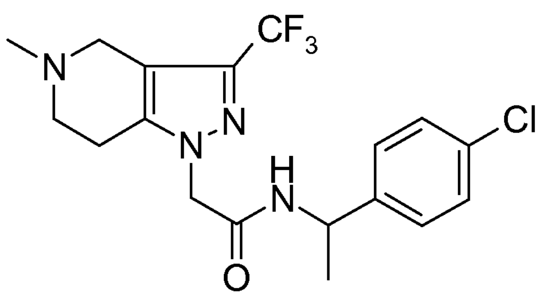

A solution of N-[1-(4-chlorophenyl)ethyl]-2-[3-(trifluoromethyl)-4,5,6,7-tetrahydro-1H-pyrazolo [4,3-c]pyridin-1-yl]acetamide (65 mg, 0.17 mmol), and 37 % aqueous formaldehyde solution (0. 1 mL) in formic acid (5 mL) was heated to reflux for 3 hours. The reaction mixture was adjust ed to pH 7 with saturated sodium carbonate aqueous solution, extracted with DCM, washed w ith saturated saline, dried over sodium sulfate, and concentrated under reduced pressure. The obtained residue was purified by column chromatography on silica gel (PE/EA = 3:1) to give t he titled compound (55 mg, yield 80%) as a white solid.
MS Calcd.: 400; MS Found: 401 (M+H).
$^1$ H NMR (300 MHz, $CDCl_3$) δ ppm 1.40 (d, J = 6.9 Hz, 3H), 2.50 (s, 3H), 2.67-2.80 (m, 4H), 3.4 8 (d, J = 14.4 Hz, 1H), 3.55 (d, J = 14.4 Hz, 1H), 4.64 (d, J = 16.5 Hz, 1H), 4.70 (d, J = 16.5 Hz, 1H), 4.95-5.05 (m, 1H), 6.67 (d, J = 7.5 Hz, 1H), 7.11-7.16 (m, 2H), 7.25-7.30 (m, 2H)

Example 218

N-(5-Chloro-2-methoxyphenyl)-2-[5-(1-methylethyl)-3-(trifluoromethyl)-4,5,6,7-tetrahydro-1H-pyrazolo[4,3-c]pyridin-1-yl]acetamide

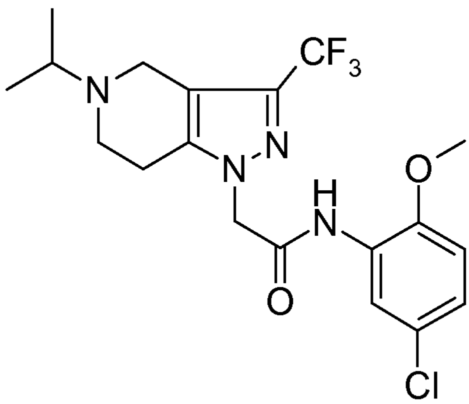

To a solution of N-(5-chloro-2-methoxyphenyl)-2-[3-(trifluoromethyl)-4,5,6,7-tetrahydro-1H-py razolo[4,3-c]pyridin-1-yl] acetamide (100 mg, 0.26 mmol), and acetone (150 mg, 2.60 mmol) in methanol (20 mL) stirred at room temperature, was added sodium cyanoborohydride (82 mg, 1 .30 mmol). The mixture was stirred at room temperature overnight, and concentrated under r educed pressure. To the residue, was added ice water (20 mL) carefully, and the mixture was extracted with dichloromethane. The organic layer was washed with saturated saline, dried o ver anhydrous sodium sulfate, and concentrated under reduced pressure. The obtained solid w as washed with diethyl ether to give the titled compound (35 mg, yield 31%) as a yellow solid.
MS Calcd.: 430; MS Found: 431 (M+H).
$^1$ H NMR (400 MHz, $CDCl_3$) δ ppm 1.13 (d, J = 6.8 Hz, 6H), 2.74 (t, J = 5.6 Hz, 2H), 2.83 (t, J = 5.6 Hz, 2H), 2.98-3.04 (m, 1H), 3.65 (s, 2H), 3.80 (s, 3H), 4.82 (s, 2H), 6.74 (d, J = 8.4 Hz, 1H), 7.01 (dd, J = 8.8, 2.4 Hz, 1H), 8.37 (d, J = 2.4 Hz, 1H), 8.88 (br s, 1H).

Example 219

N-(5-Chloro-2-methoxyphenyl)-2-[5-(cyclopropylmethyl)-3-(trifluoromethyl)-4,5,6,7-tetrahydro-1H-pyrazolo[4,3-c]pyridin-1-yl]acetamide

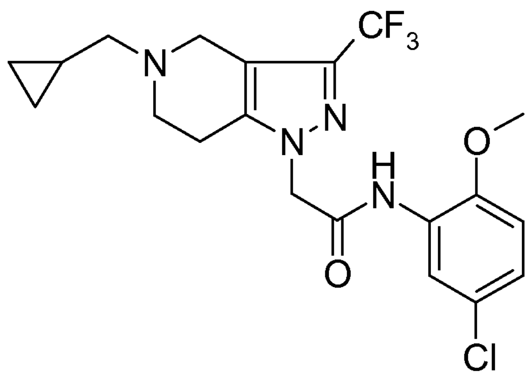

A solution of N-(5-chloro-2-methoxyphenyl)-2-[3-(trifluoromethyl)-4,5,6,7-tetrahydro-1H-pyraz olo[4,3-c]pyridin-1-yl] acetamide (100 mg, 0.26 mmol), (bromomethy)cyclopropane (104 mg, 0.7 7 mmol), and potassium carbonate (178 mg, 1.30 mmol) in acetone (150 mL) was heated to refl ux overnight. The reaction mixture was concentrated under reduced pressure. The obtained re sidue was purified by column chromatography on silica gel (PE/EA = 3:1) to give the titled co mpound (57 mg, yield 50%) as a white solid.

MS Calcd.: 442; MS Found: 443 (M+H).
$^1$ H NMR (400 MHz, $CDCl_3$) δ ppm 0.18-0.22 (m, 2H), 0.59-0.64 (m, 2H), 0.95-0.97 (m, 1H), 2.5 2 (d, J = 6.4 Hz, 2H), 2.80 (t, J = 5.6 Hz, 2H), 2.94 (t, J = 5.6 Hz, 2H), 3.70 (s, 2H), 3.83 (s, 3H), 4.86 (s, 2H), 6.78 (d, J = 8.8 Hz, 1H), 7.04 (dd, J = 8.8, 2.4 Hz, 1H), 8.41 (d, J = 2.4 Hz, 1H), 8.8 8 (s, 1H).

Example 220

Ethyl [1-{2-[(5-chloro-2-methoxyphenyl)amino]-2-oxoethyl}-3-(trifluoromethyl)-1,4,6,7-tetrahy dro-5H-pyrazolo[4,3-c]pyridin-5-yl] acetate

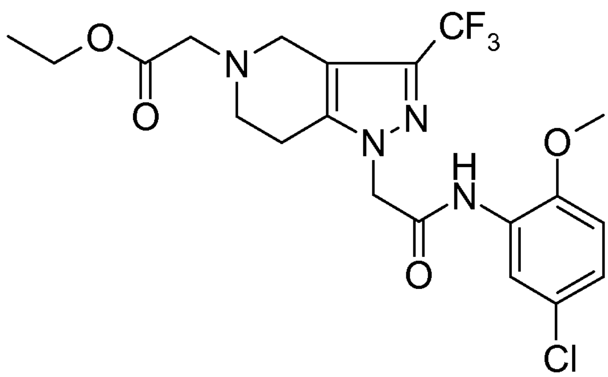

A solution of N-(5-chloro-2-methoxyphenyl)-2-[3-(trifluoromethyl)-4,5,6,7-tetrahydro-1H-pyraz olo[4,3-c]pyridin-1-yl] acetamide (1100 mg, 2.83 mmol), ethyl bromoacetate (568 mg, 3.40 mmol ), and potassium carbonate (1956 mg, 14.17 mmol) in acetone (150 mL) was heated to reflux o vernight. After the solvent was evaporated off, water was added thereto, and the mixture was extracted with DCM. The organic layer was washed with saturated saline, dried over anhydro us sodium sulfate, and concentrated under reduced pressure. The residue was washed with di ethyl ether, and filtered to give the titled compound (1080 mg, yield 80%) as a white solid.
MS Calcd.: 474; MS Found: 475 (M+H).
$^1$ H NMR (400 MHz, $CDCl_3$) δ ppm 1.29 (t, J = 6.8 Hz, 3H), 2.79 (t, J = 5.6 Hz, 2H), 3.00 (t, J = 5.6 Hz, 2H), 3.48 (s, 2H), 3.78-3.80 (m, 5H), 4.21 (q, J = 6.8 Hz, 2H), 4.84 (s, 2H), 6.75 (d, J = 8. 8 Hz, 1H), 7.01 (dd, J = 8.8, 2.4 Hz, 1H), 8.37 (d, J = 2.8 Hz, 1H), 8.78 (br s, 1H).

Example 221

[1-{2-[(5-Chloro-2-methoxyphenyl)amino]-2-oxoethyl}-3-(trifluoromethyl)-1,4,6,7-tetrahydro-5 H-pyrazolo[4,3-c]pyridin-5-yl]acetic acid

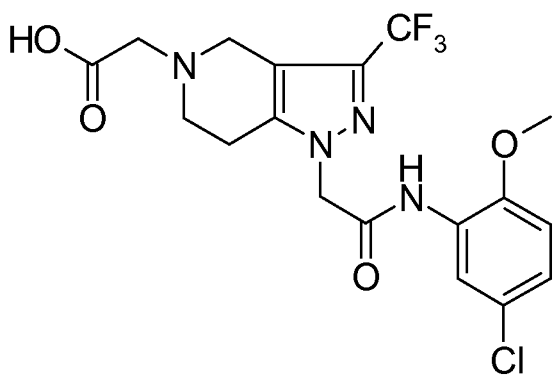

To a solution of ethyl [1-{2-[(5-chloro-2-methoxyphenyl)amino]-2-oxoethyl}-3-(trifluoromethyl)-1,4,6,7-tetrahydro-5H-pyrazolo[4,3-c]pyridin-5-yl]acetate (50 mg, 0.11 mmol) in THF (2 mL), a nd methanol (2 mL), was added a solution of lithium hydride (20 mg) in water (1 mL). The mix ture was stirred at room temperature overnight, and the solvent was evaporated off under red uced pressure. The residue was adjusted to pH 7 with 4N hydrochloric acid. White solids were precipitated, filtered, and washed with cooled water. And the filtrate was dried under reduce d pressure to give the titled compound (25 mg, yield 53%) as a white solid.
MS Calcd.: 446; MS Found: 447 (M+H).
$^1$ H NMR (400 MHz, $CD_3$ OD) δ ppm 2.98 (t, J = 5.6 Hz, 2H), 3.40 (t, J = 5.6 Hz, 2H), 3.71 (s, 2 H), 3.93 (s, 3H), 4.34 (s, 2H), 5.17 (s, 2H), 7.03 (d, J = 8.8 Hz, 1H), 7.12 (dd, J = 8.8, 2.8 Hz, 1H ), 8.17 (d, J = 2.4 Hz, 1H).

Example 222

2-[5-(2-Amino-2-oxoethyl)-3-(trifluoromethyl)-4,5,6,7-tetrahydro-1H-pyrazolo[4,3-c]pyridin-1-y 1]-N-(5-chloro-2-methoxyphenyl)acetamide

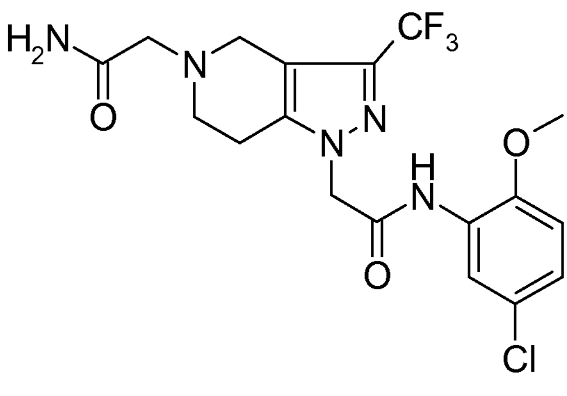

A solution of ethyl [1-{2-[(5-chloro-2-methoxyphenyl)amino]-2-oxoethyl}-3-(trifluoromethyl)-1,4 ,6,7-tetrahydro-5H-pyrazolo[4,3-c]pyridin-5-yl]acetate (200 mg, 0.42 mmol) in 2.0 M ammonia/ methanol (40 mL) was stirred at room temperature overnight. The reaction mixture was conce ntrated under reduced pressure. The obtained residue was purified by column chromatograph y on silica gel (DCM/methanol = 10:1) to give the titled compound (105 mg, yield 56%) as a wh ite solid.
MS Calcd.: 445, MS Found: 446 (M+H).
$^1$ H NMR (400 MHz, $CDCl_3$) δ ppm 2.80 (t, J = 5.6 Hz, 2H), 2.93 (t, J = 5.6 Hz, 2H), 3.24 (s, 2H ), 3.70 (s, 2H), 3.82 (s,

3H), 4.86 (s, 2H), 5.52 (br s, 1H), 6.77 (d, J = 8.8 Hz, 1H), 6.91 (br s, 1H), 7.02 (dd, J = 8.8, 2.8 Hz, 1H), 8.38 (d, J = 2.8 Hz, 1H), 8.83 (br s, 1H).

Example 223

2-[1-{2-[(5-Chloro-2-methoxyphenyl)amino]-2-oxoethyl}-3-(trifluoromethyl)-1,4,6,7-tetrahydro-5H-pyrazolo[4,3-c]pyridin-5-yl]-N-methylacetamide

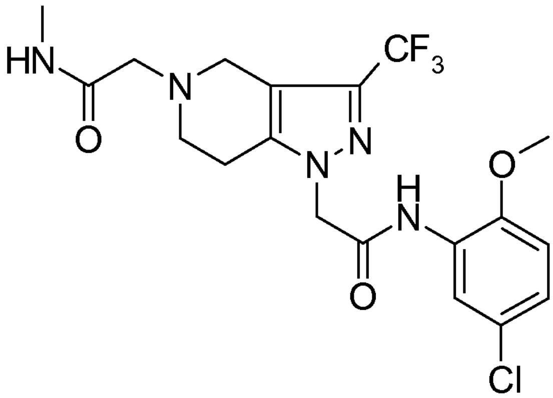

A mixture of ethyl [1-{2-[(5-chloro-2-methoxyphenyl)amino]-2-oxoethyl}-3-(trifluoromethyl)-1,4 ,6,7-tetrahydro-5H-pyrazolo[4,3-c]pyridin-5-yl]acetate (150 mg, 0.32 mmol), and 40 % methyla mine aqueous solution (30 mL) was stirred at room temperature overnight. The reaction mixt ure was concentrated under reduced pressure. The obtained residue was purified by column c hromatography on silica gel (DCM/methanol = 10:1) to give the titled compound (80 mg, yield 54%) as a white solid.
MS Calcd.: 459; MS Found: 460 (M+H).
$^1$ H NMR (400 MHz, $CDCl_3$ ) δ ppm 2.83 (t, J = 5.6 Hz, 2H), 2.88 (d, J = 4.8 Hz, 3H), 2.93 (t, J = 5.6 Hz, 2H), 3.27 (s, 2H), 3.70 (s, 2H), 3.85 (s, 3H), 4.88 (s, 2H), 6.80 (d, J = 8.8 Hz, 1H), 7.04-7 .07 (2H, m), 8.41 (d, J = 2.8 Hz, 1H), 8.86 (br s, 1H).

Example 224

2-[1-{2-[(5-Chloro-2-methoxyphenyl)amino]-2-oxoethyl}-3-(trifluoromethyl)-1,4,6,7-tetrahydro-5H-pyrazolo[4,3-c]pyridin-5-yl]-N,N-dimethylacetamide

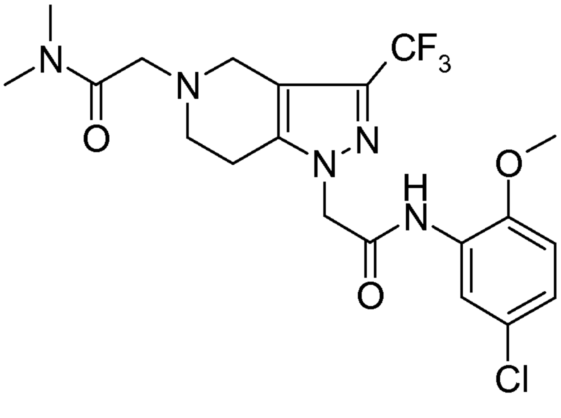

A mixture of ethyl [1-{2-[(5-chloro-2-methoxyphenyl)amino]-2-oxoethyl}-3-(trifluoromethyl)-1,4 ,6,7-tetrahydro-5H-pyrazolo[4,3-c]pyridin-5-yl]acetate (200 mg, 0.42 mmol), and 2.0 M dimeth ylamine/ethanol solution (20 mL), in a sealed tube, was stirred at 70 °C overnight. After coolin g, the solvent was evaporated off under reduced pressure, and the obtained residue was prepa rative HPLC to give the titled compound (29 mg, yield 14%) as a white solid.
MS Calcd.: 473; MS Found: 474 (M+H).
$^1$ H NMR (400 MHz, DMSO-$d_6$ ) δ ppm 2.59-2.62 (m, 2H), 2.80-2.82 (m, 5H), 3.00 (s, 3H), 3.44 ( s, 2H), 3.70 (s, 2H), 3.88 (s, 3H), 5.16 (s, 2H), 7.10-7.18 (m, 2H), 8.09 (d, J = 2.8 Hz, 1H), 9.83 (s , 1H).

Example 225

N-(5-Chloro-2-methoxyphenyl)-2-[5-(2-hydroxyethyl)-3-(trifluoromethyl)-4,5,6,7-tetrahydro-1 H-pyrazolo[4,3-c]pyridin-1-yl]acetamide

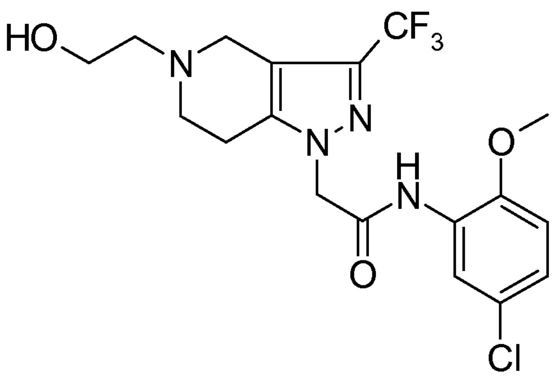

To a solution of ethyl [1-{2-[(5-chloro-2-methoxyphenyl)amino]-2-oxoethyl}-3-(trifluoromethyl)-1,4,6,7-tetrahydro-5H-pyrazolo[4,3-c]pyridin-5-yl]acetate (150 mg, 0.32 mmol) in THF (10 mL) stirred at ice-bath temperature, was added lithium aluminium hydride (24 mg, 0.64 mmol). T he mixture was stirred for 3 hours. To the he reaction mixture, was added sodium sulfate deca hydrate (750 mg). The mixture was stirred overnight, and concentrated under reduced pressu re. The obtained residue was purified by column chromatography on silica gel (DCM/methanol = 10:1) to give the titled compound (85 mg, yield 61%) as a white solid.
MS Calcd.: 432; MS Found: 433 (M+H)..
$^1$ H NMR (300 MHz, CDCl3 ) δ ppm 2.75-2.79 (m, 4H), 2.90 (t, J = 5.4 Hz, 2H), 3.65 (s, 2H), 3.7 1 (t, J = 5.4 Hz, 2H), 3.81 (s, 3H), 4.84 (s, 2H), 6.75 (d, J = 8.7 Hz, 1H), 7.01 (dd, J = 8.7, 2.4 Hz , 1H), 8.37 (d, J = 2.4 Hz, 1H), 8.82 (br s, 1H).

Example 226

2-[5-Acetyl-3-(trifluoromethyl)-4,5,6,7-tetrahydro-1H-pyrazolo[4,3-c]pyridin-1-yl]-N-(5-chloro-2-methoxyphenyl)acetamide

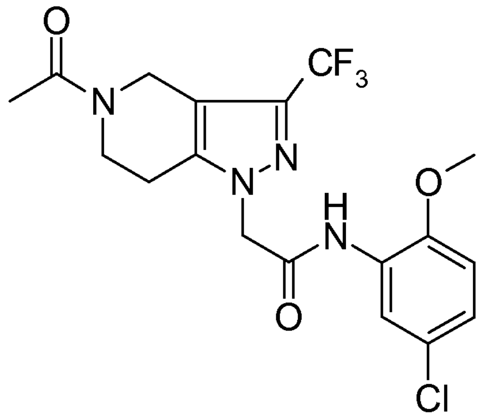

To a solution of N-(5-chloro-2-methoxyphenyl)-2-[3-(trifluoromethyl)-4,5,6,7-tetrahydro-1H-py razolo[4,3-c]pyridin-1-yl] acetamide (150 mg, 0.39 mmol), and triethylamine (0.8 mL) in dichlor omethane (20 mL) stirred on an ice bath, was added acetyl chloride (60 mg, 0.77 mmol), the m ixture was stirred for 2 hours at room temperature. The reaction mixture was concentrated un der reduced pressure, and the residue was purified by column chromatography on silica gel (P E/EA = 3:1) to give the titled compound (83 mg, yield 49%) as a yellow solid.
MS Calcd.: 430, MS Found: 431 (M+H).
$^1$ H NMR (400 MHz, $CDCl_3$ ) δ ppm 2.21 (s, 1.5H), 2.22 (s, 1.5H), 2.79 (t, J = 6.0 Hz, 1H), 2.85 ( t, J = 5.6 Hz, 1H), 3.79 (t, J = 6.0 Hz, 1H), 3.83 (s, 1.5H), 3.85 (s, 1.5H), 3.96 (t, J = 6.0 Hz, 1H), 4.58 (s, 1H), 4.74 (s, 1H), 4.89 (s, 2H), 6.79 (dd, J = 8.4, 2.0 Hz, 1H), 7.05 (dd, J = 8.4, 2.4 Hz, 1 H), 8.39 (s, 1H), 8.76 (s, 0.5H), 8.80 (s, 0.5H).

Example 227

N-(5-Chloro-2-methoxyphenyl)-2-[5-(methylsulfonyl)-3-(trifluoromethyl)-4,5,6,7-tetrahydro-1 H-pyrazolo[4,3-c]pyridin-1-yl]acetamide

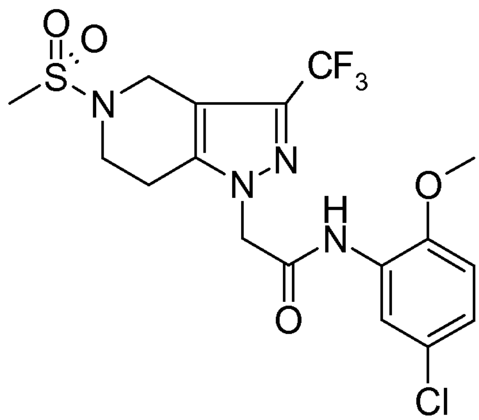

To a solution of N-(5-chloro-2-methoxyphenyl)-2-[3-(trifluoromethyl)-4,5,6,7-tetrahydro-1H-py razolo[4,3-c]pyridin-1-yl] acetamide (200 mg, 0.52 mmol) in pyridine (10 mL) stirred at ice-bat h temperature, methanesulfonyl chloride (102 mg, 1.04 mmol) was added thereto, and the mix ture was stirred for 2 hours at room temperature. The reaction mixture was concentrated und er reduced pressure, and the residue was purified by column chromatography on silica gel (PE /EA = 2:1) to give the titled compound (138 mg, yield 57%) as a white solid.
MS Calcd.: 466, MS Found: 467 (M+H).
$^1$ H NMR (400 MHz, $CDCl_3$) δ ppm 2.91-2.94 (m, 5H), 3.68 (t, J = 5.6 Hz, 2H), 3.85 (s, 3H), 4.4 5 (s, 2H), 4.91 (s, 2H), 6.79 (d, J = 8.4 Hz, 1H), 7.05 (dd, J = 8.4, 2.8 Hz, 1H), 8.37 (d, J = 2.8 H z, 1H), 8.69 (br s, 1H).

Example 228

1-{2-[(5-Chloro-2-methoxyphenyl)amino]-2-oxoethyl}-3-(trifluoromethyl)-1,4,6,7-tetrahydro-5 H-pyrazolo[4,3-c]pyridine-5-carboxamide

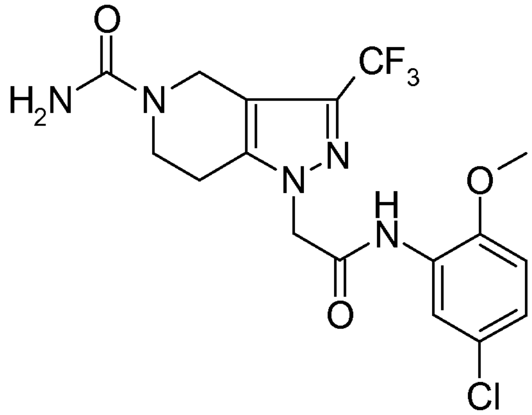

A solution of N-(5-chloro-2-methoxyphenyl)-2-[3-(trifluoromethyl)-4,5,6,7-tetrahydro-1H-pyraz olo[4,3-c]pyridin-1-yl] acetamide (200 mg, 0.52 mmol), and bis(trichloromethyl) carbonate (152 mg, 0.52 mmol) in ethyl acetate (50 mL) was heated to reflux for 3 hours. After the solvent wa s evaporated off, DCM (50 mL) was added thereto, and then a solution of 2.0 M ammonia/met hanol (2 mL) was added. The mixture was stirred for 1 hour at room temperature. The reactio n mixture was concentrated under reduced pressure. The obtained residue was purified by col umn chromatography on silica gel (DCM/methanol = 10:1) to give the titled compound (105 m g, yield 47%) as a white solid.
MS Calcd.: 431, MS Found: 432 (M+H).
$^1H$ NMR (400 MHz, $CDCl_3$ ) δ ppm 2.83 (t, J = 7.2 Hz, 2H), 3.79 (t, J = 5.6 Hz, 2H), 3.84 (s, 3H ), 4.53 (s, 2H), 4.66 (s, 2H), 4.90 (s, 2H), 6.79 (d, J = 8.8 Hz, 1H), 7.05 (dd, J = 8.8, 2.4 Hz, 1H), 8.39 (d, J = 2.4 Hz, 1H), 8.77 (br s, 1H).

Example 229

1-{2-[(5-Chloro-2-methoxyphenyl)amino]-2-oxoethyl}-N-methyl-3-(trifluoromethyl)-1,4,6,7-tetr ahydro-5H-pyrazolo [4,3-c]pyridine-5-carboxamide

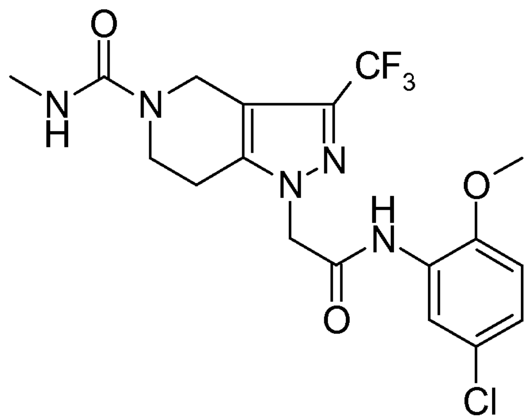

A solution of N-(5-chloro-2-methoxyphenyl)-2-[3-(trifluoromethyl)-4,5,6,7-tetrahydro-1H-pyraz olo[4,3-c]pyridin-1-yl] acetamide (300 mg, 0.78 mmol), and bis(trichloromethyl) carbonate (228 mg, 0.78 mmol) in ethyl acetate (100 mL) was heated to reflux for 3 hours. After the solvent w as evaporated off, DCM (80 mL), and 40 % methylamine aqueous solution (3 mL) was added t hereto. The mixture was stirred for 1 hour at room temperature. The reaction mixture was co

ncentrated under reduced pressure. The obtained residue was purified by column chromatogr aphy on silica gel (DCM/ methanol = 10:1) to give the titled compound (240 mg, yield 69%) as a white solid.
MS Calcd.: 445, MS Found: 446 (M+H).
$^1$H NMR (300 MHz, $CDCl_3$) δ ppm 2.77 (t, J = 5.7 Hz, 2H), 2.84 (d, J = 4.5 Hz, 3H), 3.76 (t, J = 5.7 Hz, 2H), 3.81 (s, 3H), 4.44 (s, 2H), 4.51-4.54 (m, 1H), 4.86 (s, 2H), 6.75 (d, J = 8.7 Hz, 1H), 7.02 (dd, J = 8.7, 2.4 Hz, 1H), 8.36 (d, J = 2.7 Hz, 1H), 8.76 (br s, 1H).

Example 230

1-{2-[(5-Chloro-2-methoxyphenyl)amino]-2-oxoethyl}-N,N-dimethyl-3-(trifluoromethyl)-1,4,6,7-tetrahydro-5H-pyrazolo [4,3-c]pyridine-5-carboxamide

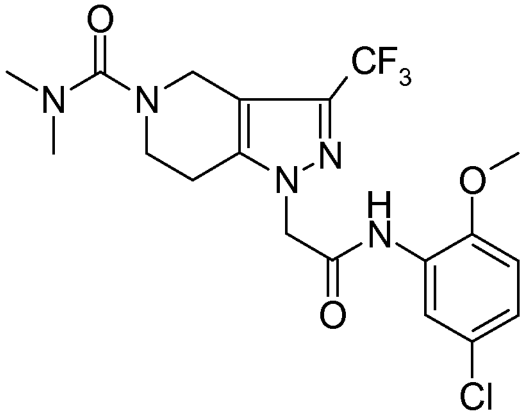

A solution of N-(5-chloro-2-methoxyphenyl)-2-[3-(trifluoromethyl)-4,5,6,7-tetrahydro-1H-pyraz olo[4,3-c]pyridin-1-yl] acetamide (100 mg, 0.26 mmol), and bis(trichloromethyl) carbonate (76 mg, 0.26 mmol) in ethyl acetate (50 mL) was heated to reflux for 3 hours. After the solvent wa s evaporated off, DCM (30 mL), and 2.0 M dimethylamine/ethanol solution (1 mL) were added thereto. The mixture was stirred for 1 hour at room temperature. The reaction mixture was co ncentrated under reduced pressure. The obtained residue was purified by column chromatogr aphy on silica gel (DCM/methanol = 10:1) to give the titled compound (80 mg, yield 67%) as a white solid.
MS Calcd.: 459, MS Found: 460 (M+H).
$^1$H NMR (400 MHz, $CDCl_3$ ) δ ppm 2.84 (t, J = 5.6 Hz, 2H), 2.87 (s, 6H), 3.53 (t, J = 5.6 Hz, 2H ), 3.81 (s, 3H), 4.35 (s, 2H), 4.85 (s, 2H), 6.76 (d, J = 8.8 Hz, 1H), 7.02 (dd, J = 8.8, 2.4 Hz, 1H), 8.40 (d, J = 2.4 Hz, 1H), 8.85 (br s, 1H).

Example 231

4-Oxo-3-({[3-(trifluoromethyl)-4,5,6,7-tetrahydro-1H-indazol-1-yl]acetyl}amino)-4,5,6,7-tetrahy dropyrazolo[1,5-a]pyridine-2-carboxamide

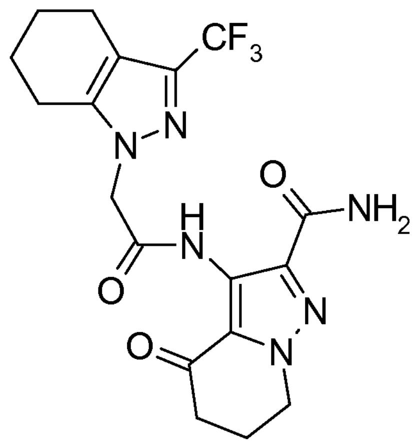

A solution of 3-amino-4-oxo-4,5,6,7-tetrahydropyrazolo[1,5-a]pyridine-2-carboxamide (194 mg, 1.00 mmol), [3-(trifluoromethyl)-4,5,6,7-tetrahydro-1H-indazol-1-yl]acetic acid (500 mg, 2.00 m mol), WSC (641 mg, 2.50 mmol), and DIEA (387 mg, 3.00 mmol) in 1,4-dioxane (10 mL) was he ated at 86 °C for overnight. The reaction mixture was concentrated under reduced pressure, a nd the residue was purified by preparative HPLC to give the titled compound (110 mg, yield 2 6%) as a white solid.
MS Calcd.: 424; MS Found: 425 (M+H).
$^1$ H NMR (300 MHz, DMSO-$d_6$ ) δ ppm 1.65-1.75 (m, 4H), 2.24-2.28 (m, 2H), 2.49-2.53 (m, 2H), 2.62-2.67 (m, 4H), 4.34-4.38 (m, 2H), 4.96 (s, 2H), 7.40 (br s, 1H), 7.62 (br s, 1H), 9.88 (br s, 1H).

Example 232

4-Oxo-3-({[3-(trifluoromethyl)-4,5,6,7-tetrahydro-1H-indazol-1-yl]acetyl}amino)-4,5,6,7-tetrahy dropyrazolo[1,5-a]pyrazine-2-carboxamide

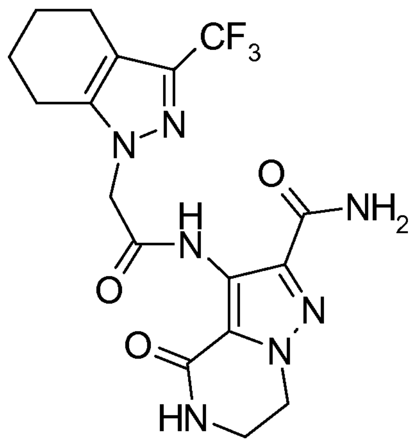

A mixture of 3-amino-4-oxo-4,5,6,7-tetrahydropyrazolo[1,5-a]pyrazine-2-carboxamide (50 mg, 0.26 mmol), [3-(trifluoromethyl)-4,5,6,7-tetrahydro-1H-indazol-1-yl]acetic acid (101 mg, 0.38 m mol), 2-chloro-1-methylpyridinium iodide (131 mg, 0.51 mmol), N,N-diisopropylethylamine (0. 15 mL), and 1,4-dioxane (10 mL) was stirred 80 °C for 5 hours. The solvent was evaporated off , and the residue was purified by preparative HPLC to give the titled compound (25 mg, yield 23%) as a white solid.
MS Calcd.: 425; MS Found: 426 (M+H).
$^1$ H NMR (300 MHz, DMSO-$d_6$ ) δ ppm 1.63-1.79 (m, 4H), 2.51-2.55 (m, 2H), 2.59-2.65 (m, 2H), 3.56-3.63 (m, 2H), 4.28-4.33 (m, 2H), 4.93 (s, 2H), 7.33 (br s, 1H), 7.52 (br s, 1H), 8.27-8.28 (m, 1H), 9.77 (br s, 1H).

Example 233

5-Methyl-4-oxo-3-({[3-(trifluoromethyl)-4,5,6,7-tetrahydro-1H-indazol-1-yl]acetyl}amino)-4,5,6, 7-tetrahydropyrazolo [1,5-a]pyrazine-2-carboxamide

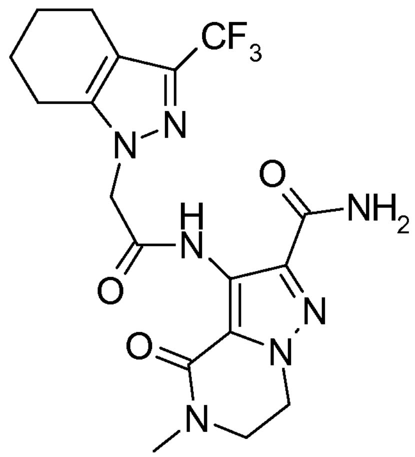

A mixture of 3-amino-5-methyl-4-oxo-4,5,6,7-tetrahydropyrazolo[1,5-a]pyrazine-2-carboxamid e (84 mg, 0.4 mmol), [3-(trifluoromethyl)-4,5,6,7-tetrahydro-1H-indazol-1-yl]acetic acid (150 m g, 0.6 mmol), 2-chloro-1-methylpyridinium iodide (205 mg, 0.8 mmol), N,N-diisopropylethylam ine (0.2 mL), and 1,4-dioxane (10 mL) was stirred at 80 °C for 5 hours. The solvent was evapor ated off, and the residue was purified by preparative HPLC to give the titled compound (62 m g, yield 35%) as a white solid.

MS Calcd.: 439; MS Found: 440 (M+H).

$^1$ H NMR (300 MHz, DMSO-$d_6$ ) δ ppm 1.66-1.76 (m, 4H), 2.51-2.62 (m, 4H), 2.99 (s, 3H), 3.77 ( t, J = 6.0 Hz, 2H), 4.38 (t, J = 6.0 Hz, 2H), 4.93 (s, 2H), 7.32 (br s, 1H), 7.51 (br s, 1H), 9.75 (br s, 1H).

Example 234

N,5-Dimethyl-4-oxo-3-({[3-(trifluoromethyl)-4,5,6,7-tetrahydro-1H-indazol-1-yl]acetyl}amino)-4 ,5,6,7-tetrahydropyrazolo[1,5-a]pyrazine-2-carboxamide

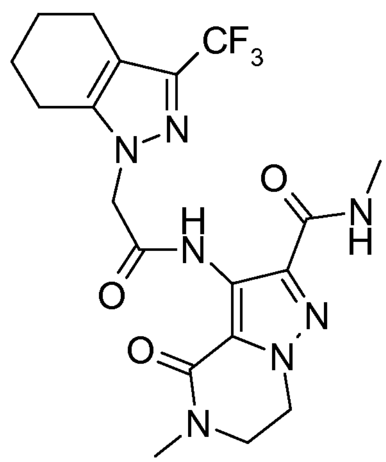

A mixture of 3-amino-N,5-dimethyl-4-oxo-4,5,6,7-tetrahydropyrazolo[1,5-a]pyrazine-2-carboxa mide (84 mg, 0.4 mmol),

[3-(trifluoromethyl)-4,5,6,7-tetrahydro-1H-indazol-1-yl]acetic acid (15 0 mg, 0.6 mmol), 2-chloro-1-methylpyridinium iodide (205 mg, 0.8 mmol), N,N-diisopropylethy lamine (0.2 mL), and 1,4-dioxane (10 mL) was stirred at 80 °C for 5 hours. The solvent was ev aporated off, and the residue was purified by preparative HPLC to give the titled compound (5 0 mg, yield 28%) as a white solid.
MS Calcd.: 453; MS Found: 454 (M+H).
$^1$ H NMR (300 MHz, DMSO-$d_6$) δ ppm 1.66-1.75 (m, 4H), 2.49-2.60 (m, 4H), 2.70 (d, J = 4.8 Hz, 3H), 2.99 (s, 3H), 3.77 (t, J = 6.0 Hz, 2H), 4.38 (t, J = 6.0 Hz, 2H), 4.93 (s, 2H), 8.09-8.12 (m, 1 H), 9.75 (br s, 1H).

Example 235

3-Methyl-1-[4-(pyrrolidin-1-ylcarbonyl)benzyl]-4,5,6,7-tetrahydro-1H-indazole

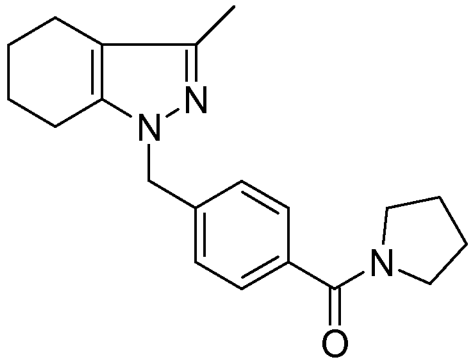

A solution of 2-acetylcyclohexanone (465 mg, 3.42 mmol), and [4-(hydrazinylmethyl)phenyl](p yrrolidin-1-yl)methanone (750 mg, 3.42 mmol) in ethanol (20 mL) was heated to reflux overnig ht. The solvent was evaporated off under reduced pressure, and the obtained residue was puri fied by preparative HPLC to give the titled compound (46 mg, yield 4%) as a white solid.
MS Calcd.: 323; MS Found: 324(M+H).
$^1$ H NMR (400 MHz, $CDCl_3$)δ ppm 1.72-1.94 (m, 8H), 2.18 (s, 3H), 2.39-2.42 (m, 4H), 3.37-3.41 (m, 2H), 3.61-3.64 (m, 2H), 5.17 (s, 2H), 7.17 (d, J = 8.0 Hz, 2H), 7.42-7.46 (m, 2H).

Example 236

Methyl 4-[(3-ethyl-4,5,6,7-tetrahydro-1H-indazol-1-yl)methyl]benzoate

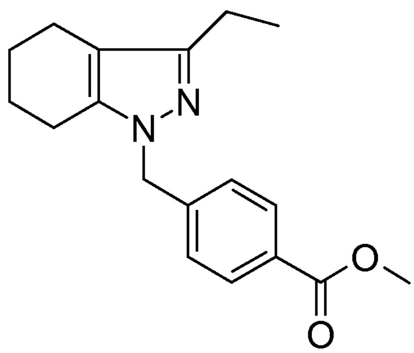

A solution of 2-propanoylcyclohexanone (540 mg, 3.51 mmol), methyl 4-(hydrazinylmethyl)ben zoate (390 mg, 2.19 mmol), and p-toluenesulfonic acid (25 mg) in toluene (20 mL) was stirred a t 90 °C for 3 hours. The solvent was evaporated off under reduced pressure, and the residue w as purified by preparative HPLC to give the titled compound (80 mg, yield

12%).
MS Calcd.: 298; MS Found: 299(M+H).
$^1$ H NMR (400 MHz, $CDCl_3$ )δ ppm 1.04 (t, J = 7.6 Hz, 3H), 1.77-1.86 (m, 4H), 2.47-2.54 (m, 4H) , 2.70 (t, J = 6.0 Hz, 2H), 3.93 (s, 3H), 5.31 (s, 2H), 7.15-7.17 (m, 2H), 7.99-8.01 (m, 2H).

Example 237

4-[(3-Ethyl-4,5,6,7-tetrahydro-1H-indazol-1-yl)methyl]benzoic acid

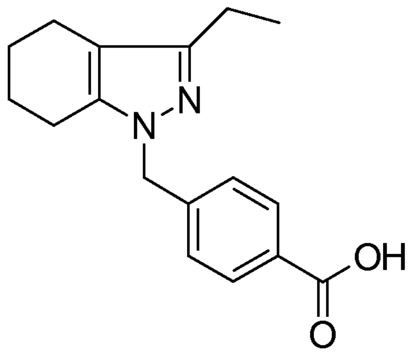

A solution of methyl 4-[(3-ethyl-4,5,6,7-tetrahydro-1H-indazol-1-yl)methyl]benzoate (80 mg, 0. 27 mmol), and lithium hydroxide monohydrate (45 mg, 1.07 mmol) in THF (3 mL), methanol ( 3 mL), and water (1.5 mL) was stirred at room temperature overnight. The solvent was evapor ated off under reduced pressure, and the residue was washed with ethyl acetate (10 mL). The aqueous layer was neutralized with 1N hydrochloric acid (2 mL), extracted twice with ethyl ac etate (10 mL). The organic layer was washed with brine (10 mL), dried over sodium sulfate, a nd concentrated under reduced pressure to give the titled compound (69 mg, yield 90%).
MS Calcd.: 284; MS Found: 285(M+H).

Example 238

3-Ethyl-1-[4-(pyrrolidin-1-ylcarbonyl)benzyl]-4,5,6,7-tetrahydro-1H-indazole

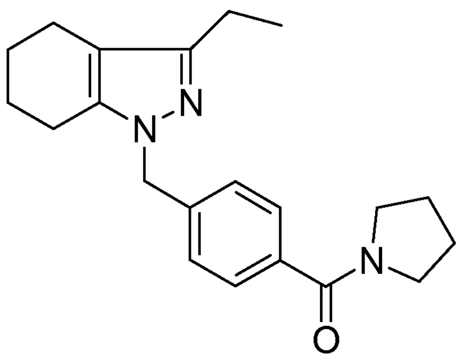

A mixed solution of 4-[(3-ethyl-4,5,6,7-tetrahydro-1H-indazol-1-yl)methyl]benzoic acid (69 mg, 0.24 mmol), pyrrolidine (0.1 mL), WSC (119 mg, 0.36 mmol), HOBt (49 mg, 0.36 mmol), and D IEA (0.2 mL, 1.20 mmol) in DCM (3 mL) was stirred at room temperature overnight. To the re action mixture, DCM (10 mL) was added, and the mixture was washed with 1N aqueous sodiu m hydroxide, 1N hydrochloric acid, and concentrated under reduced pressure. The obtained re sidue was purified by column chromatography on silica gel (DCM/methanol = 10:1) to give the titled compound (50 mg,

yield 62%) as a white solid.
MS Calcd.: 337; MS Found: 338(M+H).
$^1$ H NMR (400 MHz, $CDCl_3$ )δ ppm 1.24 (t, J = 7.6 Hz, 3H), 1.70-1.77 (m, 4H), 1.85-1.97 (m, 4H) , 2.39-2.46 (m, 4H), 2.60(q, J = 7.6 Hz, 2H), 3.40 (t, J = 6.8 Hz, 2H), 3.64 (t, J = 6.8 Hz, 2H), 5.1 9 (s, 2H), 7.09 (d, J = 8.4 Hz, 2H), 7.46-7.48 (m, 2H).

Example 239

Methyl 4-[(3-(1-methylethyl)-4,5,6,7-tetrahydro-1H-indazol-1-yl)methyl]benzoate

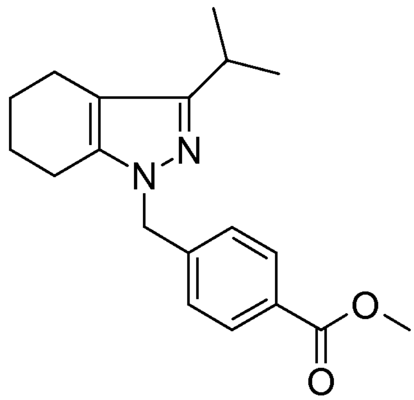

A solution of 2-(2-methylpropanoyl)cyclohexanone (433 mg, 2.58 mmol), methyl 4-(hydrazinyl methyl)benzoate (460 mg, 2.58 mmol), and p-toluenesulfonic acid (100 mg) in toluene (30 mL) was stirred at 90 °C for 3 hours. The solvent was evaporated off under reduced pressure, and t he residue was purified by preparative HPLC to give the titled compound (100 mg, yield 12%).
MS Calcd.: 312; MS Found: 313(M+H).
$^1$ H NMR (400 MHz, $CDCl_3$ ) δ ppm 1.31 (d, J = 7.2 Hz, 6H), 1.71-1.78 (m, 4H), 2.40 (t, J = 7.2 Hz, 2H), 2.52 (t, J = 7.2 Hz, 2H), 2.97-3.04 (m, 1H), 3.92 (s, 3H), 5.25 (s, 2H), 7.11-7.13 (m, 2H) , 7.98-8.00 (m, 2H).

Example 240

4-[(3-(1-Methylethyl)-4,5,6,7-tetrahydro-1H-indazol-1-yl)methyl]benzoic acid

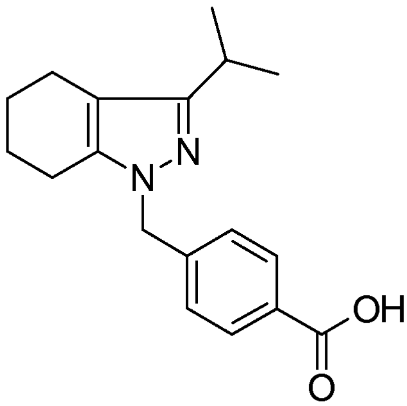

A mixture of methyl 4-[(3-(1-methylethyl)-4,5,6,7-tetrahydro-1H-indazol-1-yl)methyl]benzoate (100 mg, 0.32 mmol), and lithium hydroxide monohydrate (54 mg, 1.28 mmol) in THF (3 mL), methanol (3 mL), and water (1.5 mL) was stirred at

room temperature overnight. The solvent was evaporated off under reduced pressure, and the residue was washed with ethyl acetate (1 0 mL). The aqueous layer was neutralized with 1N hydrochloric acid (5 mL), extracted twice w ith ethyl acetate (10 mL). The organic layer was washed with brine (10 mL), dried over sodiu m sulfate, and concentrated under reduced pressure to give the titled compound (89 mg, yield 93%).
MS Calcd.: 298; MS Found: 299(M+H).

Example 241

3-(1-Methylethyl)-1-[4-(pyrrolidin-1-ylcarbonyl)benzyl]-4,5,6,7-tetrahydro-1H-indazole

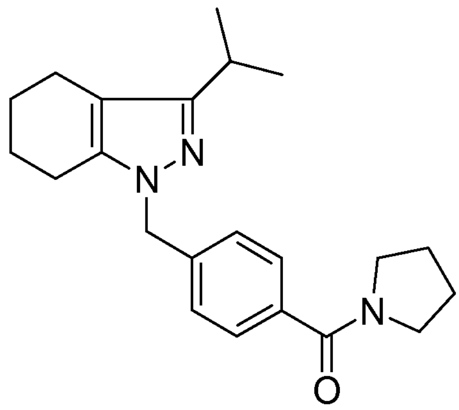

A mixed solution of 4-[(3-(1-methylethyl)-4,5,6,7-tetrahydro-1H-indazol-1-yl)methyl]benzoic ac id (89 mg, 0.30 mmol), pyrrolidine (0.1 mL), WSC (86 mg, 0.45 mmol), HOBt (61 mg, 0.45 mmo 1), and DIEA (0.2 mL, 1.50 mmol) in DCM (5 mL) was stirred at room temperature overnight. To the reaction mixture, was added DCM (10 mL), and the mixture was washed with 1N aque ous sodium hydroxide, and 1N hydrochloric acid, and concentrated under reduced pressure. T he obtained residue was purified by column chromatography on silica gel (DCM/methanol = 1 0:1) to give the titled compound (70 mg, yield 66%) as a white solid.
MS Calcd.: 351; MS Found: 352(M+H).
$^1$ H NMR (400 MHz, $CDCl_3$ ):ppm 1.28 (d, J = 6.4 Hz, 6H), 1.68-1.75 (m, 4H), 1.84-1.89 (m, 2H), 1.91-1.96 (m, 2H), 2.38 (t, J = 5.6 Hz, 2H), 2.48 (t, J = 5.6 Hz, 2H), 2.94-3.01 (m, 1H), 3.39 (t, J = 6.4 Hz, 2H), 3.63 (t, J = 6.8 Hz, 2H), 5.19 (s, 2H), 7.06 (d, J = 8.4 Hz, 2H), 7.43 (d, J = 8.4 Hz , 1H), 7.44 (d, J = 8.4 Hz, 1H).

Example 242

Methyl 4-[(3-butyl-4,5,6,7-tetrahydro-1H-indazol-1-yl)methyl]benzoate

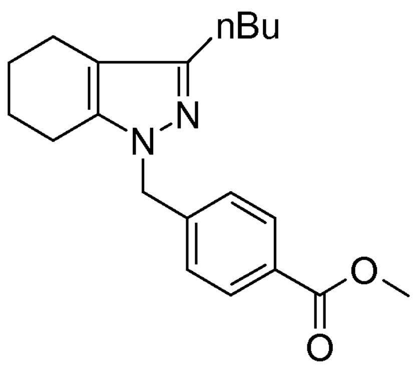

A solution of 2-pentanoylcyclohexanone (920 mg, 5.06 mmol), methyl 4-(hydrazinylmethyl)ben zoate (900 mg, 5.06 mmol), and p-toluenesulfonic acid (150 mg) in toluene (50 mL) was stirred at 90 °C for 3 hours. The solvent was evaporated off under reduced pressure, and the residue was purified by preparative HPLC to give the titled compound (250 mg, yield 15%).
MS Calcd.: 326; MS Found: 327(M+H).
$^1$ H NMR (400 MHz, $CDCl_3$) δ ppm 0.93 (t, J = 7.2 Hz, 3H), 1.34-1.44 (m, 2H), 1.55-1.77 (m, 6H ), 2.36-2.44 (m, 4H), 2.53-2.58 (m, 2H), 3.89 (s, 3H), 5.21 (s, 2H), 7.08-7.11 (m, 2H), 7.94-7.98 ( m, 2H).

Example 243

4-[(3-Butyl-4,5,6,7-tetrahydro-1H-indazol-1-yl)methyl]benzoic acid

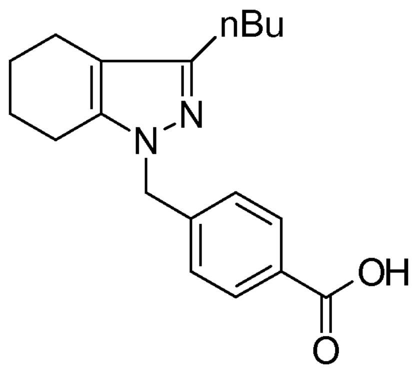

A solution of methyl 4-[(3-butyl-4,5,6,7-tetrahydro-1H-indazol-1-yl)methyl]benzoate (250 mg, 0.77 mmol), and lithium hydroxide monohydrate (129 mg, 3.07 mmol) in THF (6 mL), methan ol (6 mL), and water (3 mL) was stirred at room temperature overnight. The solvent was evap orated off under reduced pressure, and the residue was washed with ethyl acetate (20 mL). Th e aqueous layer was neutralized with 1N hydrochloric acid (10 mL), extracted twice with ethyl acetate (20 mL). The organic layer was washed with brine (20 mL), dried over sodium sulfate, and concentrated under reduced pressure to give the titled compound (200 mg, yield 83%).
MS Calcd.: 312; MS Found: 313(M+H).
$^1$ H NMR (400 MHz, $CDCl_3$ ) δ ppm 0.98 (t, J = 7.2 Hz, 3H), 1.39-1.45 (m, 2H), 1.74-1.85 (m, 6H ), 2.48-2.53 (m, 4H), 2.76-2.80 (m, 2H), 5.55 (s, 2H), 7.34-7.36 (m, 2H), 8.00-8.02 (m, 2H).

Example 244

3-Butyl-1-[4-(pyrrolidin-1-ylcarbonyl)benzyl]-4,5,6,7-tetrahydro-1H-indazole

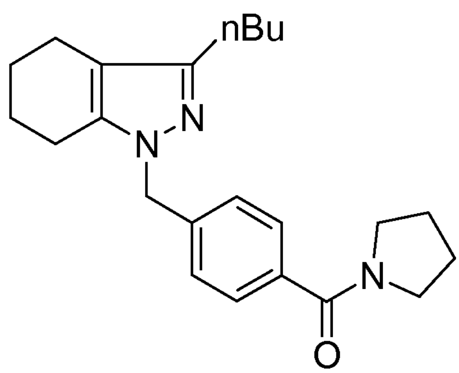

A solution of 4-[(3-butyl-4,5,6,7-tetrahydro-1H-indazol-1-yl)methyl]benzoic acid (100 mg, 0.32 mmol), pyrrolidine (0.1 mL), WSC (92 mg, 0.48 mmol), HOBt (65 mg, 0.48 mmol), and DIEA (0 .26 mL, 1.60 mmol) in DCM (5 mL) was stirred at room temperature overnight. To the reactio n mixture, was added DCM (10 mL), the mixture was washed with 1N aqueous sodium hydrox ide, and 1N hydrochloric acid, and concentrated under reduced pressure. The obtained residue was purified by column chromatography on silica gel (DCM/methanol = 10:1) to give the titled compound (80 mg, yield 68%) as a white solid.
MS Calcd.: 365; MS Found: 366(M+H).
$^1$ H NMR (400 MHz, $CDCl_3$ )δ ppm 0.93 (t, J = 7.2 Hz, 3H), 1.33-1.43 (m, 2H), 1.57-1.64 (m, 2H) , 1.67-1.77 (m, 4H), 1.82-1.87 (m, 2H), 1.93-1.98 (m, 2H), 2.37-2.44 (m, 4H), 2.54-2.58 (m, 2H), 3.39 (t, J = 6.8 Hz, 2H), 3.63 (t, J = 6.8 Hz, 2H), 5.18 (s, 2H), 7.07 (d, J = 8.4 Hz, 2H), 7.44 (d, J = 8.0 Hz, 2H).

Example 245

1-[4-(Pyrrolidin-1-ylcarbonyl)benzyl]-3-(2,2,2-trifluoroethyl)-4,5,6,7-tetrahydro-1H-indazole

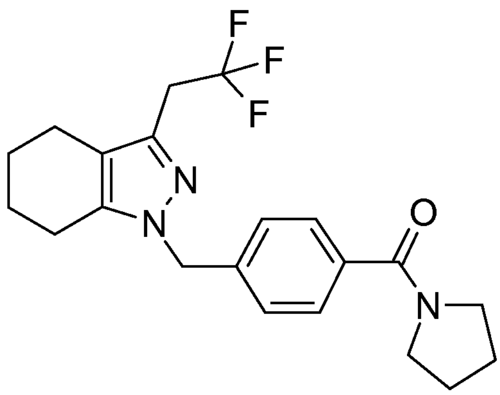

A solution of 3-(2,2,2-trifluoroethyl)-4,5,6,7-tetrahydro-1H-indazole (200 mg, 0.98 mmol), [4-(b romomethy)phenyl](pyrrolidin-1-yl)methanone (392 mg, 1.47 mmol) and potassium carbonate (676 mg, 4.90 mmol) in DMF (10 mL) was stirred at room temperature overnight. To the react ion mixture, was added water (30 mL), and the mixture was extracted with ethyl acetate (20 mL). The organic layer was concentrated under reduced pressure, and the residue was purifie d by preparative HPLC to give the titled compound (15 mg, yield 4%) as a colorless oil.
MS Calcd.: 391; MS Found: 392(M+H).
$^1$ H NMR (400 MHz, $CDCl_3$ )δ ppm 1.67-1.78 (m, 4H), 1.84-1.89 (m, 2H), 1.92-1.97 (m, 2H), 2.4 1-2.47 (m, 4H), 3.34-3.42 (m, 4H), 3.63 (t, J = 6.8 Hz, 2H), 5.22 (s, 2H), 7.08 (d, J = 8.0 Hz, 2H), 7.46 (d, J = 8.0 Hz, 2H).

Example 246

Methyl 1-[4-(pyrrolidin-1-ylcarbonyl)benzyl]-4,5,6,7-tetrahydro-1H-indazole-3-carboxylate

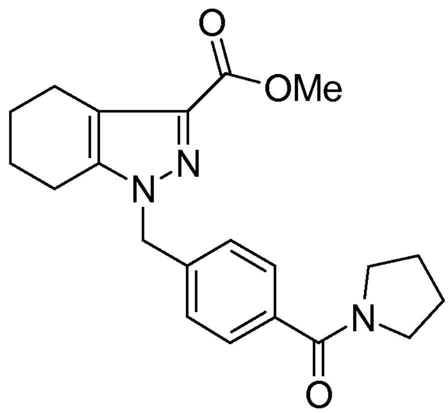

A mixture of methyl 4,5,6,7-tetrahydro-1H-indazole-3-carboxylate (1.72 g, 9.56 mmol), [4-(bro momethy)phenyl](pyrrolidin-1-yl)methanone (2.04 g, 11.47 mmol), potassium carbonate (3.96 g, 28.68 mmol), and DMF (100 mL) was stirred at 100 °C for 2 hours. To the reaction mixture, was added water (300 mL), and the mixture was extracted with ethyl acetate (200 mL). The or ganic layer was concentrated under reduced pressure, and the residue was purified by column chromatography on silica gel (EA/PE = 1:1) to give the titled compound (620 mg, yield 17%) a s a yellow oily substance.
MS Calcd.: 367; MS Found: 368(M+H).
$^1$ H NMR (300 MHz, $CDCl_3$ )δ ppm 1.68-1.77 (m, 4H), 1.85-1.98 (m, 4H), 2.41 (t, J = 5.7 Hz, 2H) , 2.76 (t, J = 5.7 Hz, 2H), 3.39 (t, J = 6.6 Hz, 2H), 3.63 (t, J = 6.9 Hz, 2H), 3.92 (s, 3H), 5.33 (s, 2 H), 7.11-7.14 (m, 2H), 7.46(dd, J = 6.6, 1.8 Hz, 2H).

Example 247

{1-[4-(Pyrrolidin-1-ylmethyl)benzyl]-4,5,6,7-tetrahydro-1H-indazol-3-yl}methanol

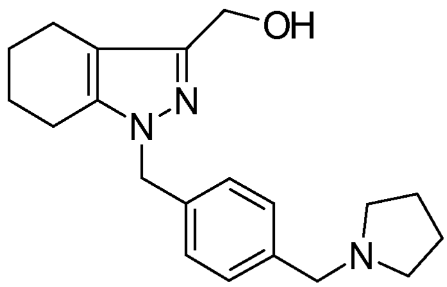

A solution of methyl 1-[4-(pyrrolidin-1-ylcarbonyl)benzyl]-4,5,6,7-tetrahydro-1H-indazole-3-car boxylate (350 mg, 0.95 mmol) in THF (10 mL) was cooled to 0 °C, lithium aluminium hydride ( 80 mg, 1.90 mmol) was added thereto, and the mixture was stirred at 0 °C for 2 hours. The rea ction mixture was added to sodium sulfate decahydrate (612 mg) at 0 °C, and then the mixtur e was stirred at room temperature for 2 hours. The insoluble material was removed by filtrati on, and the filtrate was concentrated under reduced pressure. The residue was purified by pre parative HPLC to give the titled compound (65 mg, yield 21%) as a colorless oil.
MS Calcd.: 325; MS Found: 326(M+H).
$^1$ H NMR (400 MHz, $CDCl_3$ )δ ppm 1.68-1.78 (m, 8H), 2.45-2.50 (m, 8H), 3.58 (s, 2H), 4.63 (s, 2 H), 5.16 (s, 2H), 7.05 (d, J = 8.0 Hz, 2H), 7.26 (d, J = 8.0 Hz, 2H).

Example 248

2-[6-Acetyl-3-(trifluoromethyl)-4,5,6,7-tetrahydro-1H-pyrazolo[3,4-c]pyridin-1-yl]-N-(5-chloro-2-methoxyphenyl)acetamide

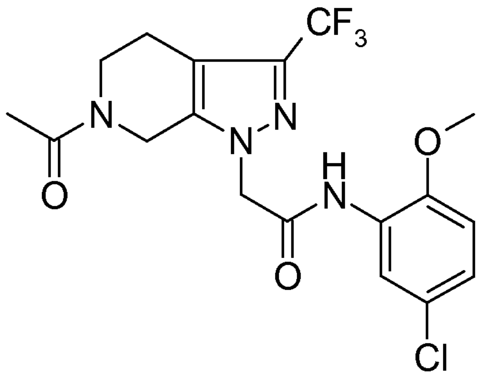

To a stirred solution in an ice bath of N-(5-chloro-2-methoxyphenyl)-2-[3-(trifluoromethyl)-4,5, 6,7-tetrahydro-1H-pyrazolo [3,4-c]pyridin-1-yl]acetamide (100 mg, 0.26 mmol), and triethylami ne (0.5 mL) in dichloromethane (20 mL), was added acetyl chloride (40 mg, 0.52 mmol), and th e mixture was stirred for 2 hours at room temperature. The reaction mixture was concentrate d under reduced pressure. The residue was purified by column chromatography on silica gel ( PE/EA = 3:1) to give the titled compound (53 mg, yield 47%) as a yellow solid.
MS Calcd.: 430, MS Found: 431 (M+H).
$^1$ H NMR (400 MHz, $CDCl_3$ ) δ ppm 2.22 (s, 3H), 2.81 (t, J = 5.6 Hz, 2H), 3.71 (t, J = 5.6 Hz, 2H ), 3.84 (s, 3H), 4.77 (s, 2H), 4.90 (s, 2H), 6.77 (d, J = 8.8 Hz, 1H), 7.03 (dd, J = 8.8, 2.4 Hz, 1H), 8.37 (d, J = 2.4 Hz, 1H), 8.75 (br s, 1H).

Example 249

5,7-Bis(trifluoromethyl)-3-(5-{[3-(trifluoromethyl)-4,5,6,7-tetrahydro-1H-indazol-1-yl]methyl}-1,2,4-oxadiazol-3-yl)pyrazolo[1,5-a]pyrimidine

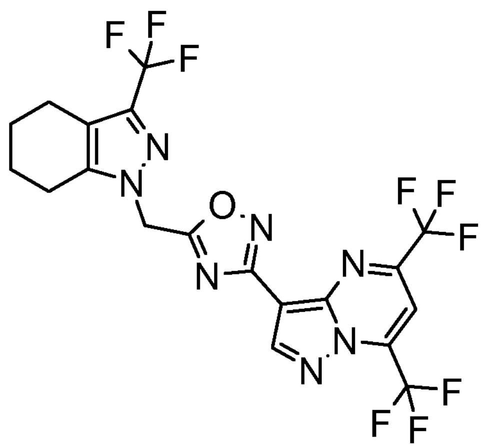

A 0.2M solution of [3-(trifluoromethyl)-4,5,6,7-tetrahydro-1H-indazol-1-yl]acetic acid in DMF ( 500 μl, 0.1 mmol), and a 0.2M solution of N'-hydroxy-5,7-bis(trifluoromethyl)pyrazolo[1,5-a]py rimidine-3-carboximidamide in DMF (500 μl, 0.1 mmol) were mixed. To the mixture, was adde d a mixed 0.2 M solution of 1-ethyl-3-(3-dimethylaminopropyl)carbodiimide hydrochloride and 1-hydroxybenzotriazole monohydrate in DMF (1000 μl, 0.2 mmol), and the mixture was stirre d for 3 hours at room temperature. The reaction solution was diluted with ethyl acetate, wash ed with 5% sodium hydrogen carbonate aqueous solution, and concentrated under reduced pre ssure. The obtained oily substance was dissolved in pyridine (3 ml), and stirred at 115 °C for 1 5 hours while heating. The reaction mixture was concentrated under reduced pressure. The ob tained crude product was preparative HPLC to give the titled compound (17 mg) (yield 32%).
MS (ESI+) :526 (M+H)
$^1$ H NMR (400 MHz, $CDCl_3$) δ ppm 1.75 - 1.83 (2 H, m), 1.85 - 1.93 (2 H, m), 2.60 - 2.65 (2 H, m ), 2.71 - 2.76 (2 H, m), 5.60 (2 H, s), 7.63 (1 H, s), 8.93 (1 H, s).

Example 250

5-Phenyl-7-(trifluoromethyl)-3-(5-[3-(trifluoromethyl)-4,5,6,7-tetrahydro-1H-indazol-1-yl]met hyl}-1,2,4-oxadiazol-3-yl) pyrazolo[1,5-a]pyrimidine

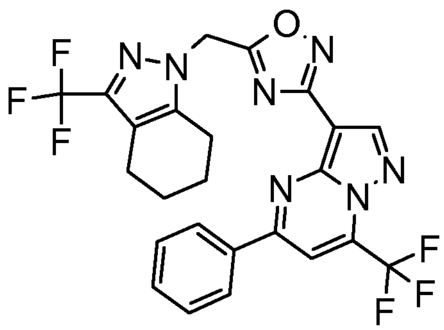

Using N'-hydroxy-5-phenyl-7-(trifluoromethyl)pyrazolo[1,5-a]pyrimidine-3-carboximidamide, t he titled compound was obtained in the same manner as in Example 249.
Yield: 25.0 mg
MS (ESI+) :534 (M+H)

Example 251

5-tert-Butyl-7-(trifluoromethyl)-3-(5-{[3-(trifluoromethyl)-4,5,6,7-tetrahydro-1H-indazol-1-yl]m ethyl}-1,2,4-oxadiazol-3-yl)pyrazolo[1,5-a]pyrimidine

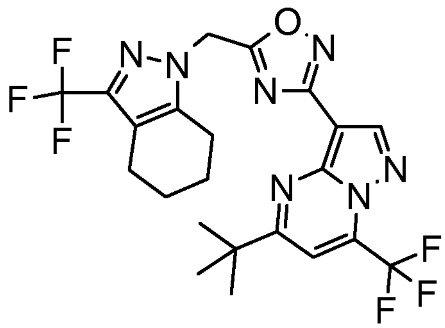

Using 5-tert-butyl-N'-hydroxy-7-(trifluoromethyl)pyrazolo[1,5-a]pyrimidine-3-carboximidamid e, the titled compound was obtained in the same manner as in Example 249.
Yield: 13.0 mg
MS (ESI+) :514 (M+H)

Example 252

1-{[3-(1H-Indol-3-yl)-1,2,4-oxadiazol-5-yl]methyl}-3-(trifluoromethyl)-4,5,6,7-tetrahydro-1H-in dazole

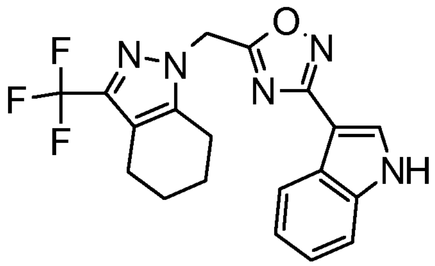

Using N'-hydroxy-1H-indole-3-carboximidamide, the titled compound was obtained in the sam e manner as in Example 249.
Yield: 8.3 mg
MS (ESI+) :388(M+H)

Example 253

1-{[3-(1-Benzothlophen-3-yl)-1,2,4-oxadiazol-5-yllmethyl}-3-(trifluoromethyl)-4,5,6,7-tetrahydr o-1H-indazole

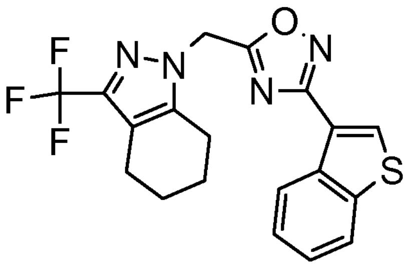

Using N'-hydroxy-1-benzothiophene-3-carboximidamide, the titled compound was obtained in the same manner as in Example 249.
Yield: 22.7 mg
MS (ESI+) :405(M+H)
$^1$ H NMR (400 MHz, $CDCl_3$) δ ppm 1.75 - 1.82 (2 H, m), 1.85 - 1.93 (2 H, m), 2.60 - 2.66 (2 H, m ), 2.68 - 2.73 (2 H, m), 5.57 (2 H, s), 7.42 - 7.55 (2 H, m), 7.91 (1 H, d, J=7.9 Hz), 8.35 (1 H, s), 8 .61 (1 H, d, J=7.9 Hz)

Example 254

1-{[3-(1H-Pyrrolo[2,3-b]pyridin-3-yl)-1,2,4-oxadiazol-5-yl]methyl}-3-(trifluoromethyl)-4,5,6,7-te trahydro-1H-indazole

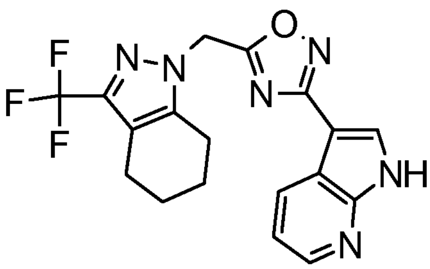

Using N'-hydroxy-1H-pyrrolo[2,3-b]pyrimidine-3-carboximidamide, the titled compound was o btained in the same manner as in Example 249.

Yield: 4.7 mg
MS (ESI+) :389(M+H)

Example 255

1-{[3-(1H-indol-2-yl)-1,2,4-oxadiazol-5-yl]methyl}-3-(trifluoromethyl)-4,5,6,7-tetrahydro-1H-ind azole

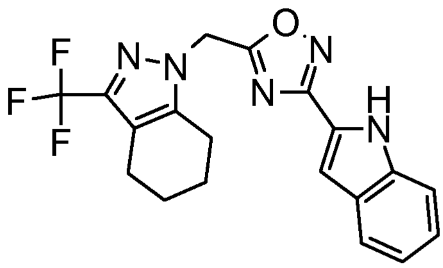

Using N'-hydroxy-1H-indole-2-carboximidamide, the titled compound was obtained in the sam e manner as in Example 249.
Yield: 8.2 mg
MS (ESI+) :388(M+H)

Example 256

1-[(3-Phenyl-1,2,4-oxadiazol-5-yl)methyl]-3-(trifluoromethyl)-4,5,6,7-tetrahydro-1H-indazole

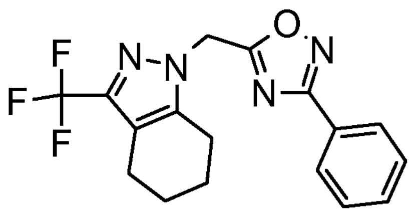

Using N'-hydroxybenzenecarboximidamide, the titled compound was obtained in the same ma nner as in Example 249.
Yield: 18.7 mg
MS (ESI+) :349(M+H)
$^1$ H NMR (400 MHz, $CDCl_3$) δ ppm 1.74 - 1.82 (2 H, m), 1.84 - 1.92 (2 H, m), 2.59 - 2.65 (2 H, m ), 2.66 - 2.70 (2 H, m), 5.54 (2 H, s), 7.45 - 7.55 (3 H, m), 8.03 - 8.08 (2 H, m).

Example 257

1-{[3-(3-Fluorophenyl)-1,2,4-oxadiazol-5-yl]methyl}-3-(trifluoromethyl)-4,5,6,7-tetrahydro-1H-i ndazole

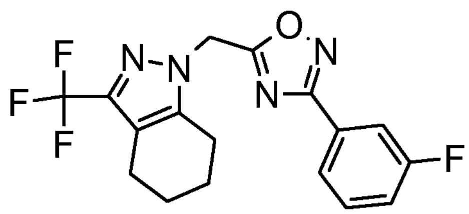

Using 3-fluoro-N'-hydroxybenzenecarboximidamide, the titled compound was obtained in the s ame manner as in Example 249.
Yield: 16.5 mg
MS (ESI+) :367(M+H)

Example 258

N,N-Dimethyl-4-(5-{[3-(trifluoromethyl)-4,5,6,7-tetrahydro-1H-indazol-1-yl]methyl}-1,2,4-oxad iazol-3-yl)aniline

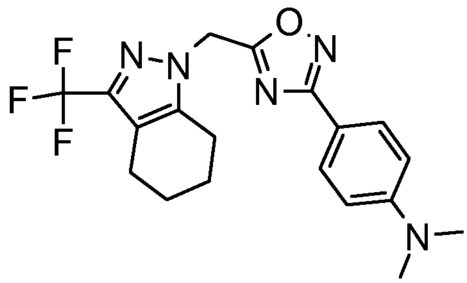

Using 4-(dimethylamino)-N'-hydroxybenzenecarboximidamide, the titled compound was obtai ned in the same manner as in Example 249.
Yield: 7.0 mg
MS (ESI+) :392(M+H)

Example 259

1-[(3-Biphenyl-4-yl-1,2,4-oxadiazol-5-yl)methyl]-3-(trifluoromethyl)-4,5,6,7-tetrahydro-1H-ind azole

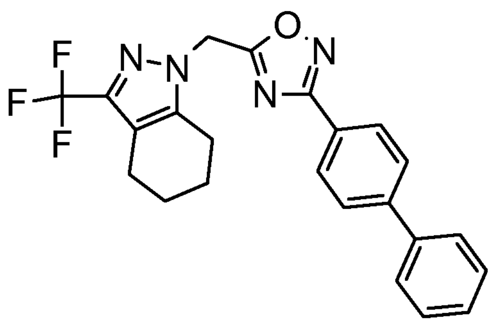

Using N'-hydroxybiphenyl-4-carboximidamide, the titled compound was obtained in the same manner as in Example 249.
Yield: 21.6 mg
MS (ESI+) :425(M+H)

Example 260

1-({3-[(4-Chlorophenoxy)methyl]-1,2,4-oxadiazol-5-yl}methyl)-3-(trifluoromethyl)-4,5,6,7-tetra hydro-1H-indazole

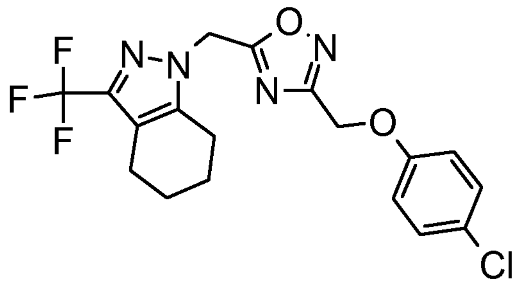

Using 2-(4-chlorophenoxy)-N'-hydroxyethanimidamide, the titled compound was obtained in t he same manner as in Example 249.
Yield: 20.7 mg
MS (ESI+) :413(M+H)

Example 261

1-[(3-Pyridin-2-yl-1,2,4-oxadiazol-5-yl)methyl]-3-(trifluoromethyl)-4,5,6,7-tetrahydro-1H-indaz ole

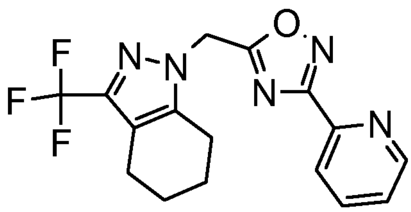

Using N'-hydroxypyridine-2-carboximidamide, the titled compound was obtained in the same manner as in Example 249.
Yield: 23.4 mg
MS (ESI+) :350(M+H)

Example 262

1-[(3-Pyridin-3-yl-1,2,4-oxadiazol-5-yl)methyl]-3-(trifluoromethyl)-4,5,6,7-tetrahydro-1H-indaz ole

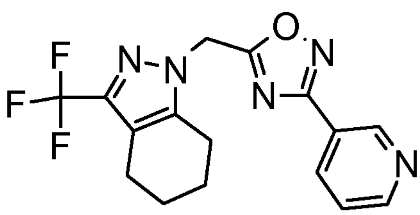

Using N'-hydroxypyrimidine-3-carboximidamide, the titled compound was obtained in the sa me manner as in Example

249.
Yield: 22.8 mg
MS (ESI+) :350(M+H)

Example 263

1-[(3-Pyridin-4-yl-1,2,4-oxadiazol-5-yl)methyl]-3-(trifluoromethyl)-4,5,6,7-tetrahydro-1H-indaz ole

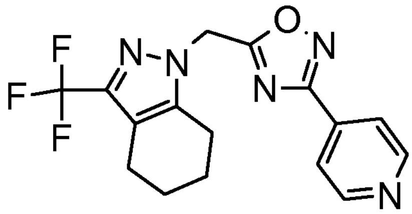

Using N'-hydroxypyrimidine-4-carboximidamide, the titled compound was obtained in the sa me manner as in Example 249.
Yield: 22.2 mg
MS (ESI+) :350(M+H)

Example 264

1-[(3-Pyrimidin-2-yl-1,2,4-oxadiazol-5-yl)methyl]-3-(trifluoromethyl)-4,5,6,7-tetrahydro-1H-ind azole

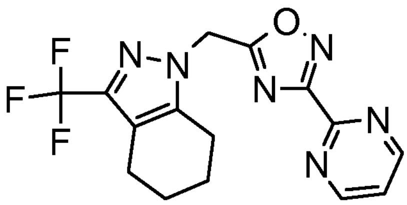

Using N'-hydroxypyrimidine-2-carboximidamide, the titled compound was obtained in the sa me manner as in Example 249.
Yield: 19.6 mg
MS (ESI+) :351(M+H)
$^1$ H NMR (400 MHz, CDCl3) δ ppm 1.73 - 1.81 (2 H, m), 1.82 - 1.90 (2 H, m), 2.58 - 2.68 (4 H, m ), 5.63 (2 H, s), 7.49 (1 H, t, J=4.9 Hz), 8.98 (2 H, d, J=4.9 Hz)

Example 265

1-[(3-Thiophen-2-yl-1,2,4-oxadiazol-5-yl)methyl]-3-(trifluoromethyl)-4,5,6,7-tetrahydro-1H-ind azole

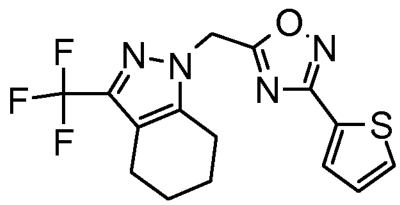

Using N'-hydroxythiophene-2-carboximidamide, the titled compound was obtained in the sam e manner as in Example 249.
Yield: 23.2 mg
MS (ESI+) :355(M+H)
$^1$H NMR (400 MHz, CDCl3) δ ppm 1.74 - 1.82 (2 H, m), 1.84 - 1.91 (2 H, m), 2.60 - 2.64 (2 H, m ), 2.64 - 2.69 (2 H, m), 5.52 (2 H, s), 7.14 - 7.17 (1 H, m), 7.52 (1 H, d, J=4.9 Hz), 7.79 (1 H, d, J =3.8 Hz)

Example 266

1-({3-[5-Methyl-7-(trifluoromethyl)pyrazolo[1,5-a]pyrimidin-3-yl]-l,2,4-oxadiazol-5-yl}methyl)-3-(trifluoromethyl)-4,5,6,7-tetrahydro-lH-pyrazolo[4,3-c]pyridine

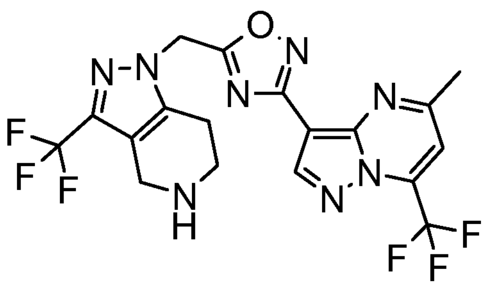

Using [3-(trifluoromethyl)-4,5,6,7-tetrahydro-1H-pyrazolo[4,3-c]pyridin-1-yl]acetic acid, and N '-hydroxy-5-methyl-7-(trifluoromethyl)pyrazolo[1,5-a]pyrimidine-3-carboximidamide, the titled compound was obtained in the same manner as in Example 249.
Yield: 21.0 mg
MS (ESI+) :473(M+H)

Example 267

1-({3-[5-Methyl-7-(trifluoromethyl)pyrazolo[1,5-a]pyrimidin-3-yl]-1,2,4-oxadiazol-5-yl}methyl)-3-(trifluoromethyl)-4,5,6,7-tetrahydro-1H-pyrazolo[3,4-c]pyridine

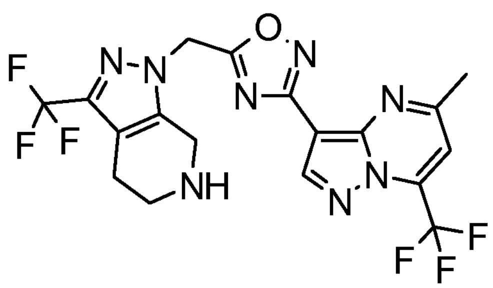

Using [3-(trifluoromethyl)-4,5,6,7-tetrahydro-1H-pyrazolo[3,4-c]pyridin-1-yl]acetic acid, and N '-hydroxy-5-methyl-7-(trifluoromethyl)pyrazolo[1,5-a]pyrimidine-3-carboximidamide, the titled compound was obtained in the same manner as in Example 249.
Yield: 24.2 mg
MS (ESI+) :473(M+H)
$^{1}$ H NMR (400 MHz, $CDCl_3$) δ ppm 2.64 - 2.70 (2 H, m), 2.84 (3 H, s), 3.05 - 3.10 (2 H, m), 4.04 ( 2 H, br. s.), 5.56 (2 H, s), 7.23 (1 H, s), 8.71 (1 H, s)

Example 268

1-({3-[5-Methyl-7-(trifluoromethyl)pyrazolo[1,5-a]pyrimidin-3-yl]-1,2,4-oxadiazol-5-yl}methyl)-3-(trifluoromethyl)-1,4,6,7-tetrahydropyrano[4,3-c]pyrazole

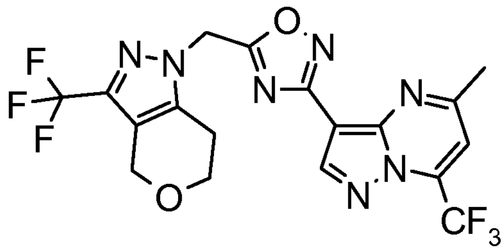

Using [3-(trifluoromethyl)-6,7-dihydropyrano[4,3-c]pyrazole-1(4H)-yl]acetic acid, and N'-hydro xy-5-methyl-7-(trifluoromethyl)pyrazolo[1,5-a]pyrimidine-3-carboximidamide, the titled compo und was obtained in the same manner as in Example 249.
Yield: 18.5 mg
MS (ESI+) :474(M+H)
$^{1}$ H NMR (400 MHz, $CDCl_3$) δ ppm 2.84 (3 H, s), 2.84 - 2.89 (2 H, m), 3.96 - 4.01 (2 H, m), 4.75 ( 2 H, br. s.), 5.63 (2 H, s), 7.23 (1 H, s), 8.71 (1 H, s).

Example 269

1-[(3-Pyrazin-2-yl-1,2,4-oxadiazol-5-yl)methyl]-3-(trifluoromethyl)-1,4,6,7-tetrahydropyrano[4, 3-c]pyrazole

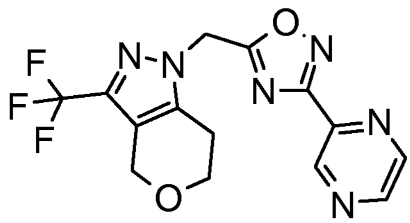

Using [3-(trifluoromethyl)-6,7-dihydropyrano[4,3-c]pyrazole-1(4H)-yl]acetic acid, and pyrazin e-2-carboximidamide, the titled compound was obtained in the same manner as in Example 24 9.
Yield: 16.0 mg
MS (ESI+) :353(M+H)
$^{1}$ H NMR (400 MHz, $CDCl_3$) δ ppm 2.83 - 2.88 (2 H, m), 3.97 - 4.01 (2 H, m), 4.74 (2 H, br. s.), 5 .66 (2 H, s), 8.75 - 8.79 (2 H, m), 9.35 (1 H, s).

Example 270

1-{[3-(5,7-Dimethylpyrazolo[1,5-a]pyrimidin-3-yl)-1,2,4-oxadiazol-5-yl]methyl}-3-(trifluoromet hyl)-1,4,6,7-tetrahydropyrano[4,3-c]pyrazole

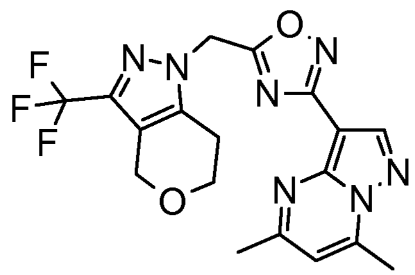

Using [3-(trifluoromethyl)-6,7-dihydropyrano[4,3-c]pyrazole-1(4H)-yl]acetic acid, and N'-hydro xy-5,7-dimethylpyrazolo [1,5-a]pyrimidine-3-carboximidamide, the titled compound was obtain ed in the same manner as in Example 249.
Yield: 18.4 mg
MS (ESI+) :420(M+H)
$^1$ H NMR (400 MHz, $CDCl_3$) δ ppm 2.71 (3 H, s), 2.81 (3 H, s), 2.84 - 2.87 (2 H, m), 3.96 - 4.00 ( 2 H, m), 4.74 (2 H, br. s.), 5.61 (2 H, s), 6.77 (1 H, s), 8.59 (1 H, s).

Example 271

5,7-Dimethyl-3-(5-{[3-(trifluoromethyl)-5,6-dihydrocyclopenta[c]pyrazole-1(4H)-yl]methyl}-1,2, 4-oxadiazol-3-yl)pyrazolo[1,5-a]pyrimidine

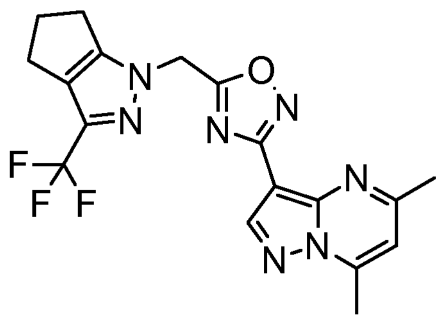

[3-(Trifluoromethyl)-5,6-dihydrocyclopenta[c]pyrazol-1(4H)-yl]acetic acid (103 mg, 0.5 mmol), and N'-hydroxy-5,7-dimethylpyrazolo[1,5-a]pyrimidine-3-carboximidamide (118 mg, 0.5 mmol) was dissolved in DMF (7 ml), 1-ethyl-3-(3-dimethylaminopropyl)carbodiimide hydrochloride ( 192 mg, 1 mmol), and 1-hydroxybenzotriazole monohydrate (135 mg, 1 mmol) was added there to, and the mixture was stirred for 3 hours at room temperature. The reaction solution was dil uted with ethyl acetate, washed with 5% sodium hydrogen carbonate aqueous solution, and co ncentrated under reduced pressure. The obtained oily substance was dissolved in pyridine (10 ml), stirred at 115 °C for 15 hours while heating. The reaction mixture was concentrated unde r reduced pressure. The obtained crude product was purified by silica gel chromatography(dev eloping solvent: hexane/ethyl acetate (70 : 30 - 0 : 100)) to give the titled compound (54 mg) as a colorless solid. (yield 27%). MS (ESI+) :404(M+H)
$^1$ H NMR (400 MHz, $CDCl_3$) δ ppm 2.60 - 2.67 (2 H, m), 2.71 (3 H, s), 2.73 - 2.80 (4 H, m), 2.81 ( 3 H, s), 5.57 (2 H, s), 6.77 (1 H, s), 8.61 (1 H, s).

Example 272

1-[(5-Thiophen-2-yl-1,3,4-oxadiazol-2-yl)methyl]-3-(trifluoromethyl)-4,5,6,7-tetrahydro-1H-ind azole

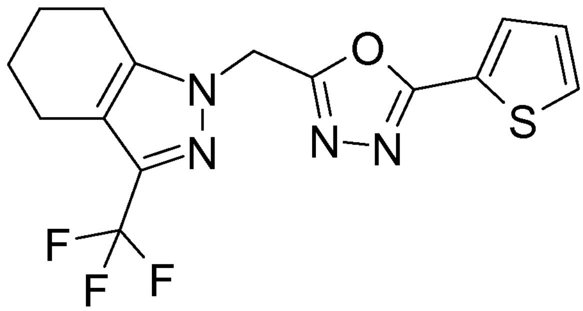

[3-(Trifluoromethyl)-4,5,6,7-tetrahydro-1H-indazol-1-yl]acetic acid (25 mg, 0.1 mmol), and thio phene-2-carbohydrazide (14 mg, 0.1 mmol) were dissolved in acetonitrile (3 ml). To the solutio n, were added 2-chloro-1,3-dimethylimidazolium chloride (100 mg, 0.6 mmol), and triethylami ne (140 mg, 1.4 mmol) at room temperature. The mixture was heated for 10 minutes to 80 °C, and further stirred for 4 hours. The reaction mixture was concentrated under reduced pressur e. The obtained oily substance was dissolved in ethyl acetate, washed with 5% sodium hydroge n carbonate aqueous solution. The organic layer was concentrated under reduced pressure. Th e obtained crude product was purified by preparative HPLC to give the titled compound (9 mg ) as a colorless solid (yield 25%).
MS (ESI+) :355 (M+H)
$^1$ H NMR (400 MHz, $CDCl_3$) δ ppm 1.71 - 1.79 (2 H, m), 1.82 - 1.89 (2 H, m), 2.56 - 2.62 (2 H, m ), 2.70 (2 H, t, J=6.1 Hz), 5.51 (2 H, s), 7.15 - 7.19 (1 H, m), 7.57 (1 H, d, J=4.9 Hz), 7.76 (1 H, d , J=3.8 Hz).

Example 273

1-[(5-Thiophen-2-yl-1,3,4-oxadiazol-2-yl)methyl]-3-(trifluoromethyl)-1,4,5,6-tetrahydrocyclope nta[c]pyrazole

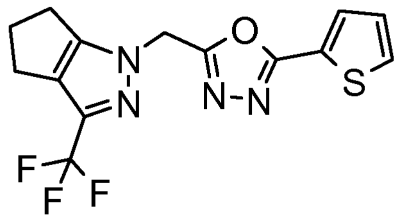

Using [3-(trifluoromethyl)-5,6-dihydrocyclopenta[c]pyrazole-1(4H)-yl]acetic acid, and thiophen e-2-carbohydrazide, the titled compound was obtained in the same manner as in Example 272.
Yield: 11.7 mg
MS (ESI+) :341 (M+H)
$^1$ H NMR (400 MHz, $CDCl_3$) δ ppm 2.58 - 2.65 (2 H, m), 2.70 - 2.79 (4 H, m), 5.51 (2 H, s), 7.18 ( 1 H, dd, J=4.9, 3.8 Hz), 7.59 (1 H, dd, J=5.1, 0.9 Hz), 7.77 (1 H, dd, J=3.8, 0.9 Hz)

Example 274

1-[(5-Thiophen-2-yl-1,3,4-oxadiazol-2-yl)methyl]-3-(trifluoromethyl)-1,4,6,7-tetrahydropyrano[ 4,3-c]pyrazole

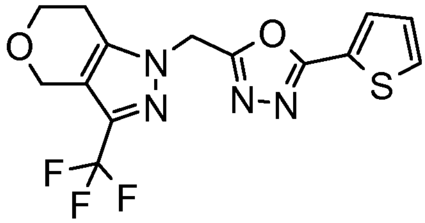

Using [3-(trifluoromethyl)-6,7-dihydropyrano[4,3-c]pyrazole-1(4H)-yl]acetic acid, the titled co mpound was obtained in the same manner as in Example 272.
Yield: 7.2 mg
MS (ESI+) :357 (M+H)
$^1$ H NMR (400 MHz, $CDCl_3$) δ ppm 2.83 - 2.88 (2 H, m), 3.94 - 3.98 (2 H, m), 4.72 (2 H, br. s.), 5 .56 (2 H, s), 7.18 (1 H, dd, J=5.0, 3.9 Hz), 7.59 (1 H, dd, J=5.1, 0.9 Hz), 7.77 (1 H, dd, J=3.8, 1.1 Hz).

Example 275

5,7-Dimethyl-3-(5-{[3-(trifluoromethyl)-5,6-dihydrocyclopenta[c]pyrazole-1(4H)-yl]methyl}-1,3, 4-oxadiazol-2-yl)pyrazolo[1,5-a]pyrimidine

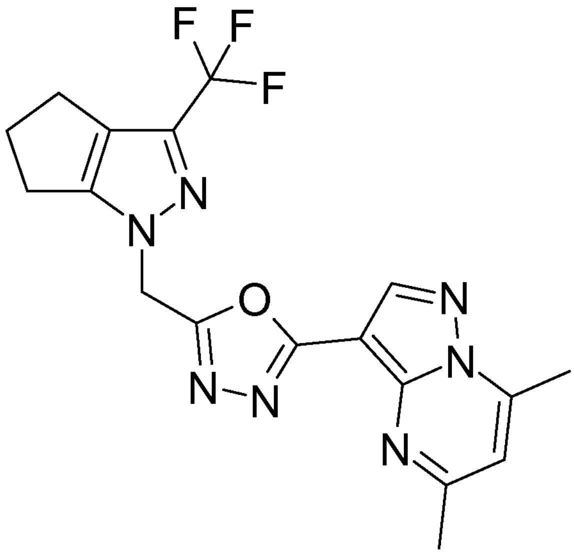

[3-(Trifluoromethyl)-5,6-dihydrocyclopenta[c]pyrazol-1(4H)-yl]acetic acid (117 mg, 0.5 mmol), and 5, 7-dimethylpyrazolo[1,5-a]pyrimidine-3-carbohydrazide (105 mg, 0.5 mmol) of Reference Example 68, were dissolved in acetonitrile (10 ml), 2-chloro-1,3-dimethylimidazolium chloride (500 mg, 3 mmol), and triethylamine (707 mg, 7 mmol) was stirred at room temperature for 1 0 minutes, and then heated to reflux for 4 hours. The reaction mixture was concentrated unde r reduced pressure to give an oily substance. The oily substance was dissolved in ethyl acetate , and washed with 5% potassium hydrogen sulfate aqueous solution, and 5% sodium hydrogen carbonate aqueous solution. The organic layer was dried over anhydrous magnesium sulfate, and concentrated under reduced pressure to give a crude product. The obtained crude product was purified by silica gel chromatography [developing solvent: hexane/ethyl acetate (70:30-0:1 00)] to give the titled compound (55 mg) as a colorless solid (yield 27%).
MS (ESI+) :404 (M+H)
$^1$ H NMR (400 MHz, $CDCl_3$) δ ppm 2.55 - 2.65 (2 H, m), 2.65-2.73 (2 H, m), 2.71 (3 H, s), 2.75-2. 85 (2 H, m), 2.81 (3 H, s), 5.57 (2 H, s), 6.80 (1 H, s), 8.60 (1 H, s)

Example 276

5,7-Dimethyl-3-(5-{[3-(trifluoromethyl)-4,5,6,7-tetrahydro-1H-indazol-1-yl]methyl}-1,2,4-oxadi azol-3-yl)pyrazolo[1,5-a] pyrimidine

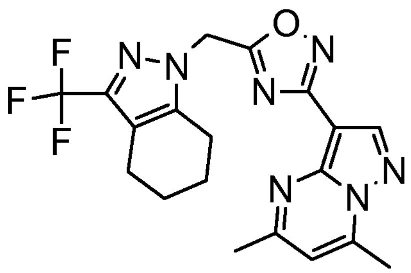

A 0.2M solution of [3-(trifluoromethyl)-4,5,6,7-tetrahydro-1H-indazol-1-yl]acetic acid in DMF ( 500 μl, 0.1 mmol), and a 0.2M solution of N'-hydroxy-5,7-bis(trifluoromethyl)pyrazolo[1,5-a]py rimidine-3-carboximidamide in DMF (500 μl, 0.1 mmol) were mixed. To the mixture, was adde d a mixed solution of 1-ethyl-3-(3-dimethylaminopropyl)carbodiimide hydrochloride 0.2 M, and 1-hydroxybenzotriazole monohydrate 0.2 M in DMF(1000 μl, 0.2 mmol), and the mixture was stirred at room temperature for 3 hours. The reaction solution was diluted with ethyl acetate, washed with a 5% sodium hydrogen carbonate aqueous solution, and concentrated under redu ced pressure. The obtained oily substance was dissolved in pyridine (3 ml), and stirred while h eating at 115 °C for 15 hours. The reaction mixture was concentrated under reduced pressure to give a crude product. The obtained crude product was purified by preparative HPLC to give the titled compound (16 mg) (yield 39%).
MS (ESI+) :418 (M+H)
$^1$ H NMR (400 MHz, $CDCl_3$) δ ppm 1.74 - 1.81 (2 H, m), 1.83 - 1.90 (2 H, m), 2.59 - 2.64 (2 H, m ), 2.65 - 2.70 (2 H, m), 2.71 (3 H, s), 2.81 (3 H, s), 5.57 (2 H, s), 6.76 (1 H, s), 8.60 (1 H, s).

Example 277

1-[(3-Pyrazin-2-yl-1,2,4-oxadiazol-5-yl)methyl]-3-(trifluoromethyl)-4,5,6,7-tetrahydro-1H-indaz ole

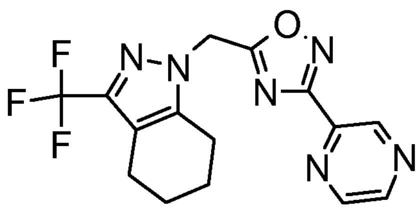

A 0.2M solution of [3-(trifluoromethyl)-4,5,6,7-tetrahydro-1H-indazol-1-yl]acetic acid in DMF (500 μl, 0.1 mmol), and a 0.2M solution of N'-hydroxy-5,7-bis(trifluoromethyl)pyrazolo[1,5-a]p yrimidine-3-carboximidamide in DMF (500 μl, 0.1 mmol)were mixed. To the mixture, was add ed a mixed 0.2 M solution of 1-ethyl-3-(3-dimethylaminopropyl)carbodiimide hydrochloride an d 1-hydroxybenzotriazole monohydrate in DMF (1000 μl, 0.2 mmol), and the mixture was stirr ed for 3 hours at room temperature. The reaction solution was diluted with ethyl acetate, was hed with a 5% sodium hydrogen carbonate aqueous solution, and concentrated under reduced pressure. The obtained oily substance was dissolved in pyridine (3 ml), and stirred while heati ng at 115 °C for 15 hours. The reaction mixture was concentrated under reduced pressure to gi ve a crude product. The obtained crude product was purified by preparative HPLC to give the titled compound (21 mg) (yield 58%).
MS (ESI+) :351(M+H)
$^1$ H NMR (400 MHz, $CDCl_3$) δ ppm 1.74 - 1.83 (2 H, m), 1.84 - 1.92 (2 H, m), 2.59 - 2.65 (2 H, m ), 2.66 - 2.70 (2 H, m), 5.61 (2 H, s), 8.74 - 8.80 (2 H, m), 9.35 (1 H, s).

Example 278

5-Methyl-7-(trifluoromethyl)-3-(5-{[3-(trifluoromethyl)-5,6-dihydrocyclopenta[c]pyrazole-1(4H )-yl]methyl}-1,2,4-oxadiazol-3-yl)pyrazolo[1,5-a]pyrimidine

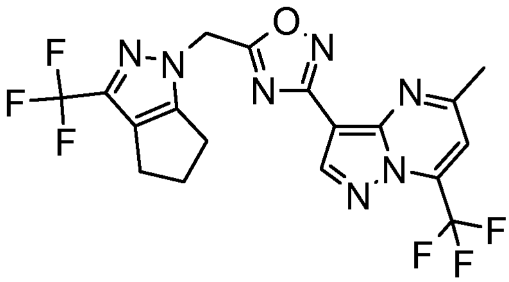

A solution of [3-(trifluoromethyl)-4,5,6,7-tetrahydro-1H-indazol-1-yl]acetic acid in 0.2M DMF ( 500 µl, 0.1 mmol), and a solution of N'-hydroxy-5,7-bis(trifluoromethyl)pyrazolo[1,5-a]pyrimidi ne-3-carboximidamide in 0.2M DMF (500 µl, 0.1 mmol)were mixed. To the mixture, was added a mixed solution of 1-ethyl-3-(3-dimethylaminopropyl)carbodiimide hydrochloride 0.2 M, and 1-hydroxybenzotriazole monohydrate 0.2 M in DMF (1000 µl, 0.2 mmol), and the mixture was stirred for 3 hours at room temperature. The reaction solution was diluted with ethyl acetate, washed with a 5% sodium hydrogen carbonate aqueous solution, and concentrated under redu ced pressure. The obtained oily substance was dissolved in pyridine (3 ml), and stirred while h eating at 115 oC for 15 hours. The reaction mixture was concentrated under reduced pressure to give a crude product. The obtained crude product was purified by preparative HPLC to give the titled compound (2.7 mg) (yield 6%).
MS (ESI+) :458(M+H)

$^1$ H NMR (400 MHz, $CDCl_3$) δ ppm 2.61 - 2.68 (2 H, m), 2.73 - 2.83 (4 H, m), 2.84 (3 H, s), 5.58 ( 2 H, s), 7.23 (1 H, s), 8.72 (1 H, s).

Example 279

1-[(3-Thiophen-2-yl-1,2,4-oxadiazol-5-yl)methyl]-3-(trifluoromethyl)-1,4,6,7-tetrahydropyrano[ 4,3-c]pyrazole

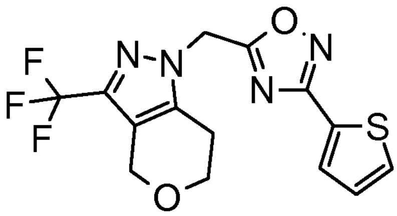

A 0.2M solution of [3-(trifluoromethyl)-4,5,6,7-tetrahydro-1H-indazol-1-yl]acetic acid in DMF ( 500 µl, 0.1 mmol), and a 0.2M solution of benzothiophene-2-carboximidamide in DMF (500 µl, 0.1 mmol) were mixed. To the mixture, was added a mixed 0.2 M solution of 1-ethyl-3-(3-dimet hylaminopropyl)carbodiimide hydrochloride and 1-hydroxybenzotriazole monohydrate in DMF (1000 µl, 0.2 mmol), and the mixture was stirred for 3 hours at room temperature. The reacti on solution was diluted with ethyl acetate, washed with a 5% sodium hydrogen carbonate aqu eous solution, concentrated under reduced pressure. The obtained oily substance was dissolve d in pyridine (3 ml), and stirred while heating at 115 oC for 15 hours. The reaction mixture w as concentrated under reduced pressure to give a crude product. The obtained crude product w as purified by preparative HPLC to give the titled compound (17 mg) (yield 49%).
MS (ESI+) :357(M+H)
$^1$ H NMR (400 MHz, $CDCl_3$) δ ppm 2.81 - 2.87 (2 H, m), 3.95 - 4.00 (2 H, m), 4.74 (2 H, br. s.), 5 .56 (2 H, s), 7.16 (1 H, dd, J=4.9, 4.0 Hz), 7.53 (1 H, d, J=4.9 Hz), 7.79 (1 H, d, J=3.6 Hz).

Example 280

1,2-Dimethyl-6-(methylsulfanyl)-N-(2-[3-(trifluoromethyl)-4,5,6,7-tetrahydro-1H-indazol-1-yl]e thyl}-1H-thieno[3,4-d]imidazole-4-carboxamide

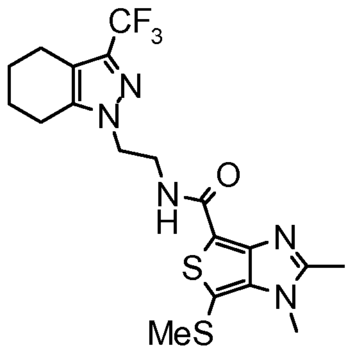

A solution of 1,2-dimethyl-6-(methylsulfanyl)-1H-thieno[3,4-d]imidazole-4-carboxylic acid (50 mg, 0.21 mmol), 2-[3-(trifluoromethyl)-4,5,6,7-tetrahydro-1H-indazol-1-yl]ethanamine hydroch loride (56 mg, 0.21 mmol), HOBt (42 mg, 0.31 mmol), triethylamine (0.029 ml, 0.21 mmol), WS C (60 mg, 0.31 mmol), and DMF (4 ml) was stirred at room temperature for 4 hours. To the re action mixture, was added water. The mixture was extracted with ethyl acetate. The organic 1 ayer was washed with a saturated sodium hydrogen carbonate aqueous solution, water, and s aturated saline, dried over magnesium sulfate, and concentrated under reduced pressure. The obtained residue was recrystallized from ethyl acetate-hexane to give the titled compound (66 mg) as white crystals (yield 70%).
MS (ESI+) :458 (M+H)
1H NMR (300 MHz, $CDCl_3$) δ ppm 1.61 - 1.78 (4 H, m), 2.46 (3 H, s), 2.49 (3 H, s), 2.52 - 2.63 ( 4 H, m), 3.81 (3 H, s), 3.84 - 3.92 (2 H, m), 4.20 - 4.29 (2 H, m), 7.71 - 7.81 (1 H, m).

Example 281

1,2-Dimethyl-4-(methylsulfanyl)-N-{2-[3-(trifluoromethyl)-4,5,6,7-tetrahydro-1H-indazol-1-yl]e thyl}-1H-thieno[3,4-d]imidazole-6-carboxamide

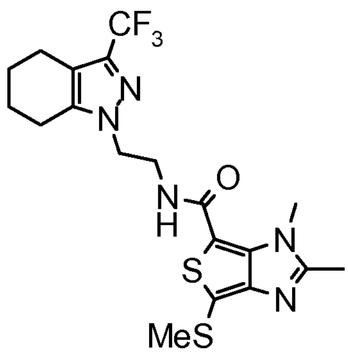

A solution of 1,2-dimethyl-4-(methylsulfanyl)-1H-thieno[3,4-d]imidazole-6-carboxylic acid (24 mg, 0.10 mmol), 2-[3-(trifluoromethyl)-4,5,6,7-tetrahydro-1H-indazol-1-yl]ethanamine (25 mg, 0.13 mmol), HOBt (21 mg, 0.15 mmol), WSC (30 mg, 0.15 mmol), and DMF (3 ml) was stirred at room temperature for 13 hours. To the reaction mixture, was added water. The mixture wa s extracted with ethyl acetate. The organic layer was washed with a saturated sodium hydrog en carbonate aqueous solution, water, and saturated saline, dried over magnesium sulfate, an d concentrated under reduced pressure. The obtained residue was purified by column chromat ography on silica gel [developing solvent: hexane-ethyl

acetate (50:50-30:70)], recrystallized fr om ethyl acetate-hexane to give the titled compound (17 mg) as white crystals (yield 36%).
MS (ESI+) :458 (M+H)
1H NMR (300 MHz, $CDCl_3$ ) δ ppm 1.67 - 1.87 (4 H, m), 2.50 (3 H, s), 2.52 - 2.65 (7 H, m), 3.76 - 3.85 (2 H, m), 3.98 (3 H, s), 4.16 - 4.25 (2 H, m), 6.36 - 6.46 (1 H, m).

Example 282

1,2-dimethyl-N-{2-[3-(trifluoromethyl)-4,5,6,7-tetrahydro-1H-indazol-1-yl]ethyl}-1H-thieno[3, 4-d]imidazole-4-carboxamide

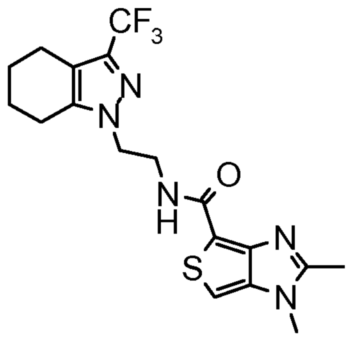

A solution of 1,2-dimethyl-1H-thieno[3,4-d]imidazole-4-carboxylic acid (12 mg, 0.04 mmol), 2-[ 3-(trifluoromethyl)-4,5,6,7-tetrahydro-1H-indazol-1-yl]ethanamine hydrochloride (8.7 mg, 0.04 mmol), HOBt (9 mg, 0.07 mmol), triethylamine (0.007 ml, 0.05 mmol), WSC (12.8 mg, 0.07 m mol), and DMF (3 ml) was stirred at room temperature for 13 hours. To the reaction mixture, was added water. The mixture was extracted with ethyl acetate. The organic layer was washe d with a saturated sodium hydrogen carbonate aqueous solution, water, and saturated saline, dried over magnesium sulfate, and concentrated under reduced pressure. The obtained residu e was purified by column chromatography on silica gel [basic silica gel, developing solvent: he xane-ethyl acetate (40:60-0:100)], recrystallized from ethyl acetate-hexane to give the titled co mpound (12 mg) as white crystals (yield 66%).
MS (ESI+) :412 (M+H)
1H NMR (300 MHz, $CDCl_3$ ) δ ppm 1.67 - 1.85 (4 H, m), 2.50 (3 H, s), 2.53 - 2.65 (4 H, m), 3.79 - 3.88 (2 H, m), 4.00 (3 H, s), 4.18 - 4.26 (2 H, m), 6.47 (1 H, brs.), 7.02 (1 H, s).

Example 283

5-Methyl-7-(trifluoromethyl)-3-(5-{[3-(trifluoromethyl)-4,5,6,7-tetrahydro-1H-indazol-1-yl]met hyl}-1,2,4-oxadiazol-3-yl) pyrazolo[1,5-a]pyrimidine

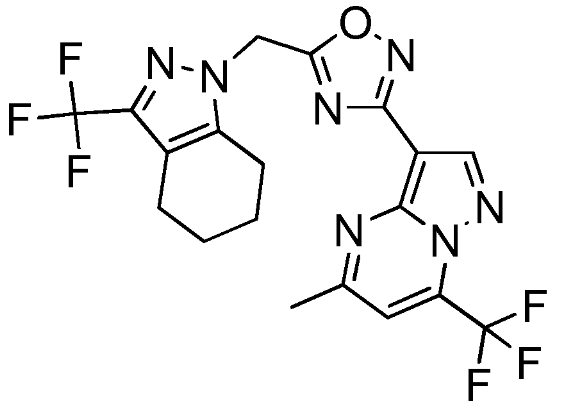

To a solution of [3-(trifluoromethyl)-4,5,6,7-tetrahydro-1H-indazol-1-yl]acetic acid (731 mg, 2.9 5 mmol) in THF (7 ml), were added oxalyl chloride (0.53 ml, 6.13 mmol), and DMF (1 drop) at 0 °C, and the mixture was stirred at the same temperature for 2 hours. The reaction mixture was concentrated under reduced pressure, and the residue was dissolved in pyridine (6 ml). N-hydroxy-5-methyl-7-(trifluoromethyl)pyrazolo[1,5-a]pyrimidine-3-carboximidamide (636 mg, 2 .45 mmol) was added thereto, and the mixture was stirred at room temperature for 5 hours. T he acid chloride was prepared again in the same manner as described above, dissolved in N,N-dimethylacetamide (5 ml), and added to the reaction mixture. The mixture was stirred at roo m temperature for 5 hours, and at 70 °C for 5 hours. To the reaction mixture, was added water , and the mixture was extracted with ethyl acetate. The organic layer was washed with satura ted saline, dried over sodium sulfate, and concentrated under reduced pressure. The obtained residue was purified by preparative HPLC. The obtained crystals were recrystallized from eth yl acetate-hexane to give the titled compound (350 mg) (yield 30%).
MS (ESI+) :472 (M+H).
$^1$ H NMR (300 MHz, $CDCl_3$) δ ppm 1.71 - 1.93 (4 H, m), 2.62 (2H, t, J= 6.0 Hz), 2.69 (2H, t, J= 6.0 Hz), 2.83 (3 H, s), 5.58 (2 H, s), 7.21 (1 H, s), 8.71 (1 H, s).

Example 284

5-Methyl-3-(methylsulfanyl)-4-oxo-N-{2-[3-(trifluoromethyl)-4,5,6,7-tetrahydro-1H-indazol-1-yl ]ethyl}-5,6-dihydro-4H-thieno[3,4-c]pyrrole-1-carboxamide

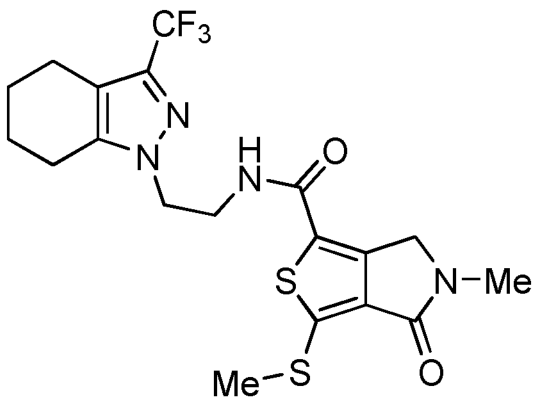

A mixture of 2-[3-(trifluoromethyl)-4,5,6,7-tetrahydro-1H-indazol-1-yl]ethanamine hydrochlori de (32 mg, 0.119 mmol), 5-methyl-3-(methylsulfanyl)-4-oxo-5,6-dihydro-4H-thieno[3,4-c]pyrrol e-1-carboxylic acid (29 mg, 0.119 mmol), WSC (27 mg, 0.143 mmol), HOBt (19 mg, 0.143 mmol ), triethylamine (0.058 ml, 0.417 mmol), and DMF (1 ml) was stirred at room temperature for 15 hours. To the reaction mixture, was added water, and the mixture was extracted with ethyl acetate. The organic layer was washed with water, and saturated saline, dried over sodium s ulfate, and concentrated under reduced pressure. The obtained crystals were washed with diis opropyl ether to give the titled compound (45 mg) (yield 83%).

MS (ESI+) :459 (M+H).
$^1$ H NMR (300 MHz, $CDCl_3$) δ ppm 1.70 - 1.90 (4 H, m), 2.56-2.62 (4H, m), 2.69 (3 H, s), 3.10 (3 H, s), 3.80 - 3.92 (2 H, m), 4.13 - 4.24 (2 H, m), 4.42 (2 H, s), 6.85 (1 H, t, J=4.9 Hz).

Example 285

6-Methyl-7-oxo-N-{2-[3-(trifluoromethyl)-4,5,6,7-tetrahydro-1H-indazol-1-yl]ethyl}-4,5,6,7-tetr ahydrothieno[2,3-c]pyridine-3-carboxamide

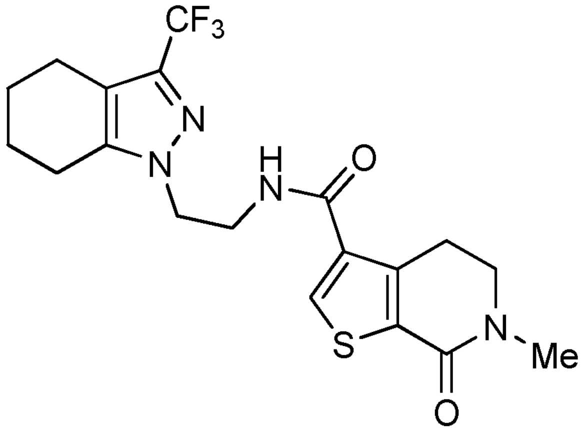

A mixture of 2-[3-(trifluoromethyl)-4,5,6,7-tetrahydro-1H-indazol-1-yl]ethanamine hydrochlori de (35 mg, 0.130 mmol), 6-methyl-7-oxo-4,5,6,7-tetrahydrothieno[2,3-c]pyridine-3-carboxylic ac id (27 mg, 0.130 mmol), WSC (30 mg, 0.156 mmol), HOBt (21 mg, 0.156 mmol), triethylamine ( 0.063 ml, 0.455 mmol), and DMF (1 ml) was stirred at room temperature for 15 hours. To the r eaction mixture, was added water, and the mixture was extracted with ethyl acetate. The orga nic layer was washed with water, and saturated saline, dried over sodium sulfate, and concent rated under reduced pressure. The obtained crystals were washed with diethyl ether to give t he titled compound (40 mg) (yield 72%).
MS (ESI+) :427 (M+H).
$^1$ H NMR (300 MHz, $CDCl_3$) δ ppm 1.67 - 1.89 (4 H, m), 2.50 - 2.67 (4 H, m), 3.10 (3 H, s), 3.16 - 3.27 (2 H, m), 3.61 (2 H, t, J=7.2 Hz), 3.81-3.86 (2 H, m), 4.16 - 4.26 (2 H, m), 6.91 (1 H, br. s.), 7.80 (1 H, s).

Example 286

3-(Methylsulfanyl)-4-oxo-N-{2-[3-(trifluoromethyl)-4,5,6,7-tetrahydro-1H-indazol-1-yl]ethyl}-4, 5,6,7-tetrahydro-2-benzothiophene-1-carboxamide

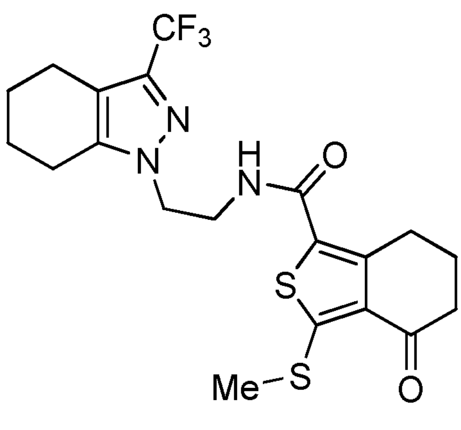

A mixture of 2-[3-(trifluoromethyl)-4,5,6,7-tetrahydro-1H-indazol-1-yl]ethanamine hydrochlori de (35 mg, 0.130 mmol), 3-methylsulfanyl-4-oxo-4,5,6,7-tetrahydro-2-benzothiophene-1-carbox ylic acid (27 mg, 0.130 mmol), WSC (30 mg, 0.156 mmol), HOBt (21 mg, 0.156 mmol), triethyla mine (0.063 ml, 0.455 mmol), and DMF (1 ml) was stirred at room temperature for 15 hours. T o the reaction mixture, was added water, and the mixture was extracted with ethyl acetate. T he organic layer was washed with water, and saturated saline, dried over sodium sulfate, and concentrated under reduced pressure. The obtained crystals were washed with diethyl ether t o give the titled compound (40 mg) (yield 72%).
MS (ESI+) :458 (M+H).
$^1$ H NMR (300 MHz, $CDCl_3$) δ ppm 1.69 - 1.91 (4 H, m), 1.99 - 2.15 (2 H, m), 2.48 - 2.64 (9 H, m ), 3.07 (2 H, t, J=6.2 Hz), 3.81 - 3.93 (2 H, m), 4.21 (2 H, dd, J=6.4, 4.2 Hz), 6.92 (1 H, br. s.).

Example 287

4-Hydroxy-3-(methylsulfanyl)-N-(2-[3-(trifluoromethyl)-4,5,6,7-tetrahydro-1H-indazol-1-yl]eth yl}-4,5,6,7-tetrahydro-2-benzothiophene-1-carboxamide

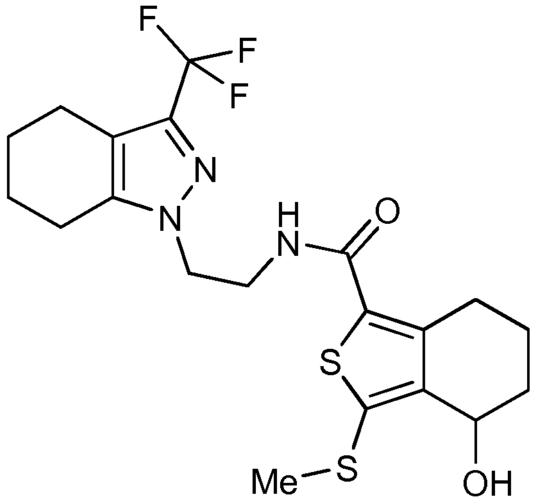

To a solution of 3-(methylsulfanyl)-4-oxo-N-{2-[3-(trifluoromethyl)-4,5,6,7-tetrahydro-1H-indaz ol-1-yl]ethyl}-4,5,6,7-tetrahydro-2-benzothiophene-1-carboxamide (194 mg, 0.424 mmol) in met hanol (2 ml), was added sodium borohydride (24 mg, 0.636 mmol) on ice, and the mixture was stirred at room temperature for 8 hours. Sodium borohydride (24 mg, 0.636 mmol) was added thereto on ice, and the mixture was stirred at room temperature for 15 hours. To the reaction mixture, was added water, and the mixture was extracted with ethyl acetate. The organic laye r was washed with saturated saline, dried over sodium sulfate, and concentrated under reduce d pressure. The obtained residue was crystallized from diethyl ether-diisopropyl ether to give the titled compound (103 mg) (yield 53%).
MS (ESI+) :460 (M+H).
$^1$ H NMR (300 MHz, $CDCl_3$) δ ppm 1.74 - 1.99 (8 H, m), 2.31 (1 H, t, J = 2.1 Hz), 2.56 (3 H, s), 2 .57 - 2.60 (4 H, m), 2.64-2.75 (1H, m), 2.93-3.07 (1H, m), 3.85-3.89 (2H, m), 4.17-4.21 (2H, m), 4 .90-4.93 (1H, m), 6.71 (1H, br s).

Example 288

3-(Methylsulfanyl)-N-{2-[3-(trifluoromethyl)-4,5,6,7-tetrahydro-1H-indazol-1-yl]ethyl}-4,5,6,7-t etrahydro-2-benzothiophene-1-carboxamide

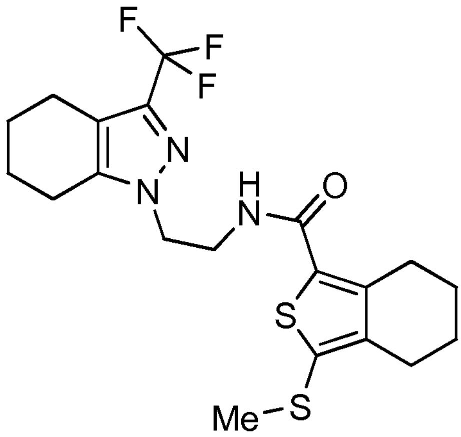

To a solution of 4-hydroxy-3-(methylsulfanyl)-N-{2-[3-(trifluoromethyl)-4,5,6,7-tetrahydro-1H-i ndazol-1-yl]ethyl}-4,5,6,7-tetrahydro-2-benzothiophene-1-carboxamide (34 mg, 0.0740 mmol) i n acetonitrile (0.7 ml), were added trifluoroacetic acid (0.055 ml, 0.740 mmol), and triethylsila ne (0.018 ml, 0.111 mmol) on ice, and the mixture was stirred at room temperature for 2 hours . To the reaction mixture, was added a saturated sodium hydrogen carbonate aqueous solution , and the mixture was extracted ethyl acetate. The organic layer was washed with saturated s aline, dried over sodium sulfate, and concentrated under reduced pressure. The obtained resid ue was purified by column chromatography on silica gel [developing solvent: hexane-ethyl acet ate (90:10-60:40)] to give the titled compound (27 mg) (yield 82%).
MS (ESI+) :444 (M+H).
$^1$ H NMR (300 MHz, $CDCl_3$) δ ppm 1.66 - 1.90 (8 H, m), 2.48 (3 H, s), 2.52 - 2.67 (6 H, m), 2.86 ( 2 H, br. s.), 3.81 - 3.94 (2 H, m), 4.14 - 4.29 (2 H, m), 6.60 (1 H, br. s.).

Example 289

2,3-Dimethyl-5-(trifluoromethyl)-N-(2-[3-(trifluoromethyl)-4,5,6,7-tetrahydro-1H-indazol-1-yl] ethyl}imidazo[1,2-a]pyridine-8-carboxamide

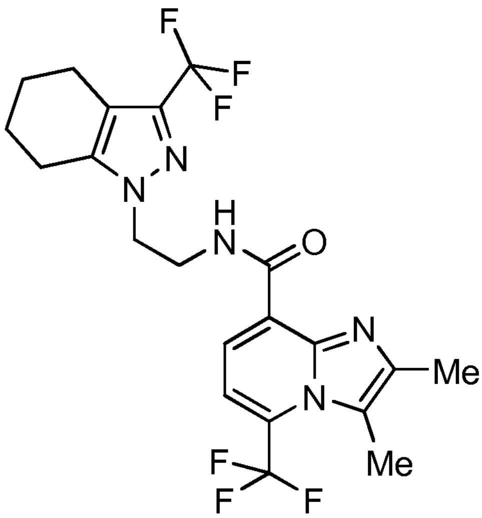

To a solution of butyl 2,3-dimethyl-5-(trifluoromethyl)imidazo[1,2-a]pyridine-8-carboxylate (43 mg, 0.137 mmol) in methanol (1 ml), was added 2N aqueous sodium hydroxide (0.89 ml, 8.39 mmol), and the mixture was stirred at room temperature for 5 hours. The reaction mixture wa s adjusted to pH 4 by 1N hydrochloric acid, and concentrated under reduced pressure. A mixtu re of the obtained 2,3-dimethyl-5-(trifluoromethyl)imidazo[1,2-a]pyridine-8-carboxylic acid, 2-[ 3-(trifluoromethyl)-4,5,6,7-tetrahydro-1H-indazol-1-yl]ethanamine (32 mg, 0.137 mmol), WSC (32 mg, 0.164 mmol),

HOBt (22 mg, 0.164 mmol), triethylamine (0.095 ml, 0.685 mmol), and D MF (1 ml) was stirred at room temperature for 15 hours. To the reaction mixture, water was a dded, and the mixture was extracted with ethyl acetate. The organic layer was washed with s aturated saline, dried over sodium sulfate, and concentrated under reduced pressure. The obt ained residue was purified by column chromatography on silica gel [developing solvent: hexan e-ethyl acetate (95:5-70:30)]. The obtained crystals were washed with diisopropyl ether, and h exane to give the titled compound (11 mg) (yield 17%).
MS (ESI+) :474 (M+H).
$^1$ H NMR (300 MHz, $CDCl_3$) δ ppm 1.66 (4 H, br. s.), 2.41 (3 H, s), 2.49 - 2.65 (7 H, m), 3.97-4.0 3 (2H, m), 4.30 (2 H, t, J=6.2 Hz), 7.46 (1 H, d, J=7.9 Hz), 8.12 (1 H, d, J=7.5 Hz), 10.76 (1 H, b r. s.).

Example 290

N-(3-Chlorophenyl)-2-[3-(trifluoromethyl)-4,5,6,7-tetrahydro-1H-indazol-1-yl]acetamide

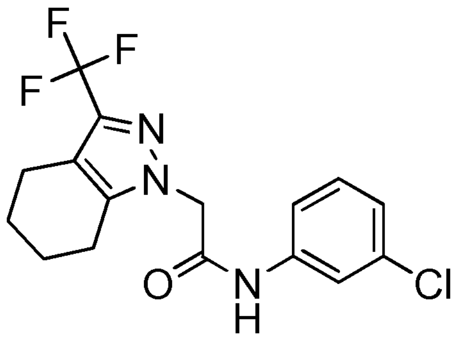

The title compound was obtained in the same manner as in Example 3.
Yield: 5.1 mg
MS (ESI+) :358 (M+H)

Example 291

2-({[3-(Trifluoromethyl)-4,5,6,7-tetrahydro-1H-indazol-1-yl]acetyl}amino)benzamide

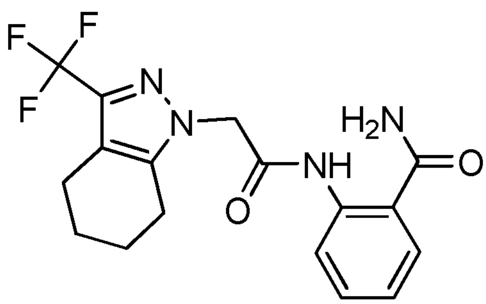

The title compound was obtained in the same manner as in Example 3.
Yield: 4.2 mg
MS (ESI+) :367 (M+H)

Example 292

N-(4-Methoxyphenyl)-2-[3-(trifluoromethyl)-4,5,6,7-tetrahydro-1H-indazol-1-yl]acetamide

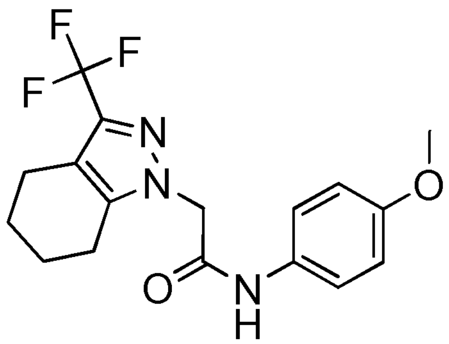

The title compound was obtained in the same manner as in Example 3.
Yield: 5.3 mg
MS (ESI+) :354 (M+H)

Example 293

N-Pyridin-3-yl-2-[3-(trifluoromethyl)-4,5,6,7-tetrahydro-1H-indazol-1-yl]acetamide

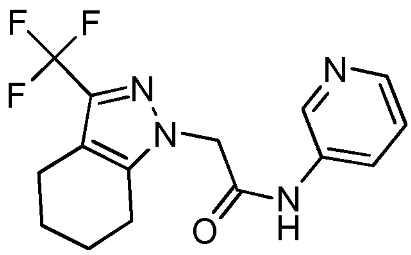

The title compound was obtained in the same manner as in Example 3.
Yield: 4.3 mg
MS (ESI+) :325 (M+H)

Example 294

N-Pyridin-2-yl-2-[3-(trifluoromethyl)-4,5,6,7-tetrahydro-1H-indazol-1-yl]acetamide

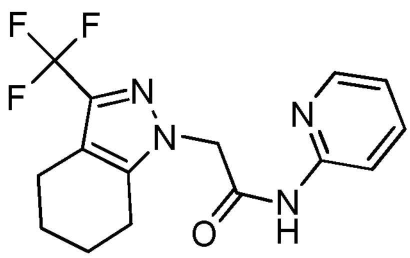

The title compound was obtained in the same manner as in Example 3.
Yield: 2.3 mg
MS (ESI+) :325 (M+H)

Example 295

N-(2-Methylphenyl)-2-[3-(trifluoromethyl)-4,5,6,7-tetrahydro-1H-indazol-1-yl]acetamide

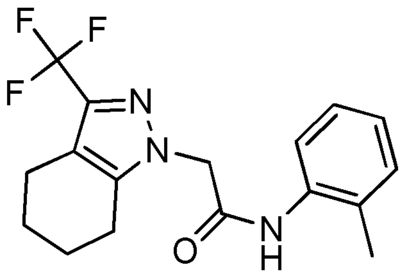

The title compound was obtained in the same manner as in Example 3.
Yield: 4.5 mg
MS (ESI+) :338 (M+H)

Example 296

N-{4-[(1-Methylethyl)sulfamoyl]phenyl}-2-[3-(trifluoromethyl)-4,5,6,7-tetrahydro-1H-indazol-1-yl]acetamide

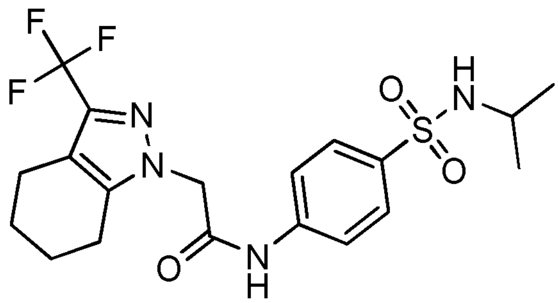

The title compound was obtained in the same manner as in Example 3.
Yield: 1.4 mg
MS (ESI+) :445 (M+H)

Example 297

1-Methyl-3-propyl-4-({[3-(trifluoromethyl)-4,5,6,7-tetrahydro-1H-indazol-1-yl]acetyl}amino)-1 H-pyrazole-5-carboxamide

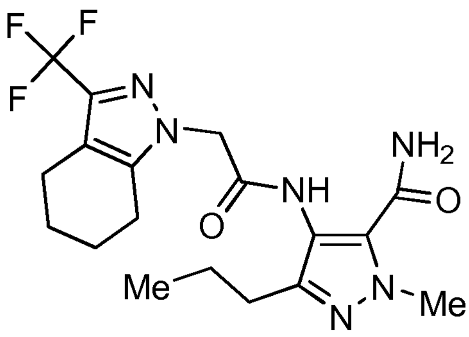

The title compound was obtained in the same manner as in Example 3.
Yield: 7.9 mg
MS (ESI+) :413 (M+H)

Example 298

4-({[3-(Trifluoromethyl)-4,5,6,7-tetrahydro-1H-indazol-1-yl]acetyl}amino)-1H-imidazole-5-carb oxamide

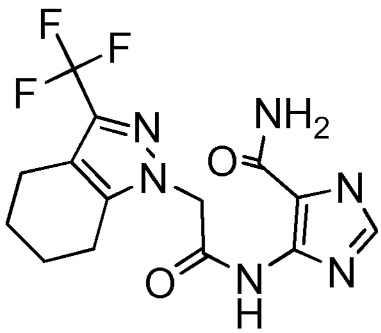

The title compound was obtained in the same manner as in Example 3.
Yield: 3.1 mg
MS (ESI+) :357 (M+H)

Example 299

N-(1-Methyl-1H-pyrazol-3-yl)-2-[3-(trifluoromethyl)-4,5,6,7-tetrahydro-1H-indazol-1-yl]acetam ide

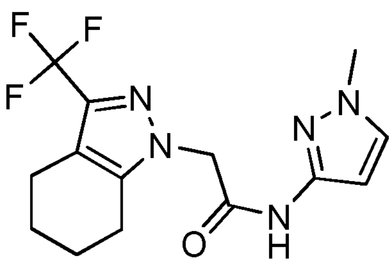

The title compound was obtained in the same manner as in Example 3.
Yield: 4.8 mg

MS (ESI+) :328 (M+H)

Example 300

3-({[3-(Trifluoromethyl)-4,5,6,7-tetrahydro-1H-indazol-1-yl]acetyl}amino)thiophene-2-carboxa mide

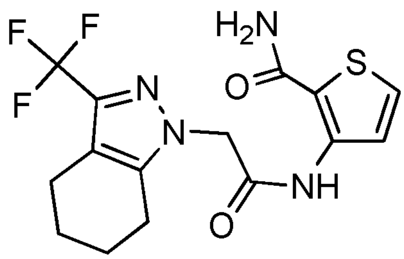

The title compound was obtained in the same manner as in Example 3.
Yield: 1.2 mg
MS (ESI+) :373 (M+H)

Example 301

1-Methyl-4-({[3-(trifluoromethyl)-4,5,6,7-tetrahydro-1H-indazol-1-yl]acetyl}amino)-1H-pyrazol e-5-carboxamide

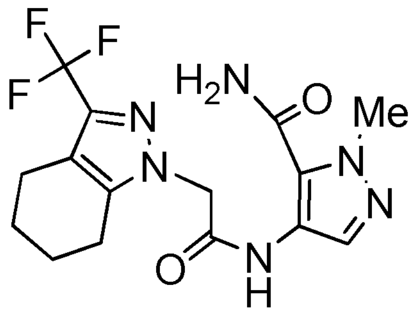

The title compound was obtained in the same manner as in Example 3.
Yield: 8.1 mg
MS (ESI+) :371 (M+H)

Example 302

1-Ethyl-4-({[3-(trifluoromethyl)-4,5,6,7-tetrahydro-1H-indazol-1-yl]acetyl}amino)-1H-pyrazole-5-carboxamide

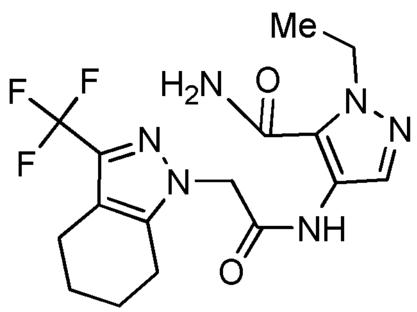

The title compound was obtained in the same manner as in Example 3.
Yield: 7.2 mg
MS (ESI+) :385 (M+H)

Example 303

5-Chloro-2-({[3-(trifluoromethyl)-4,5,6,7-tetrahydro-1H-indazol-1-yl]acetyl}amino)benzamide

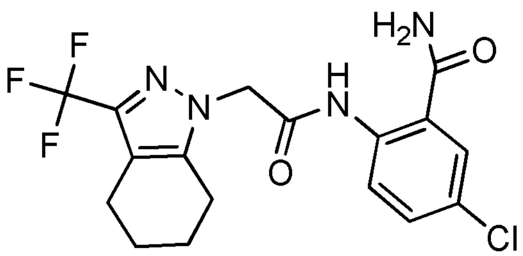

The title compound was obtained in the same manner as in Example 3.
Yield: 1.4 mg
MS (ESI+) :401 (M+H)

Example 304

3-({[3-(Trifluoromethyl)-4,5,6,7-tetrahydro-1H-indazol-1-yl]acetyl}amino)benzamide

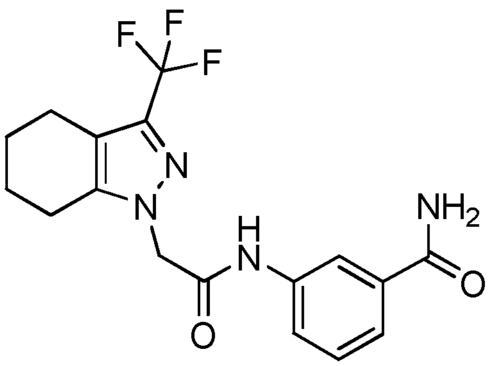

The title compound was obtained in the same manner as in Example 3.
Yield: 11.1 mg
MS (ESI+) :367 (M+H)

Example 305

4-Methoxy-3-({[3-(trifluoromethyl)-4,5,6,7-tetrahydro-1H-indazol-1-yl]acetyl}amino)benzamide

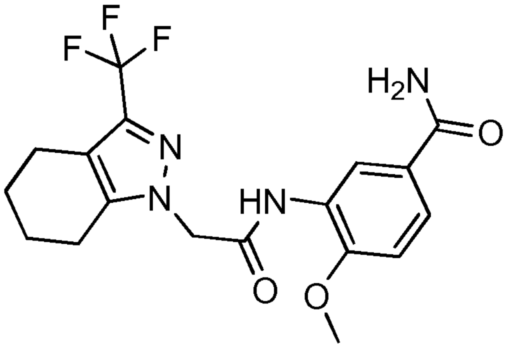

The title compound was obtained in the same manner as in Example 3.
Yield: 12.9 mg
MS (ESI+) :397 (M+H)

Example 306

N-(2-Chlorophenyl)-2-[3-(trifluoromethyl)-4,5,6,7-tetrahydro-1H-indazol-1-yl]acetamide

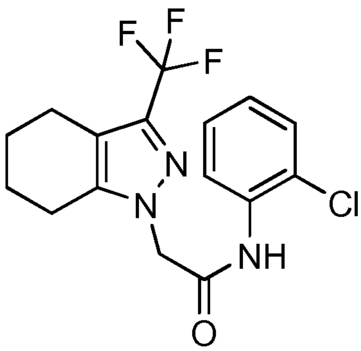

The title compound was obtained in the same manner as in Example 3.
Yield: 1.5 mg
MS (ESI+) :358 (M+H)

Example 307

N-[1-(4-Chlorophenyl)ethyl]-2-[3-(trifluoromethyl)-4,5,6,7-tetrahydro-1H-indazol-1-yl]acetami de

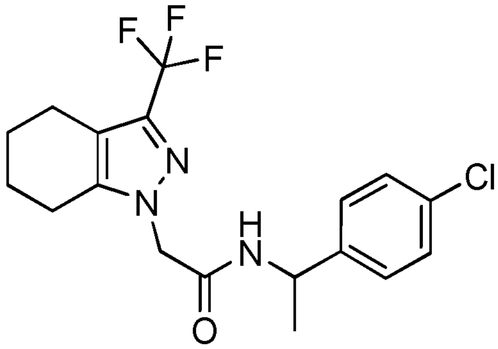

The title compound was obtained in the same manner as in Example 3.
Yield: 13.2 mg
MS (ESI+) :386 (M+H)

Example 308

N-(1-Furan-2-ylethyl)-2-[3-(trifluoromethyl)-4,5,6,7-tetrahydro-1H-indazol-1-yllacetamide

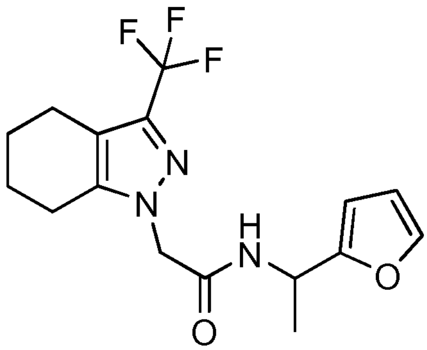

The title compound was obtained in the same manner as in Example 3.
Yield: 13.8 mg
MS (ESI+) :342 (M+H)

Example 309

1-{2-[3-(4-Methylphenyl)pyrrolidin-1-yl]-2-oxoethyl}-3-(trifluoromethyl)-4,5,6,7-tetrahydro-1H-indazole

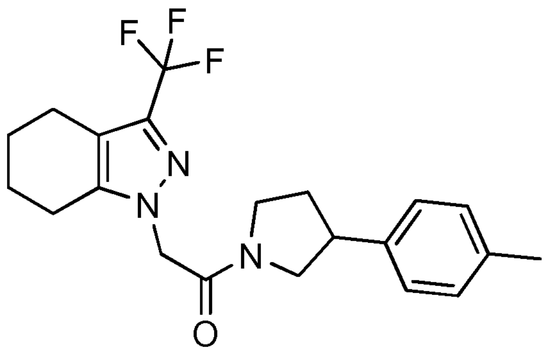

The title compound was obtained in the same manner as in Example 3.
Yield: 17.1 mg
MS (ESI+) :392 (M+H)

Example 310

1-[2-(2-Furan-2-yl-1,3-thiazolidin-3-yl)-2-oxoethyl]-3-(trifluoromethyl)-4,5,6,7-tetrahydro-1H-i ndazole

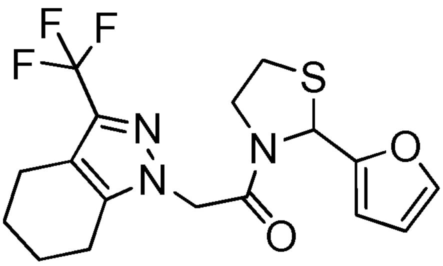

The title compound was obtained in the same manner as in Example 3.
Yield: 13.8 mg
MS (ESI+) :386 (M+H)

Example 311

N-(3-Oxo-2,3-dihydro-1H-isoindol-4-yl)-2-[3-(trifluoromethyl)-4,5,6,7-tetrahydro-1H-indazol-1-yl]acetamide

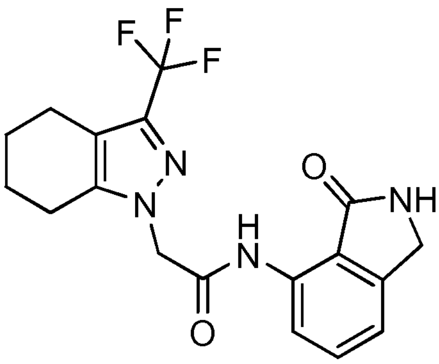

The title compound was obtained in the same manner as in Example 3.
Yield: 2.8 mg

MS (ESI+) :379 (M+H)

Example 312

N-(1-Thiophen-2-ylethyl)-2-[3-(trifluoromethyl)-4,5,6,7-tetrahydro-1H-indazol-1-yl]acetamide

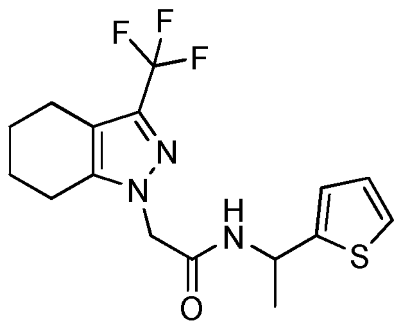

The title compound was obtained in the same manner as in Example 3.
Yield: 12.1 mg
MS (ESI+) :358 (M+H)

Example 313

tert-butyl 2-[1-({[3-(trifluoromethyl)-4,5,6,7-tetrahydro-1H-indazol-1-yl]acetyl}amino)ethyl]-1 H-pyrrole-1-carboxylate

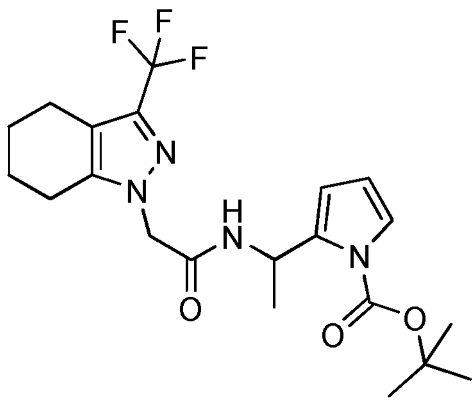

The title compound was obtained in the same manner as in Example 3.
Yield: 1.8 mg
MS (ESI+) :441 (M+H)

Example 314

1-Methyl-4-({[3-(trifluoromethyl)-4,5,6,7-tetrahydro-1H-indazol-1-yl]acetyl}amino)-1H-pyrazol e-3-carboxamide

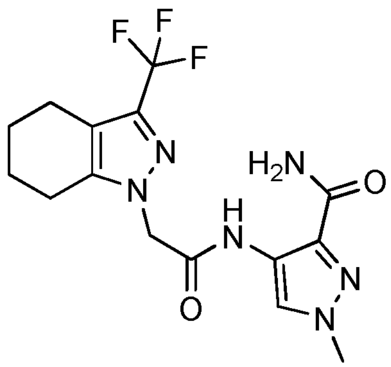

The title compound was obtained in the same manner as in Example 3.
Yield: 14 mg
MS (ESI+) :371 (M+H)

Example 315

N-(2,2-Difluoro-1,3-benzodioxol-4-yl)-2-[3-(trifluoromethyl)-4,5,6,7-tetrahydro-1H-indazol-1-yl] acetamide

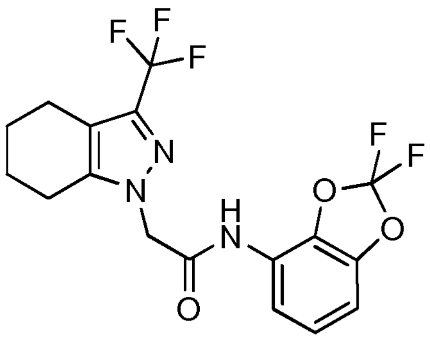

The title compound was obtained in the same manner as in Example 3.
Yield: 0.6 mg
MS (ESI+) :404 (M+H)

Example 316

5-Fluoro-2-({[3-(trifluoromethyl)-4,5,6,7-tetrahydro-1H-indazol-1-yl]acetyl}amino)benzamide

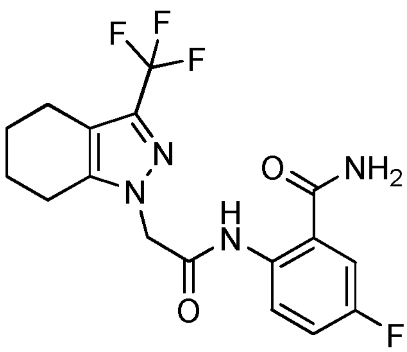

The title compound was obtained in the same manner as in Example 3.
Yield: 28.4 mg
MS (ESI+) :385 (M+H)

Example 317

5-Methyl-2-({[3-(trifluoromethyl)-4,5,6,7-tetrahydro-1H-indazol-1-yl]acetyl}amino)benzamide

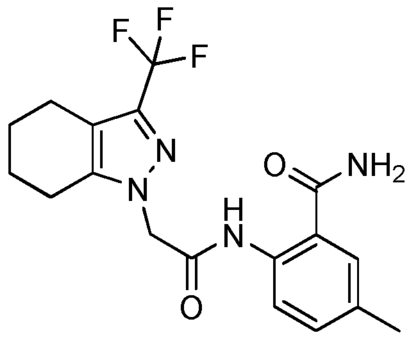

The title compound was obtained in the same manner as in Example 3.
Yield: 19.4 mg
MS (ESI+) :381 (M+H)

Example 318

N,N-dimethyl-4-({[3-(trifluoromethyl)-4,5,6,7-tetrahydro-1H-indazol-1-yl]acetyl}amino)benzam ide

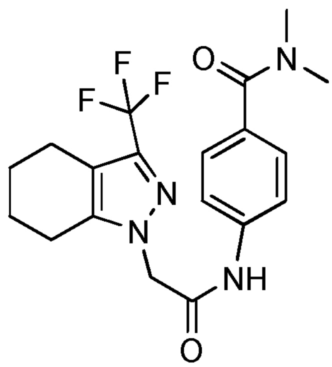

The title compound was obtained in the same manner as in Example 3.
Yield: 11.6 mg
MS (ESI+) :395 (M+H)

Example 319

N-[4-Chloro-2-(trifluoromethoxy)phenyl]-2-[3-(trifluoromethyl)-4,5,6,7-tetrahydro-1H-indazol-1-yl]acetamide

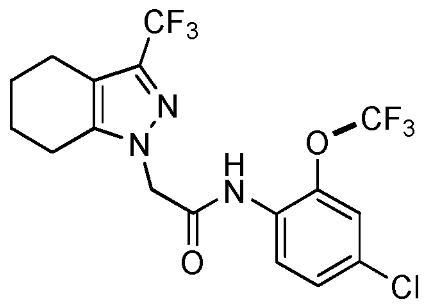

The title compound was obtained in the same manner as in Example 3.
Yield: 0.3 mg
MS (ESI+) :442 (M+H)

Example 320

N-(1-Pyridin-3-ylethyl)-2-[3-(trifluoromethyl)-4,5,6,7-tetrahydro-1H-indazol-1-yl]acetamide

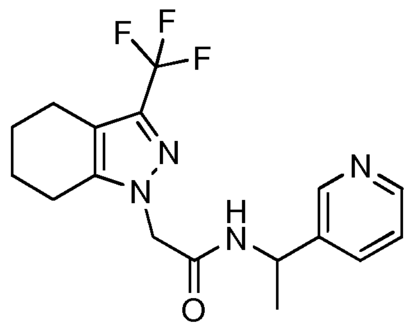

The title compound was obtained in the same manner as in Example 3.
Yield: 18.5 mg
MS (ESI+) :353 (M+H)

Example 321

N-(1-Pyridin-2-ylethyl)-2-[3-(trifluoromethyl)-4,5,6,7-tetrahydro-1H-indazol-1-yl]acetamide

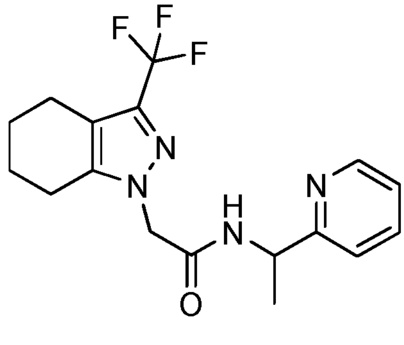

The title compound was obtained in the same manner as in Example 3.
Yield: 14.5 mg

MS (ESI+) :353 (M+H)

Example 322

N-(3-Cyclopropyl-1-methyl-1H-pyrazol-5-yl)-2-[3-(trifluoromethyl)-4,5,6,7-tetrahydro-1H-indaz ol-1-yl]acetamide

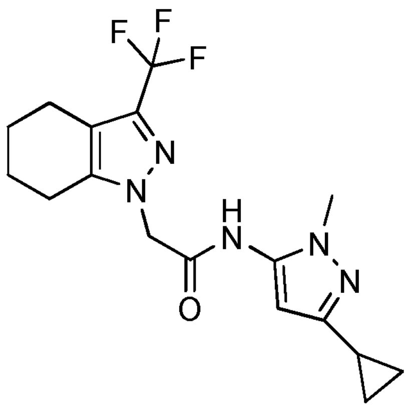

The title compound was obtained in the same manner as in Example 3.
Yield: 9.2 mg
MS (ESI+) :368 (M+H)

Example 323

N-(1-Ethyl-1H-pyrazol-5-yl)-2-[3-(trifluoromethyl)-4,5,6,7-tetrahydro-1H-indazol-1-yl]acetami de

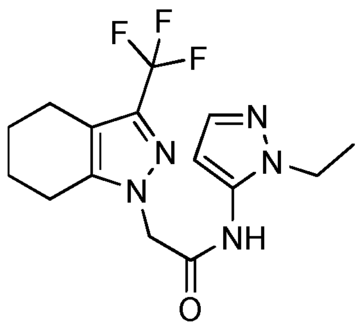

The title compound was obtained in the same manner as in Example 3.
Yield: 8.3 mg
MS (ESI+) :342 (M+H)

Example 324

N-(2-{[4-tert-Butyl-6-(trifluoromethyl)pyrimidin-2-yl]sulfanyl}ethyl)-2-[3-(trifluoromethyl)-4,5, 6,7-tetrahydro-1H-indazol-1-yl]acetamide

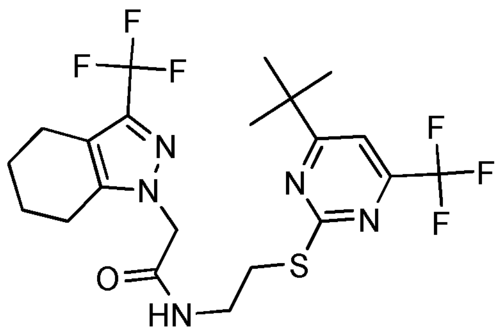

The title compound was obtained in the same manner as in Example 3.
Yield: 16.7 mg
MS (ESI+) :510 (M+H)

Example 325

N-[2-Methoxy-5-(trifluoromethyl)phenyl]-2-[3-(trifluoromethyl)-4,5,6,7-tetrahydro-1H-indazol-1-yl]acetamide

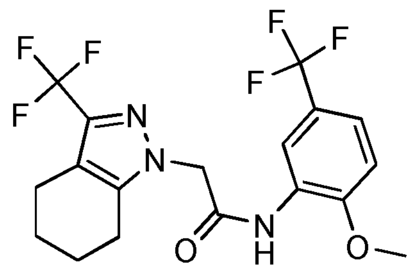

A 0.12 M solution of [3-(trifluoromethyl)-4,5,6,7-tetrahydro-1H-indazol-1-yl]acetic acid in DM F (500 μl, 60 μmol), and a 0.144 M solution of 2-methoxy-5-trifluoromethylaniline in DMF (50 0 μl, 72 μmol), and a 0.24 M solution of HATU and DIEA in DMF (500 μl, 120 μmol) were mix ed at room temperature. The mixture was stirred at 60 °C for 24 hours. The reaction mixture was cooled to room temperature, and extracted by adding ethyl acetate (3 ml), and 2 % sodium hydrogen carbonate aqueous solution (1.5 ml). The organic layer was collected with upper pha se sep tube (Wako Pure Chemical Industries). The solvent was evaporated off under reduced p ressure. The obtained residue was dissolved in DMSO-acetonitrile (1:4) (1 ml), and purified by preparative HPLC to give the titled compound.
Yield: 3.6 mg
MS (ESI+) :422 (M+H)

Example 326

N-[3-(1,3-oxazol-5-yl)phenyl]-2-[3-(trifluoromethyl)-4,5,6,7-tetrahydro-1H-indazol-1-yl]acetami de

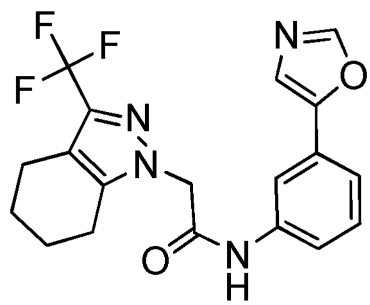

The titled compound was obtained in the same manner as in Example 325.
Yield: 8.2 mg
MS (ESI+) :391 (M+H)

Example 327

N-(3-Thiophen-2-yl-1H-pyrazol-5-yl)-2-[3-(trifluoromethyl)-4,5,6,7-tetrahydro-1H-indazol-1-yl] acetamide

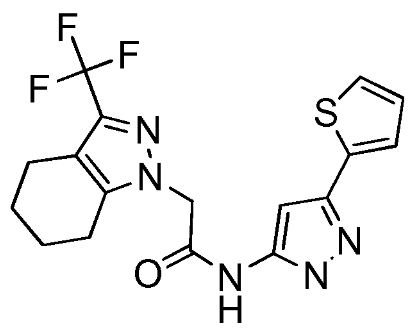

The titled compound was obtained in the same manner as in Example 325.
Yield: 3.6 mg
MS (ESI+) :396 (M+H)

Example 328

N-(3-Furan-2-yl-1H-pyrazol-5-yl)-2-[3-(trifluoromethyl)-4,5,6,7-tetrahydro-1H-indazol-1-yl]ace tamide

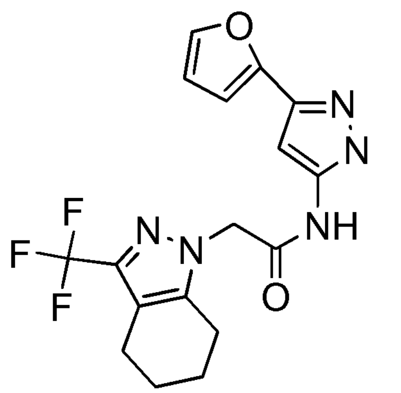

The titled compound was obtained in the same manner as in Example 325.
Yield: 2.8 mg
MS (ESI+) :380 (M+H)

Example 329

N-[4-Fluoro-2-(trifluoromethyl)benzyl]-2-[3-(trifluoromethyl)-4,5,6,7-tetrahydro-1H-indazol-1-yl]acetamide

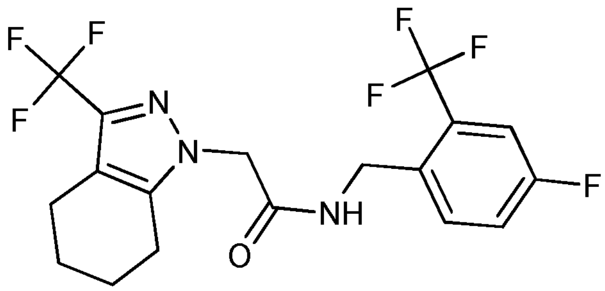

The titled compound was obtained in the same manner as in Example 325.
Yield: 14.4 mg
MS (ESI+) :424 (M+H)

Example 330

N-(2-Chloro-4-fluorobenzyl)-2-[3-(trifluoromethyl)-4,5,6,7-tetrahydro-1H-indazol-1-yl]acetamid e

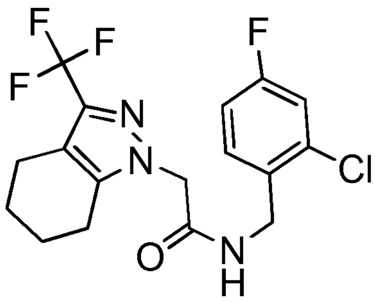

The titled compound was obtained in the same manner as in Example 325.
Yield: 11.9 mg
MS (ESI+) :390 (M+H)

Example 331

N-[4-Fluoro-3-(trifluoromethyl)benzyl]-2-[3-(trifluoromethyl)-4,5,6,7-tetrahydro-1H-indazol-1-yl]acetamide

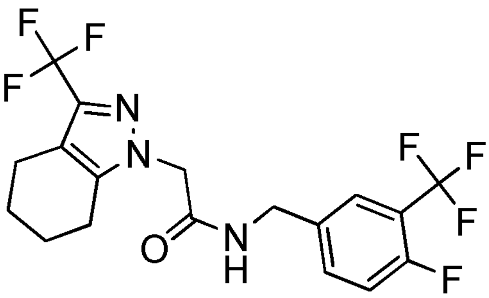

The titled compound was obtained in the same manner as in Example 325.
Yield: 16.3 mg
MS (ESI+) :424 (M+H)

Example 332

N-(3-Chloro-4-fluorobenzyl)-2-[3-(trifluoromethyl)-4,5,6,7-tetrahydro-1H-indazol-1-yl]acetamid e

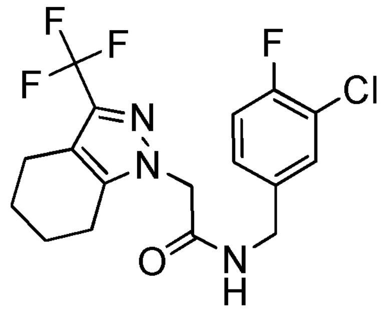

The titled compound was obtained in the same manner as in Example 325.
Yield: 14.1 mg
MS (ESI+) :390 (M+H)

Example 333

N-[2-(Trifluoromethoxy)benzyl]-2-[3-(trifluoromethyl)-4,5,6,7-tetrahydro-1H-indazol-1-yl]aceta mide

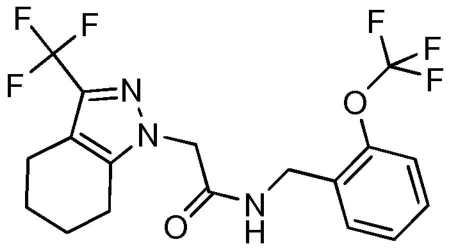

The titled compound was obtained in the same manner as in Example 325.
Yield: 14.3 mg
MS (ESI+) :422 (M+H)

Example 334

N-(5-Chloro-2,4-dimethoxyphenyl)-2-[3-(trifluoromethyl)-4,5,6,7-tetrahydro-1H-indazol-1-yl]ac etamide

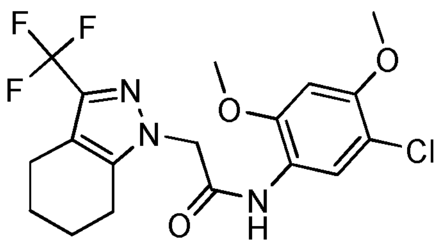

The titled compound was obtained in the same manner as in Example 325.
Yield: 11.9 mg
MS (ESI+) :418 (M+H)

Example 335

N-(3-Chloro-2-methoxyphenyl)-2-[3-(trifluoromethyl)-4,5,6,7-tetrahydro-1H-indazol-1-yl]aceta mide

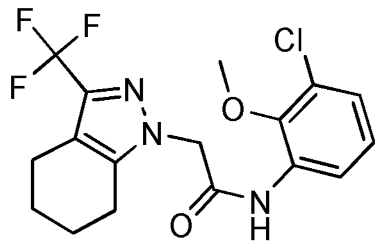

The titled compound was obtained in the same manner as in Example 325.
Yield: 2.3 mg
MS (ESI+) :388 (M+H)

Example 336

N-(1H-Indol-3-ylmethyl)-2-[3-(trifluoromethyl)-4,5,6,7-tetrahydro-1H-indazol-1-yl]acetamide

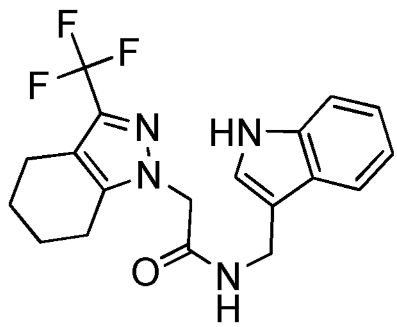

The titled compound was obtained in the same manner as in Example 325.
Yield: 6.0 mg
MS (ESI+) :377 (M+H)

Example 337

N-{[3-(1-Methylethyl)isoxazol-5-yl]methyl}-2-[3-(trifluoromethyl)-4,5,6,7-tetrahydro-1H-indazo 1-1-yl]acetamide

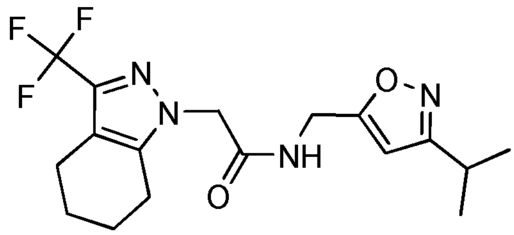

The titled compound was obtained in the same manner as in Example 325.
Yield: 12.1 mg
MS (ESI+) :371 (M+H)

Example 338

N-[(3-Ethylisoxazol-5-yl)methyl]-2-[3-(trifluoromethyl)-4,5,6,7-tetrahydro-1H-indazol-1-yl]acet amide

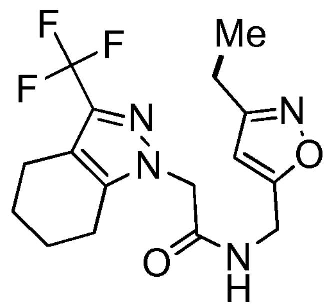

The titled compound was obtained in the same manner as in Example 325.
Yield: 11.6 mg
MS (ESI+) :357 (M+H)

Example 339

N-[(1-Ethyl-1H-pyrazol-4-yl)methyl]-2-[3-(trifluoromethyl)-4,5,6,7-tetrahydro-1H-indazol-1-yl] acetamide

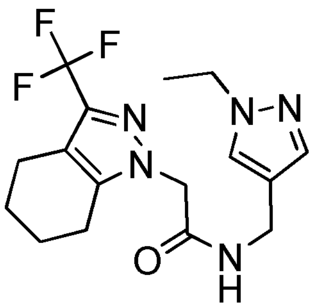

The titled compound was obtained in the same manner as in Example 325.
Yield: 11.3 mg
MS (ESI+) :356 (M+H)

Example 340

N- [(1-Ethyl-1H-pyrazol-3-yl)methyl]-2- [3-(trifluoromethyl)-4,5,6,7-tetrahydro-1H-indazol-1-yl] acetamide

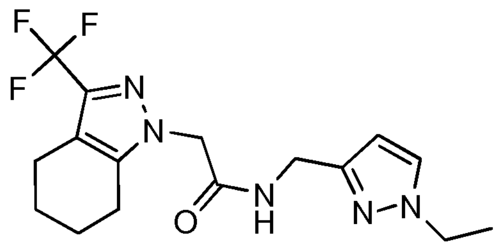

The titled compound was obtained in the same manner as in Example 325.
Yield: 10.9 mg
MS (ESI+) :356 (M+H)

Example 341

N-[(5-tert-Butyl-1H-pyrazol-3-yl)methyl]-2-[3-(trifluoromethyl)-4,5,6,7-tetrahydro-1H-indazol-1-yl] acetamide

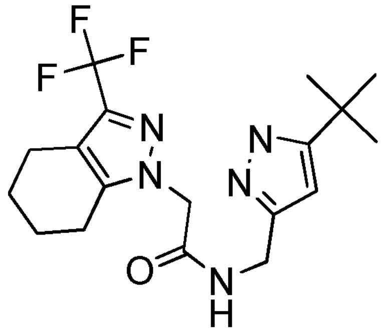

The titled compound was obtained in the same manner as in Example 325.
Yield: 1.2 mg
MS (ESI+) :384 (M+H)

Example 342

N-[(3-Benzyl-1,2,4-oxadiazol-5-yl)methyl]-2-[3-(trifluoromethyl)-4,5,6,7-tetrahydro-1H-indazo 1-1-yl]acetamide

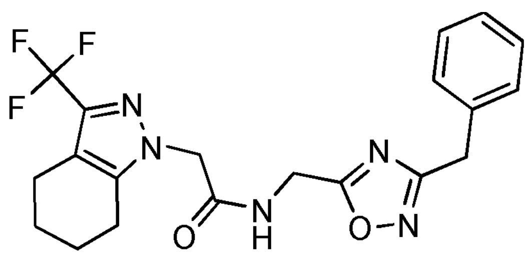

The titled compound was obtained in the same manner as in Example 325.
Yield: 14 mg
MS (ESI+) :420 (M+H)

Example 343

2-[3-(Trifluoromethyl)-4,5,6,7-tetrahydro-1H-indazol-1-yl]-N-(1,3,5-trimethyl-1H-pyrazol-4-yl) acetamide

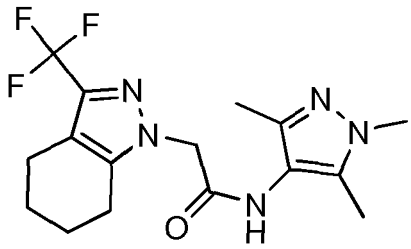

The titled compound was obtained in the same manner as in Example 325.
Yield: 10.6 mg
MS (ESI+) :356 (M+H)

Example 344

N-(1,5-dimethyl-1H-pyrazol-4-yl)-2-[3-(trifluoromethyl)-4,5,6,7-tetrahydro-1H-indazol-1-yl]ace tamide

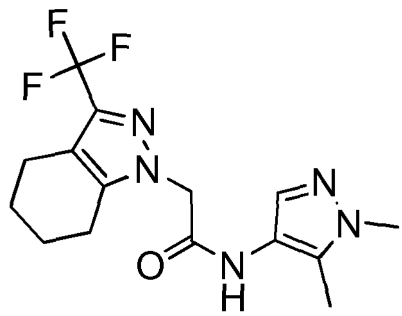

The titled compound was obtained in the same manner as in Example 325.
Yield: 16.2 mg
MS (ESI+) :342 (M+H)

Example 345

N-[(3-Methylthiophen-2-yl)methyl]-2-[3-(trifluoromethyl)-4,5,6,7-tetrahydro-1H-indazol-1-yl]a cetamide

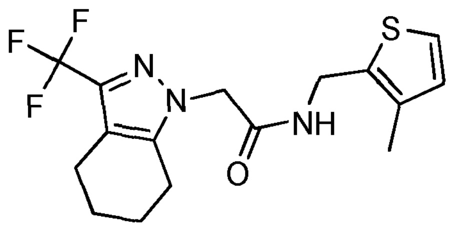

The titled compound was obtained in the same manner as in Example 325.
Yield: 10.5 mg
MS (ESI+) :358 (M+H)

Example 346

N-{3-[(Trifluoromethyl)sulfanyl]phenyl}-2-[3-(trifluoromethyl)-4,5,6,7-tetrahydro-1H-indazol-1-yl]acetamide

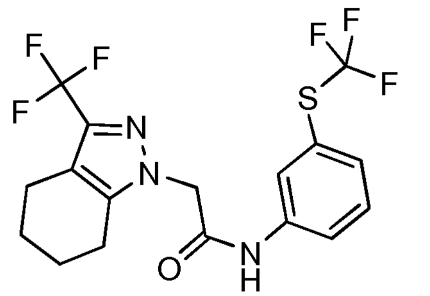

The titled compound was obtained in the same manner as in Example 325.
Yield: 6.0 mg
MS (ESI+) :424 (M+H)

Example 347

N-[3-(Methylsulfonyl)phenyl]-2-[3-(trifluoromethyl)-4,5,6,7-tetrahydro-1H-indazol-1-yl]acetam ide

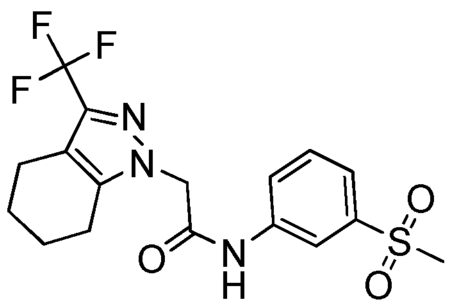

The titled compound was obtained in the same manner as in Example 325.
Yield: 3.5 mg
MS (ESI+) :402 (M+H)

Example 348

N-(3,5-Dimethoxybenzyl)-2-[3-(trifluoromethyl)-4,5,6,7-tetrahydro-1H-indazol-1-yl]acetamide

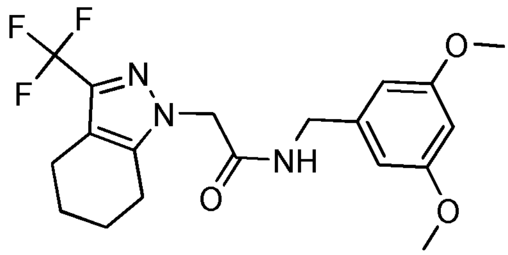

The titled compound was obtained in the same manner as in Example 325.
Yield: 13.7 mg
MS (ESI+) :398 (M+H)

Example 349

N-(6-Chloroimidazo[1,2-b]pyridazine-3-yl)-2-[3-(trifluoromethyl)-4,5,6,7-tetrahydro-1H-indazo 1-1-yl]acetamide

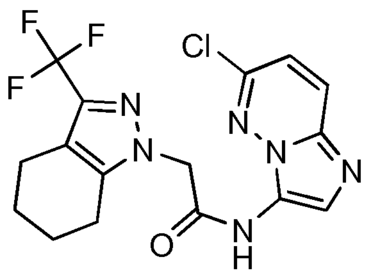

The titled compound was obtained in the same manner as in Example 325.
Yield: 1.1 mg
MS (ESI+) :399 (M+H)

Example 350

N-[3-(1-Methylethyl)phenyl]-2-[3-(trifluoromethyl)-4,5,6,7-tetrahydro-1H-indazol-1-yl]acetami de

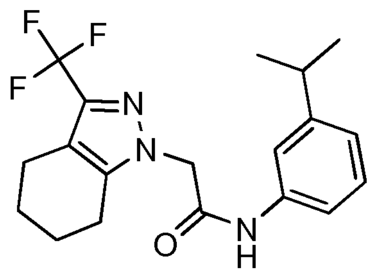

The titled compound was obtained in the same manner as in Example 325.
Yield: 9.9 mg

MS (ESI+) :366 (M+H)

Example 351

N-(5-tert-Butyl-2-methoxyphenyl)-2-[3-(trifluoromethyl)-4,5,6,7-tetrahydro-1H-indazol-1-yl]ac etamide

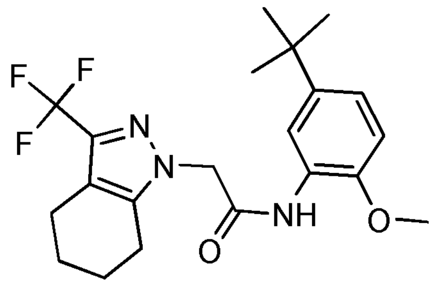

The titled compound was obtained in the same manner as in Example 325.
Yield: 12.1 mg
MS (ESI+) :410 (M+H)

Example 352

N-[3-(2-Methyl-1,3-thiazol-4-yl)phenyl]-2-[3-(trifluoromethyl)-4,5,6,7-tetrahydro-1H-indazol-1-yl]acetamide

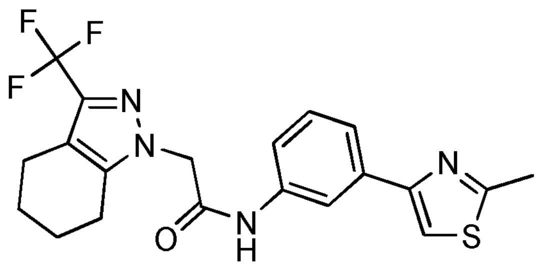

The titled compound was obtained in the same manner as in Example 325.
Yield: 11.5 mg
MS (ESI+) :421 (M+H)

Example 353

N-(5-Furan-2-yl-1H-pyrazol-3-yl)-2-[3-(trifluoromethyl)-4,5,6,7-tetrahydro-1H-indazol-1-yl]ace tamide

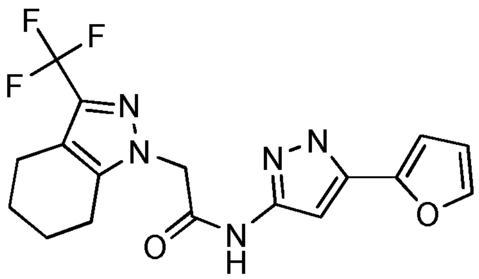

The titled compound was obtained in the same manner as in Example 325.
Yield: 4.9 mg
MS (ESI+) :380 (M+H)

Example 354

N-(3-Methylbenzyl)-2-[3-(trifluoromethyl)-4,5,6,7-tetrahydro-1H-indazol-1-yl]acetamide

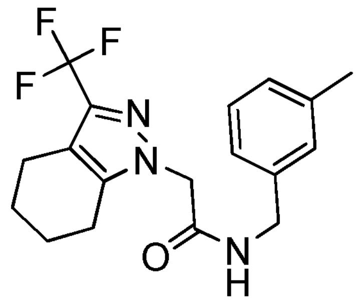

The titled compound was obtained in the same manner as in Example 325.
Yield: 10.2 mg
MS (ESI+) :352 (M+H)

Example 355

N-(3-Methoxybenzyl)-2-[3-(trifluoromethyl)-4,5,6,7-tetrahydro-1H-indazol-1-yl]acetamide

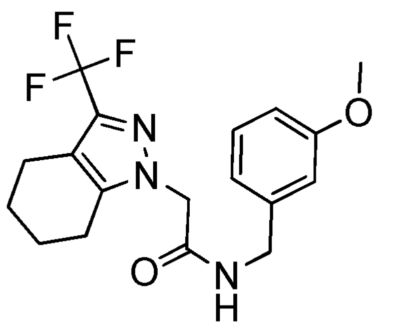

The titled compound was obtained in the same manner as in Example 325.
Yield: 11.2 mg
MS (ESI+) :368 (M+H)

Example 356

N-(2,3-Dihydro-1H-inden-4-yl)-2-[3-(trifluoromethyl)-4,5,6,7-tetrahydro-1H-indazol-1-yl]aceta mide

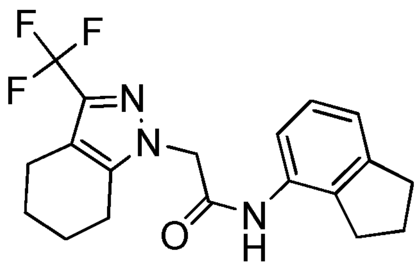

The titled compound was obtained in the same manner as in Example 325.
Yield: 8.3 mg
MS (ESI+) :364 (M+H)

Example 357

Ethyl 3-({[3-(trifluoromethyl)-4,5,6,7-tetrahydro-1H-indazol-1-yl]acetyl}amino)benzoate

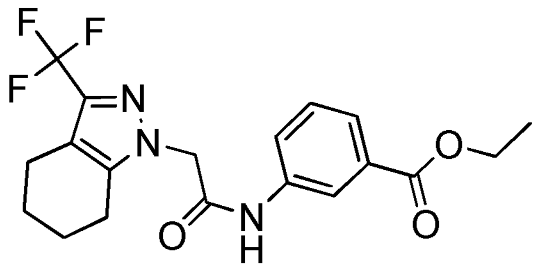

The titled compound was obtained in the same manner as in Example 325.
Yield: 9.4 mg
MS (ESI+) :396 (M+H)

Example 358

N-(3-Ethylphenyl)-2-[3-(trifluoromethyl)-4,5,6,7-tetrahydro-1H-indazol-1-yl]acetamide

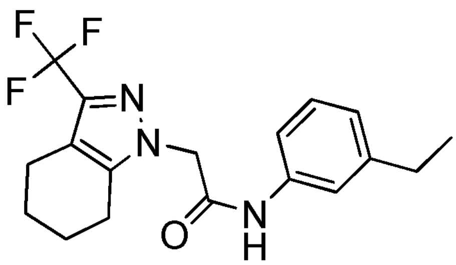

The titled compound was obtained in the same manner as in Example 325.

Yield: 9.2 mg
MS (ESI+) :352 (M+H)

Example 359

N-(2,3-dihydro-1H-inden-5-yl)-2-[3-(trifluoromethyl)-4,5,6,7-tetrahydro-1H-indazol-1-yl]aceta mide

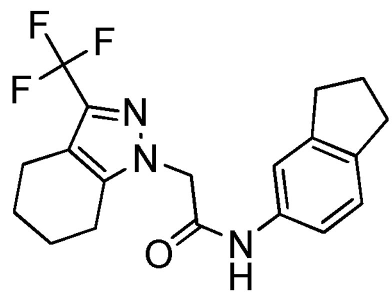

The titled compound was obtained in the same manner as in Example 325.
Yield: 10.1 mg
MS (ESI+) :364 (M+H)

Example 360

N-[3-(Phenylcarbonyl)phenyl]-2-[3-(trifluoromethyl)-4,5,6,7-tetrahydro-1H-indazol-1-yl]aceta mide

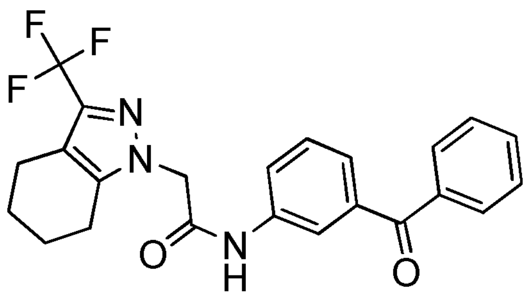

The titled compound was obtained in the same manner as in Example 325.
Yield: 9.3 mg
MS (ESI+) :428 (M+H)

Example 361

N-[1-(2-Fluorobenzyl)-1H-pyrazol-3-yl]-2-[3-(trifluoromethyl)-4,5,6,7-tetrahydro-1H-indazol-1-yl]acetamide

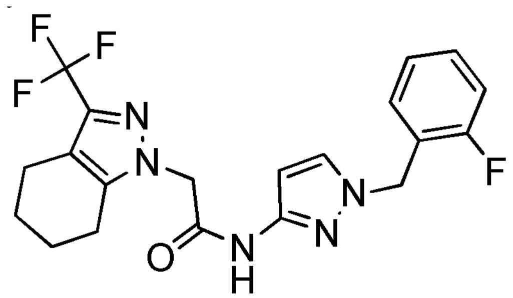

The titled compound was obtained in the same manner as in Example 325.
Yield: 18.4 mg
MS (ESI+) :422 (M+H)

Example 362

N-(1-Ethyl-3,5-dimethyl-1H-pyrazol-4-yl)-2-[3-(trifluoromethyl)-4,5,6,7-tetrahydro-1H-indazol-1-yl]acetamide

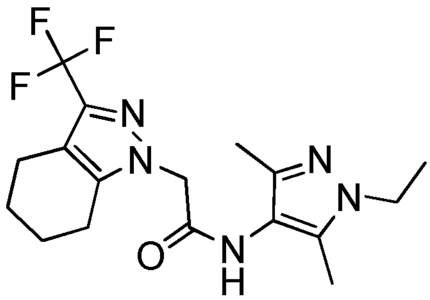

The titled compound was obtained in the same manner as in Example 325.
Yield: 12.3 mg
MS (ESI+) :370 (M+H)

Example 363

N-(4-Methoxybiphenyl-3-yl)-2-[3-(trifluoromethyl)-4,5,6,7-tetrahydro-1H-indazol-1-yl]acetami de

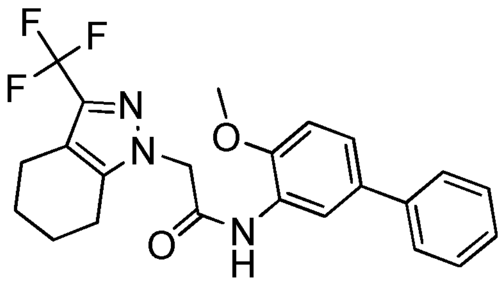

The titled compound was obtained in the same manner as in Example 325.
Yield: 7.7 mg
MS (ESI+) :430 (M+H)

Example 364

N-(2-Methoxy-5-methylphenyl)-2-[3-(trifluoromethyl)-4,5,6,7-tetrahydro-1H-indazol-1-yl]aceta mide

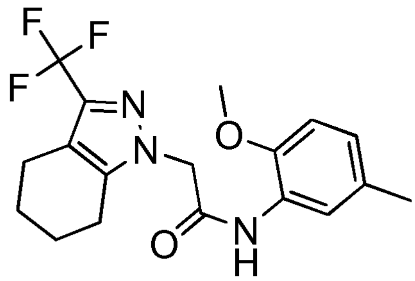

The titled compound was obtained in the same manner as in Example 325.
Yield: 8.8 mg
MS (ESI+) :368 (M+H)

Example 365

N-(3-Cyanophenyl)-2-[3-(trifluoromethyl)-4,5,6,7-tetrahydro-1H-indazol-1-yl]acetamide

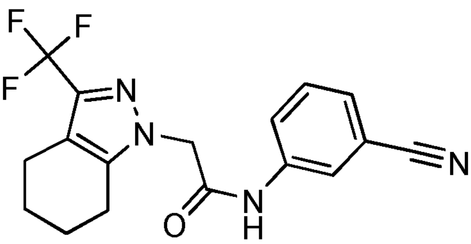

The titled compound was obtained in the same manner as in Example 325.
Yield: 3.0 mg
MS (ESI+) :349 (M+H)

Example 366

N-[3-(1-Hydroxyethyl)phenyl]-2-[3-(trifluoromethyl)-4,5,6,7-tetrahydro-1H-indazol-1-yl]aceta mide

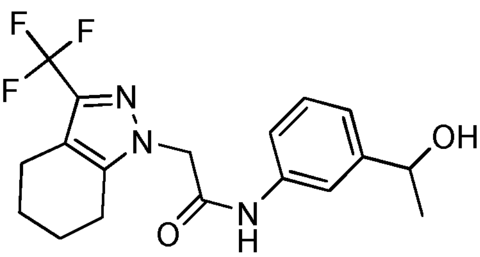

The titled compound was obtained in the same manner as in Example 325.

Yield: 3.5 mg
MS (ESI+) :368 (M+H)

Example 367

N-[3-(Trifluoromethoxy)phenyl]-2-[3-(trifluoromethyl)-4,5,6,7-tetrahydro-1H-indazol-1-yl]acet amide

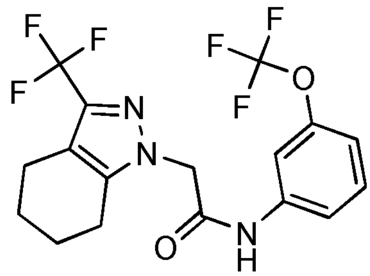

The titled compound was obtained in the same manner as in Example 325.
Yield: 1.4 mg
MS (ESI+) :408 (M+H)

Example 368

N-(3-Phenoxyphenyl)-2-[3-(trifluoromethyl)-4,5,6,7-tetrahydro-1H-indazol-1-yl]acetamide

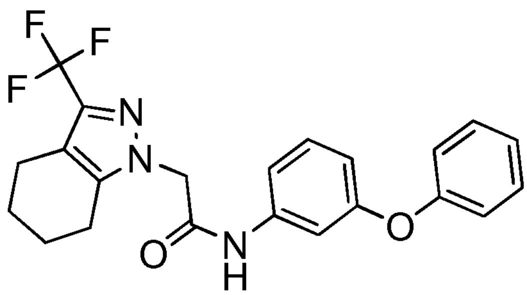

The titled compound was obtained in the same manner as in Example 325.
Yield: 3.8 mg
MS (ESI+) :416 (M+H)

Example 369

N-Biphenyl-3-yl-2-[3-(trifluoromethyl)-4,5,6,7-tetrahydro-1H-indazol-1-yl]acetamide

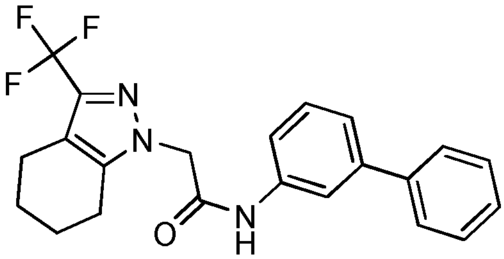

The titled compound was obtained in the same manner as in Example 325.
Yield: 5.0 mg
MS (ESI+) :400 (M+H)

Example 370

N-(Pyridin-2-ylmethyl)-2-[3-(trifluoromethyl)-4,5,6,7-tetrahydro-1H-indazol-1-yl]acetamide

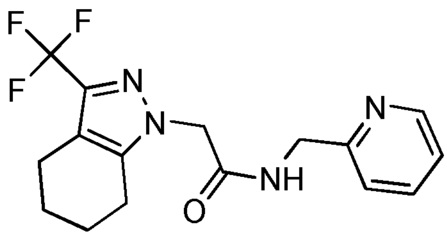

The titled compound was obtained in the same manner as in Example 325.
Yield: 5.2 mg
MS (ESI+) :339 (M+H)

Example 371

N-[4-(Dimethylamino)benzyl]-2-[3-(trifluoromethyl)-4,5,6,7-tetrahydro-1H-indazol-1-yl]acetam ide

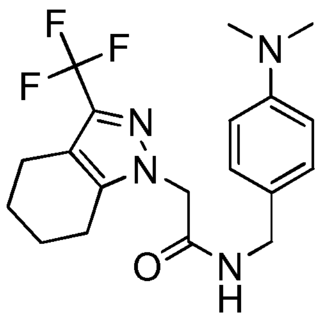

The titled compound was obtained in the same manner as in Example 325.
Yield: 5.9 mg
MS (ESI+) :381 (M+H)

Example 372

N-[4-(1-Methylethyl)benzyl]-2-[3-(trifluoromethyl)-4,5,6,7-tetrahydro-1H-indazol-1-yl]acetami de

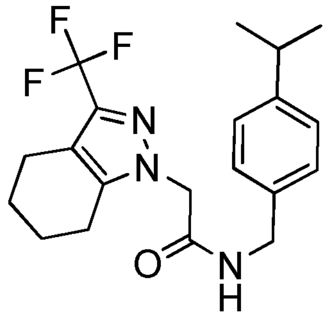

The titled compound was obtained in the same manner as in Example 325.
Yield: 5.9 mg
MS (ESI+) :380 (M+H)

Example 373

N-[4-(Trifluoromethoxy)benzyl]-2-[3-(trifluoromethyl)-4,5,6,7-tetrahydro-1H-indazol-1-yl]aceta mide

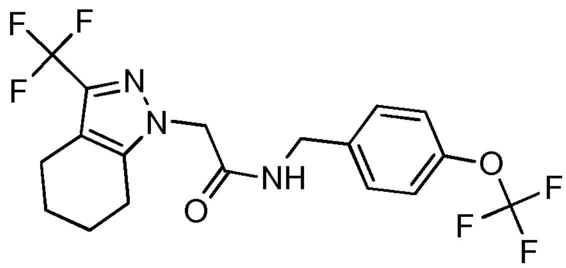

The titled compound was obtained in the same manner as in Example 325.
Yield: 8.6 mg
MS (ESI+) :422 (M+H)

Example 374

N-[3-(Trifluoromethyl)benzyl]-2-[3-(trifluoromethyl)-4,5,6,7-tetrahydro-1H-indazol-1-yl]aceta mide

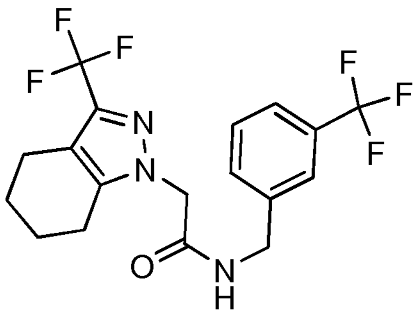

The titled compound was obtained in the same manner as in Example 325.
Yield: 10 mg
MS (ESI+) :406 (M+H)

Example 375

N-(3-Chlorobenzyl)-2-[3-(trifluoromethyl)-4,5,6,7-tetrahydro-1H-indazol-1-yl]acetamide

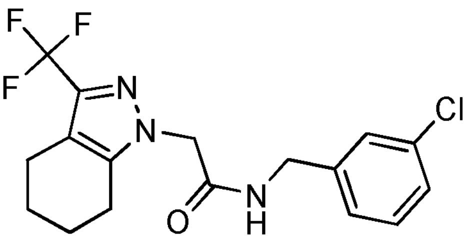

The titled compound was obtained in the same manner as in Example 325.
Yield: 6.3 mg
MS (ESI+) :372 (M+H)

Example 376

N-[3,5-Bis(trifluoromethyl)benzyl]-2-[3-(trifluoromethyl)-4,5,6,7-tetrahydro-1H-indazol-1-yl]ac etamide

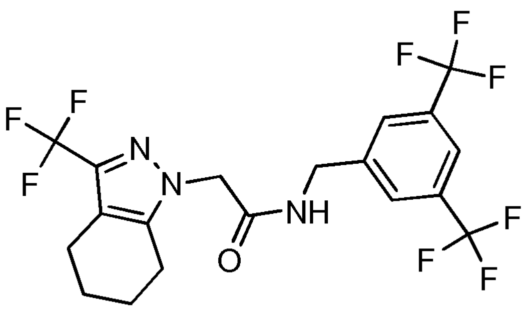

The titled compound was obtained in the same manner as in Example 325.
Yield: 8.5 mg
MS (ESI+) :474 (M+H)

Example 377

N-[3-Fluoro-5-(trifluoromethyl)benzyl]-2-[3-(trifluoromethyl)-4,5,6,7-tetrahydro-1H-indazol-1-yl]acetamide

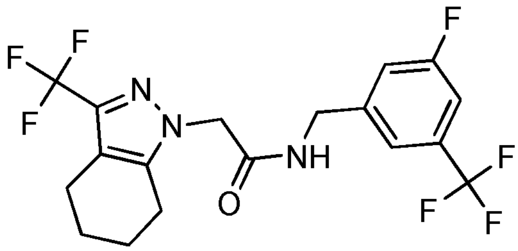

The titled compound was obtained in the same manner as in Example 325.
Yield: 5.1 mg
MS (ESI+) :424 (M+H)

Example 378

N-(3,5-Dimethylbenzyl)-2-[3-(trifluoromethyl)-4,5,6,7-tetrahydro-1H-indazol-1-yl]acetamide

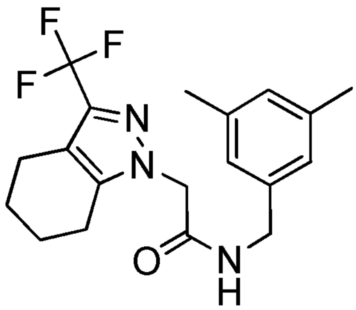

The titled compound was obtained in the same manner as in Example 325.
Yield: 5.7 mg
MS (ESI+) :366 (M+H)

Example 379

N-(3,4-Dichlorobenzyl)-2-[3-(trifluoromethyl)-4,5,6,7-tetrahydro-1H-indazol-1-yl]acetamide

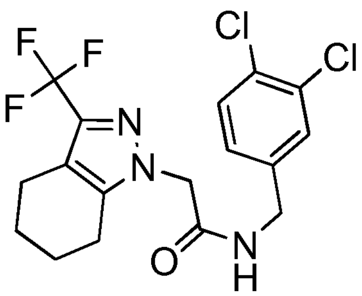

The titled compound was obtained in the same manner as in Example 325.
Yield: 6.4 mg
MS (ESI+) :406 (M+H)

Example 380

N-(2-Thiophen-2-ylethyl)-2-[3-(trifluoromethyl)-4,5,6,7-tetrahydro-1H-indazol-1-yl]acetamide

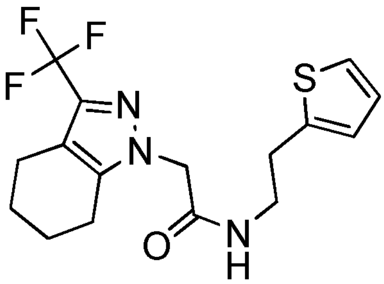

The titled compound was obtained in the same manner as in Example 325.
Yield: 3.5 mg
MS (ESI+) :358 (M+H)

Example 381

N-(2,5-Diethoxyphenyl)-2-[3-(trifluoromethyl)-4,5,6,7-tetrahydro-1H-indazol-1-yl]acetamide

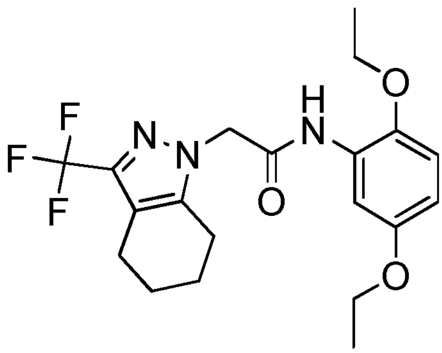

The titled compound was obtained in the same manner as in Example 325.
Yield: 3.9 mg
MS (ESI+) :412 (M+H)

Example 382

N-[3-(2-{[4-(Acetylamino)phenyl]amino}-1,3-thiazol-4-yl)phenyl]-2-[3-(trifluoromethyl)-4,5,6,7-tetrahydro-1H-indazol-1-yl]acetamide

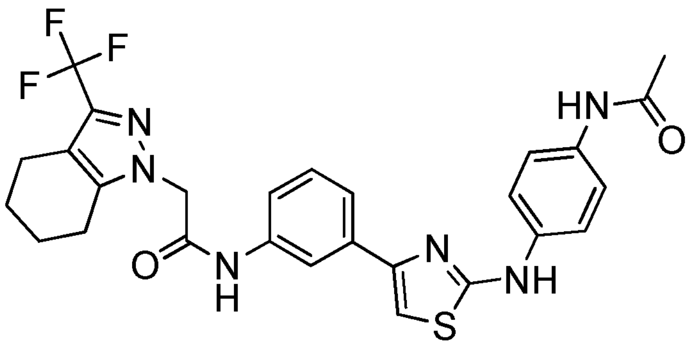

The titled compound was obtained in the same manner as in Example 325.
Yield: 4.9 mg
MS (ESI+) :555 (M+H)

Example 383

N-[3-(6,7,8,9-Tetrahydro-5H-[1,2,4]triazolo[4,3-a]azepin-3-yl)phenyl]-2-[3-(trifluoromethyl)-4,5 ,6,7-tetrahydro-1H-indazol-1-yl]acetamide

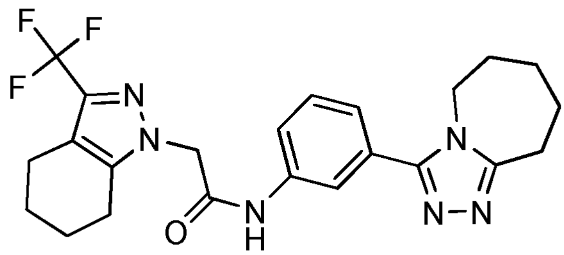

The titled compound was obtained in the same manner as in Example 325.
Yield: 3.9 mg
MS (ESI+) :459 (M+H)

Example 384

N-[3-(1,3-Benzothiazol-2-yl)phenyl]-2-[3-(trifluoromethyl)-4,5,6,7-tetrahydro-1H-indazol-1-yl]a cetamide

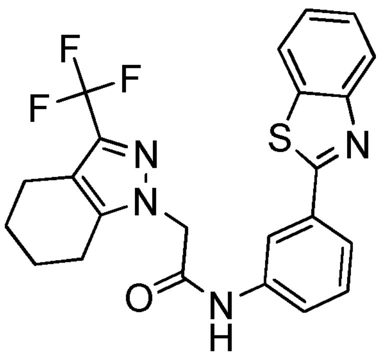

The titled compound was obtained in the same manner as in Example 325.
Yield: 3.4 mg
MS (ESI+) :457 (M+H)

Example 385

N-(2,1,3-Benzothiadiazol-4-ylmethyl)-2-[3-(trifluoromethyl)-4,5,6,7-tetrahydro-1H-indazol-1-yl ]acetamide

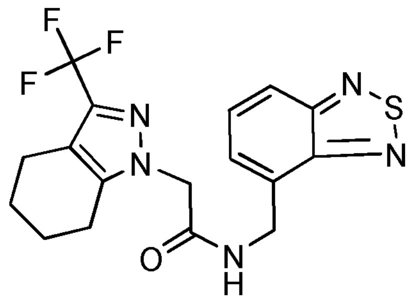

The titled compound was obtained in the same manner as in Example 325.
Yield: 1.0 mg
MS (ESI+) :396 (M+H)

Example 386

N-(1-Benzothiophen-7-ylmethyl)-2-[3-(trifluoromethyl)-4,5,6,7-tetrahydro-1H-indazol-1-yl]acet amide

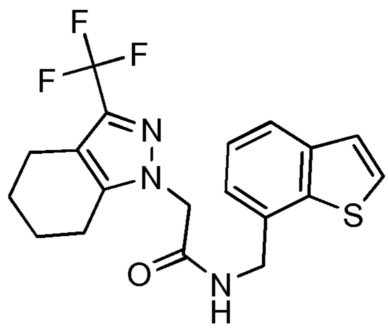

The titled compound was obtained in the same manner as in Example 325.
Yield: 5.0 mg
MS (ESI+) :394 (M+H)

Example 387

N-(1-Benzothiophen-4-ylmethyl)-2-[3-(trifluoromethyl)-4,5,6,7-tetrahydro-1H-indazol-1-yl]acet amide

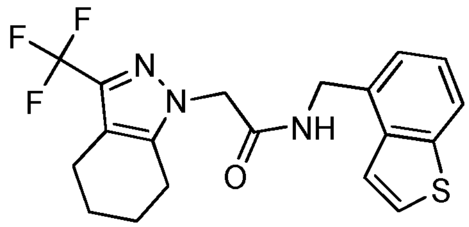

The titled compound was obtained in the same manner as in Example 325.
Yield: 5.9 mg
MS (ESI+) :394 (M+H)

Example 388

N-(1-Benzothiophen-3-ylmethyl)-2-[3-(trifluoromethyl)-4,5,6,7-tetrahydro-1H-indazol-1-yl]acet amide

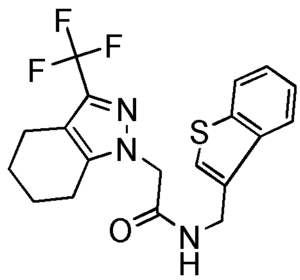

The titled compound was obtained in the same manner as in Example 325.
Yield: 5.2 mg
MS (ESI+) :394 (M+H)

Example 389

N-{[5-(4-Chlorophenyl)thiophen-2-yl]methyl}-2-[3-(trifluoromethyl)-4,5,6,7-tetrahydro-1H-inda zol-1-yl]acetamide

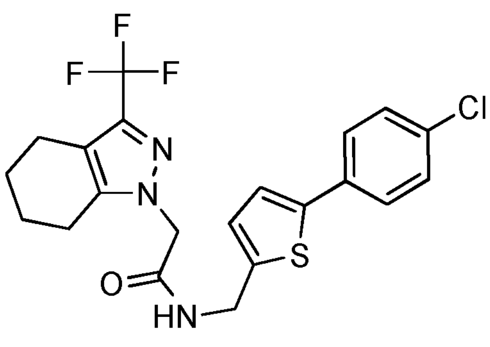

The titled compound was obtained in the same manner as in Example 325.
Yield: 2.5 mg
MS (ESI+) :454 (M+H)

Example 390

N-(1-Thiophen-2-ylcyclopropyl)-2-[3-(trifluoromethyl)-4,5,6,7-tetrahydro-1H-indazol-1-yl]aceta mide

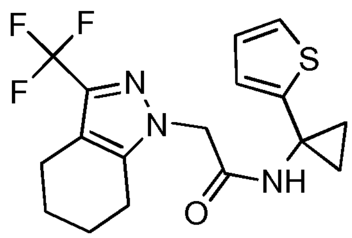

The titled compound was obtained in the same manner as in Example 325.
Yield: 5.6 mg
MS (ESI+) :370 (M+H)

Example 391

N-{3-[(Dimethylamino)methyl]phenyl}-2-[3-(trifluoromethyl)-4,5,6,7-tetrahydro-1H-indazol-1-yl]acetamide

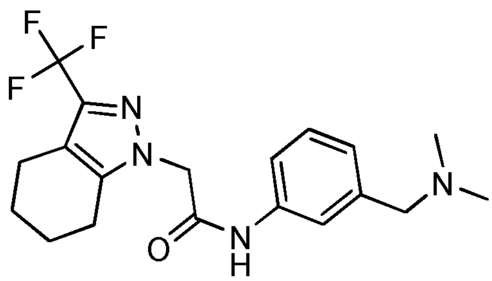

The titled compound was obtained in the same manner as in Example 325.
Yield: 3.0 mg
MS (ESI+) :381 (M+H)

Example 392

N-(3-Chloro-4-methylbenzyl)-2-[3-(trifluoromethyl)-4,5,6,7-tetrahydro-1H-indazol-1-yl]acetami de

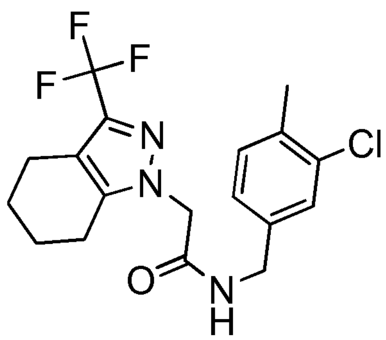

The titled compound was obtained in the same manner as in Example 325.
Yield: 6.0 mg
MS (ESI+) :386 (M+H)

Example 393

N-[3-(Methoxymethyl)benzyl]-2-[3-(trifluoromethyl)-4,5,6,7-tetrahydro-1H-indazol-1-yl]acetam ide

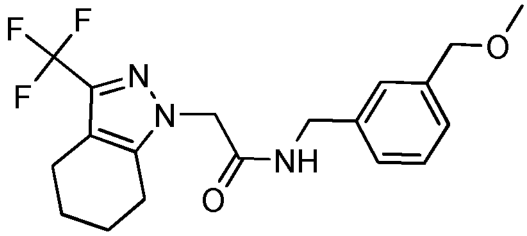

The titled compound was obtained in the same manner as in Example 325.
Yield: 5.7 mg
MS (ESI+) :382 (M+H)

Example 394

N-[3-(2,2,2-Trifluoroethoxy)benzyl]-2-[3-(trifluoromethyl)-4,5,6,7-tetrahydro-1H-indazol-1-yl]a cetamide

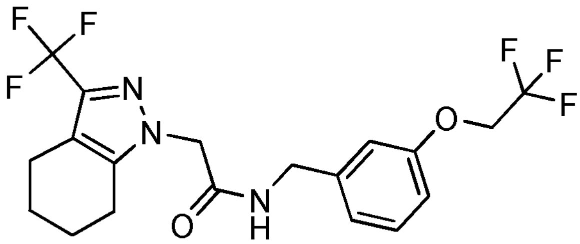

The titled compound was obtained in the same manner as in Example 325.
Yield: 7.1 mg
MS (ESI+) :436 (M+H)

Example 395

N-[3-(Cyclopropylmethoxy)benzyl]-2-[3-(trifluoromethyl)-4,5,6,7-tetrahydro-1H-indazol-1-yl]ac etamide

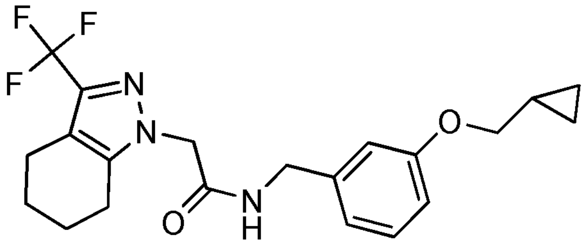

The titled compound was obtained in the same manner as in Example 325.
Yield: 6.8 mg
MS (ESI+) :408 (M+H)

Example 396

N-(1-Benzofuran-5-ylmethyl)-2-[3-(trifluoromethyl)-4,5,6,7-tetrahydro-1H-indazol-1-yl]acetam ide

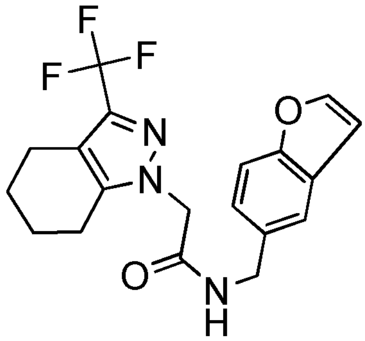

The titled compound was obtained in the same manner as in Example 325.
Yield: 5.4 mg
MS (ESI+) :378 (M+H)

Example 397

N-[(1-Methyl-1H-benzotriazol-5-yl)methyl]-2-[3-(trifluoromethyl)-4,5,6,7-tetrahydro-1H-indazo l-1-yl]acetamide

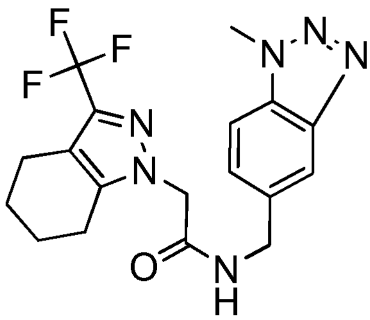

The titled compound was obtained in the same manner as in Example 325.
Yield: 2.8 mg
MS (ESI+) :393 (M+H)

Example 398

N-[3-(4-Methylpiperazin-1-yl)benzyl]-2-[3-(trifluoromethyl)-4,5,6,7-tetrahydro-1H-indazol-1-yl ]acetamide

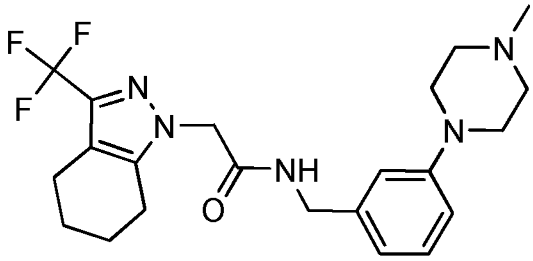

The titled compound was obtained in the same manner as in Example 325.
Yield: 6.4 mg
MS (ESI+) :436 (M+H)

Example 399

N-(3-Pyridin-2-ylbenzyl)-2-[3-(trifluoromethyl)-4,5,6,7-tetrahydro-1H-indazol-1-yl]acetamide

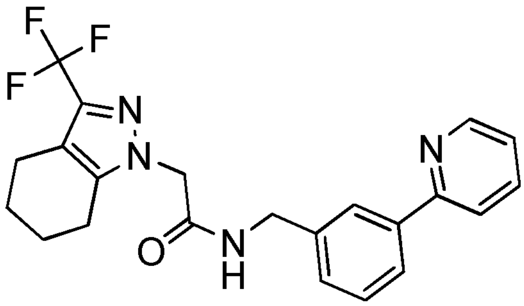

The titled compound was obtained in the same manner as in Example 325.
Yield: 2.0 mg
MS (ESI+) :415 (M+H)

Example 400

N-[(1,4-Dimethyl-1,2,3,4-tetrahydroquinoxalin-6-yl)methyl]-2-[3-(trifluoromethyl)-4,5,6,7-tetra hydro-1H-indazol-1-yl] acetamide

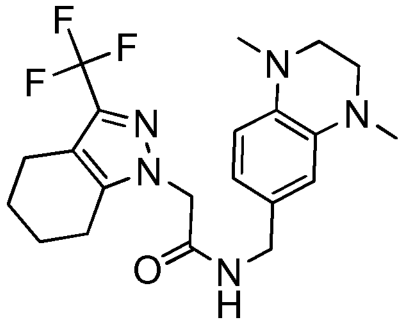

The titled compound was obtained in the same manner as in Example 325.
Yield: 4.4 mg
MS (ESI+) :422 (M+H)

Example 401

N-[3-(Pyridin-2-yloxy)benzyl]-2-[3-(trifluoromethyl)-4,5,6,7-tetrahydro-1H-indazol-1-yl]acetam ide

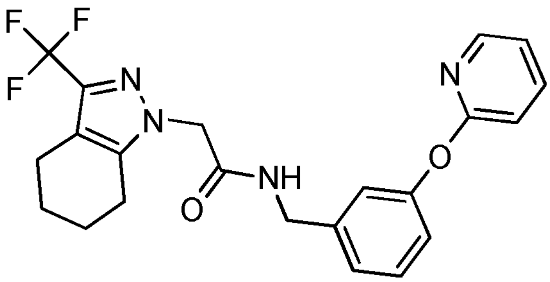

The titled compound was obtained in the same manner as in Example 325.
Yield: 6.9 mg
MS (ESI+) :431 (M+H)

Example 402

N-[4-(Pyridin-2-yloxy)benzyl]-2-[3-(trifluoromethyl)-4,5,6,7-tetrahydro-1H-indazol-1-yl]acetam ide

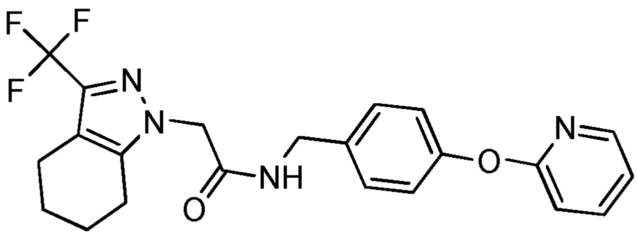

The titled compound was obtained in the same manner as in Example 325.
Yield: 6.2 mg
MS (ESI+) :431 (M+H)

Example 403

N-[2-(Pyridin-2-yloxy)benzyl]-2-[3-(trifluoromethyl)-4,5,6,7-tetrahydro-1H-indazol-1-yl]acetam ide

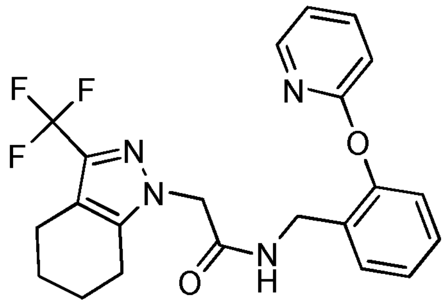

The titled compound was obtained in the same manner as in Example 325.
Yield: 6.4 mg
MS (ESI+) :431 (M+H)

Example 404

N-(1-Benzothiophen-2-ylmethyl)-2-[3-(trifluoromethyl)-4,5,6,7-tetrahydro-1H-indazol-1-yl]acet amide

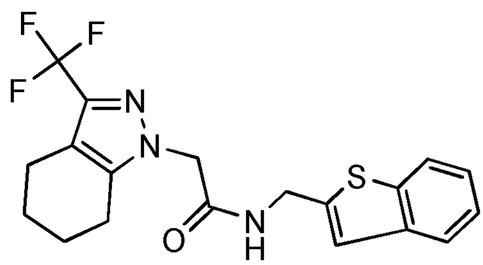

The titled compound was obtained in the same manner as in Example 325.
Yield: 6.3 mg
MS (ESI+) :394 (M+H)

Example 405

N-Pyridin-4-yl-2-[3-(trifluoromethyl)-4,5,6,7-tetrahydro-1H-indazol-1-yl]acetamide

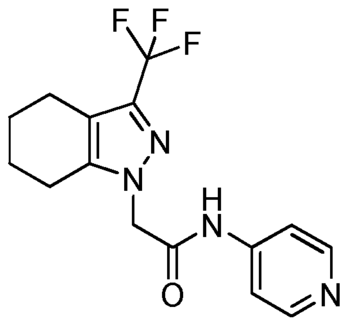

A solution of [3-(trifluoromethyl)-4,5,6,7-tetrahydro-1H-indazol-1-yl]acetic acid (0.248 g, 1.00 mmol), 4-aminopyridine (0.104 g, 1.10 mmol), HATU (0.418 g, 1.10 mmol) in N,N-dimethylfor mamide (5 ml) was stirred at room temperature overnight. Water was added thereto, and the mixture was extracted with ethyl acetate. The organic layer was washed with saturated sodiu m hydrogen carbonate aqueous solution, and saturated saline, dried over sodium sulfate, and concentrated under reduced pressure. The obtained residue was purified by column chromato graphy on basic silica gel [developing solvent: ethyl acetate], and the obtained solid was recrys tallized from ethyl acetate-hexane to give the titled compound (0.18 g) as colorless crystals (yi eld 56%).
MS (ESI+) :325 (M+H)
1H NMR (300 MHz, $CDCl_3$) δ ppm 1.72 - 1.83 (2 H, m), 1.83 - 1.94 (2 H, m), 2.63 (4 H, t, J = 6. 0 Hz), 4.82 (2 H, s), 7.40 (2 H, dd, J = 4.7, 1.5 Hz), 8.51 (2 H, dd, J = 4.8, 1.6 Hz), 8.87 (1 H, br. s.).

Example 406

1-Methyl-5-({[3-(trifluoromethyl)-4,5,6,7-tetrahydro-1H-indazol-1-yl]acetyl}amino)-1H-pyrazol e-4-carboxamide

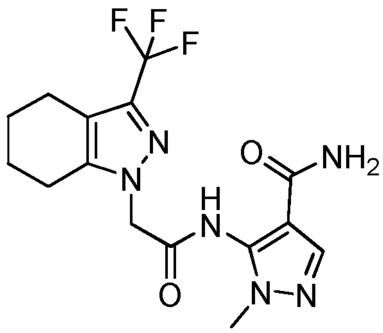

To a solution of [3-(trifluoromethyl)-4,5,6,7-tetrahydro-1H-indazol-1-yl]acetic acid (0.20 g, 0.81 mmol) in tetrahydrofuran (3 ml), were added oxalyl chloride (0.14 ml, 1.61 mmol), and N,N-di methylformamide (1 drop), and the mixture was stirred at room temperature for 1 hour. The r eaction solution was concentrated under reduced pressure, N,N-dimethylacetamide (3 ml) was added thereto, and cooled to 0 °C. 5-Amino-1-methyl-1H-pyrazole-4-carboxamide (0.14 g, 0.97 mmol) was added thereto, and the mixture was stirred at room temperature overnight. Satura ted saline was added thereto, and the mixture was extracted with ethyl acetate-tetrahydrofur an(1:1). The organic layer was washed with saturated saline, dried over sodium sulfate, and c oncentrated under reduced pressure. The obtained residue was recrystallized from diisopropyl alcohol-ethyl acetate, and recrystallized again from hexane-ethyl acetate to give the titled co mpound (49 mg, yield 16%) as colorless crystals.
MS (ESI+) :371 (M+H)
1H NMR (300 MHz, DMSO-$d_6$) δ ppm 1.61 - 1.81 (4 H, m), 2.49 - 2.66 (4 H, m), 3.58 (3 H, s), 5. 11 (2 H, s), 7.10 (1 H, br. s.), 7.40 (1 H, br. s.), 7.85 (1 H, s), 10.39 (1 H, br. s.).

Example 407

N-Methyl-2-({[3-(trifluoromethyl)-4,5,6,7-tetrahydro-1H-indazol-1-yl]acetyl}amino)benzamide

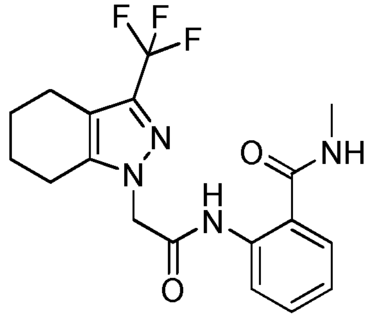

To a solution of [3-(trifluoromethyl)-4,5,6,7-tetrahydro-1H-indazol-1-yl]acetic acid (0.204 g, 0.8 2 mmol) in tetrahydrofuran (5 ml), oxalyl chloride (0.139 ml, 1.64 mmol), and N,N-dimethylfor mamide (1 drop) were added, and stirred at room temperature for 1 hour. The reaction solutio n was concentrated under reduced pressure, N,N-dimethylacetamide (5 ml) was added thereto , and cooled to 0 °C. 2-Amino-N-methylbenzamide (0.134 g, 0.98 mmol) was added thereto, and the mixture was stirred at room temperature overnight. Water was added thereto, and the mi xture was extracted with ethyl acetate. The organic layer was washed with water, and saturat ed saline, dried over sodium sulfate, and concentrated under reduced pressure. The obtained r esidue was purified by basic silica gel chromatography [developing solvent: hexane-ethyl aceta te (9:1-7:3)]. The obtained solid was recrystallized from hexane-ethyl acetate to give the titled compound (82 mg, yield 26%) as colorless crystals.
MS (ESI+) :381 (M+H)
1H NMR (300 MHz, $CDCl_3$) δ ppm 1.71 - 1.93 (4 H, m), 2.57 - 2.69 (4 H, m), 2.93 (3 H, d, J = 4. 5 Hz), 4.88 (2 H, s), 6.15 (1 H, br. s.), 7.05 - 7.12 (1 H, m), 7.37 - 7.42 (1 H, m), 7.42 - 7.50 (1 H, m), 8.55 (1 H, d, J = 8.3 Hz), 10.93 - 11.28 (1 H, m) .

Example 408

N,N-dimethyl-2-({[3-(trifluoromethyl)-4,5,6,7-tetrahydro-1H-indazol-1-yl]acetyl}amino)benzam ide

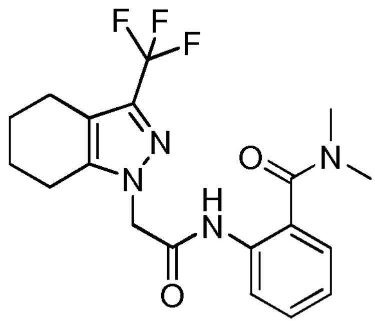

To a solution of [3-(trifluoromethyl)-4,5,6,7-tetrahydro-1H-indazol-1-yl]acetic acid (0.208 g, 0.8 4 mmol) in tetrahydrofuran (5 ml), were added oxalyl chloride (0.142 ml, 1.68 mmol), and N, N-dimethylformamide (1 drop), and stirred at room temperature for 1 hour. The reaction solut ion was concentrated under reduced pressure, N,N-dimethylacetamide (3 ml) was added there to, and cooled to 0 °C. 2-Amino-N,N-dimethylbenzamide (0.166 g, 1.01 mmol) was added theret o, and the mixture was stirred at room temperature overnight. Water was added thereto, and the mixture was extracted with ethyl acetate. The organic layer was washed with water, and s aturated saline, dried over sodium sulfate, and concentrated under reduced pressure. The obt ained residue was purified by silica gel chromatography [developing solvent: hexane-ethyl ace tate (9:1-1:1)]. The obtained solid was recrystallized from hexane-ethyl acetate to give the title d compound (217 mg, yield 66%) as colorless crystals.

MS (ESI+) :395 (M+H)
$^1$H NMR (300 MHz, $CDCl_3$) δ ppm 1.75 - 1.94 (4 H, m), 2.58 - 2.70 (4 H, m), 2.94 - 3.09 (6 H, m ), 4.85 (2 H, s), 7.07 - 7.14 (1 H, m), 7.19 - 7.23 (1 H, m), 7.34 - 7.43 (1 H, m), 8.27 - 8.32 (1 H, m), 9.01 (1 H, br. s.)

Example 409

3-Methyl-5-({[3-(trifluoromethyl)-4,5,6,7-tetrahydro-1H-indazol-1-yl]acetyl}amino)isoxazole-4-c arboxamide

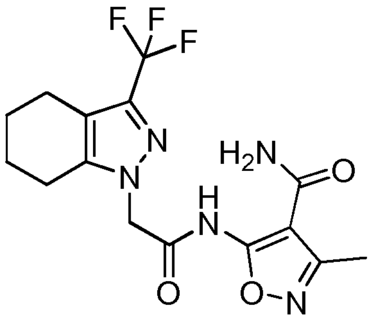

To a solution of [3-(trifluoromethyl)-4,5,6,7-tetrahydro-1H-indazol-1-yl]acetic acid (0.207 g, 0.8 3 mmol) in tetrahydrofuran (5 ml), were added oxalyl chloride (0.141 ml, 1.67 mmol), and N, N-dimethylformamide (1 drop), and the mixture was stirred for 1 hour at room temperature. T he reaction solution was concentrated under reduced pressure, N,N-dimethylacetamide (3 ml) was added thereto, and cooled to 0 °C. 2-Amino-N,N-dimethylbenzamide (0.140 g, 1.00 mmol) was added thereto, and the mixture was stirred at room temperature overnight. Water was ad ded thereto, and the mixture was extracted with ethyl acetate. The organic layer was washed with saturated saline, dried over sodium sulfate, and concentrated under reduced pressure. T he obtained residue was purified by silica gel chromatography [developing solvent: hexane-eth yl acetate (9:1-1:9)], and following basic silica gel chromatography [developing solvent: hexan e-ethyl acetate (9:1-1:9)]. The obtained solid was recrystallized from hexane-ethyl acetate to gi ve the titled compound (17 mg, yield 6%) as colorless crystals.
MS (ESI+) :372 (M+H)
1H NMR (300 MHz, $CDCl_3$) δ ppm 1.73 - 1.92 (4 H, m), 2.45 (3 H, s), 2.56 - 2.67 (4 H, m), 5.03 ( 2 H, s), 5.87 (2 H, br. s.), 10.77 (1 H, br. s.)

Example 410

3-({[3-(Trifluoromethyl)-4,5,6,7-tetrahydro-1H-indazol-1-yl]acetyl}amino)-1-benzofuran-2-carb oxamide

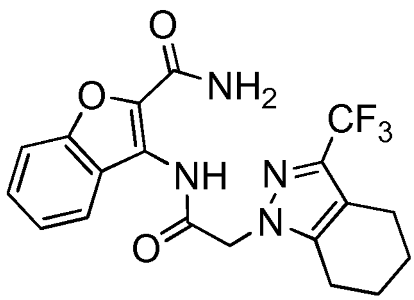

A mixture of [3-(trifluoromethyl)-4,5,6,7-tetrahydro-1H-indazol-1-yl]acetic acid (86 mg, 0.488 mmol), 3-amino-1-benzofuran-2-carboxamide (133 mg, 0.537 mmol), HATU (223 mg, 0.586 m mol), diisopropylamine (0.13 ml, 0.732 mmol), and DMF (1 ml) was stirred at room temperatu re for 60 hours. HATU (223 mg, 0.586 mmol), and diisopropylamine (0.13 ml, 0.732 mmol) was added thereto. The mixture was stirred at room temperature for 15 hours. To the reaction mi xture, was added water, and the mixture was extracted with ethyl acetate. The organic layer was saturated saline, and washed

with water, dried over sodium sulfate, and concentrated un der reduced pressure. The obtained crystals were washed with ethyl acetate-diisopropyl ether to give the titled compound (90 mg) (yield 45%).
MS (ESI+) :407 (M+H).
$^1$H NMR (300 MHz, $CDCl_3$) δ ppm 1.69 - 2.05 (4 H, m), 2.55 - 2.80 (2 H, m), 5.01 (2 H, s), 6.01 ( 2 H, br. s.), 7.19 - 7.33 (1 H, m), 7.35 - 7.49 (2 H, m), 8.38 (1 H, d, J=8.0 Hz), 9.99 (1 H, s).

Example 411

N-(5-Chloro-2,2-dimethyl-2,3-dihydro-1-benzofuran-7-yl)-2-[3-(trifluoromethyl)-4,5,6,7-tetrahy dro-1H-indazol-1-yl] acetamide

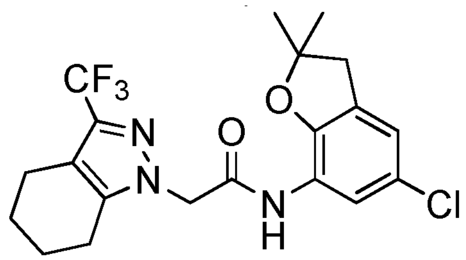

A mixture of [3-(trifluoromethyl)-4,5,6,7-tetrahydro-1H-indazol-1-yl]acetic acid (99 mg, 0.398 mmol), 5-chloro-2,2-dimethyl-2,3-dihydro-1-benzofuran-7-amine (75 mg, 0.379 mmol), WSC (8 7 mg, 0.455 mmol), HOBt (61 mg, 0.455 mmol), triethylamine (0.13 ml, 0.948 mmol), and DM F (1 ml) was stirred at room temperature for 5 hours. To the reaction mixture, was added wat er, and the mixture was extracted with ethyl acetate. The organic layer was washed with sali ne, and saturated saline, dried over sodium sulfate, and concentrated under reduced pressure. The obtained crystals were washed with diisopropyl ether-hexane to give the titled compound (105 mg) (yield 62%).
MS (ESI+) :428 (M+H).
$^1$H NMR (300 MHz, $CDCl_3$) δ ppm 1.43 (6 H, s), 1.82 (4 H, m, J=15.3, 15.3, 15.1, 5.9 Hz), 2.62 ( 4 H, t, J=6.1 Hz), 2.98 (2 H, s), 4.81 (2 H, s), 6.85 (1 H, d, J=2.1 Hz), 8.08 (1 H, d, J=2.1 Hz), 8. 39 (1 H, br. s.).

Reference Example 412

N-(1-Phenylethyl)-2-[3-(trifluoromethyl)-4,5,6,7-tetrahydro-1H-indazol-1-yl]acetamide

The title compound was obtained in the same manner as in Example 3.
Yield: 9.0 mg
MS (ESI+) :352 (M+H)

Reference Example 413

N-(1-Methyl-1-phenylethyl)-2-[3-(trifluoromethyl)-4,5,6,7-tetrahydro-1H-indazol-1-yl]acetamid e

The title compound was obtained in the same manner as in Example 3.
Yield: 13.4 mg
MS (ESI+) :366 (M+H)

Reference Example 414

N-(1-Phenylpropyl)-2-[3-(trifluoromethyl)-4,5,6,7-tetrahydro-1H-indazol-1-yl]acetamide

The title compound was obtained in the same manner as in Example 3.
Yield: 12.8 mg
MS (ESI+) :366 (M+H)

Reference Example 415

N-[2-(Dimethylamino)-1-phenylethyl]-2-[3-(trifluoromethyl)-4,5,6,7-tetrahydro-1H-indazol-1-yl ]acetamide

The title compound was obtained in the same manner as in Example 3.
Yield: 22.9 mg
MS (ESI+) :395 (M+H)

Preparation Example 1

(1) Compound of Working Example 1 10.0 g
(2) Lactose 70.0 g
(3) Corn starch 50.0 g
(4) Soluble starch 7.0 g
(5) Magnesium stearate 3.0 g

The compound of Working Example 1 (10.0 g) and magnesium stearate (3.0 g) are granulated in a 70 mL aqueous solution of soluble starch (7.0 g as soluble starch), and the granules are t hen dried and mixed with the lactose (70.0 g) and corn starch (50.0 g) (the lactose, corn starch, soluble starch, and magnesium stearate should all be compliant with the Japanese Pharmaco poeia, Fourteenth Edition). The mixture is compressed to obtain tablets.

Preparation Example 2

(1) Compound of Working Example 3 10.0 g
(2) Lactose 70.0 g
(3) Corn starch 50.0 g
(4) Soluble starch 7.0 g
(5) Magnesium stearate 3.0 g

The compound of Working Example 3 (10.0 g) and magnesium stearate (3.0 g) are granulated in a 70 mL aqueous solution of soluble starch (7.0 g as soluble starch), and the granules are t hen dried and mixed with the lactose (70.0 g) and corn starch (50.0 g) (the lactose, corn starch, soluble starch, and magnesium stearate should all be compliant with the Japanese Pharmaco poeia, Fourteenth Edition). The mixture is compressed to obtain tablets.

Test Example 1

(1) Construction of expression genes

Human GluR1 flip cDNA was amplified by PCR using the forward primer ACTGAATTCGCC ACCATGCAGCACATTTTTGCCTTCTTCTGC (SEQ ID: 1), and the reverse primer CCGCGGC CGCTTACAATCCCGTGGCTCCCAAG (SEQ ID: 2), which had been artificially synthesized u sing human brain-derived cDNA (BD Bioscience) as template. The amplified product was dige sted with the restriction enzymes EcoRI and NotI (Takara Shuzo Co., Ltd.), and was then inco rporated at the same site of pcDNA3.1(+) (Invitrogen) to construct the pcDNA3.1(+)/human Gl uR1 flip gene. Human stargazin cDNA was amplified by PCR using the forward primer GGTC TCGAGGCCACCATGGGGCTGTTTGATCGAGGTGTTCA (SEQ ID: 3), and the reverse prime r GTTGGATCCTTATACGGGGGTGGTCCGGCGGTTGGCTGTG (SEQ ID: 4), which had been artificially synthesized using human hippocampal cDNA as template. The amplified product was digested with the restriction enzymes XhoI and BamHI (Takara Shuzo Co., Ltd.), and wa s then incorporated at the same site of pcDNA3.1(-) (Invitrogen) to construct the pcDNA3.1 Ze o(-)/human stargazin gene.

(2) Construction of cells expressing GluR1 flip/stargazin

CHO-K1 cells passaged in culture media (Ham's F12 media (Invitrogen) supplemented with 10% inactivated fetal bovine serum (Morgate), penicillin, and streptomycin (Invitrogen)) were separated using 0.05% trypsin diluted with D-PBS(-), and 0.53 mM EDTA (Invitrogen). The se parated cells were suspended in culture media and harvested by

centrifugation at 1,000 rpm. The harvested cells were re-suspended in D-PBS(-) and introduced into a 0.4 cm electroporatio n cuvette (BioRad). The pcDNA3.1(+)/human GluR1 flip gene (5 μg) and pcDNA3.1 Zeo(-)/hum an stargazin gene (15 μg) were added, and were introduced into the CHO-K1 cells using a Gen e Pulser II (BioRad) at 950 μFd and 250 mV. The cells were incubated overnight in culture me dia, and on the next day 96-well plates were seeded with 250 cells/well using selection media ( Zeocin (Invitrogen) in a concentration of 250 μg/mL in culture media). Drug resistant clones w ere selected, and clones expressing GluR1 flip/stargazin were selected by the following assay u sing calcium influx as an indicator.

(3) Assay of AMPA receptor potentiation by compounds, using calcium influx as an indicator

96-well black-bottomed clear plates (Costar) were seeded with the CHO-K1/GluR1 flip/starga zin expressing cells ($2x10^4$ cells/well), and were incubated for 2 days at 37°C in a $CO_2$ incubat or (Sanyo). The cell plate media was removed, and assay buffer A (D-MEM (Invitrogen), 0.1% BSA (Serological Protein, Inc.), and 20 mM HEPES (Invitrogen)) was added to a concentration of 50 μL/well. A calcium indicator (Calcium Kit II-Fluo4 for TKB, Dojindo Laboratories) conta ining 2.5 mM probenecid (Dojindo Laboratories) was added to a concentration of 50 μL/well, a nd the plates were allowed to stand for 1 hour at 37°C in a $CO_2$ incubator. The cell plates wer e set up on a CellLux (PerkinElmer), a 50 μL mixture of 9 mM glutamic acid diluted (final con centration 3 mM) with assay buffer B (HBSS (Invitrogen), 0.1% BSA, and 20 mM HEPES) an d the compound to be tested (concentration of compound to be detected: 30 μM) was added, an d the change in the level of fluorescence over a period of 3 minutes was determined. The comp ound activity was calculated using the following equation, wherein the change in fluorescence in wells containing glutamic acid (final concentration 3 mM) and 300 μM cyclothiazide (TOCR IS) was defined as 100%, and the change in fluorescence in wells containing only glutamic aci d (final concentration 3 mm) was defined as 0%.

$$\text{Activity (\%)} = (X\text{-}C) / (T\text{-}C) \times 100$$

T: change in fluorescence in wells containing glutamic acid (final concentration 3 mM) and 30 0 μM cyclothiazide
C: change in fluorescence in wells containing only glutamic acid (final concentration 3 mM)
X: change in fluorescence in wells containing compound to be detected

The test results were shown in the following table.

| Example No. | % |
|---|---|
| 145 | 85 |
| 185 | 88 |
| 190 | 70 |
| 196 | 80 |
| 226 | 74 |
| 229 | 78 |
| 272 | 86 |
| 276 | 79 |
| 277 | 75 |
| 278 | 82 |
| 279 | 62 |
| 280 | 86 |
| 283 | 76 |
| 287 | 68 |

[Industrial Applicability]

The compounds of the present invention are useful as a drug for preventing or treating depre ssion, schizophrenia, attention-deficit hyperactivity disorder (ADHD), or so on.

## Claims

**1.** A compound represented by the formula

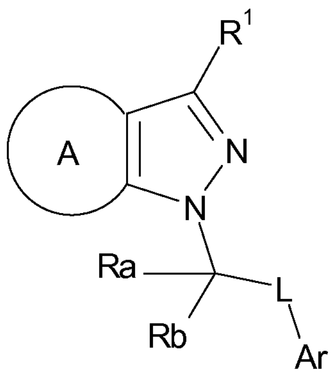

(I)

wherein
$R^1$ represents

(1) a halogen atom,
(2) cyano group,
(3) alkyl group optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carbamoyl group, and
mono- or di-alkylcarbamoyl groups,
(4) optionally substituted cycloalkyl group,

(5) group represented by the formula:

$-OR^{x1}$,
$-SR^{x2}$,
$-CO-R^{x2}$,
$-CS-R^{x2}$,
$-SO-R^{x2}$,
$-SO_2-R^{x2}$,
$-NH_2$, or
$-NR^{x1}R^{x2}$

(where $R^{x1}$ represents a substituent selected from the following substituent group A, and $R^{x2}$ represents hydrogen or a substituent selected from the following substituent group B),
or
(6) optionally substituted non-aromatic heterocyclic group;

Ra and Rb each independently represent a hydrogen atom or $C_{1-4}$ alkyl group;
L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1), either of which is optionally substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) optionally substituted cycloalkyl groups,
(e) groups represented by the formula:

$-OR^{y1}$,
$-SR^{y1}$,
$-CO- R^{y1}$,
$-CS- R^{y1}$,
$-SO- R^{y1}$,
$-SO_2-R^{y1}$, or
$-NR^{y1}R^{y2}$

(where $R^{y1}$ and $R^{y2}$ each independently represent a hydrogen atom or 1 or 2 substituents selected from the following substituent group A),

(f) oxo group, and
(g) optionally substituted non-aromatic heterocyclic groups;

Ar represents
a substituted phenyl group, or
optionally substituted 5- or 6-membered aromatic heterocyclic group
(when the phenyl group or aromatic heterocyclic group has 2 or more substituents, two adjacent substituents together may form an optionally substituted 5- to 8-membered ring).
Provided that, when L is a bond, Ar is a substituted phenyl group or substituted 5- or 6-membered aromatic heterocyclic group (when the phenyl group or aromatic heterocyclic group has 2 or more substituents, two adjacent substituents together may form an optionally substituted 5- to 8-membered ring).
Substituent group A consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,
(xii) $C_{3-8}$ cycloalkenyl groups,
(xiii) cross-linked $C_{7-10}$ cycloalkyl groups optionally substituted with $C_{1-6}$ alkyl group(s),
(xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfinyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.

Substituent group B consists of the groups of substituent group A except for $C_{1-6}$ alkoxy groups optionally substituted with halogen atom(s), and 6- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.
(Provided that

(1) the compounds in which $R^1$ is -CO-NHR$^t$ (wherein $R^t$ is an optionally substituted $C_4$ or higher hydrocarbon group.);
(2) compounds represented by the formula

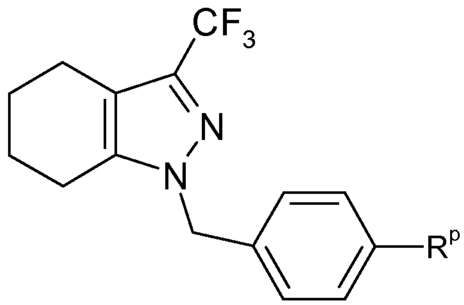

[wherein $R^p$ represents a substituent.];
(3) compounds represented by the formula

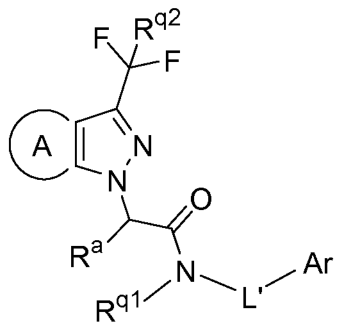

[wherein
$R^{q2}$ represents a hydrogen atom or fluorine atom,
$R^{q1}$ represents a hydrogen atom or substituent,
L' represents a bond, or a spacer in which the number of atoms in the main chain is 1 to 6, Ring A represents an optionally substituted non-aromatic carbon ring of 4-8 carbon atoms, and the other symbols are synonymous with the above.];
(4) compounds represented by the formula

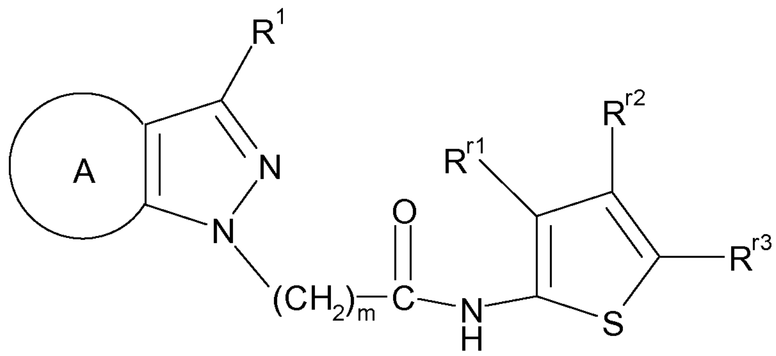

[wherein
$R^1$ represents trifluoromethyl,
$R^{r1}$ represents hydroxymethyl, carboxyl, or optionally substituted carbamoyl,
$R^{r2}$ and $R^{r3}$ may each independently represent hydrogen, $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur,
and oxygen atoms,
Ring A represents cyclohexene, and
m represents the integer 2.]; and
(5) compounds represented by the formulas

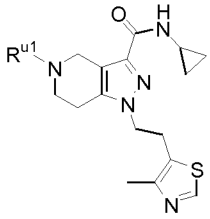
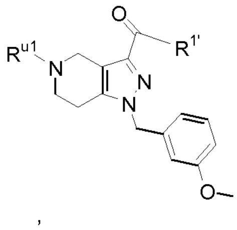
,
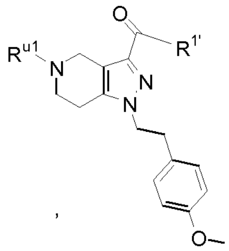
,
or
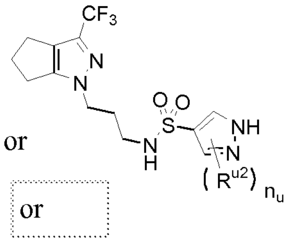
or

[wherein
$R^{1'}$ represents a dimethylamino group, monoethylamino group, or monocyclopropylamino group,
$R^{u1}$ represents $-CO-R^{u1'}$ ($R^{u1'}$ represents a substituent.), optionally substituted $C_{1-4}$ alkyl group, cycloalkyl group, or optionally substituted 6-membered non-aromatic heterocycle,
$R^{u2}$ represents an optionally halogenated $C_{1-2}$ alkyl group, and
$n_u$ represents an integer of 1 to 3.]; and
(6)
3-(3,6-dimethyl-4-oxo-4,5,6,7-tetrahydro-1H-indazol-1-yl)propyl 4-methylbenzenesulfonate;
3-(5-bromo-3,6-dimethyl-4,7-dioxo-4,7-dihydro-1H-indazol-1-yl)propyl 4-methylbenzenesulfonate;
3-(5-amino-3,6-dimethyl-4,7-dioxo-4,7-dihydro-1H-indazol-1-yl)propyl 4-methylbenzenesulfonate;
2-bromo-4-[(3-methyl-4-oxo-4,5,6,7-tetrahydro-1H-indazol-1-yl)methyl]benzonitrile;
[1-(4-fluorobenzyl)-1,4,5,6,7,8-hexahydrocyclohepta[c]pyrazol-3-yl]methanol;
[1-(4-methoxybenzyl)-1,4,5,6,7,8-hexahydrocyclohepta[c]pyrazol-3-yl]methanol;
methyl 4-ethyl-5-methyl-2-({2-[3-(trifluoromethyl)-5,6-dihydrocyclopenta[c]pyrazol-1(4H)-yl]butanoyl}amino) thiophene-3-carboxylate;
methyl 5-ethyl-2-({2-[3-(trifluoromethyl)-5,6-dihydrocyclopenta[c]pyrazol-1(4H)-yl]butanoyl}amino)thiophene-3-carboxylate; and
ethyl 4-methyl-2-({2-[3-(trifluoromethyl)-5,6-dihydrocyclopenta[c]pyrazol-1(4H)-yl]butanoyl}amino)-1,3-thiazole-5-carboxylate
are excluded.)

or a salt thereof.

2. The compound according to claim 1, wherein $R^1$ is an optionally halogenated $C_{1-6}$ alkyl.

3. The compound according to claim 1, wherein Ra and Rb are hydrogen atoms.

4. The compound according to claim 1, wherein L is a bond, $-CONH-$, $-CH_2CH_2CONH-$, $-CH_2CH(CH_3)CONH-$, $-CH_2CH_2CH_2CONH-$, $-CH_2CONH-$, $-CH_2NHCO-$, $-CH_2-$, or $-CH_2O-$.

5. The compound according to claim 1, wherein $R^1$ is an optionally halogenated $C_{1-6}$ alkyl,
Ra and Rb are hydrogen atoms, and
L is a bond, $-CONH-$, $-CH_2CH_2CONH-$, $-CH_2CH(CH_3)CONH-$, $-CH_2$ ONH-, $-CH_2CONH-$,$-CH_2NHCO-$, $-CH_2-$, or $-CH_2O-$. or

6. A compound selected from
1-{[[4-(pyrrolidin-1-ylcarbonyl)-1,3-thiazol-2-yl]methyl}-3-(trifluoromethyl)-4,5,6,7-tetrahydro-1H-indazole,
2-({[3-(trifluoromethyl)-4,5,6,7-tetrahydro-1H-pyrazolo[3,4-c]pyridin-1-yl]acetyl}amino)-4,5,6,7-tetrahydro-1-benzothiophene-3-carboxamide,
2-({[3-(trifluoromethyl)-4,5,6,7-tetrahydro-1H-pyrazolo[4,3-c]pyridin-1-yl]acetyl}amino)-4,5,6,7-tetrahydro-1-benzothiophene-3-carboxamide,
1,2-dimethyl-6-(methylsulfanyl)-N-{2-[3-(trifluoromethyl)-4,5,6,7-tetrahydro-1H-indazol-1-yl]ethyl}-1H-thieno[3,4-d]imidazole-4-carboxamide,
5-methyl-7-(trifluoromethyl)-3-(5-{[3-(trifluoromethyl)-4,5,6,7-tetrahydro-1H-indazol-1-yl]methyl}-1,2,4-oxadiazol-3-yl)pyrazolo[1,5-a]pyrimidine,
N-(5-chloro-2-methoxyphenyl)-2-[3-(trifluoromethyl)-4,5,6,7-tetrahydro-1H-pyrazolo[3,4-c]pyridin-1-yl]acetamide,
4-hydroxy-3-(methylsulfanyl)-N-{2-[3-(trifluoromethyl)-4,5,6,7-tetrahydro-1H-indazol-1-yl]ethyl}-4,5,6,7-tetrahydro-2-benzothiophene-1-carboxamide,
2-[5-acetyl-3-(trifluoromethyl)-4,5,6,7-tetrahydro-1H-pyrazolo[4,3-c]pyridin-1-yl]-N-(5-chloro-2-methoxyphenyl) acetamide,
5,7-dimethyl-3-(5-{[3-(trifluoromethyl)-4,5,6,7-tetrahydro-1H-indazol-1-yl]methyl}-1,2,4-oxadiazol-3-yl)pyrazolo [1,5-a]pyrimidine,
1-[(3-pyrazin-2-yl-1,2,4-oxadiazol-5-yl)methyl]-3-(trifluoromethyl)-4,5,6,7-tetrahydro-1H-indazole,
5-methyl-7-(trifluoromethyl)-3-(5-{[3-(trifluoromethyl)-5,6-dihydrocyclopenta[c]pyrazol-1(4H)-yl]methyl}-1,2,4-oxadiazol-3-yl)pyrazolo[1,5-a]pyrimidine,
1-{2-[(5-chloro-2-methoxyphenyl)amino]-2-oxoethyl}-N-methyl-3-(trifluoromethyl)-1,4,6,7-tetrahydro-5H-pyrazolo [4,3-c]pyridine-5-carboxamide,
1-[(3-thiophen-2-yl-1,2,4-oxadiazol-5-yl)methyl]-3-(trifluoromethyl)-1,4,6,7-tetrahydropyrano[4,3-c]pyrazole, and
1-[(5-thiophen-2-yl-1,3,4-oxadiazol-2-yl)methyl]-3-(trifluoromethyl)-4,5,6,7-tetrahydro-1H-indazole,
or a salt thereof.

7. A prodrug of the compound according to claim 1.

8. A pharmaceutical comprising the compound according to claim 1 or a prodrug thereof.

9. The pharmaceutical according to claim 8, which is an AMPA receptor potentiator.

10. The AMPA receptor potentiator according to claim 9, which is a drug for preventing or treating depression, schizophrenia, or attention-deficit hyperactivity disorder (ADHD).

11. An AMPA receptor potentiator comprising a compound represented by the formula

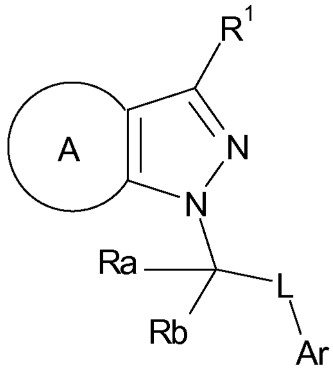
(I)

wherein
$R^1$ represents

(1) a halogen atom,
(2) cyano group,
(3) alkyl group optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,

carbamoyl group, and
mono- or di-alkylcarbamoyl groups,
(4) optionally substituted cycloalkyl group,
(5) group represented by the formula:

$-OR^{x1}$,
$-SR^{x2}$,
$-CO-R^{x2}$,
$-CS-R^{x2}$,
$-SO-R^{x2}$,
$-SO_2-R^{x2}$,
$-NH_2$, or
$-NR^{x1}R^{x2}$

(where $R^{x1}$ represents a substituent selected from the following substituent group A, and $R^{x2}$ represents hydrogen or a substituent selected from the following substituent group A),
or
(6) optionally substituted non-aromatic heterocyclic group;
Ra and Rb each independently represent a hydrogen atom or $C_{1-4}$ alkyl group;
L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1), either of which is optionally substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) optionally substituted cycloalkyl groups,
(e) groups represented by the formula:

$-OR^{y1}$,
$-SR^{y1}$,
$-CO- R^{y1}$,
$-CS- R^{y1}$,
$-SO- R^{y1}$,
$-SO_2-R^{y1}$, or
$-NR^{y1}R^{y2}$

(where $R^{y1}$ and $R^{y2}$ each independently represent hydrogen or 1 or 2 substituents selected from the following substituent group A),
(f) oxo group, and
(g) optionally substituted non-aromatic heterocyclic groups;

Ar represents
an optionally substituted aryl group, or optionally substituted 5- or 6-membered aromatic heterocyclic group (when the aryl group or aromatic heterocyclic group has 2 or more substituents, two adjacent substituents together may form an optionally substituted 5- to 8-membered ring).
Provided that, when L is a bond, Ar is not an unsubstituted phenyl group or unsubstituted 5- or 6-membered aromatic heterocyclic group.
Substituent group A consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,
(xii) $C_{3-8}$ cycloalkenyl groups,
(xiii) cross-linked $C_{7-10}$ cycloalkyl groups optionally substituted with $C_{1-6}$ alkyl group(s),
(xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfinyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms [in which the non-aromatic heterocyclic groups are optionally substituted with $C_{1-6}$ alkyl groups.

(Provided that

(1) compounds represented by the formula

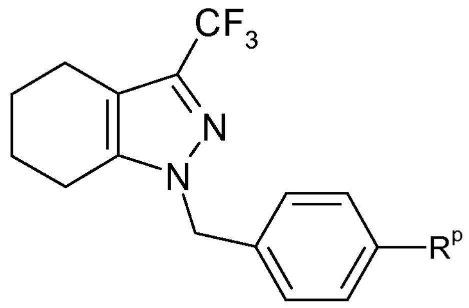

[wherein $R^p$ represents a substituent.];

(2) compounds represented by the formula

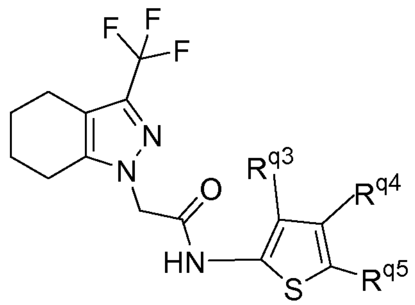

[wherein

$R^{q3}$ represents a hydrogen atom or substituent, and

$R^{q4}$ and $R^{q5}$, which may be the same or different, represent $C_{1-6}$ alkyl groups or are bonded together to form a 6-membered non-aromatic ring.];

(3) compounds represented by the formula

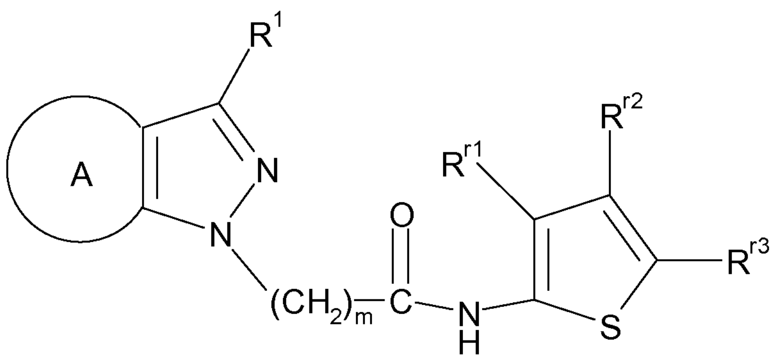

[wherein

$R^1$ represents trifluoromethyl,

$R^{r1}$ represents hydroxymethyl, carboxyl, or optionally substituted carbamoyl,

$R^{r2}$ and $R^{r3}$ may each independently represent hydrogen, a $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,

Ring A represents cyclohexene, and

m represents the integer 2.]

are excluded.)

or a salt thereof.

**12.** The AMPA receptor potentiator according to claim 11, which is a drug for preventing or treating depression, schizophrenia, or attention-deficit hyperactivity disorder (ADHD).

**13.** A method for preventing or treating diseases involving the AMPA receptor in mammals, comprising administering to such mammals a compound represented by the formula

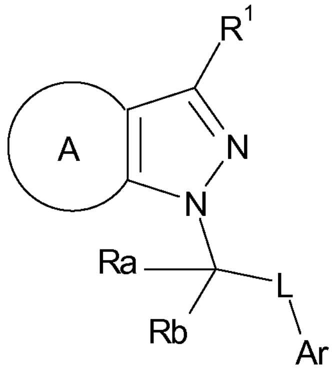
(I)

wherein
$R^1$ represents

(1) a halogen atom,
(2) cyano group,
(3) alkyl group optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carbamoyl group, and
mono- or di-alkylcarbamoyl groups,
(4) optionally substituted cycloalkyl group,
(5) group represented by the formula:

$-OR^{x1}$,
$-SR^{x2}$,
$-CO-R^{x2}$,
$-CS-R^{x2}$,
$-SO-R^{x2}$,
$-SO_2-R^{x2}$,
$-NH_2$, or
$-NR^{x1}R^{x2}$

(where $R^{x1}$ represents a substituent selected from the following substituent group A, and $R^{x2}$ represents hydrogen or a substituent selected from the following substituent group A),
or
(6) optionally substituted non-aromatic heterocyclic group;
Ra and Rb each independently represent a hydrogen atom or $C_{1-4}$ alkyl group;

L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1), either of which is optionally substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) optionally substituted cycloalkyl groups,
(e) groups represented by the formula:

$-OR^{y1}$,
$-SR^{y1}$,
$-CO- R^{y1}$,
$-CS- R^{y1}$,
$-SO- R^{y1}$,
$-SO_2-R^{y1}$, or
$-NR^{y1}R^{y2}$

(where $R^{y1}$ and $R^{y2}$ each independently represent hydrogen or 1 or 2 substituents selected from the following substituent group A),
(f) oxo group, and
(g) optionally substituted non-aromatic heterocyclic groups;

Ar represents
an optionally substituted aryl group, or optionally substituted 5- or 6-membered aromatic heterocyclic group (when the aryl group or aromatic heterocyclic group has 2 or more substituents, two adjacent substituents together may form an optionally substituted 5- to 8-membered ring).
Provided that, when L is a bond, Ar is not an unsubstituted phenyl group or unsubstituted 5- or 6-membered aromatic heterocyclic group.
Substituent group A consists of

(i) halogen atoms,
(ii) cyano group,
(iii) nitro group,
(iv) amino group,
(v) mono- or di-$C_{1-6}$ alkylamino groups,

(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,
(xii) $C_{3-8}$ cycloalkenyl groups,
(xiii) cross-linked $C_{7-10}$ cycloalkyl groups optionally substituted with $C_{1-6}$ alkyl group(s),
(xiv) hydroxy group,
(xv) $C_{1-6}$ alkoxy groups optionally substituted with halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfinyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms [in which the non-aromatic heterocyclic groups are optionally substituted with $C_{1-6}$ alkyl groups.

(Provided that

(1) compounds represented by the formula

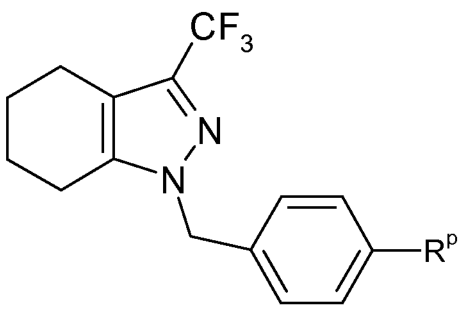

[wherein $R^p$ represents a substituent.];
(2) compounds represented by the formula

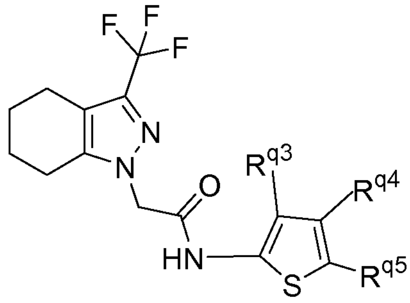

[wherein
$R^{q3}$ represents a hydrogen atom or substituent, and
$R^{q4}$ and $R^{q5}$, which may be the same or different, represent $C_{1-6}$ alkyl groups or are bonded together to form a 6-membered non-aromatic ring.];
(3) compounds represented by the formula

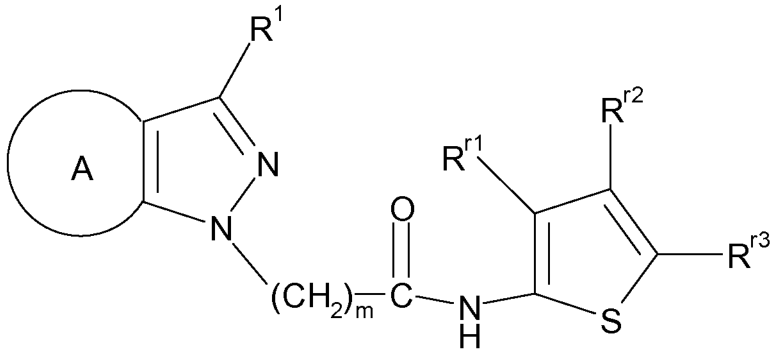

[wherein
$R^1$ represents trifluoromethyl,
$R^{r1}$ represents hydroxymethyl, carboxyl, or optionally substituted carbamoyl,
$R^{r2}$ and $R^{r3}$ may each independently represent hydrogen, a $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,
Ring A represents cyclohexene, and
m represents the integer 2.]

are excluded.)
or a salt thereof or a prodrug thereof.

**14.** The method according to claim 13, wherein the disease involving the AMPA receptor is depression, schizophrenia, or attention-deficit hyperactivity disorder (ADHD).

**15.** The use of a compound for the production of an AMPA receptor potentiator, represented by the formula

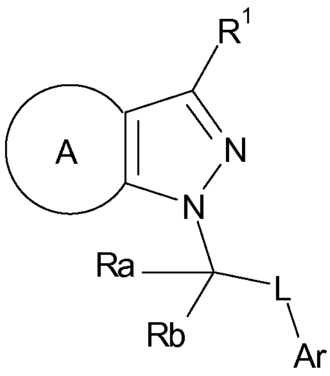

(I)

wherein
$R^1$ represents

(1) a halogen atom,
(2) cyano group,
(3) alkyl group optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carbamoyl group, and
mono- or di-alkylcarbamoyl groups,
(4) optionally substituted cycloalkyl group,
(5) group represented by the formula:

$-OR^{x1}$,
$-SR^{x2}$,
$-CO-R^{x2}$,
$-CS-R^{x2}$,
$-SO-R^{x2}$,
$-SO_2-R^{x2}$,
$-NH_2$, or
$-NR^{x1}R^{x2}$

(where $R^{x1}$ represents a substituent selected from the following substituent group A, and $R^{x2}$ represents hydrogen or a substituent selected from the following substituent group A),
or
(6) optionally substituted non-aromatic heterocyclic group;
Ra and Rb each independently represent a hydrogen atom or $C_{1-4}$ alkyl group;
L represents a bond, or a spacer in which the number of atoms in the main chain is 1 to 8;
Ring A represents

(1) a non-aromatic carbon ring of 4-8 carbon atoms, or
(2) 4- to 8-membered non-aromatic heterocycle which may have 1 to 3 hetero atoms selected from nitrogen, oxygen, and sulfur atoms (provided that, the number of nitrogen atoms is 0 or 1), either of which is optionally

substituted with one or more substituents selected from

(a) halogen atoms,
(b) cyano group,
(c) alkyl groups optionally substituted with substituent(s) selected from
halogen atoms,
cyano group,
hydroxy group,
alkoxy groups,
cycloalkyl groups,
amino group optionally substituted with 1 or 2 substituents selected from the following substituent group A,
mercapto group,
alkylsulfanyl groups,
alkylsulfinyl groups,
alkylsulfonyl groups,
mono- or di-alkyl-sulfamoyl groups,
alkanoyloxy groups,
alkanoyl groups,
carboxyl group,
alkoxycarbonyl groups,
carbamoyl group, and
mono- or di-alkylcarbamoyl groups,
(d) optionally substituted cycloalkyl groups,
(e) groups represented by the formula:

$-OR^{y1}$,
$-SR^{y1}$,
$-CO- R^{y1}$,
$-CS- R^{y1}$,
$-SO- R^{y1}$,
$-SO_2-R^{y1}$, or
$-NR^{y1}R^{y2}$

(where $R^{y1}$ and $R^{y2}$ each independently represent hydrogen or 1 or 2 substituents selected from the following substituent group A),
(f) oxo group, and
(g) optionally substituted non-aromatic heterocyclic groups;

Ar represents
an optionally substituted aryl group, or optionally substituted 5- or 6-membered aromatic heterocyclic group (when the aryl group or aromatic heterocyclic group has 2 or more substituents, two adjacent substituents together may form an optionally substituted 5- to 8-membered ring).
Provided that, when L is a bond, Ar is not an unsubstituted phenyl group or unsubstituted 5- or 6-membered aromatic heterocyclic group.
Substituent group A consists of

(i) halogen atoms,
(ii) cyano group,
(iii) nitro group,
(iv) amino group,
(v) mono- or di-$C_{1-6}$ alkylamino groups,
(vi) $C_{1-6}$ alkyl-carbonylamino groups,
(vii) $C_{1-6}$ alkoxy-carbonylamino groups,
(viii) ureido group,
(ix) $C_{1-6}$ alkyl-ureido groups,
(x) $C_{1-6}$ alkyl groups optionally substituted with halogen atom(s),
(xi) $C_{3-8}$ cycloalkyl groups,

(xii) $C_{3-8}$ cycloalkenyl groups,
(xiii) cross-linked $C_{7-10}$ cycloalkyl groups optionally substituted with $C_{1-6}$ alkyl group(s),
(xiv) hydroxy group,
(xv) $C_{1-6}$ alkoxy groups optionally substituted halogen atom(s),
(xvi) formyl group,
(xvii) carboxyl group,
(xviii) $C_{1-6}$ alkoxy-carbonyl groups,
(xix) $C_{1-6}$ alkyl-carbonyl groups,
(xx) $C_{3-8}$ cycloalkyl-carbonyl groups,
(xxi) carbamoyl group,
(xxii) mono- or di-$C_{1-6}$ alkyl-carbamoyl groups,
(xxiii) 3- to 8-membered non-aromatic heterocycle-carbonyl groups having 1 to 4 hetero atoms in addition to carbon atoms, selected from nitrogen, sulfur, and oxygen atoms,
(xxiv) thiocarbamoyl group,
(xxv) mercapto group,
(xxvi) $C_{1-6}$ alkylsulfanyl groups,
(xxvii) $C_{1-6}$ alkylsulfinyl groups,
(xxviii) $C_{1-6}$ alkylsulfonyl groups,
(xxix) $C_{3-8}$ cycloalkylsulfonyl groups,
(xxx) aminosulfonyl group,
(xxxi) mono- or di-N-$C_{1-6}$ alkylaminosulfonyl groups, and
(xxxii) 3- to 8-membered non-aromatic heterocyclic groups having 1 to 4 hetero atoms in addition to carbon atoms, selected from nitrogen, sulfur, and oxygen atoms ,in which the non-aromatic heterocyclic groups are optionally substituted with $C_{1-6}$ alkyl groups.

(Provided that

(1) compounds represented by the formula

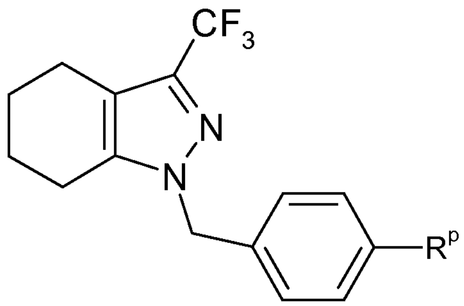

[wherein $R^p$ represents a substituent.];
(2) compounds represented by the formula

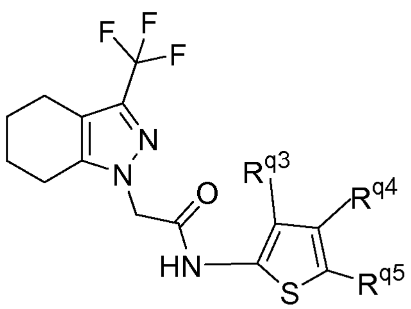

[wherein
$R^{q3}$ represents a hydrogen atom or substituent, and

$R^{q4}$ and $R^{q5}$, which may be the same or different, represent $C_{1-6}$ alkyl groups or are bonded together to form a 6-membered non-aromatic ring.];
(3) compounds represented by the formula

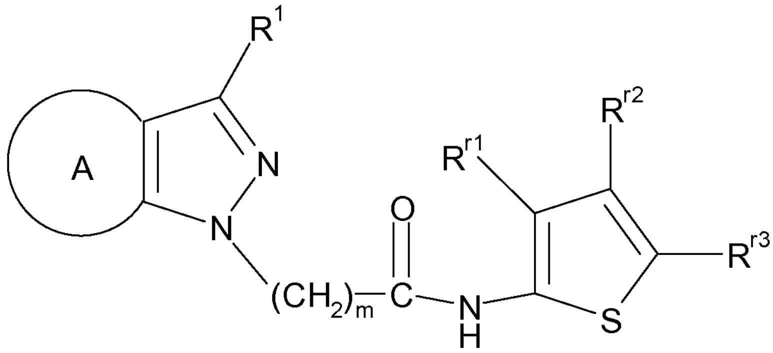

[wherein
$R^1$ represents trifluoromethyl,
$R^{r1}$ represents hydroxymethyl, carboxyl, or optionally substituted carbamoyl,
$R^{r2}$ and $R^{r3}$ may each independently represent hydrogen, a $C_{1-4}$ alkyl, or $C_{3-8}$ cycloalkyl, or $R^{r2}$ and $R^{r3}$ may together form a unsaturated carbon ring of 5-6 carbon atoms or a 5- or 6-membered unsaturated heterocycle having 1 or more hetero atoms in addition to carbon atoms, selected from nitrogen, sulfur, and oxygen atoms,
Ring A represents cyclohexene, and
m represents the integer 2.]
are excluded.)

or a salt thereof or a prodrug thereof.

**16.** The use according to claim 15, wherein the AMPA receptor potentiator is a drug for preventing or treating depression, schizophrenia, or attention-deficit hyperactivity disorder (ADHD).

## INTERNATIONAL SEARCH REPORT

International application No. PCT/JP2009/001337

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | FR 2875230 A1 (Sanofi Aventis), 17 March, 2006 (17.03.06), Preparation 3.8-3.10 & WO 2006/030124 A1 | 1,3,4 |
| X | JP 5-221998 A (Ortho Pharmaceutical Corp.), 31 August, 1993 (31.08.93), Compound No.186 & US 5134155 A & US 5315012 A & US 5387693 A & US 5250561 A & US 5134155 A | 1,3,4,7,8 |
| A | WO 2007/107539 A1 (GLAXO GROUP LTD.), 27 September, 2007 (27.09.07), Full text & US 2008/0045532 A1 & EP 1996555 A1 | 1-12,15,16 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
15 April, 2009 (15.04.09)

Date of mailing of the international search report
28 April, 2009 (28.04.09)

Name and mailing address of the ISA/
Japanese Patent Office

Facsimile No.

Authorized officer

Telephone No.

Form PCT/ISA/210 (second sheet) (April 2007)

**INTERNATIONAL SEARCH REPORT**

International application No.
PCT/JP2009/001337

C (Continuation). DOCUMENTS CONSIDERED TO BE RELEVANT

| Category* | Citation of document, with indication, where appropriate, of the relevant passages | Relevant to claim No. |
|---|---|---|
| A | WO 2008/003452 A1 (N.V.ORGANON),<br>10 January, 2008 (10.01.08),<br>Full text<br>& US 2008/0255086 A1 | 1-12,15,16 |

Form PCT/ISA/210 (continuation of second sheet) (April 2007)

INTERNATIONAL SEARCH REPORT

International application No.
PCT/JP2009/001337

Continuation of A. CLASSIFICATION OF SUBJECT MATTER
(International Patent Classification (IPC))

*C07D231/56*(2006.01)i, *A61K31/416*(2006.01)i, *A61K31/4178*(2006.01)i, *A61K31/4245*(2006.01)i, *A61K31/427*(2006.01)i, *A61K31/437*(2006.01)i, *A61K31/4439*(2006.01)i, *A61K31/496*(2006.01)i, *A61K31/4985*(2006.01)i, *A61K31/519*(2006.01)i, *A61K31/55*(2006.01)i, *A61P25/14*(2006.01)i, *A61P25/18*(2006.01)i, *A61P25/24*(2006.01)i, *A61P25/28*(2006.01)i, *A61P43/00*(2006.01)i, *C07D401/06*(2006.01)i, *C07D401/12*(2006.01)i, *C07D401/14*(2006.01)i, *C07D403/12*(2006.01)i, *C07D405/14*(2006.01)i, *C07D409/12*(2006.01)i, *C07D409/14*(2006.01)i, *C07D413/06*(2006.01)i, *C07D413/12*(2006.01)i, *C07D413/14*(2006.01)i, *C07D417/06*(2006.01)i, *C07D417/12*(2006.01)i, *C07D417/14*(2006.01)i, *C07D471/04*(2006.01)i, *C07D487/04*(2006.01)i, *C07D491/052*(2006.01)i, *C07D495/04*(2006.01)i

(According to International Patent Classification (IPC) or to both national classification and IPC)

Continuation of B. FIELDS SEARCHED
Minimum documentation searched (International Patent Classification (IPC))

C07D231/56, A61K31/416, A61K31/4178, A61K31/4245, A61K31/427, A61K31/437, A61K31/4439, A61K31/496, A61K31/4985, A61K31/519, A61K31/55, A61P25/14, A61P25/18, A61P25/24, A61P25/28, A61P43/00, C07D401/06, C07D401/12, C07D401/14, C07D403/12, C07D405/14, C07D409/12, C07D409/14, C07D413/06, C07D413/12, C07D413/14, C07D417/06, C07D417/12, C07D417/14, C07D471/04, C07D487/04, C07D491/052, C07D495/04

Minimum documentation searched (classification system followed by classification symbols)

Form PCT/ISA/210 (extra sheet) (April 2007)

**INTERNATIONAL SEARCH REPORT**

International application No.
PCT/JP2009/001337

**Box No. II Observations where certain claims were found unsearchable (Continuation of item 2 of first sheet)**

This international search report has not been established in respect of certain claims under Article 17(2)(a) for the following reasons:

1. [X] Claims Nos.: 13, 14
because they relate to subject matter not required to be searched by this Authority, namely:
Claims 13 and 14 include the methods for treatment of the human body or animal body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007107539 A
- WO 2008003452 A

### Non-patent literature cited in the description

- Iyakuhin No Kaihatsu. Molecular Design. Hirokawa Shoten, 1990, vol. 7, 163-198
- *Bioorg. Med. Chem. Lett.,* 1997, vol. 7 (1), 733

SEQUENCE LISTING

<110> Takeda Pharmaceutical Company Limited

<120> Heterocyclic compound

<130> TKDWO080136P

<150> JP 2008-077479
<151> 2008-03-25

<160> 4

<170> PatentIn version 3.4

<210> 1
<211> 42
<212> DNA
<213> Artificial

<220>
<223> Forward primer for GluR1 flip cDNA

<400> 1
actgaattcg ccaccatgca gcacattttt gccttcttct gc 42

<210> 2
<211> 32
<212> DNA
<213> Artificial

<220>
<223> Reverse primer for GluR1 flip cDNA

<400> 2
ccgcggccgc ttacaatccc gtggctccca ag 32

<210> 3
<211> 41
<212> DNA
<213> Artificial

<220>
<223> Forward primer for stargazin cDNA

<400> 3
ggtctcgagg ccaccatggg gctgtttgat cgaggtgttc a 41

<210> 4
<211> 40
<212> DNA
<213> Artificial

<220>
<223> Reverse primer for stargazin cDNA

<400> 4
gttggatcct tatacggggg tggtccggcg gttggctgtg 40